# EUROPEAN PATENT APPLICATION

(11) **EP 3 238 745 A1**
(43) Date of publication of application: **01.11.2017**
(21) Application number: 15873156.2
(22) Date of filing: 24.12.2015
(51) Int. Cl.: A61K 45/06, A61K 31/7105, A61K 31/711, A61P 35/00, A61P 43/00, C12N 15/113, C12Q 1/02

(54) **CELL DEATH-INDUCING AGENT, CYTOSTATIC AGENT, AND PHARMACEUTICAL COMPOSITION FOR TREATMENT OF DISEASES CAUSED BY ABNORMAL CELL GROWTH**

(30) Priority: 26.12.2014 JP 2014266198; 06.07.2015 JP 2015135494; 18.12.2015 JP 2015247725
(71) Applicant: Nitto Denko Corporation, Ibaraki-shi, Osaka 567-8680 (JP)
(72) Inventor: TANAKA Hiroyuki, Ibaraki-shi Osaka 567-8680 (JP); MINOMI Kenjirou, Ibaraki-shi Osaka 567-8680 (JP); NIITSU Yoshiro, Sapporo-shi Hokkaido 064-0823 (JP)
(74) Representative: Hoffmann Eitle
(86) International application number: PCT/JP2015/085991
(87) International publication number: WO 2016/104588

(57) **Abstract**

It is intended to induce cell death and inhibit cell growth for cancer cells.

The agents of the present invention comprise, as active ingredients, a drug inhibiting GST-π and a drug inhibiting a homeostasis-related protein that exhibits synthetic lethality when inhibited together with GST-π.

## Description

### Technical Field

The present invention relates to a cell death-inducing agent and a cell growth-inhibiting agent for a cancer cell, and a pharmaceutical composition for the treatment of a disease caused by abnormal cell growth and further relates to a method for screening for a cell death-inducing agent and/or a cell growth-inhibiting agent.

### Background Art

Typical examples of diseases caused by abnormal cell growth can include cancers. Cancers are diseases in which cells grow in an uncontrolled manner due to mutations, epigenetic abnormalities, etc., in genes. A large number of gene abnormalities in cancers have already been reported (e.g., Futreal et al., Nat Rev Cancer. 2004; 4 (3): 177-83), most of which are considered to have some relation to signal transduction involved in cell growth, differentiation, or survival. Moreover, such gene abnormalities cause abnormal signal transduction in cells constituted by normal molecules. This may bring about the activation or deactivation of a particular signal cascade and eventually become partly responsible for the abnormal growth of the cells.

Although a principal objective of cancer treatment in early times was to inhibit cell growth itself, such treatment physiologically inhibited even the growth of normal cells and therefore involved adverse reactions such as alopecia, gastrointestinal disturbances, and myelosuppression. Accordingly, therapeutic drugs for cancers based on novel ideas such as molecular target drugs, which target cancer-specific gene abnormalities or abnormal signal transduction, are under development in order to prevent such adverse reactions.

A cancer is thought to occur by the accumulation of abnormalities in various cancer genes, tumor suppressor genes, DNA repair enzyme genes, and the like in the same cell. RAS gene, FOS gene, MYC gene, and BCL-2 gene, etc., are known as the cancer genes. Among cancer-specific gene abnormalities, a mutation is found in KRAS gene in approximately 95% pancreatic cancer, approximately 45% colorectal cancer, and many other cancers with high frequency. The KRAS protein is a G protein that is localized to the inner side of a cell membrane. RAS including KRAS forms a cascade where RAS activates RAF such as C-RAF or B-RAF, and subsequently, this RAF activates MEK, which then activates MAPK. When a point mutation takes place in KRAS, GTPase activity is reduced so that GTP-bound active forms are maintained to thereby constitutively sustain signals to downstream pathway, resulting in abnormal cell growth. As typified by the KRAS gene, the cancer genes cause abnormal cell growth which in turn progresses to the malignant transformation of the cell and eventually a cancer as a disease.

Incidentally, glutathione-S-transferase (GST), an enzyme catalyzing glutathione conjugation, is known as an enzyme that converts a substance such as a drug to a watersoluble substance through coupling with glutathione (GSH). GST is typically classified, on the basis of amino acid sequences, into 6 types of isozymes: α, µ, ω, π, θ, and ζ. Among them, particularly, the expression of GST-π (glutathione S-transferase pi, also called GSTP1) is increased in various cancer cells. The possibility has been pointed out that this is partly responsible for resistance to some anticancer agents. In fact, it is known that when an antisense DNA against GST-π or a GST-π inhibitor is allowed to act on a GST-π-overexpressing cancer cell line that exhibits drug resistance, the drug resistance is inhibited (Takahashi and Niitsu, Gan To Kagaku Ryoho. 1994; 21 (7): 945-51; Ban et al., Cancer Res. 1996; 56 (15): 3577-82; and Nakajima et al., J Pharmacol Exp Ther. 2003; 306 (3): 861-9). In addition, a recent report has showed that when an siRNA against GST-π is allowed to act on a GST-π-overexpressing androgen-independent prostate cancer cell line, its growth is inhibited so that apoptosis is increased (Hokaiwado et al., Carcinogenesis. 2008; 29 (6): 1134-8).

It is also known that GST-π forms a complex with c-Jun N-terminal kinase (JNK) to inhibit JNK activity (Adler et.al, EMBO J. 1999, 18, 1321-1334). It is further known that GST-π participates in the S-glutathionylation of proteins associated with the stress response of cells (Townsend, et.al, J. Biol. Chem. 2009, 284, 436-445). In addition, it is known that GST-π contributes to a protective effect against cell death induced by reactive oxygen species (ROS) (Yin et.al, Cancer Res. 2000 60, 4053-4057). Thus, it can be understood that among the GST isozymes, GST-π has various features and functions.

It has been reported that when an siRNA against GST-π is allowed to act on a cancer cell line having a mutation in KRAS, the activation of Akt is inhibited so that autophagy is enhanced whereas apoptosis is moderately induced (Nishita et al., AACR 102nd Annual Meeting, Abstract No. 1065). WO2012/176282 discloses that use of a drug inhibiting GST-π and an autophagy inhibitor such as 3-methyladenine as active ingredients can induce the apoptosis of cancer cells. Furthermore, WO2014/098210 discloses that: when the expression of GST-π and the expression of Akt or the like are inhibited at the same time, cell growth is inhibited to induce cell death; and autophagy induced by the inhibition of GST-π expression is significantly inhibited by inhibiting the expression of GST-π and the expression Akt or the like at the same time.

However, much still remains unknown about the relation of the expression of GST-π in cancer cells to cell growth or cell death, the role of GST-π in signal transduction, etc.

### Summary of Invention

### Object to Be Attained by the Invention

Thus, an object of the present invention is to provide an agent having a cell death-inducing effect and/or a cell growth-inhibiting effect on a cancer cell, to provide a pharmaceutical composition for the treatment of a disease caused by abnormal cell growth, and to provide a method for screening for a cell death-inducing agent and/or a cell growth-inhibiting agent.

### Means for Attaining the Object

The present inventors have conducted diligent studies in light of the object mentioned above and consequently completed the present invention by finding that a homeostasis-related protein that exhibits synthetic lethality when inhibited together with GST-π is inhibited along with the inhibition of GST-π in a cancer cell, whereby cell death is more strongly induced and cell growth is more strongly inhibited as compared with the case where either of them is inhibited. The present invention encompasses the following:
(1) A cell death-inducing agent for a cancer cell comprising, as active ingredients, a drug inhibiting GST-π and a drug inhibiting a homeostasis-related protein that exhibits synthetic lethality when inhibited together with GST-π.
(2) A cell growth-inhibiting agent for a cancer cell comprising, as active ingredients, a drug inhibiting GST-π and a drug inhibiting a homeostasis-related protein that exhibits synthetic lethality when inhibited together with GST-π.
(3) The agent according to (1) or (2), wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.
(4) The agent according to (3), wherein the cell cycle-regulating protein that exhibits synthetic lethality along with the inhibition of GST-π is at least one cell cycle-regulating protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK.
(5) The agent according to (3), wherein the cell cycle-regulating protein that exhibits synthetic lethality along with the inhibition of GST-π is at least one protein selected from the group consisting of p21, RNPC1, CCNL1, MCM8, CCNB3, and MCMDC1.
(6) The agent according to (3), wherein the anti-apoptosis-related protein that exhibits synthetic lethality along with the inhibition of GST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.
(7) The agent according to (3), wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.
(8) The agent according to (1) or (2), wherein the drug is a substance selected from the group consisting of an RNAi molecule, a ribozyme, an antisense nucleic acid, a DNA/RNA chimeric polynucleotide, and a vector for expressing at least one of them.
(9) The agent according to (1) or (2), wherein the drug inhibiting a homeostasis-related protein is a compound that acts on the homeostasis-related protein.
(10) The agent according to (1), wherein the agent induces apoptosis.
(11) The agent according to (1) or (2), wherein the cancer cell is a cancer cell highly expressing GST-π.
(12) A pharmaceutical composition for the treatment of a disease caused by abnormal cell growth, comprising an agent according to any of (1) to (11).
(13) The pharmaceutical composition according to (12), wherein the disease is a cancer.
(14) The pharmaceutical composition according to (13), wherein the cancer is a cancer highly expressing GST-π.
(15) A method for screening for a cell death-inducing agent and/or a cell growth-inhibiting agent for a cancer cell that is used together with a drug inhibiting GST-π, comprising a step of selecting a drug inhibiting a homeostasis-related protein that exhibits synthetic lethality when inhibited together with GST-π.
(16) The screening method according to (14), wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.
(17) The screening method according to (16), wherein the cell cycle-regulating protein that exhibits synthetic lethality along with the inhibition of GST-π is at least one protein selected from the group consisting of ATM, CDC25A, p21, PRKDC, RBBP8, SKP2, MCM 10, RNPC1, CCNL1, CENPH, BRSK1, MCM8, CCNB3, MCMDC1 and MYLK.
(18) The screening method according to (16), wherein the cell cycle-regulating protein that exhibits synthetic lethality along with the inhibition of GST-π is at least one protein selected from the group consisting of p21, RNPC1, CCNL1, MCM8, CCNB3, and MCMDC1.
(19) The screening method according to (16), wherein the anti-apoptosis-related protein that exhibits synthetic lethality along with the inhibition of GST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.
(20) The screening method according to (16), wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.
(21) The screening method according to any of (15) to (20), comprising the steps of: contacting a test substance with a cancer cell; measuring the expression level of the homeostasis-related protein in the cell; and selecting the test substance as a drug inhibiting the homeostasis-related protein when the expression level is decreased compared with that measured in the absence of the test substance.
(22) A method for screening for a cell death-inducing agent and/or cell growth-inhibiting agent for a cancer cell that is used together with a drug inhibiting a homeostasis-related protein that exhibits synthetic lethality when inhibited together with GST-π, comprising a step of selecting a drug inhibiting GST-π.
(23) The screening method according to (22), wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.
(24) The screening method according to (22), wherein the cell cycle-regulating protein that exhibits synthetic lethality along with the inhibition of GST-π is at least one protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK.
(25) The screening method according to (23), wherein the cell cycle-regulating protein that exhibits synthetic lethality along with the inhibition of GST-π is at least one protein selected from the group consisting of p21, RNPC1, CCNL1, MCM8, CCNB3, and MCMDC1.
(26) The screening method according to (23), wherein the anti-apoptosis-related protein that exhibits synthetic lethality along with the inhibition of GST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.
(27) The screening method according to (23), wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.
(28) The screening method according to any of (22) to (27), comprising the steps of: contacting a test substance with a cancer cell; measuring the expression level of GST-π in the cell; and selecting the test substance as a drug inhibiting GST-π when the expression level is decreased compared with that measured in the absence of the test substance.
(29) A method for screening for a cell death-inducing agent and/or a cell growth-inhibiting agent, comprising a step of selecting a drug inhibiting GST-π and homeostasis-related protein that exhibits synthetic lethality when inhibited together with GST-π.
(30) The screening method according to (29), wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.
(31) The screening method according to (30), wherein the cell cycle-regulating protein that exhibits synthetic lethality along with the inhibition of GST-π is at least one cell cycle-regulating protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK.
(32) The screening method according to (30), wherein the cell cycle-regulating protein that exhibits synthetic lethality along with the inhibition of GST-π is at least one protein selected from the group consisting of p21, RNPC1, CCNL1, MCM8, CCNB3, and MCMDC1.
(33) The screening method according to (30), wherein the anti-apoptosis-related protein that exhibits synthetic lethality along with the inhibition of GST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.
(34) The screening method according to (30), wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.
(35) The screening method according to any of (29) to (34), comprising the steps of: contacting a test substance with a cancer cell; measuring the expression level of GST-π and the expression level of the homeostasis-related protein that exhibits synthetic lethality when inhibited together with GST-π, in the cell; and selecting the test substance as a drug inhibiting GST-π and the homeostasis-related protein that exhibits synthetic lethality when inhibited together with GST-π when the expression level of GST-π and the expression level of the homeostasis-related protein that exhibits synthetic lethality when inhibited together with GST-π are both decreased compared with those measured in the absence of the test substance.

This description includes part or all of the contents as disclosed in the description and/or drawings of Japanese Patent Application No. 2014-266198, Japanese Patent Application No. 2015-135494 and Japanese Patent Application No. 2015-247725, which are priority documents of the present application.

### Effects of the Invention

The cell death-inducing agent according to the present invention can very strongly induce cell death for a cancer cell. Accordingly, the cell death-inducing agent according to the present invention can exert very high efficacy as a pharmaceutical composition for the treatment of a disease caused by the abnormal growth of the cancer cell.

Moreover, the cell growth-inhibiting agent according to the present invention can very strongly inhibit cell growth for a cancer cell. Accordingly, the cell growth-inhibiting agent according to the present invention can exert very high efficacy as a pharmaceutical composition for the treatment of a disease caused by the abnormal growth of the cancer cell.

Furthermore, the screening method according to the present invention can select a drug that very strongly induces cell death and/or inhibits cell growth for a cancer cell.

### BRIEF DESCRIPTION OF THE DRAWINGS

Figure 1 is a characteristic diagram showing results of assaying GST-π mRNA and p21 mRNA in cells expressing mutated KRAS when an siRNA inhibiting the expression of GST-π and/or an siRNA inhibiting the expression of p21 was allowed to act thereon.
Figure 2 is a characteristic diagram showing results of quantifying over time p21 mRNA when GST-π and p21 were both knocked down.
Figure 3 is a characteristic diagram showing results of measuring the number of cells when GST-π and p21 were both knocked down.
Figure 4 is a characteristic diagram showing results of measuring the number of cells when GST-π and p21 were both knocked down three times.
Figure 5 is a characteristic diagram showing results of measuring the number of cells when GST-π and p21 were both knocked down three times.
Figure 6 is a photograph taken for the phase difference image of A549 cells when GST-π and p21 were both knocked down three times.
Figure 7 is a photograph taken for the phase difference image of MIA PaCa-2 cells when GST-π and p21 were both knocked down three times.
Figure 8 is a photograph taken for the phase difference image of PANC-1 cells when GST-π and p21 were both knocked down three times.
Figure 9 is a photograph taken for the phase difference image of HCT116 cells when GST-π and p21 were both knocked down three times.
Figure 10 is a photograph taken for the phase difference image of M7609 cells when GST-π was knocked down three times and β-galactosidase staining was carried out.
Figure 11 is a characteristic diagram showing results of quantifying the expression of PUMA gene when GST-π and p21 were both knocked down.
Figure 12 is a characteristic diagram showing results of comparing relative survival rates when GST-π and a candidate protein (cell cycle-regulating protein) exhibiting synthetic lethality were knocked down each alone and when GST-π and the candidate protein were both knocked down.
Figure 13 is a characteristic diagram showing results of comparing relative survival rates when GST-π and a candidate protein (anti-apoptosis-related protein) exhibiting synthetic lethality were knocked down each alone and when GST-π and the candidate protein were both knocked down.
Figure 14 is a characteristic diagram showing results of comparing relative survival rates when GST-π and a candidate protein (PI3K signaling pathway-related protein) exhibiting synthetic lethality were knocked down each alone and when GST-π and the candidate protein were both knocked down.
Figure 15 is a characteristic diagram showing results of comparing relative survival rates when GST-π and MYLK were knocked down each alone and when GST-π and MYLK were both knocked down.

### DESCRIPTION OF THE PREFERRED EMBODIMENTS

The cell death-inducing agent and the cell growth-inhibiting agent according to the present invention comprise, as active ingredients, a drug inhibiting GST-π and a drug inhibiting a homeostasis-related protein that exhibits synthetic lethality when inhibited together with GST-π. The cell death-inducing agent and the cell growth-inhibiting agent according to the present invention exhibit a cell death-inducing effect and a cell growth-inhibiting effect on a cancer cell. In this context, the cancer cell is a cell that exhibits abnormal growth attributed to genes (cancer-related genes).

Of the cancer-related genes, examples of cancer genes can include KRAS gene, FOS gene, MYC gene, BCL-2 gene, and SIS gene. Also, of the cancer-related genes, examples of tumor suppressor genes can include RB gene, p53 gene, BRCA1 gene, NF1 gene, and p73 gene. However, the cancer cell is not limited to cancer cells in which these specific cancer-related genes are involved, and the agents of the present invention can be applied to a wide range of cells that exhibit abnormal cell growth.

Particularly, it is preferable that the cell death-inducing agent and the cell growth-inhibiting agent according to the present invention should be applied to a cancer cell highly expressing GST-π, among the cancer cells. In this context, the cancer cell highly expressing GST-π means a cell having a significantly higher expression level of GST-π than that of a normal cell, among the cells that exhibit abnormal cell growth (so-called cancer cells). The expression level of GST-π can be measured according to a standard method such as RT-PCR or microarrays.

In many cases, one example of the cancer cell highly expressing GST-π can include a cancer cell expressing mutated KRAS. Specifically, it is preferable that the cell death-inducing agent and the cell growth-inhibiting agent according to the present invention should be applied to the cancer cell expressing mutated KRAS.

The mutated KRAS means a protein having an amino acid sequence in which mutation(s) such as deletion, substitution, addition, and/or insertion is introduced in the amino acid sequence of wild-type KRAS. In this context, the mutation in the mutated KRAS is a so-called gain of function mutation. Specifically, in the cell expressing mutated KRAS, for example, GTPase activity is reduced due to the mutation so that GTP-bound active forms are maintained to thereby constitutively sustain signals to downstream pathway, resulting in abnormal cell growth as compared with the cell expressing wild-type KRAS. Examples of a gene encoding the mutated KRAS include a gene having a mutation at at least one of codon 12, codon 13, and codon 61 in the wild-type KRAS gene. Particularly, mutations at codons 12 and 13 are preferable for the mutated KRAS. Specific examples thereof include mutations by which glycine encoded by codon 12 of the KRAS gene is replaced with serine, aspartic acid, valine, cysteine, alanine, or arginine, and mutations by which glycine encoded by codon 13 of the KRAS gene is changed to aspartic acid.

As used herein, GST-π refers to an enzyme that is encoded by GSTP1 gene and catalyzes glutathione conjugation. GST-π is present in various animals including humans, and its sequence information is also publicly known (e.g., human: NM_000852 (NP_000843), rat: NM_012577 (NP_036709), mouse: NM_013541 (NP_038569), etc.; the numbers represent accession numbers of the NCBI database, and the nucleotide sequence and the amino acid sequence are indicated outside and inside the parentheses, respectively). As one example, the nucleotide sequence of the coding region of the human GST-π gene registered in the database is shown in SEQ ID NO: 1, and the amino acid sequence of the human GST-π protein encoded by this human GST-π gene is shown in SEQ ID NO: 2.

As used herein, the homeostasis-related protein that exhibits synthetic lethality when inhibited together with GST-π is a protein that results in a significantly high death rate of a cancer cell when inhibited together with GST-π as compared with the death rate of the cancer cell brought about by the inhibition of GST-π alone. This protein has the function of participating in cell homeostasis. In this context, the synthetic lethality means a phenomenon in which lethality is exerted or significantly enhanced for a cell or an individual by a combination of defects of a plurality of genes, though only one of the gene defects leads to no or low lethality. Particularly, in the present specification, the synthetic lethality means lethality for a cancer cell.

In the present specification, examples of the homeostasis-related protein that exhibits synthetic lethality when inhibited together with GST-π can include a cell cycle-regulating protein, an anti-apoptosis-related protein, and a PI3K signaling pathway-related protein. The cell cycle-regulating protein is a protein having the function of regulating cell cycle. The anti-apoptosis-related protein is a protein having the function of suppressively participating in apoptosis. The PI3K signaling pathway-related protein is a protein, except for Akt1, included in proteins involved in the PI3K/AKT signaling pathway.

Also, the protein having the function of regulating cell cycle is meant to include every protein involved in cell cycle consisting of the G1 phase (resting stage before DNA replication), the S phase (DNA synthesis stage), G2 (resting stage before cell division), and the M phase (cell division stage). More specifically, examples of the regulation of cell cycle can include each event of the regulation of the mechanism of promoting the G1 phase → the S phase → the G2 phase → the M phase in order, the regulation of progression at the G1 phase to the S phase, and the regulation of progression at the G2 phase to the M phase. Thus, the cell cycle-regulating protein can be, for example, a protein that participates in the progression of these events in cell cycle and a protein that positively or negatively regulates these events. Further specifically, examples of the cell cycle-regulating protein include cyclin-dependent kinases (CDKs) essential for the initiation of the S phase and the M phase. The activity of the cyclin-dependent kinases is positively regulated by the binding of cyclins. Also, the activity of the cyclin-dependent kinases is negatively regulated by cyclin-dependent kinase inhibitors (CKIs) such as p21 (CIP1/WAF1) and tyrosine kinases. Thus, these proteins regulating the activity of the cyclin-dependent kinases, i.e., cyclins, cyclin-dependent kinase inhibitors (such as p21), and tyrosine kinases, are also included in the cell cycle-regulating protein.

Specifically, examples of the cell cycle-regulating protein that exhibits synthetic lethality when inhibited together with GST-π can include at least one cell cycle-regulating protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK. Of these 15 types of cell cycle-regulating proteins, one type of cell cycle-regulating protein may be inhibited together with GST-π, or two or more types of cell cycle-regulating proteins may be inhibited together with GST-π.

Particularly, it is preferable for the cell cycle-regulating protein that at least one cell cycle-regulating protein selected from the group consisting of p21, RNPC1, CCNL1, MCM8, CCNB3, and MCMDC1 should be inhibited together with GST-π. These 6 types of cell cycle-regulating proteins have a relatively low rate of cell growth inhibition when inhibited each alone, and exhibit a remarkably high cell growth-inhibiting effect only when inhibited together with GST-π. That is, it can be said that a drug inhibiting any of these 6 types of cell cycle-regulating proteins is excellent in safety by itself. Thus, it is preferable that the cell cycle-regulating protein that exhibits synthetic lethality when inhibited together with GST-π should be selected from these 6 types of cell cycle-regulating proteins.

p21 is a cell cycle-regulating protein that is encoded by CDKN1A gene and belongs to the CIP/KIP family. This protein has the function of inhibiting cell cycle progression at the G1 phase and the G2/M phase by inhibiting the effect of a cyclin-CDK complex through binding to the complex. Specifically, the p21 gene undergoes activation by p53 (one of tumor suppressor genes). It has been reported that upon activation of p53 due to DNA damage or the like, p53 activates p21 so that the cell cycle is arrested at the G1 phase and the G2/M phase. In addition, p21 also has the function of inhibiting apoptosis and has been reported to have the effect of protecting a cell from apoptosis induced by a chemotherapeutic agent or the like in *in vitro* and animal experiments (Gartel and Tyner, 2002; and Abbs and Dutta, 2009). p21 is present in various animals including humans, and its sequence information is also publicly known (e.g., human: NM_000389.4, NM_078467.2, NM_001291549.1, NM_001220778.1, NM_001220777.1 (NP_001207707.1, NP_001278478.1, NP_001207706.1, NP_510867.1, NP_000380.1), etc.; the numbers represent accession numbers of the NCBI database, and the nucleotide sequence and the amino acid sequence are indicated outside and inside the parentheses, respectively). As one example, the nucleotide sequence of the human CDKN1A gene registered in the database as NM_000389.4 is shown in SEQ ID NO: 3, and the amino acid sequence of the human p21 protein encoded by this human CDKN1A gene is shown in SEQ ID NO: 4. In the present specification, p21 is not limited to a protein consisting of the amino acid sequence of SEQ ID NO: 4 encoded by the nucleotide sequence of SEQ ID NO: 3. As for p21, sequence information has been registered with a plurality of accession numbers as mentioned above, and a plurality of transcript variants are present. The nucleotide sequence of SEQ ID NO: 3 represents the nucleotide sequence of one of these transcript variants.

RNPC1 is an RNA-binding protein encoded by RNPC1 gene and refers to a protein that is targeted by p53. RNPC1 is present in various animals including humans, and its sequence information is also publicly known (e.g., human: NM_017495.5, NM_183425.2, NM_001291780.1, XM_005260446.1 (XP_005260503.1, NP_059965.2, NP_906270.1, NP_001278709.1), etc.; the numbers represent accession numbers of the NCBI database, and the basic acid sequence and the amino acid sequence are indicated outside and inside the parentheses, respectively). As one example, the nucleotide sequence of the human RNPC1 gene registered in the database as NM_017495.5 is shown in SEQ ID NO: 5, and the amino acid sequence of the human RNPC 1 protein encoded by this human RNPC 1 gene is shown in SEQ ID NO: 6. In the present specification, RNPC1 is not limited to a protein consisting of the amino acid sequence of SEQ ID NO: 6 encoded by the nucleotide sequence of SEQ ID NO: 5. As for RNPC1, sequence information has been registered with a plurality of accession numbers as mentioned above, and a plurality of transcript variants are present. The nucleotide sequence of SEQ ID NO: 5 represents the nucleotide sequence of one of these transcript variants.

CCNL1 refers to cyclin-L1 encoded by CCNL1 gene. CCNL1 is present in various animals including humans, and its sequence information is also publicly known (e.g., human: NM_020307.2, XM_005247647.2, XM_005247648.1, XM_005247649.1, XM_005247650.1, M_005247651.1, M_006713710.1, M_006713711.1 (XP_005247704.1, XP_005247705.1, XP_005247706.1, XP_005247707.1, XP_005247708.1, XP_006713773.1, NP_064703.1), etc.; the numbers represent accession numbers of the NCBI database, and the nucleotide sequence and the amino acid sequence are indicated outside and inside the parentheses, respectively). As one example, the nucleotide sequence of the human CCNL1 gene registered in the database as NM_020307.2 is shown in SEQ ID NO: 7, and the amino acid sequence of the human CCNL1 protein encoded by this human CCNL1 gene is shown in SEQ ID NO: 8. In the present specification, CCNL1 is not limited to a protein consisting of the amino acid sequence of SEQ ID NO: 8 encoded by the nucleotide sequence of SEQ ID NO: 7. As for CCNL1, sequence information has been registered with a plurality of accession numbers as mentioned above, and a plurality of transcript variants are present. The nucleotide sequence of SEQ ID NO: 7 represents the nucleotide sequence of one of these transcript variants.

MCM8 refers to mini-chromosome maintenance 8 encoded by MCM8 gene. MCM8 is present in various animals including humans, and its sequence information is also publicly known (e.g., human: M_032485.5, NM_182802.2, NM_001281520.1, NM_001281521.1, NM_001281522.1, XM_005260859.1 (XP_005260916.1, NP_115874.3, NP_001268449.1, NP_877954.1, NP_001268450.1, NP_001268451.1), etc.; the numbers represent accession numbers of the NCBI database, and the nucleotide sequence and the amino acid sequence are indicated outside and inside the parentheses, respectively). As one example, the nucleotide sequence of the human MCM8 gene registered in the database as NM_032485.5 is shown in SEQ ID NO: 9, and the amino acid sequence of the human MCM8 protein encoded by this human MCM8 gene is shown in SEQ ID NO: 10. In the present specification, MCM8 is not limited to a protein consisting of the amino acid sequence of SEQ ID NO: 10 encoded by the nucleotide sequence of SEQ ID NO: 9. As for MCM8, sequence information has been registered with a plurality of accession numbers as mentioned above, and a plurality of transcript variants are present. The nucleotide sequence of SEQ ID NO: 9 represents the nucleotide sequence of one of these transcript variants.

CCNB3 refers to cyclin-B3 encoded by CCNB3 gene. CCNB3 is present in various animals including humans, and its sequence information is also publicly known (e.g., human: NM_033670.2, M_033031.2, XM_006724610.1 (NP_391990.1, NP_149020.2, XP_006724673.1), etc.; the numbers represent accession numbers of the NCBI database, and the nucleotide sequence and the amino acid sequence are indicated outside and inside the parentheses, respectively). As one example, the nucleotide sequence of the human CCNB3 gene registered in the database as NM_033670.2 is shown in SEQ ID NO: 11, and the amino acid sequence of the human CCNB3 protein encoded by this human CCNB3 gene is shown in SEQ ID NO: 12. In the present specification, CCNB3 is not limited to a protein consisting of the amino acid sequence of SEQ ID NO: 12 encoded by the nucleotide sequence of SEQ ID NO: 11. As for CCNB3, sequence information has been registered with a plurality of accession numbers as mentioned above, and a plurality of transcript variants are present. The nucleotide sequence of SEQ ID NO: 11 represents the nucleotide sequence of one of these transcript variants.

MCMDC1 refers to mini-chromosome maintenance deficient domain containing 1 encoded by MCMDC1 gene. MCMDC1 is present in various animals including humans, and its sequence information is also publicly known (e.g., human: NM_017696.2, NM_153255.4 (NP_060166.2, NP_694987.1), etc.; the numbers represent accession numbers of the NCBI database, and the nucleotide sequence and the amino acid sequence are indicated outside and inside the parentheses, respectively). As one example, the nucleotide sequence of the human MCMDC1 gene registered in the database as NM_017696.2 is shown in SEQ ID NO: 13, and the amino acid sequence of the human MCMDC1 protein encoded by this human MCMDC1 gene is shown in SEQ ID NO: 14. In the present specification, MCMDC1 is not limited to a protein consisting of the amino acid sequence of SEQ ID NO: 14 encoded by the nucleotide sequence of SEQ ID NO: 13. As for MCMDC1, sequence information has been registered with a plurality of accession numbers as mentioned above, and a plurality of transcript variants are present. The nucleotide sequence of SEQ ID NO: 13 represents the nucleotide sequence of one of these transcript variants.

ATM is ATM serine/threonine kinase encoded by ATM gene and refers to a protein that belongs to the PI3/PI4 kinase family. ATM is present in various animals including humans, and its sequence information is also publicly known (e.g., human: NM_000051.3, XM_005271561.2, XM_005271562.2, XM_005271564.2, XM_006718843.1, M_006718844.1, XM_006718845.1 (NP_000042.3, XP_005271618.2, XP_005271619.2, XP_005271621.2, XP_006718906.1, XP_006718907.1, XP_006718908.1), etc.; the numbers represent accession numbers of the NCBI database, and the nucleotide sequence and the amino acid sequence are indicated outside and inside the parentheses, respectively). As one example, the nucleotide sequence of the human ATM gene registered in the database as NM_000051.3 is shown in SEQ ID NO: 15, and the amino acid sequence of the human ATM protein encoded by this human ATM gene is shown in SEQ ID NO: 16. In the present specification, ATM is not limited to a protein consisting of the amino acid sequence of SEQ ID NO: 16 encoded by the nucleotide sequence of SEQ ID NO: 15. As for ATM, sequence information has been registered with a plurality of accession numbers as mentioned above, and a plurality of transcript variants are present. The nucleotide sequence of SEQ ID NO: 15 represents the nucleotide sequence of one of these transcript variants.

CDC25A is phosphatase that is encoded by CDC25A gene and belongs to the CDC25 family, and refers to a protein that activates CDC2 by dephosphorylation. CDC25A is present in various animals including humans, and its sequence information is also publicly known (e.g., human: NM_001789.2, NM_201567.1, XM_006713434.1, XM_006713435.1, XM_006713436.1 (NP_001780.2, NP_963861.1, XP_006713497.1, XP_006713498.1, XP_006713499.1), etc.; the numbers represent accession numbers of the NCBI database, and the nucleotide sequence and the amino acid sequence are indicated outside and inside the parentheses, respectively). As one example, the nucleotide sequence of the human CDC25A gene registered in the database as NM_001789.2 is shown in SEQ ID NO: 17, and the amino acid sequence of the human CDC25A protein encoded by this human CDC25A gene is shown in SEQ ID NO: 18. In the present specification, CDC25A is not limited to a protein consisting of the amino acid sequence of SEQ ID NO: 18 encoded by the nucleotide sequence of SEQ ID NO: 17. As for CDC25A, sequence information has been registered with a plurality of accession numbers as mentioned above, and a plurality of transcript variants are present. The nucleotide sequence of SEQ ID NO: 17 represents the nucleotide sequence of one of these transcript variants.

PRKDC is a catalytic subunit protein of DNA-dependent protein kinase encoded by PRKDC gene and refers to a protein that belongs to the PI3/PI4 kinase family. PRKDC is present in various animals including humans, and its sequence information is also publicly known (e.g., human: NM_006904.6, NM_001081640.1 (NP_008835.5, NP_001075109.1), etc.; the numbers represent accession numbers of the NCBI database, and the basic acid sequence and the amino acid sequence are indicated outside and inside the parentheses, respectively). As one example, the nucleotide sequence of the human PRKDC gene registered in the database as NM_006904.6 is shown in SEQ ID NO: 19, and the amino acid sequence of the human PRKDC protein encoded by this human PRKDC gene is shown in SEQ ID NO: 20. In the present specification, PRKDC is not limited to a protein consisting of the amino acid sequence of SEQ ID NO: 20 encoded by the nucleotide sequence of SEQ ID NO: 19. As for PRKDC, sequence information has been registered with a plurality of accession numbers as mentioned above, and a plurality of transcript variants are present. The nucleotide sequence of SEQ ID NO: 19 represents the nucleotide sequence of one of these transcript variants.

RBBP8 is retinoblastoma binding protein 8 encoded by RBBP8 gene and refers to a nuclear protein that binds directly to retinoblastoma protein. RBBP8 is present in various animals including humans, and its sequence information is also publicly known (e.g., human: NM_002894.2, NM_203291.1, NM_203292.1, XM_005258325.1, XM_005258326.1, XM_006722519.1, XM_006722520.1, XM_006722521.1, XM_006722522.1 (NP_002885.1, NP_976036.1, NP_976037.1, XP_005258382.1, XP_005258383.1, XP_006722582.1, XP_006722583.1, XP_006722584.1, XP_006722585.1), etc.; the numbers represent accession numbers of the NCBI database, and the nucleotide sequence and the amino acid sequence are indicated outside and inside the parentheses, respectively). As one example, the nucleotide sequence of the human RBBP8 gene registered in the database as NM_002894.2 is shown in SEQ ID NO: 21, and the amino acid sequence of the human RBBP8 protein encoded by this human RBBP8 gene is shown in SEQ ID NO: 22. In the present specification, RBBP8 is not limited to a protein consisting of the amino acid sequence of SEQ ID NO: 22 encoded by the nucleotide sequence of SEQ ID NO: 21. As for RBBP8, sequence information has been registered with a plurality of accession numbers as mentioned above, and a plurality of transcript variants are present. The nucleotide sequence of SEQ ID NO: 21 represents the nucleotide sequence of one of these transcript variants.

SKP2 is S-phase kinase-associated protein 2 encoded by SKP2 gene and refers to a protein that belongs to the Fbox protein, which is one of four subunits of E3 ubiquitin protein ligase. SKP2 is present in various animals including humans, and its sequence information is also publicly known (e.g., human: NM_005983.3, NM_032637.3, NM_001243120.1, XM_006714487.1 (NP_005974.2, NP_116026.1, NP_001230049.1, XP_006714550.1), etc.; the numbers represent accession numbers of the NCBI database, and the nucleotide sequence and the amino acid sequence are indicated outside and inside the parentheses, respectively). As one example, the nucleotide sequence of the coding region of the human SKP2 gene registered in the database as NM_005983.3 is shown in SEQ ID NO: 23, and the amino acid sequence of the human SKP2 protein encoded by this human SKP2 gene is shown in SEQ ID NO: 24. In the present specification, SKP2 is not limited to a protein consisting of the amino acid sequence of SEQ ID NO: 24 encoded by the nucleotide sequence of SEQ ID NO: 23. As for SKP2, sequence information has been registered with a plurality of accession numbers as mentioned above, and a plurality of transcript variants are present. The nucleotide sequence of SEQ ID NO: 23 represents the nucleotide sequence of one of these transcript variants.

MCM10 refers to mini-chromosome maintenance 10 encoded by MCM10 gene. MCM10 is present in various animals including humans, and its sequence information is also publicly known (e.g., human: NM_182751.2, NM_018518.4 (NP_877428.1, NP_060988.3), etc.; the numbers represent accession numbers of the NCBI database, and the nucleotide sequence and the amino acid sequence are indicated outside and inside the parentheses, respectively). As one example, the nucleotide sequence of the human MCM10 gene registered in the database as NM_182751.2 is shown in SEQ ID NO: 25, and the amino acid sequence of the human MCM10 protein encoded by this human MCM10 gene is shown in SEQ ID NO: 26. In the present specification, MCM10 is not limited to a protein consisting of the amino acid sequence of SEQ ID NO: 26 encoded by the nucleotide sequence of SEQ ID NO: 25. As for MCM10, sequence information has been registered with a plurality of accession numbers as mentioned above, and a plurality of transcript variants are present. The nucleotide sequence of SEQ ID NO: 25 represents the nucleotide sequence of one of these transcript variants.

CENPH is centromere protein H encoded by CENPH gene and refers to one of proteins constituting activated kinetochore located on the centromere. CENPH is present in various animals including humans, and its sequence information is also publicly known (e.g., human: NM_022909.3 (NP_075060.1), etc.; the numbers represent accession numbers of the NCBI database, and the nucleotide sequence and the amino acid sequence are indicated outside and inside the parentheses, respectively). As one example, the nucleotide sequence of the human CENPH gene registered in the database as NM_022909.3 is shown in SEQ ID NO: 27, and the amino acid sequence of the human CENPH protein encoded by this human CENPH gene is shown in SEQ ID NO: 28. In the present specification, CENPH is not limited to a protein consisting of the amino acid sequence of SEQ ID NO: 28 encoded by the nucleotide sequence of SEQ ID NO: 27. As for CENPH, there is the possibility that a plurality of transcript variants are present. The nucleotide sequence of SEQ ID NO: 27 represents the nucleotide sequence of a transcript variant.

BRSK1 is serine/threonine kinase encoded by BRSK1 gene and refers to kinase that acts at cell cycle checkpoint in DNA damage. BRSK1 is present in various animals including humans, and its sequence information is also publicly known (e.g., human: NM_032430.1, XM_005259327.1, XR_430213.1 (NP_115806.1, XP_005259384.1), etc.; the numbers represent accession numbers of the NCBI database, and the nucleotide sequence and the amino acid sequence are indicated outside and inside the parentheses, respectively). As one example, the nucleotide sequence of the human BRSK1 gene registered in the database as NM_032430.1 is shown in SEQ ID NO: 29, and the amino acid sequence of the human BRSK1 protein encoded by this human BRSK1 gene is shown in SEQ ID NO: 30. In the present specification, BRSK1 is not limited to a protein consisting of the amino acid sequence of SEQ ID NO: 30 encoded by the nucleotide sequence of SEQ ID NO: 29. As for BRSK1, sequence information has been registered with a plurality of accession numbers as mentioned above, and a plurality of transcript variants are present. The nucleotide sequence of SEQ ID NO: 29 represents the nucleotide sequence of one of these transcript variants.

MYLK, myosin light chain kinase, is a calcium/calmodulin-dependent enzyme that phosphorylates myosin regulatory light chains so that myosin interaction with actin filaments is facilitated to produce contractile activity. The gene encoding MYLK encodes both smooth muscle and non-muscle isoforms. MYLK is present in various animals including humans, and its sequence information is also publicly known (e.g., human: NM_053028.3, NM_053026.3, NM_053027.3, NM_053025.3, NM_053031.2, NM_053032.2, XM_011512862.1, XM_011512861.1, XM_011512860.1 (NP_444256.3, NP_444254.3, NP_444255.3, NP_444253.3, NP_444259.1, NP_444260.1, XP_011511164.1, XP_011511163.1, XP_011511162.1), etc.; the numbers represent accession numbers of the NCBI database, and the nucleotide sequence and the amino acid sequence are indicated outside and inside the parentheses, respectively). As one example, the nucleotide sequence of the human MYLK gene registered in the database as NM_053028.3 is shown in SEQ ID NO: 41, and the amino acid sequence of the human MYLK protein encoded by this human MYLK gene is shown in SEQ ID NO: 42. In the present specification, MYLK is not limited to a protein consisting of the amino acid sequence of SEQ ID NO: 42 encoded by the nucleotide sequence of SEQ ID NO: 41. As for MYLK, sequence information has been registered with a plurality of accession numbers as mentioned above, and a plurality of transcript variants are present. The nucleotide sequence of SEQ ID NO: 41 represents the nucleotide sequence of one of these transcript variants.

On the other hand, the protein having the function of suppressively participating in apoptosis means a protein having the function of inhibiting apoptosis by inhibiting mechanisms such as karyopyknosis, cell contraction, membrane blebbing, and DNA fragmentation. The function of suppressively participating in apoptosis is meant to include both of the function of inhibiting apoptosis and the function of inhibiting a factor promoting apoptosis. Examples of the factor promoting apoptosis can include many factors such as caspase, Fas, and TNFR.

Specifically, examples of the anti-apoptosis-related protein that exhibits synthetic lethality when inhibited together with GST-π can include at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A. Of these 20 types of anti-apoptosis-related proteins, one type of anti-apoptosis-related protein may be inhibited together with GST-π, or two or more types of anti-apoptosis-related proteins may be inhibited together with GST-π.

AATF was identified on the basis of its interaction with MAP3K12/DLK, a protein kinase known to be involved in the induction of cell apoptosis. AATF contains a leucine zipper, which is a characteristic motif of transcription factors, and has been shown to exhibit strong transactivation activity when fused to Gal4 DNA-binding domain. Overexpression of the gene encoding AATF is known to inhibit MAP3K12-induced apoptosis. AATF is present in various animals including humans, and its sequence information is also publicly known (e.g., human: NM_012138.3, XM_011546799.1, XM_011524611.1, XR_951958.1, XR_934439.1 (NP_036270.1, XP_011545101.1, XP_011522913.1), etc.; the numbers represent accession numbers of the NCBI database, and the nucleotide sequence and the amino acid sequence are indicated outside and inside the parentheses, respectively). As one example, the nucleotide sequence of the human AATF gene registered in the database as NM_012138.3 is shown in SEQ ID NO: 39, and the amino acid sequence of the human AATF protein encoded by this human AATF gene is shown in SEQ ID NO: 40. In the present specification, AATF is not limited to a protein consisting of the amino acid sequence of SEQ ID NO: 40 encoded by the nucleotide sequence of SEQ ID NO: 39. As for AATF, sequence information has been registered with a plurality of accession numbers as mentioned above, and a plurality of transcript variants are present. The nucleotide sequence of SEQ ID NO: 39 represents the nucleotide sequence of one of these transcript variants.

ALOX12, arachidonate 12-lipoxygenase, is known to be involved in atherosclerosis, osteoporosis, and the like. ALOX12 is also known to positively regulate angiogenesis through regulation of the expression of the vascular endothelial growth factor and to play a role in apoptotic process by promoting the survival of vascular smooth muscle cells or the like. ALOX12 is present in various animals including humans, and its sequence information is also publicly known (e.g., human: NM_000697.2, M_011523780.1 (NP_000688.2, XP_011522082.1, AAH69557.1), etc.; the numbers represent accession numbers of the NCBI database, and the nucleotide sequence and the amino acid sequence are indicated outside and inside the parentheses, respectively). As one example, the nucleotide sequence of the human ALOX12 gene registered in the database as NM_000697.2 is shown in SEQ ID NO: 43, and the amino acid sequence of the human ALOX12 protein encoded by this human ALOX12 gene is shown in SEQ ID NO: 44. In the present specification, ALOX12 is not limited to a protein consisting of the amino acid sequence of SEQ ID NO: 44 encoded by the nucleotide sequence of SEQ ID NO: 43. As for ALOX12, sequence information has been registered with a plurality of accession numbers as mentioned above, and a plurality of transcript variants are present. The nucleotide sequence of SEQ ID NO: 43 represents the nucleotide sequence of one of these transcript variants.

ANXA1 is a membrane-localized protein that binds to phospholipids. ANXA1 inhibits phospholipase A2 and has anti-inflammatory activity. ANXA1 is present in various animals including humans, and its sequence information is also publicly known (e.g., human: NM_000700.2, XM_011518609.1, XM_011518608.1 (NP_000691.1, AAH34157.1), etc.; the numbers represent accession numbers of the NCBI database, and the nucleotide sequence and the amino acid sequence are indicated outside and inside the parentheses, respectively). As one example, the nucleotide sequence of the human ANXA1 gene registered in the database as NM_000700.2 is shown in SEQ ID NO: 45, and the amino acid sequence of the human ANXA1 protein encoded by this human ANXA1 gene is shown in SEQ ID NO: 46. In the present specification, ANXA1 is not limited to a protein consisting of the amino acid sequence of SEQ ID NO: 46 encoded by the nucleotide sequence of SEQ ID NO: 45. As for ANXA1, sequence information has been registered with a plurality of accession numbers as mentioned above, and a plurality of transcript variants are present. The nucleotide sequence of SEQ ID NO: 45 represents the nucleotide sequence of one of these transcript variants.

ANXA4 belongs to the annexin family of calcium-dependent phospholipid-binding proteins. This protein has possible interactions with ATP and is known to have *in vitro* anticoagulant activity and to inhibit phospholipase A2. ANXA4 is present in various animals including humans, and its sequence information is also publicly known (e.g., human: NM_001153.3, XM_011532805.1 (NP_001144.1, XP_011531107.1, AAH63672.1, AAH00182.1, AAH11659.1), etc.; the numbers represent accession numbers of the NCBI database, and the nucleotide sequence and the amino acid sequence are indicated outside and inside the parentheses, respectively). As one example, the nucleotide sequence of the human ANXA4 gene registered in the database as NM_001153.3 is shown in SEQ ID NO: 47, and the amino acid sequence of the human ANXA4 protein encoded by this human ANXA4 gene is shown in SEQ ID NO: 48. In the present specification, ANXA4 is not limited to a protein consisting of the amino acid sequence of SEQ ID NO: 48 encoded by the nucleotide sequence of SEQ ID NO: 47. As for ANXA4, sequence information has been registered with a plurality of accession numbers as mentioned above, and a plurality of transcript variants are present. The nucleotide sequence of SEQ ID NO: 47 represents the nucleotide sequence of one of these transcript variants.

API5 is an apoptosis inhibitory protein whose expression is known to prevent apoptosis after growth factor deprivation. API5 suppresses the transcription factor E2F1-induced apoptosis and also interacts with and negatively regulates Acinus, a nuclear factor involved in apoptotic DNA fragmentation. API5 is present in various animals including humans, and its sequence information is also publicly known (e.g., human: NM_001142930.1, NM_006595.3, NM_001243747.1, NM_001142931.1, XM_006718359.2, NR_024625.1 (NP_001136402.1, NP_001136403.1, NP_001230676.1, NP_006586.1, XP_006718422.1), etc.; the numbers represent accession numbers of the NCBI database, and the nucleotide sequence and the amino acid sequence are indicated outside and inside the parentheses, respectively). As one example, the nucleotide sequence of the human API5 gene registered in the database as NM_001142930.1 is shown in SEQ ID NO: 49, and the amino acid sequence of the human API5 protein encoded by this human API5 gene is shown in SEQ ID NO: 50. In the present specification, API5 is not limited to a protein consisting of the amino acid sequence of SEQ ID NO: 50 encoded by the nucleotide sequence of SEQ ID NO: 49. As for API5, sequence information has been registered with a plurality of accession numbers as mentioned above, and a plurality of transcript variants are present. The nucleotide sequence of SEQ ID NO: 49 represents the nucleotide sequence of one of these transcript variants.

ATF5 is known to be involved in diseases caused by human T-cell leukemia virus type 1. ATF5 is a transcriptional activator that binds to the cAMP response element (CRE) present in many viral promoters, etc., and is known to inhibit the differentiation of neuroprogenitor cells into neurons. ATF5 is present in various animals including humans, and its sequence information is also publicly known (e.g., human: NM_012068.5, NM_001193646.1, NM_001290746.1, XM_011526629.1 (NP_036200.2, NP_001277675.1, NP_001180575.1, XP_011524931.1), etc.; the numbers represent accession numbers of the NCBI database, and the nucleotide sequence and the amino acid sequence are indicated outside and inside the parentheses, respectively). As one example, the nucleotide sequence of the human ATF5 gene registered in the database as NM_012068.5 is shown in SEQ ID NO: 51, and the amino acid sequence of the human ATF5 protein encoded by this human ATF5 gene is shown in SEQ ID NO: 52. In the present specification, ATF5 is not limited to a protein consisting of the amino acid sequence of SEQ ID NO: 52 encoded by the nucleotide sequence of SEQ ID NO: 51. As for ATF5, sequence information has been registered with a plurality of accession numbers as mentioned above, and a plurality of transcript variants are present. The nucleotide sequence of SEQ ID NO: 51 represents the nucleotide sequence of one of these transcript variants.

AVEN is a protein known as an apoptosis, caspase activation inhibitor and is known to be involved in schizoid personality disorder and alexithymia. AVEN is also known to inhibit apoptosis mediated by Apaf-1. AVEN is present in various animals including humans, and its sequence information is also publicly known (e.g., human: NM_020371.2, XM_011521820.1, XM_005254563.2, M_011521819.1, XM_011521818.1 (NP NP_065104.1, XP_011520122.1, XP_011520121.1, XP_011520120.1, XP_005254620.1, AAH63533.1, AAF91470.1), etc.; the numbers represent accession numbers of the NCBI database, and the nucleotide sequence and the amino acid sequence are indicated outside and inside the parentheses, respectively). As one example, the nucleotide sequence of the human AVEN gene registered in the database as NM_020371.2 is shown in SEQ ID NO: 53, and the amino acid sequence of the human AVEN protein encoded by this human AVEN gene is shown in SEQ ID NO: 54. In the present specification, AVEN is not limited to a protein consisting of the amino acid sequence of SEQ ID NO: 54 encoded by the nucleotide sequence of SEQ ID NO: 53. As for AVEN, sequence information has been registered with a plurality of accession numbers as mentioned above, and a plurality of transcript variants are present. The nucleotide sequence of SEQ ID NO: 53 represents the nucleotide sequence of one of these transcript variants.

AZU1 is a protein contained in azurophil granules and has monocyte chemotactic and antimicrobial activity. AZU1 is an important multifunctional inflammatory mediator. AZU1 is present in various animals including humans, and its sequence information is also publicly known (e.g., human: NM_001700.3 (NP_001691.1, EAW69592.1, AAH93933.1, AAH93931.1, AAH69495.1), etc.; the numbers represent accession numbers of the NCBI database, and the nucleotide sequence and the amino acid sequence are indicated outside and inside the parentheses, respectively). As one example, the nucleotide sequence of the human AZU1 gene registered in the database as NM_001700.3 is shown in SEQ ID NO: 55, and the amino acid sequence of the human AZU1 protein encoded by this human AZU1 gene is shown in SEQ ID NO: 56. In the present specification, AZU1 is not limited to a protein consisting of the amino acid sequence of SEQ ID NO: 56 encoded by the nucleotide sequence of SEQ ID NO: 55. As for AZU1, sequence information has been registered with a plurality of accession numbers as mentioned above, and a plurality of transcript variants are present. The nucleotide sequence of SEQ ID NO: 55 represents the nucleotide sequence of one of these transcript variants.

BAG1 binds to BCL2, a membrane protein that inhibits a pathway leading to apoptosis or programmed cell death. BAG1 enhances the anti-apoptotic effects of BCL2 and represents a link between growth factor receptors and anti-apoptotic mechanisms. BAG1 is present in various animals including humans, and its sequence information is also publicly known (e.g., human: NM_004323.5, NM_001172415.1 (NP_004314.5, NP_001165886.1, AAH14774.2), etc.; the numbers represent accession numbers of the NCBI database, and the nucleotide sequence and the amino acid sequence are indicated outside and inside the parentheses, respectively). As one example, the nucleotide sequence of the human BAG1 gene registered in the database as NM_004323.5 is shown in SEQ ID NO: 57, and the amino acid sequence of the human BAG1 protein encoded by this human BAG1 gene is shown in SEQ ID NO: 58. In the present specification, BAG1 is not limited to a protein consisting of the amino acid sequence of SEQ ID NO: 58 encoded by the nucleotide sequence of SEQ ID NO: 57. As for BAG1, sequence information has been registered with a plurality of accession numbers as mentioned above, and a plurality of transcript variants are present. The nucleotide sequence of SEQ ID NO: 57 represents the nucleotide sequence of one of these transcript variants.

BCL2L1 belongs to the BCL-2 protein family. Members of this protein family form hetero- or homodimers and act as anti- or pro-apoptotic regulators that are involved in a wide variety of cellular activities. BCL2L1 is located at the outer mitochondrial membrane and has been shown to regulate outer mitochondrial membrane channel opening. BCL2L1 is present in various animals including humans, and its sequence information is also publicly known (e.g., human: NM_38578.1, NM_001191.2, XM_011528966.1, XM_011528965.1, XM_011528961.1, XM_011528960.1, XM_011528964.1, XM_011528963.1, XM_011528962.1, XM_005260487.3, XM_005260486.2 (NP_612815.1, NP_001182.1, AAH19307.1, XP_011527268.1, XP_011527267.1, XP_011527266.1, XP_011527265.1, XP_011527264.1, XP_011527263.1, XP_011527262.1, XP_005260544.1, XP_005260543.1), etc.; the numbers represent accession numbers of the NCBI database, and the nucleotide sequence and the amino acid sequence are indicated outside and inside the parentheses, respectively). As one example, the nucleotide sequence of the human BCL2L1 gene registered in the database as NM_138578.1 is shown in SEQ ID NO: 59, and the amino acid sequence of the human BCL2L1 protein encoded by this human BCL2L1 gene is shown in SEQ ID NO: 60. In the present specification, BCL2L1 is not limited to a protein consisting of the amino acid sequence of SEQ ID NO: 60 encoded by the nucleotide sequence of SEQ ID NO: 59. As for BCL2L1, sequence information has been registered with a plurality of accession numbers as mentioned above, and a plurality of transcript variants are present. The nucleotide sequence of SEQ ID NO: 59 represents the nucleotide sequence of one of these transcript variants.

BFAR, a bifunctional apoptosis regulator, has anti-apoptotic activity, both against apoptosis triggered via cell death-receptors and against apoptosis triggered via mitochondrial factors. BFAR is present in various animals including humans, and its sequence information is also publicly known (e.g., human: NM_016561.2, XM_006725196.2, XM_011546704.1, XM_005255350.2, XM_011522520.1 (NP_057645.1, XP_011545006.1, XP_011520822.1, XP_006725259.1, XP_005255407.1, AAH03054.1), etc.; the numbers represent accession numbers of the NCBI database, and the nucleotide sequence and the amino acid sequence are indicated outside and inside the parentheses, respectively). As one example, the nucleotide sequence of the human BFAR gene registered in the database as NM_16561.2 is shown in SEQ ID NO: 61, and the amino acid sequence of the human BFAR protein encoded by this human BFAR gene is shown in SEQ ID NO: 62. In the present specification, BFAR is not limited to a protein consisting of the amino acid sequence of SEQ ID NO: 62 encoded by the nucleotide sequence of SEQ ID NO: 61. As for BFAR, sequence information has been registered with a plurality of accession numbers as mentioned above, and a plurality of transcript variants are present. The nucleotide sequence of SEQ ID NO: 61 represents the nucleotide sequence of one of these transcript variants.

CFLAR, a regulator of apoptosis, is known to be structurally similar to caspase-8. However, CFLAR lacks caspase activity and is cleaved into two peptides by caspase-8. CFLAR is present in various animals including humans, and its sequence information is also publicly known (e.g., human: NM_003879.5, NM_001202519.1, NM_001202518.1, NM_001308043.1, NM_001308042.1, NM_001202517.1, NM_001202516.1, NM_001127184.2, NM_001202515.1, NM_001127183.2, XM_011512100.1 (NP_003870.4, NP_001294972.1, NP_001294971.1, NP_001189448.1, NP_001189446.1, NP_001189445.1, NP_001189444.1, NP_001120656.1, XP_011510402.1), etc.; the numbers represent accession numbers of the NCBI database, and the nucleotide sequence and the amino acid sequence are indicated outside and inside the parentheses, respectively). As one example, the nucleotide sequence of the human CFLAR gene registered in the database as NM_003879.5 is shown in SEQ ID NO: 63, and the amino acid sequence of the human CFLAR protein encoded by this human CFLAR gene is shown in SEQ ID NO: 64. In the present specification, CFLAR is not limited to a protein consisting of the amino acid sequence of SEQ ID NO: 64 encoded by the nucleotide sequence of SEQ ID NO: 63. As for CFLAR, sequence information has been registered with a plurality of accession numbers as mentioned above, and a plurality of transcript variants are present. The nucleotide sequence of SEQ ID NO: 63 represents the nucleotide sequence of one of these transcript variants.

IL2, interleukin 2, is a secreted cytokine that is important for the proliferation of T and B lymphocytes. IL2 is present in various animals including humans, and its sequence information is also publicly known (e.g., human: NM_000586.3 (NP_000577.2), etc.; the numbers represent accession numbers of the NCBI database, and the nucleotide sequence and the amino acid sequence are indicated outside and inside the parentheses, respectively). As one example, the nucleotide sequence of the human IL2 gene registered in the database as NM_000586.3 is shown in SEQ ID NO: 65, and the amino acid sequence of the human IL2 protein encoded by this human IL2 gene is shown in SEQ ID NO: 66. In the present specification, IL2 is not limited to a protein consisting of the amino acid sequence of SEQ ID NO: 66 encoded by the nucleotide sequence of SEQ ID NO: 65. As for IL2, there is the possibility that a plurality of transcript variants are present. The nucleotide sequence of SEQ ID NO: 65 represents the nucleotide sequence of a transcript variant.

MALT1 is encoded by a gene that is recurrently rearranged in chromosomal translocation with baculoviral IAP repeat-containing protein 3 (also known as apoptosis inhibitor 2) and immunoglobulin heavy chain locus in mucosa-associated lymphoid tissue lymphomas. MALT1 may activate NFκB. MALT1 is present in various animals including humans, and its sequence information is also publicly known (e.g., human: NM_173844.2, NM_006785.3, XM_011525794.1 (NP_776216.1, NP_006776.1, XP_011524096.1), etc.; the numbers represent accession numbers of the NCBI database, and the nucleotide sequence and the amino acid sequence are indicated outside and inside the parentheses, respectively). As one example, the nucleotide sequence of the human MALT1 gene registered in the database as NM_006785.3 is shown in SEQ ID NO: 67, and the amino acid sequence of the human MALT1 protein encoded by this human MALT1 gene is shown in SEQ ID NO: 68. In the present specification, MALT1 is not limited to a protein consisting of the amino acid sequence of SEQ ID NO: 68 encoded by the nucleotide sequence of SEQ ID NO: 67. As for MALT1, sequence information has been registered with a plurality of accession numbers as mentioned above, and a plurality of transcript variants are present. The nucleotide sequence of SEQ ID NO: 67 represents the nucleotide sequence of one of these transcript variants.

MCL1 is an anti-apoptotic protein, which is a member of the Bcl-2 family. The longest variant resulting from the alternative splicing of the MCL1 gene enhances cell survival by inhibiting apoptosis, while the alternatively spliced shorter variants promote apoptosis and induce cell death. MCL1 is present in various animals including humans, and its sequence information is also publicly known (e.g., human: NM_021960.4, NM_001197320.1, NM_182763.2 (NP_068779.1, NP_001184249.1, NP_877495.1), etc.; the numbers represent accession numbers of the NCBI database, and the nucleotide sequence and the amino acid sequence are indicated outside and inside the parentheses, respectively). As one example, the nucleotide sequence of the human MCL1 gene registered in the database as NM_021960.4 is shown in SEQ ID NO: 69, and the amino acid sequence of the human MCL1 protein encoded by this human MCL1 gene is shown in SEQ ID NO: 70. In the present specification, MCL1 is not limited to a protein consisting of the amino acid sequence of SEQ ID NO: 70 encoded by the nucleotide sequence of SEQ ID NO: 69. As for MCL1, sequence information has been registered with a plurality of accession numbers as mentioned above, and a plurality of transcript variants are present. The nucleotide sequence of SEQ ID NO: 69 represents the nucleotide sequence of one of these transcript variants.

MKL1 is known to interact with the transcription factor myocardin, a key regulator of smooth muscle cell differentiation. MKL1 is predominantly nuclear and helps transduce signals from the cytoskeleton to the nucleus. The MKL1 gene is involved in a translocation event that creates a fusion of this gene and the RNA-binding motif protein-15 gene. MKL1 is present in various animals including humans, and its sequence information is also publicly known (e.g., human: NM_001282662.1, NM_001282660.1, M_020831.4, NM_001282661.1, XM_011530287.1, XM-011530286.1, XM-011530285.1, XM 011530284.1, XM_011530283.1, XM_005261691.3 (NP_001269591.1, NP_001269589.1, NP_065882.1, NP_001269590.1, XP_011528589.1, XP_011528588.1, XP_011528587.1, XP_011528586.1, XP_011528585.1, XP_005261751.1, XP_005261749.1, XP_005261748.1), etc.; the numbers represent accession numbers of the NCBI database, and the nucleotide sequence and the amino acid sequence are indicated outside and inside the parentheses, respectively). As one example, the nucleotide sequence of the human MKL1 gene registered in the database as NM_001282662.1 is shown in SEQ ID NO: 71, and the amino acid sequence of the human MKL1 protein encoded by this human MKL1 gene is shown in SEQ ID NO: 72. In the present specification, MKL1 is not limited to a protein consisting of the amino acid sequence of SEQ ID NO: 72 encoded by the nucleotide sequence of SEQ ID NO: 71. As for MKL1, sequence information has been registered with a plurality of accession numbers as mentioned above, and a plurality of transcript variants are present. The nucleotide sequence of SEQ ID NO: 71 represents the nucleotide sequence of one of these transcript variants.

MPO, myeloperoxidase, is a heme protein that is synthesized during myeloid differentiation and constitutes the major component of neutrophil azurophil granules. MPO produces hypohalous acids central to the microbicidal activity of neutrophils. MPO is present in various animals including humans, and its sequence information is also publicly known (e.g., human: NM_000250.1, XM_011524823.1, XM_011524822**.**1, XM_011524821.1 (NP_000241.1), etc.; the numbers represent accession numbers of the NCBI database, and the nucleotide sequence and the amino acid sequence are indicated outside and inside the parentheses, respectively). As one example, the nucleotide sequence of the human MPO gene registered in the database as NM_000250.1 is shown in SEQ ID NO: 73, and the amino acid sequence of the human MPO protein encoded by this human MPO gene is shown in SEQ ID NO: 74. In the present specification, MPO is not limited to a protein consisting of the amino acid sequence of SEQ ID NO: 74 encoded by the nucleotide sequence of SEQ ID NO: 73. As for MPO, sequence information has been registered with a plurality of accession numbers as mentioned above, and a plurality of transcript variants are present. The nucleotide sequence of SEQ ID NO: 73 represents the nucleotide sequence of one of these transcript variants.

MTL5, a metallothionein-like protein, has been shown to be expressed specifically in the mouse testis and ovary. Metallothionein may play a central role in the regulation of cell growth and differentiation and be involved in spermatogenesis. MTL5 is present in various animals including humans, and its sequence information is also publicly known (e.g., human: NM_004923.3, NM_001039656.1, XM_011545404.1, XM_011545403.1, XM_011545402.1 (NP_001034745.1, NP_004914.2, XP_011543706.1, XP_011543705.1, XP_011543704.1), etc.; the numbers represent accession numbers of the NCBI database, and the nucleotide sequence and the amino acid sequence are indicated outside and inside the parentheses, respectively). As one example, the nucleotide sequence of the human MTL5 gene registered in the database as NM_004923.3 is shown in SEQ ID NO: 75, and the amino acid sequence of the human MTL5 protein encoded by this human MTL5 gene is shown in SEQ ID NO: 76. In the present specifcation, MTL5 is not limited to a protein consisting of the amino acid sequence of SEQ ID NO: 76 encoded by the nucleotide sequence of SEQ ID NO: 75. As for MTL5, sequence information has been registered with a plurality of accession numbers as mentioned above, and a plurality of transcript variants are present. The nucleotide sequence of SEQ ID NO: 75 represents the nucleotide sequence of one of these transcript variants.

MYBL2 is a nuclear protein that belongs to the MYB family of transcription factors and is involved in cell cycle progression. MYBL2 is phosphorylated by cyclin A/cyclin-dependent kinase 2 during the S-phase of the cell cycle and possesses both activator and repressor activities. MYBL2 is present in various animals including humans, and its sequence information is also publicly known (e.g., human: NM_001278610.1, NM_002466.3 (NP_001265539.1, NP_002457.1), etc.; the numbers represent accession numbers of the NCBI database, and the nucleotide sequence and the amino acid sequence are indicated outside and inside the parentheses, respectively). As one example, the nucleotide sequence of the human MYBL2 gene registered in the database as NM_002466.3 is shown in SEQ ID NO: 77, and the amino acid sequence of the human MYBL2 protein encoded by this human MYBL2 gene is shown in SEQ ID NO: 78. In the present specification, MYBL2 is not limited to a protein consisting of the amino acid sequence of SEQ ID NO: 78 encoded by the nucleotide sequence of SEQ ID NO: 77. As for MYBL2, sequence information has been registered with a plurality of accession numbers as mentioned above, and a plurality of transcript variants are present. The nucleotide sequence of SEQ ID NO: 77 represents the nucleotide sequence of one of these transcript variants.

MYO18A, myosin 18A, is known to be involved in 8p11 myeloproliferative syndrome. MYO18A has motor activity and ATPase activity. MYO18A is present in various animals including humans, and its sequence information is also publicly known (e.g., human: NM_203318.1, NM_078471.3 (NP_976063.1, NP_510880.2), etc.; the numbers represent accession numbers of the NCBI database, and the nucleotide sequence and the amino acid sequence are indicated outside and inside the parentheses, respectively). As one example, the nucleotide sequence of the human MYO18A gene registered in the database as NM_078471.3 is shown in SEQ ID NO: 79, and the amino acid sequence of the human MYO18A protein encoded by this human MYO18A gene is shown in SEQ ID NO: 80. In the present specification, MYO18A is not limited to a protein consisting of the amino acid sequence of SEQ ID NO: 80 encoded by the nucleotide sequence of SEQ ID NO: 79. As for MYO18A, sequence information has been registered with a plurality of accession numbers as mentioned above, and a plurality of transcript variants are present. The nucleotide sequence of SEQ ID NO: 79 represents the nucleotide sequence of one of these transcript variants.

On the other hand, the PI3K signaling pathway-related protein is a protein, except for Akt1, included in proteins involved in the PI3K/AKT signaling pathway. In other words, the PI3K signaling pathway-related protein is a PI3K/AKT signaling pathway-related protein except for Akt1. The PI3K/AKT signaling pathway is disclosed in, for example, Cell 2007 Jun 29; 129 (7): 1261-74.

Specifically, examples of the PI3K signaling pathway-related protein that exhibits synthetic lethality when inhibited together with GST-π can include at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF. Of these 14 types of PI3K signaling pathway-related proteins, one type of PI3K signaling pathway-related protein may be inhibited together with GST-π, or two or more types of PI3K signaling pathway-related proteins may be inhibited together with GST-π.

Particularly, it is preferable for the PI3K signaling pathway-related protein that at least one PI3K signaling pathway-related protein selected from the group consisting of MTOR, IRAK1, IRS1, MYD88, NFKB1, PIK3CG, and RAC1 should be inhibited together with GST-π. These 7 types of PI3K signaling pathway-related proteins exhibit a remarkably high cell growth-inhibiting effect when each inhibited together with GST-π compared with when inhibited each alone or when GST-π is inhibited alone. Thus, it is preferable that the PI3K signaling pathway-related protein that exhibits synthetic lethality when inhibited together with GST-π should be selected from these 7 types of PI3K signaling pathway-related proteins.

MTOR, mechanistic target of rapamycin, is a target protein of rapamycin and is known as a serine-threonine kinase. MTOR belongs to the phosphatidylinositol kinase-related kinase family and is known to mediate the response of cells to stresses such as DNA damage and nutrient deprivation. MTOR is also known to act as a target of cell cycle arrest and the immunosuppressive effect of FKBP12-rapamycin complexes. MTOR is present in various animals including humans, and its sequence information is also publicly known (e.g., human: NM_004958.3, XM_005263438.1, XM_011541166.1 (NP_004949.1, XP_005263495.1, XP_005263495.1), etc.; the numbers represent accession numbers of the NCBI database, and the nucleotide sequence and the amino acid sequence are indicated outside and inside the parentheses, respectively). As one example, the nucleotide sequence of the human MTOR gene registered in the database as NM_004958.3 is shown in SEQ ID NO: 81, and the amino acid sequence of the human MTOR protein encoded by this human MTOR gene is shown in SEQ ID NO: 82. In the present specification, MTOR is not limited to a protein consisting of the amino acid sequence of SEQ ID NO: 82 encoded by the nucleotide sequence of SEQ ID NO: 81. As for MTOR, sequence information has been registered with a plurality of accession numbers as mentioned above, and a plurality of transcript variants are present. The nucleotide sequence of SEQ ID NO: 81 represents the nucleotide sequence of one of these transcript variants.

IRAK1, interleukin 1 receptor associated kinase 1, is a serine-threonine kinase that acts on interleukin 1 receptors by stimulation. IRAK1 is partly responsible for IL1-induced upregulation associated with a transcription factor NFκB. IRAK1 is present in various animals including humans, and its sequence information is also publicly known (e.g., human: NM_001025243.1, NM_001025242.1, NM_001569.3, XM_011531158.1, XM_005274668.2 (NP_001020414.1, NP_001020413.1, NP_001560.2, XP_011529460.1, XP_005274725.1), etc.; the numbers represent accession numbers of the NCBI database, and the nucleotide sequence and the amino acid sequence are indicated outside and inside the parentheses, respectively). As one example, the nucleotide sequence of the human IRAK1 gene registered in the database as NM_001025243.1 is shown in SEQ ID NO: 83, and the amino acid sequence of the human IRAK1 protein encoded by this human IRAK1 gene is shown in SEQ ID NO: 84. In the present specification, IRAK1 is not limited to a protein consisting of the amino acid sequence of SEQ ID NO: 84 encoded by the nucleotide sequence of SEQ ID NO: 83. As for IRAK1, sequence information has been registered with a plurality of accession numbers as mentioned above, and a plurality of transcript variants are present. The nucleotide sequence of SEQ ID NO: 83 represents the nucleotide sequence of one of these transcript variants.

IRS1, insulin receptor substrate 1, is known as a protein that is phosphorylated by insulin receptor tyrosine kinase. IRS1 is present in various animals including humans, and its sequence information is also publicly known (e.g., human: NM_005544.2 (NP_005535.1), etc.; the numbers represent accession numbers of the NCBI database, and the nucleotide sequence and the amino acid sequence are indicated outside and inside the parentheses, respectively). As one example, the nucleotide sequence of the human IRS1 gene registered in the database as NM_005544.2 is shown in SEQ ID NO: 85, and the amino acid sequence of the human IRS1 protein encoded by this human IRS1 gene is shown in SEQ ID NO: 86. In the present specification, IRS1 is not limited to a protein consisting of the amino acid sequence of SEQ ID NO: 86 encoded by the nucleotide sequence of SEQ ID NO: 85. As for IRS1, there is the possibility that a plurality of transcript variants are present. The nucleotide sequence of SEQ ID NO: 85 represents the nucleotide sequence of one of these transcript variants.

MYD88, myeloid differentiation primary response 88, is a cytoplasmic adaptor protein that plays a key role in innate and adaptive immune responses. MYD88 functions as an essential signal transducer in the interleukin 1 and Toll-like receptor signaling pathways. These signaling pathways regulate the activation of many inflammatory genes. MYD88 has an N-terminal death domain and a C-terminal Toll-interleukin 1 receptor domain. MYD88 is present in various animals including humans, and its sequence information is also publicly known (e.g., human: NM_001172567.1, NM_002468.4, NM_001172569.1, NM_001172568.1, NM_001172566.1, XM_005265172.1, XM_006713170.1 (NP_001166038.1, NP_002459.2, NP_001166040.1, NP_001166039.1, NP_001166037.1, XP_005265229.1, XP_006713233.1), etc.; the numbers represent accession numbers of the NCBI database, and the nucleotide sequence and the amino acid sequence are indicated outside and inside the parentheses, respectively). As one example, the nucleotide sequence of the human MYD88 gene registered in the database as NM_001172567.1 is shown in SEQ ID NO: 87, and the amino acid sequence of the human MYD88 protein encoded by this human MYD88 gene is shown in SEQ ID NO: 88. In the present specification, MYD88 is not limited to a protein consisting of the amino acid sequence of SEQ ID NO: 88 encoded by the nucleotide sequence of SEQ ID NO: 87. As for MYD88, sequence information has been registered with a plurality of accession numbers as mentioned above, and a plurality of transcript variants are present. The nucleotide sequence of SEQ ID NO: 87 represents the nucleotide sequence of one of these transcript variants.

NFKB1, nuclear factor of kappa light polypeptide gene enhancer in B-cells 1, is a 105 kD protein that is processed into a 50 kD protein during translation by 26S proteasome. The 105 kD protein is a Rel protein-specific transcription inhibitor, and the 50 kD protein is a DNA-binding subunit in a NFκ-B protein (NFKB) complex. NFKB1 is present in various animals including humans, and its sequence information is also publicly known (e.g., human: NM_003998.3, NM_001165412.1, XM_011532006.1, XM_011532007.1, XM_011532008.1, XM_011532009.1 (NP_003989.2, NP_001158884.1, XP_011530308.1, XP_011530309.1, XP_011530310.1, XP_011530311.1), etc.; the numbers represent accession numbers of the NCBI database, and the nucleotide sequence and the amino acid sequence are indicated outside and inside the parentheses, respectively). As one example, the nucleotide sequence of the human NFKB1 gene registered in the database as NM_003998.3 is shown in SEQ ID NO: 89, and the amino acid sequence of the human NFKB1 protein encoded by this human NFKB1 gene is shown in SEQ ID NO: 90. In the present specification, NFKB1 is not limited to a protein consisting of the amino acid sequence of SEQ ID NO: 90 encoded by the nucleotide sequence of SEQ ID NO: 89. As for NFKB1, sequence information has been registered with a plurality of accession numbers as mentioned above, and a plurality of transcript variants are present. The nucleotide sequence of SEQ ID NO: 89 represents the nucleotide sequence of one of these transcript variants.

PIK3CG, phosphatidylinositol-4,5-bisphosphate 3-kinase, catalytic subunit gamma, is known to form PI3K by binding to a p85 regulatory subunit, as with other class 1 catalytic subunits (p110-α, p110-β, and p110-δ). PIK3CG is present in various animals including humans, and its sequence information is also publicly known (e.g., human: NM_001282426.1, NM_001282427.1, NM_002649.3, XM_011516316.1, XM_011516317.1, XM_005250443.2 (NP_001269355.1, NP_001269356.1, NP_002640.2, XP_011514618.1, XP_011514619.1, XP_005250500.1), etc.; the numbers represent accession numbers of the NCBI database, and the nucleotide sequence and the amino acid sequence are indicated outside and inside the parentheses, respectively). As one example, the nucleotide sequence of the human PIK3CG gene registered in the database as NM_001282426.1 is shown in SEQ ID NO: 91, and the amino acid sequence of the human PIK3CG protein encoded by this human PIK3CG gene is shown in SEQ ID NO: 92. In the present specification, PIK3CG is not limited to a protein consisting of the amino acid sequence of SEQ ID NO: 92 encoded by the nucleotide sequence of SEQ ID NO: 91. As for PIK3CG, sequence information has been registered with a plurality of accession numbers as mentioned above, and a plurality of transcript variants are present. The nucleotide sequence of SEQ ID NO: 91 represents the nucleotide sequence of one of these transcript variants.

RAC1, ras-related C3 botulinum toxin substrate 1 (rho family, small GTP binding protein Rac1), is known as GTPase belonging to the RAS superfamily as a small GTP-binding protein. Factors belonging to this superfamily regulate various intracellular events including growth, cytoskeletal reorganization, and protein kinase activation. RAC1 is present in various animals including humans, and its sequence information is also publicly known (e.g., human: NM_018890.3, NM_006908.4 (NP_061485.1, NP_008839.2), etc.; the numbers represent accession numbers of the NCBI database, and the nucleotide sequence and the amino acid sequence are indicated outside and inside the parentheses, respectively). As one example, the nucleotide sequence of the human RAC1 gene registered in the database as NM_018890.3 is shown in SEQ ID NO: 93, and the amino acid sequence of the human RAC1 protein encoded by this human RAC1 gene is shown in SEQ ID NO: 94. In the present specification, RAC1 is not limited to a protein consisting of the amino acid sequence of SEQ ID NO: 94 encoded by the nucleotide sequence of SEQ ID NO: 93. As for RAC1, sequence information has been registered with a plurality of accession numbers as mentioned above, and a plurality of transcript variants are present. The nucleotide sequence of SEQ ID NO: 93 represents the nucleotide sequence of one of these transcript variants.

AKT3, v-akt murine thymoma viral oncogene homolog 3, is also called PKB and known as a member of the serine-threonine protein kinase family AKT. AKT kinases are known as regulatory factors in the cell signaling system that responds to insulin and growth factors. These kinases are involved in various biological processes including glycogen synthesis and glucose uptake as long as cell growth, differentiation, apoptosis, and tumorigenesis. This kinase has been found to be stimulated by platelet-derived growth factor (PDGF), insulin, and insulin-like growth factor 1 (IGF1). AKT3 is present in various animals including humans, and its sequence information is also publicly known (e.g., human: NM_181690.2, NM_001206729.1, NM_005465.4, XM_005272995.2, XM_011544011.1, XM_005272994.3, XM_005272997.3, XM_011544014.1, XM_011544012.1, XM_011544013.1, XM_006711726.2 (NP_859029.1, NP_001193658.1, NP_005456.1, XP_005273052.1, XP_011542313.1, XP_005273051.1, XP_005273054.1, XP_011542316.1, XP_011542314.1, XP_011542315.1, XP_006711789.1), etc.; the numbers represent accession numbers of the NCBI database, and the nucleotide sequence and the amino acid sequence are indicated outside and inside the parentheses, respectively). As one example, the nucleotide sequence of the human AKT3 gene registered in the database as NM_181690.2 is shown in SEQ ID NO: 95, and the amino acid sequence of the human AKT3 protein encoded by this human AKT3 gene is shown in SEQ ID NO: 96. In the present specification, AKT3 is not limited to a protein consisting of the amino acid sequence of SEQ ID NO: 96 encoded by the nucleotide sequence of SEQ ID NO: 95. As for AKT3, sequence information has been registered with a plurality of accession numbers as mentioned above, and a plurality of transcript variants are present. The nucleotide sequence of SEQ ID NO: 95 represents the nucleotide sequence of one of these transcript variants.

EIF4B, eukaryotic translation initiation factor 4B, is known as a protein that is involved in the PI3K-Akt signaling pathway and signals by GPCR. EIF4B is necessary for the binding of mRNA to ribosome and has functions closely related to EIF4-F and EIF4-A. EIF4B is known to bind to or near the 5'-terminal cap of mRNA in the presence of EIF4-F and ATP to stimulate ATPase activity and to stimulate the ATP-dependent RNA unwinding activity of EIF4-A and EIF4-F. EIF4B is present in various animals including humans, and its sequence information is also publicly known (e.g., human: NM_001300821.1, NM_001417.5, M_006719274.1 (NP_001287750.1, NP_001408.2, XP_006719337.1), etc.; the numbers represent accession numbers of the NCBI database, and the nucleotide sequence and the amino acid sequence are indicated outside and inside the parentheses, respectively). As one example, the nucleotide sequence of the human EIF4B gene registered in the database as NM_001300821.1 is shown in SEQ ID NO: 97, and the amino acid sequence of the human EIF4B protein encoded by this human EIF4B gene is shown in SEQ ID NO: 98. In the present specification, EIF4B is not limited to a protein consisting of the amino acid sequence of SEQ ID NO: 98 encoded by the nucleotide sequence of SEQ ID NO: 97. As for EIF4B, sequence information has been registered with a plurality of accession numbers as mentioned above, and a plurality of transcript variants are present. The nucleotide sequence of SEQ ID NO: 97 represents the nucleotide sequence of one of these transcript variants.

EIF4E, eukaryotic translation initiation factor 4E, is known as a protein that constitutes a eukaryotic translation initiation factor 4F complex, which recognizes the 5'-terminal 7-methylguanosine cap structure of mRNA. EIF4E aids in translation initiation by recruiting ribosomes to the 5'-terminal cap structure. The binding of EIF4E to the 4F complex becomes a rate-limiting stage in translation initiation. EIF4E is present in various animals including humans, and its sequence information is also publicly known (e.g., human: NM_001968.3, NM_001130679.1, NM_001130678.1, XM_006714126.2, XM_006714127.2 (NP_001959.1, NP_001124151.1, NP_001124150.1, XP_006714189.1, XP_006714190.1), etc.; the numbers represent accession numbers of the NCBI database, and the nucleotide sequence and the amino acid sequence are indicated outside and inside the parentheses, respectively). As one example, the nucleotide sequence of the human EIF4E gene registered in the database as NM_001968.3 is shown in SEQ ID NO: 99, and the amino acid sequence of the human EIF4E protein encoded by this human EIF4E gene is shown in SEQ ID NO: 100. In the present specification, EIF4E is not limited to a protein consisting of the amino acid sequence of SEQ ID NO: 100 encoded by the nucleotide sequence of SEQ ID NO: 99. As for EIF4E, sequence information has been registered with a plurality of accession numbers as mentioned above, and a plurality of transcript variants are present. The nucleotide sequence of SEQ ID NO: 99 represents the nucleotide sequence of one of these transcript variants.

ILK, integrin linked kinase, is a protein having a kinase-like domain and four ankyrin-like repeats and is known to control integrin-mediated signal transduction by binding to the cytoplasmic domain of β integrin in the cell membrane. ILK is present in various animals including humans, and its sequence information is also publicly known (e.g., human: NM_004517.3, NM_001278441.1, NM_001014794.2, NM_001278442.1, NM_001014795.2, XM_005252904.3, XM_005252905.1, XM_011520065.1 (NP_004508.1, NP_001265370.1, NP_001014794.1, NP_001265371.1, NP_001014795.1, XP_005252961.1, XP_005252962.1, XP_011518367.1), etc.; the numbers represent accession numbers of the NCBI database, and the nucleotide sequence and the amino acid sequence are indicated outside and inside the parentheses, respectively). As one example, the nucleotide sequence of the human ILK gene registered in the database as NM_004517.3 is shown in SEQ ID NO: 101, and the amino acid sequence of the human ILK protein encoded by this human ILK gene is shown in SEQ ID NO: 102. In the present specification, ILK is not limited to a protein consisting of the amino acid sequence of SEQ ID NO: 102 encoded by the nucleotide sequence of SEQ ID NO: 101. As for ILK, sequence information has been registered with a plurality of accession numbers as mentioned above, and a plurality of transcript variants are present. The nucleotide sequence of SEQ ID NO: 101 represents the nucleotide sequence of one of these transcript variants.

MTCP1, mature T-cell proliferation 1, was identified, as is known, by involvement in some t(X;14) translocations associated with the growth of mature T cells. This region has a complicated structure with a common promoter and 5' exons spliced to two different sets of 3' exons that encode two different proteins. The upstream 13 kD protein is considered to belong to the TCL1 family and participate in leukemia induction. MTCP1 is present in various animals including humans, and its sequence information is also publicly known (e.g., human: NM_001018025.3 (NP_001018025.1), etc.; the numbers represent accession numbers of the NCBI database, and the nucleotide sequence and the amino acid sequence are indicated outside and inside the parentheses, respectively). As one example, the nucleotide sequence of the human MTCP1 gene registered in the database as NM_001018025.3 is shown in SEQ ID NO: 103, and the amino acid sequence of the human MTCP1 protein encoded by this human MTCP1 gene is shown in SEQ ID NO: 104. In the present specification, MTCP1 is not limited to a protein consisting of the amino acid sequence of SEQ ID NO: 104 encoded by the nucleotide sequence of SEQ ID NO: 103. As for MTCP1, there is the possibility that a plurality of transcript variants are present. The nucleotide sequence of SEQ ID NO: 103 represents the nucleotide sequence of one of these transcript variants.

PIK3CA, phosphatidylinositol-4,5-bisphosphate 3-kinase, catalytic subunit alpha, is a 110 kD catalytic subunit in phosphatidylinositol 3-kinase and phosphorylates PtdIns, PtdIns4P, and PtdIns(4,5)P2 by use of ATP. PIK3CA is present in various animals including humans, and its sequence information is also publicly known (e.g., human: NM_006218.2, XM_006713658.2, XM_011512894.1 (NP_006209.2, XP_006713721.1, XP_011511196.1), etc.; the numbers represent accession numbers of the NCBI database, and the nucleotide sequence and the amino acid sequence are indicated outside and inside the parentheses, respectively). As one example, the nucleotide sequence of the human PIK3CA gene registered in the database as NM_006218.2 is shown in SEQ ID NO: 105, and the amino acid sequence of the human PIK3CA protein encoded by this human PIK3CA gene is shown in SEQ ID NO: 106. In the present specification, PIK3CA is not limited to a protein consisting of the amino acid sequence of SEQ ID NO: 106 encoded by the nucleotide sequence of SEQ ID NO: 105. As for PIK3CA, sequence information has been registered with a plurality of accession numbers as mentioned above, and a plurality of transcript variants are present. The nucleotide sequence of SEQ ID NO: 105 represents the nucleotide sequence of one of these transcript variants.

SRF, serum response factor, is known as a ubiquitous nuclear protein that stimulates cell growth and differentiation. SRF belongs to the transcription factor MADS (MCM1, Agamous, Deficiens, and SRF) box superfamily. SRF binds to a serum response element (SRE) in the promoter region of a target gene. SRF regulates the activation of many immediate early genes such as c-fos and thereby participates in cell cycle regulation, apoptosis, cell growth, and cell differentiation. SRF is present in various animals including humans, and its sequence information is also publicly known (e.g., human: NM_003131.3, NM_001292001.1 (NP_003122.1, NP_001278930.1), etc.; the numbers represent accession numbers of the NCBI database, and the nucleotide sequence and the amino acid sequence are indicated outside and inside the parentheses, respectively). As one example, the nucleotide sequence of the human SRF gene registered in the database as NM_003131.3 is shown in SEQ ID NO: 107, and the amino acid sequence of the human SRF protein encoded by this human SRF gene is shown in SEQ ID NO: 108. In the present specification, SRF is not limited to a protein consisting of the amino acid sequence of SEQ ID NO: 108 encoded by the nucleotide sequence of SEQ ID NO: 107. As for SRF, sequence information has been registered with a plurality of accession numbers as mentioned above, and a plurality of transcript variants are present. The nucleotide sequence of SEQ ID NO: 107 represents the nucleotide sequence of one of these transcript variants.

Although GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF can be identified by specific nucleotide sequences and amino acid sequences as mentioned above, the possibility must be taken into consideration that mutations occur in the nucleotide sequences or the amino acid sequences among organism individuals.

Specifically, GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF are not limited to proteins having the same sequences as the amino acid sequences registered in the database and include proteins that have sequences differing from these sequences by 1 or 2 or more, typically, 1 or several, for example, 1, 2, 3, 4, 5, 6, 7, 8, 9, or 10 amino acids and have functions equivalent to GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF.

Moreover, GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF include ones that consist of nucleotide sequences having 70% or higher, 80% or higher, 90% or higher, 95% or higher, or 97% or higher identity to the specific nucleotide sequences mentioned above and encode proteins having functions equivalent to GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG 1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF.

The specific functions of GST-π, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF are as mentioned above.

In the present specification, the phrases such as "as used herein", "used herein", "in the present specification", and "described herein" mean that the description subsequent thereto is applied to all aspects of the invention described in the present specification, unless otherwise specified. Also, all technical terms and scientific terms used herein have the same meanings as those commonly understood by those skilled in the art unless otherwise defined. All patents, bulletins, and other publications cited herein are incorporated herein by reference in their entirety.

The "drug inhibiting GST-π" used herein is not limited and includes, for example, drugs inhibiting the production and/or activity of GST-π and drugs promoting the degradation and/or deactivation of GST-π. Examples of the drug inhibiting the production of GST-π include, but are not limited to, RNAi molecules against DNA encoding GST-π, ribozymes, antisense nucleic acids, DNA/RNA chimeric polynucleotides, and vectors for expressing them.

Alternatively, any compound that acts on GST-π can be used as the drug inhibiting GST-π. An organic compound (an amino acid, a polypeptide or a derivative thereof, a low-molecular-weight compound, a sugar, a high-molecular-weight compound, etc.), an inorganic compound, or the like can be used as such a compound. Also, such a compound may be any of natural and nonnatural substances. Examples of the derivative of the polypeptide include modified polypeptides obtained by adding modifying groups, and variant polypeptides obtained by altering amino acid residues. In addition, such a compound may be a single compound or may be a compound library, an expression product of a gene library, a cell extract, a cell culture supernatant, a product by a fermentation microorganism, a marine organism extract, a plant extract, or the like. That is, the "drug inhibiting GST-π" is not limited to nucleic acids such as RNAi molecules and includes any compound.

Specifically, examples of the drug inhibiting the activity of GST-π include, but are not limited to, substances binding to GST-π, for example, glutathione, glutathione analogs (e.g., those described in WO 95/08563, WO 96/40205, WO 99/54346, Non Patent Literature 4, etc.), ketoprofen (Non Patent Literature 2), indomethacin (Hall et al., Cancer Res. 1989; 49 (22): 6265-8), ethacrynic acid, piriprost (Tew et al., Cancer Res. 1988; 48 (13): 3622-5), anti-GST-π antibodies, and dominant negative mutants of GST-π. These drugs are commercially available or can be appropriately produced on the basis of publicly known techniques.

RNAi molecules against DNA encoding GST-π, ribozymes, antisense nucleic acids, DNA/RNA chimeric polynucleotides, and vectors for expressing them are preferable as the drug inhibiting the production or activity of GST-π because of high specificity and a low possibility of adverse reactions.

The inhibition of GST-π can be determined when the expression or activity of GST-π in the cell is inhibited as compared with the case the GST-π-inhibiting agent is not allowed to act thereon. The expression of GST-π can be evaluated without limitations by a known arbitrary approach, for example, an immunoprecipitation method using an anti-GST-π antibody, EIA, ELISA, IRA, IRMA, Western blotting, an immunohistochemical method, an immunocytochemical method, flow cytometry, or various hybridization methods, Northern blotting, Southern blotting, or various PCR methods which employ nucleic acids specifically hybridizing to a nucleic acid encoding GST-π or a unique fragment thereof, or a transcript (e.g., mRNA) or a splicing product of the nucleic acid.

Also, the activity of GST-π can be evaluated without limitations by analyzing the known activity of GST-π, for example, binding activity against a protein such as Raf-1 (particularly, phosphorylated Raf-1) or EGFR (particularly, phosphorylated EGFR) by a known arbitrary method, for example, an immunoprecipitation method, Western blotting, mass spectrometry, a pull-down method, or a surface plasmon resonance (SPR) method.

The "drug inhibiting a homeostasis-related protein that exhibits synthetic lethality when inhibited together with GST-π" used herein is not limited and includes, for example, drugs inhibiting the production and/or activity of the protein and drugs promoting the degradation and/or deactivation of the protein. Examples of the drug inhibiting the production of the protein include, but are not limited to, RNAi molecules against DNA encoding the homeostasis-related protein, ribozymes, antisense nucleic acids, DNA/RNA chimeric polynucleotides, and vectors for expressing them. Alternatively, any compound that acts on the protein can be used as the drug inhibiting the activity of the homeostasis-related protein or the drug promoting the degradation and/or deactivation of the homeostasis-related protein. An organic compound (an amino acid, a polypeptide or a derivative thereof, a low-molecular-weight compound, a sugar, a high-molecular-weight compound, etc.), an inorganic compound, or the like can be used as such a compound. Also, such a compound may be any of natural and nonnatural substances. Examples of the derivative of the polypeptide include modified polypeptides obtained by adding modifying groups, and variant polypeptides obtained by altering amino acid residues. In addition, such a compound may be a single compound or may be a compound library, an expression product of a gene library, a cell extract, a cell culture supernatant, a product by a fermentation microorganism, a marine organism extract, a plant extract, or the like. That is, the "drug inhibiting a homeostasis-related protein that exhibits synthetic lethality when inhibited together with GST-π" is not limited to nucleic acids such as RNAi molecules and includes any compound.

More specifically, examples of the drug inhibiting the activity of p21 among the cell cycle-regulating proteins that exhibit synthetic lethality when inhibited together with GST-π include, but are not limited to: butyrolactone I (Sax et al., Cell Cycle, Jan; 1 (1): 90-6,2002), which is a low-molecular-weight compound promoting the proteasomal degradation of the p21 protein while also inhibiting the enzymatic activity of CDC2, CDK2, and CDK5; quetiapine (Kondo et al., Transl. Psychiatry, Apr 2; 3: e243, 2013), which is a psychotropic drug reportedly specifically inhibiting the expression of p21 in the nerve cells or oligodendrocytes of CD-1 mice; Sorafenib (Inoue et al., Cancer Biology & Therapy, 12: 9, 827-836, 2011), which is a low-molecular-weight compound specifically inhibiting p21 without the involvement of p53, p27, or Akt and inhibiting multikinase for Raf, VEGFR, PDGFR, and the like; UC2288 (Wettersten et al., Cancer Biology & Therapy, 14 (3), 278-285, 2013), which is a low-molecular-weight compound specifically inhibiting p21 without the involvement of p53 or Akt; and RNAi molecules against DNA encoding p21, ribozymes, antisense nucleic acids, DNA/RNA chimeric polynucleotides, and vectors for expressing them, anti-p21 antibodies, and dominant negative variants of p21. These drugs are commercially available or can be appropriately produced on the basis of publicly known techniques.

Examples of the drug inhibiting the activity of RNPC1 among the cell cycle-regulating proteins that exhibit synthetic lethality when inhibited together with GST-π include, but are not limited to, RNAi molecules against DNA encoding RNPC1, ribozymes, antisense nucleic acids, DNA/RNA chimeric polynucleotides, and vectors for expressing them, anti-RNPC1 antibodies, and dominant negative variants of RNPC1. These drugs are commercially available or can be appropriately produced on the basis of publicly known techniques.

Examples of the drug inhibiting the activity of CCNL1 among the cell cycle-regulating proteins that exhibit synthetic lethality when inhibited together with GST-π include, but are not limited to, RNAi molecules against DNA encoding CCNL1, ribozymes, antisense nucleic acids, DNA/RNA chimeric polynucleotides, and vectors for expressing them, anti-CCNL1 antibodies, and dominant negative variants of CCNL1. These drugs are commercially available or can be appropriately produced on the basis of publicly known techniques.

Examples of the drug inhibiting the activity of MCM8 among the cell cycle-regulating proteins that exhibit synthetic lethality when inhibited together with GST-π include, but are not limited to, RNAi molecules against DNA encoding MCM8, ribozymes, antisense nucleic acids, DNA/RNA chimeric polynucleotides, and vectors for expressing them, anti-MCM8 antibodies, and dominant negative variants of MCM8. These drugs are commercially available or can be appropriately produced on the basis of publicly known techniques.

Examples of the drug inhibiting the activity of CCNB3 among the cell cycle-regulating proteins that exhibit synthetic lethality when inhibited together with GST-π include, but are not limited to, RNAi molecules against DNA encoding CCNB3, ribozymes, antisense nucleic acids, DNA/RNA chimeric polynucleotides, and vectors for expressing them, anti-CCNB3 antibodies, and dominant negative variants of CCNB3. These drugs are commercially available or can be appropriately produced on the basis of publicly known techniques.

Examples of the drug inhibiting the activity of MCMDC1 among the cell cycle-regulating proteins that exhibit synthetic lethality when inhibited together with Gust-π include, but are not limited to, RNAi molecules against DNA encoding MCMDC1, ribozymes, antisense nucleic acids, DNA/RNA chimeric polynucleotides, and vectors for expressing them, anti-MCMDC1 antibodies, and dominant negative variants of MCMDC1. These drugs are commercially available or can be appropriately produced on the basis of publicly known techniques.

Examples of the drug inhibiting the activity of ATM among the cell cycle-regulating proteins that exhibit synthetic lethality when inhibited together with GST-π include, but are not limited to: CGK 733 (Won et al., Nat. Chem. Biol. 2, 369, 2006), which is a low-molecular-weight compound selectively inhibiting the kinase activity of ATM and ATR; KU-55933 (Lau et al., Nat. Cell Biol. 7, 493, 2005), KU-60019 (Zirkin et al., J Biol Chem. Jul 26; 288 (30): 21770-83, 2013), and CP-466722 (Rainey et al., Cancer Res. Sep 15; 68 (18) :7466-74, 2008), which are low-molecular-weight compounds selectively inhibiting the kinase activity of ATM; and RNAi molecules against DNA encoding ATM, ribozymes, antisense nucleic acids, DNA/RNA chimeric polynucleotides, and vectors for expressing them, anti-ATM antibodies, and dominant negative variants of ATM. These drugs are commercially available or can be appropriately produced on the basis of publicly known techniques.

Examples of the drug inhibiting the activity of CDC25A among the cell cycle-regulating proteins that exhibit synthetic lethality when inhibited together with GST-π include, but are not limited to: NSC95397 (Lazo JS et al., Mol. Pharmacol. 61: 720-728, 2002), which is a low-molecular-weight compound inhibiting the phosphatase activity of each of human CDC25A, human CDC25B, and human CDC25C; SC alpha alpha delta 09 (Rice, R.L. et al., Biochemistry 36 (50): 15965-15974, 1997), which is a low-molecular-weight compound inhibiting the phosphatase activity of each of human CDC25A, human CDC25B, human CDC25C, and human tyrosine phosphatase PTB1B; and RNAi molecules against DNA encoding CDC25A, ribozymes, antisense nucleic acids, DNA/RNA chimeric polynucleotides, and vectors for expressing them, anti-CDC25A antibodies, and dominant negative variants of CDC25A. These drugs are commercially available or can be appropriately produced on the basis of publicly known techniques.

Examples of the drug inhibiting the activity of PRKDC among the cell cycle-regulating proteins that exhibit synthetic lethality when inhibited together with GST-π include, but are not limited to, RNAi molecules against DNA encoding PRKDC, ribozymes, antisense nucleic acids, DNA/RNA chimeric polynucleotides, and vectors for expressing them, anti-PRKDC antibodies, and dominant negative variants of PRKDC. These drugs are commercially available or can be appropriately produced on the basis of publicly known techniques.

Examples of the drug inhibiting the activity of RBBP8 among the cell cycle-regulating proteins that exhibit synthetic lethality when inhibited together with GST-π include, but are not limited to, RNAi molecules against DNA encoding RBBP8, ribozymes, antisense nucleic acids, DNA/RNA chimeric polynucleotides, and vectors for expressing them, anti-RBBP8 antibodies, and dominant negative variants of RBBP8. These drugs are commercially available or can be appropriately produced on the basis of publicly known techniques.

Examples of the drug inhibiting the activity of SKP2 among the cell cycle-regulating proteins that exhibit synthetic lethality when inhibited together with GST-π include, but are not limited to, RNAi molecules against DNA encoding SKP2, ribozymes, antisense nucleic acids, DNA/RNA chimeric polynucleotides, and vectors for expressing them, anti-SKP2 antibodies, and dominant negative variants of SKP2. These drugs are commercially available or can be appropriately produced on the basis of publicly known techniques.

Examples of the drug inhibiting the activity of MCM10 among the cell cycle-regulating proteins that exhibit synthetic lethality when inhibited together with GST-π include, but are not limited to, RNAi molecules against DNA encoding MCM10, ribozymes, antisense nucleic acids, DNA/RNA chimeric polynucleotides, and vectors for expressing them, anti-MCM10 antibodies, and dominant negative variants of MCM10. These drugs are commercially available or can be appropriately produced on the basis of publicly known techniques.

Examples of the drug inhibiting the activity of CENPH among the cell cycle-regulating proteins that exhibit synthetic lethality when inhibited together with GST-π include, but are not limited to, RNAi molecules against DNA encoding CENPH, ribozymes, antisense nucleic acids, DNA/RNA chimeric polynucleotides, and vectors for expressing them, anti-CENPH antibodies, and dominant negative variants of CENPH. These drugs are commercially available or can be appropriately produced on the basis of publicly known techniques.

Examples of the drug inhibiting the activity of BRSK1 among the cell cycle-regulating proteins that exhibit synthetic lethality when inhibited together with GST-π include, but are not limited to, RNAi molecules against DNA encoding BRSK1, ribozymes, antisense nucleic acids, DNA/RNA chimeric polynucleotides, and vectors for expressing them, anti-BRSK1 antibodies, and dominant negative variants of BRSK1. These drugs are commercially available or can be appropriately produced on the basis of publicly known techniques.

Examples of the drug inhibiting the activity of MYLK among the cell cycle-regulating proteins that exhibit synthetic lethality when inhibited together with GST-π include, but are not limited to, HA-100 Dihydrochloride (Gerard et al., J Clin Invest. Jan; 77 (1): 61-5. 1986), which is a low-molecular-weight compound inhibiting the activity of MYLK in addition to the inhibition of the activity of various protein kinases (PKA and PKC, etc.); Staurosporine (Tamaoki et al., Biochem. Biophys. Res. Commun. 135: 397-402. 1986), which is a low-molecular-weight compound inhibiting the activity of MYLK in addition to the inhibition of the activity of various protein kinases (PKA, PKC, PKG, CaMK, CaMKII, and phosphorylase kinase, etc.); Calphostin C (Kobayashi et al., Biochem. Biophys. Res. Commun. 159: 548-553. 1989), which is a low-molecular-weight compound serving as a selective inhibitor of PKC and also inhibiting the activity of MYLK; Piceatannol (Oliver et al., J. Biol. Chem. 269: 29697-29703. 1994), which is a low-molecular-weight compound serving as a tyrosine kinase inhibitor and also inhibiting the activity of MYLK; A-3 Hydrochloride (Inagaki et al., Mol. Pharmacol. 29, 577. 1986), which is a low-molecular-weight compound inhibiting the activity of MYLK in addition to the inhibition of the activity of various casein kinases and protein kinases; H-7 Dihydrochloride (Kawamoto et al., Biochem. Biophys. Res. Commun. 125: 258-264. 1984), which is a low-molecular-weight compound serving as a cell-permeable serine/threonine kinase inhibitor and also inhibiting the activity of MYLK; H-9 Hydrochloride (Wolf et al., J. Biol. Chem. 260: 15718-15722. 1985), which is a low-molecular-weight compound inhibiting the activity of MYLK in addition to the inhibition of the activity of various protein kinases (PKA and PKC, etc.); W-5 (Hidaka et al., Mol. Pharmacol. 20: 571-578. 1981), which is a low-molecular-weight compound serving as a calmodulin (CaM) antagonist and also inhibiting the activity of MYLK; W-7 (Hidaka et al., Proc. Natl. Acad. Sci. U.S.A. 78: 4354-4357. 1981), which is a low-molecular-weight compound serving as a calmodulin (CaM) antagonist and also inhibiting the activity of MYLK; W-13 Isomer Hydrochloride (Hidaka et al., Proc. Natl. Acad. Sci. U.S.A. 78: 4354-4357. 1981), which is a low-molecular-weight compound inhibiting the activity of MYLK in addition to the inhibition of the activity of various protein kinases (PKA and PKC, etc.); ML-7 Dihydrochloride (Saitoh et al., J. Biol. Chem. 262: 7796-7801. 1987), which is a low-molecular-weight compound inhibiting the activity of MYLK in addition to the inhibition of the activity of various protein kinases (PKA and PKC, etc.); ML-9 (Saitoh et al., Biochem. Biophys. Res. Commun. 140: 280-287. 1986), which is a low-molecular-weight compound selectively inhibiting MYLK and CaMK; Myricetin (Hagiwara et al., Biochem. Pharmacol. 37: 2987-2992. 1988), which is a low-molecular-weight compound inhibiting the activity of MYLK in addition to the inhibition of the activity of various casein kinases and protein kinases; E6 Berbamine (Hu et al., Biochem. Pharmacol. Oct 20; 44 (8): 1543-7. 1992), which is a low-molecular-weight compound serving as a cell-permeable calmodulin (CaM) antagonist and also inhibiting the activity of MYLK; K-252a (Kase et al., J. Antibiot. 39: 1059-1065. 1986), which is a low-molecular-weight compound inhibiting the activity of MYLK in addition to the inhibition of the activity of various protein kinases; K-252b (Nakanishi et al., J. Antibiot. 39: 1066-1071. 1986), which is a low-molecular-weight compound inhibiting the activity of MYLK in addition to the inhibition of the activity of various protein kinases; Altenusin (Rosett et al., Biochem. J. 67: 390-400. 1957), which is a low-molecular-weight compound serving as a c-Src inhibitor and also inhibiting the activity of MYLK; and RNAi molecules against DNA encoding MYLK, ribozymes, antisense nucleic acids, DNA/RNA chimeric polynucleotides, and vectors for expressing them, anti-MYLK antibodies, and dominant negative variants of MYLK. These drugs are commercially available or can be appropriately produced on the basis of publicly known techniques.

On the other hand, examples of the drug inhibiting the activity of AATF among the anti-apoptosis-related proteins that exhibit synthetic lethality when inhibited together with GST-π include, but are not limited to, RNAi molecules against DNA encoding AATF, ribozymes, antisense nucleic acids, DNA/RNA chimeric polynucleotides, and vectors for expressing them, anti-AATF antibodies, and dominant negative variants of AATF. These drugs are commercially available or can be appropriately produced on the basis of publicly known techniques.

Examples of the drug inhibiting the activity of ALOX12 among the anti-apoptosis-related proteins that exhibit synthetic lethality when inhibited together with GST-π include, but are not limited to, RNAi molecules against DNA encoding ALOX12, ribozymes, antisense nucleic acids, DNA/RNA chimeric polynucleotides, and vectors for expressing them, anti-ALOX12 antibodies, and dominant negative variants of ALOX12. These drugs are commercially available or can be appropriately produced on the basis of publicly known techniques.

Examples of the drug inhibiting the activity of ANXA1 among the anti-apoptosis-related proteins that exhibit synthetic lethality when inhibited together with GST-π include, but are not limited to, RNAi molecules against DNA encoding ANXA1, ribozymes, antisense nucleic acids, DNA/RNA chimeric polynucleotides, and vectors for expressing them, anti-ANXA1 antibodies, and dominant negative variants of ANXA1. These drugs are commercially available or can be appropriately produced on the basis of publicly known techniques.

Examples of the drug inhibiting the activity of ANXA4 among the anti-apoptosis-related proteins that exhibit synthetic lethality when inhibited together with GST-π include, but are not limited to, RNAi molecules against DNA encoding ANXA4, ribozymes, antisense nucleic acids, DNA/RNA chimeric polynucleotides, and vectors for expressing them, anti-ANXA4 antibodies, and dominant negative variants of ANXA4. These drugs are commercially available or can be appropriately produced on the basis of publicly known techniques.

Examples of the drug inhibiting the activity of API5 among the anti-apoptosis-related proteins that exhibit synthetic lethality when inhibited together with GST-π include, but are not limited to, RNAi molecules against DNA encoding API5, ribozymes, antisense nucleic acids, DNA/RNA chimeric polynucleotides, and vectors for expressing them, anti-API5 antibodies, and dominant negative variants of API5. These drugs are commercially available or can be appropriately produced on the basis of publicly known techniques.

Examples of the drug inhibiting the activity of ATF5 among the anti-apoptosis-related proteins that exhibit synthetic lethality when inhibited together with GST-π include, but are not limited to, RNAi molecules against DNA encoding AATF5, ribozymes, antisense nucleic acids, DNA/RNA chimeric polynucleotides, and vectors for expressing them, anti-ATF5 antibodies, and dominant negative variants of ATF5. These drugs are commercially available or can be appropriately produced on the basis of publicly known techniques.

Examples of the drug inhibiting the activity of AVEN among the anti-apoptosis-related proteins that exhibit synthetic lethality when inhibited together with GST-π include, but are not limited to, RNAi molecules against DNA encoding AVEN, ribozymes, antisense nucleic acids, DNA/RNA chimeric polynucleotides, and vectors for expressing them, anti-AVEN antibodies, and dominant negative variants of AVEN. These drugs are commercially available or can be appropriately produced on the basis of publicly known techniques.

Examples of the drug inhibiting the activity of AZU1 among the anti-apoptosis-related proteins that exhibit synthetic lethality when inhibited together with GST-π include, but are not limited to, RNAi molecules against DNA encoding AZU1, ribozymes, antisense nucleic acids, DNA/RNA chimeric polynucleotides, and vectors for expressing them, anti-AZU1 antibodies, and dominant negative variants of AZU1. These drugs are commercially available or can be appropriately produced on the basis of publicly known techniques.

Examples of the drug inhibiting the activity of BAG 1 among the anti-apoptosis-related proteins that exhibit synthetic lethality when inhibited together with GST-π include, but are not limited to, RNAi molecules against DNA encoding BAG1, ribozymes, antisense nucleic acids, DNA/RNA chimeric polynucleotides, and vectors for expressing them, anti-BAG1 antibodies, and dominant negative variants of BAG1. These drugs are commercially available or can be appropriately produced on the basis of publicly known techniques.

Examples of the drug inhibiting the activity of BCL2L1 among the anti-apoptosis-related proteins that exhibit synthetic lethality when inhibited together with GST-π include, but are not limited to, RNAi molecules against DNA encoding BCL2L1, ribozymes, antisense nucleic acids, DNA/RNA chimeric polynucleotides, and vectors for expressing them, anti-BCL2L1 antibodies, and dominant negative variants of BCL2L1. These drugs are commercially available or can be appropriately produced on the basis of publicly known techniques.

Examples of the drug inhibiting the activity of BFAR among the anti-apoptosis-related proteins that exhibit synthetic lethality when inhibited together with GST-π include, but are not limited to, RNAi molecules against DNA encoding BFAR, ribozymes, antisense nucleic acids, DNA/RNA chimeric polynucleotides, and vectors for expressing them, anti-BFAR antibodies, and dominant negative variants of BFAR. These drugs are commercially available or can be appropriately produced on the basis of publicly known techniques.

Examples of the drug inhibiting the activity of CFLAR among the anti-apoptosis-related proteins that exhibit synthetic lethality when inhibited together with GST-π include, but are not limited to, RNAi molecules against DNA encoding CFLAR, ribozymes, antisense nucleic acids, DNA/RNA chimeric polynucleotides, and vectors for expressing them, anti-CFLAR antibodies, and dominant negative variants of CFLAR. These drugs are commercially available or can be appropriately produced on the basis of publicly known techniques.

Examples of the drug inhibiting the activity of IL2 among the anti-apoptosis-related proteins that exhibit synthetic lethality when inhibited together with GST-π include, but are not limited to, RNAi molecules against DNA encoding IL2, ribozymes, antisense nucleic acids, DNA/RNA chimeric polynucleotides, and vectors for expressing them, anti-IL2 antibodies, and dominant negative variants of IL2. These drugs are commercially available or can be appropriately produced on the basis of publicly known techniques.

Examples of the drug inhibiting the activity of MALT1 among the anti-apoptosis-related proteins that exhibit synthetic lethality when inhibited together with GST-π include, but are not limited to, RNAi molecules against DNA encoding MALT1, ribozymes, antisense nucleic acids, DNA/RNA chimeric polynucleotides, and vectors for expressing them, anti-MALT1 antibodies, and dominant negative variants of MALT1. These drugs are commercially available or can be appropriately produced on the basis of publicly known techniques.

Examples of the drug inhibiting the activity of MCL1 among the anti-apoptosis-related proteins that exhibit synthetic lethality when inhibited together with GST-π include, but are not limited to, Synribo (omacetaxine mepesuccinate), which is approved as a therapeutic agent for chronic myelocytic leukemia, RNAi molecules against DNA encoding MCL1, ribozymes, antisense nucleic acids, DNA/RNA chimeric polynucleotides, and vectors for expressing them, anti-MCL1 antibodies, and dominant negative variants of MCL1. These drugs are commercially available or can be appropriately produced on the basis of publicly known techniques.

Examples of the drug inhibiting the activity of MKL1 among the anti-apoptosis-related proteins that exhibit synthetic lethality when inhibited together with GST-π include, but are not limited to, RNAi molecules against DNA encoding MKL1, ribozymes, antisense nucleic acids, DNA/RNA chimeric polynucleotides, and vectors for expressing them, anti-MKL1 antibodies, and dominant negative variants of MKL1. These drugs are commercially available or can be appropriately produced on the basis of publicly known techniques.

Examples of the drug inhibiting the activity of MPO among the anti-apoptosis-related proteins that exhibit synthetic lethality when inhibited together with GST-π include, but are not limited to, RNAi molecules against DNA encoding MPO, ribozymes, antisense nucleic acids, DNA/RNA chimeric polynucleotides, and vectors for expressing them, anti-MPO antibodies, and dominant negative variants of MPO. These drugs are commercially available or can be appropriately produced on the basis of publicly known techniques.

Examples of the drug inhibiting the activity of MTL5 among the anti-apoptosis-related proteins that exhibit synthetic lethality when inhibited together with GST-π include, but are not limited to, RNAi molecules against DNA encoding MTL5, ribozymes, antisense nucleic acids, DNA/RNA chimeric polynucleotides, and vectors for expressing them, anti-MTL5 antibodies, and dominant negative variants of MTL5. These drugs are commercially available or can be appropriately produced on the basis of publicly known techniques.

Examples of the drug inhibiting the activity of MYBL2 among the anti-apoptosis-related proteins that exhibit synthetic lethality when inhibited together with GST-π include, but are not limited to, RNAi molecules against DNA encoding MYBL2, ribozymes, antisense nucleic acids, DNA/RNA chimeric polynucleotides, and vectors for expressing them, anti-MYBL2 antibodies, and dominant negative variants of MYBL2. These drugs are commercially available or can be appropriately produced on the basis of publicly known techniques.

Examples of the drug inhibiting the activity of MYO18A among the anti-apoptosis-related proteins that exhibit synthetic lethality when inhibited together with GST-π include, but are not limited to, RNAi molecules against DNA encoding MYO18A, ribozymes, antisense nucleic acids, DNA/RNA chimeric polynucleotides, and vectors for expressing them, anti-MYO18A antibodies, and dominant negative variants of MYO18A. These drugs are commercially available or can be appropriately produced on the basis of publicly known techniques.

On the other hand, examples of the drug inhibiting the activity of MTOR among the PI3K signaling pathway-related proteins that exhibit synthetic lethality when inhibited together with GST-π include, but are not limited to, Rapamycin (Chang et al., Trends Pharmacol. Sci. 12: 218-223. 1991), which is a low-molecular-weight compound serving as a macrolide immunosuppressant and inhibiting the activity of MTOR; Everolimus (Weisblum et al., Br. Med. Bull. 40: 47-53. 1984), which is a low-molecular-weight compound serving as a rapamycin-derived macrolide immunosuppressant and inhibiting the activity of MTOR; BEZ235 (Maira et al., Mol. Cancer Ther. 7 (7): 1851-1863. 2008), which is a low-molecular-weight compound serving as a PI3K tyrosine kinase inhibitor and inhibiting the activity of MTOR; AZD8055 (Huang et al., J Biol Chem. Nov 18; 286 (46): 40002-12. 2011), which is a low-molecular-weight compound selectively inhibiting the kinase activity of MTOR; PI-103 (Demyanets et al., 2010. Basic Res Cardiol. Mar; 106 (2): 217-31. 2011), which is a pyridinylfuranopyrimidine compound and is a low-molecular-weight compound inhibiting the kinase activity of DNA-PK, PI3K, and MTOR; and Niclosamide (Balgi et al., PLoS One. Sep 22; 4 (9): e7124. 2009), PP242 (Bao et al. J. Cell Biol. 210 (7): 1153-64. 2015), Timosaponin AIII (King et.al, PLoS One. Sep 30;4 (9):e7283 2009), KU 0063794 (Garcia-Martinez et al. Biochem. J. 421 (1): 29-42. 2009), AZD2014 (Guichard et al. Mol. Cancer. Ther. 14 (11): 2508-18. 2015), Temsirolimus (Raymond et al. J. Clin. Oncol. 22: 2336-2347. 2004), Palomid 529 (Xue et al. Cancer Res. 68 (22): 9551-7. 2008), Fisetin (Suh et.al, Carcinogenesis. Aug;31 (8):1424-33 2010), GDC-0980 (Wallin et al. Mol. Cancer Ther. 10 (12): 2426-36. 2011), SF1126 (Garlich et al. Cancer Res. 68 (1): 206-15. 2008), CH5132799 (Tanaka et al. Clin Cancer Res. 17 (10): 3272-81. 2011), WYE-354 (Yu et al. Cancer Res. 69 (15): 6232-40. 2009), Compound 401 (Ballou et al. J. Biol. Chem. 282, 24463. 2007), Ridaforolimus (Gadducci et al.: Gynecol. Endocrinol. 24, 239. 2008), GSK 1059615 (Steven et al. ACS. Med. Chem. Lett. 1 (1): 39-43 2010), PF-04691502 (Yuan et al. Mol. Cancer Ther. 10 (11): 2189-99. 2011), PP121 (Apsel et al. Nat. Chem. Biol. 4 (11): 691-9. 2008), OSI-027 (Bhaqwat et al. Mol. Cancer Ther. 10 (8): 1394-406. 2011), WYE-125132 (Yu et al. Cancer Res. 70 (2): 621-31. 2010), Umirolimus (Baldo et al. 2008. Curr. Cancer Drug Targets. 8 (8): 647-65. 2008), WAY-600 (Yu et al. Cancer Res. 69 (15): 6232-40. 2009), WYE-687 (Yu et al. Cancer Res. 69 (15): 6232-40. 2009), PKI-179 (Venkatesan et al. Bioorg. Med. Chem. Lett. 20 (19): 5869-73. 2010), PF-05212384 (Akintunde et al. J. Hematol. Oncol. 6: 88. 2013), CAY10626 (Rameh et al. J. Biol. Chem. 274: 8347-8350. 1999), NVP-BGT226 (Chang et al. Clin. Cancer Res. 17 (22): 7116-26. 2011), XL-147 derivative 1 (Akintunde et al. J. Hematol. Oncol. 6: 88. 2013), XL 388 (Eduardo et al. Mol Cancer Ther. 10 (3): 395-403. 2011), Torin 1 (Liu et al. J. Med. Chem. 53 (19): 7146-55. 2010) and RNAi molecules against DNA encoding MTOR, ribozymes, antisense nucleic acids, DNA/RNA chimeric polynucleotides, and vectors for expressing them, anti-MTOR antibodies, and dominant negative variants of MTOR. These drugs are commercially available or can be appropriately produced on the basis of publicly known techniques.

Examples of the drug inhibiting the activity of IRAK1 among the PI3K signaling pathway-related proteins that exhibit synthetic lethality when inhibited together with GST-π include, but are not limited to, Interleukin-1 Receptor-Associated-Kinase-1/4 Inhibitor (Bhattacharyya et al., Am. J. Physiol. Gastrointest. Liver Physiol. 293, G429. 2007), which is a benzimidazole compound and is a low-molecular-weight compound specifically inhibiting the activity of IL-1 kinase; and RNAi molecules against DNA encoding IRAK1, ribozymes, antisense nucleic acids, DNA/RNA chimeric polynucleotides, and vectors for expressing them, anti-IRAK1 antibodies, and dominant negative variants of IRAK1. These drugs are commercially available or can be appropriately produced on the basis of publicly known techniques.

Examples of the drug inhibiting the activity of IRS1 among the PI3K signaling pathway-related proteins that exhibit synthetic lethality when inhibited together with GST-π include, but are not limited to, RNAi molecules against DNA encoding IRS1, ribozymes, antisense nucleic acids, DNA/RNA chimeric polynucleotides, and vectors for expressing them, anti-IRS1 antibodies, and dominant negative variants of IRS1. These drugs are commercially available or can be appropriately produced on the basis of publicly known techniques.

Examples of the drug inhibiting the activity of MYD88 among the PI3K signaling pathway-related proteins that exhibit synthetic lethality when inhibited together with GST-π include, but are not limited to, MyD88 Inhibitory Peptide Pepinh-MYD (Derossi et al., J. Biol. Chem., 269: 10444-50. 1994), which is a MYD88-derived 26-amino acid peptide inhibiting homodimerization; TJ-M2010 (Li et al., Transplant Proc. 45 (5): 1842-5. 2013), which is a low-molecular-weight compound specifically inhibiting MYD88; and RNAi molecules against DNA encoding MYD88, ribozymes, antisense nucleic acids, DNA/RNA chimeric polynucleotides, and vectors for expressing them, anti-MYD88 antibodies, and dominant negative variants of MYD88. These drugs are commercially available or can be appropriately produced on the basis of publicly known techniques.

Examples of the drug inhibiting the activity of NFKB1 among the PI3K signaling pathway-related proteins that exhibit synthetic lethality when inhibited together with GST-π include, but are not limited to, BAY 11-7085 (Pierce et al., J. Biol. Chem. 272: 21096-21103. 1997), which is a low-molecular-weight compound inhibiting the activity of both NF-κB activation and IκBα phosphorylation; Helenalin (Lyss et al., J. Biol. Chem. 273 (50): 33508-16. 1998), which is a low-molecular-weight compound serving as an apoptosis-inducing anticancer agent and inhibiting NF-κB; Caffeic Acid Phenethyl Ester (Sud'ina et al., FEBS Lett. Aug 23; 329 (1-2): 21-4. 1993), which is a low-molecular-weight compound serving as an inhibitor of HIV integrase and tyrosine kinase and specifically inhibiting the activity of NF-κB; and NFκB Activation Inhibitor II, JSH-23 (Shin et al., FEBS Lett. 571: 50-54. 2004), QNZ (Tobe et al. Bioorg. Med. Chem. 11: 3869-3878. 2003), Andrographolide (Yu et al. Planta Med. Dec;69 (12):1075-9. 2003), Curcumin (Asai et al. J. Nutr. 131: 2932-2935. 2001), Aspirin (Kopp et al. Science. 265: 956-959. 1994), Sulfasalazine (Liptay et al. Br. J. Pharmacol. 128, 1361. 1999), Rocaglamide (Engelmeier et al. J. Agric. Food Chem. 48: 1400-1404. 2000), SM 7368 (Lee et al. Biochem. Biophys. Res. Commun. 336: 716-722. 2005), Sulindac Sulfide (Meade et al. J. Biol. Chem. 268: 6610-6614. 1993), Trichodion (Erkel et al. FEBS Lett. 477, 219. 2000), CHS-828 (Hovstadius et al. Clin. Cancer Res. 8 (9): 2843-50. 2002), Z-VRPR-FMK (Hailfinger et al. Proc. Natl. Acad. Sci. U.S.A. 106 (47): 19946-19951. 2009), Sodium Salicylate (Kopp et al. Science. 265: 956-959. 1994), 4-Aminosalicylic Acid (Eberhardt et al. Biochem. Biophys. Res. Commun. 200: 163-170. 1994), Ethyl 3,4-Dihydroxycinnamate (Nakayama et al. Biosci. Biotechnol. Biochem. 60: 316-318. 1996), CAY10512 (Heynekamp et al. J Med Chem 49 7182-7189 2006), N-Stearoyl Phytosphingosine (Ryu et al. Lipids. 45 (7): 613-8. 2010), Palmitic Acid Methyl Ester (Cai et al. Toxicology. 210: 197-204. 2005), 9-Methylstreptimidone (Ishikawa et al. Bioorg. Med. Chem. Lett. 19 (6): 1726-8. 2009), Rocaglaol (Engelmeier et al. J. Agric. Food Chem. 48: 1400-1404. 2000), BAY 11-7082 (Pierce et al. J. Biol. Chem. 272: 21096-21103. 1997), and RNAi molecules against DNA encoding NFKB1, ribozymes, antisense nucleic acids, DNA/RNA chimeric polynucleotides, and vectors for expressing them, anti-NFKB1 antibodies, and dominant negative variants of NFKB1. These drugs are commercially available or can be appropriately produced on the basis of publicly known techniques.

Examples of the drug inhibiting the activity of PIK3CG among the PI3K signaling pathway-related proteins that exhibit synthetic lethality when inhibited together with GST-π include, but are not limited to, CUDC-907 (Qian et al., Clin Cancer Res. 18 (15): 4104-4113. 2012), which is a low-molecular-weight compound specifically inhibiting the activity of both HDAC and PI3K; PKI-402 (Dehnhardt et al., J Med Chem. Jan 28; 53 (2): 798-810. 2010), which is a low-molecular-weight compound specifically inhibiting the activity of both PI3K and mTOR; PF-04691502 (Yuan et al., Mol Cancer Ther, 10 (11), 2189-2199. 2011), which is a low-molecular-weight compound specifically inhibiting the activity of both PI3K and mTOR; NVP-BGT226 (Glienke et al., Tumor Biology, 33 (3): 757-765. 2012), which is a low-molecular-weight compound specifically inhibiting the activity of both PI3K and mTOR; and IPI-145 (INK1197) (Winkler et al., Chem Biol. 2013 Nov 21; 20 (11): 1364-74. 2013), SAR245409 (XL765) (Dai et.al, Endocrinology. Mar;154 (3):1247-592013), ZSTK474 (Toyama et.al, Arthritis Res Ther. 12 (3): R92. 2010), VS-5584 (SB2343) (Hart et.al, Mol Cancer Ther. 2013 Feb;12 (2):151-61. 2013), AS-605240 (Camps et.al, Nature medicine, 11 (9): 936-943. 2005), PIK-90 (Van et.al, J Cell Biol. 2006 Jul 31;174 (3):437-45. 2006), PF-4989216 (Walls et.al, Clin Cancer Res. 2014 Feb 1;20 (3):631-43. 2014), TG100-115 (Walls et.al, Proc Natl Acad Sci USA. Dec 26;103 (52):19866-71. 2006), BKM120 (Bendell et.al, J. Clin. Oncol. 30 (3): 282-90. 2012), BEZ235 Tosylate (Maira et.al, Mol Cancer Ther 2008; 7: 1851-1863. 2008), LY294002 (Maira et.al, Biochem. Soc. Trans. 37 (Pt1): 265-72. 2009), PI-103 (Raynaud et.al, Molecular Cancer Therapeutics, 8 (7): 1725-1738. 2009), XL147 (Shapiro et.al, Proc 97th Annu Meet AACR, 14-18. 2007), AS-252424 (Pomel et.al, J Med Chem. Jun 29;49 (13):3857-71. 2006), AS-604850 (Camps et.al, Nature medicine, 11 (9): 936-943. 2005), CAY10505 (Tyagi et.al, Can J Physiol Pharmacol. Jul;90 (7):881-5. 2012), CH5132799 (Ohwada et.al, Bioorganic & medicinal chemistry letters, 21 (6): 1767-1772. 2011), BAY 80-6946 (Copanlisib) (Patnaik et.al, J Clin Oncol, 29, 2011), GDC-0032 (Ndubaku et.al, J Med Chem. Jun 13;56 (11):4597-610. 2013), GSK1059615 (Knight et.al, ACS Medicinal Chemistry Letters, 1 (1): 39-43. 2010), CAL-130 (Subramaniam et.al, Cancer Cell. 21 (4):459-72. 2012), XL765 (Laird et.al, AACR-NCI-EORTC International Conference on Molecular Targets and Cancer Therapeutics. October p.B250 2007), and RNAi molecules against DNA encoding PIK3CG, ribozymes, antisense nucleic acids, DNA/RNA chimeric polynucleotides, and vectors for expressing them, anti-PIK3CG antibodies, and dominant negative variants of PIK3CG. These drugs are commercially available or can be appropriately produced on the basis of publicly known techniques.

Examples of the drug inhibiting the activity of RAC1 among the PI3K signaling pathway-related proteins that exhibit synthetic lethality when inhibited together with GST-π include, but are not limited to, NSC 23766 (Gao et al., Proc. Natl. Acad. Sci. U.S.A. 101: 7618-7623. 2004), which is a low-molecular-weight compound specifically inhibiting the activity of Rac GTPase; W56 (Gao et al., J. Biol. Chem. 276 47530. 2001), which is a peptide derived from guanine nucleotide exchange factor recognition/activation site of RAC1; and RNAi molecules against DNA encoding RAC1, ribozymes, antisense nucleic acids, DNA/RNA chimeric polynucleotides, and vectors for expressing them, anti-RAC1 antibodies, and dominant negative variants of RAC1. These drugs are commercially available or can be appropriately produced on the basis of publicly known techniques.

Examples of the drug inhibiting the activity of AKT3 among the PI3K signaling pathway-related proteins that exhibit synthetic lethality when inhibited together with GST-π include, but are not limited to, MK-2206 Dihydrochloride (Hirai et al., Mol. Cancer ther. 9 (7): 1956-67. 2010), which is a low-molecular-weight compound specifically inhibiting the activity of Akt1, Akt2, and Akt3; Triciribine (Moore et al., Biochem. Pharmacol. 38: 4037-4044. 1989), which is a low-molecular-weight compound specifically inhibiting the activity of Akt; Akt Inhibitor VIII (Barnett et al., Biochem. J. 385: 399-408. 2005), which is a cell-permeable quinoxaline compound and is a low-molecular-weight compound specifically inhibiting the activity of Akt1, Akt2, and Akt3; GSK 690693 (Rhodes et al., Cancer Res. 68 (7): 2366-2374. 2008), which is a compound derived from Aminofurazan and is a low-molecular-weight compound specifically inhibiting the activity of Akt1, Akt2, and Akt3; AT7867 (Grimshaw et al., Mol. Cancer Ther. 9 (5): 1100-10. 2010), which is a low-molecular-weight compound specifically inhibiting the activity of Akt1, Akt2, and Akt3; and RNAi molecules against DNA encoding AKT3, ribozymes, antisense nucleic acids, DNA/RNA chimeric polynucleotides, and vectors for expressing them, anti-AKT3 antibodies, and dominant negative variants of AKT3. These drugs are commercially available or can be appropriately produced on the basis of publicly known techniques.

Examples of the drug inhibiting the activity of EIF4B among the PI3K signaling pathway-related proteins that exhibit synthetic lethality when inhibited together with GST-π include, but are not limited to, RNAi molecules against DNA encoding EIF4B, ribozymes, antisense nucleic acids, DNA/RNA chimeric polynucleotides, and vectors for expressing them, anti-EIF4B antibodies, and dominant negative variants of EIF4B. These drugs are commercially available or can be appropriately produced on the basis of publicly known techniques.

Examples of the drug inhibiting the activity of EIF4E among the PI3K signaling pathway-related proteins that exhibit synthetic lethality when inhibited together with GST-π include, but are not limited to, 4EGI-1 (Moerke et al., Cell. 128: 257-267. 2007), which is a low-molecular-weight compound specifically inhibiting EIF4E/EIF4G interaction; and RNAi molecules against DNA encoding EIF4E, ribozymes, antisense nucleic acids, DNA/RNA chimeric polynucleotides, and vectors for expressing them, anti-EIF4E antibodies, and dominant negative variants of EIF4E. These drugs are commercially available or can be appropriately produced on the basis of publicly known techniques.

Examples of the drug inhibiting the activity of ILK among the PI3K signaling pathway-related proteins that exhibit synthetic lethality when inhibited together with GST-π include, but are not limited to, Cpd 22 (Lee et al., J. Med. Chem. 54, 6364. 2011), which is a cell-permeable pyrazole compound and is a low-molecular-weight compound specifically inhibiting ILK; QLT0267 (Younes et al., Mol Cancer Ther. Aug; 4 (8): 1146-56. 2005), which is a low-molecular-weight compound specifically inhibiting the kinase activity of ILK; and RNAi molecules against DNA encoding ILK, ribozymes, antisense nucleic acids, DNA/RNA chimeric polynucleotides, and vectors for expressing them, anti-ILK antibodies, and dominant negative variants of ILK. These drugs are commercially available or can be appropriately produced on the basis of publicly known techniques.

Examples of the drug inhibiting the activity of MTCP1 among the PI3K signaling pathway-related proteins that exhibit synthetic lethality when inhibited together with GST-π include, but are not limited to, RNAi molecules against DNA encoding MTCP1, ribozymes, antisense nucleic acids, DNA/RNA chimeric polynucleotides, and vectors for expressing them, anti-MTCP1 antibodies, and dominant negative variants of MTCP1. These drugs are commercially available or can be appropriately produced on the basis of publicly known techniques.

Examples of the drug inhibiting the activity of PIK3CA among the PI3K signaling pathway-related proteins that exhibit synthetic lethality when inhibited together with GST-π include, but are not limited to, HS-173 (Lee et al., Cancer Lett. Jan 1; 328 (1): 152-9. 2013), which is a low-molecular-weight compound specifically inhibiting PI3K; CUDC-907 (Qian et al., Clin Cancer Res. 18 (15): 4104-4113. 2012), which is a low-molecular-weight compound specifically inhibiting the activity of both HDAC and PI3K; PKI-402 (Dehnhardt et al., J Med Chem. Jan 28; 53 (2): 798-810. 2010), which is a low-molecular-weight compound specifically inhibiting the activity of both PI3K and mTOR; PF-04691502 (Yuan et al., Mol Cancer Ther, 10 (11), 2189-2199. 2011), which is a low-molecular-weight compound specifically inhibiting the activity of both PI3K and mTOR; NVP-BGT226 (Glienke et al., Tumor Biology, 33 (3): 757-765. 2012), which is a low-molecular-weight compound specifically inhibiting the activity of both PI3K and mTOR; and BYL719 (Furet et al., Bioorg Med Chem Lett 2013; 23: 3741-3748. 2013), SAR245409 (XL765) (Dai et.al, Endocrinology. Mar;154 (3):1247-592013), ZSTK474 (Toyama et.al, Arthritis Res Ther. 12 (3): R92. 2010), VS-5584 (SB2343) (Hart et.al, Mol Cancer Ther. 2013 Feb;12 (2):151-61. 2013), PIK-75 (Zheng et.al, Mol Pharmacol. Oct;80 (4):657-64. 2011), PIK-90 (Van et.al, J Cell Biol. 2006 Jul 31;174 (3):437-45. 2006), A66 (Jamieson et.al, Biochem J. 438:53-62. 2011), CNX1351 (Nacht et.al, J Med Chem, 56 (3): 712-721. 2013), PF-4989216 (Walls et.al, Clin Cancer Res. 2014 Feb 1;20 (3):631-43. 2014), BKM120 (Bendell et.al, J. Clin. Oncol. 30 (3): 282-90. 2012), BEZ235 Tosylate (Maira et.al, Mol Cancer Ther 2008; 7: 1851-1863. 2008), LY294002 (Maira et.al, Biochem. Soc. Trans. 37 (Pt1): 265-72. 2009), PI-103 (Raynaud et.al, Molecular Cancer Therapeutics, 8 (7): 1725-1738. 2009), XL147 (Shapiro et.al, Proc 97th Annu Meet AACR, 14-18. 2007), CH5132799 (Ohwada et.al, Bioorganic & medicinal chemistry letters, 21 (6): 1767-1772. 2011), BAY 80-6946 (Copanlisib) (Patnaik et.al, J Clin Oncol, 29, 2011), GDC-0032 (Ndubaku et.al, J Med Chem. Jun 13;56 (11):4597-610. 2013), XL765 (Laird et.al, AACR-NCI-EORTC International Conference on Molecular Targets and Cancer Therapeutics. October p.B250 2007), and RNAi molecules against DNA encoding PIK3CA, ribozymes, antisense nucleic acids, DNA/RNA chimeric polynucleotides, and vectors for expressing them, anti-PIK3CA antibodies, and dominant negative variants of PIK3CA. These drugs are commercially available or can be appropriately produced on the basis of publicly known techniques.

Examples of the drug inhibiting the activity of SRF among the PI3K signaling pathway-related proteins that exhibit synthetic lethality when inhibited together with GST-π include, but are not limited to, CCG-1423 (Evelyn et al., Mol. Cancer Ther. 6, 2249. 2007), which is a cell-permeable benzamide compound and is a low-molecular-weight compound specifically inhibiting the RHO signaling pathway and SRF activation; CCG-100602 (Evelyn, et al., Bioorg Med Chem Lett 20 665-72. 2010), which is a CCG-1423 analog and is a low-molecular-weight compound specifically inhibiting the RHO signaling pathway and SRF activation; and RNAi molecules against DNA encoding SRF, ribozymes, antisense nucleic acids, DNA/RNA chimeric polynucleotides, and vectors for expressing them, anti-SRF antibodies, and dominant negative variants of SRF. These drugs are commercially available or can be appropriately produced on the basis of publicly known techniques.

Particularly, RNAi molecules against DNA encoding the protein, ribozymes, antisense nucleic acids, DNA/RNA chimeric polynucleotides, and vectors for expressing them are preferable as the drug inhibiting the production or activity of the cell cycle-regulating protein (e.g., p21) or anti-apoptosis-related protein or PI3K signaling pathway-related protein that exhibits synthetic lethality when inhibited together with GST-π, because of high specificity and a low possibility of adverse reactions.

The inhibition of the homeostasis-related protein can be determined when the expression or activity of the protein in the cell is inhibited as compared with the case the agent inhibiting the protein is not allowed to act thereon. The expression of the protein can be evaluated without limitations by a known arbitrary approach, for example, an immunoprecipitation method using an antibody, EIA, ELISA, IRA, IRMA, Western blotting, an immunohistochemical method, an immunocytochemical method, flow cytometry, or various hybridization methods, Northern blotting, Southern blotting, or various PCR methods which employ nucleic acids specifically hybridizing to a nucleic acid encoding the protein or a unique fragment thereof, or a transcript (e.g., mRNA) or a splicing product of the nucleic acid.

Also, the activity of, for example, p21 can be evaluated without limitations by analyzing the known activity of p21, for example, binding activity against cyclin-CDK2 or a cyclin-CDK1 complex by a known arbitrary method, for example, an immunoprecipitation method, Western blotting, mass spectrometry, a pull-down method, or a surface plasmon resonance (SPR) method.

As used herein, the RNAi molecule refers to an arbitrary molecule that brings about RNA interference. The RNAi molecule is not limited and includes double-stranded RNAs such as siRNA (small interfering RNA), miRNA (micro RNA), shRNA (short hairpin RNA), ddRNA (DNA-directed RNA), piRNA (Piwi-interacting RNA), rasiRNA (repeat associated siRNA), and alternatives thereof, and the like. These RNAi molecules are commercially available or may be designed and prepared on the basis of publicly known sequence information, i.e., the nucleotide sequences and/or amino acid sequences shown in SEQ ID NOs: 1 to 30, 39 to 108.

As used herein, the antisense nucleic acid includes RNA, DNA, PNA, or complexes thereof.

As used herein, the DNA/RNA chimeric polynucleotide is not limited and includes, for example, a double-stranded polynucleotide described in JP Patent Publication (Kokai) No. 2003-219893 A (2003) which consists of DNA and RNA and inhibits the expression of a target gene.

The drug inhibiting GST-π and the drug inhibiting a homeostasis-related protein that exhibits synthetic lethality when inhibited together with GST-π may be contained in a single preparation or may be separately contained in two or more preparations. In the latter case, these preparations may be administered at the same time or may be administered in a staggered manner. In the case of administration in a staggered manner, the preparation containing the drug inhibiting GST-π may be administered before or after the administration of the preparation containing the drug inhibiting a homeostasis-related protein that exhibits synthetic lethality when inhibited together with GST-π.

The cell death-inducing agent and the cell growth-inhibiting agent according to the present invention may comprise one type of the aforementioned homeostasis-related protein, or two or more types the aforementioned homeostasis-related protein. For example, two or more types of cell cycle-regulating protein; two or more types of anti-apoptosis-related proteins; or one or more types of cell cycle-regulating proteins and one or more types of anti-apoptosis-related proteins may be used as homeostasis-related proteins included in the cell death-inducing agent and the cell growth-inhibiting agent according to the present invention.

Incidentally, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF are each a homeostasis-related protein that exhibits synthetic lethality for a cancer cell when inhibited together with GST-π. Accordingly, the drug inhibiting the protein serves as an active ingredient for an agent or a composition which potentiates the induction of cell death and/or the inhibition of cell growth by the drug inhibiting GST-π (hereinafter, also referred to as a "cell death induction-potentiating agent", a "cell growth inhibition-potentiating agent", a "composition for the potentiation of cell death induction", or a "composition for the potentiation of cell growth inhibition"). In other words, the induction of cell death and/or the inhibition of cell growth by the administration of the drug inhibiting Gust-π can be potentiated by administering an effective amount of the drug inhibiting the protein.

The content of the active ingredient in the agent or the composition of the present invention may be an amount that induces cell death such as apoptosis and/or inhibits cell growth when the agent or the composition is administered. Also, an amount that does not have adverse effect exceeding advantages brought about by administration is preferable. Such an amount is publicly known or can be appropriately determined by an *in vitro* test using cultured cells or the like or by a test in model animals such as mice, rats, dogs, or pigs. Such a testing method is well known to those skilled in the art. The induction of apoptosis can be evaluated by various known approaches, for example, the detection of apoptosis-specific phenomena such as DNA fragmentation, binding of annexin V to a cell membrane, change in mitochondrial membrane potential, and activation of caspase, and TUNEL staining. Also, the inhibition of cell growth can be evaluated by various known approaches, for example, the time-dependent measurement of the number of live cells, the measurement of the size, volume, or weight of tumor, the measurement of the amount of DNA synthesized, a WST-1 method, a BrdU (bromodeoxyuridine) method, and a 3H thymidine incorporation method. The content of the active ingredient can vary depending on the dosage form of the agent or the composition. For example, in the case of using a plurality of units of compositions in one administration, the amount of the active ingredient contained in one composition unit can be set to 1 / a plurality of amounts of the active ingredient necessary for one administration. Such adjustment of the content can be appropriately carried out by those skilled in the art.

Moreover, the drug inhibiting GST-π and the drug inhibiting a homeostasis-related protein that exhibits synthetic lethality when inhibited together with GST-π can be formulated as active ingredients to thereby produce a cell death-inducing agent, a cell growth-inhibiting agent, a composition for cell death induction, or a composition for cell growth inhibition.

Furthermore, a combination of the drug inhibiting GST-π and the drug inhibiting a homeostasis-related protein that exhibits synthetic lethality when inhibited together with GST-π for use in cell death induction or cell growth inhibition can be provided. In addition, a method for inducing cell death or a method for inhibiting cell growth, comprising administering effective amounts of the drug inhibiting GST-π and the drug inhibiting a homeostasis-related protein that exhibits synthetic lethality when inhibited together with GST-π can be provided.

All of the aforementioned methods for inducing cell death such as apoptosis or inhibiting cell growth may be *in vitro* methods or may be *in vivo* methods. The drugs for these methods are as already mentioned above, and the effective amount of each drug may be an amount that induces cell death such as apoptosis and/or inhibits cell growth in the recipient cell. Also, an amount that does not have adverse effect exceeding advantages brought about by administration is preferable. Such an amount is publicly known or can be appropriately determined by an *in vitro* test using cultured cells. Such a testing method is well known to those skilled in the art. The induction of cell death or the inhibition of cell growth can be evaluated by various known approaches including those mentioned above. When the drug is administered to a predetermined cancer cell population, the effective amount does not have to bring about cell death or growth inhibition for all cells in this cell population. The effective amount may be, for example, an amount that brings about apoptosis or growth inhibition for 1% or more, 2% or more, 3% or more, 4% or more, 5% or more, 6% or more, 8% or more, 10% or more, 12% or more, 15% or more, 20% or more, and 25% or more of the cells in the cell population.

The cell death-inducing agent or the cell growth-inhibiting agent of the present invention can effectively induce cell death or inhibit cell growth even for a cancer cell and as such, is effective as an ingredient for a pharmaceutical composition for a disease caused by abnormal cell growth. Also, the drug inhibiting GST-π and the drug inhibiting a homeostasis-related protein that exhibits synthetic lethality when inhibited together with GST-π can be formulated as active ingredients to thereby produce a pharmaceutical composition for a disease caused by abnormal cell growth. Furthermore, the treatment or therapy of a disease caused by abnormal cell growth, comprising administering an effective amount of the produced pharmaceutical composition to a subject in need thereof can be provided.

The pharmaceutical composition is effective for treating a disease caused by abnormal cell growth, particularly, for treating a disease having cell death or abnormal cell growth by expressing mutated KRAS.

The disease caused by a cell expressing mutated KRAS is not limited and includes, for example, benign or malignant tumors (also referred to as cancers or malignant neoplasms), hyperplasia, keloid, Cushing syndrome, primary aldosteronism, erythroplakia, polycythemia vera, leukoplakia, hyperplastic scar, lichen planus, and lentiginosis.

Examples of the cancer according to the present invention include cancers, cancers highly expressing GST-π, and cancers caused by cells expressing mutated KRAS (also simply referred to as KRAS cancers). In many cases, the KRAS cancers are included in the cancers highly expressing GST-π. Examples thereof include, but are not limited to: sarcomas such as fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, and osteosarcoma; carcinomas such as brain tumor, head and neck cancer, breast cancer, lung cancer, esophageal cancer, stomach cancer, duodenal cancer, appendix cancer colorectal cancer, rectal cancer, liver cancer, pancreatic cancer, gallbladder cancer, bile duct cancer, anus cancer, kidney cancer, urethral cancer, urinary bladder cancer, prostate cancer, penis cancer, testis cancer, uterine cancer, ovary cancer, vulval cancer, vaginal cancer, and skin cancer; and leukemia and malignant lymphoma. In the present invention, the "cancer" includes epithelial malignant tumors and non-epithelial malignant tumors. The cancer according to the present invention may be present in an arbitrary site of the body, for example, the brain, the head and neck region, the chest, the extremities, the lung, the heart, thymus glands, the esophagus, the stomach, the small intestine (duodenum, jejunum, and ileum), the large intestine (colon, cecum, appendix, and rectum), the liver, the pancreas, the gallbladder, the anus, the kidney, urinary ducts, the urinary bladder, the prostate, the penis, the testis, the uterus, the ovary, the vulva, the vagina, the skin, striated muscles, smooth muscles, synovial membranes, cartilage, bone, thyroid glands, adrenal glands, the peritoneum, the mesenterium, bone marrow, blood, the vascular system, the lymphatic system such as lymph nodes, or lymph.

For the pharmaceutical composition, the drug inhibiting GST-π and the drug inhibiting a homeostasis-related protein that exhibits synthetic lethality when inhibited together with GST-π may be used in combination with an additional active ingredient. In this context, the use in combination includes, for example, the administration of the additional active ingredient as another preparation, and the administration of the additional active ingredient as a combination drug with at least one of the other drugs. In the case of administration as another preparation, the preparation containing the additional active ingredient may be administered before, at the same time with, or after the administration of the other preparation(s).

Examples of such an additional active ingredient include ones effective for the treatment of the disease of interest. For example, when the disease to be treated is a cancer, an anticancer agent can be used in combination therewith. Examples of the anticancer agent can include: alkylating agents such as ifosfamide, nimustine hydrochloride, cyclophosphamide, dacarbazine, melphalan, and ranimustine; metabolic antagonists such as gemcitabine hydrochloride, enocitabine, cytarabine ocfosfate, cytarabine preparations, tegafur uracil, tegafur-gimeracil-oteracil potassium combination drugs (e.g., TS-1), doxifluridine, hydroxycarbamide, fluorouracil, methotrexate, and mercaptopurine; antitumor antibiotics such as idarubicin hydrochloride, epirubicin hydrochloride, daunorubicin hydrochloride, daunorubicin citrate, doxorubicin hydrochloride, pirarubicin hydrochloride, bleomycin hydrochloride, peplomycin sulfate, mitoxantrone hydrochloride, and mitomycin C; alkaloids such as etoposide, irinotecan hydrochloride, vinorelbine tartrate, docetaxel hydrate, paclitaxel, vincristine sulfate, vindesine sulfate, and vinblastine sulfate; hormone therapy agents such as anastrozole, tamoxifen citrate, toremifene citrate, bicalutamide, flutamide, and estramustine phosphate; platinum complexes such as carboplatin, cisplatin (CDDP), and nedaplatin; anti-angiogenic agents such as thalidomide, Neovastat, and bevacizumab; and L-asparaginase.

When the active ingredients in various agents or compositions, the method of treating a subject, etc., of the present invention described herein are nucleic acids, for example, RNAi molecules, ribozymes, antisense nucleic acids, or DNA/RNA chimeric polynucleotides, they may be used directly as naked nucleic acids or may be supported by various vectors. Publicly known arbitrary vectors such as plasmid vectors, phage vectors, phagemid vectors, cosmid vectors, or virus vectors can be used as the vectors. It is preferable that the vectors should contain at least a promoter that potentiates the expression of each nucleic acid to be carried. In this case, it is preferable that the nucleic acid should be operably linked to such a promoter. The nucleic acid operably linked to the promoter means that the nucleic acid and the promoter are located such that the protein encoded by the nucleic acid is properly produced by the action of the promoter. The vectors may or may not be replicable in host cells. Also, the transcription of the gene may be performed outside the nuclei of the host cells or may be performed inside the nuclei thereof. In the latter case, the nucleic acid may be integrated into the genomes of the host cells.

Alternatively, the active ingredients may be supported by various non-viral lipid or protein carriers. Examples of such carriers include, but are not limited to, cholesterols, liposomes, antibody protomers, cyclodextrin nanoparticles, fusion peptides, aptamers, biodegradable polylactic acid copolymers, and polymers, which can enhance the efficiency of cellular uptake (see e.g., Pirollo and Chang, Cancer Res. 2008; 68 (5): 1247-50). Particularly, cationic liposomes or polymers (e.g., polyethyleneimine) are useful. Further examples of the polymers useful as such carriers include those described in, for example, US 2008/0207553 and US 2008/0312174.

In various pharmaceutical compositions of the present invention described herein, the active ingredients may be combined with an additional arbitrary ingredient unless the effects of the active ingredients are impaired. Examples of such an arbitrary ingredient include other chemotherapeutic agents, pharmacologically acceptable carriers, excipients, and diluents. Furthermore, the compositions may be coated with suitable materials, for example, enteric coatings or time-controlled disintegrating materials, according to administration routes, drug release manners, etc., and may be incorporated into suitable drug release systems.

Various agents and compositions (including various pharmaceutical compositions) of the present invention described herein may be administered without limitations through various routes including both oral and parenteral routes, for example, oral, intravenous, intramuscular, subcutaneous, local, intratumoral, rectal, intraarterial, intraportal, intraventricular, transmucosal, percutaneous, nasal, intraperitoneal, intrapulmonary, and intrauterine routes, or may be prepared into dosage forms suitable for each administration route. Arbitrary publicly known ones can be appropriately adopted for such dosage forms and preparation methods (see e.g., Hyoujun Yakuzai Gaku (Standard Pharmaceutics in English), edited by Yoshiteru Watanabe et al., Nankodo Co., Ltd., 2003).

Examples of the dosage form suitable for oral administration include, but are not limited to, powders, granules, tablets, capsules, solutions, suspensions, emulsions, gels, and syrups. Also, examples of the dosage form suitable for parenteral administration include injections such as injections in a solution state, injections in a suspension state, injections in an emulsion state, and injections of type to be prepared before use. The preparations for parenteral administration can be in the form of an aqueous or nonaqueous isotonic sterile solution or suspension.

Various agents or compositions (including various pharmaceutical compositions) of the present invention described herein may be prepared to target particular tissues or cells. The targeting can be achieved by a known arbitrary approach. In the case of intending delivery to a cancer, for example, an approach such as passive targeting by setting the size of a preparation to a diameter of 50 to 200 µm, particularly, 75 to 150 µm, suitable for the exertion of EPR (enhanced permeability and retention) effects, or active targeting using a ligand such as CD 19, HER2, transferrin receptor, folate receptor, VIP receptor, EGFR (Torchilin, AAPS J. 2007; 9 (2): E128-47), RAAG10 (JP Patent Publication (Kohyo) No. 2005-532050 A (2005)), PIPA (JP Patent Publication (Kohyo) No. 2006-506071 A (2006)), or KID3 (JP Patent Publication (Kohyo) No. 2007-529197 A (2007)), a peptide having an RGD motif or an NGR motif, F3, LyP-1 (Ruoslahti et al., J Cell Biol. 2010; 188 (6): 759-68), or the like as a targeting agent can be used without limitations. Since it is also known that retinoid or a derivative thereof is useful as a targeting agent for a cancer cell (WO 2008/120815), a carrier comprising retinoid as a targeting agent may be used. Such a carrier is described in WO 2009/036368, WO 2010/014117, WO 2012/170952, etc., in addition to the above literature.

Various agents or compositions (including various pharmaceutical compositions) of the present invention described herein can be supplied in any form and may be provided in a form that can be prepared before use, for example, a form that can be prepared by a physician and/or a pharmacist, a nurse, or other paramedical persons in a medical setting or in the neighborhood thereof, from the viewpoint of preservation stability. Such a form is particularly useful when the agent or the composition of the present invention comprises ingredients difficult to stably preserve, such as lipids, proteins, or nucleic acids. In this case, the agent or the composition of the present invention is provided as one or two or more containers comprising at least one of the components essential therefor, and is prepared before use, for example, within 24 hours before use, preferably within 3 hours before use, more preferably immediately before use. For the preparation, a reagent, a solvent, a prescription instrument, or the like usually available in the preparation location can be appropriately used.

Thus, the present invention also relates to a composition preparation kit comprising one or two or more containers comprising one or a combination of the active ingredients that may be contained in various agents or compositions of the present invention, and necessary components for various agents or compositions which are provided in the form of such a kit. The kit of the present invention may additionally comprise an instruction, for example, a manual or an electronic recording medium (CD or DVD), describing preparation methods, a method of treating a subject, etc., for various agents or compositions of the present invention. Also, the kit of the present invention may comprise all of the components for completing various agents or compositions of the present invention, but is not necessarily required to comprise all of the components. Thus, the kit of the present invention may not comprise a reagent or a solvent usually available in a medical setting, an experimental facility, or the like, for example, sterile water, saline, or a glucose solution.

The effective amount for various methods of treating a subject of the present invention described herein is, for example, an amount that reduces the symptoms of a disease or delays or terminates the progression of the disease, and is preferably an amount that inhibits or cures the disease. Also, an amount that does not have adverse effect exceeding advantages brought about by administration is preferable. Such an amount can be appropriately determined by an *in vitro* test using cultured cells or the like or by a test in model animals such as mice, rats, dogs, or pigs. Such a testing method is well known to those skilled in the art. Furthermore, the doses of the drugs used in the treatment method of the present invention are generally known to those skilled in the art or can be appropriately determined by the aforementioned tests or the like.

The specific doses of the active ingredients to be administered in the method of treating a subject of the present invention described herein can be determined in consideration of various conditions about a subject in need of treatment, for example, the severity of the symptoms, the general health state of the subject, the age, the body weight, the sex of the subject, diet, the time and frequency of administration, a drug used in combination, response to the therapy, dosage form, and compliance to the therapy.

The administration route includes various routes including both oral and parenteral routes, for example, oral, intravenous, intramuscular, subcutaneous, local, intratumoral, rectal, intraarterial, intraportal, intraventricular, transmucosal, percutaneous, nasal, intraperitoneal, intrapulmonary, and intrauterine routes.

The frequency of administration differs depending on the properties of the agent or the composition used, and conditions about the subject including those described above, and may be, for example, multiple times per day (i.e., 2, 3, 4, or 5 times a day), once a day, every few days (i.e., every 2, 3, 4, 5, 6, or 7 days), once a week, or every few weeks (i.e., every 2, 3, or 4 weeks).

As used herein, the term "subject" means an arbitrary organism individual and is preferably an animal, more preferably a mammal, further preferably a human individual. In the present invention, the subject may be healthy or may have some disease. In the case of intending the treatment of a particular disease, the subject typically means a subject having this disease or having the risk of being affected by this disease.

As used herein, the term "treatment" includes every type of medically acceptable prophylactic and/or therapeutic intervention aimed at, for example, curing, temporarily ameliorating, or preventing a disease. For example, the term "treatment" encompasses various medically acceptable interventions of interest, including the delay or termination of the progression of a disease, the involution or disappearance of a lesion, the prevention of onset or the prevention of recurrence, etc.

Incidentally, as mentioned above, ATM, CDC25A, p21, PRKDC, RBBP8, SKP2, MCM10, RNPC1, CCNL1, CENPH, BRSK1, MYLK, MCM8, CCNB3, MCMDC1, AATF, AKT1, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, MYO18A, MTOR, IRAK1, IRS1, MYD88, NFKB1, PIK3CG, RAC1, AKT3, EIF4B, EIF4E, ILK, MTCP1, PIK3CA and SRF are each a protein that exhibits synthetic lethality for a cancer cell when inhibited together with GST-π. Thus, a cell death-inducing agent and/or a cell growth-inhibiting agent for a cancer cell that is used together with a drug inhibiting GST-π can be screened for by using the inhibition of this homeostasis-related protein as an index. Specifically, a substance that can inhibit the homeostasis-related protein serves as a candidate substance for the cell death-inducing agent and/or the cell growth-inhibiting agent for a cancer cell that is used together with a drug inhibiting GST-π.

For example, a test substance is contacted with a cell expressing mutated KRAS as one example of the cancer cell, and the expression level of the homeostasis-related protein that exhibits synthetic lethality for the cell expressing mutated KRAS when inhibited together with GST-π is measured in the cell. The test substance can be selected as a candidate substance for a drug inhibiting the homeostasis-related protein, when the expression level measured after the contact of the test substance is decreased compared with the expression level measured in the absence of the test substance.

On the other hand, the drug inhibiting GST-π is a protein that exhibits synthetic lethality for a cancer cell when inhibited together with the drug inhibiting the homeostasis-related protein that exhibits synthetic lethality for a cancer cell when inhibited together with GST-π. Thus, a cell death-inducing agent and/or a cell growth-inhibiting agent for a cancer cell that is used together with a drug inhibiting the homeostasis-related protein can be screened for by using the inhibition of GST -π as an index. Specifically, a substance that can inhibit GST-π serves as a candidate substance for the cell death-inducing agent and/or the cell growth-inhibiting agent for a cancer cell that is used together with a drug inhibiting the homeostasis-related protein.

For example, a test substance is contacted with a cell expressing mutated KRAS as one example of the cancer cell, and the expression level of GST-π is measured in the cell. The test substance can be selected as a candidate substance for a drug inhibiting GST-π, when the expression level measured after the contact of the test substance is decreased compared with the expression level measured in the absence of the test substance.

Likewise, a cell death-inducing agent and/or a cell growth-inhibiting agent for a cancer cell can be screened for by using both of the inhibition of GST-π and the inhibition of the homeostasis-related protein that exhibits synthetic lethality for a cancer cell when inhibited together with GST-π as indexes. Specifically, a substance that can inhibit GST-π and can inhibit the homeostasis-related protein serves as a candidate substance for the cell death-inducing agent and/or the cell growth-inhibiting agent for a cancer cell.

For example, a test substance is contacted with a cell expressing mutated KRAS as one example of the cancer cell, and the expression level of GST -π and the expression level of the homeostasis-related protein are measured in the cell. The test substance can be selected as a candidate substance for a drug inhibiting GST-π and inhibiting the homeostasis-related protein that exhibits synthetic lethality for a cancer cell when inhibited together with GST-π, when these expression levels measured after the contact of the test substance are both decreased compared with the respective expression levels measured in the absence of the test substance.

In this context, the test substance is not limited by any means and can be any substance. The test substance may be a single substance or may be a mixture consisting of a plurality of components. The test substance may be configured to comprise an unidentified substance as in, for example, an extract from a microorganism or a culture solution, or may be configured to comprise known compositions at predetermined compositional ratios. Also, the test substance may be any of proteins, nucleic acids, lipids, polysaccharides, organic compounds, and inorganic compounds.

### EXAMPLES

Hereinafter, the present invention will be described further specifically with reference to Examples. However, the technical scope of the present invention is not intended to be limited by Examples below.

### [Experiment 1] Knockdown of GST-π and P21 by siRNAs

As examples of cancer cells, 1 × 10⁵ M7609 cells (human colorectal cancer cells having KRAS mutation) and PANC-1 cells (human pancreatic cancer cells having KRAS mutation) were inoculated to 6 cm Petri dishes and cultured for 18 hours in Roswell Park Memorial Institute 1640 (RPMI 1640, Sigma-Aldrich Corp.) supplemented with 10% fetal bovine serum (FBS) and 0.5% L-glutamine. The culture conditions were 37°C and 5% CO₂, unless otherwise specified. Moreover, as an example of cancer cells, 0.5 × 10⁵ A549 cells (human lung cancer cells having KRAS mutation) were inoculated to a 6 cm Petri dish and cultured for 18 hours in a Dulbecco's modified Eagle's medium (DMEM, Sigma-Aldrich Corp.) supplemented with 10% FBS and 1% L-glutamine. Furthermore, as an example of cancer cells, 1 × 10⁵ MIA PaCa-2 cells (human pancreatic cancer cell having KRAS mutation) were inoculated to a 6 cm Petri dish and cultured for 18 hours in DMEM supplemented with 10% FBS and 1% L-glutamine. Furthermore, as an example of cancer cells, 0.5 × 10⁵ HCT116 cells (human colorectal cancer cells having KRAS mutation) were inoculated to a 6 cm Petri dish and cultured for 18 hours in McCoy's 5A Medium (McCoy, Sigma-Aldrich Corp.) supplemented with 10% FBS and 0.5% L-glutamine.

In this experiment, first, the PANC-1, A549, or MIA PaCa-2 cells that became 20 to 30% confluent were transfected with GST-π siRNA and/or P21 siRNA using Lipofectamine RNAi MAX (Life Technologies Corp.) as follows.

The Lipofectamine/siRNA mixed solution for transfection was prepared as follows: first, a Lipofectamine solution in which 15 µL of Lipofectamine RNAi MAX and 485 µL of OPTI-MEM (Sigma-Aldrich Corp.) were mixed was prepared. Next, an siRNA solution in which a predetermined amount of 50 µM siRNA was adjusted to 500 µL with OPTI-MEM was prepared (e.g., in the case of preparing an siRNA solution used with a final concentration of 50 nM, 6 µL of 50 µM siRNA and 494 µL of OPTI-MEM were mixed), and this was mixed with the aforementioned Lipofectamine solution and left standing at room temperature for 15 minutes. siRNAs given below were used. In the description below, the upper-case letters represent RNAs, and the lower-case letters represent DNAs.

### GST-π siRNA:

Sense strand: GGGAGGCAAGACCUUCAUUtt (SEQ ID NO: 31)
Antisense strand: AAUGAAGGUCUUGCCUCCCtg (SEQ ID NO: 32)

### P21 siRNA:

Sense strand: UCCUAAGAGUGCUGGGCAUtt (SEQ ID NO: 33)
Antisense strand: AUGCCCAGCACUCUUAGGAtt (SEQ ID NO: 34)

### Control siRNA:

Sense strand: ACGUGACACGUUCGGAGAAtt (SEQ ID NO: 35)
Antisense strand: UUCUCCGAACGUGUCACGUtt (SEQ ID NO: 36)

### GST-π siRNA-2:

Sense strand: UCUCCCUCAUCUACACCAAtt (SEQ ID NO: 37)
Antisense strand: UUGGUGUAGAUGAGGGAGAtg (SEQ ID NO: 38)

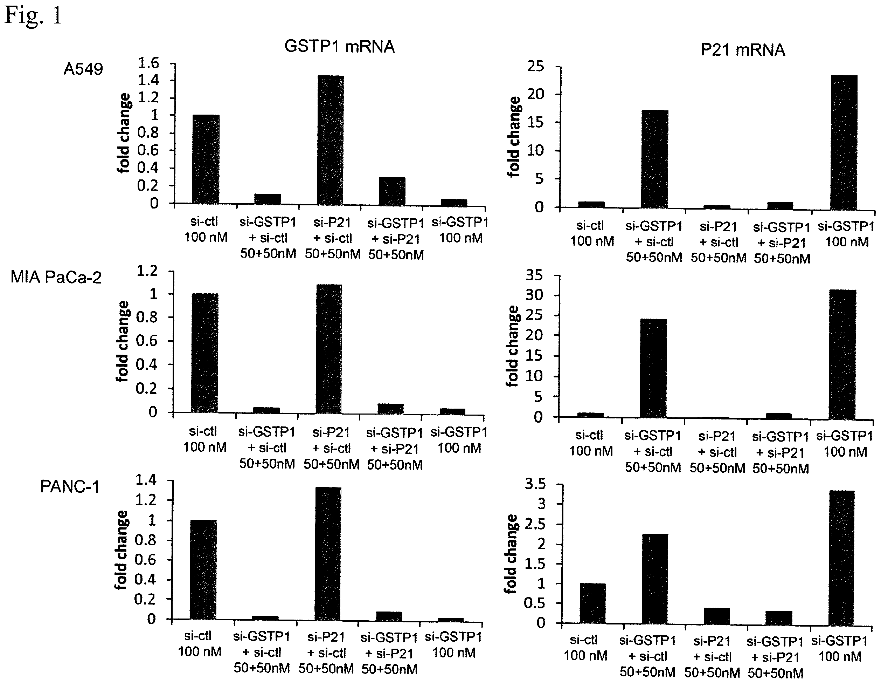

GST-π siRNA and P21 siRNA each at a final concentration of 50 nM, GST-π siRNA or P21 siRNA at a final concentration of 50 nM (both added with control siRNA at a final concentration of 50 nM), or GST-π siRNA at a final concentration of 100 nM (without the addition of control siRNA) were added to each of the Petri dishes containing the PANC-1, MIA PaCa-2, or A549 cells. For a control used, control siRNA was added at a final concentration of 100 nM. After culture for 1 day without the replacement of the medium, the GST-π mRNA level and the P21 mRNA level were quantified by quantitative PCR using 7300 Real Time PCR System (Applied Biosystems, Inc.). The results are shown in Figure 1. As shown in Figure 1, it was revealed that the amount of P21 mRNA is increased by knocking down GST-π by the siRNA.

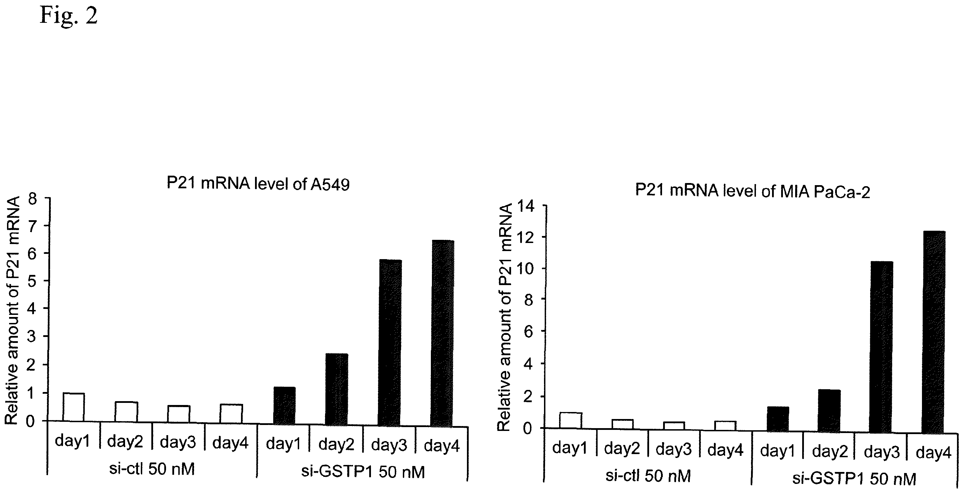

Moreover, as for the case where GST-π siRNA or control siRNA at a final concentration of 50 nM was added to each of the Petri dishes containing the A549 cells or the MIA PaCa-2 cells, the P21 mRNA level was similarly quantified every day from the day of addition of GST-π siRNA or control siRNA to the 4th day. The results are shown in Figure 2. As shown in Figure 2, it was revealed that the expression level of P21 mRNA is increased over time by knocking down GST-π by the siRNA.

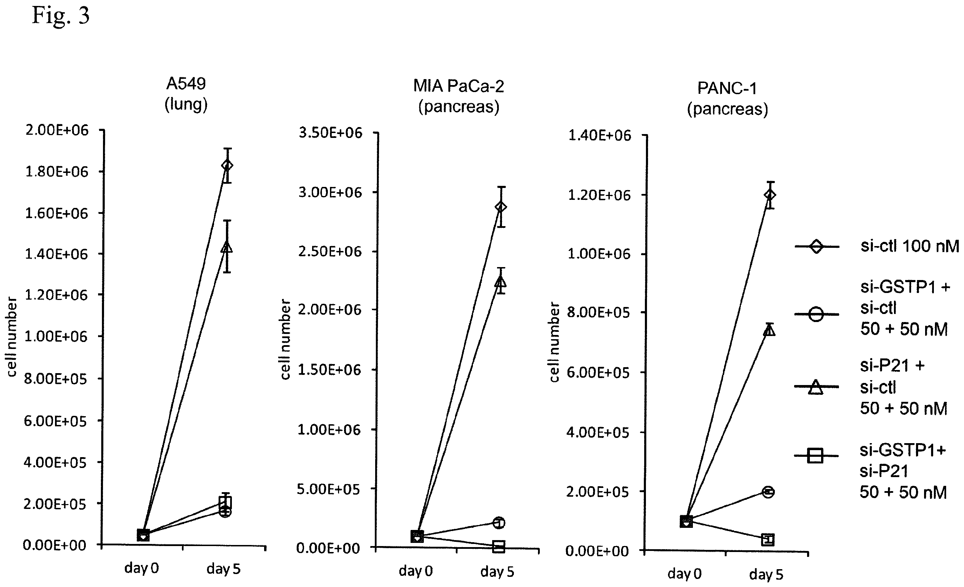

Meanwhile, the influence of GST-π siRNA and/or P21 siRNA on the number of cells was tested. First, GST-π siRNA and P21 siRNA each at a final concentration of 50 nM, or GST-π siRNA or P21 siRNA at a final concentration of 50 nM (both added with control siRNA at a final concentration of 50 nM) were added to each of the Petri dishes containing the PANC-1, MIA PaCa-2, or A549 cells. For a control used, control siRNA was added at a final concentration of 100 nM. After culture for 5 days without the replacement of the medium, the cells were dissociated and collected from the Petri dish by trypsin treatment, and the number of cells was counted. The results are shown in Figure 3. As shown in Figure 3, it is evident that when GST-π and P21 are knocked down each alone using GST-π siRNA or P21 siRNA, the number of cells cannot be decreased with respect to the number of inoculated cells, though cell growth is inhibited. However, it is evident that when GST-π and P21 are both knocked down using Gust-π siRNA and P21 siRNA, not only is growth inhibited but cell death can be induced in the PANC-1 cells and the MIA PaCa-2 cells expressing mutated KRAS.

From Figure 3, it was considered, as to the A549 cells expressing mutated KRAS, that cell death was not induced by the treatment mentioned above. Accordingly, the number of transfections with GST-π siRNA and P21 siRNA was increased, and the influence of GST-π siRNA and/or P21 siRNA on the number of cells was tested as to the A549 cells and the HCT116 cells expressing mutated KRAS.

First, for the PANC-1 cells, the MIA PaCa-2 cells, or the HCT116 cells, GST-π siRNA and P21 siRNA each at a final concentration of 25 nM, or GST-π siRNA or P21 siRNA at a final concentration of 25 nM (all added with control siRNA at a final concentration of 25 nM) were added to each of the Petri dishes, while for the A549 cells, GST-π siRNA and P21 siRNA each at a final concentration of 50 nM, or GST-π siRNA or P21 siRNA at a final concentration of 50 nM (added with control siRNA at a final concentration of 50 nM) were added to each of the Petri dishes. For a control, control siRNA was added at a final concentration of 50 nM for the PANC-1 cells, the MIA PaCa-2 cells, or the HCT116 cells and at a final concentration of 100 nM for the A549 cells. After 2 days and after 4 days, the medium was replaced (RPMI 1640 supplemented with 10% FBS for the PANC-1 cells, DMEM supplemented with 10% FBS for the A549 cells and the MIA PaCa-2 cells, and McCoy supplemented with 10% FBS for the HCT116 cells). Again, GST-π siRNA or P21 siRNA was added at a final concentration of 25 nM (all added with control siRNA at a final concentration of 25 nM) for the PANC-1 cells, the MIA PaCa-2 cells, or the HCT116 cells, and at a final concentration of 50 nM (added with control siRNA at a final concentration of 50 nM) for the A549 cell to each of the Petri dishes. In this case as well, for a control, control siRNA was added at a final concentration of 50 nM for the PANC-1 cells or the MIA PaCa-2 cells and at a final concentration of 100 nM for the A549 cells. Then, the cells were cultured without the replacement of the medium. Seven days after the cell inoculation, the cells were dissociated and collected from the Petri dish by trypsin treatment, and the number of cells was counted. In this case, the phase difference images of the cells were also taken.

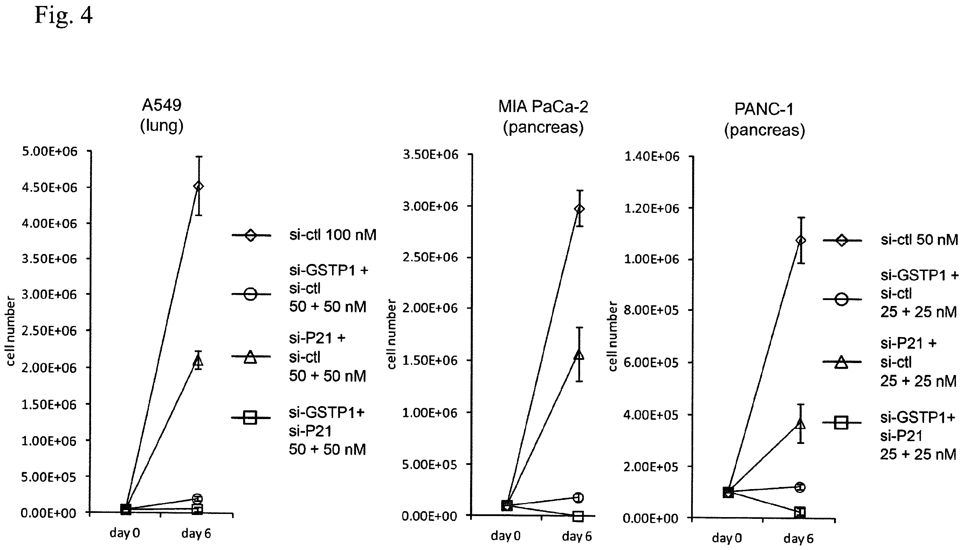

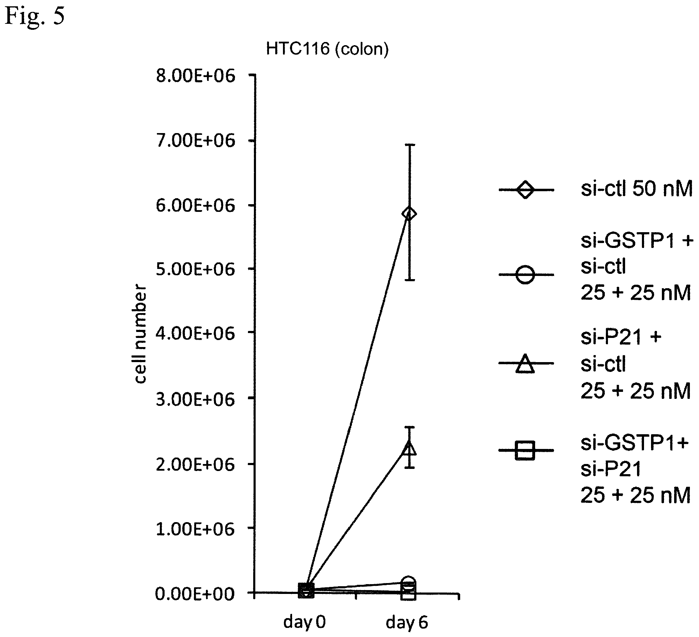

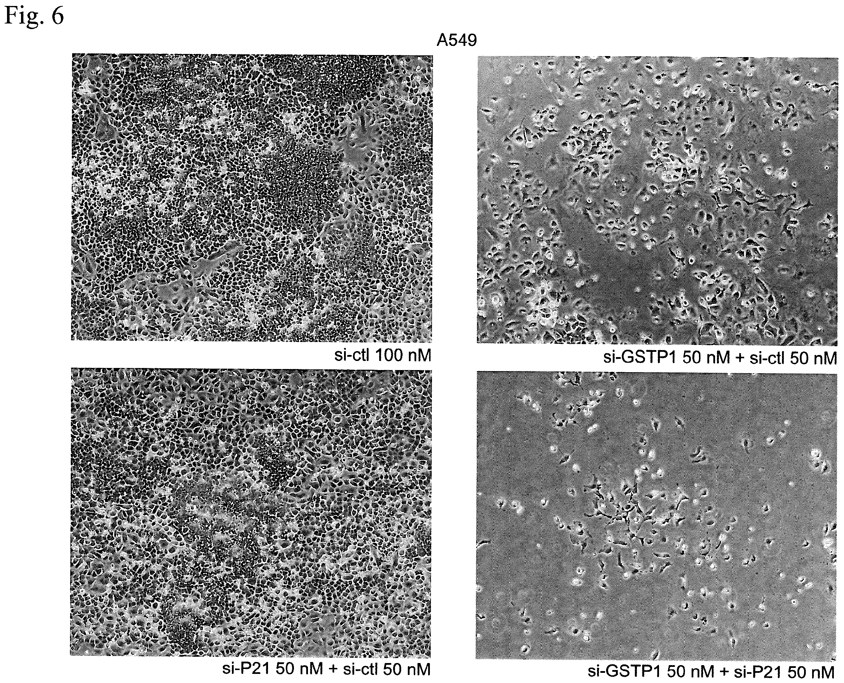

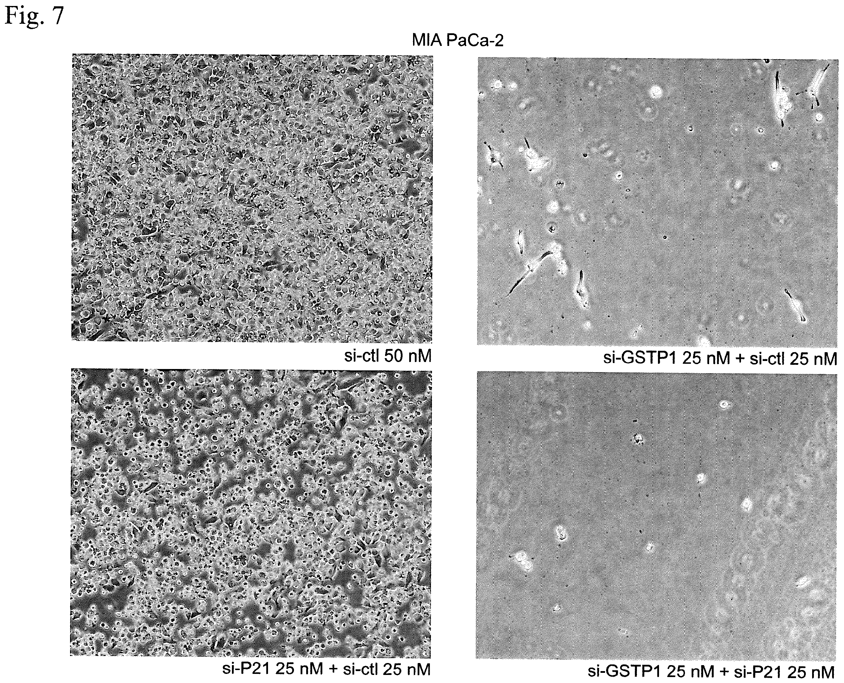

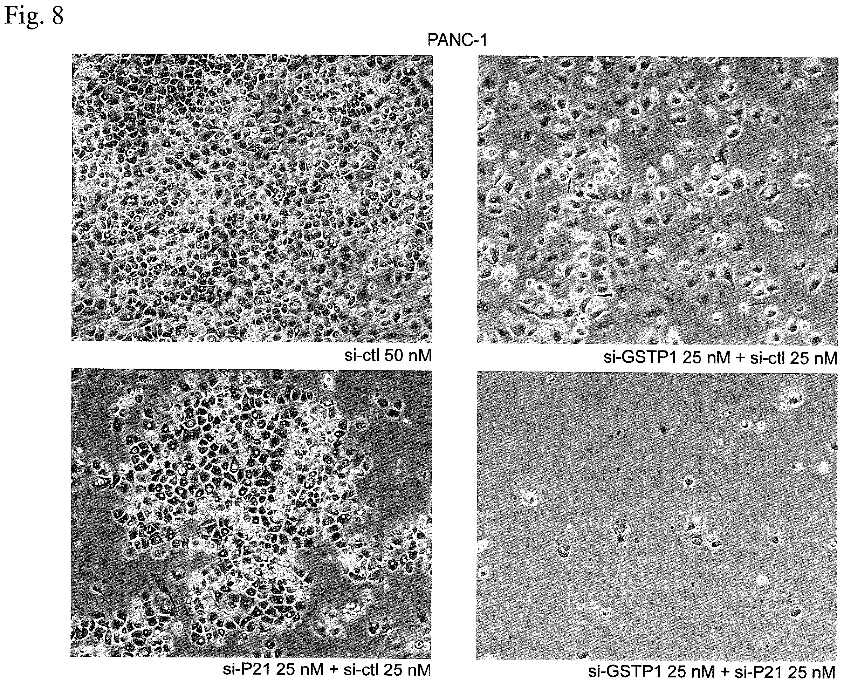

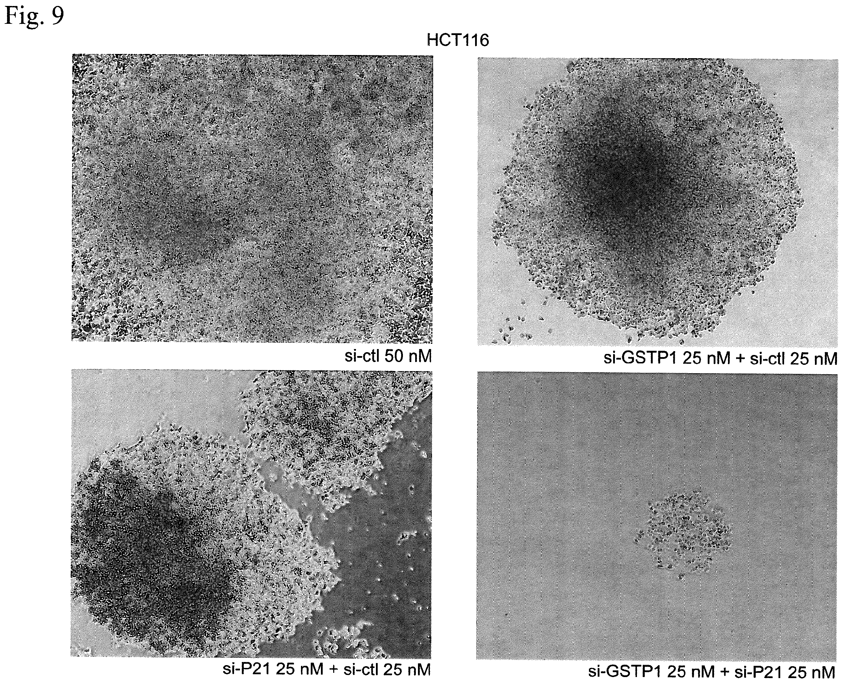

The results of measuring the number of cells for the A549 cells, the PANC-1 cells, and the MIA PaCa-2 cells are shown in Figure 4. The results of measuring the number of cells for the HCT116 cells are shown in Figure 5. Also, the phase difference image taken for the A549 cells is shown in Figure 6. The phase difference image taken for the MIA PaCa-2 cells is shown in Figure 7. The phase difference image taken for the PANC-1 cells is shown in Figure 8. The phase difference image taken for the HCT116 cells is shown in Figure 9.

As shown in Figures 4 and 5, when GST-π and P21 were both knocked down three times using GST-π siRNA and P21 siRNA, the number of the cancer cells (A549 cells, MIA PaCa-2 cells, PANC-1 cells, and HCT116 cells) expressing mutated KRAS is decreased 7 days after the cell inoculation with respect to the number of initially inoculated cells; thus it was revealed that cell death can be induced.

Moreover, as shown in Figures 6 to 9, the cells of each cell line (A549 cells, MIA PaCa-2 cells, PANC-1 cells, and HCT116 cells) expressing mutated KRAS in which GST-π was knocked down by GST-π siRNA became flat and large cells; thus it was able to be presumed that cell senescence was evoked. It was further revealed that when GST-π and P21 were both knocked down using GST-π siRNA and P21 siRNA, the cell senescence-like phenotype observed in the GST-π knockdown disappeared. From this result, it was considered that when GST-π and P21 were both knocked down using GST-π siRNA and P21 siRNA, the cell senescence evoked by the GST-π knockdown was inhibited by the P21 knockdown.

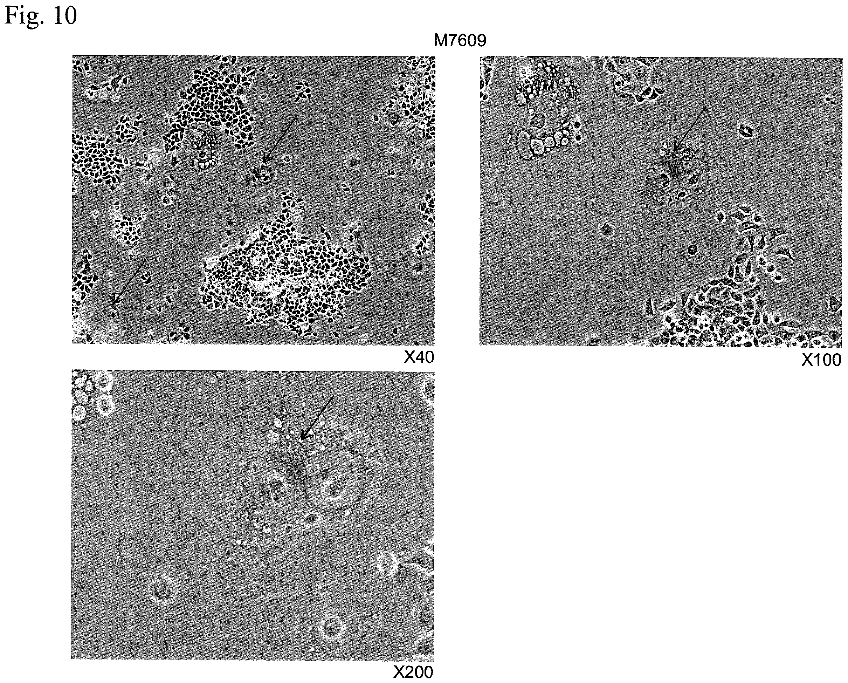

Whether the cell senescence as shown in Figures 6 to 9 was could be induced by knocking down GST-π was tested using the M7609 cells. First, GST-π siRNA was added at a final concentration of 30 nM to the Petri dish containing the M7609 cells. After 1 day and after 2 days, the medium was replaced (RPMI 1640 supplemented with 10% FBS). Again, Gust-π siRNA was added at a final concentration of 30 nM to the Petri dish containing the M7609 cells. Then, the cells were cultured with the medium replaced every other day. Thirteen days after the cell inoculation, the cells were stained using Senescence β-Galactosidase Staining Kit (Cell Signaling Technology, Inc.) according to the recommended protocol. A phase difference image was taken. The results are shown in Figure 10. As shown in Figure 10, the M7609 cells in which GST-π was knocked down became flat and large cells, and blue color development by β-galactosidase was observed in the cells having such a phenotype; thus it is evident that cell senescence was evoked.

In addition, whether the cell death induced by knocking down both GST-π and P21 in the cancer cells expressing mutated KRAS was apoptosis was tested by measuring the expression level of an apoptosis-inducing factor PUMA gene.

First, GST-π siRNA and P21 siRNA each at a final concentration of 50 nM, or GST-π siRNA or P21 siRNA at a final concentration of 50 nM (both added with control siRNA at a final concentration of 50 nM) were added to each of the Petri dishes containing the A549 cells or the MIA PaCa-2 cells. For a control, control siRNA was added at a final concentration of 100 nM. After culture for 1 day without the replacement of the medium, the PUMA mRNA level was quantified by quantitative PCR using 7300 Real Time PCR System (Applied Biosystems, Inc.).

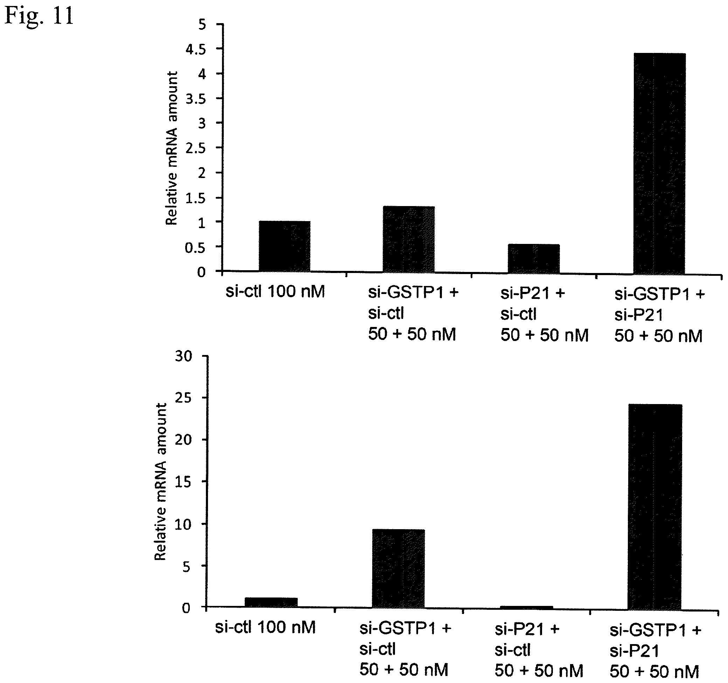

The results are shown in Figure 11. As shown in Figure 11, it was revealed that the mRNA level of the apoptosis-promoting factor PUMA is drastically increased by knocking down both GST-π and p21 using GST-π siRNA and P21 siRNA. From this result, it was revealed that the cell death induced by knocking down both GST-π and P21 is apoptosis.

Apoptosis-related protein groups are present in cells. The apoptosis-related proteins are broadly classified into two groups: an apoptosis-inhibiting protein group and an apoptosis-inducing protein group. The apoptosis-inhibiting protein group includes Bcl-2, Bcl-XL, Bcl-W, MCL-1, and Bcl-B. Also, the apoptosis-inducing protein group includes Bax, Bak, BOK, BIM, BID, BAD, NOXA, and PUMA. In general, the apoptosis-inhibiting proteins such as Bcl-2, Bcl-XL, and MCL-1 reside on mitochondrial outer membranes and inhibit the release of cytochrome C to inhibit apoptosis. On the other hand, the apoptosis-inducing protein groups such as Bax, BIM, BID, and BAD reside in cytoplasms, but translocate to mitochondrial outer membranes in response to death signals and promote the release of cytochrome C to induce apoptosis.

Also, upon activation due to DNA damage or the like, p53 promotes the transcription of Bax, NOXA, and PUMA to induce apoptosis. Particularly, PUMA is a protein that has been isolated as an apoptosis-inducing protein to be activated by p53. PUMA binds directly to Bcl-2, thereby inhibiting the apoptosis-inhibiting effect of Bcl-2 and inducing the apoptosis of the cell.

From these results of Experiment 1, it was shown that when a drug inhibiting GST-π and a drug inhibiting P21 are allowed to act on a cancer cell, cell growth can be drastically inhibited and further cell death can be strongly induced. Even if the drug inhibiting GST-π is allowed to act alone on a cancer cell, cell death cannot be induced, though cell growth can be inhibited. Even if the drug inhibiting P21 is allowed to act alone on the cell, cell growth can be inhibited merely slightly. Accordingly, it is a surprising effect that the cell death can be induced for a cancer cell by allowing both of these drugs to act thereon.

### [Experiment 2]

In Experiment 1, the synthetic lethality for cancer cells was demonstrated using GST-π siRNA and P21 siRNA. In this Experiment 2, a cell cycle-regulating protein that exhibited synthetic lethality by inhibition together with GST-π was screened for.

First, a MIA PaCa-2 cell suspension having a concentration of 1 × 10⁴ cells/mL was prepared with DMEM supplemented with 10% FBS and 1% L-glutamine, and this was inoculated at 100 µL/well to a 96-well plate and then cultured for 18 hours in DMEM supplemented with 10% FBS and 1% L-glutamine. The MIA PaCa-2 cells that became 20 to 30% confluent were transfected with Gust-π siRNA-2 and/or siRNA against a target gene using Lipofectamine RNAi MAX as follows.

The Lipofectamine/siRNA mixed solution for transfection was prepared as follows: first, 51 µL of DNase free water (Ambion, Life Technologies Corp.) was added to 0.1 nmol of each siRNA contained in Human siGENOME siRNA Library - Cell Cycle Regulation - SMART pool (GE Healthcare Dharmacon Inc.) and left standing at room temperature for 90 minutes. An siRNA solution in which this aqueous siRNA solution was supplemented with 19.9 µL of OPTI-MEM was prepared (solution A). Next, a 50 µM aqueous GST-π siRNA-2 solution and a 50 µM aqueous control siRNA solution were each diluted with OPTI-MEM in ten times to prepare diluted solutions of 5 µM GST-π siRNA-2 and 5 µM control siRNA (solution B). 31.2 µL of the solution A and 8.8 µL of the solution B were mixed (solution C). Next, a Lipofectamine solution in which 150 µL of Lipofectamine RNAi MAX and 2.35 mL of OPTI-MEM were mixed was prepared (solution D). Next, 37.5 µL of the solution C and 37.5 µL of the solution D were mixed and left standing at room temperature for 15 minutes (solution E).

The solution E was added to each well culturing MIA-PaCa-2 cell of the 96-well plate at 10 µL/well. Separately, a 50 µM aqueous control siRNA solution (5.5 µL) and OPTI-MEM (189.5 µL) were mixed to prepare a solution (solution F). Next, a 50 µM aqueous control siRNA solution was diluted with OPTI-MEM in ten times to prepare 5 µM control siRNA (solution G). 31.2 µL of the solution F and 8.8 µL of the solution G were mixed (solution H). Next, a Lipofectamine solution in which 150 µL of Lipofectamine RNAi MAX and 2.35 mL of OPTI-MEM were mixed was prepared (solution I). Next, 37.5 µL of the solution H and 37.5 µL of the solution I were mixed and left standing at room temperature for 15 minutes (solution J). The solution J was added to each well culturing MIA-PaCa-2 cell of the 96-well plate at 10 µL/well. Then, the cells were cultured in DMEM supplemented with 10% FBS and 10% L-glutamine. After 5 days, a growth evaluation test was conducted using CyQUANT NF Cell Proliferation Assay Kit (Invitrogen Corp.).

First, 1 × HBSS buffer was added to 22 µL of CyQUANT NF dye reagent to prepare a staining reaction solution for CyQUANT NF Cell proliferation Assay. The medium of the transfected cells mentioned above was aspirated, and 50 µL of the staining reaction solution was added thereto. The cells were left standing at 37°C for 30 minutes. Then, a fluorescence wavelength of 520 nm was observed in excitation with an excitation wavelength of 480 nm.

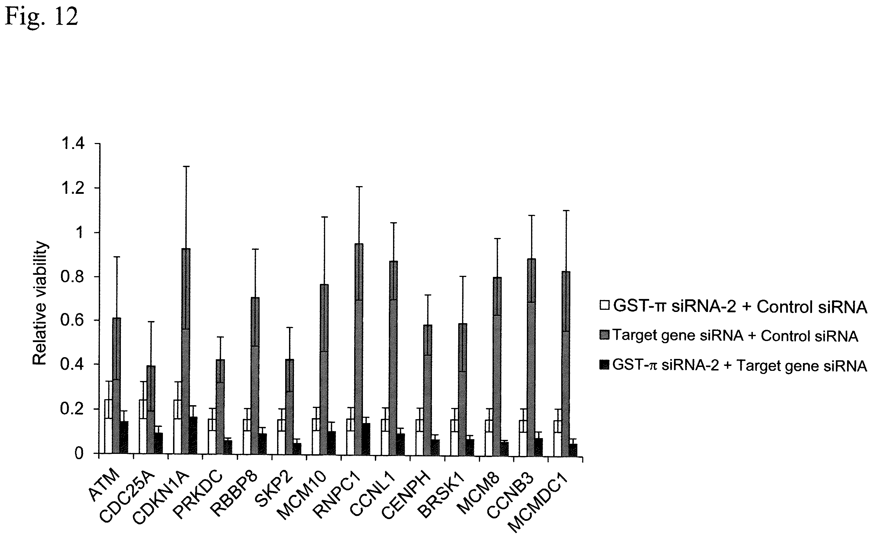

The results are shown in Figure 12. As a result of screening 170 types of genes encoding cell cycle-regulating proteins in terms of synthetic lethality with GST-π, ATM, as shown in Figure 12, CDC25A, PRKDC, RBBP8, SKP2, MCM10, RNPC1, CCNL1, CENPH, BRSK1, MCM8, CCNB3, and MCMDC1 in addition to P21 demonstrated in Experiment 1 were able to be screened for as cell cycle-regulating proteins that exhibited synthetic lethality by inhibition together with GST-π. Among them, P21, RNPC1, CCNL1, MCM8, CCNB3, and MCMDC1 were able to be screened for as cell cycle-regulating proteins that inhibited cell growth merely slightly (rate of growth inhibition: less than 20%) when inhibited alone, but exhibited synthetic lethality only when inhibited together with GST-π. Accordingly, it can be concluded that a drug inhibiting a cell cycle-regulating protein selected from P21, RNPC1, CCNL1, MCM8, CCNB3, and MCMDC1 is very low toxic in itself and is excellent in safety.

### [Experiment 3]

In Experiment 2, a cell cycle-regulating protein that exhibited synthetic lethality by inhibition together with GST-π was screened for. In this Experiment 3, a protein having an anti-apoptotic function that exhibited synthetic lethality by inhibition together with GST-π was screened for.

First, a MIA PaCa-2 cell suspension having a concentration of 1 × 10⁴ cells/mL was prepared with DMEM supplemented with 10% FBS and 1% L-glutamine, and this was inoculated at 100 µL/well to a 96-well plate and then cultured for 18 hours in DMEM supplemented with 10% FBS and 1% L-glutamine. The MIA PaCa-2 cells that became 20 to 30% confluent were transfected with GST-π siRNA-2 and/or siRNA against a target gene using Lipofectamine RNAi MAX as follows.

The Lipofectamine/siRNA mixed solution for transfection was prepared as follows: first, 51 µL of DNase free water (Ambion, Life Technologies Corp.) was added to 0.1 nmol of each siRNA contained in a custom-siRNA Library, which contains uniquely-selected 140 types of genes considered to have an anti-apoptosis function (siGENOME SMART pool Cherry-pick Library, GE Healthcare Dharmacon Inc.) and left standing at room temperature for 90 minutes. An siRNA solution in which this aqueous siRNA solution was supplemented with 19.9 µL of OPTI-MEM was prepared (solution A). Next, a 50 µM aqueous GST-π siRNA-2 solution and a 50 µM aqueous control siRNA solution were each diluted with OPTI-MEM to prepare solutions of 5 µM GST-π siRNA-2 and 5 µM control siRNA (solution B). 31.2 µL of the solution A and 8.8 µL of the solution B were mixed (solution C). Next, a Lipofectamine solution in which 150 µL of Lipofectamine RNAi MAX and 2.35 mL of OPTI-MEM were mixed was prepared (solution D). Next, 37.5 µL of the solution C and 37.5 µL of the solution D were mixed and left standing at room temperature for 15 minutes (solution E). The solution E was added to each well culturing MIA-PaCa-2 cell of the 96-well plate at 10 µL/well.

Separately, a 50 µM aqueous control siRNA solution (5.5 µL) and OPTI-MEM (189.5 µL) were mixed to prepare a solution (solution F). Next, a 50 µM aqueous control siRNA solution was diluted with OPTI-MEM in ten times to prepare 5 µM control siRNA (solution G). 31.2 µL of the solution F and 8.8 µL of the solution G were mixed (solution H). Next, a Lipofectamine solution in which 150 µL of Lipofectamine RNAi MAX and 2.35 mL of OPTI-MEM were mixed was prepared (solution I). Next, 37.5 µL of the solution H and 37.5 µL of the solution I were mixed and left standing at room temperature for 15 minutes (solution J). The solution J was added to each well culturing MIA-PaCa-2 cell of the 96-well plate at 10 µL/well. Then, the cells were cultured in DMEM supplemented with 10% FBS and 1% L-glutamine. After 5 days, a growth evaluation test was conducted using CyQUANT NF Cell Proliferation Assay Kit (Invitrogen Corp.).

First, 11 mL of 1 × HBSS buffer was added to 22 µL of CyQUANT NF dye reagent to prepare a staining reaction solution for CyQUANT NF Cell proliferation Assay. The medium of the transfected cells mentioned above was aspirated, and 50 µL of the staining reaction solution was added thereto. The cells were left standing at 37°C for 30 minutes. Then, a fluorescence wavelength of 520 nm was observed in excitation with an excitation wavelength of 480 nm.

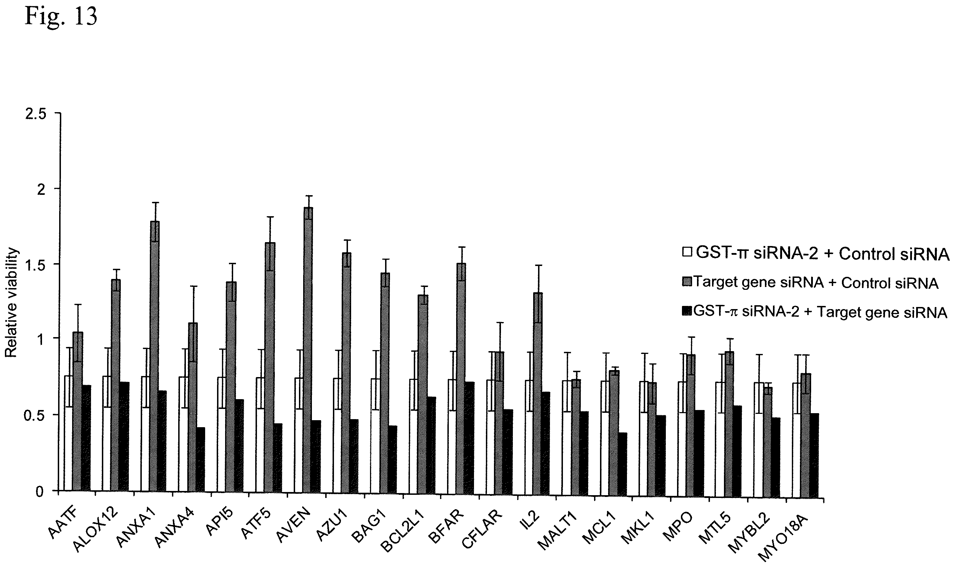

The results are shown in Figure 13. As a result of screening 140 types of genes encoding anti-apoptosis-related proteins in terms of synthetic lethality with GST-π, as shown in Figure 13, AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A were able to be screened for as anti-apoptosis-related proteins that exhibited synthetic lethality by inhibition together with GST-π.

### [Experiment 4]

In Experiment 2, a cell cycle-regulating protein that exhibited synthetic lethality by inhibition together with GST-π was screened for. In Experiment 3, a protein having an anti-apoptotic function that exhibited synthetic lethality by inhibition together with GST-π was screened for. In this Experiment 4, a PI3K signaling pathway-related protein that exhibited synthetic lethality by inhibition together with GST-π was screened for.

First, a MIA PaCa-2 cell suspension having a concentration of 1 × 10⁴ cells/mL was prepared with DMEM supplemented with 10% FBS and 1% L-glutamine, and this was inoculated at 100 µL/well to a 96-well plate and then cultured for 18 hours in DMEM supplemented with 10% FBS and 1% L-glutamine. The MIA PaCa-2 cells that became 20 to 30% confluent were transfected with GST-π siRNA-2 and/or siRNA against a target gene using Lipofectamine RNAi MAX as follows.

The Lipofectamine/siRNA mixed solution for transfection was prepared as follows: first, 51 µL of DNase free water (Ambion, Life Technologies Corp.) was added to 0.1 nmol of each siRNA contained in a custom-made siRNA library involving proprietary selected 80 types of genes thought to be involved in the PI3K signaling pathway (siGENOME SMARTpool Cherry-pick Library, GE Healthcare Dharmacon Inc.) and left standing at room temperature for 90 minutes. An siRNA solution in which this aqueous siRNA solution was supplemented with 19.9 µL of OPTI-MEM was prepared (solution A). Next, a 50 µM aqueous GST-π siRNA-2 solution and a 50 µM aqueous control siRNA solution were each diluted with OPTI-MEM in ten times to prepare diluted solutions of 5 µM GST-π siRNA-2 and 5 µM control siRNA (solution B). 31.2 µL of the solution A and 8.8 µL of the solution B were mixed (solution C). Next, a Lipofectamine solution in which 150 µL of Lipofectamine RNAi MAX and 2.35 mL of OPTI-MEM were mixed was prepared (solution D). Next, 37.5 µL of the solution C and 37.5 µL of the solution D were mixed and left standing at room temperature for 15 minutes (solution E). The solution E was added to each well culturing MIA-PaCa-2 cell of the 96-well plate at 10 µL/well.

Separately, a 50 µM aqueous control siRNA solution (5.5 µL) and OPTI-MEM (189.5 µL) were mixed to prepare a solution (solution F). Next, a 50 µM aqueous control siRNA solution was diluted with OPTI-MEM in ten times to prepare a diluted solution of 5 µM control siRNA (solution G). 31.2 µL of the solution F and 8.8 µL of the solution G were mixed (solution H). Next, a Lipofectamine solution in which 150 µL of Lipofectamine RNAi MAX and 2.35 mL of OPTI-MEM were mixed was prepared (solution I). Next, 37.5 µL of the solution H and 37.5 µL of the solution I were mixed and left standing at room temperature for 15 minutes (solution J). The solution J was added to each well culturing MIA-PaCa-2 cell of the 96-well plate at 10 µL/well to prepare a control. Then, the cells were cultured in DMEM supplemented with 10% FBS and 1% L-glutamine. After 5 days, a growth evaluation test was conducted using CyQUANT NF Cell Proliferation Assay Kit (Invitrogen Corp.).

First, 11 mL of 1 × HBSS buffer was added to 22 µL of CyQUANT NF dye reagent to prepare a staining reaction solution for CyQUANT NF Cell proliferation Assay. The medium of the transfected cells mentioned above was aspirated, and 50 µL of the staining reaction solution was added thereto. The cells were left standing at 37°C for 30 minutes. Then, a fluorescence wavelength of 520 nm was observed in excitation with an excitation wavelength of 480 nm.

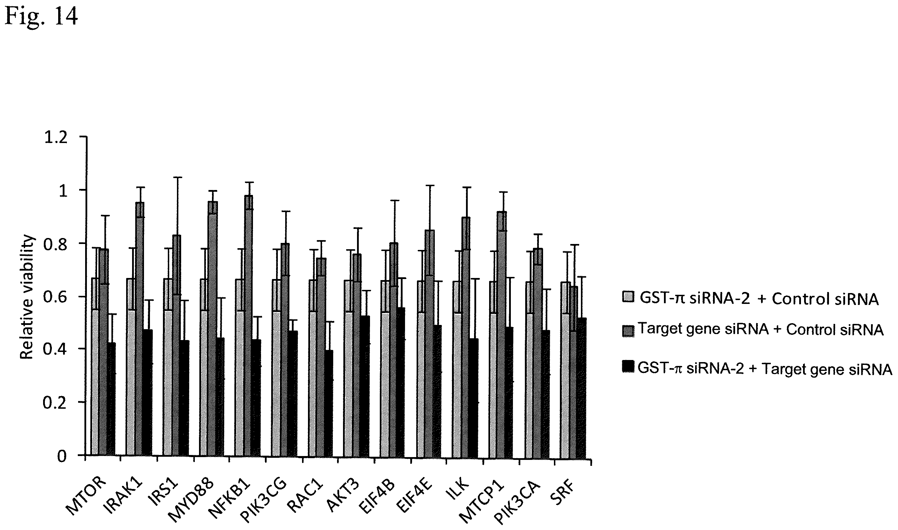

The results are shown in Figure 14. As a result of screening 80 types of genes encoding PI3K signaling pathway-related proteins in terms of synthetic lethality with GST-π, as shown in Figure 14, MTOR, IRAK1, IRS1, MYD88, NFKB1, PIK3CG, RAC1, AKT3, EIF4B, EIF4E, ILK, MTCP1, PIK3CA, and SRF were able to be screened for as PI3K signaling pathway-related proteins that exhibited synthetic lethality by inhibition together with GST-π. Among them, MTOR, IRAK1, IRS1, MYD88, NFKB1, PIK3CG, and RAC1 were found to exhibit a significantly high cell growth-inhibiting effect when inhibited together with GST-π.

### [Experiment 5]

In this Experiment 5, a cell cycle-regulating protein MYLK (HEPATOLOGY, Vol. 44, No. 1, 2006, 152-163) was studied for its synthetic lethality against A549 cells (human lung cancer cells having KRAS mutation) when inhibited together with GST-π.

First, one day before transfection with siRNA, 0.25 × 10⁵ cells/well of the A549 cells were inoculated, together with 2.25 mL of DMEM (antibiotic-free) supplemented with 10% FBS, to each well. In this experiment, a 6-well plate was used, and each sample was tested in triplicate. The cultured A549 cells were transfected with GST-π siRNA-3 and/or MYLK siRNA (two types (MYLKa and MYLKb) were used) using Lipofectamine RNAi MAX as follows.

The Lipofectamine/siRNA mixed solution for transfection was prepared as follows: first, an siRNA solution in which a 50 nM GST-π siRNA-3 solution and a 50 nM MYLK siRNA solution (0.5 µL each) were mixed and supplemented with 124 µL of OPTI-MEM was prepared. As for a control siRNA solution, siRNA solutions were also prepared as to the combination of the control siRNA solution and the GST-π siRNA-3 solution and the combination of the control siRNA solution and the MYLK siRNA solution so as to have the same siRNA concentrations. Also, a Lipofectamine solution in which 7.5 µL of Lipofectamine RNAi MAX and 117.5 µL of OPTI-MEM were mixed was prepared. Then, each prepared siRNA solution and the Lipofectamine solution were mixed and left standing at room temperature for 5 minutes.

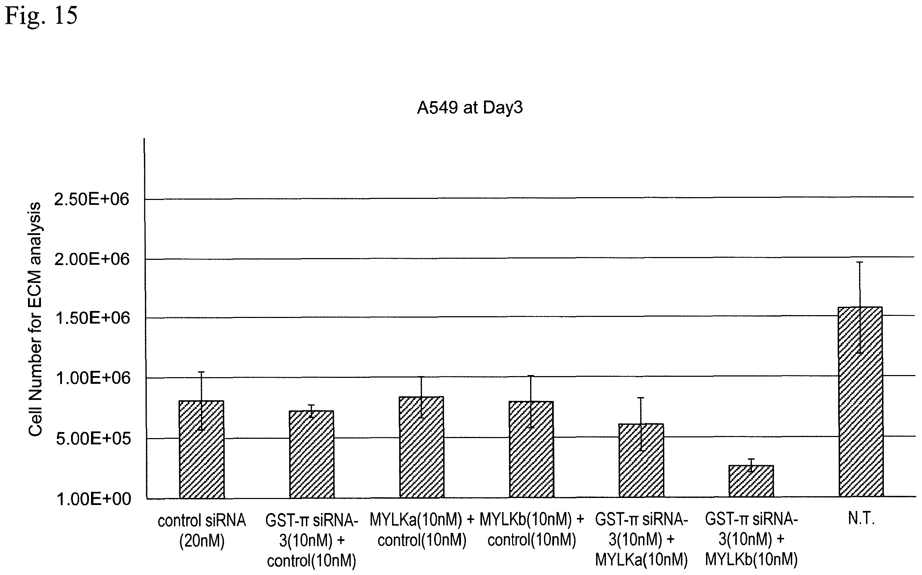

The obtained mixed solution was added dropwise to each well so that 2.5 mL thereof was finally contained in each well (final concentration of the siRNA: 20 nM). Then, the cells were cultured at 37°C for 75 hours under 5% CO₂ conditions with mild shaking. After the completion of culture, a growth evaluation test was conducted in the same way as in Experiments 1 to 4. The results are shown in Figure 15. As shown in Figure 15, MYLK, a cell cycle-regulating protein, was found to exhibit synthetic lethality against cancer cells when inhibited together with GST-π. The results shown in Figure 15 demonstrated that a GST-π-inhibiting drug and a MYLK-inhibiting drug exhibited a cell growth-inhibiting effect merely slightly when allowed to act each alone on cancer cells, whereas the GST-π-inhibiting drug and the MYLK-inhibiting drug were able to inhibit cell growth very strongly when allowed to act together on cancer cells.

siRNAs given below were used. In the description below, the upper-case letters represent RNAs, and the lower-case letters represent DNAs.

### GST-π siRNA-3:

Sense strand: CCUUUUGAGACCCUGCUGUtt (SEQ ID NO: 109)
Antisense strand: ACAGCAGGGUCUCAAAAGGtt (SEQ ID NO: 110)

### MYLKa:

Sense strand: CUGGGGAAGAAGGUGAGUAtt (SEQ ID NO: 111)
Antisense strand: UACUCACCUUCUUCCCCAGtt (SEQ ID NO: 112)

### MYLKb:

Sense strand: CAAGAUAGCCAGAGUUUAAtt (SEQ ID NO: 113)
Antisense strand: UUAAACUCUGGCUAUCUUGtt (SEQ ID NO: 114)

All publications, patents, and patent applications cited herein are incorporated herein by reference in their entirety.

## Claims

1. A cell death-inducing agent for a cancer cell comprising, as active ingredients, a drug inhibiting GST-π and a drug inhibiting a homeostasis-related protein that exhibits synthetic lethality when inhibited together with GST-π.

2. A cell growth-inhibiting agent for a cancer cell comprising, as active ingredients, a drug inhibiting Gust-π and a drug inhibiting a homeostasis-related protein that exhibits synthetic lethality when inhibited together with Gust-π.

3. The agent according to claim 1, wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.

4. The agent according to claim 3, wherein the cell cycle-regulating protein that exhibits synthetic lethality along with the inhibition of GST-π is at least one cell cycle-regulating protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK.

5. The agent according to claim 3, wherein the cell cycle-regulating protein that exhibits synthetic lethality along with the inhibition of GST-π is at least one protein selected from the group consisting of p21, RNPC1, CCNL1, MCM8, CCNB3, and MCMDC1.

6. The agent according to claim 3, wherein the anti-apoptosis-related protein that exhibits synthetic lethality along with the inhibition of GUST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.

7. The agent according to claim 3, wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.

8. The agent according to claim 1, wherein the drug is a substance selected from the group consisting of an RNAi molecule, a ribozyme, an antisense nucleic acid, a DNA/RNA chimeric polynucleotide, and a vector for expressing at least one of them.

9. The agent according to claim 1, wherein the drug inhibiting a homeostasis-related protein is a compound that acts on the homeostasis-related protein.

10. The agent according to claim 1, wherein the agent induces apoptosis.

11. The agent according to claim 1 or 2, wherein the cancer cell is a cancer cell highly expressing GST-π.

12. A pharmaceutical composition for the treatment of a disease caused by abnormal cell growth, comprising an agent according to claim 1.

13. The pharmaceutical composition according to claim 12, wherein the disease is a cancer.

14. The pharmaceutical composition according to claim 13, wherein the cancer is a cancer highly expressing GST-π.

15. A method for screening for a cell death-inducing agent and/or a cell growth-inhibiting agent for a cancer cell that is used together with a drug inhibiting GST-π, comprising a step of selecting a drug inhibiting a homeostasis-related protein that exhibits synthetic lethality when inhibited together with GST-π.

16. The screening method according to claim 15, comprising the steps of: contacting a test substance with a cancer cell; measuring the expression level of the homeostasis-related protein in the cell; and selecting the test substance as a drug inhibiting the homeostasis-related protein when the expression level is decreased compared with that measured in the absence of the test substance.

17. The screening method according to claim 15, wherein the homeostasis-related protein that exhibits synthetic lethality along with the inhibition of GST-π is a protein selected from the group consisting of a cell cycle-regulating protein, an anti-apoptosis-related protein, and a PI3K signaling pathway-related protein.

18. The screening method according to claim 17, wherein the cell cycle-regulating protein that exhibits synthetic lethality along with the inhibition of GST-π is at least one cell cycle-regulating protein selected from the group consisting of ATM, CDC25A, p21, PRKDC, RBBP8, SKP2, MCM10, RNPC1, CCNL1, CENPH, BRSK1, MCM8, CCNB3, MCMDC1 and MYLK.

19. The screening method according to claim 17, wherein the cell cycle-regulating protein that exhibits synthetic lethality along with the inhibition of GST-π is at least one protein selected from the group consisting of p21, RNPC1, CCNL1, MCM8, CCNB3, and MCMDC1.

20. The screening method according to claim 17, wherein the anti-apoptosis-related protein that exhibits synthetic lethality along with the inhibition of GST-π is at least one anti-apoptosis-related protein selected from the group consisting of AATF, ALOX12, ANXA1, ANXA4, API5, ATF5, AVEN, AZU1, BAG1, BCL2L1, BFAR, CFLAR, IL2, MALT1, MCL1, MKL1, MPO, MTL5, MYBL2, and MYO18A.

21. The screening method according to claim 16, wherein the PI3K signaling pathway-related protein that exhibits synthetic lethality along with the inhibition of GST-π is at least one PI3K signaling pathway-related protein selected from the group consisting of MTOR, IRAK1, IRS1, MYD88, NFKB1, PIK3CG, RAC1, AKT3, EIF4B, EIF4E, ILK, MTCP1, PIK3CA, and SRF.


SEQUENCE LISTING

<110>  NITTO DENKO CORPORATION

<120>  Cell death-inducing agent, Cell proliferation-inhibiting agent,
       and a pharmaceutical composition for treatment of disease
       resulting from cell proliferative abnormality

<130>  PH-6256-PCT

<150>  JP 2014-266198
<151>  2014-12-26

<150>  JP 2015-135494
<151>  2015-07-06

<150>  JP 2015-247725
<151>  2015-12-18

<160>  114

<170>  PatentIn version 3.5

<210>  1
<211>  986
<212>  DNA
<213>  Homo sapiens


<220>
<221>  CDS
<222>  (250)..(882)

<400>  1
tgggaaagag ggaaaggctt ccccggccag ctgcgcggcg actccgggga ctccagggcg      60

cccctctgcg gccgacgccc ggggtgcagc ggccgccggg gctggggccg gcgggagtcc     120

gcgggaccct ccagaagagc ggccggcgcc gtgactcagc actggggcgg agcggggcgg     180

gaccaccctt ataaggctcg gaggccgcga ggccttcgct ggagtttcgc cgccgcagtc     240

ttcgccacc atg ccg ccc tac acc gtg gtc tat ttc cca gtt cga ggc cgc     291
          Met Pro Pro Tyr Thr Val Val Tyr Phe Pro Val Arg Gly Arg
          1               5                   10

tgc gcg gcc ctg cgc atg ctg ctg gca gat cag ggc cag agc tgg aag       339
Cys Ala Ala Leu Arg Met Leu Leu Ala Asp Gln Gly Gln Ser Trp Lys
15                  20                  25                  30

gag gag gtg gtg acc gtg gag acg tgg cag gag ggc tca ctc aaa gcc       387
Glu Glu Val Val Thr Val Glu Thr Trp Gln Glu Gly Ser Leu Lys Ala
                35                  40                  45

tcc tgc cta tac ggg cag ctc ccc aag ttc cag gac gga gac ctc acc       435
Ser Cys Leu Tyr Gly Gln Leu Pro Lys Phe Gln Asp Gly Asp Leu Thr
            50                  55                  60

ctg tac cag tcc aat acc atc ctg cgt cac ctg ggc cgc acc ctt ggg       483
Leu Tyr Gln Ser Asn Thr Ile Leu Arg His Leu Gly Arg Thr Leu Gly
        65                  70                  75

ctc tat ggg aag gac cag cag gag gca gcc ctg gtg gac atg gtg aat       531

Leu Tyr Gly Lys Asp Gln Gln Glu Ala Ala Leu Val Asp Met Val Asn
    80                  85                  90

gac ggc gtg gag gac ctc cgc tgc aaa tac atc tcc ctc atc tac acc      579
Asp Gly Val Glu Asp Leu Arg Cys Lys Tyr Ile Ser Leu Ile Tyr Thr
95                  100                 105                 110

aac tat gag gcg ggc aag gat gac tat gtg aag gca ctg ccc ggg caa      627
Asn Tyr Glu Ala Gly Lys Asp Asp Tyr Val Lys Ala Leu Pro Gly Gln
                115                 120                 125

ctg aag cct ttt gag acc ctg ctg tcc cag aac cag gga ggc aag acc      675
Leu Lys Pro Phe Glu Thr Leu Leu Ser Gln Asn Gln Gly Gly Lys Thr
            130                 135                 140

ttc att gtg gga gac cag atc tcc ttc gct gac tac aac ctg ctg gac      723
Phe Ile Val Gly Asp Gln Ile Ser Phe Ala Asp Tyr Asn Leu Leu Asp
        145                 150                 155

ttg ctg ctg atc cat gag gtc cta gcc cct ggc tgc ctg gat gcg ttc      771
Leu Leu Leu Ile His Glu Val Leu Ala Pro Gly Cys Leu Asp Ala Phe
    160                 165                 170

ccc ctg ctc tca gca tat gtg ggg cgc ctc agt gcc cgg ccc aag ctc      819
Pro Leu Leu Ser Ala Tyr Val Gly Arg Leu Ser Ala Arg Pro Lys Leu
175                 180                 185                 190

aag gcc ttc ctg gcc tcc cct gag tac gtg aac ctc ccc atc aat ggc      867
Lys Ala Phe Leu Ala Ser Pro Glu Tyr Val Asn Leu Pro Ile Asn Gly
                195                 200                 205

aac ggg aaa cag tga gggttggggg gactctgagc gggaggcaga gtttgccttc      922
Asn Gly Lys Gln
            210

ctttctccag gaccaataaa atttctaaga gagctaaaaa aaaaaaaaaa aaaaaaaaaa      982

aaaa                                                                  986

<210>  2
<211>  210
<212>  PRT
<213>  Homo sapiens

<400>  2

Met Pro Pro Tyr Thr Val Val Tyr Phe Pro Val Arg Gly Arg Cys Ala
1               5                   10                  15

Ala Leu Arg Met Leu Leu Ala Asp Gln Gly Gln Ser Trp Lys Glu Glu
            20                  25                  30

Val Val Thr Val Glu Thr Trp Gln Glu Gly Ser Leu Lys Ala Ser Cys
        35                  40                  45

Leu Tyr Gly Gln Leu Pro Lys Phe Gln Asp Gly Asp Leu Thr Leu Tyr
    50                  55                  60

Gln Ser Asn Thr Ile Leu Arg His Leu Gly Arg Thr Leu Gly Leu Tyr
65                  70                  75                  80

Gly Lys Asp Gln Gln Glu Ala Ala Leu Val Asp Met Val Asn Asp Gly
                85                  90                  95

Val Glu Asp Leu Arg Cys Lys Tyr Ile Ser Leu Ile Tyr Thr Asn Tyr
            100                 105                 110

Glu Ala Gly Lys Asp Asp Tyr Val Lys Ala Leu Pro Gly Gln Leu Lys
        115                 120                 125

Pro Phe Glu Thr Leu Leu Ser Gln Asn Gln Gly Gly Lys Thr Phe Ile
    130                 135                 140

Val Gly Asp Gln Ile Ser Phe Ala Asp Tyr Asn Leu Leu Asp Leu Leu
145                 150                 155                 160

Leu Ile His Glu Val Leu Ala Pro Gly Cys Leu Asp Ala Phe Pro Leu
                165                 170                 175

Leu Ser Ala Tyr Val Gly Arg Leu Ser Ala Arg Pro Lys Leu Lys Ala
            180                 185                 190

Phe Leu Ala Ser Pro Glu Tyr Val Asn Leu Pro Ile Asn Gly Asn Gly
        195                 200                 205

Lys Gln
    210

<210>  3
<211>  2175
<212>  DNA
<213>  Homo sapiens

<220>
<221>  CDS
<222>  (126)..(620)

<400>  3
   gttgtatatc agggccgcgc tgagctgcgc cagctgaggt gtgagcagct gccgaagtca      60
   gttccttgtg gagccggagc tgggcgcgga ttcgccgagg caccgaggca ctcagaggag     120
   gcgcc atg tca gaa ccg gct ggg gat gtc cgt cag aac cca tgc ggc agc     170
         Met Ser Glu Pro Ala Gly Asp Val Arg Gln Asn Pro Cys Gly Ser
         1               5                   10                  15
   aag gcc tgc cgc cgc ctc ttc ggc cca gtg gac agc gag cag ctg agc       218
   Lys Ala Cys Arg Arg Leu Phe Gly Pro Val Asp Ser Glu Gln Leu Ser
                   20                  25                  30
   cgc gac tgt gat gcg cta atg gcg ggc tgc atc cag gag gcc cgt gag 266
   Arg Asp Cys Asp Ala Leu Met Ala Gly Cys Ile Gln Glu Ala Arg Glu
   35 40 45
   cga tgg aac ttc gac ttt gtc acc gag aca cca ctg gag ggt gac ttc 314
   Arg Trp Asn Phe Asp Phe Val Thr Glu Thr Pro Leu Glu Gly Asp Phe
   50 55 60
   gcc tgg gag cgt gtg cgg ggc ctt ggc ctg ccc aag ctc tac ctt ccc 362
   Ala Trp Glu Arg Val Arg Gly Leu Gly Leu Pro Lys Leu Tyr Leu Pro
   65 70 75
   acg ggg ccc cgg cga ggc cgg gat gag ttg gga gga ggc agg cgg cct 410
   Thr Gly Pro Arg Arg Gly Arg Asp Glu Leu Gly Gly Gly Arg Arg Pro
   80 85 90 95
   ggc acc tca cct gct ctg ctg cag ggg aca gca gag gaa gac cat gtg 458
   Gly Thr Ser Pro Ala Leu Leu Gln Gly Thr Ala Glu Glu Asp His Val
   100 105 110
   gac ctg tca ctg tct tgt acc ctt gtg cct cgc tca ggg gag cag gct 506
   Asp Leu Ser Leu Ser Cys Thr Leu Val Pro Arg Ser Gly Glu Gln Ala
   115 120 125
   gaa ggg tcc cca ggt gga cct gga gac tct cag ggt cga aaa cgg cgg 554
   Glu Gly Ser Pro Gly Gly Pro Gly Asp Ser Gln Gly Arg Lys Arg Arg
   130 135 140
   cag acc agc atg aca gat ttc tac cac tcc aaa cgc cgg ctg atc ttc 602
   Gln Thr Ser Met Thr Asp Phe Tyr His Ser Lys Arg Arg Leu Ile Phe
   145 150 155
   tcc aag agg aag ccc taa tccgcccaca ggaagcctgc agtcctggaa 650
   Ser Lys Arg Lys Pro
   160
   gcgcgagggc ctcaaaggcc cgctctacat cttctgcctt agtctcagtt tgtgtgtctt 710
   aattattatt tgtgttttaa tttaaacacc tcctcatgta catacctggg ccgccccctg 770
   ccccccagcc tctggcatta gaattattta aacaaaaact aggcggttga atgagaggtt 830
   cctaagagtg ctgggcattt ttattttatg aaatactatt taaagcctcc tcatcccgtg 890
   ttctcctttt cctctctccc ggaggttggg tgggccggct tcatgccagc tacttcctcc 950
   tccccacttg tccgctgggt ggtaccctct ggaggggtgt ggctccttcc catcgctgtc 1010
   acaggcggtt atgaaattca ccccctttcc tggacactca gacctgaatt ctttttcatt 1070
   tgagaagtaa acagatggca ctttgaaggg gcctcaccga gtgggggcat catcaaaaac 1130
   tttggagtcc cctcacctcc tctaaggttg ggcagggtga ccctgaagtg agcacagcct 1190
   agggctgagc tggggacctg gtaccctcct ggctcttgat acccccctct gtcttgtgaa 1250
   ggcaggggga aggtggggtc ctggagcaga ccaccccgcc tgccctcatg gcccctctga 1310
   cctgcactgg ggagcccgtc tcagtgttga gccttttccc tctttggctc ccctgtacct 1370
   tttgaggagc cccagctacc cttcttctcc agctgggctc tgcaattccc ctctgctgct 1430
   gtccctcccc cttgtccttt cccttcagta ccctctcagc tccaggtggc tctgaggtgc 1490
   ctgtcccacc cccaccccca gctcaatgga ctggaagggg aagggacaca caagaagaag 1550
   ggcaccctag ttctacctca ggcagctcaa gcagcgaccg ccccctcctc tagctgtggg 1610
   ggtgagggtc ccatgtggtg gcacaggccc ccttgagtgg ggttatctct gtgttagggg 1670
   tatatgatgg gggagtagat ctttctagga gggagacact ggcccctcaa atcgtccagc 1730
   gaccttcctc atccacccca tccctcccca gttcattgca ctttgattag cagcggaaca 1790
   aggagtcaga cattttaaga tggtggcagt agaggctatg gacagggcat gccacgtggg 1850
   ctcatatggg gctgggagta gttgtctttc ctggcactaa cgttgagccc ctggaggcac 1910
   tgaagtgctt agtgtacttg gagtattggg gtctgacccc aaacaccttc cagctcctgt 1970
   aacatactgg cctggactgt ttctctcggg ctccccatgt gtcctggttc ccgtttctcc 2030
   acctagactg taaacctctc gagggcaggg accacaccct gtactgttct gtgtctttca 2090
   cagctcctcc cacaatgctg aatatacagc aggtgctcaa taaatgattc ttagtgactt 2150
   tacttgtaaa aaaaaaaaaa aaaaa 2175

<210> 4
<211> 164
<212> PRT
<213> Homo sapiens

<400> 4

Met Ser Glu Pro Ala Gly Asp Val Arg Gln Asn Pro Cys Gly Ser Lys
1               5                   10                  15

Ala Cys Arg Arg Leu Phe Gly Pro Val Asp Ser Glu Gln Leu Ser Arg
            20                  25                  30

Asp Cys Asp Ala Leu Met Ala Gly Cys Ile Gln Glu Ala Arg Glu Arg
        35                  40                  45

Trp Asn Phe Asp Phe Val Thr Glu Thr Pro Leu Glu Gly Asp Phe Ala
    50                  55                  60

Trp Glu Arg Val Arg Gly Leu Gly Leu Pro Lys Leu Tyr Leu Pro Thr
65                  70                  75                  80

Gly Pro Arg Arg Gly Arg Asp Glu Leu Gly Gly Gly Arg Arg Pro Gly
                85                  90                  95

Thr Ser Pro Ala Leu Leu Gln Gly Thr Ala Glu Glu Asp His Val Asp
            100                 105                 110

Leu Ser Leu Ser Cys Thr Leu Val Pro Arg Ser Gly Glu Gln Ala Glu
        115                 120                 125

Gly Ser Pro Gly Gly Pro Gly Asp Ser Gln Gly Arg Lys Arg Arg Gln
    130                 135                 140

Thr Ser Met Thr Asp Phe Tyr His Ser Lys Arg Arg Leu Ile Phe Ser
145                 150                 155                 160

Lys Arg Lys Pro

<210>  5
<211>  2410
<212>  DNA
<213>  Homo sapiens

<220>
<221>  CDS
<222>  (185)..(904)

<400>  5
agagtccccg cagcgccggt cgggagcgca gcgcggcgca gctcggcgcg cacggcggga      60

gcggcgcgcg agtggtcggg cctggcggct ggacgggcgc ccctcgctgc cccgcgcgct      120

ccccgccgcc ccccatgagc gcagccccgc gcggcccggg tccgtaggcg gcggggcgcc      180

cccc atg ctg ctg cag ccc gcg ccg tgc gcc ccg agc gcg ggc ttc ccg      229
     Met Leu Leu Gln Pro Ala Pro Cys Ala Pro Ser Ala Gly Phe Pro
     1               5                   10                  15

cgg ccc ctg gcc gcc ccc ggc gcc atg cac ggc tcg cag aag gac acc      277
Arg Pro Leu Ala Ala Pro Gly Ala Met His Gly Ser Gln Lys Asp Thr
                20                  25                  30

acg ttc acc aag atc ttc gtg ggc ggc ctg ccg tac cac act acc gac      325
Thr Phe Thr Lys Ile Phe Val Gly Gly Leu Pro Tyr His Thr Thr Asp
            35                  40                  45

gcc tcg ctc agg aag tac ttc gag ggc ttc ggc gac atc gag gag gcc      373
Ala Ser Leu Arg Lys Tyr Phe Glu Gly Phe Gly Asp Ile Glu Glu Ala
        50                  55                  60

gtg gtc atc acc gac cgc cag acg ggc aag tcc cgc ggc tac ggc ttc      421
Val Val Ile Thr Asp Arg Gln Thr Gly Lys Ser Arg Gly Tyr Gly Phe
    65                  70                  75

gtg acc atg gcc gac cgg gcg gca gct gag agg gct tgc aaa gac ccg      469
Val Thr Met Ala Asp Arg Ala Ala Ala Glu Arg Ala Cys Lys Asp Pro
80                  85                  90                  95

aac ccc atc atc gac ggc cgc aag gcc aac gtg aac ctg gca tat ctg      517
Asn Pro Ile Ile Asp Gly Arg Lys Ala Asn Val Asn Leu Ala Tyr Leu
                100                 105                 110

ggc gcc aag ccg cgg agc ctc cag acg ggc ttt gcc att ggg gtg cag      565
Gly Ala Lys Pro Arg Ser Leu Gln Thr Gly Phe Ala Ile Gly Val Gln
            115                 120                 125

cag ctg cac ccc acc ttg atc cag cgg act tac ggg ctg acc ccg cac     613
Gln Leu His Pro Thr Leu Ile Gln Arg Thr Tyr Gly Leu Thr Pro His
        130                 135                 140

tac atc tac cca cca gcc atc gtg cag ccc agc gtg gtg atc cca gcc     661
Tyr Ile Tyr Pro Pro Ala Ile Val Gln Pro Ser Val Val Ile Pro Ala
    145                 150                 155

gcc cct gtc ccg tcg ctg tcc tcg ccc tac att gag tac acg ccg gcc     709
Ala Pro Val Pro Ser Leu Ser Ser Pro Tyr Ile Glu Tyr Thr Pro Ala
160                 165                 170                 175

agc ccg gcc tac gcc cag tac cca ccg gcc acc tat gac cag tac cca     757
Ser Pro Ala Tyr Ala Gln Tyr Pro Pro Ala Thr Tyr Asp Gln Tyr Pro
                180                 185                 190

tac gcc gcc tcg cct gcc acg gct gcc agc ttc gtg ggc tac agc tac     805
Tyr Ala Ala Ser Pro Ala Thr Ala Ala Ser Phe Val Gly Tyr Ser Tyr
            195                 200                 205

cct gcc gcc gtg ccc cag gcc ctc tca gcc gca gca ccc gcg ggc acc     853
Pro Ala Ala Val Pro Gln Ala Leu Ser Ala Ala Ala Pro Ala Gly Thr
        210                 215                 220

act ttc gtg cag tac cag gcg ccg cag ctg cag cct gac agg atg cag     901
Thr Phe Val Gln Tyr Gln Ala Pro Gln Leu Gln Pro Asp Arg Met Gln
    225                 230                 235

tga ggggcgttcc tgccccgagg actgtggcat tgtcaccttc acagcagaca     954

gagctgccag gccatgatgg gctggcgaca gcccggctga gcttcagtga ggtgccacca     1014

gcacccgtgc ctccgaagac cgctcgggca ttccgcctgc gccctgggac agcggagaga     1074

cggcttctct ttaatctagg tcccattgtg tcttgaggga ggactttaag aatgactgag     1134

aactatttaa agacgcaatc ccaggttcct tgcacaccat ggcagcctct ccttgcacct     1194

tctcctgcct ctccacactc caggttccct caggcttgtg tccccactgc tgcatcgtgg     1254

cggggtgtca cagaccctct gcagcccctg gctgccctgg actgtgcaga gatgcctgac     1314

tccagggaaa cctgaaagca agaagttaat ggactgttta ttgtaacttg atcctcccga     1374

gctgtgagcg cagtctgagg tgtgaggaca cggcctcctg ttggagtccc attttctcca     1434

tcagggcacg tgggcggctt cctcaagccc ggaggagctc ccaggcgcac aggggccgcc     1494

ggtaacaggg gccgccggcc aaaggcccct ttccagtcat agcactgaag ttgcaacttt     1554

tttcttgtaa ttgttttgct actaagataa tttcagaagt tcagtctatt ttttcagcgg     1614

atactgccgc caccaagaat ccaaaaccta tttttgactt ggagagactt gcttttgttg     1674

gttccgcccg tggagacgac gatagtgttt ctgtataata aagtgtctgc cggctcgcgg     1734

gccaggatcc tctcggtggg atgggcacca cagacaggag gcccctcagg cccgtgcggg     1794

ccactgtctg ctgccgcctg ccggggtggc agagtgagtt gtctcaggac cccgtcactg     1854

cgacgttgac actcctctcc cttcccttcc tccccaactc cccaaacact gtggaagggg     1914

agaaggaagt gatccacagc attcaggcca cttggggtct agaccatggt ggtgccagcc     1974

tggggggggc agtggccctc agctctgccc gctggagcgg ttgagtgcag aagggtgcgc 2034

ctcttccctc tacccccgca ccacctgctg tgtgccagcc tgagacggtt cctgcctgtc 2094

ttgggggttg gtggagggtg gaggcagttc tgccagccgt ggcagggctg ctatggggca 2154

tccagggctg tgggggtctg gaggagggga catgaggtga gaggtatcct ggccgagggc 2214

ggggggcagc ggggggtctc cctccggacc tacctcaggg agctgagcgt gcaggcgctc 2274

cagggcaggc ctgggacaga gtcaaggctc agagaataaa ggtagctaat ctcatcataa 2334

tatttttatt agaatgttct gatgataaaa ataaaacttg ttttctttaa agaaaaaaaa 2394

aaaaaaaaaa aaaaaa 2410

<210> 6
<211> 239
<212> PRT
<213> Homo sapiens

<400> 6

Met Leu Leu Gln Pro Ala Pro Cys Ala Pro Ser Ala Gly Phe Pro Arg
1               5                   10                  15

Pro Leu Ala Ala Pro Gly Ala Met His Gly Ser Gln Lys Asp Thr Thr
            20                  25                  30

Phe Thr Lys Ile Phe Val Gly Gly Leu Pro Tyr His Thr Thr Asp Ala
        35                  40                  45

Ser Leu Arg Lys Tyr Phe Glu Gly Phe Gly Asp Ile Glu Glu Ala Val
    50                  55                  60

Val Ile Thr Asp Arg Gln Thr Gly Lys Ser Arg Gly Tyr Gly Phe Val
65                  70                  75                  80

Thr Met Ala Asp Arg Ala Ala Ala Glu Arg Ala Cys Lys Asp Pro Asn
                85                  90                  95

Pro Ile Ile Asp Gly Arg Lys Ala Asn Val Asn Leu Ala Tyr Leu Gly
            100                 105                 110

Ala Lys Pro Arg Ser Leu Gln Thr Gly Phe Ala Ile Gly Val Gln Gln
        115                 120                 125

Leu His Pro Thr Leu Ile Gln Arg Thr Tyr Gly Leu Thr Pro His Tyr
    130                 135                 140

Ile Tyr Pro Pro Ala Ile Val Gln Pro Ser Val Val Ile Pro Ala Ala
145                 150                 155                 160

Pro Val Pro Ser Leu Ser Ser Pro Tyr Ile Glu Tyr Thr Pro Ala Ser
                165                 170                 175

Pro Ala Tyr Ala Gln Tyr Pro Pro Ala Thr Tyr Asp Gln Tyr Pro Tyr
            180                 185                 190

Ala Ala Ser Pro Ala Thr Ala Ala Ser Phe Val Gly Tyr Ser Tyr Pro
        195                 200                 205

Ala Ala Val Pro Gln Ala Leu Ser Ala Ala Ala Pro Ala Gly Thr Thr
    210                 215                 220

Phe Val Gln Tyr Gln Ala Pro Gln Leu Gln Pro Asp Arg Met Gln
225                 230                 235

<210>  7
<211>  2625
<212>  DNA
<213>  Homo sapiens

<220>
<221>  CDS
<222>  (600)..(2180)

<400>  7
atagggtcca cagccttgtg ttcttcaaat actgtaaagc agccctctcc agattccttg      60

agggtcactt tatattacac attcgtccac acaggacaac aaagtcacca cttccacccg     120

ccaaagcgcg ctcaggtcgg ccgccacttc ccttggtaca cgcggaacgc aagccgcgcc     180

gaggctaaag gaagaggccg gaatgcaggg cgaaggggcg cagagatgct agccaatgct     240

gccacctcta ttttggatat aagggatctt gtaaagctcc gttattcgtg tggctatcac     300

aaacaaggtg gtttcgaccg gccttggcta gagaaggcct cctcagcctc tgaggatccc     360

aaggtctgtc gcagcaccca acactcccct cgcaggccca gatgccgagg gcggaggcgc     420

ggcctggagg gactagttgg cgcgcggctg cccggcgtgg tgacgtcagg gcggaagcgc     480

acgcggcgtg aagcggccca gaacccggcg gtcacgggaa tatccgtcgc gccgagaacg     540

ctggttagtc ttgtttcggg ttccggctgc gttgggcttg cgtgcggctc gctaagact      599

atg gcg tcc ggg cct cat tcg aca gct act gct gcc gca gcc gcc tca       647
Met Ala Ser Gly Pro His Ser Thr Ala Thr Ala Ala Ala Ala Ala Ser
1               5                   10                  15

tcg gcc gcc cca agc gcg ggc ggc tcc agc tcc ggg acg acg acc acg       695
Ser Ala Ala Pro Ser Ala Gly Gly Ser Ser Ser Gly Thr Thr Thr Thr
            20                  25                  30

acg acg acc acg acg gga ggg atc ctg atc ggc gat cgc ctg tac tcg       743
Thr Thr Thr Thr Thr Gly Gly Ile Leu Ile Gly Asp Arg Leu Tyr Ser

35                  40                  45

gaa gtt tca ctt acc atc gac cac tct ctg att ccg gag gag agg ctc      791
Glu Val Ser Leu Thr Ile Asp His Ser Leu Ile Pro Glu Glu Arg Leu
    50                  55                  60

tcg ccc acc cca tcc atg cag gat ggg ctc gac ctg ccc agt gag acg      839
Ser Pro Thr Pro Ser Met Gln Asp Gly Leu Asp Leu Pro Ser Glu Thr
65                  70                  75                  80

gac tta cgc atc ctg ggc tgc gag ctc atc cag gcc gcc ggc att ctc      887
Asp Leu Arg Ile Leu Gly Cys Glu Leu Ile Gln Ala Ala Gly Ile Leu
                85                  90                  95

ctc cgg ctg ccg cag gtg gcg atg gca acg ggg cag gtg ttg ttt cat      935
Leu Arg Leu Pro Gln Val Ala Met Ala Thr Gly Gln Val Leu Phe His
            100                 105                 110

cgt ttt ttc tac tcc aaa tct ttc gtc aaa cac agt ttc gag att gtt      983
Arg Phe Phe Tyr Ser Lys Ser Phe Val Lys His Ser Phe Glu Ile Val
        115                 120                 125

gct atg gct tgt att aat ctt gca tca aaa atc gaa gaa gca cct aga      1031
Ala Met Ala Cys Ile Asn Leu Ala Ser Lys Ile Glu Glu Ala Pro Arg
    130                 135                 140

aga ata aga gat gtg att aat gta ttc cac cac ctc cgc cag tta aga      1079
Arg Ile Arg Asp Val Ile Asn Val Phe His His Leu Arg Gln Leu Arg
145                 150                 155                 160

gga aaa agg act cca agc ccc ctg atc ctt gat cag aac tac att aac      1127
Gly Lys Arg Thr Pro Ser Pro Leu Ile Leu Asp Gln Asn Tyr Ile Asn
                165                 170                 175

acc aaa aat caa gtt atc aaa gca gag agg agg gtg cta aag gag ttg      1175
Thr Lys Asn Gln Val Ile Lys Ala Glu Arg Arg Val Leu Lys Glu Leu
            180                 185                 190

gga ttt tgt gtt cat gtc aag cat cct cat aag atc att gtt atg tat      1223
Gly Phe Cys Val His Val Lys His Pro His Lys Ile Ile Val Met Tyr
        195                 200                 205

tta caa gtc tta gaa tgt gaa cgt aat caa acc ctg gtt caa act gcc      1271
Leu Gln Val Leu Glu Cys Glu Arg Asn Gln Thr Leu Val Gln Thr Ala
    210                 215                 220

tgg aat tac atg aat gac agt ctt cga acc aat gtg ttt gtt cga ttt      1319
Trp Asn Tyr Met Asn Asp Ser Leu Arg Thr Asn Val Phe Val Arg Phe
225                 230                 235                 240

caa cca gag act ata gca tgt gct tgc atc tac ctt gca gct aga gca      1367
Gln Pro Glu Thr Ile Ala Cys Ala Cys Ile Tyr Leu Ala Ala Arg Ala
                245                 250                 255

ctt cag att ccg ttg cca act cgt ccc cat tgg ttt ctt ctt ttt ggt      1415
Leu Gln Ile Pro Leu Pro Thr Arg Pro His Trp Phe Leu Leu Phe Gly
            260                 265                 270

act aca gaa gag gaa atc cag gaa atc tgc ata gaa aca ctt agg ctt      1463
Thr Thr Glu Glu Glu Ile Gln Glu Ile Cys Ile Glu Thr Leu Arg Leu
        275                 280                 285

tat acc aga aaa aag cca aac tat gaa tta ctg gaa aaa gaa gta gaa      1511

Tyr Thr Arg Lys Lys Pro Asn Tyr Glu Leu Leu Glu Lys Glu Val Glu
    290                 295                 300

aaa aga aaa gta gcc tta caa gaa gcc aaa tta aaa gca aag gga ttg      1559
Lys Arg Lys Val Ala Leu Gln Glu Ala Lys Leu Lys Ala Lys Gly Leu
305                 310                 315                 320

aat ccg gat gga act cca gcc ctt tca acc ctg ggt gga ttt tct cca      1607
Asn Pro Asp Gly Thr Pro Ala Leu Ser Thr Leu Gly Gly Phe Ser Pro
                325                 330                 335

gcc tcc aag cca tca tca cca aga gaa gta aaa gct gaa gag aaa tca      1655
Ala Ser Lys Pro Ser Ser Pro Arg Glu Val Lys Ala Glu Glu Lys Ser
            340                 345                 350

cca atc tcc att aat gtg aag aca gtc aaa aaa gaa cct gag gat aga      1703
Pro Ile Ser Ile Asn Val Lys Thr Val Lys Lys Glu Pro Glu Asp Arg
        355                 360                 365

caa cag gct tcc aaa agc cct tac aat ggt gta aga aaa gac agc aag      1751
Gln Gln Ala Ser Lys Ser Pro Tyr Asn Gly Val Arg Lys Asp Ser Lys
    370                 375                 380

aga agt aga aat agc aga agt gca agt cga tcg agg tca aga aca cga      1799
Arg Ser Arg Asn Ser Arg Ser Ala Ser Arg Ser Arg Ser Arg Thr Arg
385                 390                 395                 400

tca cgt tct aga tca cat act cca aga aga cac tat aat aat agg cgg      1847
Ser Arg Ser Arg Ser His Thr Pro Arg Arg His Tyr Asn Asn Arg Arg
                405                 410                 415

agt cga tct gga aca tac agc tcg aga tca aga agc agg tcc cgc agt      1895
Ser Arg Ser Gly Thr Tyr Ser Ser Arg Ser Arg Ser Arg Ser Arg Ser
            420                 425                 430

cac agt gaa agc cct cga aga cat cat aat cat ggt tct cct cac ctt      1943
His Ser Glu Ser Pro Arg Arg His His Asn His Gly Ser Pro His Leu
        435                 440                 445

aag gcc aag cat acc aga gat gat tta aaa agt tca aac aga cat ggt      1991
Lys Ala Lys His Thr Arg Asp Asp Leu Lys Ser Ser Asn Arg His Gly
    450                 455                 460

cat aaa agg aaa aaa tct cgt tct cga tct cag agc aag tct cgg gat      2039
His Lys Arg Lys Lys Ser Arg Ser Arg Ser Gln Ser Lys Ser Arg Asp
465                 470                 475                 480

cac tca gat gca gcc aag aaa cac agg cat gaa agg gga cat cat agg      2087
His Ser Asp Ala Ala Lys Lys His Arg His Glu Arg Gly His His Arg
                485                 490                 495

gac agg cgt gaa cga tct cgc tcc ttt gag agg tcc cat aaa agc aag      2135
Asp Arg Arg Glu Arg Ser Arg Ser Phe Glu Arg Ser His Lys Ser Lys
            500                 505                 510

cac cat ggt ggc agt cgc tca gga cat ggc agg cac agg cgc tga          2180
His His Gly Gly Ser Arg Ser Gly His Gly Arg His Arg Arg
        515                 520                 525

ctttctcttc ctttgagcct gcatcagttc ttggttttgc ctatctacag tgtgatgtat      2240

ggactcaatc aaaaacatta aacgcaaact gattaggatt tgatttcttg aaaccctcta      2300

ggtctctaga acactgagga cagtttcttt tgaaaagaac tatgttaatt tttttgcaca 2360

ttaaaatgcc ctagcagtat ctaattaaaa accatggtca ggttcaattg tactttatta 2420

tagttgtgta ttgtttattg ctataagaac tggagcgtga attctgtaaa aatgtatctt 2480

atttttatac agataaaatt gcagacactg ttctatttaa gtggttattt gtttaaatga 2540

tggtgaatac tttcttaaca ctggtttgtc tgcatgtgta aagattttta caaggaaata 2600

aaatacaaat cttgtttttt ctaaa 2625

<210> 8
<211> 526
<212> PRT
<213> Homo sapiens

<400> 8

Met Ala Ser Gly Pro His Ser Thr Ala Thr Ala Ala Ala Ala Ala Ser
1               5                   10                  15

Ser Ala Ala Pro Ser Ala Gly Gly Ser Ser Ser Gly Thr Thr Thr Thr
            20                  25                  30

Thr Thr Thr Thr Thr Gly Gly Ile Leu Ile Gly Asp Arg Leu Tyr Ser
        35                  40                  45

Glu Val Ser Leu Thr Ile Asp His Ser Leu Ile Pro Glu Glu Arg Leu
    50                  55                  60

Ser Pro Thr Pro Ser Met Gln Asp Gly Leu Asp Leu Pro Ser Glu Thr
65                  70                  75                  80

Asp Leu Arg Ile Leu Gly Cys Glu Leu Ile Gln Ala Ala Gly Ile Leu
                85                  90                  95

Leu Arg Leu Pro Gln Val Ala Met Ala Thr Gly Gln Val Leu Phe His
            100                 105                 110

Arg Phe Phe Tyr Ser Lys Ser Phe Val Lys His Ser Phe Glu Ile Val
        115                 120                 125

Ala Met Ala Cys Ile Asn Leu Ala Ser Lys Ile Glu Glu Ala Pro Arg
    130                 135                 140

Arg Ile Arg Asp Val Ile Asn Val Phe His His Leu Arg Gln Leu Arg
145                 150                 155                 160

Gly Lys Arg Thr Pro Ser Pro Leu Ile Leu Asp Gln Asn Tyr Ile Asn
                165                 170                 175

Thr Lys Asn Gln Val Ile Lys Ala Glu Arg Arg Val Leu Lys Glu Leu
        180                 185                 190

Gly Phe Cys Val His Val Lys His Pro His Lys Ile Ile Val Met Tyr
    195                 200                 205

Leu Gln Val Leu Glu Cys Glu Arg Asn Gln Thr Leu Val Gln Thr Ala
    210                 215                 220

Trp Asn Tyr Met Asn Asp Ser Leu Arg Thr Asn Val Phe Val Arg Phe
225                 230                 235                 240

Gln Pro Glu Thr Ile Ala Cys Ala Cys Ile Tyr Leu Ala Ala Arg Ala
                245                 250                 255

Leu Gln Ile Pro Leu Pro Thr Arg Pro His Trp Phe Leu Leu Phe Gly
            260                 265                 270

Thr Thr Glu Glu Glu Ile Gln Glu Ile Cys Ile Glu Thr Leu Arg Leu
        275                 280                 285

Tyr Thr Arg Lys Lys Pro Asn Tyr Glu Leu Leu Glu Lys Glu Val Glu
    290                 295                 300

Lys Arg Lys Val Ala Leu Gln Glu Ala Lys Leu Lys Ala Lys Gly Leu
305                 310                 315                 320

Asn Pro Asp Gly Thr Pro Ala Leu Ser Thr Leu Gly Gly Phe Ser Pro
                325                 330                 335

Ala Ser Lys Pro Ser Ser Pro Arg Glu Val Lys Ala Glu Glu Lys Ser
            340                 345                 350

Pro Ile Ser Ile Asn Val Lys Thr Val Lys Lys Glu Pro Glu Asp Arg
        355                 360                 365

Gln Gln Ala Ser Lys Ser Pro Tyr Asn Gly Val Arg Lys Asp Ser Lys
    370                 375                 380

Arg Ser Arg Asn Ser Arg Ser Ala Ser Arg Ser Arg Ser Arg Thr Arg
385                 390                 395                 400

Ser Arg Ser Arg Ser His Thr Pro Arg Arg His Tyr Asn Asn Arg Arg
                405                 410                 415

Ser Arg Ser Gly Thr Tyr Ser Ser Arg Ser Arg Ser Arg Ser Arg Ser
            420                 425                 430

His Ser Glu Ser Pro Arg Arg His His Asn His Gly Ser Pro His Leu
        435                 440                 445

Lys Ala Lys His Thr Arg Asp Asp Leu Lys Ser Ser Asn Arg His Gly
    450                 455                 460

His Lys Arg Lys Lys Ser Arg Ser Arg Ser Gln Ser Lys Ser Arg Asp
465                 470                 475                 480

His Ser Asp Ala Ala Lys Lys His Arg His Glu Arg Gly His His Arg
                485                 490                 495

Asp Arg Arg Glu Arg Ser Arg Ser Phe Glu Arg Ser His Lys Ser Lys
            500                 505                 510

His His Gly Gly Ser Arg Ser Gly His Gly Arg His Arg Arg
        515                 520                 525

<210>  9
<211>  3715
<212>  DNA
<213>  Homo sapiens

<220>
<221>  CDS
<222>  (378)..(2900)

<400>  9
ctttgagcgg aagttggatc actgaagcgc caaaaaagaa tttaggggaa gacctgattt      60

tcgcttgagg catttttgcg gcgctgtgcg ctacagacac cttctggaag ctgcggtggg     120

gaaactgagt ttcccgagcc gttgagacag atggggttca gcaccgcgcg gggacgacag     180

gaaagctgcg gctgaagaga actgcggact tggcaggagt cgcctgagga gctaagatcc     240

cggagactct tagccccgtg gtgcttctct acgggaggga gggagggaga atccggccgg     300

acagatctgc gcgtatcctg gagccggccc agttgtgaac taggagagct ttgggacctc     360

tgtcccaagc aagagag atg aat gga gag tat aga ggc aga gga ttt gga        410
                   Met Asn Gly Glu Tyr Arg Gly Arg Gly Phe Gly
                   1               5                   10

cga gga aga ttt caa agc tgg aaa agg gga aga ggt ggt ggg aac ttc       458
Arg Gly Arg Phe Gln Ser Trp Lys Arg Gly Arg Gly Gly Gly Asn Phe
            15                  20                  25

tca gga aaa tgg aga gaa aga gaa cac aga cct gat ctg agt aaa acc       506
Ser Gly Lys Trp Arg Glu Arg Glu His Arg Pro Asp Leu Ser Lys Thr
        30                  35                  40

aca gga aaa cgt act tct gaa caa acc cca cag ttt ttg ctt tca aca       554
Thr Gly Lys Arg Thr Ser Glu Gln Thr Pro Gln Phe Leu Leu Ser Thr

45                  50                  55

aag acc cca cag tca atg cag tca aca ttg gat cga ttc ata cca tat      602
Lys Thr Pro Gln Ser Met Gln Ser Thr Leu Asp Arg Phe Ile Pro Tyr
60                  65                  70                  75

aaa ggc tgg aag ctt tat ttc tct gaa gtt tac agc gat agc tct cct      650
Lys Gly Trp Lys Leu Tyr Phe Ser Glu Val Tyr Ser Asp Ser Ser Pro
                80                  85                  90

ttg att gag aag att caa gca ttt gaa aaa ttt ttc aca agg cat att      698
Leu Ile Glu Lys Ile Gln Ala Phe Glu Lys Phe Phe Thr Arg His Ile
            95                  100                 105

gat ttg tat gac aag gat gaa ata gaa aga aag gga agt att ttg gta      746
Asp Leu Tyr Asp Lys Asp Glu Ile Glu Arg Lys Gly Ser Ile Leu Val
        110                 115                 120

gat ttt aaa gaa ctg aca gaa ggt ggt gaa gta act aac ttg ata cca      794
Asp Phe Lys Glu Leu Thr Glu Gly Gly Glu Val Thr Asn Leu Ile Pro
    125                 130                 135

gat ata gca act gaa cta aga gat gca cct gag aaa acc ttg gct tgc      842
Asp Ile Ala Thr Glu Leu Arg Asp Ala Pro Glu Lys Thr Leu Ala Cys
140                 145                 150                 155

atg ggt ttg gca ata cat cag gtg tta act aag gac ctt gaa agg cat      890
Met Gly Leu Ala Ile His Gln Val Leu Thr Lys Asp Leu Glu Arg His
                160                 165                 170

gca gct gag tta caa gcc cag gaa gga ttg tct aat gat gga gaa aca      938
Ala Ala Glu Leu Gln Ala Gln Glu Gly Leu Ser Asn Asp Gly Glu Thr
            175                 180                 185

atg gta aat gtg cca cat att cat gca agg gtg tac aac tat gag cct      986
Met Val Asn Val Pro His Ile His Ala Arg Val Tyr Asn Tyr Glu Pro
        190                 195                 200

ttg aca cag ctc aag aat gtc aga gca aat tac tat gga aaa tac att      1034
Leu Thr Gln Leu Lys Asn Val Arg Ala Asn Tyr Tyr Gly Lys Tyr Ile
    205                 210                 215

gct cta aga ggg aca gtg gtt cgt gtc agt aat ata aag cct ctt tgc      1082
Ala Leu Arg Gly Thr Val Val Arg Val Ser Asn Ile Lys Pro Leu Cys
220                 225                 230                 235

acc aag atg gct ttt ctt tgt gct gca tgt gga gaa att cag agc ttt      1130
Thr Lys Met Ala Phe Leu Cys Ala Ala Cys Gly Glu Ile Gln Ser Phe
                240                 245                 250

cct ctt cca gat gga aaa tac agt ctt ccc aca aag tgt cct gtg cct      1178
Pro Leu Pro Asp Gly Lys Tyr Ser Leu Pro Thr Lys Cys Pro Val Pro
            255                 260                 265

gtg tgt cga ggc agg tca ttt act gct ctc cgc agc tct cct ctc aca      1226
Val Cys Arg Gly Arg Ser Phe Thr Ala Leu Arg Ser Ser Pro Leu Thr
        270                 275                 280

gtt acg atg gac tgg cag tca atc aaa atc cag gaa ttg atg tct gat      1274
Val Thr Met Asp Trp Gln Ser Ile Lys Ile Gln Glu Leu Met Ser Asp
    285                 290                 295

gat cag aga gaa gca ggt cgg att cca cga aca ata gaa tgt gag ctt      1322

Asp Gln Arg Glu Ala Gly Arg Ile Pro Arg Thr Ile Glu Cys Glu Leu
300                 305                 310                 315

gtt cat gat ctt gtg gat agc tgt gtc ccg gga gac aca gtg act att      1370
Val His Asp Leu Val Asp Ser Cys Val Pro Gly Asp Thr Val Thr Ile
                320                 325                 330

act gga att gtc aaa gtc tca aat gcg gaa gaa ggt tct cga aat aag      1418
Thr Gly Ile Val Lys Val Ser Asn Ala Glu Glu Gly Ser Arg Asn Lys
            335                 340                 345

aat gac aag tgt atg ttc ctt ttg tat att gaa gca aat tct att agt      1466
Asn Asp Lys Cys Met Phe Leu Leu Tyr Ile Glu Ala Asn Ser Ile Ser
        350                 355                 360

aat agc aaa gga cag aaa aca aag agt tct gag gat ggg tgt aag cat      1514
Asn Ser Lys Gly Gln Lys Thr Lys Ser Ser Glu Asp Gly Cys Lys His
    365                 370                 375

gga atg ttg atg gag ttc tca ctt aaa gac ctt tat gcc atc caa gag      1562
Gly Met Leu Met Glu Phe Ser Leu Lys Asp Leu Tyr Ala Ile Gln Glu
380                 385                 390                 395

att caa gct gaa gaa aac ctg ttt aaa ctc att gtc aac tcg ctt tgc      1610
Ile Gln Ala Glu Glu Asn Leu Phe Lys Leu Ile Val Asn Ser Leu Cys
                400                 405                 410

cct gtc att ttt ggt cat gaa ctt gtt aaa gca ggt ttg gca tta gca      1658
Pro Val Ile Phe Gly His Glu Leu Val Lys Ala Gly Leu Ala Leu Ala
            415                 420                 425

ctc ttt gga gga agc cag aaa tac gca gat gac aaa aac aga att cca      1706
Leu Phe Gly Gly Ser Gln Lys Tyr Ala Asp Asp Lys Asn Arg Ile Pro
        430                 435                 440

att cgg gga gac ccc cac atc ctt gtt gtt gga gat cca ggc cta gga      1754
Ile Arg Gly Asp Pro His Ile Leu Val Val Gly Asp Pro Gly Leu Gly
    445                 450                 455

aaa agt caa atg cta cag gca gcg tgc aat gtt gcc cca cgt ggc gtg      1802
Lys Ser Gln Met Leu Gln Ala Ala Cys Asn Val Ala Pro Arg Gly Val
460                 465                 470                 475

tat gtt tgt ggt aac acc acg acc acc tct ggt ctg acg gta act ctt      1850
Tyr Val Cys Gly Asn Thr Thr Thr Thr Ser Gly Leu Thr Val Thr Leu
                480                 485                 490

tca aaa gat agt tcc tct gga gat ttt gct ttg gaa gct ggt gcc ctg      1898
Ser Lys Asp Ser Ser Ser Gly Asp Phe Ala Leu Glu Ala Gly Ala Leu
            495                 500                 505

gta ctt ggt gat caa ggt att tgt gga atc gat gaa ttt gat aag atg      1946
Val Leu Gly Asp Gln Gly Ile Cys Gly Ile Asp Glu Phe Asp Lys Met
        510                 515                 520

ggg aat caa cat caa gcc ttg ttg gaa gcc atg gag cag caa agt att      1994
Gly Asn Gln His Gln Ala Leu Leu Glu Ala Met Glu Gln Gln Ser Ile
    525                 530                 535

agt ctt gct aag gct ggt gtg gtt tgt agc ctt cct gca aga act tcc      2042
Ser Leu Ala Lys Ala Gly Val Val Cys Ser Leu Pro Ala Arg Thr Ser
540                 545                 550                 555

att att gct gct gca aat cca gtt gga gga cat tac aat aaa gcc aaa 2090
Ile Ile Ala Ala Ala Asn Pro Val Gly Gly His Tyr Asn Lys Ala Lys
                560                 565                 570

aca gtt tct gag aat tta aaa atg ggg agt gca cta cta tcc aga ttt 2138
Thr Val Ser Glu Asn Leu Lys Met Gly Ser Ala Leu Leu Ser Arg Phe
            575                 580                 585

gat ttg gtc ttt atc ctg tta gat act cca aat gag cat cat gat cac 2186
Asp Leu Val Phe Ile Leu Leu Asp Thr Pro Asn Glu His His Asp His
        590                 595                 600

tta ctc tct gaa cat gtg att gca ata aga gct gga aag cag aga acc 2234
Leu Leu Ser Glu His Val Ile Ala Ile Arg Ala Gly Lys Gln Arg Thr
    605                 610                 615

att agc agt gcc aca gta gct cgt atg aat agt caa gat tca aat act 2282
Ile Ser Ser Ala Thr Val Ala Arg Met Asn Ser Gln Asp Ser Asn Thr
620                 625                 630                 635

tcc gta ctt gaa gta gtt tct gag aag cca tta tca gaa aga cta aag 2330
Ser Val Leu Glu Val Val Ser Glu Lys Pro Leu Ser Glu Arg Leu Lys
                640                 645                 650

gtg gtt cct gga gaa aca ata gat ccc att ccc cac cag cta ttg aga 2378
Val Val Pro Gly Glu Thr Ile Asp Pro Ile Pro His Gln Leu Leu Arg
            655                 660                 665

aag tac att ggc tat gct cgg cag tat gtg tac cca agg cta tcc aca 2426
Lys Tyr Ile Gly Tyr Ala Arg Gln Tyr Val Tyr Pro Arg Leu Ser Thr
        670                 675                 680

gaa gct gct cga gtt ctt caa gat ttt tac ctt gag ctc cgg aaa cag 2474
Glu Ala Ala Arg Val Leu Gln Asp Phe Tyr Leu Glu Leu Arg Lys Gln
    685                 690                 695

agc cag agg tta aat agc tca cca atc act acc agg cag ctg gaa tct 2522
Ser Gln Arg Leu Asn Ser Ser Pro Ile Thr Thr Arg Gln Leu Glu Ser
700                 705                 710                 715

ttg att cgt ctg aca gag gca cga gca agg ttg gaa ttg aga gag gaa 2570
Leu Ile Arg Leu Thr Glu Ala Arg Ala Arg Leu Glu Leu Arg Glu Glu
                720                 725                 730

gca acc aaa gaa gac gct gag gat ata gtg gaa att atg aaa tat agc 2618
Ala Thr Lys Glu Asp Ala Glu Asp Ile Val Glu Ile Met Lys Tyr Ser
            735                 740                 745

atg cta gga act tac tct gat gaa ttt ggg aac cta gat ttt gag cga 2666
Met Leu Gly Thr Tyr Ser Asp Glu Phe Gly Asn Leu Asp Phe Glu Arg
        750                 755                 760

tcc cag cat ggt tct gga atg agc aac agg tca aca gcg aaa aga ttt 2714
Ser Gln His Gly Ser Gly Met Ser Asn Arg Ser Thr Ala Lys Arg Phe
    765                 770                 775

att tct gct ctc aac aac gtt gct gaa aga act tat aat aat ata ttt 2762
Ile Ser Ala Leu Asn Asn Val Ala Glu Arg Thr Tyr Asn Asn Ile Phe
780                 785                 790                 795

caa ttt cat caa ctt cgg cag att gcc aaa gaa cta aac att cag gtt 2810
Gln Phe His Gln Leu Arg Gln Ile Ala Lys Glu Leu Asn Ile Gln Val
                800                 805                 810

gct gat ttt gaa aat ttt att gga tca cta aat gac cag ggt tac ctc     2858
Ala Asp Phe Glu Asn Phe Ile Gly Ser Leu Asn Asp Gln Gly Tyr Leu
            815                 820                 825

ttg aaa aaa ggc cca aaa gtt tac cag ctt caa act atg taa             2900
Leu Lys Lys Gly Pro Lys Val Tyr Gln Leu Gln Thr Met
        830                 835                 840

aaggacttca ccaagttagg gcctcctggg tttattgcag attaaagcca tctcagtgaa   2960

gatatgcgtg cacgcacaga cagacagaca cacacacaca cacacacaca cacacacaca   3020

cacacacaca cagtcaaata ctgttctctg aaaaatgatg tcccaaaagt attataatag   3080

gaaaaaagca ttaaatataa taaactaatt taagaagtga taaagtctcc agatgcagta   3140

gctcacactg taatcacagt gactcaggag gctgaggtga gaggattcct tgaggccagg   3200

gttcgagacc aaccttgggc aacatagcaa gaccccattt cttaaaaaaa aaaaaaaaaa   3260

atttaaactt agctgggtat ggtggcacat gcctatagtc tcagctactt gtgaggctga   3320

ggcaggagga ttctttgagc ccaggagttt gaggttacag tgagccacaa tcacaccaat   3380

cactgcactc cagcctgggc aataaagtaa ctcttgactc aaaaaaataa aaaaaattgt   3440

agtggtagcc atgtgttaat tgttaaataa attctccaaa gggctaaaag taaattactt   3500

ataaattttt tatagttgta tttttgacct gccttttata tgtatgaata tttcatagtt   3560

ttgcatatca gatgtaggca tacagacaaa tacataaacc aatgaatata ttacatattc   3620

tgtgttccaa taaaacttta tttatggaca ctaaaatttg aatttcataa aattttccca   3680

tgtcaagaat acaaaatact tgagttttgt tttta                              3715

<210>  10
<211>  840
<212>  PRT
<213>  Homo sapiens

<400>  10

Met Asn Gly Glu Tyr Arg Gly Arg Gly Phe Gly Arg Gly Arg Phe Gln
1               5                   10                  15

Ser Trp Lys Arg Gly Arg Gly Gly Gly Asn Phe Ser Gly Lys Trp Arg
            20                  25                  30

Glu Arg Glu His Arg Pro Asp Leu Ser Lys Thr Thr Gly Lys Arg Thr
        35                  40                  45

Ser Glu Gln Thr Pro Gln Phe Leu Leu Ser Thr Lys Thr Pro Gln Ser
    50                  55                  60

Met Gln Ser Thr Leu Asp Arg Phe Ile Pro Tyr Lys Gly Trp Lys Leu
65                  70                  75                  80

Tyr Phe Ser Glu Val Tyr Ser Asp Ser Ser Pro Leu Ile Glu Lys Ile
                85                  90                  95

Gln Ala Phe Glu Lys Phe Phe Thr Arg His Ile Asp Leu Tyr Asp Lys
            100                 105                 110

Asp Glu Ile Glu Arg Lys Gly Ser Ile Leu Val Asp Phe Lys Glu Leu
        115                 120                 125

Thr Glu Gly Gly Glu Val Thr Asn Leu Ile Pro Asp Ile Ala Thr Glu
    130                 135                 140

Leu Arg Asp Ala Pro Glu Lys Thr Leu Ala Cys Met Gly Leu Ala Ile
145                 150                 155                 160

His Gln Val Leu Thr Lys Asp Leu Glu Arg His Ala Ala Glu Leu Gln
                165                 170                 175

Ala Gln Glu Gly Leu Ser Asn Asp Gly Glu Thr Met Val Asn Val Pro
            180                 185                 190

His Ile His Ala Arg Val Tyr Asn Tyr Glu Pro Leu Thr Gln Leu Lys
        195                 200                 205

Asn Val Arg Ala Asn Tyr Tyr Gly Lys Tyr Ile Ala Leu Arg Gly Thr
    210                 215                 220

Val Val Arg Val Ser Asn Ile Lys Pro Leu Cys Thr Lys Met Ala Phe
225                 230                 235                 240

Leu Cys Ala Ala Cys Gly Glu Ile Gln Ser Phe Pro Leu Pro Asp Gly
                245                 250                 255

Lys Tyr Ser Leu Pro Thr Lys Cys Pro Val Pro Val Cys Arg Gly Arg
            260                 265                 270

Ser Phe Thr Ala Leu Arg Ser Ser Pro Leu Thr Val Thr Met Asp Trp
        275                 280                 285

Gln Ser Ile Lys Ile Gln Glu Leu Met Ser Asp Asp Gln Arg Glu Ala
    290                 295                 300

Gly Arg Ile Pro Arg Thr Ile Glu Cys Glu Leu Val His Asp Leu Val
305                 310                 315                 320

Asp Ser Cys Val Pro Gly Asp Thr Val Thr Ile Thr Gly Ile Val Lys

                325                 330                 335

Val Ser Asn Ala Glu Glu Gly Ser Arg Asn Lys Asn Asp Lys Cys Met
            340                 345                 350

Phe Leu Leu Tyr Ile Glu Ala Asn Ser Ile Ser Asn Ser Lys Gly Gln
        355                 360                 365

Lys Thr Lys Ser Ser Glu Asp Gly Cys Lys His Gly Met Leu Met Glu
    370                 375                 380

Phe Ser Leu Lys Asp Leu Tyr Ala Ile Gln Glu Ile Gln Ala Glu Glu
385                 390                 395                 400

Asn Leu Phe Lys Leu Ile Val Asn Ser Leu Cys Pro Val Ile Phe Gly
                405                 410                 415

His Glu Leu Val Lys Ala Gly Leu Ala Leu Ala Leu Phe Gly Gly Ser
            420                 425                 430

Gln Lys Tyr Ala Asp Asp Lys Asn Arg Ile Pro Ile Arg Gly Asp Pro
        435                 440                 445

His Ile Leu Val Val Gly Asp Pro Gly Leu Gly Lys Ser Gln Met Leu
    450                 455                 460

Gln Ala Ala Cys Asn Val Ala Pro Arg Gly Val Tyr Val Cys Gly Asn
465                 470                 475                 480

Thr Thr Thr Thr Ser Gly Leu Thr Val Thr Leu Ser Lys Asp Ser Ser
                485                 490                 495

Ser Gly Asp Phe Ala Leu Glu Ala Gly Ala Leu Val Leu Gly Asp Gln
            500                 505                 510

Gly Ile Cys Gly Ile Asp Glu Phe Asp Lys Met Gly Asn Gln His Gln
        515                 520                 525

Ala Leu Leu Glu Ala Met Glu Gln Gln Ser Ile Ser Leu Ala Lys Ala
    530                 535                 540

Gly Val Val Cys Ser Leu Pro Ala Arg Thr Ser Ile Ile Ala Ala Ala
545                 550                 555                 560

Asn Pro Val Gly Gly His Tyr Asn Lys Ala Lys Thr Val Ser Glu Asn
                565                 570                 575

Leu Lys Met Gly Ser Ala Leu Leu Ser Arg Phe Asp Leu Val Phe Ile
                580                 585                 590

Leu Leu Asp Thr Pro Asn Glu His His Asp His Leu Leu Ser Glu His
        595                 600                 605

Val Ile Ala Ile Arg Ala Gly Lys Gln Arg Thr Ile Ser Ser Ala Thr
    610                 615                 620

Val Ala Arg Met Asn Ser Gln Asp Ser Asn Thr Ser Val Leu Glu Val
625                 630                 635                 640

Val Ser Glu Lys Pro Leu Ser Glu Arg Leu Lys Val Val Pro Gly Glu
                645                 650                 655

Thr Ile Asp Pro Ile Pro His Gln Leu Leu Arg Lys Tyr Ile Gly Tyr
            660                 665                 670

Ala Arg Gln Tyr Val Tyr Pro Arg Leu Ser Thr Glu Ala Ala Arg Val
        675                 680                 685

Leu Gln Asp Phe Tyr Leu Glu Leu Arg Lys Gln Ser Gln Arg Leu Asn
    690                 695                 700

Ser Ser Pro Ile Thr Thr Arg Gln Leu Glu Ser Leu Ile Arg Leu Thr
705                 710                 715                 720

Glu Ala Arg Ala Arg Leu Glu Leu Arg Glu Glu Ala Thr Lys Glu Asp
                725                 730                 735

Ala Glu Asp Ile Val Glu Ile Met Lys Tyr Ser Met Leu Gly Thr Tyr
            740                 745                 750

Ser Asp Glu Phe Gly Asn Leu Asp Phe Glu Arg Ser Gln His Gly Ser
        755                 760                 765

Gly Met Ser Asn Arg Ser Thr Ala Lys Arg Phe Ile Ser Ala Leu Asn
    770                 775                 780

Asn Val Ala Glu Arg Thr Tyr Asn Asn Ile Phe Gln Phe His Gln Leu
785                 790                 795                 800

Arg Gln Ile Ala Lys Glu Leu Asn Ile Gln Val Ala Asp Phe Glu Asn
                805                 810                 815

Phe Ile Gly Ser Leu Asn Asp Gln Gly Tyr Leu Leu Lys Lys Gly Pro
            820                 825                 830

Lys Val Tyr Gln Leu Gln Thr Met
        835                 840

<210>  11
<211>  1212
<212>  DNA
<213>  Homo sapiens

<220>
<221>  CDS
<222>  (115)..(990)

<400>  11
aggtgtctgg aactggatta cagtttcttc tgattgaacg cagcctttga atactgcctg      60

gcctatcgcg aatacatccc tgccttggac aagacgcagc tgaatctcta agtg atg      117
                                                            Met
                                                            1

cta ctg cca cta cca ccc cag agc tcc aaa cct gtg cct aag aaa tct      165
Leu Leu Pro Leu Pro Pro Gln Ser Ser Lys Pro Val Pro Lys Lys Ser
            5                   10                  15

cag tcc agc aaa att gtg ccc agt cat cat gac cca tct gaa aag acg      213
Gln Ser Ser Lys Ile Val Pro Ser His His Asp Pro Ser Glu Lys Thr
        20                  25                  30

ggg gag aat tgc caa acg aag ata tct cca tct tca ctt cag gag tct      261
Gly Glu Asn Cys Gln Thr Lys Ile Ser Pro Ser Ser Leu Gln Glu Ser
    35                  40                  45

cca tct tca ctt cag gga gca ctc aaa aag aga tca gct ttt gaa gat      309
Pro Ser Ser Leu Gln Gly Ala Leu Lys Lys Arg Ser Ala Phe Glu Asp
50                  55                  60                  65

ctc act aat gtg tcc ttt gag atg acc cat gag acc ctg tac ttg gca      357
Leu Thr Asn Val Ser Phe Glu Met Thr His Glu Thr Leu Tyr Leu Ala
                70                  75                  80

gtg aag ctg gtg gat ctc tac cta atg aag gca gta tgc aag aag gat      405
Val Lys Leu Val Asp Leu Tyr Leu Met Lys Ala Val Cys Lys Lys Asp
            85                  90                  95

aag tta caa ctc ctt ggt gcc act gcc ttt atg att gca gca aaa ttt      453
Lys Leu Gln Leu Leu Gly Ala Thr Ala Phe Met Ile Ala Ala Lys Phe
        100                 105                 110

gag gag cac aac tca cct cgt gtg gat gac ttt gtg tac atc tgt gat      501
Glu Glu His Asn Ser Pro Arg Val Asp Asp Phe Val Tyr Ile Cys Asp
    115                 120                 125

gat aat tat cag cga tct gag gta ctc agc atg gaa atc aac atc ctg      549
Asp Asn Tyr Gln Arg Ser Glu Val Leu Ser Met Glu Ile Asn Ile Leu
130                 135                 140                 145

aac gtc ctc aaa tgt gac att aac att ccc atc gcc tac cat ttt ctg      597
Asn Val Leu Lys Cys Asp Ile Asn Ile Pro Ile Ala Tyr His Phe Leu
                150                 155                 160

cgc aga tat gct agg tgt atc cac acc aac atg aag aca ctg acc ttg      645

Arg Arg Tyr Ala Arg Cys Ile His Thr Asn Met Lys Thr Leu Thr Leu
            165                 170                 175

tcc cgc tac atc tgc gag atg acc ctg cag gaa tac cac tat gtc cag 693
Ser Arg Tyr Ile Cys Glu Met Thr Leu Gln Glu Tyr His Tyr Val Gln
        180                 185                 190

gag aag gct tcc aag cta gct gct gcc tcc tta ctc ctg gcc ctc tac 741
Glu Lys Ala Ser Lys Leu Ala Ala Ala Ser Leu Leu Leu Ala Leu Tyr
    195                 200                 205

atg aag aag ctc gga tac tgg gtt ccc ttc ctg gag cat tac agt ggc 789
Met Lys Lys Leu Gly Tyr Trp Val Pro Phe Leu Glu His Tyr Ser Gly
210                 215                 220                 225

tac agt atc tct gag ctt cac ccc ttg gtc aga cag ctg aac aaa ctg 837
Tyr Ser Ile Ser Glu Leu His Pro Leu Val Arg Gln Leu Asn Lys Leu
                230                 235                 240

ctg act ttc agt tct tac gat agt ctc aag gct gtg tat tac aag tat 885
Leu Thr Phe Ser Ser Tyr Asp Ser Leu Lys Ala Val Tyr Tyr Lys Tyr
            245                 250                 255

tct cac ccg gtc ttc ttt gaa gtc gcc aaa atc cct gcc ttg gat atg 933
Ser His Pro Val Phe Phe Glu Val Ala Lys Ile Pro Ala Leu Asp Met
        260                 265                 270

ttg aag ctg gag gag att ttg aac tgt gat tgt gag gct cag ggc ctg 981
Leu Lys Leu Glu Glu Ile Leu Asn Cys Asp Cys Glu Ala Gln Gly Leu
    275                 280                 285

gta ctc tag cagcagccac agggctaagc atgcatgtta acagggtata 1030
Val Leu
290

tttattctat gttcgaattt gtcttttgat cgcttttatt catttttcct ttctttgtct 1090

tttcccaaac tgataatgtt ataaatattt atgttgcttg tttttatgaa agaaaaaata 1150

ttgtcatatt tgactacaaa tttaataaaa aattaatggt tattgttaaa aaaaaaaaaa 1210

aa 1212

<210> 12
<211> 291
<212> PRT
<213> Homo sapiens

<400> 12

Met Leu Leu Pro Leu Pro Pro Gln Ser Ser Lys Pro Val Pro Lys Lys
1               5                   10                  15

Ser Gln Ser Ser Lys Ile Val Pro Ser His His Asp Pro Ser Glu Lys
            20                  25                  30

Thr Gly Glu Asn Cys Gln Thr Lys Ile Ser Pro Ser Ser Leu Gln Glu
        35                  40                  45

Ser Pro Ser Ser Leu Gln Gly Ala Leu Lys Lys Arg Ser Ala Phe Glu
    50                  55                  60

Asp Leu Thr Asn Val Ser Phe Glu Met Thr His Glu Thr Leu Tyr Leu
65                  70                  75                  80

Ala Val Lys Leu Val Asp Leu Tyr Leu Met Lys Ala Val Cys Lys Lys
                85                  90                  95

Asp Lys Leu Gln Leu Leu Gly Ala Thr Ala Phe Met Ile Ala Ala Lys
            100                 105                 110

Phe Glu Glu His Asn Ser Pro Arg Val Asp Asp Phe Val Tyr Ile Cys
        115                 120                 125

Asp Asp Asn Tyr Gln Arg Ser Glu Val Leu Ser Met Glu Ile Asn Ile
    130                 135                 140

Leu Asn Val Leu Lys Cys Asp Ile Asn Ile Pro Ile Ala Tyr His Phe
145                 150                 155                 160

Leu Arg Arg Tyr Ala Arg Cys Ile His Thr Asn Met Lys Thr Leu Thr
                165                 170                 175

Leu Ser Arg Tyr Ile Cys Glu Met Thr Leu Gln Glu Tyr His Tyr Val
            180                 185                 190

Gln Glu Lys Ala Ser Lys Leu Ala Ala Ala Ser Leu Leu Leu Ala Leu
        195                 200                 205

Tyr Met Lys Lys Leu Gly Tyr Trp Val Pro Phe Leu Glu His Tyr Ser
    210                 215                 220

Gly Tyr Ser Ile Ser Glu Leu His Pro Leu Val Arg Gln Leu Asn Lys
225                 230                 235                 240

Leu Leu Thr Phe Ser Ser Tyr Asp Ser Leu Lys Ala Val Tyr Tyr Lys
                245                 250                 255

Tyr Ser His Pro Val Phe Phe Glu Val Ala Lys Ile Pro Ala Leu Asp
            260                 265                 270

Met Leu Lys Leu Glu Glu Ile Leu Asn Cys Asp Cys Glu Ala Gln Gly
        275                 280                 285

Leu Val Leu
    290

<210>  13
<211>  4822
<212>  DNA
<213>  Homo sapiens

<220>
<221>  CDS
<222>  (16)..(3447)

<400>  13
ttacctagat tcaag atg aat agc gat caa gtt aca ctg gtt ggt caa gtg      51
                 Met Asn Ser Asp Gln Val Thr Leu Val Gly Gln Val
                 1               5                   10

ttt gag tca tat gtt tcg gaa tac cat aag aat gat att ctt cta atc      99
Phe Glu Ser Tyr Val Ser Glu Tyr His Lys Asn Asp Ile Leu Leu Ile
        15                  20                  25

ttg aag gaa agg gat gaa gat gct cat tac cca gtt gtg gtt aat gcc      147
Leu Lys Glu Arg Asp Glu Asp Ala His Tyr Pro Val Val Val Asn Ala
    30                  35                  40

atg act ctg ttt gag acc aac atg gaa atc ggg gaa tat ttc aac atg      195
Met Thr Leu Phe Glu Thr Asn Met Glu Ile Gly Glu Tyr Phe Asn Met
45                  50                  55                  60

ttc ccc agt gaa gtg ctt aca att ttt gat agt gca ctg cga agg tca      243
Phe Pro Ser Glu Val Leu Thr Ile Phe Asp Ser Ala Leu Arg Arg Ser
                65                  70                  75

gcc ttg aca att ctc cag tcc ctt tct cag cct gag gct gtt tcc atg      291
Ala Leu Thr Ile Leu Gln Ser Leu Ser Gln Pro Glu Ala Val Ser Met
            80                  85                  90

aaa cag aat ctt cat gcc agg ata tca ggt ttg cct gtc tgt cct gag      339
Lys Gln Asn Leu His Ala Arg Ile Ser Gly Leu Pro Val Cys Pro Glu
        95                  100                 105

ctg gtg agg gaa cac ata cct aaa acc aag gat gtg gga cac ttt tta      387
Leu Val Arg Glu His Ile Pro Lys Thr Lys Asp Val Gly His Phe Leu
    110                 115                 120

tct gtc act ggg aca gtg att cga aca agt ctg gtg aag gtt ctg gag      435
Ser Val Thr Gly Thr Val Ile Arg Thr Ser Leu Val Lys Val Leu Glu
125                 130                 135                 140

ttt gag cgg gat tac atg tgt aac aaa tgc aag cat gtg ttt gtg atc      483
Phe Glu Arg Asp Tyr Met Cys Asn Lys Cys Lys His Val Phe Val Ile
                145                 150                 155

aag gct gac ttt gag cag tat tac acc ttt tgc cgg cca tcc tcg tgt      531
Lys Ala Asp Phe Glu Gln Tyr Tyr Thr Phe Cys Arg Pro Ser Ser Cys
            160                 165                 170

ccc agc ttg gag agc tgt gat tcc tct aaa ttc act tgc ctc tca ggc      579
Pro Ser Leu Glu Ser Cys Asp Ser Ser Lys Phe Thr Cys Leu Ser Gly
        175                 180                 185

ttg tct tcg tct cca acc agg tgt aga gat tac cag gaa atc aaa att      627
Leu Ser Ser Ser Pro Thr Arg Cys Arg Asp Tyr Gln Glu Ile Lys Ile
    190                 195                 200

cag gaa cag gtt caa agg cta tct gtt gga agt att cca cga tct atg 675
Gln Glu Gln Val Gln Arg Leu Ser Val Gly Ser Ile Pro Arg Ser Met
205                 210                 215                 220

aag gtt att ctg gaa gat gac tta gtg gat agt tgc aaa tct ggt gat 723
Lys Val Ile Leu Glu Asp Asp Leu Val Asp Ser Cys Lys Ser Gly Asp
                225                 230                 235

gac ctc act att tac ggg att gta atg caa cgg tgg aag ccc ttt cag 771
Asp Leu Thr Ile Tyr Gly Ile Val Met Gln Arg Trp Lys Pro Phe Gln
            240                 245                 250

caa gat gtg cgc tgt gaa gtg gag ata gtc ctg aaa gca aat tac atc 819
Gln Asp Val Arg Cys Glu Val Glu Ile Val Leu Lys Ala Asn Tyr Ile
        255                 260                 265

caa gta aat aat gag cag tcc tca ggg atc atc atg gat gag gag gtc 867
Gln Val Asn Asn Glu Gln Ser Ser Gly Ile Ile Met Asp Glu Glu Val
    270                 275                 280

caa aag gaa ttc gaa gat ttt tgg gaa tac tat aag agc gat ccc ttt 915
Gln Lys Glu Phe Glu Asp Phe Trp Glu Tyr Tyr Lys Ser Asp Pro Phe
285                 290                 295                 300

gca gga agg aat gta ata ttg gct agc ttg tgc cct caa gtg ttt gga 963
Ala Gly Arg Asn Val Ile Leu Ala Ser Leu Cys Pro Gln Val Phe Gly
                305                 310                 315

atg tat cta gta aag ctt gct gtg gcc atg gtg ctg gct ggt ggg att 1011
Met Tyr Leu Val Lys Leu Ala Val Ala Met Val Leu Ala Gly Gly Ile
            320                 325                 330

caa agg act gat gct aca gga aca cgg gtc aga gga gaa tct cat ctt 1059
Gln Arg Thr Asp Ala Thr Gly Thr Arg Val Arg Gly Glu Ser His Leu
        335                 340                 345

tta ttg gtt ggg gat cct ggc aca ggg aaa tct cag ttc ctc aaa tat 1107
Leu Leu Val Gly Asp Pro Gly Thr Gly Lys Ser Gln Phe Leu Lys Tyr
    350                 355                 360

gca gca aag att aca cca aga tct gtg ctg acc aca gga att gga tct 1155
Ala Ala Lys Ile Thr Pro Arg Ser Val Leu Thr Thr Gly Ile Gly Ser
365                 370                 375                 380

act agt gca ggt ctg acg gta act gct gta aaa gac tca gga gaa tgg 1203
Thr Ser Ala Gly Leu Thr Val Thr Ala Val Lys Asp Ser Gly Glu Trp
                385                 390                 395

aat ttg gag gct ggg gca tta gtt ctt gca gat gcg ggc ctt tgc tgt 1251
Asn Leu Glu Ala Gly Ala Leu Val Leu Ala Asp Ala Gly Leu Cys Cys
            400                 405                 410

att gat gag ttc aat agc ctc aaa gag cat gat agg acc agt atc cat 1299
Ile Asp Glu Phe Asn Ser Leu Lys Glu His Asp Arg Thr Ser Ile His
        415                 420                 425

gaa gca atg gag caa caa acc ata agt gtt gct aag gct ggc ctc gtg 1347
Glu Ala Met Glu Gln Gln Thr Ile Ser Val Ala Lys Ala Gly Leu Val
    430                 435                 440

tgc aag ctg aac aca agg acc acc atc ctg gca gca acg aac ccc aaa 1395
Cys Lys Leu Asn Thr Arg Thr Thr Ile Leu Ala Ala Thr Asn Pro Lys

445                 450                 455                 460

ggc cag tac gac ccc cag gag tcc gtg tct gtg aac att gcc ctc ggc      1443
Gly Gln Tyr Asp Pro Gln Glu Ser Val Ser Val Asn Ile Ala Leu Gly
                465                 470                 475

agc cca ctc tta agt cga ttt gac ctg atc ctg gtt ttg ctt gat acc      1491
Ser Pro Leu Leu Ser Arg Phe Asp Leu Ile Leu Val Leu Leu Asp Thr
            480                 485                 490

aag aat gaa gac tgg gat cgt atc att tcc tcc ttt atc tta gaa aat      1539
Lys Asn Glu Asp Trp Asp Arg Ile Ile Ser Ser Phe Ile Leu Glu Asn
        495                 500                 505

aaa ggt tac cca agc aaa tca gag aag ctc tgg agc atg gaa aag atg      1587
Lys Gly Tyr Pro Ser Lys Ser Glu Lys Leu Trp Ser Met Glu Lys Met
    510                 515                 520

aaa acc tat ttc tgc ctc ata agg aat ctg cag ccc aca ctg tct gat      1635
Lys Thr Tyr Phe Cys Leu Ile Arg Asn Leu Gln Pro Thr Leu Ser Asp
525                 530                 535                 540

gtg ggc aat cag gtt ctt ctc cgg tac tac cag atg caa agg cag agt      1683
Val Gly Asn Gln Val Leu Leu Arg Tyr Tyr Gln Met Gln Arg Gln Ser
                545                 550                 555

gat tgc cgg aac gct gcc cgg acc acc att cgg ctg ttg gaa agc ttg      1731
Asp Cys Arg Asn Ala Ala Arg Thr Thr Ile Arg Leu Leu Glu Ser Leu
            560                 565                 570

ata cga tta gca gaa gct cat gct cgc ctg atg ttt cgt gat act gta      1779
Ile Arg Leu Ala Glu Ala His Ala Arg Leu Met Phe Arg Asp Thr Val
        575                 580                 585

act ctg gaa gac gct att acg gtg gtg tca gtc atg gag tcc tca atg      1827
Thr Leu Glu Asp Ala Ile Thr Val Val Ser Val Met Glu Ser Ser Met
    590                 595                 600

cag gga ggt gca ctg cta gga ggt gtg aat gcc ctc cac act tcc ttt      1875
Gln Gly Gly Ala Leu Leu Gly Gly Val Asn Ala Leu His Thr Ser Phe
605                 610                 615                 620

cct gaa aac cct gga gag cag tac cag aga cag tgt gaa ctt att ctg      1923
Pro Glu Asn Pro Gly Glu Gln Tyr Gln Arg Gln Cys Glu Leu Ile Leu
                625                 630                 635

gaa aag cta gag ctg cag agc ctc ttg agt gaa gag ctt aga aga ctt      1971
Glu Lys Leu Glu Leu Gln Ser Leu Leu Ser Glu Glu Leu Arg Arg Leu
            640                 645                 650

gaa agg tta cag aat cag agt gtg cac caa tcc caa cca cgg gta ttg      2019
Glu Arg Leu Gln Asn Gln Ser Val His Gln Ser Gln Pro Arg Val Leu
        655                 660                 665

gag gta gag act act cca gga tcc ttg aga aat ggt cca ggg gaa gaa      2067
Glu Val Glu Thr Thr Pro Gly Ser Leu Arg Asn Gly Pro Gly Glu Glu
    670                 675                 680

tca aac ttc aga act tca tca cag cag gaa atc aac tat agc aca cat      2115
Ser Asn Phe Arg Thr Ser Ser Gln Gln Glu Ile Asn Tyr Ser Thr His
685                 690                 695                 700

atc ttc tct cct gga ggc agc ccc gag gga agc cca gtt cta gat ccc      2163

Ile Phe Ser Pro Gly Gly Ser Pro Glu Gly Ser Pro Val Leu Asp Pro
                705                 710                 715

cca ccg cat ctg gag cct aat aga tca aca agt agg aaa cat tca gct 2211
Pro Pro His Leu Glu Pro Asn Arg Ser Thr Ser Arg Lys His Ser Ala
            720                 725                 730

cag cac aaa aat aac aga gat gac agt tta gat tgg ttt gat ttc atg 2259
Gln His Lys Asn Asn Arg Asp Asp Ser Leu Asp Trp Phe Asp Phe Met
        735                 740                 745

gca act cat cag agt gaa cct aaa aac act gtt gtt gtg tct cct cat 2307
Ala Thr His Gln Ser Glu Pro Lys Asn Thr Val Val Val Ser Pro His
    750                 755                 760

ccc aaa aca tct gga gaa aat atg gct tcg aag atc tct aac agc aca 2355
Pro Lys Thr Ser Gly Glu Asn Met Ala Ser Lys Ile Ser Asn Ser Thr
765                 770                 775                 780

tct cag ggt aag gag aag agt gag cca ggc caa agg agc aaa gtg gac 2403
Ser Gln Gly Lys Glu Lys Ser Glu Pro Gly Gln Arg Ser Lys Val Asp
                785                 790                 795

att ggg ttg ctt cca tca cca gga gag aca ggt gtt cca tgg agg gca 2451
Ile Gly Leu Leu Pro Ser Pro Gly Glu Thr Gly Val Pro Trp Arg Ala
            800                 805                 810

gac aat gtg gaa agt aac aag aaa aaa agg cta gca cta gat tct gaa 2499
Asp Asn Val Glu Ser Asn Lys Lys Lys Arg Leu Ala Leu Asp Ser Glu
        815                 820                 825

gca gca gtc tct gct gat aaa cca gac tca gta ctg act cat cat gtc 2547
Ala Ala Val Ser Ala Asp Lys Pro Asp Ser Val Leu Thr His His Val
    830                 835                 840

ccc agg aac ctg cag aag ctg tgc aaa gag agg gcc cag aag ttg tgc 2595
Pro Arg Asn Leu Gln Lys Leu Cys Lys Glu Arg Ala Gln Lys Leu Cys
845                 850                 855                 860

aga aat agc acc agg gtg cct gca cag tgc aca gtc cct tcc cat cct 2643
Arg Asn Ser Thr Arg Val Pro Ala Gln Cys Thr Val Pro Ser His Pro
                865                 870                 875

cag tcc act cct gta cat agc cca gac aga atg ctg gac tca ccc aaa 2691
Gln Ser Thr Pro Val His Ser Pro Asp Arg Met Leu Asp Ser Pro Lys
            880                 885                 890

aga aag aga ccg aaa tcc ctt gcg caa gtg gaa gag cct gca att gaa 2739
Arg Lys Arg Pro Lys Ser Leu Ala Gln Val Glu Glu Pro Ala Ile Glu
        895                 900                 905

aat gtt aag cct cca ggt tcc cct gtg gcc aaa ctg gca aaa ttt act 2787
Asn Val Lys Pro Pro Gly Ser Pro Val Ala Lys Leu Ala Lys Phe Thr
    910                 915                 920

ttc aag cag aag tca aaa ctg atc cac tcc ttt gaa gat cac agc cat 2835
Phe Lys Gln Lys Ser Lys Leu Ile His Ser Phe Glu Asp His Ser His
925                 930                 935                 940

gtg tca cct ggt gca act aaa ata gca gtt cat agt cct aaa att tcc 2883
Val Ser Pro Gly Ala Thr Lys Ile Ala Val His Ser Pro Lys Ile Ser
                945                 950                 955

cag cgt aga aca aga aga gac gca gcc ttg ccg gtg aag cgt cca gga 2931
Gln Arg Arg Thr Arg Arg Asp Ala Ala Leu Pro Val Lys Arg Pro Gly
            960                 965                 970

aag tta aca tct acc cca gga aac cag atc tcc agt cag cca cag ggt 2979
Lys Leu Thr Ser Thr Pro Gly Asn Gln Ile Ser Ser Gln Pro Gln Gly
        975                 980                 985

gag aca aag gag gtg tcg cag cag cca cca gag aaa cac gga cca aga 3027
Glu Thr Lys Glu Val Ser Gln Gln Pro Pro Glu Lys His Gly Pro Arg
    990                 995                 1000

gag aag gtg atg tgt gcc cct gag aag agg att att cag cct gaa 3072
Glu Lys Val Met Cys Ala Pro Glu Lys Arg Ile Ile Gln Pro Glu
1005                1010                1015

tta gag ctt ggg aac gag act ggg tgt gct cat ctt act tgt gag 3117
Leu Glu Leu Gly Asn Glu Thr Gly Cys Ala His Leu Thr Cys Glu
1020                1025                1030

gga gac aaa aag gaa gag gtt tca ggc agt aat aaa agc ggc aag 3162
Gly Asp Lys Lys Glu Glu Val Ser Gly Ser Asn Lys Ser Gly Lys
1035                1040                1045

gtt cat gcc tgc aca tta gcc aga ttg gca aac ttc tgc ttt act 3207
Val His Ala Cys Thr Leu Ala Arg Leu Ala Asn Phe Cys Phe Thr
1050                1055                1060

ccc cca tcg gaa tcc aaa tca aaa tcc cct cct cct gaa agg aag 3252
Pro Pro Ser Glu Ser Lys Ser Lys Ser Pro Pro Pro Glu Arg Lys
1065                1070                1075

aac cga ggt gag aga ggc cca agc tcc cct cct aca acc aca gct 3297
Asn Arg Gly Glu Arg Gly Pro Ser Ser Pro Pro Thr Thr Thr Ala
1080                1085                1090

cca atg cgt gtc agt aaa agg aaa tct ttt cag ctc cgt ggg tcc 3342
Pro Met Arg Val Ser Lys Arg Lys Ser Phe Gln Leu Arg Gly Ser
1095                1100                1105

acc gag aaa ctg att gtt tcc aaa gaa tcc ctc ttc act tta cca 3387
Thr Glu Lys Leu Ile Val Ser Lys Glu Ser Leu Phe Thr Leu Pro
1110                1115                1120

gaa cta ggt gat gaa gca ttt gat tgt gac tgg gat gaa gag atg 3432
Glu Leu Gly Asp Glu Ala Phe Asp Cys Asp Trp Asp Glu Glu Met
1125                1130                1135

aga aaa aag tca tag ttgggaaaag ctttctggtc aaatctcacc ttcttcaact 3487
Arg Lys Lys Ser
1140

ccacagagga ccttcaggat atcaatatgg tatttataaa tgtatagaac aattggccat 3547

attgaggatc actctgaata ctggctcccc cttaaggctt tctaatttca ggttaatctt 3607

catgacttaa aaagttgtat aatcagttga ggtcagtgtg ataccagcag ctgagctgaa 3667

ttaattatgt tgtgcttaat tttacaaatg gagtacttgt attcctgttc ctgaagctgt 3727

ttctgttttt tgttttgttt tgtttttaag ggggagaggt tcttccctag attaatttct 3787

tctttcattc actcaggaac aaatgtcaag aaggtagcac tcataaatct aacaaggcag 3847

atgaactctt ttctactttt tttttttttt tttgtatttt cacctggaat gggctaagta 3907

cagtgaatat aatcacttgg atgatttgcc aaaatcagac tatttttcta gtattatttt 3967

tgtattgatt tgtgtggatc aggttaaatg tgactaatgc ttttctttct ttgagaggta 4027

tccttacaat tccatgatgt tcttagagat ctggccactg gtcaaacagt acctttctga 4087

agtactgacc ttctgagttg tcctttcttt cttgagccaa cattttgtac ttcagattct 4147

tttttctctt gggggcctac cttcaaccaa gtaaaatact gtgattagaa gaagagagag 4207

tatgagccag gcacagtgac tcactcctgt agtcctagct actcggggag gctgaggcag 4267

gatgattgct taagcccagg agttcggggt tacagtgagc tgtggtcaca ccactgtatt 4327

ccagcctggg tgacagagtg agatcctgtc tctaaaaata aaataaatga agaagagaga 4387

ctatgtggta gtctcaatca aacatcatgt ctcatctacc cagctggtta atatggaaat 4447

gtgattccta ctaagttgtg attcacttgt ctttaaaacc aaggaaatta tatactgttt 4507

ttggtcagaa tagataacct caagctttgt ctttctatgt gcttttaaaa tcacttattc 4567

ctttggattc taataagggt tattaggatt cagtaattat ggactttctc ttctgaagtg 4627

tgaatttgta aactgattgt ttaattgtca gagggacttt tggacataga atactcaaaa 4687

ctatatgtat tttgtttaat tttcacttca ttcaattcac gcattgaaaa caaatttaga 4747

aaatccaatt tttcttaagc atttaaacgt tctaaaattc actaataaaa ttttctgaaa 4807

aaatttaaac tgtga 4822

<210> 14
<211> 1143
<212> PRT
<213> Homo sapiens

<400> 14

Met Asn Ser Asp Gln Val Thr Leu Val Gly Gln Val Phe Glu Ser Tyr
1               5                   10                  15

Val Ser Glu Tyr His Lys Asn Asp Ile Leu Leu Ile Leu Lys Glu Arg
            20                  25                  30

Asp Glu Asp Ala His Tyr Pro Val Val Val Asn Ala Met Thr Leu Phe
        35                  40                  45

Glu Thr Asn Met Glu Ile Gly Glu Tyr Phe Asn Met Phe Pro Ser Glu
    50                  55                  60

Val Leu Thr Ile Phe Asp Ser Ala Leu Arg Arg Ser Ala Leu Thr Ile
65                  70                  75                  80

Leu Gln Ser Leu Ser Gln Pro Glu Ala Val Ser Met Lys Gln Asn Leu
                85                  90                  95

His Ala Arg Ile Ser Gly Leu Pro Val Cys Pro Glu Leu Val Arg Glu
            100                 105                 110

His Ile Pro Lys Thr Lys Asp Val Gly His Phe Leu Ser Val Thr Gly
        115                 120                 125

Thr Val Ile Arg Thr Ser Leu Val Lys Val Leu Glu Phe Glu Arg Asp
    130                 135                 140

Tyr Met Cys Asn Lys Cys Lys His Val Phe Val Ile Lys Ala Asp Phe
145                 150                 155                 160

Glu Gln Tyr Tyr Thr Phe Cys Arg Pro Ser Ser Cys Pro Ser Leu Glu
                165                 170                 175

Ser Cys Asp Ser Ser Lys Phe Thr Cys Leu Ser Gly Leu Ser Ser Ser
            180                 185                 190

Pro Thr Arg Cys Arg Asp Tyr Gln Glu Ile Lys Ile Gln Glu Gln Val
        195                 200                 205

Gln Arg Leu Ser Val Gly Ser Ile Pro Arg Ser Met Lys Val Ile Leu
    210                 215                 220

Glu Asp Asp Leu Val Asp Ser Cys Lys Ser Gly Asp Asp Leu Thr Ile
225                 230                 235                 240

Tyr Gly Ile Val Met Gln Arg Trp Lys Pro Phe Gln Gln Asp Val Arg
                245                 250                 255

Cys Glu Val Glu Ile Val Leu Lys Ala Asn Tyr Ile Gln Val Asn Asn
            260                 265                 270

Glu Gln Ser Ser Gly Ile Ile Met Asp Glu Glu Val Gln Lys Glu Phe
        275                 280                 285

Glu Asp Phe Trp Glu Tyr Tyr Lys Ser Asp Pro Phe Ala Gly Arg Asn
    290                 295                 300

Val Ile Leu Ala Ser Leu Cys Pro Gln Val Phe Gly Met Tyr Leu Val
305                 310                 315                 320

Lys Leu Ala Val Ala Met Val Leu Ala Gly Gly Ile Gln Arg Thr Asp
                325                 330                 335

Ala Thr Gly Thr Arg Val Arg Gly Glu Ser His Leu Leu Leu Val Gly
            340                 345                 350

Asp Pro Gly Thr Gly Lys Ser Gln Phe Leu Lys Tyr Ala Ala Lys Ile
        355                 360                 365

Thr Pro Arg Ser Val Leu Thr Thr Gly Ile Gly Ser Thr Ser Ala Gly
    370                 375                 380

Leu Thr Val Thr Ala Val Lys Asp Ser Gly Glu Trp Asn Leu Glu Ala
385                 390                 395                 400

Gly Ala Leu Val Leu Ala Asp Ala Gly Leu Cys Cys Ile Asp Glu Phe
                405                 410                 415

Asn Ser Leu Lys Glu His Asp Arg Thr Ser Ile His Glu Ala Met Glu
            420                 425                 430

Gln Gln Thr Ile Ser Val Ala Lys Ala Gly Leu Val Cys Lys Leu Asn
        435                 440                 445

Thr Arg Thr Thr Ile Leu Ala Ala Thr Asn Pro Lys Gly Gln Tyr Asp
    450                 455                 460

Pro Gln Glu Ser Val Ser Val Asn Ile Ala Leu Gly Ser Pro Leu Leu
465                 470                 475                 480

Ser Arg Phe Asp Leu Ile Leu Val Leu Leu Asp Thr Lys Asn Glu Asp
                485                 490                 495

Trp Asp Arg Ile Ile Ser Ser Phe Ile Leu Glu Asn Lys Gly Tyr Pro
            500                 505                 510

Ser Lys Ser Glu Lys Leu Trp Ser Met Glu Lys Met Lys Thr Tyr Phe
        515                 520                 525

Cys Leu Ile Arg Asn Leu Gln Pro Thr Leu Ser Asp Val Gly Asn Gln
    530                 535                 540

Val Leu Leu Arg Tyr Tyr Gln Met Gln Arg Gln Ser Asp Cys Arg Asn
545                 550                 555                 560

Ala Ala Arg Thr Thr Ile Arg Leu Leu Glu Ser Leu Ile Arg Leu Ala
                565                 570                 575

Glu Ala His Ala Arg Leu Met Phe Arg Asp Thr Val Thr Leu Glu Asp
            580                 585                 590

Ala Ile Thr Val Val Ser Val Met Glu Ser Ser Met Gln Gly Gly Ala
        595                 600                 605

Leu Leu Gly Gly Val Asn Ala Leu His Thr Ser Phe Pro Glu Asn Pro
    610                 615                 620

Gly Glu Gln Tyr Gln Arg Gln Cys Glu Leu Ile Leu Glu Lys Leu Glu
625                 630                 635                 640

Leu Gln Ser Leu Leu Ser Glu Glu Leu Arg Arg Leu Glu Arg Leu Gln
                645                 650                 655

Asn Gln Ser Val His Gln Ser Gln Pro Arg Val Leu Glu Val Glu Thr
            660                 665                 670

Thr Pro Gly Ser Leu Arg Asn Gly Pro Gly Glu Glu Ser Asn Phe Arg
        675                 680                 685

Thr Ser Ser Gln Gln Glu Ile Asn Tyr Ser Thr His Ile Phe Ser Pro
    690                 695                 700

Gly Gly Ser Pro Glu Gly Ser Pro Val Leu Asp Pro Pro Pro His Leu
705                 710                 715                 720

Glu Pro Asn Arg Ser Thr Ser Arg Lys His Ser Ala Gln His Lys Asn
                725                 730                 735

Asn Arg Asp Asp Ser Leu Asp Trp Phe Asp Phe Met Ala Thr His Gln
            740                 745                 750

Ser Glu Pro Lys Asn Thr Val Val Val Ser Pro His Pro Lys Thr Ser
        755                 760                 765

Gly Glu Asn Met Ala Ser Lys Ile Ser Asn Ser Thr Ser Gln Gly Lys
    770                 775                 780

Glu Lys Ser Glu Pro Gly Gln Arg Ser Lys Val Asp Ile Gly Leu Leu
785                 790                 795                 800

Pro Ser Pro Gly Glu Thr Gly Val Pro Trp Arg Ala Asp Asn Val Glu
                805                 810                 815

Ser Asn Lys Lys Lys Arg Leu Ala Leu Asp Ser Glu Ala Ala Val Ser
            820                 825                 830

Ala Asp Lys Pro Asp Ser Val Leu Thr His His Val Pro Arg Asn Leu

835                 840                 845

Gln Lys Leu Cys Lys Glu Arg Ala Gln Lys Leu Cys Arg Asn Ser Thr
    850                 855                 860

Arg Val Pro Ala Gln Cys Thr Val Pro Ser His Pro Gln Ser Thr Pro
865                 870                 875                 880

Val His Ser Pro Asp Arg Met Leu Asp Ser Pro Lys Arg Lys Arg Pro
                885                 890                 895

Lys Ser Leu Ala Gln Val Glu Glu Pro Ala Ile Glu Asn Val Lys Pro
            900                 905                 910

Pro Gly Ser Pro Val Ala Lys Leu Ala Lys Phe Thr Phe Lys Gln Lys
        915                 920                 925

Ser Lys Leu Ile His Ser Phe Glu Asp His Ser His Val Ser Pro Gly
    930                 935                 940

Ala Thr Lys Ile Ala Val His Ser Pro Lys Ile Ser Gln Arg Arg Thr
945                 950                 955                 960

Arg Arg Asp Ala Ala Leu Pro Val Lys Arg Pro Gly Lys Leu Thr Ser
                965                 970                 975

Thr Pro Gly Asn Gln Ile Ser Ser Gln Pro Gln Gly Glu Thr Lys Glu
            980                 985                 990

Val Ser Gln Gln Pro Pro Glu Lys  His Gly Pro Arg Glu  Lys Val Met
        995                 1000                1005

Cys Ala  Pro Glu Lys Arg Ile  Ile Gln Pro Glu Leu  Glu Leu Gly
    1010                 1015                 1020

Asn Glu  Thr Gly Cys Ala His  Leu Thr Cys Glu Gly  Asp Lys Lys
    1025                 1030                 1035

Glu Glu  Val Ser Gly Ser Asn  Lys Ser Gly Lys Val  His Ala Cys
    1040                 1045                 1050

Thr Leu  Ala Arg Leu Ala Asn  Phe Cys Phe Thr Pro  Pro Ser Glu
    1055                 1060                 1065

Ser Lys  Ser Lys Ser Pro Pro  Pro Glu Arg Lys Asn  Arg Gly Glu
    1070                 1075                 1080

Arg Gly Pro Ser Ser Pro Pro Thr Thr Thr Ala Pro Met Arg Val
    1085                1090                1095

Ser Lys Arg Lys Ser Phe Gln Leu Arg Gly Ser Thr Glu Lys Leu
    1100                1105                1110

Ile Val Ser Lys Glu Ser Leu Phe Thr Leu Pro Glu Leu Gly Asp
    1115                1120                1125

Glu Ala Phe Asp Cys Asp Trp Asp Glu Glu Met Arg Lys Lys Ser
    1130                1135                1140

<210> 15
<211> 13147
<212> DNA
<213> Homo sapiens

<220>
<221> CDS
<222> (386)..(9556)

<400> 15
ccggagcccg agccgaaggg cgagccgcaa acgctaagtc gctggccatt ggtggacatg      60

gcgcaggcgc gtttgctccg acgggccgaa tgttttgggg cagtgttttg agcgcggaga     120

ccgcgtgata ctggatgcgc atgggcatac cgtgctctgc ggctgcttgg cgttgcttct     180

tcctccagaa gtgggcgctg ggcagtcacg cagggtttga accggaagcg ggagtaggta     240

gctgcgtggc taacggagaa aagaagccgt ggccgcggga ggaggcgaga ggagtcggga     300

tctgcgctgc agccaccgcc gcggttgata ctactttgac cttccgagtg cagtgacagt     360

gatgtgtgtt ctgaaattgt gaacc atg agt cta gta ctt aat gat ctg ctt      412
                            Met Ser Leu Val Leu Asn Asp Leu Leu
                            1               5

atc tgc tgc cgt caa cta gaa cat gat aga gct aca gaa cga aag aaa      460
Ile Cys Cys Arg Gln Leu Glu His Asp Arg Ala Thr Glu Arg Lys Lys
10                  15                  20                  25

gaa gtt gag aaa ttt aag cgc ctg att cga gat cct gaa aca att aaa      508
Glu Val Glu Lys Phe Lys Arg Leu Ile Arg Asp Pro Glu Thr Ile Lys
                30                  35                  40

cat cta gat cgg cat tca gat tcc aaa caa gga aaa tat ttg aat tgg      556
His Leu Asp Arg His Ser Asp Ser Lys Gln Gly Lys Tyr Leu Asn Trp
            45                  50                  55

gat gct gtt ttt aga ttt tta cag aaa tat att cag aaa gaa aca gaa      604
Asp Ala Val Phe Arg Phe Leu Gln Lys Tyr Ile Gln Lys Glu Thr Glu
        60                  65                  70

tgt ctg aga ata gca aaa cca aat gta tca gcc tca aca caa gcc tcc      652
Cys Leu Arg Ile Ala Lys Pro Asn Val Ser Ala Ser Thr Gln Ala Ser
    75                  80                  85

agg cag aaa aag atg cag gaa atc agt agt ttg gtc aaa tac ttc atc 700
Arg Gln Lys Lys Met Gln Glu Ile Ser Ser Leu Val Lys Tyr Phe Ile
90                  95                  100                 105

aaa tgt gca aac aga aga gca cct agg cta aaa tgt caa gaa ctc tta 748
Lys Cys Ala Asn Arg Arg Ala Pro Arg Leu Lys Cys Gln Glu Leu Leu
                110                 115                 120

aat tat atc atg gat aca gtg aaa gat tca tct aat ggt gct att tac 796
Asn Tyr Ile Met Asp Thr Val Lys Asp Ser Ser Asn Gly Ala Ile Tyr
            125                 130                 135

gga gct gat tgt agc aac ata cta ctc aaa gac att ctt tct gtg aga 844
Gly Ala Asp Cys Ser Asn Ile Leu Leu Lys Asp Ile Leu Ser Val Arg
        140                 145                 150

aaa tac tgg tgt gaa ata tct cag caa cag tgg tta gaa ttg ttc tct 892
Lys Tyr Trp Cys Glu Ile Ser Gln Gln Gln Trp Leu Glu Leu Phe Ser
    155                 160                 165

gtg tac ttc agg ctc tat ctg aaa cct tca caa gat gtt cat aga gtt 940
Val Tyr Phe Arg Leu Tyr Leu Lys Pro Ser Gln Asp Val His Arg Val
170                 175                 180                 185

tta gtg gct aga ata att cat gct gtt acc aaa gga tgc tgt tct cag 988
Leu Val Ala Arg Ile Ile His Ala Val Thr Lys Gly Cys Cys Ser Gln
                190                 195                 200

act gac gga tta aat tcc aaa ttt ttg gac ttt ttt tcc aag gct att 1036
Thr Asp Gly Leu Asn Ser Lys Phe Leu Asp Phe Phe Ser Lys Ala Ile
            205                 210                 215

cag tgt gcg aga caa gaa aag agc tct tca ggt cta aat cat atc tta 1084
Gln Cys Ala Arg Gln Glu Lys Ser Ser Ser Gly Leu Asn His Ile Leu
        220                 225                 230

gca gct ctt act atc ttc ctc aag act ttg gct gtc aac ttt cga att 1132
Ala Ala Leu Thr Ile Phe Leu Lys Thr Leu Ala Val Asn Phe Arg Ile
    235                 240                 245

cga gtg tgt gaa tta gga gat gaa att ctt ccc act ttg ctt tat att 1180
Arg Val Cys Glu Leu Gly Asp Glu Ile Leu Pro Thr Leu Leu Tyr Ile
250                 255                 260                 265

tgg act caa cat agg ctt aat gat tct tta aaa gaa gtc att att gaa 1228
Trp Thr Gln His Arg Leu Asn Asp Ser Leu Lys Glu Val Ile Ile Glu
                270                 275                 280

tta ttt caa ctg caa att tat atc cat cat ccg aaa gga gcc aaa acc 1276
Leu Phe Gln Leu Gln Ile Tyr Ile His His Pro Lys Gly Ala Lys Thr
            285                 290                 295

caa gaa aaa ggt gct tat gaa tca aca aaa tgg aga agt att tta tac 1324
Gln Glu Lys Gly Ala Tyr Glu Ser Thr Lys Trp Arg Ser Ile Leu Tyr
        300                 305                 310

aac tta tat gat ctg cta gtg aat gag ata agt cat ata gga agt aga 1372
Asn Leu Tyr Asp Leu Leu Val Asn Glu Ile Ser His Ile Gly Ser Arg
    315                 320                 325

gga aag tat tct tca gga ttt cgt aat att gcc gtc aaa gaa aat ttg 1420
Gly Lys Tyr Ser Ser Gly Phe Arg Asn Ile Ala Val Lys Glu Asn Leu
330                 335                 340                 345

att gaa ttg atg gca gat atc tgt cac cag gtt ttt aat gaa gat acc     1468
Ile Glu Leu Met Ala Asp Ile Cys His Gln Val Phe Asn Glu Asp Thr
                350                 355                 360

aga tcc ttg gag att tct caa tct tac act act aca caa aga gaa tct     1516
Arg Ser Leu Glu Ile Ser Gln Ser Tyr Thr Thr Thr Gln Arg Glu Ser
            365                 370                 375

agt gat tac agt gtc cct tgc aaa agg aag aaa ata gaa cta ggc tgg     1564
Ser Asp Tyr Ser Val Pro Cys Lys Arg Lys Lys Ile Glu Leu Gly Trp
        380                 385                 390

gaa gta ata aaa gat cac ctt cag aag tca cag aat gat ttt gat ctt     1612
Glu Val Ile Lys Asp His Leu Gln Lys Ser Gln Asn Asp Phe Asp Leu
    395                 400                 405

gtg cct tgg cta cag att gca acc caa tta ata tca aag tat cct gca     1660
Val Pro Trp Leu Gln Ile Ala Thr Gln Leu Ile Ser Lys Tyr Pro Ala
410                 415                 420                 425

agt tta cct aac tgt gag ctg tct cca tta ctg atg ata cta tct cag     1708
Ser Leu Pro Asn Cys Glu Leu Ser Pro Leu Leu Met Ile Leu Ser Gln
                430                 435                 440

ctt cta ccc caa cag cga cat ggg gaa cgt aca cca tat gtg tta cga     1756
Leu Leu Pro Gln Gln Arg His Gly Glu Arg Thr Pro Tyr Val Leu Arg
            445                 450                 455

tgc ctt acg gaa gtt gca ttg tgt caa gac aag agg tca aac cta gaa     1804
Cys Leu Thr Glu Val Ala Leu Cys Gln Asp Lys Arg Ser Asn Leu Glu
        460                 465                 470

agc tca caa aag tca gat tta tta aaa ctc tgg aat aaa att tgg tgt     1852
Ser Ser Gln Lys Ser Asp Leu Leu Lys Leu Trp Asn Lys Ile Trp Cys
    475                 480                 485

att acc ttt cgt ggt ata agt tct gag caa ata caa gct gaa aac ttt     1900
Ile Thr Phe Arg Gly Ile Ser Ser Glu Gln Ile Gln Ala Glu Asn Phe
490                 495                 500                 505

ggc tta ctt gga gcc ata att cag ggt agt tta gtt gag gtt gac aga     1948
Gly Leu Leu Gly Ala Ile Ile Gln Gly Ser Leu Val Glu Val Asp Arg
                510                 515                 520

gaa ttc tgg aag tta ttt act ggg tca gcc tgc aga cct tca tgt cct     1996
Glu Phe Trp Lys Leu Phe Thr Gly Ser Ala Cys Arg Pro Ser Cys Pro
            525                 530                 535

gca gta tgc tgt ttg act ttg gca ctg acc acc agt ata gtt cca gga     2044
Ala Val Cys Cys Leu Thr Leu Ala Leu Thr Thr Ser Ile Val Pro Gly
        540                 545                 550

acg gta aaa atg gga ata gag caa aat atg tgt gaa gta aat aga agc     2092
Thr Val Lys Met Gly Ile Glu Gln Asn Met Cys Glu Val Asn Arg Ser
    555                 560                 565

ttt tct tta aag gaa tca ata atg aaa tgg ctc tta ttc tat cag tta     2140
Phe Ser Leu Lys Glu Ser Ile Met Lys Trp Leu Leu Phe Tyr Gln Leu
570                 575                 580                 585

gag ggt gac tta gaa aat agc aca gaa gtg cct cca att ctt cac agt     2188
Glu Gly Asp Leu Glu Asn Ser Thr Glu Val Pro Pro Ile Leu His Ser

590                 595                 600

aat ttt cct cat ctt gta ctg gag aaa att ctt gtg agt ctc act atg        2236
Asn Phe Pro His Leu Val Leu Glu Lys Ile Leu Val Ser Leu Thr Met
            605                 610                 615

aaa aac tgt aaa gct gca atg aat ttt ttc caa agc gtg cca gaa tgt        2284
Lys Asn Cys Lys Ala Ala Met Asn Phe Phe Gln Ser Val Pro Glu Cys
        620                 625                 630

gaa cac cac caa aaa gat aaa gaa gaa ctt tca ttc tca gaa gta gaa        2332
Glu His His Gln Lys Asp Lys Glu Glu Leu Ser Phe Ser Glu Val Glu
    635                 640                 645

gaa cta ttt ctt cag aca act ttt gac aag atg gac ttt tta acc att        2380
Glu Leu Phe Leu Gln Thr Thr Phe Asp Lys Met Asp Phe Leu Thr Ile
650                 655                 660                 665

gtg aga gaa tgt ggt ata gaa aag cac cag tcc agt att ggc ttc tct        2428
Val Arg Glu Cys Gly Ile Glu Lys His Gln Ser Ser Ile Gly Phe Ser
                670                 675                 680

gtc cac cag aat ctc aag gaa tca ctg gat cgc tgt ctt ctg gga tta        2476
Val His Gln Asn Leu Lys Glu Ser Leu Asp Arg Cys Leu Leu Gly Leu
            685                 690                 695

tca gaa cag ctt ctg aat aat tac tca tct gag att aca aat tca gaa        2524
Ser Glu Gln Leu Leu Asn Asn Tyr Ser Ser Glu Ile Thr Asn Ser Glu
        700                 705                 710

act ctt gtc cgg tgt tca cgt ctt ttg gtg ggt gtc ctt ggc tgc tac        2572
Thr Leu Val Arg Cys Ser Arg Leu Leu Val Gly Val Leu Gly Cys Tyr
    715                 720                 725

tgt tac atg ggt gta ata gct gaa gag gaa gca tat aag tca gaa tta        2620
Cys Tyr Met Gly Val Ile Ala Glu Glu Glu Ala Tyr Lys Ser Glu Leu
730                 735                 740                 745

ttc cag aaa gcc aag tct cta atg caa tgt gca gga gaa agt atc act        2668
Phe Gln Lys Ala Lys Ser Leu Met Gln Cys Ala Gly Glu Ser Ile Thr
                750                 755                 760

ctg ttt aaa aat aag aca aat gag gaa ttc aga att ggt tcc ttg aga        2716
Leu Phe Lys Asn Lys Thr Asn Glu Glu Phe Arg Ile Gly Ser Leu Arg
            765                 770                 775

aat atg atg cag cta tgt aca cgt tgc ttg agc aac tgt acc aag aag        2764
Asn Met Met Gln Leu Cys Thr Arg Cys Leu Ser Asn Cys Thr Lys Lys
        780                 785                 790

agt cca aat aag att gca tct ggc ttt ttc ctg cga ttg tta aca tca        2812
Ser Pro Asn Lys Ile Ala Ser Gly Phe Phe Leu Arg Leu Leu Thr Ser
    795                 800                 805

aag cta atg aat gac att gca gat att tgt aaa agt tta gca tcc ttc        2860
Lys Leu Met Asn Asp Ile Ala Asp Ile Cys Lys Ser Leu Ala Ser Phe
810                 815                 820                 825

atc aaa aag cca ttt gac cgt gga gaa gta gaa tca atg gaa gat gat        2908
Ile Lys Lys Pro Phe Asp Arg Gly Glu Val Glu Ser Met Glu Asp Asp
                830                 835                 840

act aat gga aat cta atg gag gtg gag gat cag tca tcc atg aat cta        2956

Thr Asn Gly Asn Leu Met Glu Val Glu Asp Gln Ser Ser Met Asn Leu
            845                 850                 855

ttt aac gat tac cct gat agt agt gtt agt gat gca aac gaa cct gga 3004
Phe Asn Asp Tyr Pro Asp Ser Ser Val Ser Asp Ala Asn Glu Pro Gly
        860                 865                 870

gag agc caa agt acc ata ggt gcc att aat cct tta gct gaa gaa tat 3052
Glu Ser Gln Ser Thr Ile Gly Ala Ile Asn Pro Leu Ala Glu Glu Tyr
    875                 880                 885

ctg tca aag caa gat cta ctt ttc tta gac atg ctc aag ttc ttg tgt 3100
Leu Ser Lys Gln Asp Leu Leu Phe Leu Asp Met Leu Lys Phe Leu Cys
890                 895                 900                 905

ttg tgt gta act act gct cag acc aat act gtg tcc ttt agg gca gct 3148
Leu Cys Val Thr Thr Ala Gln Thr Asn Thr Val Ser Phe Arg Ala Ala
                910                 915                 920

gat att cgg agg aaa ttg tta atg tta att gat tct agc acg cta gaa 3196
Asp Ile Arg Arg Lys Leu Leu Met Leu Ile Asp Ser Ser Thr Leu Glu
            925                 930                 935

cct acc aaa tcc ctc cac ctg cat atg tat cta atg ctt tta aag gag 3244
Pro Thr Lys Ser Leu His Leu His Met Tyr Leu Met Leu Leu Lys Glu
        940                 945                 950

ctt cct gga gaa gag tac ccc ttg cca atg gaa gat gtt ctt gaa ctt 3292
Leu Pro Gly Glu Glu Tyr Pro Leu Pro Met Glu Asp Val Leu Glu Leu
    955                 960                 965

ctg aaa cca cta tcc aat gtg tgt tct ttg tat cgt cgt gac caa gat 3340
Leu Lys Pro Leu Ser Asn Val Cys Ser Leu Tyr Arg Arg Asp Gln Asp
970                 975                 980                 985

gtt tgt aaa act att tta aac cat gtc ctt cat gta gtg aaa aac cta 3388
Val Cys Lys Thr Ile Leu Asn His Val Leu His Val Val Lys Asn Leu
                990                 995                 1000

ggt caa agc aat atg gac tct gag aac aca agg gat gct caa gga 3433
Gly Gln Ser Asn Met Asp Ser Glu Asn Thr Arg Asp Ala Gln Gly
            1005                1010                1015

cag ttt ctt aca gta att gga gca ttt tgg cat cta aca aag gag 3478
Gln Phe Leu Thr Val Ile Gly Ala Phe Trp His Leu Thr Lys Glu
            1020                1025                1030

agg aaa tat ata ttc tct gta aga atg gcc cta gta aat tgc ctt 3523
Arg Lys Tyr Ile Phe Ser Val Arg Met Ala Leu Val Asn Cys Leu
            1035                1040                1045

aaa act ttg ctt gag gct gat cct tat tca aaa tgg gcc att ctt 3568
Lys Thr Leu Leu Glu Ala Asp Pro Tyr Ser Lys Trp Ala Ile Leu
            1050                1055                1060

aat gta atg gga aaa gac ttt cct gta aat gaa gta ttt aca caa 3613
Asn Val Met Gly Lys Asp Phe Pro Val Asn Glu Val Phe Thr Gln
            1065                1070                1075

ttt ctt gct gac aat cat cac caa gtt cgc atg ttg gct gca gag 3658
Phe Leu Ala Asp Asn His His Gln Val Arg Met Leu Ala Ala Glu
            1080                1085                1090

tca atc aat aga ttg ttc cag gac acg aag gga gat tct tcc agg 3703
Ser Ile Asn Arg Leu Phe Gln Asp Thr Lys Gly Asp Ser Ser Arg
            1095                1100                1105

tta ctg aaa gca ctt cct ttg aag ctt cag caa aca gct ttt gaa 3748
Leu Leu Lys Ala Leu Pro Leu Lys Leu Gln Gln Thr Ala Phe Glu
            1110                1115                1120

aat gca tac ttg aaa gct cag gaa gga atg aga gaa atg tcc cat 3793
Asn Ala Tyr Leu Lys Ala Gln Glu Gly Met Arg Glu Met Ser His
            1125                1130                1135

agt gct gag aac cct gaa act ttg gat gaa att tat aat aga aaa 3838
Ser Ala Glu Asn Pro Glu Thr Leu Asp Glu Ile Tyr Asn Arg Lys
            1140                1145                1150

tct gtt tta ctg acg ttg ata gct gtg gtt tta tcc tgt agc cct 3883
Ser Val Leu Leu Thr Leu Ile Ala Val Val Leu Ser Cys Ser Pro
            1155                1160                1165

atc tgc gaa aaa cag gct ttg ttt gcc ctg tgt aaa tct gtg aaa 3928
Ile Cys Glu Lys Gln Ala Leu Phe Ala Leu Cys Lys Ser Val Lys
            1170                1175                1180

gag aat gga tta gaa cct cac ctt gtg aaa aag gtt tta gag aaa 3973
Glu Asn Gly Leu Glu Pro His Leu Val Lys Lys Val Leu Glu Lys
            1185                1190                1195

gtt tct gaa act ttt gga tat aga cgt tta gaa gac ttt atg gca 4018
Val Ser Glu Thr Phe Gly Tyr Arg Arg Leu Glu Asp Phe Met Ala
            1200                1205                1210

tct cat tta gat tat ctg gtt ttg gaa tgg cta aat ctt caa gat 4063
Ser His Leu Asp Tyr Leu Val Leu Glu Trp Leu Asn Leu Gln Asp
            1215                1220                1225

act gaa tac aac tta tct tct ttt cct ttt att tta tta aac tac 4108
Thr Glu Tyr Asn Leu Ser Ser Phe Pro Phe Ile Leu Leu Asn Tyr
            1230                1235                1240

aca aat att gag gat ttc tat aga tct tgt tat aag gtt ttg att 4153
Thr Asn Ile Glu Asp Phe Tyr Arg Ser Cys Tyr Lys Val Leu Ile
            1245                1250                1255

cca cat ctg gtg att aga agt cat ttt gat gag gtg aag tcc att 4198
Pro His Leu Val Ile Arg Ser His Phe Asp Glu Val Lys Ser Ile
            1260                1265                1270

gct aat cag att caa gag gac tgg aaa agt ctt cta aca gac tgc 4243
Ala Asn Gln Ile Gln Glu Asp Trp Lys Ser Leu Leu Thr Asp Cys
            1275                1280                1285

ttt cca aag att ctt gta aat att ctt cct tat ttt gcc tat gag 4288
Phe Pro Lys Ile Leu Val Asn Ile Leu Pro Tyr Phe Ala Tyr Glu
            1290                1295                1300

ggt acc aga gac agt ggg atg gca cag caa aga gag act gct acc 4333
Gly Thr Arg Asp Ser Gly Met Ala Gln Gln Arg Glu Thr Ala Thr
            1305                1310                1315

aag gtc tat gat atg ctt aaa agt gaa aac tta ttg gga aaa cag 4378
Lys Val Tyr Asp Met Leu Lys Ser Glu Asn Leu Leu Gly Lys Gln
            1320                1325                1330

att gat cac tta ttc att agt aat tta cca gag att gtg gtg gag 4423
Ile Asp His Leu Phe Ile Ser Asn Leu Pro Glu Ile Val Val Glu
            1335                1340                1345

tta ttg atg acg tta cat gag cca gca aat tct agt gcc agt cag 4468
Leu Leu Met Thr Leu His Glu Pro Ala Asn Ser Ser Ala Ser Gln
            1350                1355                1360

agc act gac ctc tgt gac ttt tca ggg gat ttg gat cct gct cct 4513
Ser Thr Asp Leu Cys Asp Phe Ser Gly Asp Leu Asp Pro Ala Pro
            1365                1370                1375

aat cca cct cat ttt cca tcg cat gtg att aaa gca aca ttt gcc 4558
Asn Pro Pro His Phe Pro Ser His Val Ile Lys Ala Thr Phe Ala
            1380                1385                1390

tat atc agc aat tgt cat aaa acc aag tta aaa agc att tta gaa 4603
Tyr Ile Ser Asn Cys His Lys Thr Lys Leu Lys Ser Ile Leu Glu
            1395                1400                1405

att ctt tcc aaa agc cct gat tcc tat cag aaa att ctt ctt gcc 4648
Ile Leu Ser Lys Ser Pro Asp Ser Tyr Gln Lys Ile Leu Leu Ala
            1410                1415                1420

ata tgt gag caa gca gct gaa aca aat aat gtt tat aag aag cac 4693
Ile Cys Glu Gln Ala Ala Glu Thr Asn Asn Val Tyr Lys Lys His
            1425                1430                1435

aga att ctt aaa ata tat cac ctg ttt gtt agt tta tta ctg aaa 4738
Arg Ile Leu Lys Ile Tyr His Leu Phe Val Ser Leu Leu Leu Lys
            1440                1445                1450

gat ata aaa agt ggc tta gga gga gct tgg gcc ttt gtt ctt cga 4783
Asp Ile Lys Ser Gly Leu Gly Gly Ala Trp Ala Phe Val Leu Arg
            1455                1460                1465

gac gtt att tat act ttg att cac tat atc aac caa agg cct tct 4828
Asp Val Ile Tyr Thr Leu Ile His Tyr Ile Asn Gln Arg Pro Ser
            1470                1475                1480

tgt atc atg gat gtg tca tta cgt agc ttc tcc ctt tgt tgt gac 4873
Cys Ile Met Asp Val Ser Leu Arg Ser Phe Ser Leu Cys Cys Asp
            1485                1490                1495

tta tta agt cag gtt tgc cag aca gcc gtg act tac tgt aag gat 4918
Leu Leu Ser Gln Val Cys Gln Thr Ala Val Thr Tyr Cys Lys Asp
            1500                1505                1510

gct cta gaa aac cat ctt cat gtt att gtt ggt aca ctt ata ccc 4963
Ala Leu Glu Asn His Leu His Val Ile Val Gly Thr Leu Ile Pro
            1515                1520                1525

ctt gtg tat gag cag gtg gag gtt cag aaa cag gta ttg gac ttg 5008
Leu Val Tyr Glu Gln Val Glu Val Gln Lys Gln Val Leu Asp Leu
            1530                1535                1540

ttg aaa tac tta gtg ata gat aac aag gat aat gaa aac ctc tat 5053
Leu Lys Tyr Leu Val Ile Asp Asn Lys Asp Asn Glu Asn Leu Tyr
            1545                1550                1555

atc acg att aag ctt tta gat cct ttt cct gac cat gtt gtt ttt 5098
Ile Thr Ile Lys Leu Leu Asp Pro Phe Pro Asp His Val Val Phe

1560                1565                1570

aag gat ttg cgt att act cag caa aaa atc aaa tac agt aga gga        5143
Lys Asp Leu Arg Ile Thr Gln Gln Lys Ile Lys Tyr Ser Arg Gly
        1575                1580                1585

ccc ttt tca ctc ttg gag gaa att aac cat ttt ctc tca gta agt        5188
Pro Phe Ser Leu Leu Glu Glu Ile Asn His Phe Leu Ser Val Ser
        1590                1595                1600

gtt tat gat gca ctt cca ttg aca aga ctt gaa gga cta aag gat        5233
Val Tyr Asp Ala Leu Pro Leu Thr Arg Leu Glu Gly Leu Lys Asp
        1605                1610                1615

ctt cga aga caa ctg gaa cta cat aaa gat cag atg gtg gac att        5278
Leu Arg Arg Gln Leu Glu Leu His Lys Asp Gln Met Val Asp Ile
        1620                1625                1630

atg aga gct tct cag gat aat ccg caa gat ggg att atg gtg aaa        5323
Met Arg Ala Ser Gln Asp Asn Pro Gln Asp Gly Ile Met Val Lys
        1635                1640                1645

cta gtt gtc aat ttg ttg cag tta tcc aag atg gca ata aac cac        5368
Leu Val Val Asn Leu Leu Gln Leu Ser Lys Met Ala Ile Asn His
        1650                1655                1660

act ggt gaa aaa gaa gtt cta gag gct gtt gga agc tgc ttg gga        5413
Thr Gly Glu Lys Glu Val Leu Glu Ala Val Gly Ser Cys Leu Gly
        1665                1670                1675

gaa gtg ggt cct ata gat ttc tct acc ata gct ata caa cat agt        5458
Glu Val Gly Pro Ile Asp Phe Ser Thr Ile Ala Ile Gln His Ser
        1680                1685                1690

aaa gat gca tct tat acc aag gcc ctt aag tta ttt gaa gat aaa        5503
Lys Asp Ala Ser Tyr Thr Lys Ala Leu Lys Leu Phe Glu Asp Lys
        1695                1700                1705

gaa ctt cag tgg acc ttc ata atg ctg acc tac ctg aat aac aca        5548
Glu Leu Gln Trp Thr Phe Ile Met Leu Thr Tyr Leu Asn Asn Thr
        1710                1715                1720

ctg gta gaa gat tgt gtc aaa gtt cga tca gca gct gtt acc tgt        5593
Leu Val Glu Asp Cys Val Lys Val Arg Ser Ala Ala Val Thr Cys
        1725                1730                1735

ttg aaa aac att tta gcc aca aag act gga cat agt ttc tgg gag        5638
Leu Lys Asn Ile Leu Ala Thr Lys Thr Gly His Ser Phe Trp Glu
        1740                1745                1750

att tat aag atg aca aca gat cca atg ctg gcc tat cta cag cct        5683
Ile Tyr Lys Met Thr Thr Asp Pro Met Leu Ala Tyr Leu Gln Pro
        1755                1760                1765

ttt aga aca tca aga aaa aag ttt tta gaa gta ccc aga ttt gac        5728
Phe Arg Thr Ser Arg Lys Lys Phe Leu Glu Val Pro Arg Phe Asp
        1770                1775                1780

aaa gaa aac cct ttt gaa ggc ctg gat gat ata aat ctg tgg att        5773
Lys Glu Asn Pro Phe Glu Gly Leu Asp Asp Ile Asn Leu Trp Ile
        1785                1790                1795

cct cta agt gaa aat cat gac att tgg ata aag aca ctg act tgt        5818

Pro Leu Ser Glu Asn His Asp Ile Trp Ile Lys Thr Leu Thr Cys
            1800                1805                1810

gct ttt ttg gac agt gga ggc aca aaa tgt gaa att ctt caa tta 5863
Ala Phe Leu Asp Ser Gly Gly Thr Lys Cys Glu Ile Leu Gln Leu
            1815                1820                1825

tta aag cca atg tgt gaa gtg aaa act gac ttt tgt cag act gta 5908
Leu Lys Pro Met Cys Glu Val Lys Thr Asp Phe Cys Gln Thr Val
            1830                1835                1840

ctt cca tac ttg att cat gat att tta ctc caa gat aca aat gaa 5953
Leu Pro Tyr Leu Ile His Asp Ile Leu Leu Gln Asp Thr Asn Glu
            1845                1850                1855

tca tgg aga aat ctg ctt tct aca cat gtt cag gga ttt ttc acc 5998
Ser Trp Arg Asn Leu Leu Ser Thr His Val Gln Gly Phe Phe Thr
            1860                1865                1870

agc tgt ctt cga cac ttc tcg caa acg agc cga tcc aca acc cct 6043
Ser Cys Leu Arg His Phe Ser Gln Thr Ser Arg Ser Thr Thr Pro
            1875                1880                1885

gca aac ttg gat tca gag tca gag cac ttt ttc cga tgc tgt ttg 6088
Ala Asn Leu Asp Ser Glu Ser Glu His Phe Phe Arg Cys Cys Leu
            1890                1895                1900

gat aaa aaa tca caa aga aca atg ctt gct gtt gtg gac tac atg 6133
Asp Lys Lys Ser Gln Arg Thr Met Leu Ala Val Val Asp Tyr Met
            1905                1910                1915

aga aga caa aag aga cct tct tca gga aca att ttt aat gat gct 6178
Arg Arg Gln Lys Arg Pro Ser Ser Gly Thr Ile Phe Asn Asp Ala
            1920                1925                1930

ttc tgg ctg gat tta aat tat cta gaa gtt gcc aag gta gct cag 6223
Phe Trp Leu Asp Leu Asn Tyr Leu Glu Val Ala Lys Val Ala Gln
            1935                1940                1945

tct tgt gct gct cac ttt aca gct tta ctc tat gca gaa atc tat 6268
Ser Cys Ala Ala His Phe Thr Ala Leu Leu Tyr Ala Glu Ile Tyr
            1950                1955                1960

gca gat aag aaa agt atg gat gat caa gag aaa aga agt ctt gca 6313
Ala Asp Lys Lys Ser Met Asp Asp Gln Glu Lys Arg Ser Leu Ala
            1965                1970                1975

ttt gaa gaa gga agc cag agt aca act att tct agc ttg agt gaa 6358
Phe Glu Glu Gly Ser Gln Ser Thr Thr Ile Ser Ser Leu Ser Glu
            1980                1985                1990

aaa agt aaa gaa gaa act gga ata agt tta cag gat ctt ctc tta 6403
Lys Ser Lys Glu Glu Thr Gly Ile Ser Leu Gln Asp Leu Leu Leu
            1995                2000                2005

gaa atc tac aga agt ata ggg gag cca gat agt ttg tat ggc tgt 6448
Glu Ile Tyr Arg Ser Ile Gly Glu Pro Asp Ser Leu Tyr Gly Cys
            2010                2015                2020

ggt gga ggg aag atg tta caa ccc att act aga cta cga aca tat 6493
Gly Gly Gly Lys Met Leu Gln Pro Ile Thr Arg Leu Arg Thr Tyr
            2025                2030                2035

gaa cac gaa gca atg tgg ggc aaa gcc cta gta aca tat gac ctc 6538
Glu His Glu Ala Met Trp Gly Lys Ala Leu Val Thr Tyr Asp Leu
            2040                2045                2050

gaa aca gca atc ccc tca tca aca cgc cag gca gga atc att cag 6583
Glu Thr Ala Ile Pro Ser Ser Thr Arg Gln Ala Gly Ile Ile Gln
            2055                2060                2065

gcc ttg cag aat ttg gga ctc tgc cat att ctt tcc gtc tat tta 6628
Ala Leu Gln Asn Leu Gly Leu Cys His Ile Leu Ser Val Tyr Leu
            2070                2075                2080

aaa gga ttg gat tat gaa aat aaa gac tgg tgt cct gaa cta gaa 6673
Lys Gly Leu Asp Tyr Glu Asn Lys Asp Trp Cys Pro Glu Leu Glu
            2085                2090                2095

gaa ctt cat tac caa gca gca tgg agg aat atg cag tgg gac cat 6718
Glu Leu His Tyr Gln Ala Ala Trp Arg Asn Met Gln Trp Asp His
            2100                2105                2110

tgc act tcc gtc agc aaa gaa gta gaa gga acc agt tac cat gaa 6763
Cys Thr Ser Val Ser Lys Glu Val Glu Gly Thr Ser Tyr His Glu
            2115                2120                2125

tca ttg tac aat gct cta caa tct cta aga gac aga gaa ttc tct 6808
Ser Leu Tyr Asn Ala Leu Gln Ser Leu Arg Asp Arg Glu Phe Ser
            2130                2135                2140

aca ttt tat gaa agt ctc aaa tat gcc aga gta aaa gaa gtg gaa 6853
Thr Phe Tyr Glu Ser Leu Lys Tyr Ala Arg Val Lys Glu Val Glu
            2145                2150                2155

gag atg tgt aag cgc agc ctt gag tct gtg tat tcg ctc tat ccc 6898
Glu Met Cys Lys Arg Ser Leu Glu Ser Val Tyr Ser Leu Tyr Pro
            2160                2165                2170

aca ctt agc agg ttg cag gcc att gga gag ctg gaa agc att ggg 6943
Thr Leu Ser Arg Leu Gln Ala Ile Gly Glu Leu Glu Ser Ile Gly
            2175                2180                2185

gag ctt ttc tca aga tca gtc aca cat aga caa ctc tct gaa gta 6988
Glu Leu Phe Ser Arg Ser Val Thr His Arg Gln Leu Ser Glu Val
            2190                2195                2200

tat att aag tgg cag aaa cac tcc cag ctt ctc aag gac agt gat 7033
Tyr Ile Lys Trp Gln Lys His Ser Gln Leu Leu Lys Asp Ser Asp
            2205                2210                2215

ttt agt ttt cag gag cct atc atg gct cta cgc aca gtc att ttg 7078
Phe Ser Phe Gln Glu Pro Ile Met Ala Leu Arg Thr Val Ile Leu
            2220                2225                2230

gag atc ctg atg gaa aag gaa atg gac aac tca caa aga gaa tgt 7123
Glu Ile Leu Met Glu Lys Glu Met Asp Asn Ser Gln Arg Glu Cys
            2235                2240                2245

att aag gac att ctc acc aaa cac ctt gta gaa ctc tct ata ctg 7168
Ile Lys Asp Ile Leu Thr Lys His Leu Val Glu Leu Ser Ile Leu
            2250                2255                2260

gcc aga act ttc aag aac act cag ctc cct gaa agg gca ata ttt 7213
Ala Arg Thr Phe Lys Asn Thr Gln Leu Pro Glu Arg Ala Ile Phe
            2265                2270                2275

caa att aaa cag tac aat tca gtt agc tgt gga gtc tct gag tgg 7258
Gln Ile Lys Gln Tyr Asn Ser Val Ser Cys Gly Val Ser Glu Trp
            2280                2285                2290

cag ctg gaa gaa gca caa gta ttc tgg gca aaa aag gag cag agt 7303
Gln Leu Glu Glu Ala Gln Val Phe Trp Ala Lys Lys Glu Gln Ser
            2295                2300                2305

ctt gcc ctg agt att ctc aag caa atg atc aag aag ttg gat gcc 7348
Leu Ala Leu Ser Ile Leu Lys Gln Met Ile Lys Lys Leu Asp Ala
            2310                2315                2320

agc tgt gca gcg aac aat ccc agc cta aaa ctt aca tac aca gaa 7393
Ser Cys Ala Ala Asn Asn Pro Ser Leu Lys Leu Thr Tyr Thr Glu
            2325                2330                2335

tgt ctg agg gtt tgt ggc aac tgg tta gca gaa acg tgc tta gaa 7438
Cys Leu Arg Val Cys Gly Asn Trp Leu Ala Glu Thr Cys Leu Glu
            2340                2345                2350

aat cct gcg gtc atc atg cag acc tat cta gaa aag gca gta gaa 7483
Asn Pro Ala Val Ile Met Gln Thr Tyr Leu Glu Lys Ala Val Glu
            2355                2360                2365

gtt gct gga aat tat gat gga gaa agt agt gat gag cta aga aat 7528
Val Ala Gly Asn Tyr Asp Gly Glu Ser Ser Asp Glu Leu Arg Asn
            2370                2375                2380

gga aaa atg aag gca ttt ctc tca tta gcc cgg ttt tca gat act 7573
Gly Lys Met Lys Ala Phe Leu Ser Leu Ala Arg Phe Ser Asp Thr
            2385                2390                2395

caa tac caa aga att gaa aac tac atg aaa tca tcg gaa ttt gaa 7618
Gln Tyr Gln Arg Ile Glu Asn Tyr Met Lys Ser Ser Glu Phe Glu
            2400                2405                2410

aac aag caa gct ctc ctg aaa aga gcc aaa gag gaa gta ggt ctc 7663
Asn Lys Gln Ala Leu Leu Lys Arg Ala Lys Glu Glu Val Gly Leu
            2415                2420                2425

ctt agg gaa cat aaa att cag aca aac aga tac aca gta aag gtt 7708
Leu Arg Glu His Lys Ile Gln Thr Asn Arg Tyr Thr Val Lys Val
            2430                2435                2440

cag cga gag ctg gag ttg gat gaa tta gcc ctg cgt gca ctg aaa 7753
Gln Arg Glu Leu Glu Leu Asp Glu Leu Ala Leu Arg Ala Leu Lys
            2445                2450                2455

gag gat cgt aaa cgc ttc tta tgt aaa gca gtt gaa aat tat atc 7798
Glu Asp Arg Lys Arg Phe Leu Cys Lys Ala Val Glu Asn Tyr Ile
            2460                2465                2470

aac tgc tta tta agt gga gaa gaa cat gat atg tgg gta ttc cga 7843
Asn Cys Leu Leu Ser Gly Glu Glu His Asp Met Trp Val Phe Arg
            2475                2480                2485

ctt tgt tcc ctc tgg ctt gaa aat tct gga gtt tct gaa gtc aat 7888
Leu Cys Ser Leu Trp Leu Glu Asn Ser Gly Val Ser Glu Val Asn
            2490                2495                2500

ggc atg atg aag aga gac gga atg aag att cca aca tat aaa ttt 7933
Gly Met Met Lys Arg Asp Gly Met Lys Ile Pro Thr Tyr Lys Phe

2505                2510                2515

ttg cct ctt atg  tac caa ttg gct gct  aga atg ggg acc aag  atg        7978
Leu Pro Leu Met  Tyr Gln Leu Ala Ala  Arg Met Gly Thr Lys  Met
          2520                2525                2530

atg gga ggc cta  gga ttt cat gaa gtc  ctc aat aat cta atc  tct        8023
Met Gly Gly Leu  Gly Phe His Glu Val  Leu Asn Asn Leu Ile  Ser
          2535                2540                2545

aga att tca atg  gat cac ccc cat cac  act ttg ttt att ata  ctg        8068
Arg Ile Ser Met  Asp His Pro His His  Thr Leu Phe Ile Ile  Leu
          2550                2555                2560

gcc tta gca aat  gca aac aga gat gaa  ttt ctg act aaa cca  gag        8113
Ala Leu Ala Asn  Ala Asn Arg Asp Glu  Phe Leu Thr Lys Pro  Glu
          2565                2570                2575

gta gcc aga aga  agc aga ata act aaa  aat gtg cct aaa caa  agc        8158
Val Ala Arg Arg  Ser Arg Ile Thr Lys  Asn Val Pro Lys Gln  Ser
          2580                2585                2590

tct cag ctt gat  gag gat cga aca gag  gct gca aat aga ata  ata        8203
Ser Gln Leu Asp  Glu Asp Arg Thr Glu  Ala Ala Asn Arg Ile  Ile
          2595                2600                2605

tgt act atc aga  agt agg aga cct cag  atg gtc aga agt gtt  gag        8248
Cys Thr Ile Arg  Ser Arg Arg Pro Gln  Met Val Arg Ser Val  Glu
          2610                2615                2620

gca ctt tgt gat  gct tat att ata tta  gca aac tta gat gcc  act        8293
Ala Leu Cys Asp  Ala Tyr Ile Ile Leu  Ala Asn Leu Asp Ala  Thr
          2625                2630                2635

cag tgg aag act  cag aga aaa ggc ata  aat att cca gca gac  cag        8338
Gln Trp Lys Thr  Gln Arg Lys Gly Ile  Asn Ile Pro Ala Asp  Gln
          2640                2645                2650

cca att act aaa  ctt aag aat tta gaa  gat gtt gtt gtc cct  act        8383
Pro Ile Thr Lys  Leu Lys Asn Leu Glu  Asp Val Val Val Pro  Thr
          2655                2660                2665

atg gaa att aag  gtg gac cac aca gga  gaa tat gga aat ctg  gtg        8428
Met Glu Ile Lys  Val Asp His Thr Gly  Glu Tyr Gly Asn Leu  Val
          2670                2675                2680

act ata cag tca  ttt aaa gca gaa ttt  cgc tta gca gga ggt  gta        8473
Thr Ile Gln Ser  Phe Lys Ala Glu Phe  Arg Leu Ala Gly Gly  Val
          2685                2690                2695

aat tta cca aaa  ata ata gat tgt gta  ggt tcc gat ggc aag  gag        8518
Asn Leu Pro Lys  Ile Ile Asp Cys Val  Gly Ser Asp Gly Lys  Glu
          2700                2705                2710

agg aga cag ctt  gtt aag ggc cgt gat  gac ctg aga caa gat  gct        8563
Arg Arg Gln Leu  Val Lys Gly Arg Asp  Asp Leu Arg Gln Asp  Ala
          2715                2720                2725

gtc atg caa cag  gtc ttc cag atg tgt  aat aca tta ctg cag  aga        8608
Val Met Gln Gln  Val Phe Gln Met Cys  Asn Thr Leu Leu Gln  Arg
          2730                2735                2740

aac acg gaa act  agg aag agg aaa tta  act atc tgt act tat  aag        8653

Asn Thr Glu Thr Arg Lys Arg Lys Leu Thr Ile Cys Thr Tyr Lys
            2745                2750                2755

gtg gtt ccc ctc tct cag cga agt ggt gtt ctt gaa tgg tgc aca 8698
Val Val Pro Leu Ser Gln Arg Ser Gly Val Leu Glu Trp Cys Thr
            2760                2765                2770

gga act gtc ccc att ggt gaa ttt ctt gtt aac aat gaa gat ggt 8743
Gly Thr Val Pro Ile Gly Glu Phe Leu Val Asn Asn Glu Asp Gly
            2775                2780                2785

gct cat aaa aga tac agg cca aat gat ttc agt gcc ttt cag tgc 8788
Ala His Lys Arg Tyr Arg Pro Asn Asp Phe Ser Ala Phe Gln Cys
            2790                2795                2800

caa aag aaa atg atg gag gtg caa aaa aag tct ttt gaa gag aaa 8833
Gln Lys Lys Met Met Glu Val Gln Lys Lys Ser Phe Glu Glu Lys
            2805                2810                2815

tat gaa gtc ttc atg gat gtt tgc caa aat ttt caa cca gtt ttc 8878
Tyr Glu Val Phe Met Asp Val Cys Gln Asn Phe Gln Pro Val Phe
            2820                2825                2830

cgt tac ttc tgc atg gaa aaa ttc ttg gat cca gct att tgg ttt 8923
Arg Tyr Phe Cys Met Glu Lys Phe Leu Asp Pro Ala Ile Trp Phe
            2835                2840                2845

gag aag cga ttg gct tat acg cgc agt gta gct act tct tct att 8968
Glu Lys Arg Leu Ala Tyr Thr Arg Ser Val Ala Thr Ser Ser Ile
            2850                2855                2860

gtt ggt tac ata ctt gga ctt ggt gat aga cat gta cag aat atc 9013
Val Gly Tyr Ile Leu Gly Leu Gly Asp Arg His Val Gln Asn Ile
            2865                2870                2875

ttg ata aat gag cag tca gca gaa ctt gta cat ata gat cta ggt 9058
Leu Ile Asn Glu Gln Ser Ala Glu Leu Val His Ile Asp Leu Gly
            2880                2885                2890

gtt gct ttt gaa cag ggc aaa atc ctt cct act cct gag aca gtt 9103
Val Ala Phe Glu Gln Gly Lys Ile Leu Pro Thr Pro Glu Thr Val
            2895                2900                2905

cct ttt aga ctc acc aga gat att gtg gat ggc atg ggc att acg 9148
Pro Phe Arg Leu Thr Arg Asp Ile Val Asp Gly Met Gly Ile Thr
            2910                2915                2920

ggt gtt gaa ggt gtc ttc aga aga tgc tgt gag aaa acc atg gaa 9193
Gly Val Glu Gly Val Phe Arg Arg Cys Cys Glu Lys Thr Met Glu
            2925                2930                2935

gtg atg aga aac tct cag gaa act ctg tta acc att gta gag gtc 9238
Val Met Arg Asn Ser Gln Glu Thr Leu Leu Thr Ile Val Glu Val
            2940                2945                2950

ctt cta tat gat cca ctc ttt gac tgg acc atg aat cct ttg aaa 9283
Leu Leu Tyr Asp Pro Leu Phe Asp Trp Thr Met Asn Pro Leu Lys
            2955                2960                2965

gct ttg tat tta cag cag agg ccg gaa gat gaa act gag ctt cac 9328
Ala Leu Tyr Leu Gln Gln Arg Pro Glu Asp Glu Thr Glu Leu His
            2970                2975                2980

cct act ctg aat gca gat gac caa gaa tgc aaa cga aat ctc agt 9373
Pro Thr Leu Asn Ala Asp Asp Gln Glu Cys Lys Arg Asn Leu Ser
            2985                2990                2995

gat att gac cag agt ttc aac aaa gta gct gaa cgt gtc tta atg 9418
Asp Ile Asp Gln Ser Phe Asn Lys Val Ala Glu Arg Val Leu Met
            3000                3005                3010

aga cta caa gag aaa ctg aaa gga gtg gaa gaa ggc act gtg ctc 9463
Arg Leu Gln Glu Lys Leu Lys Gly Val Glu Glu Gly Thr Val Leu
            3015                3020                3025

agt gtt ggt gga caa gtg aat ttg ctc ata cag cag gcc ata gac 9508
Ser Val Gly Gly Gln Val Asn Leu Leu Ile Gln Gln Ala Ile Asp
            3030                3035                3040

ccc aaa aat ctc agc cga ctt ttc cca gga tgg aaa gct tgg gtg 9553
Pro Lys Asn Leu Ser Arg Leu Phe Pro Gly Trp Lys Ala Trp Val
            3045                3050                3055

tga tcttcagtat atgaattacc ctttcattca gcctttagaa attatatttt 9606

agcctttatt tttaacctgc caacatactt taagtaggga ttaatattta agtgaactat 9666

tgtgggtttt tttgaatgtt ggttttaata cttgatttaa tcaccactca aaaatgtttt 9726

gatggtctta aggaacatct ctgctttcac tctttagaaa taatggtcat tcgggctggg 9786

cgcagcggct cacgcctgta atcccagcac tttgggaggc cgaggtgagc ggatcacaag 9846

gtcaggagtt cgagaccagc ctggccaaga gaccagcctg gccagtatgg tgaaaccctg 9906

tctctactaa aaatacaaaa attagccgag catggtggcg ggcacctgta atcccagcta 9966

ctcgagaggc tgaggcagga gaatctcttg aacctgggag gtgaaggttg ctgtgggcca 10026

aaatcatgcc attgcactcc agcctgggtg acaagagcga aactccatct caaaaaaaaa 10086

aaaaaaaaaa cagaaacgta tttggatttt tcctagtaag atcactcagt gttactaaat 10146

aatgaagttg ttatggagaa caaatttcaa agacacagtt agtgtagtta ctattttttt 10206

aagtgtgtat taaaacttct cattctattc tctttatctt ttaagccctt ctgtactgtc 10266

catgtatgtt atctttctgt gataacttca tagattgcct tctagttcat gaattctctt 10326

gtcagatgta tataatctct tttaccctat ccattgggct tcttctttca gaaattgttt 10386

ttcatttcta attatgcatc atttttcaga tctctgtttc ttgatgtcat ttttaatgtt 10446

tttttaatgt tttttatgtc actaattatt ttaaatgtct gtacttgata gacactgtaa 10506

tagttctatt aaatttagtt cctgctgttt atatctgttg atttttgtat ttgataggct 10566

gttcatccag ttttgtcttt ttgaaaagtg agtttatttt cagcaaggct ttatctatgg 10626

gaatcttgag tgtctgttta tgtcatattc ccagggctgt tgctgcacac aagcccattc 10686

ttattttaat ttcttggctt tagggtttcc atacctgaag tgtagcataa atactgatag 10746

gagatttccc aggccaaggc aaacacactt cctcctcatc tccttgtgct agtgggcaga 10806

atatttgatt gatgcctttt tcactgagag tataagcttc catgtgtccc acctttatgg 10866

caggggtgga aggaggtaca tttaattccc actgcctgcc tttggcaagc cctgggttct 10926

ttgctcccca tatagatgtc taagctaaaa gccgtgggtt aatgagactg gcaaattgtt 10986

ccaggacagc tacagcatca gctcacatat tcacctctct ggtttttcat tcccctcatt 11046

tttttctgag acagagtctt gctctgtcac ccaggctgga gtgcagtggc atgatctcag 11106

ctcactgaaa cctctgcctc ctgggttcaa gcaattctcc tgcctcagcc tcccgagtag 11166

ctgggactac aggcgtgtgc caacacgccc ggctaatttt ttgtattttt attagagacg 11226

gagtttcacc gtgttagcca ggatggtctc gatcgcttga cctcgtgatc caccctcctc 11286

ggcctcccaa agtgctggga ttacaggtgt gagccaccgc gcccggcctc attcccctca 11346

tttttgaccg taaggatttc ccctttcttg taagttctgc tatgtattta aaagaatgtt 11406

ttctacattt tatccagcat ttctctgtgt tctgttggaa gggaagggct taggtatcta 11466

gtttgataca taggtagaag tggaacattt ctctgtcccc cagctgtcat catataagat 11526

aaacatcaga taaaaagcca cctgaaagta aaactactga ctcgtgtatt agtgagtata 11586

atctcttctc catccttagg aaaatgttca tcccagctgc ggagattaac aaatgggtga 11646

ttgagctttc tcctcgtatt tggaccttga aggttatata aatttttttc ttatgaagag 11706

ttggcatttc tttttattgc caatggcagg cactcattca tatttgatct cctcaccttc 11766

ccctccccta aaaccaatct ccagaacttt ttggactata aatttcttgg tttgacttct 11826

ggagaactgt tcagaatatt actttgcatt tcaaattaca aacttacctt ggtgtatctt 11886

tttcttacaa gctgcctaaa tgaatatttg gtatatattg gtagttttat tactatagta 11946

aatcaaggaa atgcagtaaa cttaaaatgt ctttaagaaa gccctgaaat cttcatgggt 12006

gaaattagaa attatcaact agataatagt atagataaat gaatttgtag ctaattcttg 12066

ctagttgttg catccagaga gctttgaata acatcattaa tctactcttt agccttgcat 12126

ggtatgctat gaggctcctg ttctgttcaa gtattctaat caatggcttt gaaaagttta 12186

tcaaatttac atacagatca caagcctagg agaaataact aattcacaga tgacagaatt 12246

aagattataa aagatttttt ttttgtaatt ttagtagaga cagggttgcc attgtattcc 12306

agccttggcg acagagcaag actctgcctc aaaaaaaaaa aaaaaaaggt tttggcaagc 12366

tggaactctt tctgcaaatg actaagatag aaaactgcca aggacaaatg aggagtagtt 12426

agattttgaa aatattaatc atagaatagt tgttgtatgc taagtcactg acccatatta 12486

tgtacagcat ttctgatctt tactttgcaa gattagtgat actatcccaa tacactgctg 12546

gagaaatcag aatttggaga aataagttgt ccaaggcaag aagatagtaa attataagta 12606

caagtgtaat atggacagta tctaacttga aaagatttca ggcgaaaaga atctggggtt 12666

tgccagtcag ttgctcaaaa ggtcaatgaa aaccaaatag tgaagctatc agagaagcta 12726

ataaattata gactgcttga acagttgtgt ccagattaag ggagataata gctttcccac 12786

cctactttgt gcaggtcata cctccccaaa gtgtttacct aatcagtagg ttcacaaact 12846

cttggtcatt atagtatatg cctaaaatgt atgcacttag gaatgctaaa aatttaaata 12906

tggtctaaag caaataaaag caaagaggaa aaactttgga cagcgtaaag actagaatag 12966

tcttttaaaa agaaagccag tatattggtt tgaaatatag agatgtgtcc caatttcaag 13026

tattttaatt gcaccttaat gaaattatct attttctata gattttagta ctattgaatg 13086

tattacttta ctgttacctg aatttattat aaagtgtttt tgaataaata attctaaaag 13146

c 13147

<210> 16
<211> 3056
<212> PRT
<213> Homo sapiens

<400> 16

Met Ser Leu Val Leu Asn Asp Leu Leu Ile Cys Cys Arg Gln Leu Glu
1               5                   10                  15

His Asp Arg Ala Thr Glu Arg Lys Lys Glu Val Glu Lys Phe Lys Arg
            20                  25                  30

Leu Ile Arg Asp Pro Glu Thr Ile Lys His Leu Asp Arg His Ser Asp
        35                  40                  45

Ser Lys Gln Gly Lys Tyr Leu Asn Trp Asp Ala Val Phe Arg Phe Leu
    50                  55                  60

Gln Lys Tyr Ile Gln Lys Glu Thr Glu Cys Leu Arg Ile Ala Lys Pro
65                  70                  75                  80

Asn Val Ser Ala Ser Thr Gln Ala Ser Arg Gln Lys Lys Met Gln Glu
                85                  90                  95

Ile Ser Ser Leu Val Lys Tyr Phe Ile Lys Cys Ala Asn Arg Arg Ala
            100                 105                 110

Pro Arg Leu Lys Cys Gln Glu Leu Leu Asn Tyr Ile Met Asp Thr Val
        115                 120                 125

Lys Asp Ser Ser Asn Gly Ala Ile Tyr Gly Ala Asp Cys Ser Asn Ile
    130                 135                 140

Leu Leu Lys Asp Ile Leu Ser Val Arg Lys Tyr Trp Cys Glu Ile Ser
145                 150                 155                 160

Gln Gln Gln Trp Leu Glu Leu Phe Ser Val Tyr Phe Arg Leu Tyr Leu
                165                 170                 175

Lys Pro Ser Gln Asp Val His Arg Val Leu Val Ala Arg Ile Ile His
            180                 185                 190

Ala Val Thr Lys Gly Cys Cys Ser Gln Thr Asp Gly Leu Asn Ser Lys
        195                 200                 205

Phe Leu Asp Phe Phe Ser Lys Ala Ile Gln Cys Ala Arg Gln Glu Lys
    210                 215                 220

Ser Ser Ser Gly Leu Asn His Ile Leu Ala Ala Leu Thr Ile Phe Leu
225                 230                 235                 240

Lys Thr Leu Ala Val Asn Phe Arg Ile Arg Val Cys Glu Leu Gly Asp
                245                 250                 255

Glu Ile Leu Pro Thr Leu Leu Tyr Ile Trp Thr Gln His Arg Leu Asn
            260                 265                 270

Asp Ser Leu Lys Glu Val Ile Ile Glu Leu Phe Gln Leu Gln Ile Tyr
        275                 280                 285

Ile His His Pro Lys Gly Ala Lys Thr Gln Glu Lys Gly Ala Tyr Glu
    290                 295                 300

Ser Thr Lys Trp Arg Ser Ile Leu Tyr Asn Leu Tyr Asp Leu Leu Val
305                 310                 315                 320

Asn Glu Ile Ser His Ile Gly Ser Arg Gly Lys Tyr Ser Ser Gly Phe
                325                 330                 335

Arg Asn Ile Ala Val Lys Glu Asn Leu Ile Glu Leu Met Ala Asp Ile
            340                 345                 350

Cys His Gln Val Phe Asn Glu Asp Thr Arg Ser Leu Glu Ile Ser Gln
        355                 360                 365

Ser Tyr Thr Thr Thr Gln Arg Glu Ser Ser Asp Tyr Ser Val Pro Cys
    370                 375                 380

Lys Arg Lys Lys Ile Glu Leu Gly Trp Glu Val Ile Lys Asp His Leu
385                 390                 395                 400

Gln Lys Ser Gln Asn Asp Phe Asp Leu Val Pro Trp Leu Gln Ile Ala
                405                 410                 415

Thr Gln Leu Ile Ser Lys Tyr Pro Ala Ser Leu Pro Asn Cys Glu Leu
            420                 425                 430

Ser Pro Leu Leu Met Ile Leu Ser Gln Leu Leu Pro Gln Gln Arg His
        435                 440                 445

Gly Glu Arg Thr Pro Tyr Val Leu Arg Cys Leu Thr Glu Val Ala Leu
    450                 455                 460

Cys Gln Asp Lys Arg Ser Asn Leu Glu Ser Ser Gln Lys Ser Asp Leu
465                 470                 475                 480

Leu Lys Leu Trp Asn Lys Ile Trp Cys Ile Thr Phe Arg Gly Ile Ser
                485                 490                 495

Ser Glu Gln Ile Gln Ala Glu Asn Phe Gly Leu Leu Gly Ala Ile Ile
            500                 505                 510

Gln Gly Ser Leu Val Glu Val Asp Arg Glu Phe Trp Lys Leu Phe Thr
        515                 520                 525

Gly Ser Ala Cys Arg Pro Ser Cys Pro Ala Val Cys Cys Leu Thr Leu
    530                 535                 540

Ala Leu Thr Thr Ser Ile Val Pro Gly Thr Val Lys Met Gly Ile Glu
545                 550                 555                 560

Gln Asn Met Cys Glu Val Asn Arg Ser Phe Ser Leu Lys Glu Ser Ile
                565                 570                 575

Met Lys Trp Leu Leu Phe Tyr Gln Leu Glu Gly Asp Leu Glu Asn Ser
            580                 585                 590

Thr Glu Val Pro Pro Ile Leu His Ser Asn Phe Pro His Leu Val Leu
        595                 600                 605

Glu Lys Ile Leu Val Ser Leu Thr Met Lys Asn Cys Lys Ala Ala Met
    610                 615                 620

Asn Phe Phe Gln Ser Val Pro Glu Cys Glu His His Gln Lys Asp Lys
625                 630                 635                 640

Glu Glu Leu Ser Phe Ser Glu Val Glu Glu Leu Phe Leu Gln Thr Thr
                645                 650                 655

Phe Asp Lys Met Asp Phe Leu Thr Ile Val Arg Glu Cys Gly Ile Glu

660                 665                 670

Lys His Gln Ser Ser Ile Gly Phe Ser Val His Gln Asn Leu Lys Glu
        675                 680                 685

Ser Leu Asp Arg Cys Leu Leu Gly Leu Ser Glu Gln Leu Leu Asn Asn
    690                 695                 700

Tyr Ser Ser Glu Ile Thr Asn Ser Glu Thr Leu Val Arg Cys Ser Arg
705                 710                 715                 720

Leu Leu Val Gly Val Leu Gly Cys Tyr Cys Tyr Met Gly Val Ile Ala
                725                 730                 735

Glu Glu Glu Ala Tyr Lys Ser Glu Leu Phe Gln Lys Ala Lys Ser Leu
            740                 745                 750

Met Gln Cys Ala Gly Glu Ser Ile Thr Leu Phe Lys Asn Lys Thr Asn
        755                 760                 765

Glu Glu Phe Arg Ile Gly Ser Leu Arg Asn Met Met Gln Leu Cys Thr
    770                 775                 780

Arg Cys Leu Ser Asn Cys Thr Lys Lys Ser Pro Asn Lys Ile Ala Ser
785                 790                 795                 800

Gly Phe Phe Leu Arg Leu Leu Thr Ser Lys Leu Met Asn Asp Ile Ala
                805                 810                 815

Asp Ile Cys Lys Ser Leu Ala Ser Phe Ile Lys Lys Pro Phe Asp Arg
            820                 825                 830

Gly Glu Val Glu Ser Met Glu Asp Asp Thr Asn Gly Asn Leu Met Glu
        835                 840                 845

Val Glu Asp Gln Ser Ser Met Asn Leu Phe Asn Asp Tyr Pro Asp Ser
    850                 855                 860

Ser Val Ser Asp Ala Asn Glu Pro Gly Glu Ser Gln Ser Thr Ile Gly
865                 870                 875                 880

Ala Ile Asn Pro Leu Ala Glu Glu Tyr Leu Ser Lys Gln Asp Leu Leu
                885                 890                 895

Phe Leu Asp Met Leu Lys Phe Leu Cys Leu Cys Val Thr Thr Ala Gln
            900                 905                 910

Thr Asn Thr Val Ser Phe Arg Ala Ala Asp Ile Arg Arg Lys Leu Leu
        915                 920                 925

Met Leu Ile Asp Ser Ser Thr Leu Glu Pro Thr Lys Ser Leu His Leu
    930                 935                 940

His Met Tyr Leu Met Leu Leu Lys Glu Leu Pro Gly Glu Glu Tyr Pro
945                 950                 955                 960

Leu Pro Met Glu Asp Val Leu Glu Leu Leu Lys Pro Leu Ser Asn Val
                965                 970                 975

Cys Ser Leu Tyr Arg Arg Asp Gln Asp Val Cys Lys Thr Ile Leu Asn
            980                 985                 990

His Val Leu His Val Val Lys Asn Leu Gly Gln Ser Asn Met Asp Ser
        995                 1000                1005

Glu Asn Thr Arg Asp Ala Gln Gly Gln Phe Leu Thr Val Ile Gly
    1010                1015                1020

Ala Phe Trp His Leu Thr Lys Glu Arg Lys Tyr Ile Phe Ser Val
    1025                1030                1035

Arg Met Ala Leu Val Asn Cys Leu Lys Thr Leu Leu Glu Ala Asp
    1040                1045                1050

Pro Tyr Ser Lys Trp Ala Ile Leu Asn Val Met Gly Lys Asp Phe
    1055                1060                1065

Pro Val Asn Glu Val Phe Thr Gln Phe Leu Ala Asp Asn His His
    1070                1075                1080

Gln Val Arg Met Leu Ala Ala Glu Ser Ile Asn Arg Leu Phe Gln
    1085                1090                1095

Asp Thr Lys Gly Asp Ser Ser Arg Leu Leu Lys Ala Leu Pro Leu
    1100                1105                1110

Lys Leu Gln Gln Thr Ala Phe Glu Asn Ala Tyr Leu Lys Ala Gln
    1115                1120                1125

Glu Gly Met Arg Glu Met Ser His Ser Ala Glu Asn Pro Glu Thr
    1130                1135                1140

Leu Asp Glu Ile Tyr Asn Arg Lys Ser Val Leu Leu Thr Leu Ile
    1145                1150                1155

Ala Val Val Leu Ser Cys Ser Pro Ile Cys Glu Lys Gln Ala Leu
    1160                1165                1170

Phe Ala Leu Cys Lys Ser Val Lys Glu Asn Gly Leu Glu Pro His
    1175                1180                1185

Leu Val Lys Lys Val Leu Glu Lys Val Ser Glu Thr Phe Gly Tyr
    1190                1195                1200

Arg Arg Leu Glu Asp Phe Met Ala Ser His Leu Asp Tyr Leu Val
    1205                1210                1215

Leu Glu Trp Leu Asn Leu Gln Asp Thr Glu Tyr Asn Leu Ser Ser
    1220                1225                1230

Phe Pro Phe Ile Leu Leu Asn Tyr Thr Asn Ile Glu Asp Phe Tyr
    1235                1240                1245

Arg Ser Cys Tyr Lys Val Leu Ile Pro His Leu Val Ile Arg Ser
    1250                1255                1260

His Phe Asp Glu Val Lys Ser Ile Ala Asn Gln Ile Gln Glu Asp
    1265                1270                1275

Trp Lys Ser Leu Leu Thr Asp Cys Phe Pro Lys Ile Leu Val Asn
    1280                1285                1290

Ile Leu Pro Tyr Phe Ala Tyr Glu Gly Thr Arg Asp Ser Gly Met
    1295                1300                1305

Ala Gln Gln Arg Glu Thr Ala Thr Lys Val Tyr Asp Met Leu Lys
    1310                1315                1320

Ser Glu Asn Leu Leu Gly Lys Gln Ile Asp His Leu Phe Ile Ser
    1325                1330                1335

Asn Leu Pro Glu Ile Val Val Glu Leu Leu Met Thr Leu His Glu
    1340                1345                1350

Pro Ala Asn Ser Ser Ala Ser Gln Ser Thr Asp Leu Cys Asp Phe
    1355                1360                1365

Ser Gly Asp Leu Asp Pro Ala Pro Asn Pro Pro His Phe Pro Ser
    1370                1375                1380

His Val Ile Lys Ala Thr Phe Ala Tyr Ile Ser Asn Cys His Lys
    1385                1390                1395

Thr Lys Leu Lys Ser Ile Leu Glu Ile Leu Ser Lys Ser Pro Asp
    1400                1405                1410

Ser Tyr Gln Lys Ile Leu Leu Ala Ile Cys Glu Gln Ala Ala Glu
    1415                1420                1425

Thr Asn Asn Val Tyr Lys Lys His Arg Ile Leu Lys Ile Tyr His
    1430                1435                1440

Leu Phe Val Ser Leu Leu Leu Lys Asp Ile Lys Ser Gly Leu Gly
    1445                1450                1455

Gly Ala Trp Ala Phe Val Leu Arg Asp Val Ile Tyr Thr Leu Ile
    1460                1465                1470

His Tyr Ile Asn Gln Arg Pro Ser Cys Ile Met Asp Val Ser Leu
    1475                1480                1485

Arg Ser Phe Ser Leu Cys Cys Asp Leu Leu Ser Gln Val Cys Gln
    1490                1495                1500

Thr Ala Val Thr Tyr Cys Lys Asp Ala Leu Glu Asn His Leu His
    1505                1510                1515

Val Ile Val Gly Thr Leu Ile Pro Leu Val Tyr Glu Gln Val Glu
    1520                1525                1530

Val Gln Lys Gln Val Leu Asp Leu Leu Lys Tyr Leu Val Ile Asp
    1535                1540                1545

Asn Lys Asp Asn Glu Asn Leu Tyr Ile Thr Ile Lys Leu Leu Asp
    1550                1555                1560

Pro Phe Pro Asp His Val Val Phe Lys Asp Leu Arg Ile Thr Gln
    1565                1570                1575

Gln Lys Ile Lys Tyr Ser Arg Gly Pro Phe Ser Leu Leu Glu Glu
    1580                1585                1590

Ile Asn His Phe Leu Ser Val Ser Val Tyr Asp Ala Leu Pro Leu
    1595                1600                1605

Thr Arg Leu Glu Gly Leu Lys Asp Leu Arg Arg Gln Leu Glu Leu
    1610                1615                1620

His Lys Asp Gln Met Val Asp Ile Met Arg Ala Ser Gln Asp Asn

1625                1630                1635

Pro Gln Asp Gly Ile Met Val Lys Leu Val Val Asn Leu Leu Gln
    1640                1645                1650

Leu Ser Lys Met Ala Ile Asn His Thr Gly Glu Lys Glu Val Leu
    1655                1660                1665

Glu Ala Val Gly Ser Cys Leu Gly Glu Val Gly Pro Ile Asp Phe
    1670                1675                1680

Ser Thr Ile Ala Ile Gln His Ser Lys Asp Ala Ser Tyr Thr Lys
    1685                1690                1695

Ala Leu Lys Leu Phe Glu Asp Lys Glu Leu Gln Trp Thr Phe Ile
    1700                1705                1710

Met Leu Thr Tyr Leu Asn Asn Thr Leu Val Glu Asp Cys Val Lys
    1715                1720                1725

Val Arg Ser Ala Ala Val Thr Cys Leu Lys Asn Ile Leu Ala Thr
    1730                1735                1740

Lys Thr Gly His Ser Phe Trp Glu Ile Tyr Lys Met Thr Thr Asp
    1745                1750                1755

Pro Met Leu Ala Tyr Leu Gln Pro Phe Arg Thr Ser Arg Lys Lys
    1760                1765                1770

Phe Leu Glu Val Pro Arg Phe Asp Lys Glu Asn Pro Phe Glu Gly
    1775                1780                1785

Leu Asp Asp Ile Asn Leu Trp Ile Pro Leu Ser Glu Asn His Asp
    1790                1795                1800

Ile Trp Ile Lys Thr Leu Thr Cys Ala Phe Leu Asp Ser Gly Gly
    1805                1810                1815

Thr Lys Cys Glu Ile Leu Gln Leu Leu Lys Pro Met Cys Glu Val
    1820                1825                1830

Lys Thr Asp Phe Cys Gln Thr Val Leu Pro Tyr Leu Ile His Asp
    1835                1840                1845

Ile Leu Leu Gln Asp Thr Asn Glu Ser Trp Arg Asn Leu Leu Ser
    1850                1855                1860

Thr His Val Gln Gly Phe Phe Thr Ser Cys Leu Arg His Phe Ser
    1865                1870                1875

Gln Thr Ser Arg Ser Thr Thr Pro Ala Asn Leu Asp Ser Glu Ser
    1880                1885                1890

Glu His Phe Phe Arg Cys Cys Leu Asp Lys Lys Ser Gln Arg Thr
    1895                1900                1905

Met Leu Ala Val Val Asp Tyr Met Arg Arg Gln Lys Arg Pro Ser
    1910                1915                1920

Ser Gly Thr Ile Phe Asn Asp Ala Phe Trp Leu Asp Leu Asn Tyr
    1925                1930                1935

Leu Glu Val Ala Lys Val Ala Gln Ser Cys Ala Ala His Phe Thr
    1940                1945                1950

Ala Leu Leu Tyr Ala Glu Ile Tyr Ala Asp Lys Lys Ser Met Asp
    1955                1960                1965

Asp Gln Glu Lys Arg Ser Leu Ala Phe Glu Glu Gly Ser Gln Ser
    1970                1975                1980

Thr Thr Ile Ser Ser Leu Ser Glu Lys Ser Lys Glu Glu Thr Gly
    1985                1990                1995

Ile Ser Leu Gln Asp Leu Leu Leu Glu Ile Tyr Arg Ser Ile Gly
    2000                2005                2010

Glu Pro Asp Ser Leu Tyr Gly Cys Gly Gly Gly Lys Met Leu Gln
    2015                2020                2025

Pro Ile Thr Arg Leu Arg Thr Tyr Glu His Glu Ala Met Trp Gly
    2030                2035                2040

Lys Ala Leu Val Thr Tyr Asp Leu Glu Thr Ala Ile Pro Ser Ser
    2045                2050                2055

Thr Arg Gln Ala Gly Ile Ile Gln Ala Leu Gln Asn Leu Gly Leu
    2060                2065                2070

Cys His Ile Leu Ser Val Tyr Leu Lys Gly Leu Asp Tyr Glu Asn
    2075                2080                2085

Lys Asp Trp Cys Pro Glu Leu Glu Glu Leu His Tyr Gln Ala Ala
    2090                2095                2100

Trp Arg Asn Met Gln Trp Asp His Cys Thr Ser Val Ser Lys Glu
    2105                2110                2115

Val Glu Gly Thr Ser Tyr His Glu Ser Leu Tyr Asn Ala Leu Gln
    2120                2125                2130

Ser Leu Arg Asp Arg Glu Phe Ser Thr Phe Tyr Glu Ser Leu Lys
    2135                2140                2145

Tyr Ala Arg Val Lys Glu Val Glu Glu Met Cys Lys Arg Ser Leu
    2150                2155                2160

Glu Ser Val Tyr Ser Leu Tyr Pro Thr Leu Ser Arg Leu Gln Ala
    2165                2170                2175

Ile Gly Glu Leu Glu Ser Ile Gly Glu Leu Phe Ser Arg Ser Val
    2180                2185                2190

Thr His Arg Gln Leu Ser Glu Val Tyr Ile Lys Trp Gln Lys His
    2195                2200                2205

Ser Gln Leu Leu Lys Asp Ser Asp Phe Ser Phe Gln Glu Pro Ile
    2210                2215                2220

Met Ala Leu Arg Thr Val Ile Leu Glu Ile Leu Met Glu Lys Glu
    2225                2230                2235

Met Asp Asn Ser Gln Arg Glu Cys Ile Lys Asp Ile Leu Thr Lys
    2240                2245                2250

His Leu Val Glu Leu Ser Ile Leu Ala Arg Thr Phe Lys Asn Thr
    2255                2260                2265

Gln Leu Pro Glu Arg Ala Ile Phe Gln Ile Lys Gln Tyr Asn Ser
    2270                2275                2280

Val Ser Cys Gly Val Ser Glu Trp Gln Leu Glu Glu Ala Gln Val
    2285                2290                2295

Phe Trp Ala Lys Lys Glu Gln Ser Leu Ala Leu Ser Ile Leu Lys
    2300                2305                2310

Gln Met Ile Lys Lys Leu Asp Ala Ser Cys Ala Ala Asn Asn Pro
    2315                2320                2325

Ser Leu Lys Leu Thr Tyr Thr Glu Cys Leu Arg Val Cys Gly Asn
    2330                2335                2340

Trp Leu Ala Glu Thr Cys Leu Glu Asn Pro Ala Val Ile Met Gln
    2345                2350                2355

Thr Tyr Leu Glu Lys Ala Val Glu Val Ala Gly Asn Tyr Asp Gly
    2360                2365                2370

Glu Ser Ser Asp Glu Leu Arg Asn Gly Lys Met Lys Ala Phe Leu
    2375                2380                2385

Ser Leu Ala Arg Phe Ser Asp Thr Gln Tyr Gln Arg Ile Glu Asn
    2390                2395                2400

Tyr Met Lys Ser Ser Glu Phe Glu Asn Lys Gln Ala Leu Leu Lys
    2405                2410                2415

Arg Ala Lys Glu Glu Val Gly Leu Leu Arg Glu His Lys Ile Gln
    2420                2425                2430

Thr Asn Arg Tyr Thr Val Lys Val Gln Arg Glu Leu Glu Leu Asp
    2435                2440                2445

Glu Leu Ala Leu Arg Ala Leu Lys Glu Asp Arg Lys Arg Phe Leu
    2450                2455                2460

Cys Lys Ala Val Glu Asn Tyr Ile Asn Cys Leu Leu Ser Gly Glu
    2465                2470                2475

Glu His Asp Met Trp Val Phe Arg Leu Cys Ser Leu Trp Leu Glu
    2480                2485                2490

Asn Ser Gly Val Ser Glu Val Asn Gly Met Met Lys Arg Asp Gly
    2495                2500                2505

Met Lys Ile Pro Thr Tyr Lys Phe Leu Pro Leu Met Tyr Gln Leu
    2510                2515                2520

Ala Ala Arg Met Gly Thr Lys Met Met Gly Gly Leu Gly Phe His
    2525                2530                2535

Glu Val Leu Asn Asn Leu Ile Ser Arg Ile Ser Met Asp His Pro
    2540                2545                2550

His His Thr Leu Phe Ile Ile Leu Ala Leu Ala Asn Ala Asn Arg
    2555                2560                2565

Asp Glu Phe Leu Thr Lys Pro Glu Val Ala Arg Arg Ser Arg Ile

2570                2575                2580

Thr Lys Asn Val Pro Lys Gln Ser Ser Gln Leu Asp Glu Asp Arg
    2585                2590                2595

Thr Glu Ala Ala Asn Arg Ile Ile Cys Thr Ile Arg Ser Arg Arg
    2600                2605                2610

Pro Gln Met Val Arg Ser Val Glu Ala Leu Cys Asp Ala Tyr Ile
    2615                2620                2625

Ile Leu Ala Asn Leu Asp Ala Thr Gln Trp Lys Thr Gln Arg Lys
    2630                2635                2640

Gly Ile Asn Ile Pro Ala Asp Gln Pro Ile Thr Lys Leu Lys Asn
    2645                2650                2655

Leu Glu Asp Val Val Val Pro Thr Met Glu Ile Lys Val Asp His
    2660                2665                2670

Thr Gly Glu Tyr Gly Asn Leu Val Thr Ile Gln Ser Phe Lys Ala
    2675                2680                2685

Glu Phe Arg Leu Ala Gly Gly Val Asn Leu Pro Lys Ile Ile Asp
    2690                2695                2700

Cys Val Gly Ser Asp Gly Lys Glu Arg Arg Gln Leu Val Lys Gly
    2705                2710                2715

Arg Asp Asp Leu Arg Gln Asp Ala Val Met Gln Gln Val Phe Gln
    2720                2725                2730

Met Cys Asn Thr Leu Leu Gln Arg Asn Thr Glu Thr Arg Lys Arg
    2735                2740                2745

Lys Leu Thr Ile Cys Thr Tyr Lys Val Val Pro Leu Ser Gln Arg
    2750                2755                2760

Ser Gly Val Leu Glu Trp Cys Thr Gly Thr Val Pro Ile Gly Glu
    2765                2770                2775

Phe Leu Val Asn Asn Glu Asp Gly Ala His Lys Arg Tyr Arg Pro
    2780                2785                2790

Asn Asp Phe Ser Ala Phe Gln Cys Gln Lys Lys Met Met Glu Val
    2795                2800                2805

Gln Lys Lys Ser Phe Glu Glu Lys Tyr Glu Val Phe Met Asp Val
    2810                2815                2820

Cys Gln Asn Phe Gln Pro Val Phe Arg Tyr Phe Cys Met Glu Lys
    2825                2830                2835

Phe Leu Asp Pro Ala Ile Trp Phe Glu Lys Arg Leu Ala Tyr Thr
    2840                2845                2850

Arg Ser Val Ala Thr Ser Ser Ile Val Gly Tyr Ile Leu Gly Leu
    2855                2860                2865

Gly Asp Arg His Val Gln Asn Ile Leu Ile Asn Glu Gln Ser Ala
    2870                2875                2880

Glu Leu Val His Ile Asp Leu Gly Val Ala Phe Glu Gln Gly Lys
    2885                2890                2895

Ile Leu Pro Thr Pro Glu Thr Val Pro Phe Arg Leu Thr Arg Asp
    2900                2905                2910

Ile Val Asp Gly Met Gly Ile Thr Gly Val Glu Gly Val Phe Arg
    2915                2920                2925

Arg Cys Cys Glu Lys Thr Met Glu Val Met Arg Asn Ser Gln Glu
    2930                2935                2940

Thr Leu Leu Thr Ile Val Glu Val Leu Leu Tyr Asp Pro Leu Phe
    2945                2950                2955

Asp Trp Thr Met Asn Pro Leu Lys Ala Leu Tyr Leu Gln Gln Arg
    2960                2965                2970

Pro Glu Asp Glu Thr Glu Leu His Pro Thr Leu Asn Ala Asp Asp
    2975                2980                2985

Gln Glu Cys Lys Arg Asn Leu Ser Asp Ile Asp Gln Ser Phe Asn
    2990                2995                3000

Lys Val Ala Glu Arg Val Leu Met Arg Leu Gln Glu Lys Leu Lys
    3005                3010                3015

Gly Val Glu Glu Gly Thr Val Leu Ser Val Gly Gly Gln Val Asn
    3020                3025                3030

Leu Leu Ile Gln Gln Ala Ile Asp Pro Lys Asn Leu Ser Arg Leu
    3035                3040                3045

Phe Pro Gly Trp Lys Ala Trp Val
    3050                3055

<210> 17
<211> 3717
<212> DNA
<213> Homo sapiens

<220>
<221> CDS
<222> (365)..(1939)

<400> 17
gaacagcgaa gacagcgtga gcctgggccg ttgcctcgag gctctcgccc ggcttctctt      60

gccgacccgc cacgtttgtt tggatttaat cttcaggttg ccggcgcccg cccgcccgct     120

ggcctcgcgg tgtgagaggg aagcacccgt gcctgtggct ggtggctggc gcctggaggg     180

tccgcacacc cgcccggccg cgccgcttgc ccgcggcagc cgcgtccctg aaccgcggag     240

tcgtgtttgt gtttgacccg cgggcgccgg tggcgcgcgg ccgaggccgg tgtcggcggg     300

gcggggcggt cgcggcggag gcagaggaag agggagcggg agctctgcga ggccgggcgc     360

cgcc atg gaa ctg ggc ccg gag ccc ccg cac cgc cgc cgc ctg ctc ttc     409
     Met Glu Leu Gly Pro Glu Pro Pro His Arg Arg Arg Leu Leu Phe
     1               5                   10                  15

gcc tgc agc ccc cct ccc gcg tcg cag ccc gtc gtg aag gcg cta ttt     457
Ala Cys Ser Pro Pro Pro Ala Ser Gln Pro Val Val Lys Ala Leu Phe
                20                  25                  30

ggc gct tca gcc gcc ggg gga ctg tcg cct gtc acc aac ctg acc gtc     505
Gly Ala Ser Ala Ala Gly Gly Leu Ser Pro Val Thr Asn Leu Thr Val
            35                  40                  45

act atg gac cag ctg cag ggt ctg ggc agt gat tat gag caa cca ctg     553
Thr Met Asp Gln Leu Gln Gly Leu Gly Ser Asp Tyr Glu Gln Pro Leu
        50                  55                  60

gag gtg aag aac aac agt aat ctg cag aga atg ggc tcc tcc gag tca     601
Glu Val Lys Asn Asn Ser Asn Leu Gln Arg Met Gly Ser Ser Glu Ser
    65                  70                  75

aca gat tca ggt ttc tgt cta gat tct cct ggg cca ttg gac agt aaa     649
Thr Asp Ser Gly Phe Cys Leu Asp Ser Pro Gly Pro Leu Asp Ser Lys
80                  85                  90                  95

gaa aac ctt gaa aat cct atg aga aga ata cat tcc cta cct cag aag     697
Glu Asn Leu Glu Asn Pro Met Arg Arg Ile His Ser Leu Pro Gln Lys
                100                 105                 110

ctg ttg gga tgt agt cca gct ctg aag agg agc cat tct gat tct ctt     745
Leu Leu Gly Cys Ser Pro Ala Leu Lys Arg Ser His Ser Asp Ser Leu
            115                 120                 125

gac cat gac atc ttt cag ctc atc gac cca gat gag aac aag gaa aat     793
Asp His Asp Ile Phe Gln Leu Ile Asp Pro Asp Glu Asn Lys Glu Asn
        130                 135                 140

gaa gcc ttt gag ttt aag aag cca gta aga cct gta tct cgt ggc tgc 841
Glu Ala Phe Glu Phe Lys Lys Pro Val Arg Pro Val Ser Arg Gly Cys
    145                 150                 155

ctg cac tct cat gga ctc cag gag ggt aaa gat ctc ttc aca cag agg 889
Leu His Ser His Gly Leu Gln Glu Gly Lys Asp Leu Phe Thr Gln Arg
160                 165                 170                 175

cag aac tct gcc cca gct cgg atg ctt tcc tca aat gaa aga gat agc 937
Gln Asn Ser Ala Pro Ala Arg Met Leu Ser Ser Asn Glu Arg Asp Ser
                180                 185                 190

agt gaa cca ggg aat ttc att cct ctt ttt aca ccc cag tca cct gtg 985
Ser Glu Pro Gly Asn Phe Ile Pro Leu Phe Thr Pro Gln Ser Pro Val
            195                 200                 205

aca gcc act ttg tct gat gag gat gat ggc ttc gtg gac ctt ctc gat 1033
Thr Ala Thr Leu Ser Asp Glu Asp Asp Gly Phe Val Asp Leu Leu Asp
        210                 215                 220

gga gag aat ctg aag aat gag gag gag acc ccc tcg tgc atg gca agc 1081
Gly Glu Asn Leu Lys Asn Glu Glu Glu Thr Pro Ser Cys Met Ala Ser
    225                 230                 235

ctc tgg aca gct cct ctc gtc atg aga act aca aac ctt gac aac cga 1129
Leu Trp Thr Ala Pro Leu Val Met Arg Thr Thr Asn Leu Asp Asn Arg
240                 245                 250                 255

tgc aag ctg ttt gac tcc cct tcc ctg tgt agc tcc agc act cgg tca 1177
Cys Lys Leu Phe Asp Ser Pro Ser Leu Cys Ser Ser Ser Thr Arg Ser
                260                 265                 270

gtg ttg aag aga cca gaa cga tct caa gag gag tct cca cct gga agt 1225
Val Leu Lys Arg Pro Glu Arg Ser Gln Glu Glu Ser Pro Pro Gly Ser
            275                 280                 285

aca aag agg agg aag agc atg tct ggg gcc agc ccc aaa gag tca act 1273
Thr Lys Arg Arg Lys Ser Met Ser Gly Ala Ser Pro Lys Glu Ser Thr
        290                 295                 300

aat cca gag aag gcc cat gag act ctt cat cag tct tta tcc ctg gca 1321
Asn Pro Glu Lys Ala His Glu Thr Leu His Gln Ser Leu Ser Leu Ala
    305                 310                 315

tct tcc ccc aaa gga acc att gag aac att ttg gac aat gac cca agg 1369
Ser Ser Pro Lys Gly Thr Ile Glu Asn Ile Leu Asp Asn Asp Pro Arg
320                 325                 330                 335

gac ctt ata gga gac ttc tcc aag ggt tat ctc ttt cat aca gtt gct 1417
Asp Leu Ile Gly Asp Phe Ser Lys Gly Tyr Leu Phe His Thr Val Ala
                340                 345                 350

ggg aaa cat cag gat tta aaa tac atc tct cca gaa att atg gca tct 1465
Gly Lys His Gln Asp Leu Lys Tyr Ile Ser Pro Glu Ile Met Ala Ser
            355                 360                 365

gtt ttg aat ggc aag ttt gcc aac ctc att aaa gag ttt gtt atc atc 1513
Val Leu Asn Gly Lys Phe Ala Asn Leu Ile Lys Glu Phe Val Ile Ile
        370                 375                 380

gac tgt cga tac cca tat gaa tac gag gga ggc cac atc aag ggt gca 1561
Asp Cys Arg Tyr Pro Tyr Glu Tyr Glu Gly Gly His Ile Lys Gly Ala

385                 390                 395

gtg aac ttg cac atg gaa gaa gag gtt gaa gac ttc tta ttg aag aag      1609
Val Asn Leu His Met Glu Glu Glu Val Glu Asp Phe Leu Leu Lys Lys
400                 405                 410                 415

ccc att gta cct act gat ggc aag cgt gtc att gtt gtg ttt cac tgc      1657
Pro Ile Val Pro Thr Asp Gly Lys Arg Val Ile Val Val Phe His Cys
                420                 425                 430

gag ttt tct tct gag aga ggt ccc cgc atg tgc cgg tat gtg aga gag      1705
Glu Phe Ser Ser Glu Arg Gly Pro Arg Met Cys Arg Tyr Val Arg Glu
            435                 440                 445

aga gat cgc ctg ggt aat gaa tac ccc aaa ctc cac tac cct gag ctg      1753
Arg Asp Arg Leu Gly Asn Glu Tyr Pro Lys Leu His Tyr Pro Glu Leu
        450                 455                 460

tat gtc ctg aag ggg gga tac aag gag ttc ttt atg aaa tgc cag tct      1801
Tyr Val Leu Lys Gly Gly Tyr Lys Glu Phe Phe Met Lys Cys Gln Ser
    465                 470                 475

tac tgt gag ccc cct agc tac cgg ccc atg cac cac gag gac ttt aaa      1849
Tyr Cys Glu Pro Pro Ser Tyr Arg Pro Met His His Glu Asp Phe Lys
480                 485                 490                 495

gaa gac ctg aag aag ttc cgc acc aag agc cgg acc tgg gca ggg gag      1897
Glu Asp Leu Lys Lys Phe Arg Thr Lys Ser Arg Thr Trp Ala Gly Glu
                500                 505                 510

aag agc aag agg gag atg tac agt cgt ctg aag aag ctc tga              1939
Lys Ser Lys Arg Glu Met Tyr Ser Arg Leu Lys Lys Leu
            515                 520

gggcggcagg accagccagc agcagcccaa gcttccctcc atcccccttt accctctttg      1999

ctgcagagaa acttaagcaa aggggacagc tgtgtgacat ttggagaggg ggcctgggac      2059

ttccatgcct taaacctacc tcccacactc ccaaggttgg agcccagggc atcttgctgg      2119

ctacgcctct tctgtccctg ttagacgtcc tccgtccata tcagaactgt gccacaatgc      2179

agttctgagc accgtgtcaa gctgctctga gccacagtgg gatgaaccag ccggggcctt      2239

atcgggctcc agccatctca tgaggggaga ggagacggag gggagtagag aagttacaca      2299

gaaatgctgc tggccaaata gcaaagacaa cctgggaagg aaaggtcttt gtgggataat      2359

ccatatgttt aatttattca acttcatcaa tcactttatt ttattttttt ttctaactcc      2419

tggagactta ttttactgct tcattaggtt gaaatactgc cattctaggt agggttttat      2479

tatcccaggg actacctcgg cttttaattt aaaaaaaaaa aagaagtggg taagaaaatg      2539

caaacctgtt ataagttatc ggacagaaag ctaggtgctc tgtcaccccc aggaggcgct      2599

gtggtactgg ggctgctgct atttaagcca agaactgagg tcctggtgag agcgttggac      2659

ccaggcttgg ctgcctgaca taagctaaat ctcccagacc caccactggc taccgatatc      2719

tatttggtgg gaggtgtggc cctgttcttc ctcaccccag ttccatgaca ttggctggta      2779

taggagccac agtcaggaaa gcacttgagg cagcatctgt tgggccaccc ccggctcagt      2839

gctggaatgt tgcagtgtag gtttcccagg gaaggggggt gggggtaggt gggctccaca 2899

ggatggggga ggagcatgtc cactgagtat cttccttatg ttgctgtgat attgatagct 2959

tttattttct aatttttaaa aaatggtcat attatgagtc aaagagtatc aaatcagtgt 3019

tggatggacc acccaagggt gaggagaggg gctggaagcc ctgggcatta ggagaaggga 3079

gtgggtgctg gcatggacat gactggatag aattttctca ggagggagct tggtggattt 3139

tgaaggtaaa actttctggg tttatcatgt tttaatttta gagacaggga gtgatgaatc 3199

atcaccggtt gtccccttat ctaactccat aaaagtggga atttcaaaag aacacctcat 3259

ccaaggagct ggggcagact tcattgattc tagagagacc tgtttcagtg cctactcatc 3319

cctgccctct ggtgccagcc tccttaccat cacggcttca ctgaggtgta ggtgggtttt 3379

tcttaaacag gagacagtct ctcccctctt acctcaactt cttggggtgg gaatcagtga 3439

tactggagat ggctagttgc tgtgttacgg gtttgagtta catttggcta taaaacaatc 3499

ttgttgggaa aaatgtgggg gagaggactt cttcctacac gcgcattgag acagattcca 3559

actggttaat gatattgttt gtaagaaaga gattctgttg gttgactgcc taaagagaaa 3619

ggtgggatgg ccttcagatt ataccagctt agctagcatt actaaccaac tgttggaagc 3679

tctgaaaata aaagatcttg aacccataaa aaaaaaaa 3717

<210> 18
<211> 524
<212> PRT
<213> Homo sapiens

<400> 18

Met Glu Leu Gly Pro Glu Pro Pro His Arg Arg Arg Leu Leu Phe Ala
1               5                   10                  15

Cys Ser Pro Pro Pro Ala Ser Gln Pro Val Val Lys Ala Leu Phe Gly
            20                  25                  30

Ala Ser Ala Ala Gly Gly Leu Ser Pro Val Thr Asn Leu Thr Val Thr
        35                  40                  45

Met Asp Gln Leu Gln Gly Leu Gly Ser Asp Tyr Glu Gln Pro Leu Glu
    50                  55                  60

Val Lys Asn Asn Ser Asn Leu Gln Arg Met Gly Ser Ser Glu Ser Thr
65                  70                  75                  80

Asp Ser Gly Phe Cys Leu Asp Ser Pro Gly Pro Leu Asp Ser Lys Glu
                85                  90                  95

Asn Leu Glu Asn Pro Met Arg Arg Ile His Ser Leu Pro Gln Lys Leu
            100                 105                 110

Leu Gly Cys Ser Pro Ala Leu Lys Arg Ser His Ser Asp Ser Leu Asp
        115                 120                 125

His Asp Ile Phe Gln Leu Ile Asp Pro Asp Glu Asn Lys Glu Asn Glu
    130                 135                 140

Ala Phe Glu Phe Lys Lys Pro Val Arg Pro Val Ser Arg Gly Cys Leu
145                 150                 155                 160

His Ser His Gly Leu Gln Glu Gly Lys Asp Leu Phe Thr Gln Arg Gln
                165                 170                 175

Asn Ser Ala Pro Ala Arg Met Leu Ser Ser Asn Glu Arg Asp Ser Ser
            180                 185                 190

Glu Pro Gly Asn Phe Ile Pro Leu Phe Thr Pro Gln Ser Pro Val Thr
        195                 200                 205

Ala Thr Leu Ser Asp Glu Asp Asp Gly Phe Val Asp Leu Leu Asp Gly
    210                 215                 220

Glu Asn Leu Lys Asn Glu Glu Glu Thr Pro Ser Cys Met Ala Ser Leu
225                 230                 235                 240

Trp Thr Ala Pro Leu Val Met Arg Thr Thr Asn Leu Asp Asn Arg Cys
                245                 250                 255

Lys Leu Phe Asp Ser Pro Ser Leu Cys Ser Ser Ser Thr Arg Ser Val
            260                 265                 270

Leu Lys Arg Pro Glu Arg Ser Gln Glu Glu Ser Pro Pro Gly Ser Thr
        275                 280                 285

Lys Arg Arg Lys Ser Met Ser Gly Ala Ser Pro Lys Glu Ser Thr Asn
    290                 295                 300

Pro Glu Lys Ala His Glu Thr Leu His Gln Ser Leu Ser Leu Ala Ser
305                 310                 315                 320

Ser Pro Lys Gly Thr Ile Glu Asn Ile Leu Asp Asn Asp Pro Arg Asp
                325                 330                 335

Leu Ile Gly Asp Phe Ser Lys Gly Tyr Leu Phe His Thr Val Ala Gly
            340                 345                 350

Lys His Gln Asp Leu Lys Tyr Ile Ser Pro Glu Ile Met Ala Ser Val
        355                 360                 365

Leu Asn Gly Lys Phe Ala Asn Leu Ile Lys Glu Phe Val Ile Ile Asp
    370                 375                 380

Cys Arg Tyr Pro Tyr Glu Tyr Glu Gly Gly His Ile Lys Gly Ala Val
385                 390                 395                 400

Asn Leu His Met Glu Glu Glu Val Glu Asp Phe Leu Leu Lys Lys Pro
                405                 410                 415

Ile Val Pro Thr Asp Gly Lys Arg Val Ile Val Val Phe His Cys Glu
            420                 425                 430

Phe Ser Ser Glu Arg Gly Pro Arg Met Cys Arg Tyr Val Arg Glu Arg
        435                 440                 445

Asp Arg Leu Gly Asn Glu Tyr Pro Lys Leu His Tyr Pro Glu Leu Tyr
    450                 455                 460

Val Leu Lys Gly Gly Tyr Lys Glu Phe Phe Met Lys Cys Gln Ser Tyr
465                 470                 475                 480

Cys Glu Pro Pro Ser Tyr Arg Pro Met His His Glu Asp Phe Lys Glu
                485                 490                 495

Asp Leu Lys Lys Phe Arg Thr Lys Ser Arg Thr Trp Ala Gly Glu Lys
            500                 505                 510

Ser Lys Arg Glu Met Tyr Ser Arg Leu Lys Lys Leu
        515                 520

<210>  19
<211>  13509
<212>  DNA
<213>  Homo sapiens

<220>
<221>  CDS
<222>  (58)..(12444)

<400>  19
ggggcatttc cgggtccggg ccgagcgggc gcacgcgcgg gagcgggact cggcggc        57
atg gcg ggc tcc gga gcc ggt gtg cgt tgc tcc ctg ctg cgg ctg cag       105
gag acc ttg tcc gct gcg gac cgc tgc ggt gct gcc ctg gcc ggt cat       153
caa ctg atc cgc ggc ctg ggg cag gaa tgc gtc ctg agc agc agc ccc 201
gcg gtg ctg gca tta cag aca tct tta gtt ttt tcc aga gat ttc ggt 249
ttg ctt gta ttt gtc cgg aag tca ctc aac agt att gaa ttt cgt gaa 297
tgt aga gaa gaa atc cta aag ttt tta tgt att ttc tta gaa aaa atg 345
ggc cag aag atc gca cct tac tct gtt gaa att aag aac act tgt acc 393
agt gtt tat aca aaa gat aga gct gct aaa tgt aaa att cca gcc ctg 441
gac ctt ctt att aag tta ctt cag act ttt aga agt tct aga ctc atg 489
gat gaa ttt aaa att gga gaa tta ttt agt aaa ttc tat gga gaa ctt 537
gca ttg aaa aaa aaa ata cca gat aca gtt tta gaa aaa gta tat gag 585
ctc cta gga tta ttg ggt gaa gtt cat cct agt gag atg ata aat aat 633
gca gaa aac ctg ttc cgc gct ttt ctg ggt gaa ctt aag acc cag atg 681
aca tca gca gta aga gag ccc aaa cta cct gtt ctg gca gga tgt ctg 729
aag ggg ttg tcc tca ctt ctg tgc aac ttc act aag tcc atg gaa gaa 777
gat ccc cag act tca agg gag att ttt aat ttt gta cta aag gca att 825
cgt cct cag att gat ctg aag aga tat gct gtg ccc tca gct ggc ttg 873
cgc cta ttt gcc ctg cat gca tct cag ttt agc acc tgc ctt ctg gac 921
aac tac gtg tct cta ttt gaa gtc ttg tta aag tgg tgt gcc cac aca 969
aat gta gaa ttg aaa aaa gct gca ctt tca gcc ctg gaa tcc ttt ctg 1017
aaa cag gtt tct aat atg gtg gcg aaa aat gca gaa atg cat aaa aat 1065
aaa ctg cag tac ttt atg gag cag ttt tat gga atc atc aga aat gtg 1113
gat tcg aac aac aag gag tta tct att gct atc cgt gga tat gga ctt 1161
ttt gca gga ccg tgc aag gtt ata aac gca aaa gat gtt gac ttc atg 1209
tac gtt gag ctc att cag cgc tgc aag cag atg ttc ctc acc cag aca 1257
gac act ggt gac gac cgt gtt tat cag atg cca agc ttc ctc cag tct 1305
gtt gca agc gtc ttg ctg tac ctt gac aca gtt cct gag gtg tat act 1353
cca gtt ctg gag cac ctc gtg gtg atg cag ata gac agt ttc cca cag 1401
tac agt cca aaa atg cag ctg gtg tgt tgc aga gcc ata gtg aag gtg 1449
ttc cta gct ttg gca gca aaa ggg cca gtt ctc agg aat tgc att agt 1497
act gtg gtg cat cag ggt tta atc aga ata tgt tct aaa cca gtg gtc 1545
ctt cca aag ggc cct gag tct gaa tct gaa gac cac cgt gct tca ggg 1593
gaa gtc aga act ggc aaa tgg aag gtg ccc aca tac aaa gac tac gtg 1641
gat ctc ttc aga cat ctc ctg agc tct gac cag atg atg gat tct att     1689
tta gca gat gaa gca ttt ttc tct gtg aat tcc tcc agt gaa agt ctg     1737
aat cat tta ctt tat gat gaa ttt gta aaa tcc gtt ttg aag att gtt     1785
gag aaa ttg gat ctt aca ctt gaa ata cag act gtt ggg gaa caa gag     1833
aat gga gat gag gcg cct ggt gtt tgg atg atc cca act tca gat cca     1881
gcg gct aac ttg cat cca gct aaa cct aaa gat ttt tcg gct ttc att     1929
aac ctg gtg gaa ttt tgc aga gag att ctc cct gag aaa caa gca gaa     1977
ttt ttt gaa cca tgg gtg tac tca ttt tca tat gaa tta att ttg caa     2025
tct aca agg ttg ccc ctc atc agt ggt ttc tac aaa ttg ctt tct att     2073
aca gta aga aat gcc aag aaa ata aaa tat ttc gag gga gtt agt cca     2121
aag agt ctg aaa cac tct cct gaa gac cca gaa aag tat tct tgc ttt     2169
gct tta ttt gtg aaa ttt ggc aaa gag gtg gca gtt aaa atg aag cag     2217
tac aaa gat gaa ctt ttg gcc tct tgt ttg acc ttt ctt ctg tcc ttg     2265
cca cac aac atc att gaa ctc gat gtt aga gcc tac gtt cct gca ctg     2313
cag atg gct ttc aaa ctg ggc ctg agc tat acc ccc ttg gca gaa gta     2361
ggc ctg aat gct cta gaa gaa tgg tca att tat att gac aga cat gta     2409
atg cag cct tat tac aaa gac att ctc ccc tgc ctg gat gga tac ctg        2457
aag act tca gcc ttg tca gat gag acc aag aat aac tgg gaa gtg tca        2505
gct ctt tct cgg gct gcc cag aaa gga ttt aat aaa gtg gtg tta aag        2553
cat ctg aag aag aca aag aac ctt tca tca aac gaa gca ata tcc tta        2601
gaa gaa ata aga att aga gta gta caa atg ctt gga tct cta gga gga        2649
caa ata aac aaa aat ctt ctg aca gtc acg tcc tca gat gag atg atg        2697
aag agc tat gtg gcc tgg gac aga gag aag cgg ctg agc ttt gca gtg        2745
ccc ttt aga gag atg aaa cct gtc att ttc ctg gat gtg ttc ctg cct        2793
cga gtc aca gaa tta gcg ctc aca gcc agt gac aga caa act aaa gtt        2841
gca gcc tgt gaa ctt tta cat agc atg gtt atg ttt atg ttg ggc aaa        2889
gcc acg cag atg cca gaa ggg gga cag gga gcc cca ccc atg tac cag        2937
ctc tat aag cgg acg ttt cct gtg ctg ctt cga ctt gcg tgt gat gtt        2985
gat cag gtg aca agg caa ctg tat gag cca cta gtt atg cag ctg att        3033
cac tgg ttc act aac aac aag aaa  ttt gaa agt cag gat  act gtt gcc        3081
tta cta  gaa gct ata ttg gat  gga att gtg gac cct  gtt gac agt        3126
act tta  aga gat ttt tgt ggt  cgg tgt att cga gaa  ttc ctt aaa        3171
tgg tcc att aag caa ata aca cca cag cag cag gag aag agt cca         3216
gta aac acc aaa tcg ctt ttc aag cga ctt tat agc ctt gcg ctt         3261
cac ccc aat gct ttc aag agg ctg gga gca tca ctt gcc ttt aat         3306
aat atc tac agg gaa ttc agg gaa gaa gag tct ctg gtg gaa cag         3351
ttt gtg ttt gaa gcc ttg gtg ata tac atg gag agt ctg gcc tta         3396
gca cat gca gat gag aag tcc tta ggt aca att caa cag tgt tgt         3441
gat gcc att gat cac cta tgc cgc atc att gaa aag aag cat gtt         3486
tct tta aat aaa gca aag aaa cga cgt ttg ccg cga gga ttt cca         3531
cct tcc gca tca ttg tgt tta ttg gat ctg gtc aag tgg ctt tta         3576
gct cat tgt ggg agg ccc cag aca gaa tgt cga cac aaa tcc att         3621
gaa ctc ttt tat aaa ttc gtt cct tta ttg cca ggc aac aga tcc         3666
cct aat ttg tgg ctg aaa gat gtt ctc aag gaa gaa ggt gtc tct         3711
ttt ctc atc aac acc ttt gag ggg ggt ggc tgt ggc cag ccc tcg         3756
ggc atc ctg gcc cag ccc acc ctc ttg tac ctt cgg ggg cca ttc         3801
agc ctg cag gcc acg cta tgc tgg ctg gac ctg ctc ctg gcc gcg         3846
ttg gag tgc tac aac acg ttc att ggc gag aga act gta gga gcg 3891
ctc cag gtc cta ggt act gaa gcc cag tct tca ctt ttg aaa gca 3936
gtg gct ttc ttc tta gaa agc att gcc atg cat gac att ata gca 3981
gca gaa aag tgc ttt ggc act ggg gca gca ggt aac aga aca agc 4026
cca caa gag gga gaa agg tac aac tac agc aaa tgc acc gtt gtg 4071
gtc cgg att atg gag ttt acc acg act ctg cta aac acc tcc ccg 4116
gaa gga tgg aag ctc ctg aag aag gac ttg tgt aat aca cac ctg 4161
atg aga gtc ctg gtg cag acg ctg tgt gag ccc gca agc ata ggt 4206
ttc aac atc gga gac gtc cag gtt atg gct cat ctt cct gat gtt 4251
tgt gtg aat ctg atg aaa gct cta aag atg tcc cca tac aaa gat 4296
atc cta gag acc cat ctg aga gag aaa ata aca gca cag agc att 4341
gag gag ctt tgt gcc gtc aac ttg tat ggc cct gac gcg caa gtg 4386
gac agg agc agg ctg gct gct gtt gtg tct gcc tgt aaa cag ctt 4431
cac aga gct ggg ctt ctg cat aat ata tta ccg tct cag tcc aca 4476
gat ttg cat cat tct gtt ggc aca gaa ctt ctt tcc ctg gtt tat 4521
aaa ggc att gcc cct gga gat gag aga cag tgt ctg cct tct cta 4566
gac ctc agt tgt aag cag ctg gcc agc gga ctt ctg gag tta gcc 4611
ttt gct ttt gga gga ctg tgt gag cgc ctt gtg agt ctt ctc ctg 4656
aac cca gcg gtg ctg tcc acg gcg tcc ttg ggc agc tca cag ggc 4701
agc gtc atc cac ttc tcc cat ggg gag tat ttc tat agc ttg ttc 4746
tca gaa acg atc aac acg gaa tta ttg aaa aat ctg gat ctt gct 4791
gta ttg gag ctc atg cag tct tca gtg gat aat acc aaa atg gtg 4836
agt gcc gtt ttg aac ggc atg tta gac cag agc ttc agg gag cga 4881
gca aac cag aaa cac caa gga ctg aaa ctt gcg act aca att ctg 4926
caa cac tgg aag aag tgt gat tca tgg tgg gcc aaa gat tcc cct 4971
ctc gaa act aaa atg gca gtg ctg gcc tta ctg gca aaa att tta 5016
cag att gat tca tct gta tct ttt aat aca agt cat ggt tca ttc 5061
cct gaa gtc ttt aca aca tat att agt cta ctt gct gac aca aag 5106
ctg gat cta cat tta aag ggc caa gct gtc act ctt ctt cca ttc 5151
ttc acc agc ctc act gga ggc agt ctg gag gaa ctt aga cgt gtt 5196
ctg gag cag ctc atc gtt gct cac ttc ccc atg cag tcc agg gaa 5241
ttt cct cca gga act ccg cgg ttc aat aat tat gtg gac tgc atg 5286
aaa aag ttt cta gat gca ttg gaa tta tct caa agc cct atg ttg        5331
ttg gaa ttg atg aca gaa gtt ctt tgt cgg gaa cag cag cat gtc        5376
atg gaa gaa tta ttt caa tcc agt ttc agg agg att gcc aga agg        5421
ggt tca tgt gtc aca caa gta ggc ctt ctg gaa agc gtg tat gaa        5466
atg ttc agg aag gat gac ccc cgc cta agt ttc aca cgc cag tcc        5511
ttt gtg gac cgc tcc ctc ctc act ctg ctg tgg cac tgt agc ctg        5556
gat gct ttg aga gaa ttc ttc agc aca att gtg gtg gat gcc att        5601
gat gtg ttg aag tcc agg ttt aca aag cta aat gaa tct acc ttt        5646
gat act caa atc acc aag aag atg ggc tac tat aag att cta gac        5691
gtg atg tat tct cgc ctt ccc aaa gat gat gtt cat gct aag gaa        5736
tca aaa att aat caa gtt ttc cat ggc tcg tgt att aca gaa gga        5781
aat gaa ctt aca aag aca ttg att aaa ttg tgc tac gat gca ttt        5826
aca gag aac atg gca gga gag aat cag ctg ctg gag agg aga aga        5871
ctt tac cat tgt gca gca tac aac tgc gcc ata tct gtc atc tgc        5916
tgt gtc ttc aat gag tta aaa ttt tac caa ggt ttt ctg ttt agt        5961
gaa aaa cca gaa aag aac ttg ctt att ttt gaa aat ctg atc gac        6006
ctg aag cgc cgc tat aat ttt cct gta gaa gtt gag gtt cct atg 6051
gaa aga aag aaa aag tac att gaa att agg aaa gaa gcc aga gaa 6096
gca gca aat ggg gat tca gat ggt cct tcc tat atg tct tcc ctg 6141
tca tat ttg gca gac agt acc ctg agt gag gaa atg agt caa ttt 6186
gat ttc tca acc gga gtt cag agc tat tca tac agc tcc caa gac 6231
cct aga cct gcc act ggt cgt ttt cgg aga cgg gag cag cgg gac 6276
ccc acg gtg cat gat gat gtg ctg gag ctg gag atg gac gag ctc 6321
aat cgg cat gag tgc atg gcg ccc ctg acg gcc ctg gtc aag cac 6366
atg cac aga agc ctg ggc ccg cct caa gga gaa gag gat tca gtg 6411
cca aga gat ctt cct tct tgg atg aaa ttc ctc cat ggc aaa ctg 6456
gga aat cca ata gta cca tta aat atc cgt ctc ttc tta gcc aag 6501
ctt gtt att aat aca gaa gag gtc ttt cgc cct tac gcg aag cac 6546
tgg ctt agc ccc ttg ctg cag ctg gct gct tct gaa aac aat gga 6591
gga gaa gga att cac tac atg gtg gtt gag ata gtg gcc act att 6636
ctt tca tgg aca ggc ttg gcc act cca aca ggg gtc cct aaa gat 6681
gaa gtg tta gca aat cga ttg ctt aat ttc cta atg aaa cat gtc 6726
ttt cat cca aaa aga gct gtg ttt aga cac aac ctt gaa att ata 6771
aag acc ctt gtc gag tgc tgg aag gat tgt tta tcc atc cct tat 6816
agg tta ata ttt gaa aag ttt tcc ggt aaa gat cct aat tct aaa 6861
gac aac tca gta ggg att caa ttg cta ggc atc gtg atg gcc aat 6906
gac ctg cct ccc tat gac cca cag tgt ggc atc cag agt agc gaa 6951
tac ttc cag gct ttg gtg aat aat atg tcc ttt gta aga tat aaa 6996
gaa gtg tat gcc gct gca gca gaa gtt cta gga ctt ata ctt cga 7041
tat gtt atg gag aga aaa aac ata ctg gag gag tct ctg tgt gaa 7086
ctg gtt gcg aaa caa ttg aag caa cat cag aat act atg gag gac 7131
aag ttt att gtg tgc ttg aac aaa gtg acc aag agc ttc cct cct 7176
ctt gca gac agg ttc atg aat gct gtg ttc ttt ctg ctg cca aaa 7221
ttt cat gga gtg ttg aaa aca ctc tgt ctg gag gtg gta ctt tgt 7266
cgt gtg gag gga atg aca gag ctg tac ttc cag tta aag agc aag 7311
gac ttc gtt caa gtc atg aga cat aga gat gat gaa aga caa aaa 7356
gta tgt ttg gac ata att tat aag atg atg cca aag tta aaa cca 7401
gta gaa ctc cga gaa ctt ctg aac ccc gtt gtg gaa ttc gtt tcc 7446
cat cct tct aca aca tgt agg gaa caa atg tat aat att ctc atg 7491
tgg att cat gat aat tac aga gat cca gaa agt gag aca gat aat 7536
gac tcc cag gaa ata ttt aag ttg gca aaa gat gtg ctg att caa 7581
gga ttg atc gat gag aac cct gga ctt caa tta att att cga aat 7626
ttc tgg agc cat gaa act agg tta cct tca aat acc ttg gac cgg 7671
ttg ctg gca cta aat tcc tta tat tct cct aag ata gaa gtg cac 7716
ttt tta agt tta gca aca aat ttt ctg ctc gaa atg acc agc atg 7761
agc cca gat tat cca aac ccc atg ttc gag cat cct ctg tca gaa 7806
tgc gaa ttt cag gaa tat acc att gat tct gat tgg cgt ttc cga 7851
agt act gtt ctc act ccg atg ttt gtg gag acc cag gcc tcc cag 7896
ggc act ctc cag acc cgt acc cag gaa ggg tcc ctc tca gct cgc 7941
tgg cca gtg gca ggg cag ata agg gcc acc cag cag cag cat gac 7986
ttc aca ctg aca cag act gca gat gga aga agc tca ttt gat tgg 8031
ctg acc ggg agc agc act gac ccg ctg gtc gac cac acc agt ccc 8076
tca tct gac tcc ttg ctg ttt gcc cac aag agg agt gaa agg tta 8121
cag aga gca ccc ttg aag tca gtg ggg cct gat ttt ggg aaa aaa        8166
agg ctg ggc ctt cca ggg gac gag gtg gat aac aaa gtg aaa ggt        8211
gcg gcc ggc cgg acg gac cta cta cga ctg cgc aga cgg ttt atg        8256
agg gac cag gag aag ctc agt ttg atg tat gcc aga aaa ggc gtt        8301
gct gag caa aaa cga gag aag gaa atc aag agt gag tta aaa atg        8346
aag cag gat gcc cag gtc gtt ctg tac aga agc tac cgg cac gga        8391
gac ctt cct gac att cag atc aag cac agc agc ctc atc acc ccg        8436
tta cag gcc gtg gcc cag agg gac cca ata att gca aaa cag ctc        8481
ttt agc agc ttg ttt tct gga att ttg aaa gag atg gat aaa ttt        8526
aag aca ctg tct gaa aaa aac aac atc act caa aag ttg ctt caa        8571
gac ttc aat cgt ttt ctt aat acc acc ttc tct ttc ttt cca ccc        8616
ttt gtc tct tgt att cag gac att agc tgt cag cac gca gcc ctg        8661
ctg agc ctc gac cca gcg gct gtt agc gct ggt tgc ctg gcc agc        8706
cta cag cag ccc gtg ggc atc cgc ctg cta gag gag gct ctg ctc        8751
cgc ctg ctg cct gct gag ctg cct gcc aag cga gtc cgt ggg aag        8796
gcc cgc ctc cct cct gat gtc ctc aga tgg gtg gag ctt gct aag        8841
ctg tat aga tca att gga gaa tac gac gtc ctc cgt ggg att ttt 8886
acc agt gag ata gga aca aag caa atc act cag agt gca tta tta 8931
gca gaa gcc aga agt gat tat tct gaa gct gct aag cag tat gat 8976
gag gct ctc aat aaa caa gac tgg gta gat ggt gag ccc aca gaa 9021
gcc gag aag gat ttt tgg gaa ctt gca tcc ctt gac tgt tac aac 9066
cac ctt gct gag tgg aaa tca ctt gaa tac tgt tct aca gcc agt 9111
ata gac agt gag aac ccc cca gac cta aat aaa atc tgg agt gaa 9156
cca ttt tat cag gaa aca tat cta cct tac atg atc cgc agc aag 9201
ctg aag ctg ctg ctc cag gga gag gct gac cag tcc ctg ctg aca 9246
ttt att gac aaa gct atg cac ggg gag ctc cag aag gcg att cta 9291
gag ctt cat tac agt caa gag ctg agt ctg ctt tac ctc ctg caa 9336
gat gat gtt gac aga gcc aaa tat tac att caa aat ggc att cag 9381
agt ttt atg cag aat tat tct agt att gat gtc ctc tta cac caa 9426
agt aga ctc acc aaa ttg cag tct gta cag gct tta aca gaa att 9471
cag gag ttc atc agc ttt ata agc aaa caa ggc aat tta tca tct 9516
caa gtt ccc ctt aag aga ctt ctg aac acc tgg aca aac aga tat 9561
cca gat gct aaa atg gac cca atg aac atc tgg gat gac atc atc 9606
aca aat cga tgt ttc ttt ctc agc aaa ata gag gag aag ctt acc 9651
cct ctt cca gaa gat aat agt atg aat gtg gat caa gat gga gac 9696
ccc agt gac agg atg gaa gtg caa gag cag gaa gaa gat atc agc 9741
tcc ctg atc agg agt tgc aag ttt tcc atg aaa atg aag atg ata 9786
gac agt gcc cgg aag cag aac aat ttc tca ctt gct atg aaa cta 9831
ctg aag gag ctg cat aaa gag tca aaa acc aga gac gat tgg ctg 9876
gtg agc tgg gtg cag agc tac tgc cgc ctg agc cac tgc cgg agc 9921
cgg tcc cag ggc tgc tct gag cag gtg ctc act gtg ctg aaa aca 9966
gtc tct ttg ttg gat gag aac aac gtg tca agc tac tta agc aaa 10011
aat att ctg gct ttc cgt gac cag aac att ctc ttg ggt aca act 10056
tac agg atc ata gcg aat gct ctc agc agt gag cca gcc tgc ctt 10101
gct gaa atc gag gag gac aag gct aga aga atc tta gag ctt tct 10146
gga tcc agt tca gag gat tca gag aag gtg atc gcg ggt ctg tac 10191
cag aga gca ttc cag cac ctc tct gag gct gtg cag gcg gct gag 10236
gag gag gcc cag cct ccc tcc tgg agc tgt ggg cct gca gct ggg 10281
gtg att gat gct tac atg acg ctg gca gat ttc tgt gac caa cag 10326
ctg cgc aag gag gaa gag aat gca tca gtt att gat tct gca gaa 10371
ctg cag gcg tat cca gca ctt gtg gtg gag aaa atg ttg aaa gct 10416
tta aaa tta aat tcc aat gaa gcc aga ttg aag ttt cct aga tta 10461
ctt cag att ata gaa cgg tat cca gag gag act ttg agc ctc atg 10506
aca aaa gag atc tct tcc gtt ccc tgc tgg cag ttc atc agc tgg 10551
atc agc cac atg gtg gcc tta ctg gac aaa gac caa gcc gtt gct 10596
gtt cag cac tct gtg gaa gaa atc act gat aac tac ccg cag gct 10641
att gtt tat ccc ttc atc ata agc agc gaa agc tat tcc ttc aag 10686
gat act tct act ggt cat aag aat aag gag ttt gtg gca agg att 10731
aaa agt aag ttg gat caa gga gga gtg att caa gat ttt att aat 10776
gcc tta gat cag ctc tct aat cct gaa ctg ctc ttt aag gat tgg 10821
agc aat gat gta aga gct gaa cta gca aaa acc cct gta aat aaa 10866
aaa aac att gaa aaa atg tat gaa aga atg tat gca gcc ttg ggt 10911
gac cca aag gct cca ggc ctg ggg gcc ttt aga agg aag ttt att 10956
cag act ttt gga aaa gaa ttt gat aaa cat ttt ggg aaa gga ggt      11001
tct aaa cta ctg aga atg aag ctc agt gac ttc aac gac att acc      11046
aac atg cta ctt tta aaa atg aac aaa gac tca aag ccc cct ggg      11091
aat ctg aaa gaa tgt tca ccc tgg atg agc gac ttc aaa gtg gag      11136
ttc ctg aga aat gag ctg gag att ccc ggt cag tat gac ggt agg      11181
gga aag cca ttg cca gag tac cac gtg cga atc gcc ggg ttt gat      11226
gag cgg gtg aca gtc atg gcg tct ctg cga agg ccc aag cgc atc      11271
atc atc cgt ggc cat gac gag agg gaa cac cct ttc ctg gtg aag      11316
ggt ggc gag gac ctg cgg cag gac cag cgc gtg gag cag ctc ttc      11361
cag gtc atg aat ggg atc ctg gcc caa gac tcc gcc tgc agc cag      11406
agg gcc ctg cag ctg agg acc tat agc gtt gtg ccc atg acc tcc      11451
agg tta gga tta att gag tgg ctt gaa aat act gtt acc ttg aag      11496
gac ctt ctt ttg aac acc atg tcc caa gag gag aag gcg gct tac      11541
ctg agt gat ccc agg gca ccg ccg tgt gaa tat aaa gat tgg ctg      11586
aca aaa atg tca gga aaa cat gat gtt gga gct tac atg cta atg      11631
tat aag ggc gct aat cgt act gaa aca gtc acg tct ttt aga aaa      11676
cga gaa agt aaa gtg cct gct gat ctc tta aag cgg gcc ttc gtg    11721
agg atg agt aca agc cct gag gct ttc ctg gcg ctc cgc tcc cac    11766
ttc gcc agc tct cac gct ctg ata tgc atc agc cac tgg atc ctc    11811
ggg att gga gac aga cat ctg aac aac ttt atg gtg gcc atg gag    11856
act ggc ggc gtg atc ggg atc gac ttt ggg cat gcg ttt gga tcc    11901
gct aca cag ttt ctg cca gtc cct gag ttg atg cct ttt cgg cta    11946
act cgc cag ttt atc aat ctg atg tta cca atg aaa gaa acg ggc    11991
ctt atg tac agc atc atg gta cac gca ctc cgg gcc ttc cgc tca    12036
gac cct ggc ctg ctc acc aac acc atg gat gtg ttt gtc aag gag    12081
ccc tcc ttt gat tgg aaa aat ttt gaa cag aaa atg ctg aaa aaa    12126
gga ggg tca tgg att caa gaa ata aat gtt gct gaa aaa aat tgg    12171
tac ccc cga cag aaa ata tgt tac gct aag aga aag tta gca ggt    12216
gcc aat cca gca gtc att act tgt gat gag cta ctc ctg ggt cat    12261
gag aag gcc cct gcc ttc aga gac tat gtg gct gtg gca cga gga    12306
agc aaa gat cac aac att cgt gcc caa gaa cca gag agt ggg ctt    12351
tca gaa  gag act caa gtg aag  tgc ctg atg gac cag  gca aca gac     12396
ccc aac  atc ctt ggc aga acc  tgg gaa gga tgg gag  ccc tgg atg     12441
tga ggtctgtggg agtctgcaga tagaaagcat tacattgttt aaagaatcta         12494
ctatactttg gttggcagca ttccatgagc tgattttcct gaaacactaa agagaaatgt    12554
cttttgtgct acagtttcgt agcatgagtt taaatcaaga ttatgatgag taaatgtgta    12614
tgggttaaat caaagataag gttatagtaa catcaaagat taggtgaggt ttatagaaag    12674
atagatatcc aggcttacca aagtattaag tcaagaatat aatatgtgat cagctttcaa    12734
agcatttaca agtgctgcaa gttagtgaaa cagctgtctc cgtaaatgga ggaaatgtgg    12794
ggaagccttg gaatgccctt ctggttctgg cacattggaa agcacactca gaaggcttca    12854
tcaccaagat tttgggagag taaagctaag tatagttgat gtaacattgt agaagcagca    12914
taggaacaat aagaacaata ggtaaagcta taattatggc ttatatttag aaatgactgc    12974
atttgatatt ttaggatatt tttctaggtt ttttcctttc attttattct cttctagttt    13034
tgacatttta tgatagattt gctctctaga aggaaacgtc tttatttagg agggcaaaaa    13094
ttttggtcat agcattcact tttgctattc caatctacaa ctggaagata cataaaagtg    13154
ctttgcattg aatttgggat aacttcaaaa atcccatggt tgttgttagg gatagtacta    13214
agcatttcag ttccaggaga ataaaagaaa ttcctatttg aaatgaattc ctcatttgga    13274
ggaaaaaaag catgcattct agcacaacaa gatgaaatta tggaatacaa aagtggctcc    13334
ttcccatgtg cagtccctgt ccccccccgc cagtcctcca cacccaaact gtttctgatt    13394
ggcttttagc tttttgttgt tttttttttt ccttctaaca cttgtatttg gaggctcttc    13454
tgtgattttg agaagtatac tcttgagtgt ttaataaagt ttttttccaa aagta         13509

<210>  20
<211>  4128
<212>  PRT
<213>  Homo sapiens

<400>  20

<210>  21
<211>  3279
<212>  DNA
<213>  Homo sapiens

<220>
<221>  CDS
<222>  (332)..(3025)

<400>  21
gacgtcgcgc gggctcccgg gcggggcggg tccggccgcc tccgagcccg gccggcagcc      60
cccggcctta aagcgcgggc tgtccggagg ggtcggcttt cccaccgagg atttggcact     120
ctggtgaggg aaaagggcga aagagaaaag cgagcagccg tcctttcaca gcctcagaaa     180
gtgctcgctt cccttcgggg gctttcgcga atcccgaggc aatctcggag gcggtatttg     240
acctgtccaa agacgacttg atacctctat aatgtaacag aaaaggtcag aaaatattaa     300
gcaagtagaa gtgtggagca tattaagcaa g atg aac atc tcg gga agc agc      352
tgt gga agc cct aac tct gca gat aca tct agt gac ttt aag gac ctt      400
tgg aca aaa cta aaa gaa tgt cat gat aga gaa gta caa ggt tta caa      448
gta aaa gta acc aag cta aaa cag gaa cga atc tta gat gca caa aga      496
cta gaa gaa ttc ttc acc aaa aat caa cag ctg agg gaa cag cag aaa      544
gtc ctt cat gaa acc att aaa gtt tta gaa gat cgg tta aga gca ggc      592
tta tgt gat cgc tgt gca gta act gaa gaa cat atg cgg aaa aaa cag      640
caa gag ttt gaa aat atc cgg cag cag aat ctt aaa ctt att aca gaa      688
ctt atg aat gaa agg aat act cta cag gaa gaa aat aaa aag ctt tct      736
gaa caa ctc cag cag aaa att gag aat gat caa cag cat caa gca gct      784
gag ctt gaa tgt gag gaa gac gtt att cca gat tca ccg ata aca gcc     832
ttc tca ttt tct ggc gtt aac cgg cta cga aga aag gag aac ccc cat     880
gtc cga tac ata gaa caa aca cat act aaa ttg gag cac tct gtg tgt     928
gca aat gaa atg aga aaa gtt tcc aag tct tca act cat cca caa cat     976
aat cct aat gaa aat gaa att cta gta gct gac act tat gac caa agt     1024
caa tct cca atg gcc aaa gca cat gga aca agc agc tat acc cct gat     1072
aag tca tct ttt aat tta gct aca gtt gtt gct gaa aca ctt gga ctt     1120
ggt gtt caa gaa gaa tct gaa act caa ggt ccc atg agc ccc ctt ggt     1168
gat gag ctc tac cac tgt ctg gaa gga aat cac aag aaa cag cct ttt     1216
gag gaa tct aca aga aat act gaa gat agt tta aga ttt tca gat tct     1264
act tca aag act cct cct caa gaa gaa tta cct act cga gtg tca tct     1312
cct gta ttt gga gct acc tct agt atc aaa agt ggt tta gat ttg aat     1360
aca agt ttg tcc cct tct ctt tta cag cct ggg aaa aaa aaa cat ctg     1408
aaa aca ctc cct ttt agc aac act tgt ata tct aga tta gaa aaa act     1456
aga tca aaa tct gaa gat agt gcc ctt ttc aca cat cac agt ctt ggg     1504
tct gaa gtg aac aag atc att atc cag tca tct aat aaa cag ata ctt     1552
ata aat aaa aat ata agt gaa tcc cta ggt gaa cag aat agg act gag 1600
tac ggt aaa gat tct aac act gat aaa cat ttg gag ccc ctg aaa tca 1648
ttg gga ggc cga aca tcc aaa agg aag aaa act gag gaa gaa agt gaa 1696
cat gaa gta agc tgc ccc caa gct tct ttt gat aaa gaa aat gct ttc 1744
cct ttt cca atg gat aat cag ttt tcc atg aat gga gac tgt gtg atg 1792
gat aaa cct ctg gat ctg tct gat cga ttt tca gct att cag cgt caa 1840
gag aaa agc caa gga agt gag act tct aaa aac aaa ttt agg caa gtg 1888
act ctt tat gag gct ttg aag acc att cca aag ggc ttt tcc tca agc 1936
cgt aag gcc tca gat ggc aac tgc acg ttg ccc aaa gat tcc cca ggg 1984
gag ccc tgt tca cag gaa tgc atc atc ctt cag ccc ttg aat aaa tgc 2032
tct cca gac aat aaa cca tca tta caa ata aaa gaa gaa aat gct gtc 2080
ttt aaa att cct cta cgt cca cgt gaa agt ttg gag act gag aat gtt 2128
tta gat gac ata aag agt gct ggt tct cat gag cca ata aaa ata caa 2176
acc agg tca gac cat gga gga tgt gaa ctt gca tca gtt ctt cag tta 2224
aat cca tgt aga act ggt aaa ata aag tct cta caa aac aac caa gat 2272
gta tcc ttt gaa aat atc cag tgg agt ata gat ccg gga gca gac ctt 2320
tct cag tat aaa atg gat gtt act gta ata gat aca aag gat ggc agt      2368
cag tca aaa tta gga gga gag aca gtg gac atg gac tgt aca ttg gtt      2416
agt gaa acc gtt ctc tta aaa atg aag aag caa gag cag aag gga gaa      2464
aaa agt tca aat gaa gaa aga aaa atg aat gat agc ttg gaa gat atg      2512
ttt gat cgg aca aca cat gaa gag tat gaa tcc tgt ttg gca gac agt      2560
ttc tcc caa gca gca gat gaa gag gag gaa ttg tct act gcc aca aag      2608
aaa cta cac act cat ggt gat aaa caa gac aaa gtc aag cag aaa gcg      2656
ttt gtg gag ccg tat ttt aaa ggt gat gaa aga gag act agc ttg caa      2704
aat ttt cct cat att gag gtg gtt cgg aaa aaa gag gag aga aga aaa      2752
ctg ctt ggg cac acg tgt aag gaa tgt gaa att tat tat gca gat atg      2800
cca gca gaa gaa aga gaa aag aaa ttg gct tcc tgc tca aga cac cga      2848
ttc cgc tac att cca ccc aac aca cca gag aat ttt tgg gaa gtt ggt      2896
ttt cct tcc act cag act tgt atg gaa aga ggt tat att aag gaa gat      2944
ctt gat cct tgt cct cgt cca aaa aga cgt cag cct tac aac gca ata      2992
ttt tct cca aaa ggc aag gag cag aag aca tag acgttgaaac agaaacagaa      3045
ggatgaagga cagttttttc cttcttagtt atttatagtt aaagttggta ctaaacattg      3105
atttttttga tcttctgtaa atggatttat aaatcagttt tctattgaaa atgtttgtga 3165
tattttgctt ttgcaccttt aaaacaataa ggcgctttca ttttgcactc taacttaaga 3225
gtttttactt tatgtagtga tacctaatac aattttgaaa atacaaaaaa aaaa 3279

<210> 22
<211> 897
<212> PRT
<213> Homo sapiens

<400> 22

<210>  23
<211>  3520
<212>  DNA
<213>  Homo sapiens

<220>
<221>  CDS
<222>  (221)..(1495)

<400> 23
   aattcccagc aggccttggg cctcagtgcg gccgcgaagc agagcgggct gtagagcctt 60
   gcgcgcgcag tggggatgga acgttgctag gcttagcggg tctggctgct gggggcccga 120
   gcagcacgct cggagccgcc gcgcgccaaa gcgggaatct gggaggcgag cagctctgca 180
   gttaatgcac gtattttaaa ctcccgggcc tgcggacgct atg cac agg aag cac 235
   Met His Arg Lys His
   1 5
   ctc cag gag att cca gac ctg agt agc aac gtt gcc acc agc ttc acg 283
   Leu Gln Glu Ile Pro Asp Leu Ser Ser Asn Val Ala Thr Ser Phe Thr
   10 15 20
   tgg gga tgg gat tcc agc aag act tct gaa ctg ctg tca ggc atg ggg 331
   Trp Gly Trp Asp Ser Ser Lys Thr Ser Glu Leu Leu Ser Gly Met Gly
   25 30 35
   gtc tcc gcc ctg gag aaa gag gag ccc gac agt gag aac atc ccc cag 379
   Val Ser Ala Leu Glu Lys Glu Glu Pro Asp Ser Glu Asn Ile Pro Gln
   40 45 50
   gaa ctg ctc tca aac ctg ggc cac ccg gag agc ccc cca cgg aaa cgg 427
   Glu Leu Leu Ser Asn Leu Gly His Pro Glu Ser Pro Pro Arg Lys Arg
   55 60 65
   ctg aag agc aaa ggg agt gac aaa gac ttt gtg att gtc cgc agg cct 475
   Leu Lys Ser Lys Gly Ser Asp Lys Asp Phe Val Ile Val Arg Arg Pro
   70 75 80 85
   aag cta aat cga gag aac ttt cca ggt gtt tca tgg gac tcc ctt ccg 523
   Lys Leu Asn Arg Glu Asn Phe Pro Gly Val Ser Trp Asp Ser Leu Pro
   90 95 100
   gat gag ctg ctc ttg gga atc ttt tcc tgt ctg tgc ctc cct gag ctg 571
   Asp Glu Leu Leu Leu Gly Ile Phe Ser Cys Leu Cys Leu Pro Glu Leu
   105 110 115
   cta aag gtc tct ggt gtt tgt aag agg tgg tat cgc cta gcg tct gat 619
   Leu Lys Val Ser Gly Val Cys Lys Arg Trp Tyr Arg Leu Ala Ser Asp
   120 125 130
   gag tct cta tgg cag acc tta gac ctc aca ggt aaa aat ctg cac ccg 667
   Glu Ser Leu Trp Gln Thr Leu Asp Leu Thr Gly Lys Asn Leu His Pro
   135 140 145
   gat gtg act ggt cgg ttg ctg tct caa ggg gtg att gcc ttc cgc tgc 715
   Asp Val Thr Gly Arg Leu Leu Ser Gln Gly Val Ile Ala Phe Arg Cys
   150 155 160 165
   cca cga tca ttt atg gac caa cca ttg gct gaa cat ttc agc cct ttt 763
   Pro Arg Ser Phe Met Asp Gln Pro Leu Ala Glu His Phe Ser Pro Phe
   170 175 180
   cgt gta cag cac atg gac cta tcg aac tca gtt ata gaa gtg tcc acc 811
   Arg Val Gln His Met Asp Leu Ser Asn Ser Val Ile Glu Val Ser Thr
   185 190 195
   ctc cac ggc ata ctg tct cag tgt tcc aag ttg cag aat cta agc ctg 859
   Leu His Gly Ile Leu Ser Gln Cys Ser Lys Leu Gln Asn Leu Ser Leu
   200 205 210
   gaa ggc ctg cgg ctt tcg gat ccc att gtc aat act ctc gca aaa aac     907
   Glu Gly Leu Arg Leu Ser Asp Pro Ile Val Asn Thr Leu Ala Lys Asn
       215                 220                 225
   tca aat tta gtg cga ctt aac ctt tct ggg tgt tct gga ttc tct gaa     955
   Ser Asn Leu Val Arg Leu Asn Leu Ser Gly Cys Ser Gly Phe Ser Glu
   230                 235                 240                 245
   ttt gcc ctg cag act ttg cta agc agc tgt tcc aga ctg gat gag ctg     1003
   Phe Ala Leu Gln Thr Leu Leu Ser Ser Cys Ser Arg Leu Asp Glu Leu
                   250                 255                 260
   aac ctc tcc tgg tgt ttt gat ttc act gaa aag cat gta cag gtg gct     1051
   Asn Leu Ser Trp Cys Phe Asp Phe Thr Glu Lys His Val Gln Val Ala
               265                 270                 275
   gtt gcg cat gtg tca gag acc atc acc cag ctg aat ctt agc ggc tac     1099
   Val Ala His Val Ser Glu Thr Ile Thr Gln Leu Asn Leu Ser Gly Tyr
           280                 285                 290
   aga aag aat ctc cag aaa tca gat ctc tct act tta gtt aga aga tgc     1147
   Arg Lys Asn Leu Gln Lys Ser Asp Leu Ser Thr Leu Val Arg Arg Cys
       295                 300                 305
   ccc aat ctt gtc cat cta gac tta agt gat agt gtc atg cta aag aat     1195
   Pro Asn Leu Val His Leu Asp Leu Ser Asp Ser Val Met Leu Lys Asn
   310                 315                 320                 325
   gac tgc ttt cag gaa ttt ttc cag ctc aac tac ctc caa cac cta tca     1243
   Asp Cys Phe Gln Glu Phe Phe Gln Leu Asn Tyr Leu Gln His Leu Ser
                   330                 335                 340
   ctc agt cgg tgc tat gat ata ata cct gaa act tta ctt gaa ctt gga     1291
   Leu Ser Arg Cys Tyr Asp Ile Ile Pro Glu Thr Leu Leu Glu Leu Gly
               345                 350                 355
   gaa att ccc aca cta aaa aca cta caa gtt ttt gga atc gtg cca gat     1339
   Glu Ile Pro Thr Leu Lys Thr Leu Gln Val Phe Gly Ile Val Pro Asp
           360                 365                 370
   ggt acc ctt caa ctg tta aag gaa gcc ctt cct cat cta cag att aat     1387
   Gly Thr Leu Gln Leu Leu Lys Glu Ala Leu Pro His Leu Gln Ile Asn
       375                 380                 385
   tgc tcc cat ttc acc acc att gcc agg cca act att ggc aac aaa aag     1435
   Cys Ser His Phe Thr Thr Ile Ala Arg Pro Thr Ile Gly Asn Lys Lys
   390                 395                 400                 405
   aac cag gag ata tgg ggc atc aaa tgc cga ctg aca ctg caa aag ccc     1483
   Asn Gln Glu Ile Trp Gly Ile Lys Cys Arg Leu Thr Leu Gln Lys Pro
                   410                 415                 420
   agt tgt cta tga agtatttatt gcaggatggt gtctcttctt tagaacaggg     1535
   Ser Cys Leu
   aaaataggca ggaagcccaa ttgctggagt acttagctag ttttattctt ggttttccct     1595
   ttgccttcat tctgcaagta tactagggag ccatttgaga gggaaaacta tgaaatcttg     1655
   ctttttgaaa tgattctaaa agcttctatc actgctttgc tcttaagagc caaagttgta     1715
   ggccttttga aattttagga gagtgagcct ataatttcaa gataccttaa agagcaaaat 1775
   ttgagccacc tcttccaagt gcccttctta ctaagtctat tcagaatcaa gcttaaaaat 1835
   taccaccagc aaacaatctt catagcccat ataactttta tctatttaat tttatagtat 1895
   tgctttataa gacagcttag aagaacaata agctatttgt attatgagct gaacaaaaag 1955
   agaatcatag gatagtagcg tctgaggcca tcttttctag gaataggaaa gagaaaaatg 2015
   tatttgaatt ttgcctttag atttgaaatt aggttaatag aaataagtaa ccccatgtaa 2075
   ttcaccttaa aacttaacaa aagaccaaac attacaaaac ccagagatat agaatcaata 2135
   taggatttga aggcccagca gacagttttc tatgacaggt taatctgaag tatcctgtaa 2195
   tgttcattaa gttactgtgt ttccagaatc taaattagat gagaaatata attgtggttt 2255
   tctaacttga taatcaaatt atgttaacat gggtccttta gcttttaaaa tgacttgctt 2315
   tgttttagaa aggtggtatt aatccactct ctattcttga aaatttggat gggagaattc 2375
   tgaagttgcc tgctgttttc ctttagcgct gaggttctta aggttacttt tatattactc 2435
   tggaatcaag tattttaaat tgtatttttt ttttaaatga tctctcagca ataattgttt 2495
   gaaactatcc atatataagg ttatcagacc tacagttccc taagaggaac tgcatgttct 2555
   cttcaatcag aaatatacag tagaagcagg tatatcttcc atgcagtttc agtagtaagc 2615
   actacttata cctacataag agttaaaatc cagatgtggg accttttgat accatcagtg 2675
   atatatattt ttttaaactg gtacagagaa gtgaaaagat taaattctac ttctattttt 2735
   tttttttttt tttgagacgg agtctcgctc tgtcaccaag gccggagtgc agtggtgcga 2795
   tctcggctca ctgcaagctc cgcctcccag gttcacgtca ttctcctgcc tcagcctccc 2855
   gactagctgg gactacaggc gcccaccacc acgcccggct aatttttttg tatttttagt 2915
   agagacgggg tttcaccatg ttagccagga tggtctcaat ctcctgacct catgatccgc 2975
   ccgtcttggc ctcccaaagt gctgggatta caggcatgag caactgcgcc cagccaaatt 3035
   ctacttctta aaaatcacaa aaactagttt aaattgatga cttgttcgta tgttcaaaat 3095
   gtaacaacaa aaaaagctaa caccagtcat ttatattaac tttttttttt taaatcaaaa 3155
   attgttaatg ttagaaacat actatgaagt gcctttatct gcttagacct aaggaagatt 3215
   ttaaagttgg gttgcacagg aaatgatgat gcttcaattt cttaatagtt aaaaagtgct 3275
   aaatactact tgaaattatt gtttacagat tagtgacaag agctggggtt aggatccggt 3335
   tggactctga catcggatgc cctcaaacat acagaacttc caaactcaag tccagccata 3395
   agctattttg ccaacatgtc agagtaatct gtatttttgt atgtgatttc tacttttata 3455
   gacttgtttt aaaacaataa aacacatttt tataaaaatg agtgcttaaa aaaaaaaaaa 3515
   aaaaa 3520

<210>  24
<211>  424
<212>  PRT
<213>  Homo sapiens

<400>  24

Met His Arg Lys His Leu Gln Glu Ile Pro Asp Leu Ser Ser Asn Val
1               5                   10                  15

Ala Thr Ser Phe Thr Trp Gly Trp Asp Ser Ser Lys Thr Ser Glu Leu
            20                  25                  30

Leu Ser Gly Met Gly Val Ser Ala Leu Glu Lys Glu Glu Pro Asp Ser
        35                  40                  45

Glu Asn Ile Pro Gln Glu Leu Leu Ser Asn Leu Gly His Pro Glu Ser
    50                  55                  60

Pro Pro Arg Lys Arg Leu Lys Ser Lys Gly Ser Asp Lys Asp Phe Val
65                  70                  75                  80

Ile Val Arg Arg Pro Lys Leu Asn Arg Glu Asn Phe Pro Gly Val Ser
                85                  90                  95

Trp Asp Ser Leu Pro Asp Glu Leu Leu Leu Gly Ile Phe Ser Cys Leu
            100                 105                 110

Cys Leu Pro Glu Leu Leu Lys Val Ser Gly Val Cys Lys Arg Trp Tyr
        115                 120                 125

Arg Leu Ala Ser Asp Glu Ser Leu Trp Gln Thr Leu Asp Leu Thr Gly
    130                 135                 140

Lys Asn Leu His Pro Asp Val Thr Gly Arg Leu Leu Ser Gln Gly Val
145                 150                 155                 160

Ile Ala Phe Arg Cys Pro Arg Ser Phe Met Asp Gln Pro Leu Ala Glu
                165                 170                 175

His Phe Ser Pro Phe Arg Val Gln His Met Asp Leu Ser Asn Ser Val
            180                 185                 190

Ile Glu Val Ser Thr Leu His Gly Ile Leu Ser Gln Cys Ser Lys Leu
        195                 200                 205

Gln Asn Leu Ser Leu Glu Gly Leu Arg Leu Ser Asp Pro Ile Val Asn
    210                 215                 220

Thr Leu Ala Lys Asn Ser Asn Leu Val Arg Leu Asn Leu Ser Gly Cys
225                 230                 235                 240

Ser Gly Phe Ser Glu Phe Ala Leu Gln Thr Leu Leu Ser Ser Cys Ser
                245                 250                 255

Arg Leu Asp Glu Leu Asn Leu Ser Trp Cys Phe Asp Phe Thr Glu Lys
            260                 265                 270

His Val Gln Val Ala Val Ala His Val Ser Glu Thr Ile Thr Gln Leu
        275                 280                 285

Asn Leu Ser Gly Tyr Arg Lys Asn Leu Gln Lys Ser Asp Leu Ser Thr
    290                 295                 300

Leu Val Arg Arg Cys Pro Asn Leu Val His Leu Asp Leu Ser Asp Ser
305                 310                 315                 320

Val Met Leu Lys Asn Asp Cys Phe Gln Glu Phe Phe Gln Leu Asn Tyr
                325                 330                 335

Leu Gln His Leu Ser Leu Ser Arg Cys Tyr Asp Ile Ile Pro Glu Thr
            340                 345                 350

Leu Leu Glu Leu Gly Glu Ile Pro Thr Leu Lys Thr Leu Gln Val Phe
        355                 360                 365

Gly Ile Val Pro Asp Gly Thr Leu Gln Leu Leu Lys Glu Ala Leu Pro
    370                 375                 380

His Leu Gln Ile Asn Cys Ser His Phe Thr Thr Ile Ala Arg Pro Thr
385                 390                 395                 400

Ile Gly Asn Lys Lys Asn Gln Glu Ile Trp Gly Ile Lys Cys Arg Leu
                405                 410                 415

Thr Leu Gln Lys Pro Ser Cys Leu
            420

<210>  25
<211>  4562
<212>  DNA
<213>  Homo sapiens

<220>
<221>  CDS
<222>  (129)..(2756)

<400>  25

ggttggaatt ttggcgggtt cagctgtgaa cgaagaaggc gtcccggcat cggccaagat 60

tctacattgc tcatctgggc atctgagcct ccttcgaagt ttcctgtcac aactgtcctc 120

ttgacagc atg gat gag gag gaa gac aat ctg tct ctg ctg acc gca ctg 170
Met Asp Glu Glu Glu Asp Asn Leu Ser Leu Leu Thr Ala Leu
1               5                   10

ctg gaa gaa aat gag tca gcc ttg gat tgt aat tca gaa gaa aat aac 218
Leu Glu Glu Asn Glu Ser Ala Leu Asp Cys Asn Ser Glu Glu Asn Asn
15                  20                  25                  30

ttc ttg acg cgg gaa aat ggc gag ccc gac gca ttt gat gag ctc ttt 266
Phe Leu Thr Arg Glu Asn Gly Glu Pro Asp Ala Phe Asp Glu Leu Phe
                35                  40                  45

gat gcc gac ggc gac ggt gaa tct tat aca gaa gag gct gat gat gga 314
Asp Ala Asp Gly Asp Gly Glu Ser Tyr Thr Glu Glu Ala Asp Asp Gly
            50                  55                  60

gaa aca gga gag aca aga gac gaa aag gaa aat ctg gcc act ctc ttt 362
Glu Thr Gly Glu Thr Arg Asp Glu Lys Glu Asn Leu Ala Thr Leu Phe
        65                  70                  75

gga gat atg gag gac tta aca gat gaa gaa gaa gtt ccc gca tca cag 410
Gly Asp Met Glu Asp Leu Thr Asp Glu Glu Glu Val Pro Ala Ser Gln
    80                  85                  90

tca act gaa aat agg gtc ctc cct gct cct gcc ccc agg cga gag aaa 458
Ser Thr Glu Asn Arg Val Leu Pro Ala Pro Ala Pro Arg Arg Glu Lys
95                  100                 105                 110

acg aat gaa gag ttg caa gag gaa tta agg aat ttg caa gag caa atg 506
Thr Asn Glu Glu Leu Gln Glu Glu Leu Arg Asn Leu Gln Glu Gln Met
                115                 120                 125

aag gcc tta caa gag cag cta aaa gta aca aca att aaa cag aca gca 554
Lys Ala Leu Gln Glu Gln Leu Lys Val Thr Thr Ile Lys Gln Thr Ala
            130                 135                 140

agc cca gcc cgt ctg caa aaa tcc cct gta gag aag tct ccc cgg cca 602
Ser Pro Ala Arg Leu Gln Lys Ser Pro Val Glu Lys Ser Pro Arg Pro
        145                 150                 155

cct ctt aag gag agg aga gtt cag aga att cag gag tca aca tgc ttt 650
Pro Leu Lys Glu Arg Arg Val Gln Arg Ile Gln Glu Ser Thr Cys Phe
    160                 165                 170

tct gcg gag ctt gat gtc cct gcg cta cca aga acc aag agg gtg gct 698
Ser Ala Glu Leu Asp Val Pro Ala Leu Pro Arg Thr Lys Arg Val Ala
175                 180                 185                 190

cga aca cca aag gct tca cct cca gat ccc aaa agc tca tct tca agg 746
Arg Thr Pro Lys Ala Ser Pro Pro Asp Pro Lys Ser Ser Ser Ser Arg
                195                 200                 205

atg aca agt gca ccc tcc caa ccc cta cag acg att tct cgg aac aaa 794
Met Thr Ser Ala Pro Ser Gln Pro Leu Gln Thr Ile Ser Arg Asn Lys
            210                 215                 220

cct agt ggg ata act aga ggt caa att gtg ggg acc cca gga agt tct 842
Pro Ser Gly Ile Thr Arg Gly Gln Ile Val Gly Thr Pro Gly Ser Ser
        225                 230                 235

ggg gaa acg act caa ccc atc tgt gtg gaa gcc ttc tct ggt ctg cgg 890
Gly Glu Thr Thr Gln Pro Ile Cys Val Glu Ala Phe Ser Gly Leu Arg
    240                 245                 250

ctc agg cgg cct cga gta tcc tcc aca gaa atg aac aag aaa atg acc 938
Leu Arg Arg Pro Arg Val Ser Ser Thr Glu Met Asn Lys Lys Met Thr
255                 260                 265                 270

ggc cga aaa ctg atc aga ctg tct cag atc aag gaa aag atg gcc aga 986
Gly Arg Lys Leu Ile Arg Leu Ser Gln Ile Lys Glu Lys Met Ala Arg
                275                 280                 285

gag aag ctg gaa gaa ata gat tgg gtg aca ttt ggg gtt ata ttg aag 1034
Glu Lys Leu Glu Glu Ile Asp Trp Val Thr Phe Gly Val Ile Leu Lys
            290                 295                 300

aag gtt acg cca cag agt gtg aat agt gga aaa acc ttc agc ata tgg 1082
Lys Val Thr Pro Gln Ser Val Asn Ser Gly Lys Thr Phe Ser Ile Trp
        305                 310                 315

aaa ctg aat gat ctt cgt gac ctg aca caa tgt gtg tcc ttg ttc tta 1130
Lys Leu Asn Asp Leu Arg Asp Leu Thr Gln Cys Val Ser Leu Phe Leu
    320                 325                 330

ttt gga gaa gtt cac aaa gcg ctc tgg aag acg gag cag ggg act gtc 1178
Phe Gly Glu Val His Lys Ala Leu Trp Lys Thr Glu Gln Gly Thr Val
335                 340                 345                 350

gta ggg atc ctc aat gcc aac ccc atg aag ccc aag gat ggt tca gag 1226
Val Gly Ile Leu Asn Ala Asn Pro Met Lys Pro Lys Asp Gly Ser Glu
                355                 360                 365

gag gtg tgt tta tct atc gat cat cct cag aag gtc tta att atg ggt 1274
Glu Val Cys Leu Ser Ile Asp His Pro Gln Lys Val Leu Ile Met Gly
            370                 375                 380

gaa gct ctt gac ctg gga acc tgt aaa gcc aag aag aag aat gga gag 1322
Glu Ala Leu Asp Leu Gly Thr Cys Lys Ala Lys Lys Lys Asn Gly Glu
        385                 390                 395

ccg tgc acg cag act gtg aat ttg cgt gac tgt gag tac tgt cag tac 1370
Pro Cys Thr Gln Thr Val Asn Leu Arg Asp Cys Glu Tyr Cys Gln Tyr
    400                 405                 410

cat gtc cag gct cag tac aag aag ctc agc gca aag cgt gcg gat ctg 1418
His Val Gln Ala Gln Tyr Lys Lys Leu Ser Ala Lys Arg Ala Asp Leu
415                 420                 425                 430

cag tcc acc ttc tct gga gga cga att cca aag aag ttt gcc cgc aga 1466
Gln Ser Thr Phe Ser Gly Gly Arg Ile Pro Lys Lys Phe Ala Arg Arg
                435                 440                 445

ggc acc agc ctc aaa gaa cgg ctg tgc caa gat ggc ttt tac tac gga 1514
Gly Thr Ser Leu Lys Glu Arg Leu Cys Gln Asp Gly Phe Tyr Tyr Gly
            450                 455                 460

ggg gtt tct tct gcc tcg tat gca gct tca att gca gca gct gtg gct 1562
Gly Val Ser Ser Ala Ser Tyr Ala Ala Ser Ile Ala Ala Ala Val Ala
        465                 470                 475

cct aag aag aag att caa acc act ctg agt aat ctg gtt gtt aag ggc 1610
Pro Lys Lys Lys Ile Gln Thr Thr Leu Ser Asn Leu Val Val Lys Gly

480                 485                 490

aca aac ttg atc atc cag gaa aca cgg caa aaa ctc gga ata ccc cag        1658
Thr Asn Leu Ile Ile Gln Glu Thr Arg Gln Lys Leu Gly Ile Pro Gln
495                 500                 505                 510

aag agc ctg tct tgc tct gag gag ttc aag gaa ctg atg gac ctg ccg        1706
Lys Ser Leu Ser Cys Ser Glu Glu Phe Lys Glu Leu Met Asp Leu Pro
                515                 520                 525

acg tgt gga gcc agg aac tta aaa caa cat tta gcc aaa gcc aca gct        1754
Thr Cys Gly Ala Arg Asn Leu Lys Gln His Leu Ala Lys Ala Thr Ala
            530                 535                 540

tca ggg att atg ggg agc cca aaa cca gcc atc aag tcc atc tcg gcc        1802
Ser Gly Ile Met Gly Ser Pro Lys Pro Ala Ile Lys Ser Ile Ser Ala
        545                 550                 555

tca gca ctc ttg aag caa cag aag cag cgg atg ttg gag atg agg aga        1850
Ser Ala Leu Leu Lys Gln Gln Lys Gln Arg Met Leu Glu Met Arg Arg
    560                 565                 570

agg aaa tca gaa gaa ata cag aag cga ttt ctg cag agc tca agt gaa        1898
Arg Lys Ser Glu Glu Ile Gln Lys Arg Phe Leu Gln Ser Ser Ser Glu
575                 580                 585                 590

gtt gag agc cca gct gtg cca tct tca tca aga cag ccc cct gct cag        1946
Val Glu Ser Pro Ala Val Pro Ser Ser Ser Arg Gln Pro Pro Ala Gln
                595                 600                 605

cct cca cgg aca gga tcc gag ttc ccc agg ctg gag gga gcc ccg gcc        1994
Pro Pro Arg Thr Gly Ser Glu Phe Pro Arg Leu Glu Gly Ala Pro Ala
            610                 615                 620

aca atg acg ccc aag ctg ggg cga ggt gtc ttg gaa gga gat gat gtt        2042
Thr Met Thr Pro Lys Leu Gly Arg Gly Val Leu Glu Gly Asp Asp Val
        625                 630                 635

ctc ttt tat gat gag tca cca cca cca aga cca aaa ctg agt gct tta        2090
Leu Phe Tyr Asp Glu Ser Pro Pro Pro Arg Pro Lys Leu Ser Ala Leu
    640                 645                 650

gca gaa gcc aaa aag tta gct gct atc acc aaa tta agg gca aaa ggc        2138
Ala Glu Ala Lys Lys Leu Ala Ala Ile Thr Lys Leu Arg Ala Lys Gly
655                 660                 665                 670

cag gtt ctt aca aaa aca aac cca aac agc att aag aag aaa caa aag        2186
Gln Val Leu Thr Lys Thr Asn Pro Asn Ser Ile Lys Lys Lys Gln Lys
                675                 680                 685

gac cct cag gac atc ctg gag gtg aag gaa cgt gta gaa aaa aac acc        2234
Asp Pro Gln Asp Ile Leu Glu Val Lys Glu Arg Val Glu Lys Asn Thr
            690                 695                 700

atg ttt tct tct caa gct gag gat gaa ttg gag cct gcc agg aaa aaa        2282
Met Phe Ser Ser Gln Ala Glu Asp Glu Leu Glu Pro Ala Arg Lys Lys
        705                 710                 715

agg aga gaa caa ctt gcc tat ctg gaa tct gag gaa ttt cag aaa atc        2330
Arg Arg Glu Gln Leu Ala Tyr Leu Glu Ser Glu Glu Phe Gln Lys Ile
    720                 725                 730

cta aaa gca aaa tca aaa cac aca ggc atc ctg aaa gag gcc gag gct        2378

Leu Lys Ala Lys Ser Lys His Thr Gly Ile Leu Lys Glu Ala Glu Ala
735                 740                 745                 750

gag atg cag gag cgc tac ttt gag cca ctg gtg aaa aaa gaa caa atg      2426
Glu Met Gln Glu Arg Tyr Phe Glu Pro Leu Val Lys Lys Glu Gln Met
                755                 760                 765

gaa gaa aag atg aga aac atc aga gaa gtg aag tgc cgt gtc gtg aca      2474
Glu Glu Lys Met Arg Asn Ile Arg Glu Val Lys Cys Arg Val Val Thr
            770                 775                 780

tgc aag acg tgc gcc tat acc cac ttc aag ctg ctg gag acc tgc gtc      2522
Cys Lys Thr Cys Ala Tyr Thr His Phe Lys Leu Leu Glu Thr Cys Val
        785                 790                 795

agt gag cag cat gaa tac cac tgg cat gat ggt gtg aag agg ttt ttc      2570
Ser Glu Gln His Glu Tyr His Trp His Asp Gly Val Lys Arg Phe Phe
    800                 805                 810

aaa tgt ccc tgt gga aac aga agc atc tcc ttg gac aga ctc ccg aac      2618
Lys Cys Pro Cys Gly Asn Arg Ser Ile Ser Leu Asp Arg Leu Pro Asn
815                 820                 825                 830

aag cac tgc agt aac tgt ggc ctc tac aaa tgg gaa cgg gac gga atg      2666
Lys His Cys Ser Asn Cys Gly Leu Tyr Lys Trp Glu Arg Asp Gly Met
                835                 840                 845

cta aag gaa aag act ggt cca aag ata gga gga gaa act ctg tta cca      2714
Leu Lys Glu Lys Thr Gly Pro Lys Ile Gly Gly Glu Thr Leu Leu Pro
            850                 855                 860

aga gga gaa gaa cat gct aaa ttt ctg aac agc ctt aaa taa              2756
Arg Gly Glu Glu His Ala Lys Phe Leu Asn Ser Leu Lys
        865                 870                 875

cccgaacttc agacattttc ccacagactt cctggcctcc tgtgactctg gaaagcaaag      2816

gattggctgt gtattgtcca ttgattcctg attgacgccg tcaaaaacaa atgcttgtta      2876

agcccataag ctttgcctgc ttactttctg ccattgggtt ggtttgatac cacatttaac      2936

attgacattt aagtggaaaa ccaagttatc attgtctttt ctaagctcag tgtggatgat      2996

tgcattactt cattcactga agtttttgcc caaaaattgg aaggtaaaca gagagctatg      3056

tttctgtatc ttttggttat agagtgttca cttctttatc ataacaaaat tctagtgttt      3116

atacgaacac ccagaggcaa aagaatttgg cttaattctc actccaggta agtagcttaa      3176

cttctgggct tcagttttct catctgtaaa atcaggaaga ttggactaag tgatcctgaa      3236

atgtattttt tagcactgga tttctacaaa taataaaact ttcccatcta gataatgatg      3296

atcacatagt cttgatgtac ggacattaaa agccagattt cttcattcaa ttctgttatc      3356

tctgttttac tctttgaaat tgatcaagcc actgaatcac tttgcatttc agtttatata      3416

tatagagaga aagaaggtgt ctgctcttac attattgtgg agccctgtga tagaaatatg      3476

taaaatctca tattattttt tttttaattt ttttattttt tatgacaggg tctcactatg      3536

tcaccctggc tggagtgcag tagtgcgatc gcggcacact gcagccttgg cttccctggg      3596

ctcaagcagt cctcccacct cagtctccca aatagctagg actacaggcg tgcgtgacca 3656

agcccagcta atttttgcat tttttgtaga gatggggttt tgccatgttg ctcaggctgg 3716

tctcaaactc ctgagcacta gcaatccacc cacctctgtt tccaaaaaaa aaaaaaaaat 3776

gaaaggtcaa cccctatgca aattaccaca gcaaaggttt cattcaggag attcttccat 3836

ctgggcaacc tggttttcca aatatcattt gacctaagtg aatgttgata ctagctaaag 3896

attgggtaaa ttggttgaat tattgtattg aagcttgagc tgtagctaaa agtaatttag 3956

gtttccccta agatgttatt atgttaggga cataacactt ttgggaggtt gttgtgggag 4016

atggttgatt taggttttca aaagctagaa ataaaattta catgccttag atttcataaa 4076

attctgctct aattgggtgg aaggtgctgt atctaacttg tgttcctcct aaggttatgt 4136

cctaataact attcttttag gagtatactt ctactttata gaaggttgct tttcttttta 4196

attttttcta acaaagaaaa gaataaagta tttattaata agaaccagaa agcacttgaa 4256

actgatgttt ttaatggctc atttagggta gatttattta tctcattaac ttaaaacagc 4316

tatgtgtatg aaataggtca caacagaact tgaacaccag gttggtgtct gagcaatccc 4376

tttcttatgg gaaaaacaat gttcttgttt gaacagaggg tatcattgca gtcagtattc 4436

acgtgtatat tgttatataa gttgtataat atgcttgtaa aggctgaggg tgagctgtat 4496

ctggatgcct ttttacaatt tgattttaac ttttaaaata aatttaaaac ataaaaaaaa 4556

aaaaaa 4562

<210> 26
<211> 875
<212> PRT
<213> Homo sapiens

<400> 26

Met Asp Glu Glu Glu Asp Asn Leu Ser Leu Leu Thr Ala Leu Leu Glu
1               5                   10                  15

Glu Asn Glu Ser Ala Leu Asp Cys Asn Ser Glu Glu Asn Asn Phe Leu
            20                  25                  30

Thr Arg Glu Asn Gly Glu Pro Asp Ala Phe Asp Glu Leu Phe Asp Ala
        35                  40                  45

Asp Gly Asp Gly Glu Ser Tyr Thr Glu Glu Ala Asp Asp Gly Glu Thr
    50                  55                  60

Gly Glu Thr Arg Asp Glu Lys Glu Asn Leu Ala Thr Leu Phe Gly Asp
65                  70                  75                  80

Met Glu Asp Leu Thr Asp Glu Glu Glu Val Pro Ala Ser Gln Ser Thr

85                  90                  95

Glu Asn Arg Val Leu Pro Ala Pro Ala Pro Arg Arg Glu Lys Thr Asn
            100                 105                 110

Glu Glu Leu Gln Glu Glu Leu Arg Asn Leu Gln Glu Gln Met Lys Ala
        115                 120                 125

Leu Gln Glu Gln Leu Lys Val Thr Thr Ile Lys Gln Thr Ala Ser Pro
    130                 135                 140

Ala Arg Leu Gln Lys Ser Pro Val Glu Lys Ser Pro Arg Pro Pro Leu
145                 150                 155                 160

Lys Glu Arg Arg Val Gln Arg Ile Gln Glu Ser Thr Cys Phe Ser Ala
                165                 170                 175

Glu Leu Asp Val Pro Ala Leu Pro Arg Thr Lys Arg Val Ala Arg Thr
            180                 185                 190

Pro Lys Ala Ser Pro Pro Asp Pro Lys Ser Ser Ser Ser Arg Met Thr
        195                 200                 205

Ser Ala Pro Ser Gln Pro Leu Gln Thr Ile Ser Arg Asn Lys Pro Ser
    210                 215                 220

Gly Ile Thr Arg Gly Gln Ile Val Gly Thr Pro Gly Ser Ser Gly Glu
225                 230                 235                 240

Thr Thr Gln Pro Ile Cys Val Glu Ala Phe Ser Gly Leu Arg Leu Arg
                245                 250                 255

Arg Pro Arg Val Ser Ser Thr Glu Met Asn Lys Lys Met Thr Gly Arg
            260                 265                 270

Lys Leu Ile Arg Leu Ser Gln Ile Lys Glu Lys Met Ala Arg Glu Lys
        275                 280                 285

Leu Glu Glu Ile Asp Trp Val Thr Phe Gly Val Ile Leu Lys Lys Val
    290                 295                 300

Thr Pro Gln Ser Val Asn Ser Gly Lys Thr Phe Ser Ile Trp Lys Leu
305                 310                 315                 320

Asn Asp Leu Arg Asp Leu Thr Gln Cys Val Ser Leu Phe Leu Phe Gly
                325                 330                 335

Glu Val His Lys Ala Leu Trp Lys Thr Glu Gln Gly Thr Val Val Gly
            340                 345                 350

Ile Leu Asn Ala Asn Pro Met Lys Pro Lys Asp Gly Ser Glu Glu Val
        355                 360                 365

Cys Leu Ser Ile Asp His Pro Gln Lys Val Leu Ile Met Gly Glu Ala
    370                 375                 380

Leu Asp Leu Gly Thr Cys Lys Ala Lys Lys Lys Asn Gly Glu Pro Cys
385                 390                 395                 400

Thr Gln Thr Val Asn Leu Arg Asp Cys Glu Tyr Cys Gln Tyr His Val
                405                 410                 415

Gln Ala Gln Tyr Lys Lys Leu Ser Ala Lys Arg Ala Asp Leu Gln Ser
            420                 425                 430

Thr Phe Ser Gly Gly Arg Ile Pro Lys Lys Phe Ala Arg Arg Gly Thr
        435                 440                 445

Ser Leu Lys Glu Arg Leu Cys Gln Asp Gly Phe Tyr Tyr Gly Gly Val
    450                 455                 460

Ser Ser Ala Ser Tyr Ala Ala Ser Ile Ala Ala Ala Val Ala Pro Lys
465                 470                 475                 480

Lys Lys Ile Gln Thr Thr Leu Ser Asn Leu Val Val Lys Gly Thr Asn
                485                 490                 495

Leu Ile Ile Gln Glu Thr Arg Gln Lys Leu Gly Ile Pro Gln Lys Ser
            500                 505                 510

Leu Ser Cys Ser Glu Glu Phe Lys Glu Leu Met Asp Leu Pro Thr Cys
        515                 520                 525

Gly Ala Arg Asn Leu Lys Gln His Leu Ala Lys Ala Thr Ala Ser Gly
    530                 535                 540

Ile Met Gly Ser Pro Lys Pro Ala Ile Lys Ser Ile Ser Ala Ser Ala
545                 550                 555                 560

Leu Leu Lys Gln Gln Lys Gln Arg Met Leu Glu Met Arg Arg Arg Lys
                565                 570                 575

Ser Glu Glu Ile Gln Lys Arg Phe Leu Gln Ser Ser Ser Glu Val Glu
            580                 585                 590

Ser Pro Ala Val Pro Ser Ser Ser Arg Gln Pro Pro Ala Gln Pro Pro
        595                 600                 605

Arg Thr Gly Ser Glu Phe Pro Arg Leu Glu Gly Ala Pro Ala Thr Met
    610                 615                 620

Thr Pro Lys Leu Gly Arg Gly Val Leu Glu Gly Asp Asp Val Leu Phe
625                 630                 635                 640

Tyr Asp Glu Ser Pro Pro Pro Arg Pro Lys Leu Ser Ala Leu Ala Glu
                645                 650                 655

Ala Lys Lys Leu Ala Ala Ile Thr Lys Leu Arg Ala Lys Gly Gln Val
            660                 665                 670

Leu Thr Lys Thr Asn Pro Asn Ser Ile Lys Lys Lys Gln Lys Asp Pro
        675                 680                 685

Gln Asp Ile Leu Glu Val Lys Glu Arg Val Glu Lys Asn Thr Met Phe
    690                 695                 700

Ser Ser Gln Ala Glu Asp Glu Leu Glu Pro Ala Arg Lys Lys Arg Arg
705                 710                 715                 720

Glu Gln Leu Ala Tyr Leu Glu Ser Glu Glu Phe Gln Lys Ile Leu Lys
                725                 730                 735

Ala Lys Ser Lys His Thr Gly Ile Leu Lys Glu Ala Glu Ala Glu Met
            740                 745                 750

Gln Glu Arg Tyr Phe Glu Pro Leu Val Lys Lys Glu Gln Met Glu Glu
        755                 760                 765

Lys Met Arg Asn Ile Arg Glu Val Lys Cys Arg Val Val Thr Cys Lys
    770                 775                 780

Thr Cys Ala Tyr Thr His Phe Lys Leu Leu Glu Thr Cys Val Ser Glu
785                 790                 795                 800

Gln His Glu Tyr His Trp His Asp Gly Val Lys Arg Phe Phe Lys Cys
                805                 810                 815

Pro Cys Gly Asn Arg Ser Ile Ser Leu Asp Arg Leu Pro Asn Lys His
            820                 825                 830

Cys Ser Asn Cys Gly Leu Tyr Lys Trp Glu Arg Asp Gly Met Leu Lys
        835                 840                 845

Glu Lys Thr Gly Pro Lys Ile Gly Gly Glu Thr Leu Leu Pro Arg Gly
    850                 855                 860

Glu Glu His Ala Lys Phe Leu Asn Ser Leu Lys
865                 870                 875

<210>  27
<211>  1405
<212>  DNA
<213>  Homo sapiens

<220>
<221>  CDS
<222>  (88)..(831)

<400>  27
tttcccggcg tccatttagt ggcgggaaaa gcgacctttt ctgagcgcgt ttgcctgttg      60

agtggtagcc tttcccctca accagca atg gag gag cag ccc cag atg caa gac     114
                              Met Glu Glu Gln Pro Gln Met Gln Asp
                              1               5

gcc gac gag ccc gcg gac tcc gga ggg gaa ggc cgg gca ggc ggg cca     162
Ala Asp Glu Pro Ala Asp Ser Gly Gly Glu Gly Arg Ala Gly Gly Pro
10                  15                  20                  25

ccg cag gtc gcc ggc gcc cag gcg gcg tgc agc gag gac cgc atg acc     210
Pro Gln Val Ala Gly Ala Gln Ala Ala Cys Ser Glu Asp Arg Met Thr
                30                  35                  40

ctg ctc ctc agg ctg aga gca cag aca aaa caa caa ctc tta gaa tat     258
Leu Leu Leu Arg Leu Arg Ala Gln Thr Lys Gln Gln Leu Leu Glu Tyr
            45                  50                  55

aaa tca atg gtt gat gca agt gaa gaa aaa act cca gaa caa att atg     306
Lys Ser Met Val Asp Ala Ser Glu Glu Lys Thr Pro Glu Gln Ile Met
        60                  65                  70

caa gaa aag caa atc gaa gct aaa att gaa gac ctg gaa aat gaa att     354
Gln Glu Lys Gln Ile Glu Ala Lys Ile Glu Asp Leu Glu Asn Glu Ile
    75                  80                  85

gaa gag gta aaa gtt gct ttt gag ata aaa aag ctt gca tta gac agg     402
Glu Glu Val Lys Val Ala Phe Glu Ile Lys Lys Leu Ala Leu Asp Arg
90                  95                  100                 105

atg aga ctt tca act gca ctt aaa aaa aac ctg gag aaa att agc aga     450
Met Arg Leu Ser Thr Ala Leu Lys Lys Asn Leu Glu Lys Ile Ser Arg
                110                 115                 120

cag tct agt gtg ctc atg gat aac atg aaa cac cta tta gag cta aat     498
Gln Ser Ser Val Leu Met Asp Asn Met Lys His Leu Leu Glu Leu Asn
            125                 130                 135

aaa tta ata atg aaa tca cag cag gaa tct tgg gat tta gag gaa aaa     546
Lys Leu Ile Met Lys Ser Gln Gln Glu Ser Trp Asp Leu Glu Glu Lys
        140                 145                 150

ctg ctt gat att aga aag aag aga ttg caa tta aaa caa gct tca gaa 594
Leu Leu Asp Ile Arg Lys Lys Arg Leu Gln Leu Lys Gln Ala Ser Glu
    155                 160                 165

agt aag ctt tta gaa ata cag act gaa aag aac aaa cag aag att gat 642
Ser Lys Leu Leu Glu Ile Gln Thr Glu Lys Asn Lys Gln Lys Ile Asp
170                 175                 180                 185

ttg gac agt atg gaa aac tca gag agg ata aag atc ata cga caa aac 690
Leu Asp Ser Met Glu Asn Ser Glu Arg Ile Lys Ile Ile Arg Gln Asn
                190                 195                 200

cta cag atg gag ata aaa att act act gtt att caa cat gtg ttc cag 738
Leu Gln Met Glu Ile Lys Ile Thr Thr Val Ile Gln His Val Phe Gln
            205                 210                 215

aac ctt att ttg ggg agt aaa gtc aat tgg gca gag gat cct gcc ctt 786
Asn Leu Ile Leu Gly Ser Lys Val Asn Trp Ala Glu Asp Pro Ala Leu
        220                 225                 230

aag gaa att gtt ctg cag ctt gag aag aat gtt gac atg atg taa 831
Lys Glu Ile Val Leu Gln Leu Glu Lys Asn Val Asp Met Met
    235                 240                 245

taagaattca tttctgacat attttacatt tctggcaatc tcaactctta tttggaatac 891

ttctgtgcat ttgtctgtcc accgtaattt tagaaaagca tatccataac gtttacagtt 951

gtagtacagt tgtggttagt tatttgtagt gggattgaaa gtaatttttt tctttttata 1011

tttctatatt tagtttgttt ttttgttgtt gttgtttttt gagatggagt ctcgctttgt 1071

tgcccagact ggagggcagt ggcgcgatct cggctcactg caacctctgc ctcccgggtt 1131

caagcagttc tgcctcagcc tcccaagtag ctgtgactaa aggtgcacgc cgccatgccc 1191

agctaatttt ttgtatttta gtagagacgg ggtttcaccg tgttgcccag gctgctctca 1251

gaactcctga gctcaggcag tccaccgcct cggcctaccg aagtgctagg attacagacg 1311

taagccaccg agcctggtct agtttgcatt ttttttctat cagttttata agttaagaaa 1371

taaaaggaat taatgttaaa aaaaaaaaaa aaaa 1405

<210> 28
<211> 247
<212> PRT
<213> Homo sapiens

<400> 28

Met Glu Glu Gln Pro Gln Met Gln Asp Ala Asp Glu Pro Ala Asp Ser
1               5                   10                  15

Gly Gly Glu Gly Arg Ala Gly Gly Pro Pro Gln Val Ala Gly Ala Gln
            20                  25                  30

Ala Ala Cys Ser Glu Asp Arg Met Thr Leu Leu Leu Arg Leu Arg Ala
        35                  40                  45

Gln Thr Lys Gln Gln Leu Leu Glu Tyr Lys Ser Met Val Asp Ala Ser
    50                  55                  60

Glu Glu Lys Thr Pro Glu Gln Ile Met Gln Glu Lys Gln Ile Glu Ala
65                  70                  75                  80

Lys Ile Glu Asp Leu Glu Asn Glu Ile Glu Glu Val Lys Val Ala Phe
                85                  90                  95

Glu Ile Lys Lys Leu Ala Leu Asp Arg Met Arg Leu Ser Thr Ala Leu
            100                 105                 110

Lys Lys Asn Leu Glu Lys Ile Ser Arg Gln Ser Ser Val Leu Met Asp
        115                 120                 125

Asn Met Lys His Leu Leu Glu Leu Asn Lys Leu Ile Met Lys Ser Gln
    130                 135                 140

Gln Glu Ser Trp Asp Leu Glu Glu Lys Leu Leu Asp Ile Arg Lys Lys
145                 150                 155                 160

Arg Leu Gln Leu Lys Gln Ala Ser Glu Ser Lys Leu Leu Glu Ile Gln
                165                 170                 175

Thr Glu Lys Asn Lys Gln Lys Ile Asp Leu Asp Ser Met Glu Asn Ser
            180                 185                 190

Glu Arg Ile Lys Ile Ile Arg Gln Asn Leu Gln Met Glu Ile Lys Ile
        195                 200                 205

Thr Thr Val Ile Gln His Val Phe Gln Asn Leu Ile Leu Gly Ser Lys
    210                 215                 220

Val Asn Trp Ala Glu Asp Pro Ala Leu Lys Glu Ile Val Leu Gln Leu
225                 230                 235                 240

Glu Lys Asn Val Asp Met Met
                245

<210>  29
<211>  3109
<212>  DNA
<213>  Homo sapiens

<220>
<221>  CDS
<222>  (278)..(2614)

<400> 29
gggggccggc cagaaacggg ctggggaggg ggggccccgc agcccccctg ggccatgctg 60

actcccgggg cctgaccccc ccgggccagc cccccctccc ccagctccgc ggcccgccga 120

ctgggggggg ccagcccagc cccctgggga cccccggaga ggtggggggc agccgggggg 180

gccgggacgg agcggtcgcc ggcccccacc ggagagacgg ggcgacggcc gcaggggggg 240

cggccggggg accggtcggg ccgggaccaa gggcacc atg tcg tcc ggg gcc aag 295
Met Ser Ser Gly Ala Lys
1               5

gag gga ggt ggg ggc tct ccc gcc tac cac ctc ccc cac ccc cac ccc 343
Glu Gly Gly Gly Gly Ser Pro Ala Tyr His Leu Pro His Pro His Pro
            10                  15                  20

cac cca ccc cag cac gcc caa tat gtg ggc ccc tat cgg ctg gag aag 391
His Pro Pro Gln His Ala Gln Tyr Val Gly Pro Tyr Arg Leu Glu Lys
        25                  30                  35

acg ctg ggc aaa gga cag aca ggg ctg gtt aaa ctc ggg gtc cac tgc 439
Thr Leu Gly Lys Gly Gln Thr Gly Leu Val Lys Leu Gly Val His Cys
    40                  45                  50

atc acg ggt cag aag gtc gcc atc aag atc gtg aac cgg gag aag ctg 487
Ile Thr Gly Gln Lys Val Ala Ile Lys Ile Val Asn Arg Glu Lys Leu
55                  60                  65                  70

tcg gag tcg gtg ctg atg aag gtg gag cgg gag atc gcc atc ctg aag 535
Ser Glu Ser Val Leu Met Lys Val Glu Arg Glu Ile Ala Ile Leu Lys
                75                  80                  85

ctc atc gaa cac cca cat gtc ctc aag ctc cac gac gtc tac gag aac 583
Leu Ile Glu His Pro His Val Leu Lys Leu His Asp Val Tyr Glu Asn
            90                  95                  100

aag aaa tat ttg tac ctg gtt ctg gag cac gtc tcg ggg ggt gag cta 631
Lys Lys Tyr Leu Tyr Leu Val Leu Glu His Val Ser Gly Gly Glu Leu
        105                 110                 115

ttc gac tac ctg gta aag aag ggg aga ctg acg ccc aag gag gcc cga 679
Phe Asp Tyr Leu Val Lys Lys Gly Arg Leu Thr Pro Lys Glu Ala Arg
    120                 125                 130

aag ttc ttc cgc cag att gtg tct gcg ctg gac ttc tgc cac agc tac 727
Lys Phe Phe Arg Gln Ile Val Ser Ala Leu Asp Phe Cys His Ser Tyr
135                 140                 145                 150

tcc atc tgc cac aga gac cta aag ccc gag aac ctg ctt ttg gat gag 775
Ser Ile Cys His Arg Asp Leu Lys Pro Glu Asn Leu Leu Leu Asp Glu
                155                 160                 165

aaa aac aac atc cgc att gca gac ttc ggc atg gcg tcc ctg cag gtg 823
Lys Asn Asn Ile Arg Ile Ala Asp Phe Gly Met Ala Ser Leu Gln Val
            170                 175                 180

ggg gac agc ctc ctg gag acc agc tgc ggg tcc ccc cat tat gcg tgt 871
Gly Asp Ser Leu Leu Glu Thr Ser Cys Gly Ser Pro His Tyr Ala Cys
        185                 190                 195

cca gag gtg att aag ggg gaa aaa tat gat ggc cgc cgg gca gac atg 919
Pro Glu Val Ile Lys Gly Glu Lys Tyr Asp Gly Arg Arg Ala Asp Met

200                 205                 210

tgg agc tgt gga gtc atc ctc ttc gcc ctg ctc gtg ggg gct ctg ccc      967
Trp Ser Cys Gly Val Ile Leu Phe Ala Leu Leu Val Gly Ala Leu Pro
215                 220                 225                 230

ttt gat gac gac aac ctc cgc cag ctg ctg gag aag gtg aaa cgg ggc      1015
Phe Asp Asp Asp Asn Leu Arg Gln Leu Leu Glu Lys Val Lys Arg Gly
                235                 240                 245

gtc ttc cac atg ccc cac ttc att cct cca gat tgc cag agc ctc ctg      1063
Val Phe His Met Pro His Phe Ile Pro Pro Asp Cys Gln Ser Leu Leu
            250                 255                 260

agg gga atg atc gaa gtg gag ccc gaa aaa agg ctc agt ctg gag caa      1111
Arg Gly Met Ile Glu Val Glu Pro Glu Lys Arg Leu Ser Leu Glu Gln
        265                 270                 275

att cag aaa cat cct tgg tac cta ggc ggg aaa cac gag cca gac ccg      1159
Ile Gln Lys His Pro Trp Tyr Leu Gly Gly Lys His Glu Pro Asp Pro
    280                 285                 290

tgc ctg gag cca gcc cct ggc cgc cgg gta gcc atg cgg agc ctg cca      1207
Cys Leu Glu Pro Ala Pro Gly Arg Arg Val Ala Met Arg Ser Leu Pro
295                 300                 305                 310

tcc aac gga gag ctg gac ccc gac gtc cta gag agc atg gca tca ctg      1255
Ser Asn Gly Glu Leu Asp Pro Asp Val Leu Glu Ser Met Ala Ser Leu
                315                 320                 325

ggc tgc ttc agg gac cgc gag agg ctg cat cgc gag ctg cgc agt gag      1303
Gly Cys Phe Arg Asp Arg Glu Arg Leu His Arg Glu Leu Arg Ser Glu
            330                 335                 340

gag gag aac caa gaa aag atg ata tat tat ctg ctt ttg gat cgg aag      1351
Glu Glu Asn Gln Glu Lys Met Ile Tyr Tyr Leu Leu Leu Asp Arg Lys
        345                 350                 355

gag cgg tat ccc agc tgt gag gac cag gac ctg cct ccc cgg aat gat      1399
Glu Arg Tyr Pro Ser Cys Glu Asp Gln Asp Leu Pro Pro Arg Asn Asp
    360                 365                 370

gtt gac ccc ccc cgg aag cgt gtg gat tct ccc atg ctg agc cgt cac      1447
Val Asp Pro Pro Arg Lys Arg Val Asp Ser Pro Met Leu Ser Arg His
375                 380                 385                 390

ggg aag cgg cga cca gag cgg aag tcc atg gaa gtc ctg agc atc acc      1495
Gly Lys Arg Arg Pro Glu Arg Lys Ser Met Glu Val Leu Ser Ile Thr
                395                 400                 405

gat gcc ggg ggt ggt ggc tcc cct gta ccc acc cga cgg gcc ttg gag      1543
Asp Ala Gly Gly Gly Gly Ser Pro Val Pro Thr Arg Arg Ala Leu Glu
            410                 415                 420

atg gcc cag cac agc cag aga tcc cgt agc gtc agt gga gcc tcc acg      1591
Met Ala Gln His Ser Gln Arg Ser Arg Ser Val Ser Gly Ala Ser Thr
        425                 430                 435

ggt ctg tcc tcc agc cct cta agc agc cca agg agt ccg gtc ttt tcc      1639
Gly Leu Ser Ser Ser Pro Leu Ser Ser Pro Arg Ser Pro Val Phe Ser
    440                 445                 450

ttt tca ccg gag ccg ggg gct gga gat gag gct cga ggc ggg ggc tcc      1687

Phe Ser Pro Glu Pro Gly Ala Gly Asp Glu Ala Arg Gly Gly Gly Ser
455                 460                 465                 470

ccg act tcc aaa acg cag acg ctg cct tct cgg ggc ccc agg ggt ggg      1735
Pro Thr Ser Lys Thr Gln Thr Leu Pro Ser Arg Gly Pro Arg Gly Gly
                475                 480                 485

ggc gcc ggg gag cag ccc ccg ccc ccc agt gcc cgc tcc aca ccc ctg      1783
Gly Ala Gly Glu Gln Pro Pro Pro Pro Ser Ala Arg Ser Thr Pro Leu
            490                 495                 500

ccc ggc ccc cca ggc tcc ccg cgc tcc tct ggc ggg acc ccc ttg cac      1831
Pro Gly Pro Pro Gly Ser Pro Arg Ser Ser Gly Gly Thr Pro Leu His
        505                 510                 515

tcg cct ctg cac acg ccc cgg gcc agt ccc acc ggg acc ccg ggg aca      1879
Ser Pro Leu His Thr Pro Arg Ala Ser Pro Thr Gly Thr Pro Gly Thr
    520                 525                 530

aca cca ccc ccc agc ccc ggc ggt ggc gtc ggg gga gcc gcc tgg agg      1927
Thr Pro Pro Pro Ser Pro Gly Gly Gly Val Gly Gly Ala Ala Trp Arg
535                 540                 545                 550

agt cgt ctc aac tcc atc cgc aac agc ttc ctg ggc tcc cct cgc ttt      1975
Ser Arg Leu Asn Ser Ile Arg Asn Ser Phe Leu Gly Ser Pro Arg Phe
                555                 560                 565

cac cgg cgc aag atg cag gtc cct acc gct gag gag atg tcc agc ttg      2023
His Arg Arg Lys Met Gln Val Pro Thr Ala Glu Glu Met Ser Ser Leu
            570                 575                 580

acg cca gag tcc tcc ccg gag ctg gca aaa cgc tcc tgg ttc ggg aac      2071
Thr Pro Glu Ser Ser Pro Glu Leu Ala Lys Arg Ser Trp Phe Gly Asn
        585                 590                 595

ttc atc tcc ttg gac aaa gaa gaa caa ata ttc ctc gtg cta aag gac      2119
Phe Ile Ser Leu Asp Lys Glu Glu Gln Ile Phe Leu Val Leu Lys Asp
    600                 605                 610

aaa cct ctc agc agc atc aaa gca gac atc gtc cat gcc ttt ctg tcg      2167
Lys Pro Leu Ser Ser Ile Lys Ala Asp Ile Val His Ala Phe Leu Ser
615                 620                 625                 630

atc ccc agc ctg agt cac agt gtg ctg tca cag acc agc ttc agg gcc      2215
Ile Pro Ser Leu Ser His Ser Val Leu Ser Gln Thr Ser Phe Arg Ala
                635                 640                 645

gag tac aag gcc agt ggc ggc ccc tcc gtc ttc caa aag ccc gtc cgc      2263
Glu Tyr Lys Ala Ser Gly Gly Pro Ser Val Phe Gln Lys Pro Val Arg
            650                 655                 660

ttc cag gtg gac atc agc tcc tct gag ggt cca gag ccc tcc ccg cga      2311
Phe Gln Val Asp Ile Ser Ser Ser Glu Gly Pro Glu Pro Ser Pro Arg
        665                 670                 675

cgg gac ggc agc gga ggt ggt ggc atc tac tcc gtc acc ttc act ctc      2359
Arg Asp Gly Ser Gly Gly Gly Gly Ile Tyr Ser Val Thr Phe Thr Leu
    680                 685                 690

atc tcg ggt ccc agc cgt cgg ttc aag cga gtg gtg gag acc atc cag      2407
Ile Ser Gly Pro Ser Arg Arg Phe Lys Arg Val Val Glu Thr Ile Gln
695                 700                 705                 710

gca cag ctc ctg agc act cat gac cag ccc tcc gtg cag gcc ctg gca 2455
Ala Gln Leu Leu Ser Thr His Asp Gln Pro Ser Val Gln Ala Leu Ala
                715                 720                 725

gac gag aag aac ggg gcc cag acc cgg cct gct ggt gcc cca ccc cga 2503
Asp Glu Lys Asn Gly Ala Gln Thr Arg Pro Ala Gly Ala Pro Pro Arg
            730                 735                 740

agc ctg cag ccc cca ccc ggc cgc cca gac cca gag ctg agc agc tct 2551
Ser Leu Gln Pro Pro Pro Gly Arg Pro Asp Pro Glu Leu Ser Ser Ser
        745                 750                 755

ccc cgc cga ggc ccc ccc aag gac aag aag ctc ctg gcc acc aac ggg 2599
Pro Arg Arg Gly Pro Pro Lys Asp Lys Lys Leu Leu Ala Thr Asn Gly
    760                 765                 770

acc cct ctg ccc tga ccccacgggg ccggggaggg aggggacccc cctccacccc 2654
Thr Pro Leu Pro
775

ccttccgtgc cccccaactg tgaatctgta aataaggccc aaggaacatg tcgggagggg 2714

ggtggacaca aaaaccggcc ttgccctgca gggatggggc tccacaggcc gtgcccaact 2774

gggggtggtt ctaggggaac agggggcggg ggagctgttt ctattttatt tattgattaa 2834

tttattattt tatttattga tcaatctctc tgcggggtgg ggtgggggag ggacgggagc 2894

tggttggggt ggcttagcag atccggacag ggccctctgt ccctgtgtcg tccccaaccc 2954

cctcttcccg ggcccctcct cccctggtcc tccccccacg accttctgta cggatttgct 3014

ctccggaagg aattctggtt tcgcgtgatc ctgcctgcgt ccgtgtctct gattccgccg 3074

gcggcaaaaa aaaaaaaaaa aaaaaaaaaa aaaaa 3109

<210> 30
<211> 778
<212> PRT
<213> Homo sapiens

<400> 30

Met Ser Ser Gly Ala Lys Glu Gly Gly Gly Gly Ser Pro Ala Tyr His
1               5                   10                  15

Leu Pro His Pro His Pro His Pro Pro Gln His Ala Gln Tyr Val Gly
            20                  25                  30

Pro Tyr Arg Leu Glu Lys Thr Leu Gly Lys Gly Gln Thr Gly Leu Val
        35                  40                  45

Lys Leu Gly Val His Cys Ile Thr Gly Gln Lys Val Ala Ile Lys Ile
    50                  55                  60

Val Asn Arg Glu Lys Leu Ser Glu Ser Val Leu Met Lys Val Glu Arg
65                  70                  75                  80

Glu Ile Ala Ile Leu Lys Leu Ile Glu His Pro His Val Leu Lys Leu
                85                  90                  95

His Asp Val Tyr Glu Asn Lys Lys Tyr Leu Tyr Leu Val Leu Glu His
            100                 105                 110

Val Ser Gly Gly Glu Leu Phe Asp Tyr Leu Val Lys Lys Gly Arg Leu
        115                 120                 125

Thr Pro Lys Glu Ala Arg Lys Phe Phe Arg Gln Ile Val Ser Ala Leu
    130                 135                 140

Asp Phe Cys His Ser Tyr Ser Ile Cys His Arg Asp Leu Lys Pro Glu
145                 150                 155                 160

Asn Leu Leu Leu Asp Glu Lys Asn Asn Ile Arg Ile Ala Asp Phe Gly
                165                 170                 175

Met Ala Ser Leu Gln Val Gly Asp Ser Leu Leu Glu Thr Ser Cys Gly
            180                 185                 190

Ser Pro His Tyr Ala Cys Pro Glu Val Ile Lys Gly Glu Lys Tyr Asp
        195                 200                 205

Gly Arg Arg Ala Asp Met Trp Ser Cys Gly Val Ile Leu Phe Ala Leu
    210                 215                 220

Leu Val Gly Ala Leu Pro Phe Asp Asp Asp Asn Leu Arg Gln Leu Leu
225                 230                 235                 240

Glu Lys Val Lys Arg Gly Val Phe His Met Pro His Phe Ile Pro Pro
                245                 250                 255

Asp Cys Gln Ser Leu Leu Arg Gly Met Ile Glu Val Glu Pro Glu Lys
            260                 265                 270

Arg Leu Ser Leu Glu Gln Ile Gln Lys His Pro Trp Tyr Leu Gly Gly
        275                 280                 285

Lys His Glu Pro Asp Pro Cys Leu Glu Pro Ala Pro Gly Arg Arg Val
    290                 295                 300

Ala Met Arg Ser Leu Pro Ser Asn Gly Glu Leu Asp Pro Asp Val Leu
305                 310                 315                 320

Glu Ser Met Ala Ser Leu Gly Cys Phe Arg Asp Arg Glu Arg Leu His
                325                 330                 335

Arg Glu Leu Arg Ser Glu Glu Glu Asn Gln Glu Lys Met Ile Tyr Tyr
            340                 345                 350

Leu Leu Leu Asp Arg Lys Glu Arg Tyr Pro Ser Cys Glu Asp Gln Asp
        355                 360                 365

Leu Pro Pro Arg Asn Asp Val Asp Pro Pro Arg Lys Arg Val Asp Ser
    370                 375                 380

Pro Met Leu Ser Arg His Gly Lys Arg Arg Pro Glu Arg Lys Ser Met
385                 390                 395                 400

Glu Val Leu Ser Ile Thr Asp Ala Gly Gly Gly Gly Ser Pro Val Pro
                405                 410                 415

Thr Arg Arg Ala Leu Glu Met Ala Gln His Ser Gln Arg Ser Arg Ser
            420                 425                 430

Val Ser Gly Ala Ser Thr Gly Leu Ser Ser Ser Pro Leu Ser Ser Pro
        435                 440                 445

Arg Ser Pro Val Phe Ser Phe Ser Pro Glu Pro Gly Ala Gly Asp Glu
    450                 455                 460

Ala Arg Gly Gly Gly Ser Pro Thr Ser Lys Thr Gln Thr Leu Pro Ser
465                 470                 475                 480

Arg Gly Pro Arg Gly Gly Gly Ala Gly Glu Gln Pro Pro Pro Pro Ser
                485                 490                 495

Ala Arg Ser Thr Pro Leu Pro Gly Pro Pro Gly Ser Pro Arg Ser Ser
            500                 505                 510

Gly Gly Thr Pro Leu His Ser Pro Leu His Thr Pro Arg Ala Ser Pro
        515                 520                 525

Thr Gly Thr Pro Gly Thr Thr Pro Pro Pro Ser Pro Gly Gly Gly Val
    530                 535                 540

Gly Gly Ala Ala Trp Arg Ser Arg Leu Asn Ser Ile Arg Asn Ser Phe
545                 550                 555                 560

Leu Gly Ser Pro Arg Phe His Arg Arg Lys Met Gln Val Pro Thr Ala
                565                 570                 575

Glu Glu Met Ser Ser Leu Thr Pro Glu Ser Ser Pro Glu Leu Ala Lys

580                 585                 590

Arg Ser Trp Phe Gly Asn Phe Ile Ser Leu Asp Lys Glu Glu Gln Ile
        595                 600                 605

Phe Leu Val Leu Lys Asp Lys Pro Leu Ser Ser Ile Lys Ala Asp Ile
    610                 615                 620

Val His Ala Phe Leu Ser Ile Pro Ser Leu Ser His Ser Val Leu Ser
625                 630                 635                 640

Gln Thr Ser Phe Arg Ala Glu Tyr Lys Ala Ser Gly Gly Pro Ser Val
                645                 650                 655

Phe Gln Lys Pro Val Arg Phe Gln Val Asp Ile Ser Ser Ser Glu Gly
            660                 665                 670

Pro Glu Pro Ser Pro Arg Arg Asp Gly Ser Gly Gly Gly Gly Ile Tyr
        675                 680                 685

Ser Val Thr Phe Thr Leu Ile Ser Gly Pro Ser Arg Arg Phe Lys Arg
    690                 695                 700

Val Val Glu Thr Ile Gln Ala Gln Leu Leu Ser Thr His Asp Gln Pro
705                 710                 715                 720

Ser Val Gln Ala Leu Ala Asp Glu Lys Asn Gly Ala Gln Thr Arg Pro
                725                 730                 735

Ala Gly Ala Pro Pro Arg Ser Leu Gln Pro Pro Pro Gly Arg Pro Asp
            740                 745                 750

Pro Glu Leu Ser Ser Ser Pro Arg Arg Gly Pro Pro Lys Asp Lys Lys
        755                 760                 765

Leu Leu Ala Thr Asn Gly Thr Pro Leu Pro
    770                 775

<210>  31
<211>  21
<212>  DNA
<213>  Artificial

<220>
<223>  Combined DNA/RNA molecule: Synthetic oligonucleotide

<220>
<221>  misc_RNA
<222>  (1)..(19)

<223>  RNA

<400>  31
gggaggcaag accuucauut t                                                21


<210>  32
<211>  21
<212>  DNA
<213>  Artificial

<220>
<223>  Combined DNA/RNA molecule: Synthetic oligonucleotide


<220>
<221>  misc_RNA
<222>  (1)..(19)
<223>  RNA

<400>  32
aaugaagguc uugccuccct g                                                21


<210>  33
<211>  21
<212>  DNA
<213>  Artificial

<220>
<223>  Combined DNA/RNA molecule: Synthetic oligonucleotide


<220>
<221>  misc_RNA
<222>  (1)..(19)
<223>  RNA

<400>  33
uccuaagagu gcugggcaut t                                                21


<210>  34
<211>  21
<212>  DNA
<213>  Artificial

<220>
<223>  Combined DNA/RNA molecule: Synthetic oligonucleotide


<220>
<221>  misc_RNA
<222>  (1)..(19)
<223>  RNA

<400>  34
augcccagca cucuuaggat t                                                21


<210>  35
<211>  21
<212>  DNA

<213>  Artificial

<220>
<223>  Combined DNA/RNA molecule: Synthetic oligonucleotide


<220>
<221>  misc_RNA
<222>  (1)..(19)
<223>  RNA

<400>  35
acgugacacg uucggagaat t                                                21


<210>  36
<211>  21
<212>  DNA
<213>  Artificial

<220>
<223>  Combined DNA/RNA molecule: Synthetic oligonucleotide


<220>
<221>  misc_RNA
<222>  (1)..(19)
<223>  RNA

<400>  36
uucuccgaac gugucacgut t                                                21


<210>  37
<211>  21
<212>  DNA
<213>  Artificial

<220>
<223>  Combined DNA/RNA molecule: Synthetic oligonucleotide


<220>
<221>  misc_RNA
<222>  (1)..(19)
<223>  RNA

<400>  37
ucucccucau cuacaccaat t                                                21


<210>  38
<211>  21
<212>  DNA
<213>  Artificial

<220>
<223>  Combined DNA/RNA molecule: Synthetic oligonucleotide


<220>
<221>  misc_RNA
<222>  (1)..(19)

<223>  RNA

<400>  38
uugguguaga ugagggagat g                                                21


<210>  39
<211>  2172
<212>  DNA
<213>  Homo sapiens


<220>
<221>  CDS
<222>  (252)..(1934)

<400>  39
aatcccttca accttctgcg cgcttccgcc ttcatgctag caacgcccgc agtgacgcgc      60

aggccccgcc ccctcccgcg cgcctcccgg aagtggccgg tccagagctg tggggtggcc     120

tccgcgcggt ctctggcgga gtcggggaat cggatcaagg cgagaggatc cggcagggaa     180

ggagcttcgg ggccgggggt tgggccgcac atttacgtgc gcgaagcgga gtggaccggg     240

agctggtgac g atg gcg ggg ccg cag ccc ctg gcg ctg caa ctg gaa cag      290
             Met Ala Gly Pro Gln Pro Leu Ala Leu Gln Leu Glu Gln
             1               5                   10

ttg ttg aac ccg cga cca agc gag gcg gac cct gaa gcg gac ccc gag      338
Leu Leu Asn Pro Arg Pro Ser Glu Ala Asp Pro Glu Ala Asp Pro Glu
    15                  20                  25

gaa gcc act gct gcc agg gtg att gac agg ttt gat gaa ggg gaa gat      386
Glu Ala Thr Ala Ala Arg Val Ile Asp Arg Phe Asp Glu Gly Glu Asp
30                  35                  40                  45

ggg gaa ggt gat ttc cta gta gtg ggt agc att aga aaa ctg gca tca      434
Gly Glu Gly Asp Phe Leu Val Val Gly Ser Ile Arg Lys Leu Ala Ser
                50                  55                  60

gcc tcc ctc ttg gac acg gac aaa agg tat tgc ggc aaa acc acc tct      482
Ala Ser Leu Leu Asp Thr Asp Lys Arg Tyr Cys Gly Lys Thr Thr Ser
            65                  70                  75

aga aaa gca tgg aat gaa gac cat tgg gag cag act ctg cca gga tcg      530
Arg Lys Ala Trp Asn Glu Asp His Trp Glu Gln Thr Leu Pro Gly Ser
        80                  85                  90

tct gat gag gaa ata tct gat gag gaa ggg tct gga gat gaa gat tca      578
Ser Asp Glu Glu Ile Ser Asp Glu Glu Gly Ser Gly Asp Glu Asp Ser
    95                  100                 105

gag gga ctg ggt ctg gag gaa tat gat gag gac gac ctg ggt gct gct      626
Glu Gly Leu Gly Leu Glu Glu Tyr Asp Glu Asp Asp Leu Gly Ala Ala
110                 115                 120                 125

gag gaa cag gag tgt ggt gat cac agg gag agc aag aag agc aga agc      674
Glu Glu Gln Glu Cys Gly Asp His Arg Glu Ser Lys Lys Ser Arg Ser
                130                 135                 140

cac tct gca aaa aca ccg ggc ttc agt gtc cag agt atc agt gac ttt      722
His Ser Ala Lys Thr Pro Gly Phe Ser Val Gln Ser Ile Ser Asp Phe

145                 150                 155

gag aaa ttt acc aag gga atg gat gac ctt ggg agc agt gag gag gag      770
Glu Lys Phe Thr Lys Gly Met Asp Asp Leu Gly Ser Ser Glu Glu Glu
        160                 165                 170

gaa gac gaa gag agt ggc atg gaa gaa ggg gat gac gcg gaa gac tcc      818
Glu Asp Glu Glu Ser Gly Met Glu Glu Gly Asp Asp Ala Glu Asp Ser
    175                 180                 185

caa ggc gag agt gag gaa gac agg gct gga gat aga aac agt gag gat      866
Gln Gly Glu Ser Glu Glu Asp Arg Ala Gly Asp Arg Asn Ser Glu Asp
190                 195                 200                 205

gat ggt gtg gtg atg acc ttc tct agt gtc aaa gtt tct gag gaa gtg      914
Asp Gly Val Val Met Thr Phe Ser Ser Val Lys Val Ser Glu Glu Val
                210                 215                 220

gag aaa gga aga gcc gtg aag aac cag ata gca ctg tgg gac cag ctc      962
Glu Lys Gly Arg Ala Val Lys Asn Gln Ile Ala Leu Trp Asp Gln Leu
            225                 230                 235

ttg gaa gga agg atc aaa cta caa aaa gct ctg ttg acc acc aac cag      1010
Leu Glu Gly Arg Ile Lys Leu Gln Lys Ala Leu Leu Thr Thr Asn Gln
        240                 245                 250

ctt cct caa cca gat gtt ttc cca ttg ttc aag gac aaa ggt ggc cca      1058
Leu Pro Gln Pro Asp Val Phe Pro Leu Phe Lys Asp Lys Gly Gly Pro
    255                 260                 265

gaa ttt tcc agt gcc ctg aaa aat agt cac aag gca ctt aaa gca ttg      1106
Glu Phe Ser Ser Ala Leu Lys Asn Ser His Lys Ala Leu Lys Ala Leu
270                 275                 280                 285

ttg agg tca ttg gta ggt ctt cag gaa gag ttg ctt ttc cag tac cca      1154
Leu Arg Ser Leu Val Gly Leu Gln Glu Glu Leu Leu Phe Gln Tyr Pro
                290                 295                 300

gac act aga tat cta gta gat ggg aca aag ccc aat gcg gga agt gag      1202
Asp Thr Arg Tyr Leu Val Asp Gly Thr Lys Pro Asn Ala Gly Ser Glu
            305                 310                 315

gag att tct agt gaa gat gat gag ctg gta gaa gag aag aag cag caa      1250
Glu Ile Ser Ser Glu Asp Asp Glu Leu Val Glu Glu Lys Lys Gln Gln
        320                 325                 330

cga aga agg gtc cct gca aag agg aag ctg gag atg gag gac tat ccc      1298
Arg Arg Arg Val Pro Ala Lys Arg Lys Leu Glu Met Glu Asp Tyr Pro
    335                 340                 345

agc ttc atg gca aag cgc ttt gcc gac ttt aca gtc tac agg aac cgc      1346
Ser Phe Met Ala Lys Arg Phe Ala Asp Phe Thr Val Tyr Arg Asn Arg
350                 355                 360                 365

aca ctt cag aaa tgg cac gat aag acc aaa ctg gct tct gga aaa ctg      1394
Thr Leu Gln Lys Trp His Asp Lys Thr Lys Leu Ala Ser Gly Lys Leu
                370                 375                 380

ggg aag ggt ttt ggt gcc ttt gaa cgc tca atc ttg act cag atc gac      1442
Gly Lys Gly Phe Gly Ala Phe Glu Arg Ser Ile Leu Thr Gln Ile Asp
            385                 390                 395

cat att ctg atg gac aaa gag aga tta ctt cga agg aca cag acc aag      1490

His Ile Leu Met Asp Lys Glu Arg Leu Leu Arg Arg Thr Gln Thr Lys
        400                 405                 410

cgc tct gtc tat cga gtt ctt ggc aaa cct gag cca gca gct cag cct      1538
Arg Ser Val Tyr Arg Val Leu Gly Lys Pro Glu Pro Ala Ala Gln Pro
    415                 420                 425

gtc cca gag agt ttg cca ggg gaa ccg gag atc ctt cct caa gcc cct      1586
Val Pro Glu Ser Leu Pro Gly Glu Pro Glu Ile Leu Pro Gln Ala Pro
430                 435                 440                 445

gct aat gct cat ctg aag gac ttg gat gaa gaa atc ttt gat gat gat      1634
Ala Asn Ala His Leu Lys Asp Leu Asp Glu Glu Ile Phe Asp Asp Asp
                450                 455                 460

gac ttt tac cac cag ctc ctt cga gaa ctc ata gaa cgg aag acc agc      1682
Asp Phe Tyr His Gln Leu Leu Arg Glu Leu Ile Glu Arg Lys Thr Ser
            465                 470                 475

tcc ttg gat ccc aac gat cag gtg gcc atg gga agg cag tgg ctt gca      1730
Ser Leu Asp Pro Asn Asp Gln Val Ala Met Gly Arg Gln Trp Leu Ala
        480                 485                 490

atc cag aag tta cga agc aaa atc cac aaa aaa gta gat agg aaa gcc      1778
Ile Gln Lys Leu Arg Ser Lys Ile His Lys Lys Val Asp Arg Lys Ala
    495                 500                 505

agc aaa ggc agg aaa ctt cgg ttt cat gtc ctt agc aag cta ctg agt      1826
Ser Lys Gly Arg Lys Leu Arg Phe His Val Leu Ser Lys Leu Leu Ser
510                 515                 520                 525

ttc atg gca cct att gac cat act aca atg aat gat gat gcc agg aca      1874
Phe Met Ala Pro Ile Asp His Thr Thr Met Asn Asp Asp Ala Arg Thr
                530                 535                 540

gaa ctg tac cgc tct ctt ttt ggc cag ctc cac cct ccc gac gaa ggc      1922
Glu Leu Tyr Arg Ser Leu Phe Gly Gln Leu His Pro Pro Asp Glu Gly
            545                 550                 555

cac ggg gat tga catcgcccac ctccgacacc cagtgggcgc cttggctggt      1974
His Gly Asp
        560

gcggctgctg gtccagatgg aggaaaccag tgactttatg gggctgagct agtagggaag      2034

cccctggaaa gatgctgcgt tccgaacctg tgcctaatac acgcaagggc gctgtcccgc      2094

ccaaccccgc ctttaaacgc cacaaataaa gagcattgtt accgccaaaa aaaaaaaaaa      2154

aaaaaaaaaa aaaaaaaa                                                    2172

<210>  40
<211>  560
<212>  PRT
<213>  Homo sapiens

<400>  40

Met Ala Gly Pro Gln Pro Leu Ala Leu Gln Leu Glu Gln Leu Leu Asn
1               5                   10                  15

Pro Arg Pro Ser Glu Ala Asp Pro Glu Ala Asp Pro Glu Glu Ala Thr
            20                  25                  30

Ala Ala Arg Val Ile Asp Arg Phe Asp Glu Gly Glu Asp Gly Glu Gly
        35                  40                  45

Asp Phe Leu Val Val Gly Ser Ile Arg Lys Leu Ala Ser Ala Ser Leu
    50                  55                  60

Leu Asp Thr Asp Lys Arg Tyr Cys Gly Lys Thr Thr Ser Arg Lys Ala
65                  70                  75                  80

Trp Asn Glu Asp His Trp Glu Gln Thr Leu Pro Gly Ser Ser Asp Glu
                85                  90                  95

Glu Ile Ser Asp Glu Glu Gly Ser Gly Asp Glu Asp Ser Glu Gly Leu
            100                 105                 110

Gly Leu Glu Glu Tyr Asp Glu Asp Asp Leu Gly Ala Ala Glu Glu Gln
        115                 120                 125

Glu Cys Gly Asp His Arg Glu Ser Lys Lys Ser Arg Ser His Ser Ala
    130                 135                 140

Lys Thr Pro Gly Phe Ser Val Gln Ser Ile Ser Asp Phe Glu Lys Phe
145                 150                 155                 160

Thr Lys Gly Met Asp Asp Leu Gly Ser Ser Glu Glu Glu Glu Asp Glu
                165                 170                 175

Glu Ser Gly Met Glu Glu Gly Asp Asp Ala Glu Asp Ser Gln Gly Glu
            180                 185                 190

Ser Glu Glu Asp Arg Ala Gly Asp Arg Asn Ser Glu Asp Asp Gly Val
        195                 200                 205

Val Met Thr Phe Ser Ser Val Lys Val Ser Glu Glu Val Glu Lys Gly
    210                 215                 220

Arg Ala Val Lys Asn Gln Ile Ala Leu Trp Asp Gln Leu Leu Glu Gly
225                 230                 235                 240

Arg Ile Lys Leu Gln Lys Ala Leu Leu Thr Thr Asn Gln Leu Pro Gln
                245                 250                 255

Pro Asp Val Phe Pro Leu Phe Lys Asp Lys Gly Gly Pro Glu Phe Ser
            260                 265                 270

Ser Ala Leu Lys Asn Ser His Lys Ala Leu Lys Ala Leu Leu Arg Ser
        275                 280                 285

Leu Val Gly Leu Gln Glu Glu Leu Leu Phe Gln Tyr Pro Asp Thr Arg
    290                 295                 300

Tyr Leu Val Asp Gly Thr Lys Pro Asn Ala Gly Ser Glu Glu Ile Ser
305                 310                 315                 320

Ser Glu Asp Asp Glu Leu Val Glu Glu Lys Lys Gln Gln Arg Arg Arg
                325                 330                 335

Val Pro Ala Lys Arg Lys Leu Glu Met Glu Asp Tyr Pro Ser Phe Met
            340                 345                 350

Ala Lys Arg Phe Ala Asp Phe Thr Val Tyr Arg Asn Arg Thr Leu Gln
        355                 360                 365

Lys Trp His Asp Lys Thr Lys Leu Ala Ser Gly Lys Leu Gly Lys Gly
    370                 375                 380

Phe Gly Ala Phe Glu Arg Ser Ile Leu Thr Gln Ile Asp His Ile Leu
385                 390                 395                 400

Met Asp Lys Glu Arg Leu Leu Arg Arg Thr Gln Thr Lys Arg Ser Val
                405                 410                 415

Tyr Arg Val Leu Gly Lys Pro Glu Pro Ala Ala Gln Pro Val Pro Glu
            420                 425                 430

Ser Leu Pro Gly Glu Pro Glu Ile Leu Pro Gln Ala Pro Ala Asn Ala
        435                 440                 445

His Leu Lys Asp Leu Asp Glu Glu Ile Phe Asp Asp Asp Asp Phe Tyr
    450                 455                 460

His Gln Leu Leu Arg Glu Leu Ile Glu Arg Lys Thr Ser Ser Leu Asp
465                 470                 475                 480

Pro Asn Asp Gln Val Ala Met Gly Arg Gln Trp Leu Ala Ile Gln Lys
                485                 490                 495

Leu Arg Ser Lys Ile His Lys Lys Val Asp Arg Lys Ala Ser Lys Gly
            500                 505                 510

Arg Lys Leu Arg Phe His Val Leu Ser Lys Leu Leu Ser Phe Met Ala
        515                 520                 525

Pro Ile Asp His Thr Thr Met Asn Asp Asp Ala Arg Thr Glu Leu Tyr
    530                 535                 540

Arg Ser Leu Phe Gly Gln Leu His Pro Pro Asp Glu Gly His Gly Asp
545                 550                 555                 560

<210>  41
<211>  7492
<212>  DNA
<213>  Homo sapiens

<220>
<221>  CDS
<222>  (283)..(5667)

<400>  41
ggcgctgagc gagctcggag cccgcgctgt gcgcctgcgg ccggggcgcc ccgccgagcg      60

ccggtgcccc ggctcccggg ccgccttcgc cgcgcgggaa ggattcttca aaattaacag     120

aaaccaattc gggccagctg aagagaaaaa ataaaggtgg ctcccggctg cctctgctgc     180

agttcagagc aacttcagga gcttcccagc cgagagcttc aggacgcctt tcctgtccca     240

ctggcccagt tgccacaaca aacaacagag aagacggtga cc atg ggg gat gtg        294
                                               Met Gly Asp Val
                                               1

aag ctg gtt gcc tcg tca cac att tcc aaa acc tcc ctc agt gtg gat       342
Lys Leu Val Ala Ser Ser His Ile Ser Lys Thr Ser Leu Ser Val Asp
5                   10                  15                  20

ccc tca aga gtt gac tcc atg ccc ctg aca gag gcc cct gct ttc att       390
Pro Ser Arg Val Asp Ser Met Pro Leu Thr Glu Ala Pro Ala Phe Ile
                25                  30                  35

ttg ccc cct cgg aac ctc tgc atc aaa gaa gga gcc acc gcc aag ttc       438
Leu Pro Pro Arg Asn Leu Cys Ile Lys Glu Gly Ala Thr Ala Lys Phe
            40                  45                  50

gaa ggg cgg gtc cgg ggt tac cca gag ccc cag gtg aca tgg cac aga       486
Glu Gly Arg Val Arg Gly Tyr Pro Glu Pro Gln Val Thr Trp His Arg
        55                  60                  65

aac ggg caa ccc atc acc agc ggg ggc cgc ttc ctg ctg gat tgc ggc       534
Asn Gly Gln Pro Ile Thr Ser Gly Gly Arg Phe Leu Leu Asp Cys Gly
    70                  75                  80

atc cgg ggg act ttc agc ctt gtg att cat gct gtc cat gag gag gac       582
Ile Arg Gly Thr Phe Ser Leu Val Ile His Ala Val His Glu Glu Asp
85                  90                  95                  100

agg gga aag tat acc tgt gaa gcc acc aat ggc agt ggt gct cgc cag       630
Arg Gly Lys Tyr Thr Cys Glu Ala Thr Asn Gly Ser Gly Ala Arg Gln
                105                 110                 115

gtg aca gtg gag ttg aca gta gaa gga agt ttt gcg aag cag ctt ggt       678
Val Thr Val Glu Leu Thr Val Glu Gly Ser Phe Ala Lys Gln Leu Gly

120                 125                 130

cag cct gtt gtt tcc aaa acc tta ggg gat aga ttt tca gct cca gca          726
Gln Pro Val Val Ser Lys Thr Leu Gly Asp Arg Phe Ser Ala Pro Ala
        135                 140                 145

gtg gag acc cgt cct agc atc tgg ggg gag tgc cca cca aag ttt gct          774
Val Glu Thr Arg Pro Ser Ile Trp Gly Glu Cys Pro Pro Lys Phe Ala
    150                 155                 160

acc aag ctg ggc cga gtt gtg gtc aaa gaa gga cag atg gga cga ttc          822
Thr Lys Leu Gly Arg Val Val Val Lys Glu Gly Gln Met Gly Arg Phe
165                 170                 175                 180

tcc tgc aag atc act ggc cgg ccc caa ccg cag gtc acc tgg ctc aag          870
Ser Cys Lys Ile Thr Gly Arg Pro Gln Pro Gln Val Thr Trp Leu Lys
                185                 190                 195

gga aat gtt cca ctg cag ccg agt gcc cgt gtg tct gtg tct gag aag          918
Gly Asn Val Pro Leu Gln Pro Ser Ala Arg Val Ser Val Ser Glu Lys
            200                 205                 210

aac ggc atg cag gtt ctg gaa atc cat gga gtc aac caa gat gac gtg          966
Asn Gly Met Gln Val Leu Glu Ile His Gly Val Asn Gln Asp Asp Val
        215                 220                 225

gga gtg tac acg tgc ctg gtg gtg aac ggg tcg ggg aag gcc tcg atg          1014
Gly Val Tyr Thr Cys Leu Val Val Asn Gly Ser Gly Lys Ala Ser Met
    230                 235                 240

tca gct gaa ctt tcc atc caa ggt ttg gac agt gcc aat agg tca ttt          1062
Ser Ala Glu Leu Ser Ile Gln Gly Leu Asp Ser Ala Asn Arg Ser Phe
245                 250                 255                 260

gtg aga gaa aca aaa gcc acc aat tca gat gtc agg aaa gag gtg acc          1110
Val Arg Glu Thr Lys Ala Thr Asn Ser Asp Val Arg Lys Glu Val Thr
                265                 270                 275

aat gta atc tca aag gag tcg aag ctg gac agt ctg gag gct gca gcc          1158
Asn Val Ile Ser Lys Glu Ser Lys Leu Asp Ser Leu Glu Ala Ala Ala
            280                 285                 290

aaa agc aag aac tgc tcc agc ccc cag aga ggt ggc tcc cca ccc tgg          1206
Lys Ser Lys Asn Cys Ser Ser Pro Gln Arg Gly Gly Ser Pro Pro Trp
        295                 300                 305

gct gca aac agc cag cct cag ccc cca agg gag tcc aag ctg gag tca          1254
Ala Ala Asn Ser Gln Pro Gln Pro Pro Arg Glu Ser Lys Leu Glu Ser
    310                 315                 320

tgc aag gac tcg ccc aga acg gcc ccg cag acc ccg gtc ctt cag aag          1302
Cys Lys Asp Ser Pro Arg Thr Ala Pro Gln Thr Pro Val Leu Gln Lys
325                 330                 335                 340

act tcc agc tcc atc acc ctg cag gcc gca aga gtt cag ccg gaa cca          1350
Thr Ser Ser Ser Ile Thr Leu Gln Ala Ala Arg Val Gln Pro Glu Pro
                345                 350                 355

aga gca cca ggc ctg ggg gtc cta tca cct tct gga gaa gag agg aag          1398
Arg Ala Pro Gly Leu Gly Val Leu Ser Pro Ser Gly Glu Glu Arg Lys
            360                 365                 370

agg cca gct cct ccc cgt cca gcc acc ttc ccc acc agg cag cct ggc          1446

Arg Pro Ala Pro Pro Arg Pro Ala Thr Phe Pro Thr Arg Gln Pro Gly
        375                 380                 385

ctg ggg agc caa gat gtt gtg agc aag gct gct aac agg aga atc ccc      1494
Leu Gly Ser Gln Asp Val Val Ser Lys Ala Ala Asn Arg Arg Ile Pro
    390                 395                 400

atg gag ggc cag agg gat tca gca ttc ccc aaa ttt gag agc aag ccc      1542
Met Glu Gly Gln Arg Asp Ser Ala Phe Pro Lys Phe Glu Ser Lys Pro
405                 410                 415                 420

caa agc cag gag gtc aag gaa aat caa act gtc aag ttc aga tgt gaa      1590
Gln Ser Gln Glu Val Lys Glu Asn Gln Thr Val Lys Phe Arg Cys Glu
                425                 430                 435

ggg ctt gcc gtg atg gag gtg gcc ccc tcc ttc tcc agt gtc ctg aag      1638
Gly Leu Ala Val Met Glu Val Ala Pro Ser Phe Ser Ser Val Leu Lys
            440                 445                 450

gac tgc gct gtt att gag ggc cag gat ttt gtg ctg cag tgc tcc gta      1686
Asp Cys Ala Val Ile Glu Gly Gln Asp Phe Val Leu Gln Cys Ser Val
        455                 460                 465

cgg ggg acc cca gtg ccc cgg atc act tgg ctg ctg aat ggg cag ccc      1734
Arg Gly Thr Pro Val Pro Arg Ile Thr Trp Leu Leu Asn Gly Gln Pro
    470                 475                 480

atc cag tac gct cgc tcc acc tgc gag gcc ggc gtg gct gag ctc cac      1782
Ile Gln Tyr Ala Arg Ser Thr Cys Glu Ala Gly Val Ala Glu Leu His
485                 490                 495                 500

atc cag gat gcc ctg ccg gag gac cat ggc acc tac acc tgc cta gct      1830
Ile Gln Asp Ala Leu Pro Glu Asp His Gly Thr Tyr Thr Cys Leu Ala
                505                 510                 515

gag aat gcc ttg ggg cag gtg tcc tgc agc gcc tgg gtc acc gtc cat      1878
Glu Asn Ala Leu Gly Gln Val Ser Cys Ser Ala Trp Val Thr Val His
            520                 525                 530

gaa aag aag agt agc agg aag agt gag tac ctt ctg cct gtg gct ccc      1926
Glu Lys Lys Ser Ser Arg Lys Ser Glu Tyr Leu Leu Pro Val Ala Pro
        535                 540                 545

agc aag ccc act gca ccc atc ttc ctg cag ggc ctc tct gat ctc aaa      1974
Ser Lys Pro Thr Ala Pro Ile Phe Leu Gln Gly Leu Ser Asp Leu Lys
    550                 555                 560

gtc atg gat gga agc cag gtc act atg act gtc caa gtg tca ggg aat      2022
Val Met Asp Gly Ser Gln Val Thr Met Thr Val Gln Val Ser Gly Asn
565                 570                 575                 580

cca ccc cct gaa gtc atc tgg ctg cac aat ggg aat gag atc caa gag      2070
Pro Pro Pro Glu Val Ile Trp Leu His Asn Gly Asn Glu Ile Gln Glu
                585                 590                 595

tca gag gac ttc cac ttt gaa cag aga gga act cag cac agc ctt tgt      2118
Ser Glu Asp Phe His Phe Glu Gln Arg Gly Thr Gln His Ser Leu Cys
            600                 605                 610

atc cag gaa gtg ttc ccg gag gac acg ggc acg tac acc tgc gag gcc      2166
Ile Gln Glu Val Phe Pro Glu Asp Thr Gly Thr Tyr Thr Cys Glu Ala
        615                 620                 625

tgg aac agc gct gga gag gtc cgc acc cag gcc gtg ctc acg gta caa     2214
Trp Asn Ser Ala Gly Glu Val Arg Thr Gln Ala Val Leu Thr Val Gln
    630                 635                 640

gag cct cac gat ggc acc cag ccc tgg ttc atc agt aag cct cgc tca     2262
Glu Pro His Asp Gly Thr Gln Pro Trp Phe Ile Ser Lys Pro Arg Ser
645                 650                 655                 660

gtg aca gcc tcc ctg ggc cag agt gtc ctc atc tcc tgc gcc ata gct     2310
Val Thr Ala Ser Leu Gly Gln Ser Val Leu Ile Ser Cys Ala Ile Ala
                665                 670                 675

ggt gac ccc ttt cct acc gtg cac tgg ctc aga gat ggc aaa gcc ctc     2358
Gly Asp Pro Phe Pro Thr Val His Trp Leu Arg Asp Gly Lys Ala Leu
            680                 685                 690

tgc aaa gac act ggc cac ttc gag gtg ctt cag aat gag gac gtg ttc     2406
Cys Lys Asp Thr Gly His Phe Glu Val Leu Gln Asn Glu Asp Val Phe
        695                 700                 705

acc ctg gtt cta aag aag gtg cag ccc tgg cat gcc ggc cag tat gag     2454
Thr Leu Val Leu Lys Lys Val Gln Pro Trp His Ala Gly Gln Tyr Glu
    710                 715                 720

atc ctg ctc aag aac cgg gtt ggc gaa tgc agt tgc cag gtg tca ctg     2502
Ile Leu Leu Lys Asn Arg Val Gly Glu Cys Ser Cys Gln Val Ser Leu
725                 730                 735                 740

atg cta cag aac agc tct gcc aga gcc ctt cca cgg ggg agg gag cct     2550
Met Leu Gln Asn Ser Ser Ala Arg Ala Leu Pro Arg Gly Arg Glu Pro
                745                 750                 755

gcc agc tgc gag gac ctc tgt ggt gga gga gtt ggt gct gat ggt ggt     2598
Ala Ser Cys Glu Asp Leu Cys Gly Gly Gly Val Gly Ala Asp Gly Gly
            760                 765                 770

ggt agt gac cgc tat ggg tcc ctg agg cct ggc tgg cca gca aga ggg     2646
Gly Ser Asp Arg Tyr Gly Ser Leu Arg Pro Gly Trp Pro Ala Arg Gly
        775                 780                 785

cag ggt tgg cta gag gag gaa gac ggc gag gac gtg cga ggg gtg ctg     2694
Gln Gly Trp Leu Glu Glu Glu Asp Gly Glu Asp Val Arg Gly Val Leu
    790                 795                 800

aag agg cgc gtg gag acg agg cag cac act gag gag gcg atc cgc cag     2742
Lys Arg Arg Val Glu Thr Arg Gln His Thr Glu Glu Ala Ile Arg Gln
805                 810                 815                 820

cag gag gtg gag cag ctg gac ttc cga gac ctc ctg ggg aag aag gtg     2790
Gln Glu Val Glu Gln Leu Asp Phe Arg Asp Leu Leu Gly Lys Lys Val
                825                 830                 835

agt aca aag acc cta tcg gaa gac gac ctg aag gag atc cca gcc gag     2838
Ser Thr Lys Thr Leu Ser Glu Asp Asp Leu Lys Glu Ile Pro Ala Glu
            840                 845                 850

cag atg gat ttc cgt gcc aac ctg cag cgg caa gtg aag cca aag act     2886
Gln Met Asp Phe Arg Ala Asn Leu Gln Arg Gln Val Lys Pro Lys Thr
        855                 860                 865

gtg tct gag gaa gag agg aag gtg cac agc ccc cag cag gtc gat ttt     2934
Val Ser Glu Glu Glu Arg Lys Val His Ser Pro Gln Gln Val Asp Phe
    870                 875                 880

cgc tct gtc ctg gcc aag aag ggg act tcc aag acc ccc gtg cct gag 2982
Arg Ser Val Leu Ala Lys Lys Gly Thr Ser Lys Thr Pro Val Pro Glu
885                 890                 895                 900

aag gtg cca ccg cca aaa cct gcc acc ccg gat ttt cgc tca gtg ctg 3030
Lys Val Pro Pro Pro Lys Pro Ala Thr Pro Asp Phe Arg Ser Val Leu
                905                 910                 915

ggt ggc aag aag aaa tta cca gca gag aat ggc agc agc agt gcc gag 3078
Gly Gly Lys Lys Lys Leu Pro Ala Glu Asn Gly Ser Ser Ser Ala Glu
            920                 925                 930

acc ctg aat gcc aag gca gtg gag agt tcc aag ccc ctg agc aat gca 3126
Thr Leu Asn Ala Lys Ala Val Glu Ser Ser Lys Pro Leu Ser Asn Ala
        935                 940                 945

cag cct tca ggg ccc ttg aaa ccc gtg ggc aac gcc aag cct gct gag 3174
Gln Pro Ser Gly Pro Leu Lys Pro Val Gly Asn Ala Lys Pro Ala Glu
    950                 955                 960

acc ctg aag cca atg ggc aac gcc aag cct gcc gag acc ctg aag ccc 3222
Thr Leu Lys Pro Met Gly Asn Ala Lys Pro Ala Glu Thr Leu Lys Pro
965                 970                 975                 980

atg ggc aat gcc aag cct gat gag aac ctg aaa tcc gct agc aaa gaa 3270
Met Gly Asn Ala Lys Pro Asp Glu Asn Leu Lys Ser Ala Ser Lys Glu
                985                 990                 995

gaa ctc aag aaa gac gtt aag aat gat gtg aac tgc aag aga ggc 3315
Glu Leu Lys Lys Asp Val Lys Asn Asp Val Asn Cys Lys Arg Gly
            1000                1005                1010

cat gca ggg acc aca gat aat gaa aag aga tca gag agc cag ggg 3360
His Ala Gly Thr Thr Asp Asn Glu Lys Arg Ser Glu Ser Gln Gly
            1015                1020                1025

aca gcc cca gcc ttc aag cag aag ctg caa gat gtt cat gtg gca 3405
Thr Ala Pro Ala Phe Lys Gln Lys Leu Gln Asp Val His Val Ala
            1030                1035                1040

gag ggc aag aag ctg ctg ctc cag tgc cag gtg tct tct gac ccc 3450
Glu Gly Lys Lys Leu Leu Leu Gln Cys Gln Val Ser Ser Asp Pro
            1045                1050                1055

cca gcc acc atc atc tgg acg ctg aac gga aag acc ctc aag acc 3495
Pro Ala Thr Ile Ile Trp Thr Leu Asn Gly Lys Thr Leu Lys Thr
            1060                1065                1070

acc aag ttc atc atc ctc tcc cag gaa ggc tca ctc tgc tcc gtc 3540
Thr Lys Phe Ile Ile Leu Ser Gln Glu Gly Ser Leu Cys Ser Val
            1075                1080                1085

tcc atc gag aag gca ctg cct gag gac aga ggc tta tac aag tgt 3585
Ser Ile Glu Lys Ala Leu Pro Glu Asp Arg Gly Leu Tyr Lys Cys
            1090                1095                1100

gta gcc aag aat gac gct ggc cag gcg gag tgc tcc tgc caa gtc 3630
Val Ala Lys Asn Asp Ala Gly Gln Ala Glu Cys Ser Cys Gln Val
            1105                1110                1115

acc gtg gat gat gct cca gcc agt gag aac acc aag gcc cca gag 3675
Thr Val Asp Asp Ala Pro Ala Ser Glu Asn Thr Lys Ala Pro Glu

1120                1125                1130

atg aaa tcc cgg agg ccc aag agc tct ctt cct ccc gtg cta gga        3720
Met Lys Ser Arg Arg Pro Lys Ser Ser Leu Pro Pro Val Leu Gly
        1135                1140                1145

act gag agt gat gcg act gtg aaa aag aaa cct gcc ccc aag aca        3765
Thr Glu Ser Asp Ala Thr Val Lys Lys Lys Pro Ala Pro Lys Thr
        1150                1155                1160

cct ccg aag gca gca atg ccc cct cag atc atc cag ttc cct gag        3810
Pro Pro Lys Ala Ala Met Pro Pro Gln Ile Ile Gln Phe Pro Glu
        1165                1170                1175

gac cag aag gta cgc gca gga gag tca gtg gag ctg ttt ggc aaa        3855
Asp Gln Lys Val Arg Ala Gly Glu Ser Val Glu Leu Phe Gly Lys
        1180                1185                1190

gtg aca ggc act cag ccc atc acc tgt acc tgg atg aag ttc cga        3900
Val Thr Gly Thr Gln Pro Ile Thr Cys Thr Trp Met Lys Phe Arg
        1195                1200                1205

aag cag atc cag gaa agc gag cac atg aag gtg gag aac agc gag        3945
Lys Gln Ile Gln Glu Ser Glu His Met Lys Val Glu Asn Ser Glu
        1210                1215                1220

aat ggc agc aag ctc acc atc ctg gcc gcg cgc cag gag cac tgc        3990
Asn Gly Ser Lys Leu Thr Ile Leu Ala Ala Arg Gln Glu His Cys
        1225                1230                1235

ggc tgc tac aca ctg ctg gtg gag aac aag ctg ggc agc agg cag        4035
Gly Cys Tyr Thr Leu Leu Val Glu Asn Lys Leu Gly Ser Arg Gln
        1240                1245                1250

gcc cag gtc aac ctc act gtc gtg gat aag cca gac ccc cca gct        4080
Ala Gln Val Asn Leu Thr Val Val Asp Lys Pro Asp Pro Pro Ala
        1255                1260                1265

ggc aca cct tgt gcc tct gac att cgg agc tcc tca ctg acc ctg        4125
Gly Thr Pro Cys Ala Ser Asp Ile Arg Ser Ser Ser Leu Thr Leu
        1270                1275                1280

tcc tgg tat ggc tcc tca tat gat ggg ggc agt gct gta cag tcc        4170
Ser Trp Tyr Gly Ser Ser Tyr Asp Gly Gly Ser Ala Val Gln Ser
        1285                1290                1295

tac agc atc gag atc tgg gac tca gcc aac aag acg tgg aag gaa        4215
Tyr Ser Ile Glu Ile Trp Asp Ser Ala Asn Lys Thr Trp Lys Glu
        1300                1305                1310

cta gcc aca tgc cgc agc acc tct ttc aac gtc cag gac ctg ctg        4260
Leu Ala Thr Cys Arg Ser Thr Ser Phe Asn Val Gln Asp Leu Leu
        1315                1320                1325

cct gac cac gaa tat aag ttc cgt gta cgt gca atc aac gtg tat        4305
Pro Asp His Glu Tyr Lys Phe Arg Val Arg Ala Ile Asn Val Tyr
        1330                1335                1340

gga acc agt gag cca agc cag gag tct gaa ctc aca acg gta gga        4350
Gly Thr Ser Glu Pro Ser Gln Glu Ser Glu Leu Thr Thr Val Gly
        1345                1350                1355

gag aaa cct gaa gag ccg aag gat gaa gtg gag gtg tca gat gat        4395

Glu Lys Pro Glu Glu Pro Lys Asp Glu Val Glu Val Ser Asp Asp
            1360                1365                1370

gat gag aag gag ccc gag gtt gat tac cgg aca gtg aca atc aat 4440
Asp Glu Lys Glu Pro Glu Val Asp Tyr Arg Thr Val Thr Ile Asn
            1375                1380                1385

act gaa caa aaa gta tct gac ttc tac gac att gag gag aga tta 4485
Thr Glu Gln Lys Val Ser Asp Phe Tyr Asp Ile Glu Glu Arg Leu
            1390                1395                1400

gga tct ggg aaa ttt gga cag gtc ttt cga ctt gta gaa aag aaa 4530
Gly Ser Gly Lys Phe Gly Gln Val Phe Arg Leu Val Glu Lys Lys
            1405                1410                1415

act cga aaa gtc tgg gca ggg aag ttc ttc aag gca tat tca gca 4575
Thr Arg Lys Val Trp Ala Gly Lys Phe Phe Lys Ala Tyr Ser Ala
            1420                1425                1430

aaa gag aaa gag aat atc cgg cag gag att agc atc atg aac tgc 4620
Lys Glu Lys Glu Asn Ile Arg Gln Glu Ile Ser Ile Met Asn Cys
            1435                1440                1445

ctc cac cac cct aag ctg gtc cag tgt gtg gat gcc ttt gaa gaa 4665
Leu His His Pro Lys Leu Val Gln Cys Val Asp Ala Phe Glu Glu
            1450                1455                1460

aag gcc aac atc gtc atg gtc ctg gag atc gtg tca gga ggg gag 4710
Lys Ala Asn Ile Val Met Val Leu Glu Ile Val Ser Gly Gly Glu
            1465                1470                1475

ctg ttt gag cgc atc att gac gag gac ttt gag ctg acg gag cgt 4755
Leu Phe Glu Arg Ile Ile Asp Glu Asp Phe Glu Leu Thr Glu Arg
            1480                1485                1490

gag tgc atc aag tac atg cgg cag atc tcg gag gga gtg gag tac 4800
Glu Cys Ile Lys Tyr Met Arg Gln Ile Ser Glu Gly Val Glu Tyr
            1495                1500                1505

atc cac aag cag ggc atc gtg cac ctg gac ctc aag ccg gag aac 4845
Ile His Lys Gln Gly Ile Val His Leu Asp Leu Lys Pro Glu Asn
            1510                1515                1520

atc atg tgt gtc aac aag acg ggc acc agg atc aag ctc atc gac 4890
Ile Met Cys Val Asn Lys Thr Gly Thr Arg Ile Lys Leu Ile Asp
            1525                1530                1535

ttt ggt ctg gcc agg agg ctg gag aat gcg ggg tct ctg aag gtc 4935
Phe Gly Leu Ala Arg Arg Leu Glu Asn Ala Gly Ser Leu Lys Val
            1540                1545                1550

ctc ttt ggc acc cca gaa ttt gtg gct cct gaa gtg atc aac tat 4980
Leu Phe Gly Thr Pro Glu Phe Val Ala Pro Glu Val Ile Asn Tyr
            1555                1560                1565

gag ccc atc ggc tac gcc aca gac atg tgg agc atc ggg gtc atc 5025
Glu Pro Ile Gly Tyr Ala Thr Asp Met Trp Ser Ile Gly Val Ile
            1570                1575                1580

tgc tac atc cta aac cgc ctg gac tgc acg cag tgc ctt cag cat 5070
Cys Tyr Ile Leu Asn Arg Leu Asp Cys Thr Gln Cys Leu Gln His
            1585                1590                1595

cca tgg cta atg aaa gat acc aag aac atg gag gcc aag aaa ctc 5115
Pro Trp Leu Met Lys Asp Thr Lys Asn Met Glu Ala Lys Lys Leu
            1600                1605                1610

tcc aag gac cgg atg aag aag tac atg gca aga agg aaa tgg cag 5160
Ser Lys Asp Arg Met Lys Lys Tyr Met Ala Arg Arg Lys Trp Gln
            1615                1620                1625

aaa acg ggc aat gct gtg aga gcc att gga aga ctg tcc tct atg 5205
Lys Thr Gly Asn Ala Val Arg Ala Ile Gly Arg Leu Ser Ser Met
            1630                1635                1640

gca atg atc tca ggg ctc agt ggc agg aaa tcc tca aca ggg tca 5250
Ala Met Ile Ser Gly Leu Ser Gly Arg Lys Ser Ser Thr Gly Ser
            1645                1650                1655

cca acc agc ccg ctc aat gca gaa aaa cta gaa tct gaa gaa gat 5295
Pro Thr Ser Pro Leu Asn Ala Glu Lys Leu Glu Ser Glu Glu Asp
            1660                1665                1670

gtg tcc caa gct ttc ctt gag gct gtt gct gag gaa aag cct cat 5340
Val Ser Gln Ala Phe Leu Glu Ala Val Ala Glu Glu Lys Pro His
            1675                1680                1685

gta aaa ccc tat ttc tct aag acc att cgc gat tta gaa gtt gtg 5385
Val Lys Pro Tyr Phe Ser Lys Thr Ile Arg Asp Leu Glu Val Val
            1690                1695                1700

gag gga agt gct gct aga ttt gac tgc aag att gaa gga tac cca 5430
Glu Gly Ser Ala Ala Arg Phe Asp Cys Lys Ile Glu Gly Tyr Pro
            1705                1710                1715

gac ccc gag gtt gtc tgg ttc aaa gat gac cag tca atc agg gag 5475
Asp Pro Glu Val Val Trp Phe Lys Asp Asp Gln Ser Ile Arg Glu
            1720                1725                1730

tcc cgc cac ttc cag ata gac tac gat gag gac ggg aac tgc tct 5520
Ser Arg His Phe Gln Ile Asp Tyr Asp Glu Asp Gly Asn Cys Ser
            1735                1740                1745

tta att att agt gat gtt tgc ggg gat gac gat gcc aag tac acc 5565
Leu Ile Ile Ser Asp Val Cys Gly Asp Asp Asp Ala Lys Tyr Thr
            1750                1755                1760

tgc aag gct gtc aac agt ctt gga gaa gcc acc tgc aca gca gag 5610
Cys Lys Ala Val Asn Ser Leu Gly Glu Ala Thr Cys Thr Ala Glu
            1765                1770                1775

ctc att gtg gaa acg atg gag gaa ggt gaa ggg gaa ggg gaa gag 5655
Leu Ile Val Glu Thr Met Glu Glu Gly Glu Gly Glu Gly Glu Glu
            1780                1785                1790

gaa gaa gag tga aacaaagcca gagaaaagca gtttctaagt catattaaaa 5707
Glu Glu Glu

ggactatttc tctaaaactc aaaaaaaaaa aaaaaactca agatagtaaa agcacctagt 5767

gtgatagatt atcggttagg tcatttgtgg gttgattctt cagaaacagc agttgatacc 5827

tagcagcgtt attgatgggc attaatctat gttagttggc accttaagat actagtgcag 5887

ctagatttca tttagggaaa tcaccagtaa cttgactgac caattgattt tagagagaaa 5947

gtaaccaaac caaatattta tctgggcaaa gtcataaatt ctccacttga atgcgctcat 6007

gaaaaataag gccaaaacaa gagttctggg ccacagctca gcccagaggg ttcctgggga 6067

tgggaggcct ctctctcccc accccctgac tctagagaac tgggttttct cccagtactc 6127

cagcaattca tttctgaaag cagttgagcc actttattcc aaagtacact gcagatgttc 6187

aaactctcca tttctctttc cccttccacc tgccagtttt gctgactctc aacttgtcat 6247

gagtgtaagc attaaggaca ttatgcttct tcgattctga agacaggtcc ctgctcatgg 6307

atgactctgg cttccttagg aaaatatttt tcttccaaaa tcagtaggaa atctaaactt 6367

atcccctctt tgcagatgtc tagcagcttc agacatttgg ttaagaaccc atgggaaaaa 6427

aaaaatcctt gctaatgtgg tttcctttgt aaaccaggat tcttatttgt gctgttatag 6487

aatatcagct ctgaacgtgt ggtaaagatt tttgtgtttg aatataggag aaatcagttt 6547

gctgaaaagt tagtcttaat tatctattgg ccacgatgaa acagatttca actgataaag 6607

agctggagaa ctccatgtac tttggaatct cctccaagat agccagagtt taatacatct 6667

tcattctcaa cactctccaa agaacttgac ctaccttatg ggttccatat ttttcttctt 6727

aaatgtgcat caatcatgcc ttgcccccaa cctttaaata tattcttaga cctggtaaat 6787

gcactcagac ttgcgtcttt aggaattttt aactttcttt cactacattg gcacttaaat 6847

tttttcttta taaagctttt tgaaggtcat aaacaaagac cataattgat gatagaccta 6907

atacatttcc tctgtgtgtg tgtgtaacat tccaaatact ttttttttct tttccactgt 6967

ttgtaaggtg caacaattta atatttttaa gggacttttt aagagttcct taagaaccaa 7027

tttaaaatta cttcagtgca atcctacaca gtatcaacat tagaattttg atattagtct 7087

tatgttatct tccattctat ttttatctgc tttttgctgc tagtttcaaa ctgccagtat 7147

ttttcctttt gcttttaaaa tagttacaat atttttcatg atagccacag tattgccaca 7207

gtttattata ataaagggtt tttatttgat ttagcgcatt caaagctttt ttctatcact 7267

tttgtgttca gaatataacc tttgtgtgcg tgtatgttgt gtgtgtgcat gtgtggcgta 7327

tatgtgtgtt acaggttaat gccttcttgg aattgtgtta atgttctctt ggtttattat 7387

gccatcagaa tggtaaatga gaacactaca actgtagtca gctcacaatt tttaaataaa 7447

ggataccaca gtgcatgctg tttgttcaaa aaaaaaaaaa aaaaa 7492

<210> 42
<211> 1794
<212> PRT
<213> Homo sapiens

<400> 42

Met Gly Asp Val Lys Leu Val Ala Ser Ser His Ile Ser Lys Thr Ser
1               5                   10                  15

Leu Ser Val Asp Pro Ser Arg Val Asp Ser Met Pro Leu Thr Glu Ala
            20                  25                  30

Pro Ala Phe Ile Leu Pro Pro Arg Asn Leu Cys Ile Lys Glu Gly Ala
        35                  40                  45

Thr Ala Lys Phe Glu Gly Arg Val Arg Gly Tyr Pro Glu Pro Gln Val
    50                  55                  60

Thr Trp His Arg Asn Gly Gln Pro Ile Thr Ser Gly Gly Arg Phe Leu
65                  70                  75                  80

Leu Asp Cys Gly Ile Arg Gly Thr Phe Ser Leu Val Ile His Ala Val
                85                  90                  95

His Glu Glu Asp Arg Gly Lys Tyr Thr Cys Glu Ala Thr Asn Gly Ser
            100                 105                 110

Gly Ala Arg Gln Val Thr Val Glu Leu Thr Val Glu Gly Ser Phe Ala
        115                 120                 125

Lys Gln Leu Gly Gln Pro Val Val Ser Lys Thr Leu Gly Asp Arg Phe
    130                 135                 140

Ser Ala Pro Ala Val Glu Thr Arg Pro Ser Ile Trp Gly Glu Cys Pro
145                 150                 155                 160

Pro Lys Phe Ala Thr Lys Leu Gly Arg Val Val Val Lys Glu Gly Gln
                165                 170                 175

Met Gly Arg Phe Ser Cys Lys Ile Thr Gly Arg Pro Gln Pro Gln Val
            180                 185                 190

Thr Trp Leu Lys Gly Asn Val Pro Leu Gln Pro Ser Ala Arg Val Ser
        195                 200                 205

Val Ser Glu Lys Asn Gly Met Gln Val Leu Glu Ile His Gly Val Asn
    210                 215                 220

Gln Asp Asp Val Gly Val Tyr Thr Cys Leu Val Val Asn Gly Ser Gly
225                 230                 235                 240

Lys Ala Ser Met Ser Ala Glu Leu Ser Ile Gln Gly Leu Asp Ser Ala
                245                 250                 255

Asn Arg Ser Phe Val Arg Glu Thr Lys Ala Thr Asn Ser Asp Val Arg

            260                 265                 270

Lys Glu Val Thr Asn Val Ile Ser Lys Glu Ser Lys Leu Asp Ser Leu
        275                 280                 285

Glu Ala Ala Ala Lys Ser Lys Asn Cys Ser Ser Pro Gln Arg Gly Gly
    290                 295                 300

Ser Pro Pro Trp Ala Ala Asn Ser Gln Pro Gln Pro Pro Arg Glu Ser
305                 310                 315                 320

Lys Leu Glu Ser Cys Lys Asp Ser Pro Arg Thr Ala Pro Gln Thr Pro
                325                 330                 335

Val Leu Gln Lys Thr Ser Ser Ser Ile Thr Leu Gln Ala Ala Arg Val
            340                 345                 350

Gln Pro Glu Pro Arg Ala Pro Gly Leu Gly Val Leu Ser Pro Ser Gly
        355                 360                 365

Glu Glu Arg Lys Arg Pro Ala Pro Pro Arg Pro Ala Thr Phe Pro Thr
    370                 375                 380

Arg Gln Pro Gly Leu Gly Ser Gln Asp Val Val Ser Lys Ala Ala Asn
385                 390                 395                 400

Arg Arg Ile Pro Met Glu Gly Gln Arg Asp Ser Ala Phe Pro Lys Phe
                405                 410                 415

Glu Ser Lys Pro Gln Ser Gln Glu Val Lys Glu Asn Gln Thr Val Lys
            420                 425                 430

Phe Arg Cys Glu Gly Leu Ala Val Met Glu Val Ala Pro Ser Phe Ser
        435                 440                 445

Ser Val Leu Lys Asp Cys Ala Val Ile Glu Gly Gln Asp Phe Val Leu
    450                 455                 460

Gln Cys Ser Val Arg Gly Thr Pro Val Pro Arg Ile Thr Trp Leu Leu
465                 470                 475                 480

Asn Gly Gln Pro Ile Gln Tyr Ala Arg Ser Thr Cys Glu Ala Gly Val
                485                 490                 495

Ala Glu Leu His Ile Gln Asp Ala Leu Pro Glu Asp His Gly Thr Tyr
            500                 505                 510

Thr Cys Leu Ala Glu Asn Ala Leu Gly Gln Val Ser Cys Ser Ala Trp
        515                 520                 525

Val Thr Val His Glu Lys Lys Ser Ser Arg Lys Ser Glu Tyr Leu Leu
    530                 535                 540

Pro Val Ala Pro Ser Lys Pro Thr Ala Pro Ile Phe Leu Gln Gly Leu
545                 550                 555                 560

Ser Asp Leu Lys Val Met Asp Gly Ser Gln Val Thr Met Thr Val Gln
                565                 570                 575

Val Ser Gly Asn Pro Pro Pro Glu Val Ile Trp Leu His Asn Gly Asn
            580                 585                 590

Glu Ile Gln Glu Ser Glu Asp Phe His Phe Glu Gln Arg Gly Thr Gln
        595                 600                 605

His Ser Leu Cys Ile Gln Glu Val Phe Pro Glu Asp Thr Gly Thr Tyr
    610                 615                 620

Thr Cys Glu Ala Trp Asn Ser Ala Gly Glu Val Arg Thr Gln Ala Val
625                 630                 635                 640

Leu Thr Val Gln Glu Pro His Asp Gly Thr Gln Pro Trp Phe Ile Ser
                645                 650                 655

Lys Pro Arg Ser Val Thr Ala Ser Leu Gly Gln Ser Val Leu Ile Ser
            660                 665                 670

Cys Ala Ile Ala Gly Asp Pro Phe Pro Thr Val His Trp Leu Arg Asp
        675                 680                 685

Gly Lys Ala Leu Cys Lys Asp Thr Gly His Phe Glu Val Leu Gln Asn
    690                 695                 700

Glu Asp Val Phe Thr Leu Val Leu Lys Lys Val Gln Pro Trp His Ala
705                 710                 715                 720

Gly Gln Tyr Glu Ile Leu Leu Lys Asn Arg Val Gly Glu Cys Ser Cys
                725                 730                 735

Gln Val Ser Leu Met Leu Gln Asn Ser Ser Ala Arg Ala Leu Pro Arg
            740                 745                 750

Gly Arg Glu Pro Ala Ser Cys Glu Asp Leu Cys Gly Gly Gly Val Gly
        755                 760                 765

Ala Asp Gly Gly Gly Ser Asp Arg Tyr Gly Ser Leu Arg Pro Gly Trp
    770                 775                 780

Pro Ala Arg Gly Gln Gly Trp Leu Glu Glu Glu Asp Gly Glu Asp Val
785                 790                 795                 800

Arg Gly Val Leu Lys Arg Arg Val Glu Thr Arg Gln His Thr Glu Glu
                805                 810                 815

Ala Ile Arg Gln Gln Glu Val Glu Gln Leu Asp Phe Arg Asp Leu Leu
            820                 825                 830

Gly Lys Lys Val Ser Thr Lys Thr Leu Ser Glu Asp Asp Leu Lys Glu
        835                 840                 845

Ile Pro Ala Glu Gln Met Asp Phe Arg Ala Asn Leu Gln Arg Gln Val
    850                 855                 860

Lys Pro Lys Thr Val Ser Glu Glu Glu Arg Lys Val His Ser Pro Gln
865                 870                 875                 880

Gln Val Asp Phe Arg Ser Val Leu Ala Lys Lys Gly Thr Ser Lys Thr
                885                 890                 895

Pro Val Pro Glu Lys Val Pro Pro Pro Lys Pro Ala Thr Pro Asp Phe
            900                 905                 910

Arg Ser Val Leu Gly Gly Lys Lys Lys Leu Pro Ala Glu Asn Gly Ser
        915                 920                 925

Ser Ser Ala Glu Thr Leu Asn Ala Lys Ala Val Glu Ser Ser Lys Pro
    930                 935                 940

Leu Ser Asn Ala Gln Pro Ser Gly Pro Leu Lys Pro Val Gly Asn Ala
945                 950                 955                 960

Lys Pro Ala Glu Thr Leu Lys Pro Met Gly Asn Ala Lys Pro Ala Glu
                965                 970                 975

Thr Leu Lys Pro Met Gly Asn Ala Lys Pro Asp Glu Asn Leu Lys Ser
            980                 985                 990

Ala Ser Lys Glu Glu Leu Lys Lys  Asp Val Lys Asn Asp  Val Asn Cys
        995                 1000                1005

Lys Arg  Gly His Ala Gly Thr  Thr Asp Asn Glu Lys  Arg Ser Glu
    1010                1015                1020

Ser Gln Gly Thr Ala Pro Ala Phe Lys Gln Lys Leu Gln Asp Val
    1025                1030                1035

His Val Ala Glu Gly Lys Lys Leu Leu Leu Gln Cys Gln Val Ser
    1040                1045                1050

Ser Asp Pro Pro Ala Thr Ile Ile Trp Thr Leu Asn Gly Lys Thr
    1055                1060                1065

Leu Lys Thr Thr Lys Phe Ile Ile Leu Ser Gln Glu Gly Ser Leu
    1070                1075                1080

Cys Ser Val Ser Ile Glu Lys Ala Leu Pro Glu Asp Arg Gly Leu
    1085                1090                1095

Tyr Lys Cys Val Ala Lys Asn Asp Ala Gly Gln Ala Glu Cys Ser
    1100                1105                1110

Cys Gln Val Thr Val Asp Asp Ala Pro Ala Ser Glu Asn Thr Lys
    1115                1120                1125

Ala Pro Glu Met Lys Ser Arg Arg Pro Lys Ser Ser Leu Pro Pro
    1130                1135                1140

Val Leu Gly Thr Glu Ser Asp Ala Thr Val Lys Lys Lys Pro Ala
    1145                1150                1155

Pro Lys Thr Pro Pro Lys Ala Ala Met Pro Pro Gln Ile Ile Gln
    1160                1165                1170

Phe Pro Glu Asp Gln Lys Val Arg Ala Gly Glu Ser Val Glu Leu
    1175                1180                1185

Phe Gly Lys Val Thr Gly Thr Gln Pro Ile Thr Cys Thr Trp Met
    1190                1195                1200

Lys Phe Arg Lys Gln Ile Gln Glu Ser Glu His Met Lys Val Glu
    1205                1210                1215

Asn Ser Glu Asn Gly Ser Lys Leu Thr Ile Leu Ala Ala Arg Gln
    1220                1225                1230

Glu His Cys Gly Cys Tyr Thr Leu Leu Val Glu Asn Lys Leu Gly
    1235                1240                1245

Ser Arg Gln Ala Gln Val Asn Leu Thr Val Val Asp Lys Pro Asp

1250                1255                1260

Pro Pro Ala Gly Thr Pro Cys Ala Ser Asp Ile Arg Ser Ser Ser
    1265                1270                1275

Leu Thr Leu Ser Trp Tyr Gly Ser Ser Tyr Asp Gly Gly Ser Ala
    1280                1285                1290

Val Gln Ser Tyr Ser Ile Glu Ile Trp Asp Ser Ala Asn Lys Thr
    1295                1300                1305

Trp Lys Glu Leu Ala Thr Cys Arg Ser Thr Ser Phe Asn Val Gln
    1310                1315                1320

Asp Leu Leu Pro Asp His Glu Tyr Lys Phe Arg Val Arg Ala Ile
    1325                1330                1335

Asn Val Tyr Gly Thr Ser Glu Pro Ser Gln Glu Ser Glu Leu Thr
    1340                1345                1350

Thr Val Gly Glu Lys Pro Glu Glu Pro Lys Asp Glu Val Glu Val
    1355                1360                1365

Ser Asp Asp Asp Glu Lys Glu Pro Glu Val Asp Tyr Arg Thr Val
    1370                1375                1380

Thr Ile Asn Thr Glu Gln Lys Val Ser Asp Phe Tyr Asp Ile Glu
    1385                1390                1395

Glu Arg Leu Gly Ser Gly Lys Phe Gly Gln Val Phe Arg Leu Val
    1400                1405                1410

Glu Lys Lys Thr Arg Lys Val Trp Ala Gly Lys Phe Phe Lys Ala
    1415                1420                1425

Tyr Ser Ala Lys Glu Lys Glu Asn Ile Arg Gln Glu Ile Ser Ile
    1430                1435                1440

Met Asn Cys Leu His His Pro Lys Leu Val Gln Cys Val Asp Ala
    1445                1450                1455

Phe Glu Glu Lys Ala Asn Ile Val Met Val Leu Glu Ile Val Ser
    1460                1465                1470

Gly Gly Glu Leu Phe Glu Arg Ile Ile Asp Glu Asp Phe Glu Leu
    1475                1480                1485

Thr Glu Arg Glu Cys Ile Lys Tyr Met Arg Gln Ile Ser Glu Gly
    1490                1495                1500

Val Glu Tyr Ile His Lys Gln Gly Ile Val His Leu Asp Leu Lys
    1505                1510                1515

Pro Glu Asn Ile Met Cys Val Asn Lys Thr Gly Thr Arg Ile Lys
    1520                1525                1530

Leu Ile Asp Phe Gly Leu Ala Arg Arg Leu Glu Asn Ala Gly Ser
    1535                1540                1545

Leu Lys Val Leu Phe Gly Thr Pro Glu Phe Val Ala Pro Glu Val
    1550                1555                1560

Ile Asn Tyr Glu Pro Ile Gly Tyr Ala Thr Asp Met Trp Ser Ile
    1565                1570                1575

Gly Val Ile Cys Tyr Ile Leu Asn Arg Leu Asp Cys Thr Gln Cys
    1580                1585                1590

Leu Gln His Pro Trp Leu Met Lys Asp Thr Lys Asn Met Glu Ala
    1595                1600                1605

Lys Lys Leu Ser Lys Asp Arg Met Lys Lys Tyr Met Ala Arg Arg
    1610                1615                1620

Lys Trp Gln Lys Thr Gly Asn Ala Val Arg Ala Ile Gly Arg Leu
    1625                1630                1635

Ser Ser Met Ala Met Ile Ser Gly Leu Ser Gly Arg Lys Ser Ser
    1640                1645                1650

Thr Gly Ser Pro Thr Ser Pro Leu Asn Ala Glu Lys Leu Glu Ser
    1655                1660                1665

Glu Glu Asp Val Ser Gln Ala Phe Leu Glu Ala Val Ala Glu Glu
    1670                1675                1680

Lys Pro His Val Lys Pro Tyr Phe Ser Lys Thr Ile Arg Asp Leu
    1685                1690                1695

Glu Val Val Glu Gly Ser Ala Ala Arg Phe Asp Cys Lys Ile Glu
    1700                1705                1710

Gly Tyr Pro Asp Pro Glu Val Val Trp Phe Lys Asp Asp Gln Ser
    1715                1720                1725

Ile Arg Glu Ser Arg His Phe Gln Ile Asp Tyr Asp Glu Asp Gly
    1730                1735                1740

Asn Cys Ser Leu Ile Ile Ser Asp Val Cys Gly Asp Asp Asp Ala
    1745                1750                1755

Lys Tyr Thr Cys Lys Ala Val Asn Ser Leu Gly Glu Ala Thr Cys
    1760                1765                1770

Thr Ala Glu Leu Ile Val Glu Thr Met Glu Glu Gly Glu Gly Glu
    1775                1780                1785

Gly Glu Glu Glu Glu Glu
    1790

<210>  43
<211>  2358
<212>  DNA
<213>  Homo sapiens

<220>
<221>  CDS
<222>  (54)..(2045)

<400>  43
cccgggaatc gcacaggacc cggctcccct cgcctaagct gctggggggc gcc atg      56
                                                          Met
                                                          1

ggc cgc tac cgc atc cgc gtg gcc acc ggg gcc tgg ctc ttc tcc ggg      104
Gly Arg Tyr Arg Ile Arg Val Ala Thr Gly Ala Trp Leu Phe Ser Gly
            5                   10                  15

tcg tac aac cgc gtg cag ctt tgg ctg gtc ggg acg cgc ggg gag gcg      152
Ser Tyr Asn Arg Val Gln Leu Trp Leu Val Gly Thr Arg Gly Glu Ala
        20                  25                  30

gag ctg gag ctg cag ctg cgg ccc gcg cgg ggc gag gag gag gag ttt      200
Glu Leu Glu Leu Gln Leu Arg Pro Ala Arg Gly Glu Glu Glu Glu Phe
    35                  40                  45

gat cat gac gtt gca gag gac ttg ggg ctc ctg cag ttc gtg agg ctg      248
Asp His Asp Val Ala Glu Asp Leu Gly Leu Leu Gln Phe Val Arg Leu
50                  55                  60                  65

cgc aag cac cac tgg ctg gtg gac gac gcg tgg ttc tgc gac cgc atc      296
Arg Lys His His Trp Leu Val Asp Asp Ala Trp Phe Cys Asp Arg Ile
                70                  75                  80

acg gtg cag ggc cct gga gcc tgc gcg gag gtg gcc ttc ccg tgc tac      344
Thr Val Gln Gly Pro Gly Ala Cys Ala Glu Val Ala Phe Pro Cys Tyr
            85                  90                  95

cgc tgg gtg cag ggc gag gac atc ctg agc ctg ccc gag ggc acc gcc      392
Arg Trp Val Gln Gly Glu Asp Ile Leu Ser Leu Pro Glu Gly Thr Ala
        100                 105                 110

cgc ctg cca gga gac aat gct ttg gac atg ttc cag aag cat cga gag 440
Arg Leu Pro Gly Asp Asn Ala Leu Asp Met Phe Gln Lys His Arg Glu
    115                 120                 125

aag gaa ctg aaa gac aga cag cag atc tac tgc tgg gcc acc tgg aag 488
Lys Glu Leu Lys Asp Arg Gln Gln Ile Tyr Cys Trp Ala Thr Trp Lys
130                 135                 140                 145

gaa ggg tta ccc ctg acc atc gct gca gac cgt aag gat gat cta cct 536
Glu Gly Leu Pro Leu Thr Ile Ala Ala Asp Arg Lys Asp Asp Leu Pro
                150                 155                 160

cca aat atg aga ttc cat gag gag aag agg ctg gac ttt gaa tgg aca 584
Pro Asn Met Arg Phe His Glu Glu Lys Arg Leu Asp Phe Glu Trp Thr
            165                 170                 175

ctg aag gca ggg gct ctg gag atg gcc ctc aaa cgt gtt tac acc ctc 632
Leu Lys Ala Gly Ala Leu Glu Met Ala Leu Lys Arg Val Tyr Thr Leu
        180                 185                 190

ctg agc tcc tgg aac tgc cta gaa gac ttt gat cag atc ttc tgg ggc 680
Leu Ser Ser Trp Asn Cys Leu Glu Asp Phe Asp Gln Ile Phe Trp Gly
    195                 200                 205

cag aag agt gcc ctg gct gag aag gtt cgc cag tgc tgg cag gat gat 728
Gln Lys Ser Ala Leu Ala Glu Lys Val Arg Gln Cys Trp Gln Asp Asp
210                 215                 220                 225

gag ttg ttc agc tac cag ttc ctc aat ggt gcc aac ccc atg ctg ttg 776
Glu Leu Phe Ser Tyr Gln Phe Leu Asn Gly Ala Asn Pro Met Leu Leu
                230                 235                 240

aga cgc tcg acc tct ctg ccc tcc agg cta gtg ctg ccc tcg ggg atg 824
Arg Arg Ser Thr Ser Leu Pro Ser Arg Leu Val Leu Pro Ser Gly Met
            245                 250                 255

gaa gag ctt cag gct caa ctg gag aaa gaa ctt cag aat ggt tcc ctg 872
Glu Glu Leu Gln Ala Gln Leu Glu Lys Glu Leu Gln Asn Gly Ser Leu
        260                 265                 270

ttt gaa gct gac ttc atc ctt ctg gat gga att cca gcc aac gtg atc 920
Phe Glu Ala Asp Phe Ile Leu Leu Asp Gly Ile Pro Ala Asn Val Ile
    275                 280                 285

cga gga gag aag caa tac ctg gct gcc ccc ctc gtt atg ctg aag atg 968
Arg Gly Glu Lys Gln Tyr Leu Ala Ala Pro Leu Val Met Leu Lys Met
290                 295                 300                 305

gag ccc aat ggg aag ctg cag ccc atg gtc atc cag att cag cct ccc 1016
Glu Pro Asn Gly Lys Leu Gln Pro Met Val Ile Gln Ile Gln Pro Pro
                310                 315                 320

aac ccc agc tct cca acc cca aca ctg ttc ctg ccc tca gac ccc cca 1064
Asn Pro Ser Ser Pro Thr Pro Thr Leu Phe Leu Pro Ser Asp Pro Pro
            325                 330                 335

ctt gcc tgg ctc ctg gca aag tcc tgg gtc cga aat tca gat ttc caa 1112
Leu Ala Trp Leu Leu Ala Lys Ser Trp Val Arg Asn Ser Asp Phe Gln
        340                 345                 350

ctg cac gag atc cag tat cac ttg ctg aac act cac ctg gtg gct gag 1160
Leu His Glu Ile Gln Tyr His Leu Leu Asn Thr His Leu Val Ala Glu

355                 360                 365

gtc atc gct gtc gcc acc atg cgg tgc ctc cca gga ctg cac ccc atc        1208
Val Ile Ala Val Ala Thr Met Arg Cys Leu Pro Gly Leu His Pro Ile
370                 375                 380                 385

ttc aag ttc ctg atc ccc cat atc cgc tac acc atg gaa atc aac acc        1256
Phe Lys Phe Leu Ile Pro His Ile Arg Tyr Thr Met Glu Ile Asn Thr
                390                 395                 400

cgg gcc cgg acc caa ctc atc tca gat gga gga att ttt gat aag gca        1304
Arg Ala Arg Thr Gln Leu Ile Ser Asp Gly Gly Ile Phe Asp Lys Ala
            405                 410                 415

gtg agc aca ggt gga ggg ggc cat gta cag ttg ctc cgt cgg gcg gca        1352
Val Ser Thr Gly Gly Gly Gly His Val Gln Leu Leu Arg Arg Ala Ala
        420                 425                 430

gct cag ctg acc tac tgc tcc ctc tgt cct cct gac gac ctg gct gac        1400
Ala Gln Leu Thr Tyr Cys Ser Leu Cys Pro Pro Asp Asp Leu Ala Asp
    435                 440                 445

cgg ggc ctg ctg gga ctc cca ggt gct ctc tat gcc cat gat gct tta        1448
Arg Gly Leu Leu Gly Leu Pro Gly Ala Leu Tyr Ala His Asp Ala Leu
450                 455                 460                 465

cgg ctc tgg gag atc att gcc agg tat gtg gag ggg atc gtc cac ctc        1496
Arg Leu Trp Glu Ile Ile Ala Arg Tyr Val Glu Gly Ile Val His Leu
                470                 475                 480

ttc tac caa agg gat gac ata gtg aag ggg gac cct gag ctg cag gcc        1544
Phe Tyr Gln Arg Asp Asp Ile Val Lys Gly Asp Pro Glu Leu Gln Ala
            485                 490                 495

tgg tgt cgg gag atc acg gag gtg ggg ctg tgc cag gcc cag gac cga        1592
Trp Cys Arg Glu Ile Thr Glu Val Gly Leu Cys Gln Ala Gln Asp Arg
        500                 505                 510

ggt ttc cct gtc tcc ttc cag tcc cag agt caa ctc tgc cat ttc ctc        1640
Gly Phe Pro Val Ser Phe Gln Ser Gln Ser Gln Leu Cys His Phe Leu
    515                 520                 525

acc atg tgc gtc ttc acg tgc act gcc cag cat gcc gcc atc aac cag        1688
Thr Met Cys Val Phe Thr Cys Thr Ala Gln His Ala Ala Ile Asn Gln
530                 535                 540                 545

ggc cag ctg gac tgg tat gcc tgg gtc cct aat gct cca tgc aca atg        1736
Gly Gln Leu Asp Trp Tyr Ala Trp Val Pro Asn Ala Pro Cys Thr Met
                550                 555                 560

cgg atg ccc cca ccc acc acc aag gaa gat gtg acg atg gcc aca gtg        1784
Arg Met Pro Pro Pro Thr Thr Lys Glu Asp Val Thr Met Ala Thr Val
            565                 570                 575

atg ggg tca cta cct gat gtc cgg cag gcc tgt ctt caa atg gcc atc        1832
Met Gly Ser Leu Pro Asp Val Arg Gln Ala Cys Leu Gln Met Ala Ile
        580                 585                 590

tca tgg cat ctg agt cgc cgc cag cca gac atg gtg cct ctg ggg cac        1880
Ser Trp His Leu Ser Arg Arg Gln Pro Asp Met Val Pro Leu Gly His
    595                 600                 605

cac aaa gaa aaa tat ttc tca ggc ccc aag ccc aaa gct gtg cta aac        1928

His Lys Glu Lys Tyr Phe Ser Gly Pro Lys Pro Lys Ala Val Leu Asn
610                 615                 620                 625

caa ttc cga aca gat ttg gaa aag ctg gaa aag gag att aca gcc cgg      1976
Gln Phe Arg Thr Asp Leu Glu Lys Leu Glu Lys Glu Ile Thr Ala Arg
                630                 635                 640

aat gag caa ctt gac tgg ccc tat gaa tat ctg aag ccc agc tgc ata      2024
Asn Glu Gln Leu Asp Trp Pro Tyr Glu Tyr Leu Lys Pro Ser Cys Ile
            645                 650                 655

gag aac agt gtc acc atc tga gccctagagt gactctacct gcaagatttc         2075
Glu Asn Ser Val Thr Ile
        660

acatcagctt taggactgac atttctatct tgaatttcat gctttcctaa agtctctgct    2135

gctaaggctc tatttcctcc cccagttaaa ccccctacat tagtatccca ctagcccagg    2195

ggagcagtaa actttctctg caaagactag atcctttttt acgctttgca gaccgcatag    2255

tcactgtctc aactactcag ctctcctgct gcagcatgaa ggcagccaca gacaacatgg    2315

aaatgagtgt gactatgttc caataaaact ttatggacac tga                      2358

<210>  44
<211>  663
<212>  PRT
<213>  Homo sapiens

<400>  44

Met Gly Arg Tyr Arg Ile Arg Val Ala Thr Gly Ala Trp Leu Phe Ser
1               5                   10                  15

Gly Ser Tyr Asn Arg Val Gln Leu Trp Leu Val Gly Thr Arg Gly Glu
            20                  25                  30

Ala Glu Leu Glu Leu Gln Leu Arg Pro Ala Arg Gly Glu Glu Glu Glu
        35                  40                  45

Phe Asp His Asp Val Ala Glu Asp Leu Gly Leu Leu Gln Phe Val Arg
    50                  55                  60

Leu Arg Lys His His Trp Leu Val Asp Asp Ala Trp Phe Cys Asp Arg
65                  70                  75                  80

Ile Thr Val Gln Gly Pro Gly Ala Cys Ala Glu Val Ala Phe Pro Cys
                85                  90                  95

Tyr Arg Trp Val Gln Gly Glu Asp Ile Leu Ser Leu Pro Glu Gly Thr
            100                 105                 110

Ala Arg Leu Pro Gly Asp Asn Ala Leu Asp Met Phe Gln Lys His Arg
        115                 120                 125

Glu Lys Glu Leu Lys Asp Arg Gln Gln Ile Tyr Cys Trp Ala Thr Trp
    130                 135                 140

Lys Glu Gly Leu Pro Leu Thr Ile Ala Ala Asp Arg Lys Asp Asp Leu
145                 150                 155                 160

Pro Pro Asn Met Arg Phe His Glu Glu Lys Arg Leu Asp Phe Glu Trp
                165                 170                 175

Thr Leu Lys Ala Gly Ala Leu Glu Met Ala Leu Lys Arg Val Tyr Thr
            180                 185                 190

Leu Leu Ser Ser Trp Asn Cys Leu Glu Asp Phe Asp Gln Ile Phe Trp
        195                 200                 205

Gly Gln Lys Ser Ala Leu Ala Glu Lys Val Arg Gln Cys Trp Gln Asp
    210                 215                 220

Asp Glu Leu Phe Ser Tyr Gln Phe Leu Asn Gly Ala Asn Pro Met Leu
225                 230                 235                 240

Leu Arg Arg Ser Thr Ser Leu Pro Ser Arg Leu Val Leu Pro Ser Gly
                245                 250                 255

Met Glu Glu Leu Gln Ala Gln Leu Glu Lys Glu Leu Gln Asn Gly Ser
            260                 265                 270

Leu Phe Glu Ala Asp Phe Ile Leu Leu Asp Gly Ile Pro Ala Asn Val
        275                 280                 285

Ile Arg Gly Glu Lys Gln Tyr Leu Ala Ala Pro Leu Val Met Leu Lys
    290                 295                 300

Met Glu Pro Asn Gly Lys Leu Gln Pro Met Val Ile Gln Ile Gln Pro
305                 310                 315                 320

Pro Asn Pro Ser Ser Pro Thr Pro Thr Leu Phe Leu Pro Ser Asp Pro
                325                 330                 335

Pro Leu Ala Trp Leu Leu Ala Lys Ser Trp Val Arg Asn Ser Asp Phe
            340                 345                 350

Gln Leu His Glu Ile Gln Tyr His Leu Leu Asn Thr His Leu Val Ala
        355                 360                 365

Glu Val Ile Ala Val Ala Thr Met Arg Cys Leu Pro Gly Leu His Pro

370                 375                 380

Ile Phe Lys Phe Leu Ile Pro His Ile Arg Tyr Thr Met Glu Ile Asn
385                 390                 395                 400

Thr Arg Ala Arg Thr Gln Leu Ile Ser Asp Gly Gly Ile Phe Asp Lys
                405                 410                 415

Ala Val Ser Thr Gly Gly Gly Gly His Val Gln Leu Leu Arg Arg Ala
            420                 425                 430

Ala Ala Gln Leu Thr Tyr Cys Ser Leu Cys Pro Pro Asp Asp Leu Ala
        435                 440                 445

Asp Arg Gly Leu Leu Gly Leu Pro Gly Ala Leu Tyr Ala His Asp Ala
    450                 455                 460

Leu Arg Leu Trp Glu Ile Ile Ala Arg Tyr Val Glu Gly Ile Val His
465                 470                 475                 480

Leu Phe Tyr Gln Arg Asp Asp Ile Val Lys Gly Asp Pro Glu Leu Gln
                485                 490                 495

Ala Trp Cys Arg Glu Ile Thr Glu Val Gly Leu Cys Gln Ala Gln Asp
            500                 505                 510

Arg Gly Phe Pro Val Ser Phe Gln Ser Gln Ser Gln Leu Cys His Phe
        515                 520                 525

Leu Thr Met Cys Val Phe Thr Cys Thr Ala Gln His Ala Ala Ile Asn
    530                 535                 540

Gln Gly Gln Leu Asp Trp Tyr Ala Trp Val Pro Asn Ala Pro Cys Thr
545                 550                 555                 560

Met Arg Met Pro Pro Pro Thr Thr Lys Glu Asp Val Thr Met Ala Thr
                565                 570                 575

Val Met Gly Ser Leu Pro Asp Val Arg Gln Ala Cys Leu Gln Met Ala
            580                 585                 590

Ile Ser Trp His Leu Ser Arg Arg Gln Pro Asp Met Val Pro Leu Gly
        595                 600                 605

His His Lys Glu Lys Tyr Phe Ser Gly Pro Lys Pro Lys Ala Val Leu
    610                 615                 620

Asn Gln Phe Arg Thr Asp Leu Glu Lys Leu Glu Lys Glu Ile Thr Ala
625                 630                 635                 640

Arg Asn Glu Gln Leu Asp Trp Pro Tyr Glu Tyr Leu Lys Pro Ser Cys
                645                 650                 655

Ile Glu Asn Ser Val Thr Ile
            660

<210>  45
<211>  1556
<212>  DNA
<213>  Homo sapiens

<220>
<221>  CDS
<222>  (209)..(1249)

<400>  45
acttgtttca ctttgttttt ggacatagct gagccatgta cttcaaacag aaggcagcca      60

attactaact tctggttgct aggtgtggct tcctttaaaa tcctataaaa tcagaagccc     120

aagtctccac tgccagtgtg aaatcttcag agaagaattt ctctttagtt ctttgcaaga     180

aggtagagat aaagacactt tttcaaaa atg gca atg gta tca gaa ttc ctc        232
                               Met Ala Met Val Ser Glu Phe Leu
                               1               5

aag cag gcc tgg ttt att gaa aat gaa gag cag gaa tat gtt caa act       280
Lys Gln Ala Trp Phe Ile Glu Asn Glu Glu Gln Glu Tyr Val Gln Thr
    10                  15                  20

gtg aag tca tcc aaa ggt ggt ccc gga tca gcg gtg agc ccc tat cct       328
Val Lys Ser Ser Lys Gly Gly Pro Gly Ser Ala Val Ser Pro Tyr Pro
25                  30                  35                  40

acc ttc aat cca tcc tcg gat gtc gct gcc ttg cat aag gcc ata atg       376
Thr Phe Asn Pro Ser Ser Asp Val Ala Ala Leu His Lys Ala Ile Met
                45                  50                  55

gtt aaa ggt gtg gat gaa gca acc atc att gac att cta act aag cga       424
Val Lys Gly Val Asp Glu Ala Thr Ile Ile Asp Ile Leu Thr Lys Arg
            60                  65                  70

aac aat gca cag cgt caa cag atc aaa gca gca tat ctc cag gaa aca       472
Asn Asn Ala Gln Arg Gln Gln Ile Lys Ala Ala Tyr Leu Gln Glu Thr
        75                  80                  85

gga aag ccc ctg gat gaa aca ctg aag aaa gcc ctt aca ggt cac ctt       520
Gly Lys Pro Leu Asp Glu Thr Leu Lys Lys Ala Leu Thr Gly His Leu
    90                  95                  100

gag gag gtt gtt tta gct ctg cta aaa act cca gcg caa ttt gat gct       568
Glu Glu Val Val Leu Ala Leu Leu Lys Thr Pro Ala Gln Phe Asp Ala
105                 110                 115                 120

gat gaa ctt cgt gct gcc atg aag ggc ctt gga act gat gaa gat act       616
Asp Glu Leu Arg Ala Ala Met Lys Gly Leu Gly Thr Asp Glu Asp Thr

125                 130                 135

cta att gag att ttg gca tca aga act aac aaa gaa atc aga gac att       664
Leu Ile Glu Ile Leu Ala Ser Arg Thr Asn Lys Glu Ile Arg Asp Ile
        140                 145                 150

aac agg gtc tac aga gag gaa ctg aag aga gat ctg gcc aaa gac ata       712
Asn Arg Val Tyr Arg Glu Glu Leu Lys Arg Asp Leu Ala Lys Asp Ile
    155                 160                 165

acc tca gac aca tct gga gat ttt cgg aac gct ttg ctt tct ctt gct       760
Thr Ser Asp Thr Ser Gly Asp Phe Arg Asn Ala Leu Leu Ser Leu Ala
    170                 175                 180

aag ggt gac cga tct gag gac ttt ggt gtg aat gaa gac ttg gct gat       808
Lys Gly Asp Arg Ser Glu Asp Phe Gly Val Asn Glu Asp Leu Ala Asp
185                 190                 195                 200

tca gat gcc agg gcc ttg tat gaa gca gga gaa agg aga aag ggg aca       856
Ser Asp Ala Arg Ala Leu Tyr Glu Ala Gly Glu Arg Arg Lys Gly Thr
                205                 210                 215

gac gta aac gtg ttc aat acc atc ctt acc acc aga agc tat cca caa       904
Asp Val Asn Val Phe Asn Thr Ile Leu Thr Thr Arg Ser Tyr Pro Gln
            220                 225                 230

ctt cgc aga gtg ttt cag aaa tac acc aag tac agt aag cat gac atg       952
Leu Arg Arg Val Phe Gln Lys Tyr Thr Lys Tyr Ser Lys His Asp Met
        235                 240                 245

aac aaa gtt ctg gac ctg gag ttg aaa ggt gac att gag aaa tgc ctc      1000
Asn Lys Val Leu Asp Leu Glu Leu Lys Gly Asp Ile Glu Lys Cys Leu
    250                 255                 260

aca gct atc gtg aag tgc gcc aca agc aaa cca gct ttc ttt gca gag      1048
Thr Ala Ile Val Lys Cys Ala Thr Ser Lys Pro Ala Phe Phe Ala Glu
265                 270                 275                 280

aag ctt cat caa gcc atg aaa ggt gtt gga act cgc cat aag gca ttg      1096
Lys Leu His Gln Ala Met Lys Gly Val Gly Thr Arg His Lys Ala Leu
                285                 290                 295

atc agg att atg gtt tcc cgt tct gaa att gac atg aat gat atc aaa      1144
Ile Arg Ile Met Val Ser Arg Ser Glu Ile Asp Met Asn Asp Ile Lys
            300                 305                 310

gca ttc tat cag aag atg tat ggt atc tcc ctt tgc caa gcc atc ctg      1192
Ala Phe Tyr Gln Lys Met Tyr Gly Ile Ser Leu Cys Gln Ala Ile Leu
        315                 320                 325

gat gaa acc aaa gga gat tat gag aaa atc ctg gtg gct ctt tgt gga      1240
Asp Glu Thr Lys Gly Asp Tyr Glu Lys Ile Leu Val Ala Leu Cys Gly
    330                 335                 340

gga aac taa acattccctt gatggtctca agctatgatc agaagacttt              1289
Gly Asn
345

aattatatat tttcatccta taagcttaaa taggaaagtt tcttcaacag gattacagtg    1349

tagctaccta catgctgaaa aatatagcct ttaaatcatt tttatattat aactctgtat    1409

aatagagata agtccatttt ttaaaaatgt tttccccaaa ccataaaacc ctatacaagt    1469

tgttctagta acaatacatg agaaagatgt ctatgtagct gaaaataaaa tgacgtcaca 1529

agacaaaaaa aaaaaaaaaa aaaaaaa 1556

<210> 46
<211> 346
<212> PRT
<213> Homo sapiens

<400> 46

Met Ala Met Val Ser Glu Phe Leu Lys Gln Ala Trp Phe Ile Glu Asn
1               5                   10                  15

Glu Glu Gln Glu Tyr Val Gln Thr Val Lys Ser Ser Lys Gly Gly Pro
            20                  25                  30

Gly Ser Ala Val Ser Pro Tyr Pro Thr Phe Asn Pro Ser Ser Asp Val
        35                  40                  45

Ala Ala Leu His Lys Ala Ile Met Val Lys Gly Val Asp Glu Ala Thr
    50                  55                  60

Ile Ile Asp Ile Leu Thr Lys Arg Asn Asn Ala Gln Arg Gln Gln Ile
65                  70                  75                  80

Lys Ala Ala Tyr Leu Gln Glu Thr Gly Lys Pro Leu Asp Glu Thr Leu
                85                  90                  95

Lys Lys Ala Leu Thr Gly His Leu Glu Glu Val Val Leu Ala Leu Leu
            100                 105                 110

Lys Thr Pro Ala Gln Phe Asp Ala Asp Glu Leu Arg Ala Ala Met Lys
        115                 120                 125

Gly Leu Gly Thr Asp Glu Asp Thr Leu Ile Glu Ile Leu Ala Ser Arg
    130                 135                 140

Thr Asn Lys Glu Ile Arg Asp Ile Asn Arg Val Tyr Arg Glu Glu Leu
145                 150                 155                 160

Lys Arg Asp Leu Ala Lys Asp Ile Thr Ser Asp Thr Ser Gly Asp Phe
                165                 170                 175

Arg Asn Ala Leu Leu Ser Leu Ala Lys Gly Asp Arg Ser Glu Asp Phe
            180                 185                 190

Gly Val Asn Glu Asp Leu Ala Asp Ser Asp Ala Arg Ala Leu Tyr Glu
        195                 200                 205

Ala Gly Glu Arg Arg Lys Gly Thr Asp Val Asn Val Phe Asn Thr Ile
    210                 215                 220

Leu Thr Thr Arg Ser Tyr Pro Gln Leu Arg Arg Val Phe Gln Lys Tyr
225                 230                 235                 240

Thr Lys Tyr Ser Lys His Asp Met Asn Lys Val Leu Asp Leu Glu Leu
                245                 250                 255

Lys Gly Asp Ile Glu Lys Cys Leu Thr Ala Ile Val Lys Cys Ala Thr
            260                 265                 270

Ser Lys Pro Ala Phe Phe Ala Glu Lys Leu His Gln Ala Met Lys Gly
        275                 280                 285

Val Gly Thr Arg His Lys Ala Leu Ile Arg Ile Met Val Ser Arg Ser
    290                 295                 300

Glu Ile Asp Met Asn Asp Ile Lys Ala Phe Tyr Gln Lys Met Tyr Gly
305                 310                 315                 320

Ile Ser Leu Cys Gln Ala Ile Leu Asp Glu Thr Lys Gly Asp Tyr Glu
                325                 330                 335

Lys Ile Leu Val Ala Leu Cys Gly Gly Asn
            340                 345

<210>  47
<211>  2158
<212>  DNA
<213>  Homo sapiens

<220>
<221>  CDS
<222>  (228)..(1193)

<400>  47
gccccaggtg cgcttcccct agagagggat tttccggtct cgtgggcaga ggaacaacca      60

ggaacttggg ctcagtctcc accccacagt ggggcggatc cgtcccggat aagacccgct     120

gtctggccct gagtagggtg tgacctccgc agccgcagag gaggagcgca gcccggcctc     180

gaagaacttc tgcttgggtg gctgaactct gatcttgacc tagagtc atg gcc atg       236
                                                    Met Ala Met
                                                    1

gca acc aaa gga ggt act gtc aaa gct gct tca gga ttc aat gcc atg       284
Ala Thr Lys Gly Gly Thr Val Lys Ala Ala Ser Gly Phe Asn Ala Met
    5                   10                  15

gaa gat gcc cag acc ctg agg aag gcc atg aaa ggg ctc ggc acc gat 332
Glu Asp Ala Gln Thr Leu Arg Lys Ala Met Lys Gly Leu Gly Thr Asp
20                  25                  30                  35

gaa gac gcc att att agc gtc ctt gcc tac cgc aac acc gcc cag cgc 380
Glu Asp Ala Ile Ile Ser Val Leu Ala Tyr Arg Asn Thr Ala Gln Arg
                40                  45                  50

cag gag atc agg aca gcc tac aag agc acc atc ggc agg gac ttg ata 428
Gln Glu Ile Arg Thr Ala Tyr Lys Ser Thr Ile Gly Arg Asp Leu Ile
            55                  60                  65

gac gac ctg aag tca gaa ctg agt ggc aac ttc gag cag gtg att gtg 476
Asp Asp Leu Lys Ser Glu Leu Ser Gly Asn Phe Glu Gln Val Ile Val
        70                  75                  80

ggg atg atg acg ccc acg gtg ctg tat gac gtg caa gag ctg cga agg 524
Gly Met Met Thr Pro Thr Val Leu Tyr Asp Val Gln Glu Leu Arg Arg
    85                  90                  95

gcc atg aag gga gcc ggc act gat gag ggc tgc cta att gag atc ctg 572
Ala Met Lys Gly Ala Gly Thr Asp Glu Gly Cys Leu Ile Glu Ile Leu
100                 105                 110                 115

gcc tcc cgg acc cct gag gag atc cgg cgc ata agc caa acc tac cag 620
Ala Ser Arg Thr Pro Glu Glu Ile Arg Arg Ile Ser Gln Thr Tyr Gln
                120                 125                 130

cag caa tat gga cgg agc ctt gaa gat gac att cgc tct gac aca tcg 668
Gln Gln Tyr Gly Arg Ser Leu Glu Asp Asp Ile Arg Ser Asp Thr Ser
            135                 140                 145

ttc atg ttc cag cga gtg ctg gtg tct ctg tca gct ggt ggg agg gat 716
Phe Met Phe Gln Arg Val Leu Val Ser Leu Ser Ala Gly Gly Arg Asp
        150                 155                 160

gaa gga aat tat ctg gac gat gct ctc gtg aga cag gat gcc cag gac 764
Glu Gly Asn Tyr Leu Asp Asp Ala Leu Val Arg Gln Asp Ala Gln Asp
    165                 170                 175

ctg tat gag gct gga gag aag aaa tgg ggg aca gat gag gtg aaa ttt 812
Leu Tyr Glu Ala Gly Glu Lys Lys Trp Gly Thr Asp Glu Val Lys Phe
180                 185                 190                 195

cta act gtt ctc tgt tcc cgg aac cga aat cac ctg ttg cat gtg ttt 860
Leu Thr Val Leu Cys Ser Arg Asn Arg Asn His Leu Leu His Val Phe
                200                 205                 210

gat gaa tac aaa agg ata tca cag aag gat att gaa cag agt att aaa 908
Asp Glu Tyr Lys Arg Ile Ser Gln Lys Asp Ile Glu Gln Ser Ile Lys
            215                 220                 225

tct gaa aca tct ggt agc ttt gaa gat gct ctg ctg gct ata gta aag 956
Ser Glu Thr Ser Gly Ser Phe Glu Asp Ala Leu Leu Ala Ile Val Lys
        230                 235                 240

tgc atg agg aac aaa tct gca tat ttt gct gaa aag ctc tat aaa tcg 1004
Cys Met Arg Asn Lys Ser Ala Tyr Phe Ala Glu Lys Leu Tyr Lys Ser
    245                 250                 255

atg aag ggc ttg ggc acc gat gat aac acc ctc atc aga gtg atg gtt 1052
Met Lys Gly Leu Gly Thr Asp Asp Asn Thr Leu Ile Arg Val Met Val
260                 265                 270                 275

tct cga gca gaa att gac atg ttg gat atc cgg gca cac ttc aag aga     1100
Ser Arg Ala Glu Ile Asp Met Leu Asp Ile Arg Ala His Phe Lys Arg
                280                 285                 290

ctc tat gga aag tct ctg tac tcg ttc atc aag ggt gac aca tct gga     1148
Leu Tyr Gly Lys Ser Leu Tyr Ser Phe Ile Lys Gly Asp Thr Ser Gly
            295                 300                 305

gac tac agg aaa gta ctg ctt gtt ctc tgt gga gga gat gat taa         1193
Asp Tyr Arg Lys Val Leu Leu Val Leu Cys Gly Gly Asp Asp
        310                 315                 320

aataaaaatc ccagaaggac aggaggattc tcaacacttt gaattttttt aacttcattt   1253

ttctacactg ctattatcat tatctcagaa tgcttatttc caattaaaac gcctacagct   1313

gcctcctaga atatagactg tctgtattat tattcaccta taattagtca ttatgatgct   1373

ttaaagctgt acttgcattt caaagcttat aagatataaa tggagatttt aaagtagaaa   1433

taaatatgta ttccatgttt ttaaaagatt actttctact ttgtgtttca cagacattga   1493

atatattaaa ttattccata ttttcttttc agtgaaaaat tttttaaatg gaagactgtt   1553

ctaaaatcac ttttttccct aatccaattt ttagagtggc tagtagtttc ttcatttgaa   1613

attgtaagca tccggtcagt aagaatgccc atccagtttt ctatatttca tagtcaaagc   1673

cttgaaagca tctacaaatc tcttttttta ggttttgtcc atagcatcag ttgatcctta   1733

ctaagttttt catgggagac ttccttcatc acatcttatg ttgaaatcac tttctgtagt   1793

caaagtatac caaaaccaat ttatctgaac taaattctaa agtatggtta tacaaaccat   1853

atacatctgg ttaccaaaca taaatgctga acattccata ttattatagt taatgtctta   1913

atccagcttg caagtgaatg gaaaaaaaaa taagcttcaa actaggtatt ctgggaatga   1973

tgtaatgctc tgaatttagt atgatataaa gaaaactttt ttgtgctaaa aatacttttt   2033

aaaatcaatt ttgttgattg tagtaatttc tatttgcact gtgcctttca actccagaaa   2093

cattctgaag atgtacttgg atttaattaa aaagttcact ttgtaagaaa aaaaaaaaaa   2153

aaaaa                                                               2158

<210>  48
<211>  321
<212>  PRT
<213>  Homo sapiens

<400>  48

Met Ala Met Ala Thr Lys Gly Gly Thr Val Lys Ala Ala Ser Gly Phe
1               5                   10                  15

Asn Ala Met Glu Asp Ala Gln Thr Leu Arg Lys Ala Met Lys Gly Leu
            20                  25                  30

Gly Thr Asp Glu Asp Ala Ile Ile Ser Val Leu Ala Tyr Arg Asn Thr
        35                  40                  45

Ala Gln Arg Gln Glu Ile Arg Thr Ala Tyr Lys Ser Thr Ile Gly Arg
    50                  55                  60

Asp Leu Ile Asp Asp Leu Lys Ser Glu Leu Ser Gly Asn Phe Glu Gln
65                  70                  75                  80

Val Ile Val Gly Met Met Thr Pro Thr Val Leu Tyr Asp Val Gln Glu
                85                  90                  95

Leu Arg Arg Ala Met Lys Gly Ala Gly Thr Asp Glu Gly Cys Leu Ile
            100                 105                 110

Glu Ile Leu Ala Ser Arg Thr Pro Glu Glu Ile Arg Arg Ile Ser Gln
        115                 120                 125

Thr Tyr Gln Gln Gln Tyr Gly Arg Ser Leu Glu Asp Asp Ile Arg Ser
    130                 135                 140

Asp Thr Ser Phe Met Phe Gln Arg Val Leu Val Ser Leu Ser Ala Gly
145                 150                 155                 160

Gly Arg Asp Glu Gly Asn Tyr Leu Asp Asp Ala Leu Val Arg Gln Asp
                165                 170                 175

Ala Gln Asp Leu Tyr Glu Ala Gly Glu Lys Lys Trp Gly Thr Asp Glu
            180                 185                 190

Val Lys Phe Leu Thr Val Leu Cys Ser Arg Asn Arg Asn His Leu Leu
        195                 200                 205

His Val Phe Asp Glu Tyr Lys Arg Ile Ser Gln Lys Asp Ile Glu Gln
    210                 215                 220

Ser Ile Lys Ser Glu Thr Ser Gly Ser Phe Glu Asp Ala Leu Leu Ala
225                 230                 235                 240

Ile Val Lys Cys Met Arg Asn Lys Ser Ala Tyr Phe Ala Glu Lys Leu
                245                 250                 255

Tyr Lys Ser Met Lys Gly Leu Gly Thr Asp Asp Asn Thr Leu Ile Arg
            260                 265                 270

Val Met Val Ser Arg Ala Glu Ile Asp Met Leu Asp Ile Arg Ala His
        275                 280                 285

Phe Lys Arg Leu Tyr Gly Lys Ser Leu Tyr Ser Phe Ile Lys Gly Asp
    290                 295                 300

Thr Ser Gly Asp Tyr Arg Lys Val Leu Leu Val Leu Cys Gly Gly Asp
305                 310                 315                 320

Asp

<210>  49
<211>  3782
<212>  DNA
<213>  Homo sapiens

<220>
<221>  CDS
<222>  (174)..(1748)

<400>  49
gtctttacgt agacgtcggg ggcgcagccg cgctgtgcgc ggtgactggc ggctgcactg      60

gcggcagctg gaggtgtaat agtgcgggta gtgggtttgg agaagttccg aggcggcggt     120

ggcgccggtc aggacaagga tagcggaacc gggccctggg cttgtcgctc acc atg        176
                                                           Met
                                                           1

ccg aca gta gag gag ctt tac cgc aat tat ggc atc ctg gcc gat gcc       224
Pro Thr Val Glu Glu Leu Tyr Arg Asn Tyr Gly Ile Leu Ala Asp Ala
            5                   10                  15

acg gag caa gtg ggc cag cat aaa gat gcc tat caa gtg ata ttg gat       272
Thr Glu Gln Val Gly Gln His Lys Asp Ala Tyr Gln Val Ile Leu Asp
        20                  25                  30

ggt gtg aaa ggt ggt act aag gaa aag cga tta gca gct caa ttt att       320
Gly Val Lys Gly Gly Thr Lys Glu Lys Arg Leu Ala Ala Gln Phe Ile
    35                  40                  45

ccg aaa ttc ttt aag cat ttt cca gaa ttg gct gat tct gct atc aat       368
Pro Lys Phe Phe Lys His Phe Pro Glu Leu Ala Asp Ser Ala Ile Asn
50                  55                  60                  65

gca cag tta gac ctc tgt gag gat gaa gat gta tct att cga cgt caa       416
Ala Gln Leu Asp Leu Cys Glu Asp Glu Asp Val Ser Ile Arg Arg Gln
                70                  75                  80

gca att aaa gaa ctg cct caa ttt gcc act gga gaa aat ctt cct cga       464
Ala Ile Lys Glu Leu Pro Gln Phe Ala Thr Gly Glu Asn Leu Pro Arg
            85                  90                  95

gtg gca gat ata cta acg caa ctt ttg cag aca gat gac tct gca gaa       512
Val Ala Asp Ile Leu Thr Gln Leu Leu Gln Thr Asp Asp Ser Ala Glu
        100                 105                 110

ttt aac cta gtg aac aat gcc cta tta agt ata ttt aaa atg gat gca       560
Phe Asn Leu Val Asn Asn Ala Leu Leu Ser Ile Phe Lys Met Asp Ala
    115                 120                 125

aaa ggg act tta ggt ggg ttg ttc agc caa ata ctt caa gga gag gac     608
Lys Gly Thr Leu Gly Gly Leu Phe Ser Gln Ile Leu Gln Gly Glu Asp
130                 135                 140                 145

att gtt aga gaa cga gca att aaa ttc ctt tct aca aaa ctt aag act     656
Ile Val Arg Glu Arg Ala Ile Lys Phe Leu Ser Thr Lys Leu Lys Thr
                150                 155                 160

tta cca gat gaa gtc tta aca aag gaa gtg gaa gag ctt ata cta act     704
Leu Pro Asp Glu Val Leu Thr Lys Glu Val Glu Glu Leu Ile Leu Thr
            165                 170                 175

gaa tcc aaa aag gtc cta gaa gat gtg act ggt gaa gaa ttt gtt cta     752
Glu Ser Lys Lys Val Leu Glu Asp Val Thr Gly Glu Glu Phe Val Leu
        180                 185                 190

ttt atg aag ata ctg tct ggg tta aaa agc tta cag aca gtg agt gga     800
Phe Met Lys Ile Leu Ser Gly Leu Lys Ser Leu Gln Thr Val Ser Gly
    195                 200                 205

aga cag caa ctt gta gag ttg gtg gct gaa cag gcc gac cta gaa cag     848
Arg Gln Gln Leu Val Glu Leu Val Ala Glu Gln Ala Asp Leu Glu Gln
210                 215                 220                 225

acc ttc aat ccc tcg gat cct gac tgt gtg gac agg ctc tta cag tgc     896
Thr Phe Asn Pro Ser Asp Pro Asp Cys Val Asp Arg Leu Leu Gln Cys
                230                 235                 240

act cgg cag gca gta ccc ctc ttc tct aaa aat gtc cat tcc aca agg     944
Thr Arg Gln Ala Val Pro Leu Phe Ser Lys Asn Val His Ser Thr Arg
            245                 250                 255

ttt gtg aca tat ttc tgt gag cag gtt ctc cct aac ctc ggt acc ttg     992
Phe Val Thr Tyr Phe Cys Glu Gln Val Leu Pro Asn Leu Gly Thr Leu
        260                 265                 270

act acc cca gtg gaa ggt ctt gat ata cag ttg gag gta ttg aaa ttg     1040
Thr Thr Pro Val Glu Gly Leu Asp Ile Gln Leu Glu Val Leu Lys Leu
    275                 280                 285

ttg gcg gag atg agt tca ttt tgt ggt gac atg gaa aaa cta gaa aca     1088
Leu Ala Glu Met Ser Ser Phe Cys Gly Asp Met Glu Lys Leu Glu Thr
290                 295                 300                 305

aat tta agg aaa cta ttt gat aag tta ttg gaa tac atg ccc ctc cct     1136
Asn Leu Arg Lys Leu Phe Asp Lys Leu Leu Glu Tyr Met Pro Leu Pro
                310                 315                 320

cca gaa gag gca gaa aat gga gag aat gct ggt aat gaa gaa ccc aag     1184
Pro Glu Glu Ala Glu Asn Gly Glu Asn Ala Gly Asn Glu Glu Pro Lys
            325                 330                 335

cta cag ttc agt tat gtg gaa tgt ttg ttg tac agt ttt cac cag ttg     1232
Leu Gln Phe Ser Tyr Val Glu Cys Leu Leu Tyr Ser Phe His Gln Leu
        340                 345                 350

ggc cga aaa ctt cca gat ttc tta aca gcc aaa ctg aat gca gaa aag     1280
Gly Arg Lys Leu Pro Asp Phe Leu Thr Ala Lys Leu Asn Ala Glu Lys
    355                 360                 365

ctc aaa gat ttc aaa atc agg ctg cag tac ttt gca cgg ggc ctg caa     1328
Leu Lys Asp Phe Lys Ile Arg Leu Gln Tyr Phe Ala Arg Gly Leu Gln

370                 375                 380                 385

gtt tat atc aga caa ctt cgc tta gct ctc cag ggt aaa acg ggt gag     1376
Val Tyr Ile Arg Gln Leu Arg Leu Ala Leu Gln Gly Lys Thr Gly Glu
                390                 395                 400

gcc tta aaa aca gaa gag aac aag att aaa gtc gtt gca ttg aaa ata     1424
Ala Leu Lys Thr Glu Glu Asn Lys Ile Lys Val Val Ala Leu Lys Ile
            405                 410                 415

aca aac aat atc aat gtt tta atc aag gat ctc ttc cac att cct cct     1472
Thr Asn Asn Ile Asn Val Leu Ile Lys Asp Leu Phe His Ile Pro Pro
        420                 425                 430

tct tat aag agc aca gta aca cta tcc tgg aaa cct gta caa aag gtt     1520
Ser Tyr Lys Ser Thr Val Thr Leu Ser Trp Lys Pro Val Gln Lys Val
    435                 440                 445

gag att ggg caa aag aga gcc agt gaa gat aca act tca ggt tca cca     1568
Glu Ile Gly Gln Lys Arg Ala Ser Glu Asp Thr Thr Ser Gly Ser Pro
450                 455                 460                 465

ccc aag aaa tct tca gca gga cca aaa aga gat gcc agg cag att tat     1616
Pro Lys Lys Ser Ser Ala Gly Pro Lys Arg Asp Ala Arg Gln Ile Tyr
                470                 475                 480

aac cct ccc agt ggg aaa tat agc agc aat ttg ggc aac ttt aat tat     1664
Asn Pro Pro Ser Gly Lys Tyr Ser Ser Asn Leu Gly Asn Phe Asn Tyr
            485                 490                 495

gag cag aga gga gcc ttc agg gga agt aga ggt ggc cga ggt tgg ggc     1712
Glu Gln Arg Gly Ala Phe Arg Gly Ser Arg Gly Gly Arg Gly Trp Gly
        500                 505                 510

aca cga gga aat cgt agt cgg gga aga ctc tac tga ataagacatc          1758
Thr Arg Gly Asn Arg Ser Arg Gly Arg Leu Tyr
    515                 520

agcattcttc agcattgtca tgagcttaat atacttaaat tctactactc attggattgc   1818

cggggatgtc cctttaaaca gactgctgcc ttcagctaaa aacttaatgt tctttatacc   1878

tttgtatgta tgacctactt ttgtaacaga ccatggttgt gtccaaggta aaaccacagt   1938

gatatttttg gatgctttgt ctgcaatctt gacttgtttt tgcagtatca ttattcagac   1998

ttcaaattgt gaatctttta aacatcttga taatttgttg ttgagagctg ttcattctaa   2058

aatgtaatga aattcagtct agttctgctg ataaagatca tcagttttga aaggttactg   2118

attttcctct tccctcttag ttttttaccc aatatatgga gaagagtaat ggtcaatctt   2178

aacattttgt tttaattgtt taataaagct gctgggcagt ggtgcagcat tcctacctag   2238

tgtcataaaa gcaaaatact tacatagctt tcttaaaata taggaatgac attacatttt   2298

taggagaaag taagttgctt tgcaccgcct acttaattct tttccatata ttgtgataca   2358

aacttttgaa tatggaatct tactatttga atagaaatgt gtatgtataa tatacataca   2418

tacataagca tatatgtgtg tgtgtgtgtg tatatatata tatatgcatg ctgtgaaact   2478

tgactacaca acataaatca ctttttaaat tccaggaacg ggtagtctga cacggtgatt   2538

atccttttga ggctgaatcc gttattaact tgttatttag gtttttactc ccagtagcaa 2598

gggattctaa gttagttgca cttacatgat tattgttatt taaaactaag aataaaggct 2658

gcattttcaa agataaattg gaattgctgt tggtgaaata acaaccaaaa tactgaatct 2718

gatgtacata caggtttcta caggaagaga tggtataatt tacaatttgg agatttaata 2778

accagggcta cccagaaaaa gtgacttgat aacatggtac caataagtaa gggatgctct 2838

ctcggtttgc ttttgccact ttcaagattt taacttctca ggttattaat caaaattatt 2898

gtataagtta gccaatagaa tttttaggtt aaaacaacag atggggggtt tgtggagtgt 2958

ttaatgtcat gggcattttt agtagcatag accctttgtt ctgcatttga atgtttcgta 3018

tatttttgtt tcacagttaa tcttccctcc ccaagtttgc tattcaaatc aactgcctga 3078

atgacatttc tagtagtctg atgtattttt ctgaggaata gtttgtgatt ccaatgcagg 3138

tgtcttcatt accattacct ctacactgca gaagaagcaa aactccttta ttagaattac 3198

tgcacatgtg tatggggaaa atagttctga aaggctagaa tgatacaagt gagcaaaagt 3258

tggtcagctt ggctatggag tggtggcaat aatctctaaa cattccaaaa gaccatgagc 3318

tgaacctaaa ctcccttgga atctgaacaa aggaatataa aattgccatt tgaaaactga 3378

ccagctaatc tggacctcag agatagatca gccagtggcc caaagccatt tcaagtacag 3438

aaattataga gactacagct aaataaattt gaacattaaa tataatttta ccactttttg 3498

tctttataag catatttgta aactcagaac tgagcagaag tgactttact ttctcaagtt 3558

tgatactgag ttgactgttc ccttatccct cacccttccc cttccctttc ctaaggcaat 3618

agtgcacaac ttaggttatt tttgcttccg aatttgaatg aaaaacttaa tgccatggat 3678

ttttttcttt tgcaagacac ctgtttatca tcttgtttaa atgtaaatgt ccccttatgc 3738

ttttgaaata aatttccttt tgtaattttg aaaaaaaaaa aaaa 3782

<210> 50
<211> 524
<212> PRT
<213> Homo sapiens

<400> 50

Met Pro Thr Val Glu Glu Leu Tyr Arg Asn Tyr Gly Ile Leu Ala Asp
1               5                   10                  15

Ala Thr Glu Gln Val Gly Gln His Lys Asp Ala Tyr Gln Val Ile Leu
            20                  25                  30

Asp Gly Val Lys Gly Gly Thr Lys Glu Lys Arg Leu Ala Ala Gln Phe
        35                  40                  45

Ile Pro Lys Phe Phe Lys His Phe Pro Glu Leu Ala Asp Ser Ala Ile
    50                  55                  60

Asn Ala Gln Leu Asp Leu Cys Glu Asp Glu Asp Val Ser Ile Arg Arg
65                  70                  75                  80

Gln Ala Ile Lys Glu Leu Pro Gln Phe Ala Thr Gly Glu Asn Leu Pro
                85                  90                  95

Arg Val Ala Asp Ile Leu Thr Gln Leu Leu Gln Thr Asp Asp Ser Ala
            100                 105                 110

Glu Phe Asn Leu Val Asn Asn Ala Leu Leu Ser Ile Phe Lys Met Asp
        115                 120                 125

Ala Lys Gly Thr Leu Gly Gly Leu Phe Ser Gln Ile Leu Gln Gly Glu
    130                 135                 140

Asp Ile Val Arg Glu Arg Ala Ile Lys Phe Leu Ser Thr Lys Leu Lys
145                 150                 155                 160

Thr Leu Pro Asp Glu Val Leu Thr Lys Glu Val Glu Glu Leu Ile Leu
                165                 170                 175

Thr Glu Ser Lys Lys Val Leu Glu Asp Val Thr Gly Glu Glu Phe Val
            180                 185                 190

Leu Phe Met Lys Ile Leu Ser Gly Leu Lys Ser Leu Gln Thr Val Ser
        195                 200                 205

Gly Arg Gln Gln Leu Val Glu Leu Val Ala Glu Gln Ala Asp Leu Glu
    210                 215                 220

Gln Thr Phe Asn Pro Ser Asp Pro Asp Cys Val Asp Arg Leu Leu Gln
225                 230                 235                 240

Cys Thr Arg Gln Ala Val Pro Leu Phe Ser Lys Asn Val His Ser Thr
                245                 250                 255

Arg Phe Val Thr Tyr Phe Cys Glu Gln Val Leu Pro Asn Leu Gly Thr
            260                 265                 270

Leu Thr Thr Pro Val Glu Gly Leu Asp Ile Gln Leu Glu Val Leu Lys
        275                 280                 285

Leu Leu Ala Glu Met Ser Ser Phe Cys Gly Asp Met Glu Lys Leu Glu
    290                 295                 300

Thr Asn Leu Arg Lys Leu Phe Asp Lys Leu Leu Glu Tyr Met Pro Leu
305                 310                 315                 320

Pro Pro Glu Glu Ala Glu Asn Gly Glu Asn Ala Gly Asn Glu Glu Pro
                325                 330                 335

Lys Leu Gln Phe Ser Tyr Val Glu Cys Leu Leu Tyr Ser Phe His Gln
            340                 345                 350

Leu Gly Arg Lys Leu Pro Asp Phe Leu Thr Ala Lys Leu Asn Ala Glu
        355                 360                 365

Lys Leu Lys Asp Phe Lys Ile Arg Leu Gln Tyr Phe Ala Arg Gly Leu
    370                 375                 380

Gln Val Tyr Ile Arg Gln Leu Arg Leu Ala Leu Gln Gly Lys Thr Gly
385                 390                 395                 400

Glu Ala Leu Lys Thr Glu Glu Asn Lys Ile Lys Val Val Ala Leu Lys
                405                 410                 415

Ile Thr Asn Asn Ile Asn Val Leu Ile Lys Asp Leu Phe His Ile Pro
            420                 425                 430

Pro Ser Tyr Lys Ser Thr Val Thr Leu Ser Trp Lys Pro Val Gln Lys
        435                 440                 445

Val Glu Ile Gly Gln Lys Arg Ala Ser Glu Asp Thr Thr Ser Gly Ser
    450                 455                 460

Pro Pro Lys Lys Ser Ser Ala Gly Pro Lys Arg Asp Ala Arg Gln Ile
465                 470                 475                 480

Tyr Asn Pro Pro Ser Gly Lys Tyr Ser Ser Asn Leu Gly Asn Phe Asn
                485                 490                 495

Tyr Glu Gln Arg Gly Ala Phe Arg Gly Ser Arg Gly Gly Arg Gly Trp
            500                 505                 510

Gly Thr Arg Gly Asn Arg Ser Arg Gly Arg Leu Tyr
        515                 520

<210>  51
<211>  2293
<212>  DNA
<213>  Homo sapiens

<220>
<221>  CDS
<222>  (583)..(1431)

<400>  51
atttgggaga gtgctgtgac tcatgctgga ctctaaccca cgagggtttc tcagagtcag      60

cagctggggg atgaagaagt gaaaagtgac tggcaggaaa ttctgcaagc aaggaaaggg     120

aaagagaaat gaactggtgc aggtctgcgg gaagagaatg aggctggatc ctcaaaatca     180

caggaggaag caggcccaga cctcagaggc agaaagagaa agaaaccaga gcttagagtc     240

aggaggagga aaccagaccc cggagccaca aggagagggc tggatccccg gctcagaggg     300

aagagtgtcg ccgcctctgc ctgcgtagcc ccggccatgg ctctgtagcc tcgacccctt     360

tgtgcccccg gcccgtctcc gcgctcacca cgcctgcgct ctccgctccc accttctttc     420

ttcagccgag gccgccgccg cctctccttg ctgcagccat ggagtcttcc actttcgcct     480

tggtgcctgt cttcgcccac ctgagcatcc tccagagcct cgtgccagct gctggtgcag     540

cctctcctgt tgccatcagt gcccagcacc tgtgctacag cc atg tca ctc ctg     594
                                               Met Ser Leu Leu
                                               1

gcg acc ctg ggg ctg gag ctg gac agg gcc ctg ctc cca gct agt ggg     642
Ala Thr Leu Gly Leu Glu Leu Asp Arg Ala Leu Leu Pro Ala Ser Gly
5                   10                  15                  20

ctg gga tgg ctc gta gac tat ggg aaa ctc ccc ccg gcc cct gcc ccc     690
Leu Gly Trp Leu Val Asp Tyr Gly Lys Leu Pro Pro Ala Pro Ala Pro
                25                  30                  35

ctg gct ccc tat gag gtc ctt ggg gga gcc ctg gag ggc ggg ctt cca     738
Leu Ala Pro Tyr Glu Val Leu Gly Gly Ala Leu Glu Gly Gly Leu Pro
            40                  45                  50

gtg ggg gga gag ccc ctg gca ggt gat ggc ttc tct gac tgg atg act     786
Val Gly Gly Glu Pro Leu Ala Gly Asp Gly Phe Ser Asp Trp Met Thr
        55                  60                  65

gag cga gtt gat ttc aca gct ctc ctc cct ctg gag cct ccc tta ccc     834
Glu Arg Val Asp Phe Thr Ala Leu Leu Pro Leu Glu Pro Pro Leu Pro
    70                  75                  80

ccc ggc acc ctc ccc caa cct tcc cca acc cca cct gac ctg gaa gct     882
Pro Gly Thr Leu Pro Gln Pro Ser Pro Thr Pro Pro Asp Leu Glu Ala
85                  90                  95                  100

atg gcc tcc ctc ctc aag aag gag ctg gaa cag atg gaa gac ttc ttc     930
Met Ala Ser Leu Leu Lys Lys Glu Leu Glu Gln Met Glu Asp Phe Phe
                105                 110                 115

cta gat gcc ccg ccc ctc cca cca ccc tcc ccg ccg cca cta cca cca     978
Leu Asp Ala Pro Pro Leu Pro Pro Pro Ser Pro Pro Pro Leu Pro Pro
            120                 125                 130

cca cca cta cca cca gcc ccc tcc ctc ccc ctg tcc ctc ccc tcc ttt    1026
Pro Pro Leu Pro Pro Ala Pro Ser Leu Pro Leu Ser Leu Pro Ser Phe
        135                 140                 145

gac ctc ccc cag ccc cct gtc ttg gat act ctg gac ttg ctg gcc atc     1074
Asp Leu Pro Gln Pro Pro Val Leu Asp Thr Leu Asp Leu Leu Ala Ile
    150                 155                 160

tac tgc cgc aac gag gcc ggg cag gag gaa gtg ggg atg ccg cct ctg     1122
Tyr Cys Arg Asn Glu Ala Gly Gln Glu Glu Val Gly Met Pro Pro Leu
165                 170                 175                 180

ccc ccg cca cag cag ccc cct cct cct tct cca cct caa cct tct cgc     1170
Pro Pro Pro Gln Gln Pro Pro Pro Pro Ser Pro Pro Gln Pro Ser Arg
                185                 190                 195

ctg gcc ccc tac cca cat cct gcc acc acc cga ggg gac cgc aag caa     1218
Leu Ala Pro Tyr Pro His Pro Ala Thr Thr Arg Gly Asp Arg Lys Gln
            200                 205                 210

aag aag aga gac cag aac aag tcg gcg gct ctg agg tac cgc cag cgg     1266
Lys Lys Arg Asp Gln Asn Lys Ser Ala Ala Leu Arg Tyr Arg Gln Arg
        215                 220                 225

aag cgg gca gag ggt gag gcc ctg gag ggc gag tgc cag ggg ctg gag     1314
Lys Arg Ala Glu Gly Glu Ala Leu Glu Gly Glu Cys Gln Gly Leu Glu
    230                 235                 240

gca cgg aat cgc gag ctg aag gaa cgg gca gag tcc gtg gag cgc gag     1362
Ala Arg Asn Arg Glu Leu Lys Glu Arg Ala Glu Ser Val Glu Arg Glu
245                 250                 255                 260

atc cag tac gtc aag gac ctg ctc atc gag gtt tac aag gcc cgg agc     1410
Ile Gln Tyr Val Lys Asp Leu Leu Ile Glu Val Tyr Lys Ala Arg Ser
                265                 270                 275

cag agg acc cgt agc tgc tag aagggcaggg gtgtggcttc tgggggctgg     1461
Gln Arg Thr Arg Ser Cys
            280

tcttcagctc tggcgccttc atccccctgc ctctaccttc attccaaacc cctctcggcc     1521

gggtgcagtg gcttatgctt gtaatcccag cactttggga ggccaaggca ggaggatcgt     1581

ttgaggccag gaggtcaata ccagcctggg caacatagta agaccctgtc tctattaaaa     1641

aaaaaaaatc aacccttctt ccccaccaaa ccacccaact cctctctact cttatccttt     1701

tatcctctgt ctctgcttat cacctctctt gcgtatttct ggatctcctt ccctcctttc     1761

tcgtccaaat catgaaatgt ttggccttag tcaatgtcta tgcccgtcac ataacagccg     1821

aggcaccgag gcccacaggg aagcagctgg gagcttggaa acctggtctc ttgaatttca     1881

aacctggttt cttacaggtg gttgtctggg gtgggtggag tggcgacagg atagagctga     1941

aggactatgc aaatgaggaa gtaagtcagg gcgggctttg agaaggggac ccatatccta     2001

caggcaaaaa gcaggctagg tgaccttggg acactacgct aagggaggga ggctaaaggc     2061

ggccaggttt gcagtgcggg aagatgagca ggccagtggg aggaggggca gggcagggct     2121

gtagttggtg actgggtgtt cattttagct ctaagaaaaa aaatcagtgt ttcgtgaagg     2181

tgttggagag gggctgtgtc tgggtgaggg atggcggggt actgattttt ttgggaggtt     2241

atgagcaaaa ataaaacgaa acatttcctc tggcaaaaaa aaaaaaaaaa aa     2293

<210>  52
<211>  282
<212>  PRT
<213>  Homo sapiens

<400>  52

Met Ser Leu Leu Ala Thr Leu Gly Leu Glu Leu Asp Arg Ala Leu Leu
1               5                   10                  15

Pro Ala Ser Gly Leu Gly Trp Leu Val Asp Tyr Gly Lys Leu Pro Pro
            20                  25                  30

Ala Pro Ala Pro Leu Ala Pro Tyr Glu Val Leu Gly Gly Ala Leu Glu
        35                  40                  45

Gly Gly Leu Pro Val Gly Gly Glu Pro Leu Ala Gly Asp Gly Phe Ser
    50                  55                  60

Asp Trp Met Thr Glu Arg Val Asp Phe Thr Ala Leu Leu Pro Leu Glu
65                  70                  75                  80

Pro Pro Leu Pro Pro Gly Thr Leu Pro Gln Pro Ser Pro Thr Pro Pro
                85                  90                  95

Asp Leu Glu Ala Met Ala Ser Leu Leu Lys Lys Glu Leu Glu Gln Met
            100                 105                 110

Glu Asp Phe Phe Leu Asp Ala Pro Pro Leu Pro Pro Pro Ser Pro Pro
        115                 120                 125

Pro Leu Pro Pro Pro Pro Leu Pro Pro Ala Pro Ser Leu Pro Leu Ser
    130                 135                 140

Leu Pro Ser Phe Asp Leu Pro Gln Pro Pro Val Leu Asp Thr Leu Asp
145                 150                 155                 160

Leu Leu Ala Ile Tyr Cys Arg Asn Glu Ala Gly Gln Glu Glu Val Gly
                165                 170                 175

Met Pro Pro Leu Pro Pro Pro Gln Gln Pro Pro Pro Pro Ser Pro Pro
            180                 185                 190

Gln Pro Ser Arg Leu Ala Pro Tyr Pro His Pro Ala Thr Thr Arg Gly
        195                 200                 205

Asp Arg Lys Gln Lys Lys Arg Asp Gln Asn Lys Ser Ala Ala Leu Arg
    210                 215                 220

Tyr Arg Gln Arg Lys Arg Ala Glu Gly Glu Ala Leu Glu Gly Glu Cys
225                 230                 235                 240

Gln Gly Leu Glu Ala Arg Asn Arg Glu Leu Lys Glu Arg Ala Glu Ser
                245                 250                 255

Val Glu Arg Glu Ile Gln Tyr Val Lys Asp Leu Leu Ile Glu Val Tyr
            260                 265                 270

Lys Ala Arg Ser Gln Arg Thr Arg Ser Cys
        275                 280

<210>  53
<211>  1551
<212>  DNA
<213>  Homo sapiens

<220>
<221>  CDS
<222>  (57)..(1145)

<400>  53
gggcgtctcc gccgcagctc ggctcccgcg cgctcagcac cgccagcggc ggccag atg      59
                                                              Met
                                                              1

cag gcg gag cga gga gct cgg gga ggc cgt ggg cgg cgg cca ggc cgc      107
Gln Ala Glu Arg Gly Ala Arg Gly Gly Arg Gly Arg Arg Pro Gly Arg
            5                   10                  15

ggc cgg cct ggc gga gat cgc cac agc gag cgg ccc gga gcc gca gcg      155
Gly Arg Pro Gly Gly Asp Arg His Ser Glu Arg Pro Gly Ala Ala Ala
        20                  25                  30

gcg gta gcc aga ggc ggc ggc gga ggc ggc ggc ggg gac gga ggc gga      203
Ala Val Ala Arg Gly Gly Gly Gly Gly Gly Gly Gly Asp Gly Gly Gly
    35                  40                  45

cgc cgg ggc cgt ggc cgt ggc cgg ggc ttc cgc ggc gct cgc gga ggc      251
Arg Arg Gly Arg Gly Arg Gly Arg Gly Phe Arg Gly Ala Arg Gly Gly
50                  55                  60                  65

cga gga gga gga ggc gcc ccg cga ggc agc cgc cgg gag ccg gga ggc      299
Arg Gly Gly Gly Gly Ala Pro Arg Gly Ser Arg Arg Glu Pro Gly Gly
                70                  75                  80

tgg ggc gca ggg gcc agc gcg ccg gtt gaa gat gac agc gat gca gag      347
Trp Gly Ala Gly Ala Ser Ala Pro Val Glu Asp Asp Ser Asp Ala Glu
            85                  90                  95

acc tat gga gaa gag aat gat gaa cag gga aat tat tct aaa aga aag      395
Thr Tyr Gly Glu Glu Asn Asp Glu Gln Gly Asn Tyr Ser Lys Arg Lys
        100                 105                 110

att gtc tct aac tgg gat cga tat caa gat att gaa aaa gag gtc aat      443
Ile Val Ser Asn Trp Asp Arg Tyr Gln Asp Ile Glu Lys Glu Val Asn

115                 120                 125

aat gaa agt gga gag tca cag agg gga aca gat ttc agt gtc ctc ctt        491
Asn Glu Ser Gly Glu Ser Gln Arg Gly Thr Asp Phe Ser Val Leu Leu
130                 135                 140                 145

agc tct gca ggg gac tca ttc tca cag ttc cgg ttt gct gag gag aaa        539
Ser Ser Ala Gly Asp Ser Phe Ser Gln Phe Arg Phe Ala Glu Glu Lys
                150                 155                 160

gaa tgg gat agt gaa gct tct tgt cca aaa cag aat tca gca ttt tat        587
Glu Trp Asp Ser Glu Ala Ser Cys Pro Lys Gln Asn Ser Ala Phe Tyr
            165                 170                 175

gtg gat agt gag tta ttg gtt cga gcc ctt caa gag ctg cct ctc tgc        635
Val Asp Ser Glu Leu Leu Val Arg Ala Leu Gln Glu Leu Pro Leu Cys
        180                 185                 190

ctc cga ctc aac gtt gct gcc gaa ctg gtc cag ggt aca gtt cct tta        683
Leu Arg Leu Asn Val Ala Ala Glu Leu Val Gln Gly Thr Val Pro Leu
    195                 200                 205

gag gtt cct cag gtg aaa cca aag aga act gat gat ggc aag gga tta        731
Glu Val Pro Gln Val Lys Pro Lys Arg Thr Asp Asp Gly Lys Gly Leu
210                 215                 220                 225

ggg atg cag tta aag ggg ccc ttg ggg cct gga gga agg ggg ccc atc        779
Gly Met Gln Leu Lys Gly Pro Leu Gly Pro Gly Gly Arg Gly Pro Ile
                230                 235                 240

ttt gag ctg aaa tct gtg gct gct ggc tgc cct gtg ttg ctg ggc aaa        827
Phe Glu Leu Lys Ser Val Ala Ala Gly Cys Pro Val Leu Leu Gly Lys
            245                 250                 255

gac aac cca agc ccg ggt cct tca agg gat tct cag aaa ccc act tcc        875
Asp Asn Pro Ser Pro Gly Pro Ser Arg Asp Ser Gln Lys Pro Thr Ser
        260                 265                 270

cca ctg cag tca gca gga gac cat ttg gaa gaa gaa cta gat ctg ttg        923
Pro Leu Gln Ser Ala Gly Asp His Leu Glu Glu Glu Leu Asp Leu Leu
    275                 280                 285

ctt aat tta gat gca cct ata aaa gag gga gat aac atc tta cca gat        971
Leu Asn Leu Asp Ala Pro Ile Lys Glu Gly Asp Asn Ile Leu Pro Asp
290                 295                 300                 305

cag acg tct cag gac ctg aaa tcc aag gaa gat ggg gag gtg gtc caa        1019
Gln Thr Ser Gln Asp Leu Lys Ser Lys Glu Asp Gly Glu Val Val Gln
                310                 315                 320

gag gaa gaa gtt tgt gca aaa cca tct gtg act gaa gaa aaa aac atg        1067
Glu Glu Glu Val Cys Ala Lys Pro Ser Val Thr Glu Glu Lys Asn Met
            325                 330                 335

gaa cct gag caa cca agt acc tcc aaa aat gtt acc gag gaa gag ctg        1115
Glu Pro Glu Gln Pro Ser Thr Ser Lys Asn Val Thr Glu Glu Glu Leu
        340                 345                 350

gaa gac tgg ttg gac agc atg att tcc taa aaaggggaaa aaaagtgcct        1165
Glu Asp Trp Leu Asp Ser Met Ile Ser
    355                 360

gaagcaaatc ttggttgcct tctaacggca ggtgggcata aggctgtcct tcaggaccag        1225

ccagtttaca agcatgtctc aagctagtgt gttccattat gctcacagca gtaaatgcct 1285

acctctgtgt ttgacatctg aaagaataca ttgaagcagc ttgttgcatt tgtttttctg 1345

gcttagtaat ctaatagatt tccttaaggg caggagatag actctggccc ttgtttctag 1405

cctccttcct tgcagtgttt acaacatagc cagtgtttac agcatagcag atgctgctgc 1465

tgattaagag aatagatgca aacaaggcat gcatttggcc aaaataaaca aatgctggtc 1525

tgtccaaaaa aaaaaaaaaa aaaaaa 1551

<210> 54
<211> 362
<212> PRT
<213> Homo sapiens

<400> 54

Met Gln Ala Glu Arg Gly Ala Arg Gly Gly Arg Gly Arg Arg Pro Gly
1               5                   10                  15

Arg Gly Arg Pro Gly Gly Asp Arg His Ser Glu Arg Pro Gly Ala Ala
            20                  25                  30

Ala Ala Val Ala Arg Gly Gly Gly Gly Gly Gly Gly Gly Asp Gly Gly
        35                  40                  45

Gly Arg Arg Gly Arg Gly Arg Gly Arg Gly Phe Arg Gly Ala Arg Gly
    50                  55                  60

Gly Arg Gly Gly Gly Gly Ala Pro Arg Gly Ser Arg Arg Glu Pro Gly
65                  70                  75                  80

Gly Trp Gly Ala Gly Ala Ser Ala Pro Val Glu Asp Asp Ser Asp Ala
                85                  90                  95

Glu Thr Tyr Gly Glu Glu Asn Asp Glu Gln Gly Asn Tyr Ser Lys Arg
            100                 105                 110

Lys Ile Val Ser Asn Trp Asp Arg Tyr Gln Asp Ile Glu Lys Glu Val
        115                 120                 125

Asn Asn Glu Ser Gly Glu Ser Gln Arg Gly Thr Asp Phe Ser Val Leu
    130                 135                 140

Leu Ser Ser Ala Gly Asp Ser Phe Ser Gln Phe Arg Phe Ala Glu Glu
145                 150                 155                 160

Lys Glu Trp Asp Ser Glu Ala Ser Cys Pro Lys Gln Asn Ser Ala Phe
                165                 170                 175

Tyr Val Asp Ser Glu Leu Leu Val Arg Ala Leu Gln Glu Leu Pro Leu
            180                 185                 190

Cys Leu Arg Leu Asn Val Ala Ala Glu Leu Val Gln Gly Thr Val Pro
        195                 200                 205

Leu Glu Val Pro Gln Val Lys Pro Lys Arg Thr Asp Asp Gly Lys Gly
    210                 215                 220

Leu Gly Met Gln Leu Lys Gly Pro Leu Gly Pro Gly Gly Arg Gly Pro
225                 230                 235                 240

Ile Phe Glu Leu Lys Ser Val Ala Ala Gly Cys Pro Val Leu Leu Gly
                245                 250                 255

Lys Asp Asn Pro Ser Pro Gly Pro Ser Arg Asp Ser Gln Lys Pro Thr
            260                 265                 270

Ser Pro Leu Gln Ser Ala Gly Asp His Leu Glu Glu Glu Leu Asp Leu
        275                 280                 285

Leu Leu Asn Leu Asp Ala Pro Ile Lys Glu Gly Asp Asn Ile Leu Pro
    290                 295                 300

Asp Gln Thr Ser Gln Asp Leu Lys Ser Lys Glu Asp Gly Glu Val Val
305                 310                 315                 320

Gln Glu Glu Glu Val Cys Ala Lys Pro Ser Val Thr Glu Glu Lys Asn
                325                 330                 335

Met Glu Pro Glu Gln Pro Ser Thr Ser Lys Asn Val Thr Glu Glu Glu
            340                 345                 350

Leu Glu Asp Trp Leu Asp Ser Met Ile Ser
        355                 360

<210>  55
<211>  912
<212>  DNA
<213>  Homo sapiens

<220>
<221>  CDS
<222>  (17)..(772)

<400>  55
acagacctgc cccgcc atg acc cgg ctg aca gtc ctg gcc ctg ctg gct ggt        52
                  Met Thr Arg Leu Thr Val Leu Ala Leu Leu Ala Gly

1                   5                   10

ctg ctg gcg tcc tcg agg gcc ggc tcc agc ccc ctt ttg gac atc gtt        100
Leu Leu Ala Ser Ser Arg Ala Gly Ser Ser Pro Leu Leu Asp Ile Val
        15                  20                  25

ggc ggc cgg aag gcg agg ccc cgc cag ttc ccg ttc ctg gcc tcc att        148
Gly Gly Arg Lys Ala Arg Pro Arg Gln Phe Pro Phe Leu Ala Ser Ile
    30                  35                  40

cag aat caa ggc agg cac ttc tgc ggg ggt gcc ctg atc cat gcc cgc        196
Gln Asn Gln Gly Arg His Phe Cys Gly Gly Ala Leu Ile His Ala Arg
45                  50                  55                  60

ttc gtg atg acc gcg gcc agc tgc ttc caa agc cag aac ccc ggg gtt        244
Phe Val Met Thr Ala Ala Ser Cys Phe Gln Ser Gln Asn Pro Gly Val
                65                  70                  75

agc acc gtg gtg ctg ggt gcc tat gac ctg agg cgg cgg gag agg cag        292
Ser Thr Val Val Leu Gly Ala Tyr Asp Leu Arg Arg Arg Glu Arg Gln
            80                  85                  90

tcc cgc cag acg ttt tcc atc agc agc atg agc gag aat ggc tac gac        340
Ser Arg Gln Thr Phe Ser Ile Ser Ser Met Ser Glu Asn Gly Tyr Asp
        95                  100                 105

ccc cag cag aac ctg aac gac ctg atg ctg ctt cag ctg gac cgt gag        388
Pro Gln Gln Asn Leu Asn Asp Leu Met Leu Leu Gln Leu Asp Arg Glu
    110                 115                 120

gcc aac ctc acc agc agc gtg acg ata ctg cca ctg cct ctg cag aac        436
Ala Asn Leu Thr Ser Ser Val Thr Ile Leu Pro Leu Pro Leu Gln Asn
125                 130                 135                 140

gcc acg gtg gaa gcc ggc acc aga tgc cag gtg gcc ggc tgg ggg agc        484
Ala Thr Val Glu Ala Gly Thr Arg Cys Gln Val Ala Gly Trp Gly Ser
                145                 150                 155

cag cgc agt ggg ggg cgt ctc tcc cgt ttt ccc agg ttt gtc aac gtg        532
Gln Arg Ser Gly Gly Arg Leu Ser Arg Phe Pro Arg Phe Val Asn Val
            160                 165                 170

act gtg acc ccc gag gac cag tgt cgc ccc aac aac gtg tgc acc ggt        580
Thr Val Thr Pro Glu Asp Gln Cys Arg Pro Asn Asn Val Cys Thr Gly
        175                 180                 185

gtg ctc acc cgc cgc ggt ggc atc tgc aat ggg gac ggg ggc acc ccc        628
Val Leu Thr Arg Arg Gly Gly Ile Cys Asn Gly Asp Gly Gly Thr Pro
    190                 195                 200

ctc gtc tgc gag ggc ctg gcc cac ggc gtg gcc tcc ttt tcc ctg ggg        676
Leu Val Cys Glu Gly Leu Ala His Gly Val Ala Ser Phe Ser Leu Gly
205                 210                 215                 220

ccc tgt ggc cga ggc cct gac ttc ttc acc cga gtg gcg ctc ttc cga        724
Pro Cys Gly Arg Gly Pro Asp Phe Phe Thr Arg Val Ala Leu Phe Arg
                225                 230                 235

gac tgg atc gat ggt gtt ctc aac aac ccg gga ccg ggg cca gcc tag        772
Asp Trp Ile Asp Gly Val Leu Asn Asn Pro Gly Pro Gly Pro Ala
            240                 245                 250

gggggcctgt gacctcccat ggagcccagc cccgccctcc acacctccgg cgctccgcac      832

ccacctccca cggccccgcc cctgcccccg ctccggccag aggggccctg gctgtaataa 892

agaagccgat ctctcctctg 912

<210> 56
<211> 251
<212> PRT
<213> Homo sapiens

<400> 56

Met Thr Arg Leu Thr Val Leu Ala Leu Leu Ala Gly Leu Leu Ala Ser
1               5                   10                  15

Ser Arg Ala Gly Ser Ser Pro Leu Leu Asp Ile Val Gly Gly Arg Lys
            20                  25                  30

Ala Arg Pro Arg Gln Phe Pro Phe Leu Ala Ser Ile Gln Asn Gln Gly
        35                  40                  45

Arg His Phe Cys Gly Gly Ala Leu Ile His Ala Arg Phe Val Met Thr
    50                  55                  60

Ala Ala Ser Cys Phe Gln Ser Gln Asn Pro Gly Val Ser Thr Val Val
65                  70                  75                  80

Leu Gly Ala Tyr Asp Leu Arg Arg Arg Glu Arg Gln Ser Arg Gln Thr
                85                  90                  95

Phe Ser Ile Ser Ser Met Ser Glu Asn Gly Tyr Asp Pro Gln Gln Asn
            100                 105                 110

Leu Asn Asp Leu Met Leu Leu Gln Leu Asp Arg Glu Ala Asn Leu Thr
        115                 120                 125

Ser Ser Val Thr Ile Leu Pro Leu Pro Leu Gln Asn Ala Thr Val Glu
    130                 135                 140

Ala Gly Thr Arg Cys Gln Val Ala Gly Trp Gly Ser Gln Arg Ser Gly
145                 150                 155                 160

Gly Arg Leu Ser Arg Phe Pro Arg Phe Val Asn Val Thr Val Thr Pro
                165                 170                 175

Glu Asp Gln Cys Arg Pro Asn Asn Val Cys Thr Gly Val Leu Thr Arg
            180                 185                 190

Arg Gly Gly Ile Cys Asn Gly Asp Gly Gly Thr Pro Leu Val Cys Glu
        195                 200                 205

Gly Leu Ala His Gly Val Ala Ser Phe Ser Leu Gly Pro Cys Gly Arg
    210                 215                 220

Gly Pro Asp Phe Phe Thr Arg Val Ala Leu Phe Arg Asp Trp Ile Asp
225                 230                 235                 240

Gly Val Leu Asn Asn Pro Gly Pro Gly Pro Ala
                245                 250

<210>  57
<211>  3885
<212>  DNA
<213>  Homo sapiens

<220>
<221>  CDS
<222>  (88)..(1125)

<400>  57
aggccggggc ggggctggga agtagtcggg cggggttgtg agacgccgcg ctcagcttcc      60

atcgctgggc ggtcaacaag tgcgggc ctg gct cag cgc ggg ggg gcg cgg aga     114
                              Leu Ala Gln Arg Gly Gly Ala Arg Arg
                              1               5

ccg cga ggc gac cgg gag cgg ctg ggt tcc cgg ctg cgc gcc ctt cgg     162
Pro Arg Gly Asp Arg Glu Arg Leu Gly Ser Arg Leu Arg Ala Leu Arg
10                  15                  20                  25

cca ggc cgg gag ccg cgc cag tcg gag ccc ccg gcc cag cgt ggt ccg     210
Pro Gly Arg Glu Pro Arg Gln Ser Glu Pro Pro Ala Gln Arg Gly Pro
                30                  35                  40

cct ccc tct cgg cgt cca cct gcc cgg agt act gcc agc ggg cat gac     258
Pro Pro Ser Arg Arg Pro Pro Ala Arg Ser Thr Ala Ser Gly His Asp
            45                  50                  55

cga ccc acc agg ggc gcc gcc gcc ggc gct cgc agg ccg cgg atg aag     306
Arg Pro Thr Arg Gly Ala Ala Ala Gly Ala Arg Arg Pro Arg Met Lys
        60                  65                  70

aag aaa acc cgg cgc cgc tcg acc cgg agc gag gag ttg acc cgg agc     354
Lys Lys Thr Arg Arg Arg Ser Thr Arg Ser Glu Glu Leu Thr Arg Ser
    75                  80                  85

gag gag ttg acc ctg agt gag gaa gcg acc tgg agt gaa gag gcg acc     402
Glu Glu Leu Thr Leu Ser Glu Glu Ala Thr Trp Ser Glu Glu Ala Thr
90                  95                  100                 105

cag agt gag gag gcg acc cag ggc gaa gag atg aat cgg agc cag gag     450
Gln Ser Glu Glu Ala Thr Gln Gly Glu Glu Met Asn Arg Ser Gln Glu
                110                 115                 120

gtg acc cgg gac gag gag tcg acc cgg agc gag gag gtg acc agg gag     498
Val Thr Arg Asp Glu Glu Ser Thr Arg Ser Glu Glu Val Thr Arg Glu
            125                 130                 135

gaa atg gcg gca gct ggg ctc acc gtg act gtc acc cac agc aat gag     546
Glu Met Ala Ala Ala Gly Leu Thr Val Thr Val Thr His Ser Asn Glu
        140                 145                 150

aag cac gac ctt cat gtt acc tcc cag cag ggc agc agt gaa cca gtt     594
Lys His Asp Leu His Val Thr Ser Gln Gln Gly Ser Ser Glu Pro Val
    155                 160                 165

gtc caa gac ctg gcc cag gtt gtt gaa gag gtc ata ggg gtt cca cag     642
Val Gln Asp Leu Ala Gln Val Val Glu Glu Val Ile Gly Val Pro Gln
170                 175                 180                 185

tct ttt cag aaa ctc ata ttt aag gga aaa tct ctg aag gaa atg gaa     690
Ser Phe Gln Lys Leu Ile Phe Lys Gly Lys Ser Leu Lys Glu Met Glu
                190                 195                 200

aca ccg ttg tca gca ctt gga ata caa gat ggt tgc cgg gtc atg tta     738
Thr Pro Leu Ser Ala Leu Gly Ile Gln Asp Gly Cys Arg Val Met Leu
            205                 210                 215

att ggg aaa aag aac agt cca cag gaa gag gtt gaa cta aag aag ttg     786
Ile Gly Lys Lys Asn Ser Pro Gln Glu Glu Val Glu Leu Lys Lys Leu
        220                 225                 230

aaa cat ttg gag aag tct gtg gag aag ata gct gac cag ctg gaa gag     834
Lys His Leu Glu Lys Ser Val Glu Lys Ile Ala Asp Gln Leu Glu Glu
    235                 240                 245

ttg aat aaa gag ctt act gga atc cag cag ggt ttt ctg ccc aag gat     882
Leu Asn Lys Glu Leu Thr Gly Ile Gln Gln Gly Phe Leu Pro Lys Asp
250                 255                 260                 265

ttg caa gct gaa gct ctc tgc aaa ctt gat agg aga gta aaa gcc aca     930
Leu Gln Ala Glu Ala Leu Cys Lys Leu Asp Arg Arg Val Lys Ala Thr
                270                 275                 280

ata gag cag ttt atg aag atc ttg gag gag att gac aca ctg atc ctg     978
Ile Glu Gln Phe Met Lys Ile Leu Glu Glu Ile Asp Thr Leu Ile Leu
            285                 290                 295

cca gaa aat ttc aaa gac agt aga ttg aaa agg aaa ggc ttg gta aaa     1026
Pro Glu Asn Phe Lys Asp Ser Arg Leu Lys Arg Lys Gly Leu Val Lys
        300                 305                 310

aag gtt cag gca ttc cta gcc gag tgt gac aca gtg gag cag aac atc     1074
Lys Val Gln Ala Phe Leu Ala Glu Cys Asp Thr Val Glu Gln Asn Ile
    315                 320                 325

tgc cag gag act gag cgg ctg cag tct aca aac ttt gcc ctg gcc gag     1122
Cys Gln Glu Thr Glu Arg Leu Gln Ser Thr Asn Phe Ala Leu Ala Glu
330                 335                 340                 345

tga ggtgtagcag aaaaaggctg tgctgccctg aagaatggcg ccaccagctc     1175

tgccgtctct ggagcggaat ttacctgatt tcttcagggc tgctgggggc aactggccat     1235

ttgccaattt tcctactctc acactggttc tcaatgaaaa atagtgtctt tgtgattttg     1295

agtaaagctc ctatctgttt tctccttctg tctctgtggt tgtactgtcc agcaatccac     1355

cttttctgga gagggccacc tctgcccaaa ttttcccagc tgtttggacc tctgggtgct     1415

ttctttgggc tggtgagagc tctaatttgc cttgggccag tttcaggttt ataggccccc     1475

tcagtcttca gatacatgag ggcttctttg ctcttgtgat cgtgtagtcc catagctgta 1535

aaaccagaat caccaggagg ttgcacctag tcaggaatat tgggaatggc ctagaacaag 1595

gtgtttggca cataagtaga ccacttatcc ctcattgtga cctaattcca gagcatctgg 1655

ctgggttgtt gggttctaga ctttgtcctc acctcccagt gaccctgact agccacaggc 1715

catgagatac cagggggccg ttccttggat ggagcctgtg gttgatgcaa ggcttccttg 1775

tccccaagca agtcttcaga aggttagaac ccagtgttga ctgagtctgt gcttgaaacc 1835

aggccagagc catggattag gaagggcaaa gagaaggcac cagaatgagt aaagcaggca 1895

ggtggtgaag ccaaccataa acttctcagg agtgacatgt gcttccttca aaggcatttt 1955

tgttaaccat atccttctga gttctatgtt tccttcacag ctgttctatc cattttgtgg 2015

actgtccccc acccccaccc catcattgtt tttaaaaaat taaggcctgg cgcagcagct 2075

catgcctata atcccagcac tttgggaggc tgaggcgggc ggatcacttg aggccaggag 2135

tttgagacca gcccaggcaa catagcaaaa ccccattctg ctttaaaaaa aaaaaaaaaa 2195

aaaattagct tggcgtagtg gcatgtgcct ataatcccag ctactgggga ggctgaggca 2255

caagaatcat ttgaacctgg gaggtagagg ttgctgtgag ccgagattac gcccctgcac 2315

tccagcctgg gtcacagagt gagactccat ctcagaaaaa aaaaaaattg agtcaggtgc 2375

agtagctcct tcctgtagtc ccagctactt gggaggctga ggctagagga tcacttgagc 2435

ccaggagttt gagtctagtc tgggcaacat agcaagaccc catctctaaa atttaagtaa 2495

gtaaaagtag ataaataaaa agaaaaaaaa actgtttatg tgctcatcat aaagtagaag 2555

agtggtttgc tttttttttt ttttttggat taatgaggaa atcattctgt ggctctagtc 2615

ataatttatg cttaataaca ttgatagtag ccctttgcgc tataactcta cctaaagact 2675

cacatcattt ggcagagaga gagtcgttga agtcccagga attcaggact gggcaggtta 2735

agacctcaga caaggtagta gaggtagact tgtggacaag gctcgggtcc cagcccaccg 2795

caccccaact ttaatcagag tggttcacta ttgatctatt tttgtgtgat agctgtgtgg 2855

cgtgggccac aacatttaat gagaagttac tgtgcaccaa actgccgaac accattctaa 2915

actattcata tatattagtc atttaattct tacataactt gagaggtaga cagatatcct 2975

tattttagag atgaggaaac caagagaact taggtcatta gcgcaaggtt gtagagtaag 3035

cggcaaagcc aagacacaaa gctgggtggt ttggtttcag agccagtgct tttcccctct 3095

actgtactgc ctctcaacca acacagggtt gcacaggccc attctctgat ttttttcctc 3155

ttgtcctctg cctctccctc tagctcccac ttcctctctg ctctagttca ttttctttag 3215

agcagcccga gtgatcatga agtgcaaatc ttgccatgtc agtcccctgc ttagaaccct 3275

ccaatggctc actttctctt taggcaaaag tctttacccc atgccttctc ccatctcatc 3335

tcaaccccct catttgttgg ctgtctgctg tcagccactc ttctttcagg tcctcagatg 3395

cactgcaccc tctcctgcct gggggtcttt gctcctgcta ctacctctgc ttgaacagct 3455

cctcaccttc cttcctccaa ccctaccctt gtataggtga cttttgttca tccttcagaa 3515

ttcaactcac atgtctcttg catggagaac cctcacctac tgtgttgaga ccctgtccag 3575

cccccaggtg ggatcctctc tcgacttccc atacatttct ttcacagcat ttacatagtc 3635

catgatagtt tacttgtggg attatttggt taatctttgc ctttaacacc agggttcctt 3695

gggtgaagga gcttctttat cttggtaaca gcattatttc aagcataact tgtaatatag 3755

ttatattaca tatataacat atatatatat aacataacat atataacata tataacaagc 3815

ataacttgtt atatagtctt gtatatagta agacctcaat aaatatttgg agaacaaaaa 3875

aaaaaaaaaa 3885

<210> 58
<211> 345
<212> PRT
<213> Homo sapiens

<400> 58

Met Ala Gln Arg Gly Gly Ala Arg Arg Pro Arg Gly Asp Arg Glu Arg
1               5                   10                  15

Leu Gly Ser Arg Leu Arg Ala Leu Arg Pro Gly Arg Glu Pro Arg Gln
            20                  25                  30

Ser Glu Pro Pro Ala Gln Arg Gly Pro Pro Pro Ser Arg Arg Pro Pro
        35                  40                  45

Ala Arg Ser Thr Ala Ser Gly His Asp Arg Pro Thr Arg Gly Ala Ala
    50                  55                  60

Ala Gly Ala Arg Arg Pro Arg Met Lys Lys Lys Thr Arg Arg Arg Ser
65                  70                  75                  80

Thr Arg Ser Glu Glu Leu Thr Arg Ser Glu Glu Leu Thr Leu Ser Glu
                85                  90                  95

Glu Ala Thr Trp Ser Glu Glu Ala Thr Gln Ser Glu Glu Ala Thr Gln
            100                 105                 110

Gly Glu Glu Met Asn Arg Ser Gln Glu Val Thr Arg Asp Glu Glu Ser
        115                 120                 125

Thr Arg Ser Glu Glu Val Thr Arg Glu Glu Met Ala Ala Ala Gly Leu
    130                 135                 140

Thr Val Thr Val Thr His Ser Asn Glu Lys His Asp Leu His Val Thr
145                 150                 155                 160

Ser Gln Gln Gly Ser Ser Glu Pro Val Val Gln Asp Leu Ala Gln Val
                165                 170                 175

Val Glu Glu Val Ile Gly Val Pro Gln Ser Phe Gln Lys Leu Ile Phe
            180                 185                 190

Lys Gly Lys Ser Leu Lys Glu Met Glu Thr Pro Leu Ser Ala Leu Gly
        195                 200                 205

Ile Gln Asp Gly Cys Arg Val Met Leu Ile Gly Lys Lys Asn Ser Pro
    210                 215                 220

Gln Glu Glu Val Glu Leu Lys Lys Leu Lys His Leu Glu Lys Ser Val
225                 230                 235                 240

Glu Lys Ile Ala Asp Gln Leu Glu Glu Leu Asn Lys Glu Leu Thr Gly
                245                 250                 255

Ile Gln Gln Gly Phe Leu Pro Lys Asp Leu Gln Ala Glu Ala Leu Cys
            260                 265                 270

Lys Leu Asp Arg Arg Val Lys Ala Thr Ile Glu Gln Phe Met Lys Ile
        275                 280                 285

Leu Glu Glu Ile Asp Thr Leu Ile Leu Pro Glu Asn Phe Lys Asp Ser
    290                 295                 300

Arg Leu Lys Arg Lys Gly Leu Val Lys Lys Val Gln Ala Phe Leu Ala
305                 310                 315                 320

Glu Cys Asp Thr Val Glu Gln Asn Ile Cys Gln Glu Thr Glu Arg Leu
                325                 330                 335

Gln Ser Thr Asn Phe Ala Leu Ala Glu
            340                 345

<210>  59
<211>  2575
<212>  DNA
<213>  Homo sapiens

<220>
<221>  CDS
<222>  (367)..(1068)

<400> 59
ggaggaggaa gcaagcgagg gggctggttc ctgagcttcg caattcctgt gtcgccttct 60

gggctcccag cctgccgggt cgcatgatcc ctccggccgg agctggtttt tttgccagcc 120

accgcgaggc cggctgagtt accggcatcc ccgcagccac ctcctctccc gacctgtgat 180

acaaaagatc ttccgggggc tgcacctgcc tgcctttgcc taaggcggat ttgaatctct 240

ttctctccct tcagaatctt atcttggctt tggatcttag aagagaatca ctaaccagag 300

acgagactca gtgagtgagc aggtgttttg gacaatggac tggttgagcc catccctatt 360

ataaaa atg tct cag agc aac cgg gag ctg gtg gtt gac ttt ctc tcc 408
Met Ser Gln Ser Asn Arg Glu Leu Val Val Asp Phe Leu Ser
1               5                   10

tac aag ctt tcc cag aaa gga tac agc tgg agt cag ttt agt gat gtg 456
Tyr Lys Leu Ser Gln Lys Gly Tyr Ser Trp Ser Gln Phe Ser Asp Val
15                  20                  25                  30

gaa gag aac agg act gag gcc cca gaa ggg act gaa tcg gag atg gag 504
Glu Glu Asn Arg Thr Glu Ala Pro Glu Gly Thr Glu Ser Glu Met Glu
                35                  40                  45

acc ccc agt gcc atc aat ggc aac cca tcc tgg cac ctg gca gac agc 552
Thr Pro Ser Ala Ile Asn Gly Asn Pro Ser Trp His Leu Ala Asp Ser
            50                  55                  60

ccc gcg gtg aat gga gcc act ggc cac agc agc agt ttg gat gcc cgg 600
Pro Ala Val Asn Gly Ala Thr Gly His Ser Ser Ser Leu Asp Ala Arg
        65                  70                  75

gag gtg atc ccc atg gca gca gta aag caa gcg ctg agg gag gca ggc 648
Glu Val Ile Pro Met Ala Ala Val Lys Gln Ala Leu Arg Glu Ala Gly
    80                  85                  90

gac gag ttt gaa ctg cgg tac cgg cgg gca ttc agt gac ctg aca tcc 696
Asp Glu Phe Glu Leu Arg Tyr Arg Arg Ala Phe Ser Asp Leu Thr Ser
95                  100                 105                 110

cag ctc cac atc acc cca ggg aca gca tat cag agc ttt gaa cag gta 744
Gln Leu His Ile Thr Pro Gly Thr Ala Tyr Gln Ser Phe Glu Gln Val
                115                 120                 125

gtg aat gaa ctc ttc cgg gat ggg gta aac tgg ggt cgc att gtg gcc 792
Val Asn Glu Leu Phe Arg Asp Gly Val Asn Trp Gly Arg Ile Val Ala
            130                 135                 140

ttt ttc tcc ttc ggc ggg gca ctg tgc gtg gaa agc gta gac aag gag 840
Phe Phe Ser Phe Gly Gly Ala Leu Cys Val Glu Ser Val Asp Lys Glu
        145                 150                 155

atg cag gta ttg gtg agt cgg atc gca gct tgg atg gcc act tac ctg 888
Met Gln Val Leu Val Ser Arg Ile Ala Ala Trp Met Ala Thr Tyr Leu
    160                 165                 170

aat gac cac cta gag cct tgg atc cag gag aac ggc ggc tgg gat act 936
Asn Asp His Leu Glu Pro Trp Ile Gln Glu Asn Gly Gly Trp Asp Thr
175                 180                 185                 190

ttt gtg gaa ctc tat ggg aac aat gca gca gcc gag agc cga aag ggc 984
Phe Val Glu Leu Tyr Gly Asn Asn Ala Ala Ala Glu Ser Arg Lys Gly

195                 200                 205

cag gaa cgc ttc aac cgc tgg ttc ctg acg ggc atg act gtg gcc ggc      1032
Gln Glu Arg Phe Asn Arg Trp Phe Leu Thr Gly Met Thr Val Ala Gly
        210                 215                 220

gtg gtt ctg ctg ggc tca ctc ttc agt cgg aaa tga ccagacactg           1078
Val Val Leu Leu Gly Ser Leu Phe Ser Arg Lys
        225                 230

accatccact ctaccctccc acccccttct ctgctccacc acatcctccg tccagccgcc    1138

attgccacca ggagaaccac tacatgcagc ccatgcccac ctgcccatca cagggttggg    1198

cccagatctg gtcccttgca gctagttttc tagaatttat cacacttctg tgagaccccc    1258

acacctcagt tcccttggcc tcagaattca caaaatttcc acaaaatctg tccaaaggag    1318

gctggcaggt atggaagggt ttgtggctgg gggcaggagg gccctacctg attggtgcaa    1378

cccttacccc ttagcctccc tgaaaatgtt tttctgccag ggagcttgaa agttttcaga    1438

acctcttccc cagaaaggag actagattgc ctttgttttg atgtttgtgg cctcagaatt    1498

gatcattttc cccccactct ccccacacta acctgggttc cctttccttc catccctacc    1558

ccctaagagc catttagggg ccacttttga ctagggattc aggctgcttg ggataaagat    1618

gcaaggacca ggactccctc ctcacctctg gactggctag agtcctcact cccagtccaa    1678

atgtcctcca gaagcctctg gctagaggcc agccccaccc aggagggagg gggctatagc    1738

tacaggaagc accccatgcc aaagctaggg tggcccttgc agttcagcac caccctagtc    1798

ccttcccctc cctggctccc atgaccatac tgagggacca actgggccca agacagatgc    1858

cccagagctg tttatggcct cagctgcctc acttcctaca agagcagcct gtggcatctt    1918

tgccttgggc tgctcctcat ggtgggttca ggggactcag ccctgaggtg aaagggagct    1978

atcaggaaca gctatgggag ccccagggtc ttccctacct caggcaggaa gggcaggaag    2038

gagagcctgc tgcatggggt ggggtagggc tgactagaag ggccagtcct gcctggccag    2098

gcagatctgt gccccatgcc tgtccagcct gggcagccag gctgccaagg ccagagtggc    2158

ctggccagga gctcttcagg cctccctctc tcttctgctc cacccttggc ctgtctcatc    2218

cccaggggtc ccagccaccc cgggctctct gctgtacata tttgagacta gtttttattc    2278

cttgtgaaga tgatatacta tttttgttaa gcgtgtctgt atttatgtgt gaggagctgc    2338

tggcttgcag tgcgcgtgca cgtggagagc tggtgcccgg agattggacg gcctgatgct    2398

ccctcccctg ccctggtcca gggaagctgg ccgagggtcc tggctcctga ggggcatctg    2458

cccctccccc aacccccacc ccacacttgt tccagctctt tgaaatagtc tgtgtgaagg    2518

tgaaagtgca gttcagtaat aaactgtgtt tactcagtga aaaaaaaaaa aaaaaaa       2575

<210>  60
<211>  233

<212>  PRT
<213>  Homo sapiens

<400>  60

Met Ser Gln Ser Asn Arg Glu Leu Val Val Asp Phe Leu Ser Tyr Lys
1               5                   10                  15

Leu Ser Gln Lys Gly Tyr Ser Trp Ser Gln Phe Ser Asp Val Glu Glu
            20                  25                  30

Asn Arg Thr Glu Ala Pro Glu Gly Thr Glu Ser Glu Met Glu Thr Pro
        35                  40                  45

Ser Ala Ile Asn Gly Asn Pro Ser Trp His Leu Ala Asp Ser Pro Ala
    50                  55                  60

Val Asn Gly Ala Thr Gly His Ser Ser Ser Leu Asp Ala Arg Glu Val
65                  70                  75                  80

Ile Pro Met Ala Ala Val Lys Gln Ala Leu Arg Glu Ala Gly Asp Glu
                85                  90                  95

Phe Glu Leu Arg Tyr Arg Arg Ala Phe Ser Asp Leu Thr Ser Gln Leu
            100                 105                 110

His Ile Thr Pro Gly Thr Ala Tyr Gln Ser Phe Glu Gln Val Val Asn
        115                 120                 125

Glu Leu Phe Arg Asp Gly Val Asn Trp Gly Arg Ile Val Ala Phe Phe
    130                 135                 140

Ser Phe Gly Gly Ala Leu Cys Val Glu Ser Val Asp Lys Glu Met Gln
145                 150                 155                 160

Val Leu Val Ser Arg Ile Ala Ala Trp Met Ala Thr Tyr Leu Asn Asp
                165                 170                 175

His Leu Glu Pro Trp Ile Gln Glu Asn Gly Gly Trp Asp Thr Phe Val
            180                 185                 190

Glu Leu Tyr Gly Asn Asn Ala Ala Ala Glu Ser Arg Lys Gly Gln Glu
        195                 200                 205

Arg Phe Asn Arg Trp Phe Leu Thr Gly Met Thr Val Ala Gly Val Val
    210                 215                 220

Leu Leu Gly Ser Leu Phe Ser Arg Lys
225                 230

<210>  61
<211>  3060
<212>  DNA
<213>  Homo sapiens

<220>
<221>  CDS
<222>  (282)..(1634)

<400>  61
gggagtggcc gctctaggca gcggggaggt cgcggggttg aggggggttg tgaaaggaga      60

gcggcctctc ctctatggtc acggggccgg ggcacgcttc ccccactctg tcttgttact     120

tccggtagcg aagcctctcc ctcttcctct gctcccgcgg ggtctgtgct gagaataatg     180

gcccggttgg cccgggacga gtggaatgat taatgatgtt ttgcagcagt tttctacgtc     240

tgaaattttt tatgtctctg gaacccagaa tttgctaaga g atg gag gaa cct cag     296
                                              Met Glu Glu Pro Gln
                                              1               5

aaa agc tat gtg aac aca atg gac ctt gag aga gat gaa cct ctc aaa     344
Lys Ser Tyr Val Asn Thr Met Asp Leu Glu Arg Asp Glu Pro Leu Lys
                10                  15                  20

agc acc ggc cct cag att tct gtt agt gaa ttt tct tgc cac tgc tgc     392
Ser Thr Gly Pro Gln Ile Ser Val Ser Glu Phe Ser Cys His Cys Cys
            25                  30                  35

tac gac atc ctg gtt aac ccc acc acc ttg aac tgt ggg cac agc ttc     440
Tyr Asp Ile Leu Val Asn Pro Thr Thr Leu Asn Cys Gly His Ser Phe
        40                  45                  50

tgc cgt cac tgc ctt gct tta tgg tgg gca tct tca aag aaa aca gaa     488
Cys Arg His Cys Leu Ala Leu Trp Trp Ala Ser Ser Lys Lys Thr Glu
    55                  60                  65

tgt cca gaa tgc aga gaa aaa tgg gaa ggt ttc ccc aaa gtc agt att     536
Cys Pro Glu Cys Arg Glu Lys Trp Glu Gly Phe Pro Lys Val Ser Ile
70                  75                  80                  85

ctc ctc agg gat gcc att gaa aag tta ttt cct gat gcc att aga ctg     584
Leu Leu Arg Asp Ala Ile Glu Lys Leu Phe Pro Asp Ala Ile Arg Leu
                90                  95                  100

aga ttt gaa gac att cag cag aat aat gac ata gtc caa agt ctt gca     632
Arg Phe Glu Asp Ile Gln Gln Asn Asn Asp Ile Val Gln Ser Leu Ala
            105                 110                 115

gcc ttt cag aaa tat ggg aat gat cag att cct tta gct cct aac aca     680
Ala Phe Gln Lys Tyr Gly Asn Asp Gln Ile Pro Leu Ala Pro Asn Thr
        120                 125                 130

ggc cga gcg aat cag cag atg gga ggg gga ttc ttt tcc ggt gtg ctc     728
Gly Arg Ala Asn Gln Gln Met Gly Gly Gly Phe Phe Ser Gly Val Leu
    135                 140                 145

aca gct tta act gga gtg gca gtg gtc ctg ctc gtc tat cac tgg agc     776
Thr Ala Leu Thr Gly Val Ala Val Val Leu Leu Val Tyr His Trp Ser

150                 155                 160                 165

agc agg gaa tct gaa cac gac ctc ctg gtc cac aag gct gtg gcc aaa      824
Ser Arg Glu Ser Glu His Asp Leu Leu Val His Lys Ala Val Ala Lys
                170                 175                 180

tgg acg gcg gaa gaa gtt gtc ctc tgg ctg gag cag ctg ggc cct tgg      872
Trp Thr Ala Glu Glu Val Val Leu Trp Leu Glu Gln Leu Gly Pro Trp
            185                 190                 195

gca tct ctt tac agg gaa agg ttt tta tct gaa cga gta aat gga agg      920
Ala Ser Leu Tyr Arg Glu Arg Phe Leu Ser Glu Arg Val Asn Gly Arg
        200                 205                 210

ttg ctt tta act ttg aca gag gaa gaa ttt tcc aag acg ccc tat acc      968
Leu Leu Leu Thr Leu Thr Glu Glu Glu Phe Ser Lys Thr Pro Tyr Thr
    215                 220                 225

ata gaa aac agc agc cac agg aga gcc atc ctc atg gag cta gaa cgt     1016
Ile Glu Asn Ser Ser His Arg Arg Ala Ile Leu Met Glu Leu Glu Arg
230                 235                 240                 245

gtc aaa gca tta ggc gtg aag ccc ccc cag aat ctc tgg gaa tat aag     1064
Val Lys Ala Leu Gly Val Lys Pro Pro Gln Asn Leu Trp Glu Tyr Lys
                250                 255                 260

gct gtg aac cca ggc agg tcc ctg ttc ctg cta tac gcc ctc aag agc     1112
Ala Val Asn Pro Gly Arg Ser Leu Phe Leu Leu Tyr Ala Leu Lys Ser
            265                 270                 275

tcc ccc agg ctg agt ctg ctc tac ctg tac ctg ttt gac tac acc gac     1160
Ser Pro Arg Leu Ser Leu Leu Tyr Leu Tyr Leu Phe Asp Tyr Thr Asp
        280                 285                 290

acc ttc cta cct ttc atc cac acc atc tgc cct ctg caa gaa gac agc     1208
Thr Phe Leu Pro Phe Ile His Thr Ile Cys Pro Leu Gln Glu Asp Ser
    295                 300                 305

tct ggg gag gac atc gtc acc aag ctt ctg gat ctt aag gag cct acg     1256
Ser Gly Glu Asp Ile Val Thr Lys Leu Leu Asp Leu Lys Glu Pro Thr
310                 315                 320                 325

tgg aag cag tgg aga gag ttc ctg gtc aaa tac tcc ttc ctt cca tac     1304
Trp Lys Gln Trp Arg Glu Phe Leu Val Lys Tyr Ser Phe Leu Pro Tyr
                330                 335                 340

cag ctg att gct gag ttt gct tgg gac tgg ttg gag gtc cat tac tgg     1352
Gln Leu Ile Ala Glu Phe Ala Trp Asp Trp Leu Glu Val His Tyr Trp
            345                 350                 355

aca tca cgg ttt ctc atc atc aat gct atg tta ctc tca gtt ctg gaa     1400
Thr Ser Arg Phe Leu Ile Ile Asn Ala Met Leu Leu Ser Val Leu Glu
        360                 365                 370

tta ttc tcc ttt tgg aga atc tgg tcg aga agt gaa ctg aag acc gtg     1448
Leu Phe Ser Phe Trp Arg Ile Trp Ser Arg Ser Glu Leu Lys Thr Val
    375                 380                 385

cct cag agg atg tgg agc cat ttc tgg aaa gta tca acg cag ggg ctt     1496
Pro Gln Arg Met Trp Ser His Phe Trp Lys Val Ser Thr Gln Gly Leu
390                 395                 400                 405

ttt gtg gcc atg ttc tgg ccc ctc atc cct cag ttt gtt tgc aac tgt     1544

Phe Val Ala Met Phe Trp Pro Leu Ile Pro Gln Phe Val Cys Asn Cys
                410                 415                 420

ttg ttt tac tgg gcc ctg tac ttt aac cca att att aac att gat ctt      1592
Leu Phe Tyr Trp Ala Leu Tyr Phe Asn Pro Ile Ile Asn Ile Asp Leu
            425                 430                 435

gtg gtc aag gaa ctc cgg cgg ctg gaa acc cag gtg ttg tga              1634
Val Val Lys Glu Leu Arg Arg Leu Glu Thr Gln Val Leu
        440                 445                 450

ctggcactgc ccaggctgag actcttcaag tcccgctgac gtctgagctt tgatgcttaa    1694

gaggggtgag gcagggagcg gacttcctat tttctaccct cagtaaaaca aggtgctgct    1754

ttgtatatca aaagctccaa ccatgtcctc tccccctcag cctgtgggtg gcacgagcaa    1814

ggactgacat ccgcacaggg aggattgtct gtttggctga cacagcagca gcccttccca    1874

cccagccacc ttcctcacag ggactaggag gctcagtccc caacggctgg caagactcag    1934

ggtcctcagt ggacatggtg tgggtgacat cagaagggtg ccacatcagt cccctcccca    1994

acctcagtga ctgacagagg atccggatct cagagcctga gaccaggttt attggggcct    2054

ggcctgtcct ctaagtcaag tttaggaaaa caaggataag attctgtcat aggcatagag    2114

agttgcacat aaaaaatacc gaagaaaacc caaaattcaa tcaacaattc tgtcttattg    2174

aagagttgct aggattcaga gtaaaactca aaggattcag tttgagccta gaatgatggt    2234

tagacttgta gtcactgggc ttttgttttg ctttatggaa atcattgaag gtctggatcc    2294

ctttctctga atggagagat tgagagggat gtcgggcagt tcccattaga tttagtggcc    2354

ttcatgttat tcagaattgt tttggtgata cctcacccct gtaatcccag cactttgggt    2414

gggtgaggca ggcggatcac ttgaagccag gacttcaaga ccagcttggc caacatggtg    2474

aaacctcatc tctactaaaa atacaaaaat tagccaagtg tgatggcaca tacctgtaat    2534

cccagctact tggaattgga aatcgcctga acccaggagg cggaggttgc agggagggag    2594

actgcaccac tgcacttcag cctgggtgac agagggagac tctgtcttaa aaaaaaaaaa    2654

aaaatcatct gtaaaataaa ttccgggata gtcgttttgt tcaaggaaat gttttgtaaa    2714

ttgagctcac actatataat ctttattgtc ctatcctgat gtataataca gcaggtataa    2774

ttacaccaag cgctatagtt ataaatatgg catgaagtga actatggcct tttatttcct    2834

tccagtgtga acacagcagg tgtgagatgt catcttggaa gacaggcctt gcagaaatag    2894

gcctacatcc aaaatattat cttgtgactc catgaaccat tcattaaccc tttgtatctt    2954

tgagtgaaaa ttttactcaa aagttgcatc tggaagttcg aagaaattac ttgaaataaa    3014

aataaagatt tctatataga taaaacctaa aaaaaaaaaa aaaaaa                  3060

<210>  62
<211>  450
<212>  PRT

<213>  Homo sapiens

<400>  62

Met Glu Glu Pro Gln Lys Ser Tyr Val Asn Thr Met Asp Leu Glu Arg
1               5                   10                  15

Asp Glu Pro Leu Lys Ser Thr Gly Pro Gln Ile Ser Val Ser Glu Phe
            20                  25                  30

Ser Cys His Cys Cys Tyr Asp Ile Leu Val Asn Pro Thr Thr Leu Asn
        35                  40                  45

Cys Gly His Ser Phe Cys Arg His Cys Leu Ala Leu Trp Trp Ala Ser
    50                  55                  60

Ser Lys Lys Thr Glu Cys Pro Glu Cys Arg Glu Lys Trp Glu Gly Phe
65                  70                  75                  80

Pro Lys Val Ser Ile Leu Leu Arg Asp Ala Ile Glu Lys Leu Phe Pro
                85                  90                  95

Asp Ala Ile Arg Leu Arg Phe Glu Asp Ile Gln Gln Asn Asn Asp Ile
            100                 105                 110

Val Gln Ser Leu Ala Ala Phe Gln Lys Tyr Gly Asn Asp Gln Ile Pro
        115                 120                 125

Leu Ala Pro Asn Thr Gly Arg Ala Asn Gln Gln Met Gly Gly Gly Phe
    130                 135                 140

Phe Ser Gly Val Leu Thr Ala Leu Thr Gly Val Ala Val Val Leu Leu
145                 150                 155                 160

Val Tyr His Trp Ser Ser Arg Glu Ser Glu His Asp Leu Leu Val His
                165                 170                 175

Lys Ala Val Ala Lys Trp Thr Ala Glu Glu Val Val Leu Trp Leu Glu
            180                 185                 190

Gln Leu Gly Pro Trp Ala Ser Leu Tyr Arg Glu Arg Phe Leu Ser Glu
        195                 200                 205

Arg Val Asn Gly Arg Leu Leu Leu Thr Leu Thr Glu Glu Glu Phe Ser
    210                 215                 220

Lys Thr Pro Tyr Thr Ile Glu Asn Ser Ser His Arg Arg Ala Ile Leu
225                 230                 235                 240

Met Glu Leu Glu Arg Val Lys Ala Leu Gly Val Lys Pro Pro Gln Asn
                245                 250                 255

Leu Trp Glu Tyr Lys Ala Val Asn Pro Gly Arg Ser Leu Phe Leu Leu
            260                 265                 270

Tyr Ala Leu Lys Ser Ser Pro Arg Leu Ser Leu Leu Tyr Leu Tyr Leu
        275                 280                 285

Phe Asp Tyr Thr Asp Thr Phe Leu Pro Phe Ile His Thr Ile Cys Pro
    290                 295                 300

Leu Gln Glu Asp Ser Ser Gly Glu Asp Ile Val Thr Lys Leu Leu Asp
305                 310                 315                 320

Leu Lys Glu Pro Thr Trp Lys Gln Trp Arg Glu Phe Leu Val Lys Tyr
                325                 330                 335

Ser Phe Leu Pro Tyr Gln Leu Ile Ala Glu Phe Ala Trp Asp Trp Leu
            340                 345                 350

Glu Val His Tyr Trp Thr Ser Arg Phe Leu Ile Ile Asn Ala Met Leu
        355                 360                 365

Leu Ser Val Leu Glu Leu Phe Ser Phe Trp Arg Ile Trp Ser Arg Ser
    370                 375                 380

Glu Leu Lys Thr Val Pro Gln Arg Met Trp Ser His Phe Trp Lys Val
385                 390                 395                 400

Ser Thr Gln Gly Leu Phe Val Ala Met Phe Trp Pro Leu Ile Pro Gln
                405                 410                 415

Phe Val Cys Asn Cys Leu Phe Tyr Trp Ala Leu Tyr Phe Asn Pro Ile
            420                 425                 430

Ile Asn Ile Asp Leu Val Val Lys Glu Leu Arg Arg Leu Glu Thr Gln
        435                 440                 445

Val Leu
    450

<210>  63
<211>  10640
<212>  DNA
<213>  Homo sapiens

<220>
<221> CDS
<222> (466)..(1908)

<400> 63
atactcagtc acacaagcca tagcaggaaa cagcgagctt gcagcctcac cgacgagtct 60

caactaaaag ggactcccgg agctaggggt ggggactcgg cctcacacag tgagtgccgg 120

ctattggact tttgtccagt gacagctgag acaacaagga ccacgggagg aggtgtagga 180

gagaagcgcc gcgaacagcg atcgcccagc accaagtccg cttccaggct ttcggtttct 240

ttgcctccat cttgggtgcg ccttcccggc gtctagggga gcgaaggctg aggtggcagc 300

ggcaggagag tccggccgcg acaggacgaa ctcccccact ggaaaggatt ctgaaagaaa 360

tgaagtcagc cctcagaaat gaagttgact gcctgctggc tttctgttga ctggcccgga 420

gctgtactgc aagacccttg tgagcttccc tagtctaaga gtagg atg tct gct gaa 477
Met Ser Ala Glu
1

gtc atc cat cag gtt gaa gaa gca ctt gat aca gat gag aag gag atg 525
Val Ile His Gln Val Glu Glu Ala Leu Asp Thr Asp Glu Lys Glu Met
5                   10                  15                  20

ctg ctc ttt ttg tgc cgg gat gtt gct ata gat gtg gtt cca cct aat 573
Leu Leu Phe Leu Cys Arg Asp Val Ala Ile Asp Val Val Pro Pro Asn
                25                  30                  35

gtc agg gac ctt ctg gat att tta cgg gaa aga ggt aag ctg tct gtc 621
Val Arg Asp Leu Leu Asp Ile Leu Arg Glu Arg Gly Lys Leu Ser Val
            40                  45                  50

ggg gac ttg gct gaa ctg ctc tac aga gtg agg cga ttt gac ctg ctc 669
Gly Asp Leu Ala Glu Leu Leu Tyr Arg Val Arg Arg Phe Asp Leu Leu
        55                  60                  65

aaa cgt atc ttg aag atg gac aga aaa gct gtg gag acc cac ctg ctc 717
Lys Arg Ile Leu Lys Met Asp Arg Lys Ala Val Glu Thr His Leu Leu
    70                  75                  80

agg aac cct cac ctt gtt tcg gac tat aga gtg ctg atg gca gag att 765
Arg Asn Pro His Leu Val Ser Asp Tyr Arg Val Leu Met Ala Glu Ile
85                  90                  95                  100

ggt gag gat ttg gat aaa tct gat gtg tcc tca tta att ttc ctc atg 813
Gly Glu Asp Leu Asp Lys Ser Asp Val Ser Ser Leu Ile Phe Leu Met
                105                 110                 115

aag gat tac atg ggc cga ggc aag ata agc aag gag aag agt ttc ttg 861
Lys Asp Tyr Met Gly Arg Gly Lys Ile Ser Lys Glu Lys Ser Phe Leu
            120                 125                 130

gac ctt gtg gtt gag ttg gag aaa cta aat ctg gtt gcc cca gat caa 909
Asp Leu Val Val Glu Leu Glu Lys Leu Asn Leu Val Ala Pro Asp Gln
        135                 140                 145

ctg gat tta tta gaa aaa tgc cta aag aac atc cac aga ata gac ctg 957
Leu Asp Leu Leu Glu Lys Cys Leu Lys Asn Ile His Arg Ile Asp Leu
    150                 155                 160

aag aca aaa atc cag aag tac aag cag tct gtt caa gga gca ggg aca 1005
Lys Thr Lys Ile Gln Lys Tyr Lys Gln Ser Val Gln Gly Ala Gly Thr
165                 170                 175                 180

agt tac agg aat gtt ctc caa gca gca atc caa aag agt ctc aag gat 1053
Ser Tyr Arg Asn Val Leu Gln Ala Ala Ile Gln Lys Ser Leu Lys Asp
                185                 190                 195

cct tca aat aac ttc agg ctc cat aat ggg aga agt aaa gaa caa aga 1101
Pro Ser Asn Asn Phe Arg Leu His Asn Gly Arg Ser Lys Glu Gln Arg
            200                 205                 210

ctt aag gaa cag ctt ggc gct caa caa gaa cca gtg aag aaa tcc att 1149
Leu Lys Glu Gln Leu Gly Ala Gln Gln Glu Pro Val Lys Lys Ser Ile
        215                 220                 225

cag gaa tca gaa gct ttt ttg cct cag agc ata cct gaa gag aga tac 1197
Gln Glu Ser Glu Ala Phe Leu Pro Gln Ser Ile Pro Glu Glu Arg Tyr
    230                 235                 240

aag atg aag agc aag ccc cta gga atc tgc ctg ata atc gat tgc att 1245
Lys Met Lys Ser Lys Pro Leu Gly Ile Cys Leu Ile Ile Asp Cys Ile
245                 250                 255                 260

ggc aat gag aca gag ctt ctt cga gac acc ttc act tcc ctg ggc tat 1293
Gly Asn Glu Thr Glu Leu Leu Arg Asp Thr Phe Thr Ser Leu Gly Tyr
                265                 270                 275

gaa gtc cag aaa ttc ttg cat ctc agt atg cat ggt ata tcc cag att 1341
Glu Val Gln Lys Phe Leu His Leu Ser Met His Gly Ile Ser Gln Ile
            280                 285                 290

ctt ggc caa ttt gcc tgt atg ccc gag cac cga gac tac gac agc ttt 1389
Leu Gly Gln Phe Ala Cys Met Pro Glu His Arg Asp Tyr Asp Ser Phe
        295                 300                 305

gtg tgt gtc ctg gtg agc cga gga ggc tcc cag agt gtg tat ggt gtg 1437
Val Cys Val Leu Val Ser Arg Gly Gly Ser Gln Ser Val Tyr Gly Val
    310                 315                 320

gat cag act cac tca ggg ctc ccc ctg cat cac atc agg agg atg ttc 1485
Asp Gln Thr His Ser Gly Leu Pro Leu His His Ile Arg Arg Met Phe
325                 330                 335                 340

atg gga gat tca tgc cct tat cta gca ggg aag cca aag atg ttt ttt 1533
Met Gly Asp Ser Cys Pro Tyr Leu Ala Gly Lys Pro Lys Met Phe Phe
                345                 350                 355

att cag aac tat gtg gtg tca gag ggc cag ctg gag gac agc agc ctc 1581
Ile Gln Asn Tyr Val Val Ser Glu Gly Gln Leu Glu Asp Ser Ser Leu
            360                 365                 370

ttg gag gtg gat ggg cca gcg atg aag aat gtg gaa ttc aag gct cag 1629
Leu Glu Val Asp Gly Pro Ala Met Lys Asn Val Glu Phe Lys Ala Gln
        375                 380                 385

aag cga ggg ctg tgc aca gtt cac cga gaa gct gac ttc ttc tgg agc 1677
Lys Arg Gly Leu Cys Thr Val His Arg Glu Ala Asp Phe Phe Trp Ser
    390                 395                 400

ctg tgt act gcg gac atg tcc ctg ctg gag cag tct cac agc tca cca 1725
Leu Cys Thr Ala Asp Met Ser Leu Leu Glu Gln Ser His Ser Ser Pro
405                 410                 415                 420

tcc ctg tac ctg cag tgc ctc tcc cag aaa ctg aga caa gaa aga aaa 1773
Ser Leu Tyr Leu Gln Cys Leu Ser Gln Lys Leu Arg Gln Glu Arg Lys
                425                 430                 435

cgc cca ctc ctg gat ctt cac att gaa ctc aat ggc tac atg tat gat 1821
Arg Pro Leu Leu Asp Leu His Ile Glu Leu Asn Gly Tyr Met Tyr Asp
            440                 445                 450

tgg aac agc aga gtt tct gcc aag gag aaa tat tat gtc tgg ctg cag 1869
Trp Asn Ser Arg Val Ser Ala Lys Glu Lys Tyr Tyr Val Trp Leu Gln
        455                 460                 465

cac act ctg aga aag aaa ctt atc ctc tcc tac aca taa gaaaccaaaa 1918
His Thr Leu Arg Lys Lys Leu Ile Leu Ser Tyr Thr
    470                 475                 480

ggctgggcgt agtggctcac acctgtaatc ccagcacttt gggaggccaa ggagggcaga 1978

tcacttcagg tcaggagttc gagaccagcc tggccaacat ggtaaacgct gtccctagta 2038

aaaatacaaa aattagctgg gtgtgggtgt gggtacctgt attcccagtt acttgggagg 2098

ctgaggtggg aggatctttt gaacccagga gttcagggtc atagcatgct gtgattgtgc 2158

ctacgaatag ccactgcata ccaacctggg caatatagca agatcccatc tctttaaaaa 2218

aaaaaaaaaa ggacaggaac tatcttactc aatgtattag tcatgtttct ctagagggac 2278

agaactaata ggatacatgt atataaaaag gggagtttat taaggagtat tgactcacat 2338

gatcacaggg ttaggtccca caataggtca tctgcaagca aggaagccaa ttcaagtccc 2398

aaagctgaag aacttggagt ccaatgtttg agggcaggaa gcattcagca tgagagaaag 2458

atggaggcca gaagactaca ccagtctagt ctttccatgt tttgcctgct tttattctgg 2518

cagtgctggc agctgattag atggtgccca cccagattga ggatggtctg cctttcccag 2578

tccactgact caaatgttaa atctcctttg gcagcaccct cacagatgta cccgggaaca 2638

ctttgcatcc ttctattcaa tcaagttgat actcagtatt aaccatcaca gtccatttgg 2698

gcaactatac caaattacca tagaccaggt gacttaaaca gcagttattt ctcacagttc 2758

cggaggctgg gaaatccaac atctaagtgg tagcatatct ggtgtctggt aaggcatgct 2818

tccagatctt accagatgtc agtcttttga tgttctcaca tggcagaaaa agaggatgca 2878

aactctcaag tatatcttta agggcacaaa ttccattcat gagggctcta ccctcatcac 2938

ctaattacct cccaaaggcc ccaccttctg atactgtcac tttggggata ctgtctcccc 2998

tttgaattct ggggggaata caaacattca gtttgtaaca atagccttat gatttagagg 3058

ttacttgttc attcacctag acctcaaatt gcattttaca gctagtcaag tatatctttc 3118

tctgatttga tagtgtgacc taaaagggga ccattgtttg aaatatcatt agagttgctt 3178

attattatta ttattattat tattattatt attattatta ttattgagac agagtttcat 3238

tctgctgccc aggctggagt gcagtggcat catcttggct cattgcaacc tctgccttct 3298

gggttcaagc gattctcctg cctcagcctc ccgagtagct gggattacag gctcctgcca 3358

ccacacccgg ctaatttttg tatttttagt ggagacaggg tttccaccat gttggccagc 3418

gtggtcttga actcctgacc tcaggtgatt caccagcctc ggcctcccaa agtgctggga 3478

ttacaggtgt gagccactgc acctggccta ttattatttt taaatttttt ttttttaatt 3538

gatcattctt gggtgtttct cacagagggt gatttggcag ggtcacagga caatagtgga 3598

gggaaggtca gcagataaac aagtgaacaa aggtctctgg ttttcctagg cagaggaccc 3658

tgcggccttc cgcagtgttt gtgtccctgg gtacttgaga ttagggagtg gtgatgactc 3718

ttaaggagca tgctgccttc aagcatctgt ttaacaaagc acatcttgca ctgcccttaa 3778

tccatttaac cctgagtgga cacagcacat gtttcagaga gcacagggtt gggggtaagg 3838

tcatagatca acagcatcct aaggcagaag aatttttctt agtacagaac aaaatgaagt 3898

ctcccatgtc tacttctttc tacacagaca cagcaacaat ctgatttctc tatcttttcc 3958

ccacctttcc ccttttctta ttccacaaaa ccgccatcgt catcatggcc tgttctcaat 4018

gagctgttgg gtacacctcc cagacggggt ggcggctggg cagaggggct cctcacttcc 4078

cagatggggc ggccaggcgg acgcgccccc cacctccctc ccggacggga tagctggccg 4138

ggcgggggct gaccccccac ctccctcccc gacggggcgg ctggccgggc gggggctgac 4198

ccccacgcct ccctcccgga cggggcggct gccaggcgga ggggctcctc acttctcaga 4258

cggggtggct gctgggcgga gacgctcctc acttcccaga cagggtggct gtcgggcgga 4318

ggggctcctc acttctcaga cggggcagct gcgggcggag gggctcctca cttctcagac 4378

ggggtggccg ggcagagaag ctcctcacat cccagacggg ggggcggggc agaggcgctc 4438

cccacatctc agacgatggg cggccgggca gagacgctcc tcacttcatc ccagacgggg 4498

tggcggccgg gcagaagctg taatctcggc accctggggg gccaaggcag gcggctggga 4558

ggcggaggcc gtagccagct gagatcacac cactgcactc cagcctgggc aacattgagc 4618

actgagtgga cgagactctg cccgcaatcc cggcacctcg ggaggccgag gctggcagat 4678

cactcgcagt caggagctgg agaccagccc ggccaacaca gtgaaaccct gtctccacca 4738

aaaaaatacg aaaaccagtc aggcgtggcg gcgcccgcaa tggcaggcac gcggcaggcc 4798

gaggcgggag aatcaggcag ggaggctgca gtgagccgag atggcagcag tacagtccag 4858

cttcggctcg gcatcagagg gagaccgtgg ggagagggag aagagaggga gggggagagg 4918

gctattttta aaatttttta aaattgctga acaggggtac ctctgggcag tgtgtcagaa 4978

taccactttt taaatatttt atgatttatt tatttttcta tttcttgagg ttttaactga 5038

tgtgtatctg tatgtctatt tgtgtatatt ttgtcatgat catgtaacag agtctgaaaa 5098

gtgtcgaaga gacagttttc aggaacaaca agcaattatt cctactttcc aagttatttt 5158

gatgccatgg tggctcatac ctataatctg agtactttgg gaggctgagg tggactgatc 5218

acttgagccc aggagtttga gaccagcctg ggcaacatag caagactcca tctctacaaa 5278

aaaagacaaa atttagctga gcgtggtggc gtgttcctgt agtcccagct acttgggagg 5338

ctgaagtgag tggatcccct gagcccagag aggtcaaggt tgtgatgagc tgtgatcaca 5398

ccactgcact tcagcatggg agacagagtg agaccctgtt tcagaaaaaa taaataaata 5458

aaaccaccag caccacaaac aacaacaaaa agttattttg tacttgtttt gagcacagga 5518

ctcctgaggg tatctttgca tttaatatta cataggggtg ccagtgggaa gtaatgtgta 5578

tgcttggcct catgagctaa aaccctgtgt taattatgac agaaggaaag tgtgtgagag 5638

agatcttaac tacctagcag ctctagctgc catcttgaac catgaagata cgggccacac 5698

gtaggggtag ctgggtagtg agcagcaaga agccttgttg gatgagggca cgaaggagca 5758

gaatcactgg aatcactgtg tcagccctaa ttacctacct ctggactttt atgtgagggg 5818

aaaaaaaatt gacagtttat atttatctca acctagttaa cccaagtgat gcattgttat 5878

gagattaaaa tgtttggagg ccgggtgcgg tggctcacgc ctataatccc agccctttgg 5938

gaggccaagg cgggcggatc acgaggtcag gagatcaaga ccatcctggc taacatgtaa 5998

aaccccgtct ctactaaaaa tacaaaaaat tagccaggcg ttgtggcggt cgcctgtagt 6058

ccctgctatt tgggaggccg aggcaagaga acggcatgaa cctgggaggt ggagcttgca 6118

gcgagctgag atcttgccac tgcactccag cctgggcgac agtgcgagac tctgtctcaa 6178

aaataaataa ataaataaat aataaataaa atgtttggaa tgttggcttc atccctggga 6238

tgcaaggctg gttcaacata cgcaaatcaa gaaacataat tcatcacata aacagaacta 6298

aagacaaaaa ccacatgatt atctcaatag atacagaaaa ggccttcaat aaaattcaac 6358

gttgcttcat gttaaaaact ctcaataaac taggtattga tggaaaatat ctcaaaataa 6418

taaccattta tgacaaaccc acagccatta tcatactgaa tgggcaaaag ctggaagcat 6478

tccccttgaa aactggcaca agacagggat gccgtctcac cactcctatt taacatagta 6538

ttggaagttc tggccaagaa aatcaggcaa gagaaacaaa taaggggtat tcaaatagga 6598

aaagaggaag taaaactgtg tttgcagatg acatgatact atatctagaa aaccccatta 6658

tctccaccca aaagttcctt aagctgataa gcaacttcag caaagtctca ggatacaaaa 6718

tcaatgtgca gaaatcacaa gcattctata caccaacaat acacaagcag agagccaaat 6778

catgaatgaa ctcccattca cagttgctag aaagagaata aaatacctag gaatacagct 6838

aataagatgt gaaggatctc ttcaaggaga actacaaacc actgctcaag gaaataagag 6898

aggacacaaa tgaaaaaaca ttccattctc gtggatagga agaatcaata tcatgaaaat 6958

ggccatacta cccaaagtaa tttataggtt cattgctatt cccattaaac tactattgac 7018

attcttcaca gaattagaaa aaaactactt taaaattcaa atggaaccaa aaaagagccc 7078

gtataaccaa gacaacaata agcaaaaaga acaaagctgg aagcatcaca ctacccaact 7138

tcaaagtata ctgcaaggct acagtagcca aaatggcatg gtactggtac aaaaacagac 7198

acatagacca atggaacaga atagagacca gagaaagaag accacacatc tacagccatc 7258

tgatcatcga caaacctgac aaaaacaagc aatggggaaa agattcccta tttaataaat 7318

ggtgctggga aaactggcta gccatatgca gaaaattgaa actgacccct tccttacacc 7378

ttatacaaaa attaactcaa gattaaagac ttaatgtaaa acctaaaact ataaaaaccc 7438

tagaagaaaa tctatttaat accattcaag acataggcac aagcaaaggt ttcatgacaa 7498

aaacatcaaa agcaattgca acaaaagcaa aaattacaaa tgggatctaa ttaaactaaa 7558

gagctcctgc acagcaaaag aaactatcat tagagtgaac aggcaaccta cagaatggga 7618

gaacattttt gcaatctatc catctgacaa aggtctaata tccagaacct acaaggaact 7678

taaaacaaat ttacaaggaa aaaaacaacc ccatcaaaaa gtggacaaag gacatgaaca 7738

gacacttctc aaaagaagac atttatgtgg ccaacaaaca tataaaaaaa agctcaacct 7798

tactgatcat tagagaaatg caaaggagaa ccacaatgag ataccatctc atgccggtca 7858

gaatggtgat tattaaaaag tcaaaaaaca acagatgctg gcgaggctgt ggagaagtag 7918

gaacactttt acattgttgg tgggaatgta aattagttca accgttgtgg aagtgtgtgt 7978

ggctattcct caaagatcta gaactagaaa tactatttgt cccagcaatc ccattactgg 8038

gtatataccc aaaggaatat aaaccatttt attataaaga tacatgcaca tttttgttca 8098

ttgcagcact cttcacaata gcaaagacac aatagcaaat gcccatcaaa gatagactgg 8158

ataaagaaaa tgtggtacat atacaccatg gaatactgtg cagtgcagcc attacagctt 8218

ttggtgatac agtgaatcag atttttcatt aattctttta attggttatt actgaacgtg 8278

aaaaagtaat gtttgtattg aaatcttgag tctggccatg tttctatttt aaattcataa 8338

agaattctaa caagaggaat tccaagaatg tcataaatgg atgtttctcc atggatgaag 8398

gaactgtttt attcacttgc tgataattca gcctaatcca gtttgacatc atatagataa 8458

gtagttgaat tatggattta aaatacatat catttctaaa ctccaaaggt aatacttatt 8518

taaatggttt tgaaaatata gaaaggcaca atttcttttt aaatctgtta ttctccacca 8578

ccactcaatc tgtctatcat ctatctctcc attcattctt ccatttgttt atatctgtta 8638

atctttgtat gtgttcatgt atagctttta catgattgga atcataatgc atattccatt 8698

ttgaagtctg ctttttttta cacaaaaata tgttgtgaat attttcctat attatgaaat 8758

atcattagct gagcttttag aattgactgc atgttttggt accatttaga tatagtttaa 8818

gatacttaga agttatgtgg ctttgccact atggatgaat cttatttact caatattaat 8878

tacttacaaa taacctcacc taaacactac tcagccataa aaaggaatga attaatgaca 8938

ttcacagcaa cctggagact attactctaa aggaagtaac tgaggaatgg aaaaccaaac 8998

attgtatgtt ctcactcata agtgggagat aagctatgag gatgcaaagg cataagaagg 9058

atacaatgga ctttggggac ttaggggaaa gggtgggagg ggggtgaagg ataaaagaat 9118

acaaattggg ttcagtgtat actgctcagg tgatgggtgc accagaatct cacaagtaac 9178

cacttaatta cttacgcatg taaccagata ccacctgttc cccaaacacc tatggaaata 9238

attttgtttt tttttttaaa aaaggaatga gatcatgtcc tttgcaggga catggatgaa 9298

gctggaagcc attatcctca gcaaactaac agaggagcag gaaaccaaac accacatgtt 9358

ctcacttgta agcggaagct gaacaatgag aacacacgga cacagggatg agatcaacac 9418

acactggggc ctgatgcagg ggccgtagcg gggagagcat caggataact agctaatgca 9478

tgtggggctt aatacctagg tgataggttg ataggtgcag caaaccacca tgggacacgt 9538

ttacctatgt aacaaacccg cacatcctgc acttgtatcc agaacttaaa atattttaaa 9598

aatctttaga gaatacaaaa aaaaaaaaaa agattcttca atgcatacac aataaaattg 9658

cagttcagtc aaacattgga agtctttctc tgactgtcta gttggtatct tcattttcag 9718

cttcttcaag atcccactcc aaacactgtt agctcagcca aattgaacag ctcatatctc 9778

ctacctctgg atctttggtt ctggtgattg tatatttctg gaccatctgg aaccccagca 9838

tatcacccta ccccacatct ccacatcccc aaaatataac catacttcaa gggcagttca 9898

aataccatct ccttctatcc tccatgaagt cagttatctc ttccattgga attatcgccc 9958

cctctcctga acagtactat ttcgtgtgaa tctcctccaa gccttctttt cattttatat 10018

ctcatgctgt aattcttgga aagtatgctg tagctcaagt gcagaattct catcagtttt 10078

atctttatat ctctcctaaa cactttacct gatgaagagc ctggcataca cataaatata 10138

tattgaatga atcagtgatg gattgaaaag agaaatgatg gatctcctaa attttaactt 10198

ttataaaata ttttgataca ttcatgacct tactttagca agcaatgaac gtgatgtaaa 10258

ctattgttga tatagttttt atattggaag tgtaagtagt ttgtggcatg ggattgtgac 10318

atatcctagg tttcctcatc ttctttttat tgaaatgtaa ttcacaagcc ataaaatttg 10378

cccctttaaa gtaaatgatg cagtggattt tagtatattt acagagttgt gcaatcatca 10438

ccactatcta attccagaac atttccatct acctagaaac tccataccag tgagctgcca 10498

ctctaatcct cctcttcccc cagcctctag aaacaataat ccattttctg tctctatgat 10558

ttgcctgttc tagatatttt ataaaaataa acatgtggcc tttcgtgtct gacttccttc 10618

acttaaaaaa aaaaaaaaaa aa 10640

<210> 64
<211> 480
<212> PRT
<213> Homo sapiens

<400>  64

Met Ser Ala Glu Val Ile His Gln Val Glu Glu Ala Leu Asp Thr Asp
1               5                   10                  15

Glu Lys Glu Met Leu Leu Phe Leu Cys Arg Asp Val Ala Ile Asp Val
            20                  25                  30

Val Pro Pro Asn Val Arg Asp Leu Leu Asp Ile Leu Arg Glu Arg Gly
        35                  40                  45

Lys Leu Ser Val Gly Asp Leu Ala Glu Leu Leu Tyr Arg Val Arg Arg
    50                  55                  60

Phe Asp Leu Leu Lys Arg Ile Leu Lys Met Asp Arg Lys Ala Val Glu
65                  70                  75                  80

Thr His Leu Leu Arg Asn Pro His Leu Val Ser Asp Tyr Arg Val Leu
                85                  90                  95

Met Ala Glu Ile Gly Glu Asp Leu Asp Lys Ser Asp Val Ser Ser Leu
            100                 105                 110

Ile Phe Leu Met Lys Asp Tyr Met Gly Arg Gly Lys Ile Ser Lys Glu
        115                 120                 125

Lys Ser Phe Leu Asp Leu Val Val Glu Leu Glu Lys Leu Asn Leu Val
    130                 135                 140

Ala Pro Asp Gln Leu Asp Leu Leu Glu Lys Cys Leu Lys Asn Ile His
145                 150                 155                 160

Arg Ile Asp Leu Lys Thr Lys Ile Gln Lys Tyr Lys Gln Ser Val Gln
                165                 170                 175

Gly Ala Gly Thr Ser Tyr Arg Asn Val Leu Gln Ala Ala Ile Gln Lys
            180                 185                 190

Ser Leu Lys Asp Pro Ser Asn Asn Phe Arg Leu His Asn Gly Arg Ser
        195                 200                 205

Lys Glu Gln Arg Leu Lys Glu Gln Leu Gly Ala Gln Gln Glu Pro Val
    210                 215                 220

Lys Lys Ser Ile Gln Glu Ser Glu Ala Phe Leu Pro Gln Ser Ile Pro
225                 230                 235                 240

Glu Glu Arg Tyr Lys Met Lys Ser Lys Pro Leu Gly Ile Cys Leu Ile

245                         250                         255

Ile Asp Cys Ile Gly Asn Glu Thr Glu Leu Leu Arg Asp Thr Phe Thr
            260                 265                 270

Ser Leu Gly Tyr Glu Val Gln Lys Phe Leu His Leu Ser Met His Gly
        275                 280                 285

Ile Ser Gln Ile Leu Gly Gln Phe Ala Cys Met Pro Glu His Arg Asp
    290                 295                 300

Tyr Asp Ser Phe Val Cys Val Leu Val Ser Arg Gly Gly Ser Gln Ser
305                 310                 315                 320

Val Tyr Gly Val Asp Gln Thr His Ser Gly Leu Pro Leu His His Ile
                325                 330                 335

Arg Arg Met Phe Met Gly Asp Ser Cys Pro Tyr Leu Ala Gly Lys Pro
            340                 345                 350

Lys Met Phe Phe Ile Gln Asn Tyr Val Val Ser Glu Gly Gln Leu Glu
        355                 360                 365

Asp Ser Ser Leu Leu Glu Val Asp Gly Pro Ala Met Lys Asn Val Glu
    370                 375                 380

Phe Lys Ala Gln Lys Arg Gly Leu Cys Thr Val His Arg Glu Ala Asp
385                 390                 395                 400

Phe Phe Trp Ser Leu Cys Thr Ala Asp Met Ser Leu Leu Glu Gln Ser
                405                 410                 415

His Ser Ser Pro Ser Leu Tyr Leu Gln Cys Leu Ser Gln Lys Leu Arg
            420                 425                 430

Gln Glu Arg Lys Arg Pro Leu Leu Asp Leu His Ile Glu Leu Asn Gly
        435                 440                 445

Tyr Met Tyr Asp Trp Asn Ser Arg Val Ser Ala Lys Glu Lys Tyr Tyr
    450                 455                 460

Val Trp Leu Gln His Thr Leu Arg Lys Lys Leu Ile Leu Ser Tyr Thr
465                 470                 475                 480

<210>  65
<211>  822
<212>  DNA
<213>  Homo sapiens

<220>
<221>  CDS
<222>  (56)..(517)

<400>  65
agttccctat cactctcttt aatcactact cacagtaacc tcaactcctg ccaca atg      58
                                                             Met
                                                             1

tac agg atg caa ctc ctg tct tgc att gca cta agt ctt gca ctt gtc      106
Tyr Arg Met Gln Leu Leu Ser Cys Ile Ala Leu Ser Leu Ala Leu Val
            5                   10                  15

aca aac agt gca cct act tca agt tct aca aag aaa aca cag cta caa      154
Thr Asn Ser Ala Pro Thr Ser Ser Ser Thr Lys Lys Thr Gln Leu Gln
        20                  25                  30

ctg gag cat tta ctg ctg gat tta cag atg att ttg aat gga att aat      202
Leu Glu His Leu Leu Leu Asp Leu Gln Met Ile Leu Asn Gly Ile Asn
    35                  40                  45

aat tac aag aat ccc aaa ctc acc agg atg ctc aca ttt aag ttt tac      250
Asn Tyr Lys Asn Pro Lys Leu Thr Arg Met Leu Thr Phe Lys Phe Tyr
50                  55                  60                  65

atg ccc aag aag gcc aca gaa ctg aaa cat ctt cag tgt cta gaa gaa      298
Met Pro Lys Lys Ala Thr Glu Leu Lys His Leu Gln Cys Leu Glu Glu
                70                  75                  80

gaa ctc aaa cct ctg gag gaa gtg cta aat tta gct caa agc aaa aac      346
Glu Leu Lys Pro Leu Glu Glu Val Leu Asn Leu Ala Gln Ser Lys Asn
            85                  90                  95

ttt cac tta aga ccc agg gac tta atc agc aat atc aac gta ata gtt      394
Phe His Leu Arg Pro Arg Asp Leu Ile Ser Asn Ile Asn Val Ile Val
        100                 105                 110

ctg gaa cta aag gga tct gaa aca aca ttc atg tgt gaa tat gct gat      442
Leu Glu Leu Lys Gly Ser Glu Thr Thr Phe Met Cys Glu Tyr Ala Asp
    115                 120                 125

gag aca gca acc att gta gaa ttt ctg aac aga tgg att acc ttt tgt      490
Glu Thr Ala Thr Ile Val Glu Phe Leu Asn Arg Trp Ile Thr Phe Cys
130                 135                 140                 145

caa agc atc atc tca aca ctg act tga taattaagtg cttcccactt            537
Gln Ser Ile Ile Ser Thr Leu Thr
                150

aaaacatatc aggccttcta tttatttaaa tatttaaatt ttatatttat tgttgaatgt    597

atggtttgct acctattgta actattattc ttaatcttaa aactataaat atggatcttt    657

tatgattctt tttgtaagcc ctaggggctc taaaatggtt tcacttattt atcccaaaat    717

atttattatt atgttgaatg ttaaatatag tatctatgta gattggttag taaaactatt    777

taataaattt gataaatata aaaaaaaaaa aaaaaaaaaa aaaaa                    822


<210>  66

<211>  153
<212>  PRT
<213>  Homo sapiens

<400>  66

Met Tyr Arg Met Gln Leu Leu Ser Cys Ile Ala Leu Ser Leu Ala Leu
1               5                   10                  15

Val Thr Asn Ser Ala Pro Thr Ser Ser Ser Thr Lys Lys Thr Gln Leu
            20                  25                  30

Gln Leu Glu His Leu Leu Leu Asp Leu Gln Met Ile Leu Asn Gly Ile
        35                  40                  45

Asn Asn Tyr Lys Asn Pro Lys Leu Thr Arg Met Leu Thr Phe Lys Phe
    50                  55                  60

Tyr Met Pro Lys Lys Ala Thr Glu Leu Lys His Leu Gln Cys Leu Glu
65                  70                  75                  80

Glu Glu Leu Lys Pro Leu Glu Glu Val Leu Asn Leu Ala Gln Ser Lys
                85                  90                  95

Asn Phe His Leu Arg Pro Arg Asp Leu Ile Ser Asn Ile Asn Val Ile
            100                 105                 110

Val Leu Glu Leu Lys Gly Ser Glu Thr Thr Phe Met Cys Glu Tyr Ala
        115                 120                 125

Asp Glu Thr Ala Thr Ile Val Glu Phe Leu Asn Arg Trp Ile Thr Phe
    130                 135                 140

Cys Gln Ser Ile Ile Ser Thr Leu Thr
145                 150

<210>  67
<211>  5046
<212>  DNA
<213>  Homo sapiens

<220>
<221>  CDS
<222>  (259)..(2733)

<400>  67
cgaggctccg tgccccgccc cccgggtgcc ccgccccttt gcgcggctgg cgcggccagc      60

aggccaggct cccctcggca aacctgtcta attggggcgg ggagcggagc ttcctcctct     120

gagggccgtg ccgcgctgcc agatttgttc ttccgcccct gcctccgcgg ctcggaggcg     180

agcggaaggt gccccggggc cgaggcccgt gacggggcgg gcgggagccc cggcagtccg 240

gggtcgccgg cgagggcc atg tcg ctg ttg ggg gac ccg cta cag gcc ctg 291
Met Ser Leu Leu Gly Asp Pro Leu Gln Ala Leu
1               5                   10

ccg ccc tcg gcc gcc ccc acg ggg ccg ctg ctc gcc cct ccg gcc ggc 339
Pro Pro Ser Ala Ala Pro Thr Gly Pro Leu Leu Ala Pro Pro Ala Gly
            15                  20                  25

gcg acc ctc aac cgc ctg cgg gag ccg ctg ctg cgg agg ctc agc gag 387
Ala Thr Leu Asn Arg Leu Arg Glu Pro Leu Leu Arg Arg Leu Ser Glu
        30                  35                  40

ctc ctg gat cag gcg ccc gag ggc cgg ggc tgg agg aga ctg gcg gag 435
Leu Leu Asp Gln Ala Pro Glu Gly Arg Gly Trp Arg Arg Leu Ala Glu
    45                  50                  55

ctg gcg ggg agt cgc ggg cgc ctc cgc ctc agt tgc cta gac ctg gag 483
Leu Ala Gly Ser Arg Gly Arg Leu Arg Leu Ser Cys Leu Asp Leu Glu
60                  65                  70                  75

cag tgt tct ctt aag gta ctg gag cct gaa gga agc ccc agc ctg tgt 531
Gln Cys Ser Leu Lys Val Leu Glu Pro Glu Gly Ser Pro Ser Leu Cys
                80                  85                  90

ctg ctg aag tta atg ggt gaa aaa ggt tgc aca gtc aca gaa ttg agt 579
Leu Leu Lys Leu Met Gly Glu Lys Gly Cys Thr Val Thr Glu Leu Ser
            95                  100                 105

gat ttc ctg cag gct atg gaa cac act gaa gtt ctt cag ctt ctc agc 627
Asp Phe Leu Gln Ala Met Glu His Thr Glu Val Leu Gln Leu Leu Ser
        110                 115                 120

ccc cca gga ata aag att act gta aac cca gag tca aag gca gtc ttg 675
Pro Pro Gly Ile Lys Ile Thr Val Asn Pro Glu Ser Lys Ala Val Leu
    125                 130                 135

gct gga cag ttt gtg aaa ctg tgt tgc cgg gca act gga cat cct ttt 723
Ala Gly Gln Phe Val Lys Leu Cys Cys Arg Ala Thr Gly His Pro Phe
140                 145                 150                 155

gtt caa tat cag tgg ttc aaa atg aat aaa gag att cca aat gga aat 771
Val Gln Tyr Gln Trp Phe Lys Met Asn Lys Glu Ile Pro Asn Gly Asn
                160                 165                 170

aca tca gag ctt att ttt aat gca gtg cat gta aaa gat gca ggc ttt 819
Thr Ser Glu Leu Ile Phe Asn Ala Val His Val Lys Asp Ala Gly Phe
            175                 180                 185

tat gtc tgt cga gtt aat aac aat ttc acc ttt gaa ttc agc cag tgg 867
Tyr Val Cys Arg Val Asn Asn Asn Phe Thr Phe Glu Phe Ser Gln Trp
        190                 195                 200

tca cag ctg gat gtt tgc gac atc cca gag agc ttc cag aga agt gtt 915
Ser Gln Leu Asp Val Cys Asp Ile Pro Glu Ser Phe Gln Arg Ser Val
    205                 210                 215

gat ggc gtc tct gaa tcc aag ttg caa atc tgt gtt gaa cca act tcc 963
Asp Gly Val Ser Glu Ser Lys Leu Gln Ile Cys Val Glu Pro Thr Ser
220                 225                 230                 235

caa aag ctg atg cca ggc agc aca ttg gtt tta cag tgt gtt gct gtt 1011

Gln Lys Leu Met Pro Gly Ser Thr Leu Val Leu Gln Cys Val Ala Val
                240                 245                 250

gga agc cct att cct cac tac cag tgg ttc aaa aat gaa tta cca tta      1059
Gly Ser Pro Ile Pro His Tyr Gln Trp Phe Lys Asn Glu Leu Pro Leu
        255                 260                 265

aca cat gag acc aaa aag cta tac atg gtg cct tat gtg gat ttg gaa      1107
Thr His Glu Thr Lys Lys Leu Tyr Met Val Pro Tyr Val Asp Leu Glu
    270                 275                 280

cac caa gga acc tac tgg tgt cat gta tat aat gat cga gac agt caa      1155
His Gln Gly Thr Tyr Trp Cys His Val Tyr Asn Asp Arg Asp Ser Gln
    285                 290                 295

gat agc aag aag gta gaa atc atc ata gga aga aca gat gag gca gtg      1203
Asp Ser Lys Lys Val Glu Ile Ile Ile Gly Arg Thr Asp Glu Ala Val
300                 305                 310                 315

gag tgc act gaa gat gaa tta aat aat ctt ggt cat cct gat aat aaa      1251
Glu Cys Thr Glu Asp Glu Leu Asn Asn Leu Gly His Pro Asp Asn Lys
                320                 325                 330

gag caa aca act gac cag cct ttg gcg aag gac aag gtt gcc ctt ttg      1299
Glu Gln Thr Thr Asp Gln Pro Leu Ala Lys Asp Lys Val Ala Leu Leu
        335                 340                 345

ata gga aat atg aat tac cgg gag cac ccc aag ctc aaa gct cct ttg      1347
Ile Gly Asn Met Asn Tyr Arg Glu His Pro Lys Leu Lys Ala Pro Leu
    350                 355                 360

gtg gat gtg tac gaa ttg act aac tta ctg aga cag ctg gac ttc aaa      1395
Val Asp Val Tyr Glu Leu Thr Asn Leu Leu Arg Gln Leu Asp Phe Lys
    365                 370                 375

gtg gtt tca ctg ttg gat ctt act gaa tat gag atg cgt aat gct gtg      1443
Val Val Ser Leu Leu Asp Leu Thr Glu Tyr Glu Met Arg Asn Ala Val
380                 385                 390                 395

gat gag ttt tta ctc ctt tta gac aag gga gta tat ggg tta tta tat      1491
Asp Glu Phe Leu Leu Leu Leu Asp Lys Gly Val Tyr Gly Leu Leu Tyr
                400                 405                 410

tat gca gga cat ggt tat gaa aat ttt ggg aac agc ttc atg gtc ccc      1539
Tyr Ala Gly His Gly Tyr Glu Asn Phe Gly Asn Ser Phe Met Val Pro
        415                 420                 425

gtt gat gct cca aat cca tat agg tct gaa aat tgt ctg tgt gta caa      1587
Val Asp Ala Pro Asn Pro Tyr Arg Ser Glu Asn Cys Leu Cys Val Gln
    430                 435                 440

aat ata ctg aaa ttg atg caa gaa aaa gaa act gga ctt aat gtg ttc      1635
Asn Ile Leu Lys Leu Met Gln Glu Lys Glu Thr Gly Leu Asn Val Phe
    445                 450                 455

tta ttg gat atg tgt agg aaa aga aat gac tac gat gat acc att cca      1683
Leu Leu Asp Met Cys Arg Lys Arg Asn Asp Tyr Asp Asp Thr Ile Pro
460                 465                 470                 475

atc ttg gat gca cta aaa gtc acc gcc aat att gtg ttt gga tat gcc      1731
Ile Leu Asp Ala Leu Lys Val Thr Ala Asn Ile Val Phe Gly Tyr Ala
                480                 485                 490

acg tgt caa gga gca gaa gct ttt gaa atc cag cat tct gga ttg gca 1779
Thr Cys Gln Gly Ala Glu Ala Phe Glu Ile Gln His Ser Gly Leu Ala
            495                 500                 505

aat gga atc ttt atg aaa ttt tta aaa gac aga tta tta gaa gat aag 1827
Asn Gly Ile Phe Met Lys Phe Leu Lys Asp Arg Leu Leu Glu Asp Lys
        510                 515                 520

aaa atc act gtg tta ctg gat gaa gtt gca gaa gat atg ggt aag tgt 1875
Lys Ile Thr Val Leu Leu Asp Glu Val Ala Glu Asp Met Gly Lys Cys
    525                 530                 535

cac ctt acc aaa ggc aaa cag gct cta gag att cga agt agt tta tct 1923
His Leu Thr Lys Gly Lys Gln Ala Leu Glu Ile Arg Ser Ser Leu Ser
540                 545                 550                 555

gag aag aga gca ctt act gat cca ata cag gga aca gaa tat tct gct 1971
Glu Lys Arg Ala Leu Thr Asp Pro Ile Gln Gly Thr Glu Tyr Ser Ala
                560                 565                 570

gaa tct ctt gtg cgg aat cta cag tgg gcc aag gct cat gaa ctt cca 2019
Glu Ser Leu Val Arg Asn Leu Gln Trp Ala Lys Ala His Glu Leu Pro
            575                 580                 585

gaa agt atg tgt ctt aag ttt gac tgt ggt gtt cag att caa tta gga 2067
Glu Ser Met Cys Leu Lys Phe Asp Cys Gly Val Gln Ile Gln Leu Gly
        590                 595                 600

ttt gca gct gag ttt tcc aat gtc atg atc atc tat aca agt ata gtt 2115
Phe Ala Ala Glu Phe Ser Asn Val Met Ile Ile Tyr Thr Ser Ile Val
    605                 610                 615

tac aaa cca ccg gag ata ata atg tgt gat gcc tac gtt act gat ttt 2163
Tyr Lys Pro Pro Glu Ile Ile Met Cys Asp Ala Tyr Val Thr Asp Phe
620                 625                 630                 635

cca ctt gat cta gat att gat cca aaa gat gca aat aaa ggc aca cct 2211
Pro Leu Asp Leu Asp Ile Asp Pro Lys Asp Ala Asn Lys Gly Thr Pro
                640                 645                 650

gaa gaa act ggc agc tac ttg gta tca aag gat ctt ccc aag cat tgc 2259
Glu Glu Thr Gly Ser Tyr Leu Val Ser Lys Asp Leu Pro Lys His Cys
            655                 660                 665

ctc tat acc aga ctc agt tca ctg caa aaa tta aag gaa cat cta gtc 2307
Leu Tyr Thr Arg Leu Ser Ser Leu Gln Lys Leu Lys Glu His Leu Val
        670                 675                 680

ttc aca gta tgt tta tca tat cag tac tca gga ttg gaa gat act gta 2355
Phe Thr Val Cys Leu Ser Tyr Gln Tyr Ser Gly Leu Glu Asp Thr Val
    685                 690                 695

gag gac aag cag gaa gtg aat gtt ggg aaa cct ctc att gct aaa tta 2403
Glu Asp Lys Gln Glu Val Asn Val Gly Lys Pro Leu Ile Ala Lys Leu
700                 705                 710                 715

gac atg cat cga ggt ttg gga agg aag act tgc ttt caa act tgt ctt 2451
Asp Met His Arg Gly Leu Gly Arg Lys Thr Cys Phe Gln Thr Cys Leu
                720                 725                 730

atg tct aat ggt cct tac cag agt tct gca gcc acc tca gga gga gca 2499
Met Ser Asn Gly Pro Tyr Gln Ser Ser Ala Ala Thr Ser Gly Gly Ala
            735                 740                 745

ggg cat tat cac tca ttg caa gac cca ttc cat ggt gtt tac cat tca     2547
Gly His Tyr His Ser Leu Gln Asp Pro Phe His Gly Val Tyr His Ser
        750                 755                 760

cat cct ggt aat cca agt aat gtt aca cca gca gat agc tgt cat tgc     2595
His Pro Gly Asn Pro Ser Asn Val Thr Pro Ala Asp Ser Cys His Cys
    765                 770                 775

agc cgg act cca gat gca ttt att tca agt ttc gct cac cat gct tca     2643
Ser Arg Thr Pro Asp Ala Phe Ile Ser Ser Phe Ala His His Ala Ser
780                 785                 790                 795

tgt cat ttt agt aga agt aat gtg cca gta gag aca act gat gaa ata     2691
Cys His Phe Ser Arg Ser Asn Val Pro Val Glu Thr Thr Asp Glu Ile
                800                 805                 810

cca ttt agt ttc tct gac agg ctc aga att tct gaa aaa tga             2733
Pro Phe Ser Phe Ser Asp Arg Leu Arg Ile Ser Glu Lys
            815                 820

cctccttgtt tttgaaagtt agcataattt tagatgcctg tgaaatagta ctgcacttac     2793

ataaagtgag acattgtgaa aaggcaaatt tgtatatgta gagaaagaat agtagtaact     2853

gtttcatagc aaacttcagg actttgagat gttgaaatta cattatttaa ttacagactt     2913

cctctttcta agattttgtg aattggttga atagttctat acaaatgaag tatggaggtg     2973

tgtatgttta tatgtatata acaaaatatt ttcattgtga ccactctgaa gtaagagcaa     3033

tgggaatggc attattgtag aataagtcat tgtattttta acaccagaaa gaaccttgcc     3093

gatcaccagg cataacctaa ttttatccat ggaagaaaca cagaaaggca tctaagttag     3153

agctggcacc agaactgaga cctccagaaa tctattccag tattttttcc actacacaac     3213

tgccttcctg acaggttctg agataagtgt tatgtttgta gatagagtga aatatattta     3273

tatatatata aatatataca gatacatatc tgtgtattat ctcaaggaat gtacaaactt     3333

tagtttttga ttataaggac ttcactgcaa gttttagtta agaggtttgt atataaatct     3393

gttatagaac aggctgaaat ttcttgttca taagattatg aaaccacatg agaagtgata     3453

aaatgtttgt taaagctaga tagaggttaa gaatcaagat ataatggata attttcatag     3513

ctgcctatca gaatttccca aatatttagc atcttccttg ataatatgta ttttcttctt     3573

gaatttcact ggcctaatga gataatactc ttatctttgg ctctacctaa aagttggtta     3633

aaaatgcaat tggcattaac aaggaaaaat actgaattag taattttaaa agtctcacaa     3693

agaaaatccc aggcctagat ggctgcattg ttgaattctg ccaaacatta aaattagcac     3753

taattttttg cacactgttt ctaaaagtag gagaggaaag aacacttccc aacttactct     3813

aggtcagtat taccctgata ctagactaga catcacaaga aaactataag ccaatattcc     3873

ttattaatac aaacacaaaa atcattaaca aaaatattag caaactgaat ccagcaacct     3933

ataaaaagga ttctatatca tgaccaagtg gaatttatcc caggaattca aggttggttc     3993

aacatctaaa aatcaaataa gctaatatac agtcagttct cattattcac agtaattatg 4053

ttctacagaa tattctccca taaacactga attaaatatg gaacaactgc ttttaggaga 4113

aagtgtattt gtgtatatgt gtgtatacat atgtttatct cacacacatt atgagcttga 4173

attcttaatt catcctagca aattctacct tattttacag aagtgaaagt gaggtatcag 4233

aagtgttaag tgacatgcct gaggcacccc cctaacaggt gtcagagctg aaattcaaac 4293

cccatccagc tggccccgga gccagagctt cttgtactac acagaattgc ccctgccatt 4353

tccaccctcc agtcatttct ctatgagact gaagcaggaa ggcagagcat catcttgttc 4413

agcctcagct gggaacatgt gtactgggtg actcaaattt ttcacccatt tacacatatc 4473

cacaaatgac tgcaaaagtg ccacggatat caatttgagg gttataaatt ttagcaagtt 4533

ggtaaattca caaatacata accttgaata atgaggatca actgtaccat atttaataaa 4593

gcacaaaacc cacacagatt gtcttattac agcatttgat aaaatccaaa actctttcat 4653

aaaaacactc aacaaactta ggaataaaag gaatcttcct agatatgata aatataacat 4713

ctatgaaaag cccacaccta acattatact tcatggtgat agactgaagg ctgaatgttt 4773

tccccttaag attgggaaga aggacaagga tgttcactcg gcactacttc tattcagcat 4833

tgtacttgaa gttctagcca cagcagttag gttaggaatt caaggtttgt tcaacatcta 4893

aaaatcaaat aagctaataa agaaaagagg tttatactgc aaaagaagtg aaactatatg 4953

tattcacagt tgatacatag ttgtatatag aaaatgctaa agaatccata aaaagtaata 5013

aatgagttca acaggttaaa aaaaaaaaaa aaa 5046

<210> 68
<211> 824
<212> PRT
<213> Homo sapiens

<400> 68

Met Ser Leu Leu Gly Asp Pro Leu Gln Ala Leu Pro Pro Ser Ala Ala
1               5                   10                  15

Pro Thr Gly Pro Leu Leu Ala Pro Pro Ala Gly Ala Thr Leu Asn Arg
            20                  25                  30

Leu Arg Glu Pro Leu Leu Arg Arg Leu Ser Glu Leu Leu Asp Gln Ala
        35                  40                  45

Pro Glu Gly Arg Gly Trp Arg Arg Leu Ala Glu Leu Ala Gly Ser Arg
    50                  55                  60

Gly Arg Leu Arg Leu Ser Cys Leu Asp Leu Glu Gln Cys Ser Leu Lys
65                  70                  75                  80

Val Leu Glu Pro Glu Gly Ser Pro Ser Leu Cys Leu Leu Lys Leu Met
                85                  90                  95

Gly Glu Lys Gly Cys Thr Val Thr Glu Leu Ser Asp Phe Leu Gln Ala
            100                 105                 110

Met Glu His Thr Glu Val Leu Gln Leu Leu Ser Pro Pro Gly Ile Lys
        115                 120                 125

Ile Thr Val Asn Pro Glu Ser Lys Ala Val Leu Ala Gly Gln Phe Val
    130                 135                 140

Lys Leu Cys Cys Arg Ala Thr Gly His Pro Phe Val Gln Tyr Gln Trp
145                 150                 155                 160

Phe Lys Met Asn Lys Glu Ile Pro Asn Gly Asn Thr Ser Glu Leu Ile
                165                 170                 175

Phe Asn Ala Val His Val Lys Asp Ala Gly Phe Tyr Val Cys Arg Val
            180                 185                 190

Asn Asn Asn Phe Thr Phe Glu Phe Ser Gln Trp Ser Gln Leu Asp Val
        195                 200                 205

Cys Asp Ile Pro Glu Ser Phe Gln Arg Ser Val Asp Gly Val Ser Glu
    210                 215                 220

Ser Lys Leu Gln Ile Cys Val Glu Pro Thr Ser Gln Lys Leu Met Pro
225                 230                 235                 240

Gly Ser Thr Leu Val Leu Gln Cys Val Ala Val Gly Ser Pro Ile Pro
                245                 250                 255

His Tyr Gln Trp Phe Lys Asn Glu Leu Pro Leu Thr His Glu Thr Lys
            260                 265                 270

Lys Leu Tyr Met Val Pro Tyr Val Asp Leu Glu His Gln Gly Thr Tyr
        275                 280                 285

Trp Cys His Val Tyr Asn Asp Arg Asp Ser Gln Asp Ser Lys Lys Val
    290                 295                 300

Glu Ile Ile Ile Gly Arg Thr Asp Glu Ala Val Glu Cys Thr Glu Asp
305                 310                 315                 320

Glu Leu Asn Asn Leu Gly His Pro Asp Asn Lys Glu Gln Thr Thr Asp
                325                 330                 335

Gln Pro Leu Ala Lys Asp Lys Val Ala Leu Leu Ile Gly Asn Met Asn
            340                 345                 350

Tyr Arg Glu His Pro Lys Leu Lys Ala Pro Leu Val Asp Val Tyr Glu
        355                 360                 365

Leu Thr Asn Leu Leu Arg Gln Leu Asp Phe Lys Val Val Ser Leu Leu
    370                 375                 380

Asp Leu Thr Glu Tyr Glu Met Arg Asn Ala Val Asp Glu Phe Leu Leu
385                 390                 395                 400

Leu Leu Asp Lys Gly Val Tyr Gly Leu Leu Tyr Tyr Ala Gly His Gly
                405                 410                 415

Tyr Glu Asn Phe Gly Asn Ser Phe Met Val Pro Val Asp Ala Pro Asn
            420                 425                 430

Pro Tyr Arg Ser Glu Asn Cys Leu Cys Val Gln Asn Ile Leu Lys Leu
        435                 440                 445

Met Gln Glu Lys Glu Thr Gly Leu Asn Val Phe Leu Leu Asp Met Cys
    450                 455                 460

Arg Lys Arg Asn Asp Tyr Asp Asp Thr Ile Pro Ile Leu Asp Ala Leu
465                 470                 475                 480

Lys Val Thr Ala Asn Ile Val Phe Gly Tyr Ala Thr Cys Gln Gly Ala
                485                 490                 495

Glu Ala Phe Glu Ile Gln His Ser Gly Leu Ala Asn Gly Ile Phe Met
            500                 505                 510

Lys Phe Leu Lys Asp Arg Leu Leu Glu Asp Lys Lys Ile Thr Val Leu
        515                 520                 525

Leu Asp Glu Val Ala Glu Asp Met Gly Lys Cys His Leu Thr Lys Gly
    530                 535                 540

Lys Gln Ala Leu Glu Ile Arg Ser Ser Leu Ser Glu Lys Arg Ala Leu
545                 550                 555                 560

Thr Asp Pro Ile Gln Gly Thr Glu Tyr Ser Ala Glu Ser Leu Val Arg
                565                 570                 575

Asn Leu Gln Trp Ala Lys Ala His Glu Leu Pro Glu Ser Met Cys Leu

580                 585                 590

Lys Phe Asp Cys Gly Val Gln Ile Gln Leu Gly Phe Ala Ala Glu Phe
        595                 600                 605

Ser Asn Val Met Ile Ile Tyr Thr Ser Ile Val Tyr Lys Pro Pro Glu
    610                 615                 620

Ile Ile Met Cys Asp Ala Tyr Val Thr Asp Phe Pro Leu Asp Leu Asp
625                 630                 635                 640

Ile Asp Pro Lys Asp Ala Asn Lys Gly Thr Pro Glu Glu Thr Gly Ser
                645                 650                 655

Tyr Leu Val Ser Lys Asp Leu Pro Lys His Cys Leu Tyr Thr Arg Leu
            660                 665                 670

Ser Ser Leu Gln Lys Leu Lys Glu His Leu Val Phe Thr Val Cys Leu
        675                 680                 685

Ser Tyr Gln Tyr Ser Gly Leu Glu Asp Thr Val Glu Asp Lys Gln Glu
    690                 695                 700

Val Asn Val Gly Lys Pro Leu Ile Ala Lys Leu Asp Met His Arg Gly
705                 710                 715                 720

Leu Gly Arg Lys Thr Cys Phe Gln Thr Cys Leu Met Ser Asn Gly Pro
                725                 730                 735

Tyr Gln Ser Ser Ala Ala Thr Ser Gly Gly Ala Gly His Tyr His Ser
            740                 745                 750

Leu Gln Asp Pro Phe His Gly Val Tyr His Ser His Pro Gly Asn Pro
        755                 760                 765

Ser Asn Val Thr Pro Ala Asp Ser Cys His Cys Ser Arg Thr Pro Asp
    770                 775                 780

Ala Phe Ile Ser Ser Phe Ala His His Ala Ser Cys His Phe Ser Arg
785                 790                 795                 800

Ser Asn Val Pro Val Glu Thr Thr Asp Glu Ile Pro Phe Ser Phe Ser
                805                 810                 815

Asp Arg Leu Arg Ile Ser Glu Lys
            820

<210>  69
<211>  4107
<212>  DNA
<213>  Homo sapiens

<220>
<221>  CDS
<222>  (209)..(1261)

<400>  69
gcgcaaccct ccggaagctg ccgccccttt ccccttttat gggaatactt tttttaaaaa      60

aaaagagttc gctggcgcca ccccgtagga ctggccgccc taaaaccgtg ataaaggagc      120

tgctcgccac ttctcacttc cgcttccttc cagtaaggag tcggggtctt ccccagtttt      180

ctcagccagg cggcggcggc gactggca atg ttt ggc ctc aaa aga aac gcg      232
                               Met Phe Gly Leu Lys Arg Asn Ala
                               1               5

gta atc gga ctc aac ctc tac tgt ggg ggg gcc ggc ttg ggg gcc ggc      280
Val Ile Gly Leu Asn Leu Tyr Cys Gly Gly Ala Gly Leu Gly Ala Gly
    10                  15                  20

agc ggc ggc gcc acc cgc ccg gga ggg cga ctt ttg gct acg gag aag      328
Ser Gly Gly Ala Thr Arg Pro Gly Gly Arg Leu Leu Ala Thr Glu Lys
25                  30                  35                  40

gag gcc tcg gcc cgg cga gag ata ggg gga ggg gag gcc ggc gcg gtg      376
Glu Ala Ser Ala Arg Arg Glu Ile Gly Gly Gly Glu Ala Gly Ala Val
                45                  50                  55

att ggc gga agc gcc ggc gca agc ccc ccg tcc acc ctc acg cca gac      424
Ile Gly Gly Ser Ala Gly Ala Ser Pro Pro Ser Thr Leu Thr Pro Asp
            60                  65                  70

tcc cgg agg gtc gcg cgg ccg ccg ccc att ggc gcc gag gtc ccc gac      472
Ser Arg Arg Val Ala Arg Pro Pro Pro Ile Gly Ala Glu Val Pro Asp
        75                  80                  85

gtc acc gcg acc ccc gcg agg ctg ctt ttc ttc gcg ccc acc cgc cgc      520
Val Thr Ala Thr Pro Ala Arg Leu Leu Phe Phe Ala Pro Thr Arg Arg
    90                  95                  100

gcg gcg ccg ctt gag gag atg gaa gcc ccg gcc gct gac gcc atc atg      568
Ala Ala Pro Leu Glu Glu Met Glu Ala Pro Ala Ala Asp Ala Ile Met
105                 110                 115                 120

tcg ccc gaa gag gag ctg gac ggg tac gag ccg gag cct ctc ggg aag      616
Ser Pro Glu Glu Glu Leu Asp Gly Tyr Glu Pro Glu Pro Leu Gly Lys
                125                 130                 135

cgg ccg gct gtc ctg ccg ctg ctg gag ttg gtc ggg gaa tct ggt aat      664
Arg Pro Ala Val Leu Pro Leu Leu Glu Leu Val Gly Glu Ser Gly Asn
            140                 145                 150

aac acc agt acg gac ggg tca cta ccc tcg acg ccg ccg cca gca gag      712
Asn Thr Ser Thr Asp Gly Ser Leu Pro Ser Thr Pro Pro Pro Ala Glu
        155                 160                 165

gag gag gag gac gag ttg tac cgg cag tcg ctg gag att atc tct cgg      760
Glu Glu Glu Asp Glu Leu Tyr Arg Gln Ser Leu Glu Ile Ile Ser Arg

170                 175                 180

tac ctt cgg gag cag gcc acc ggc gcc aag gac aca aag cca atg ggc      808
Tyr Leu Arg Glu Gln Ala Thr Gly Ala Lys Asp Thr Lys Pro Met Gly
185                 190                 195                 200

agg tct ggg gcc acc agc agg aag gcg ctg gag acc tta cga cgg gtt      856
Arg Ser Gly Ala Thr Ser Arg Lys Ala Leu Glu Thr Leu Arg Arg Val
                205                 210                 215

ggg gat ggc gtg cag cgc aac cac gag acg gcc ttc caa ggc atg ctt      904
Gly Asp Gly Val Gln Arg Asn His Glu Thr Ala Phe Gln Gly Met Leu
            220                 225                 230

cgg aaa ctg gac atc aaa aac gaa gac gat gtg aaa tcg ttg tct cga      952
Arg Lys Leu Asp Ile Lys Asn Glu Asp Asp Val Lys Ser Leu Ser Arg
        235                 240                 245

gtg atg atc cat gtt ttc agc gac ggc gta aca aac tgg ggc agg att     1000
Val Met Ile His Val Phe Ser Asp Gly Val Thr Asn Trp Gly Arg Ile
    250                 255                 260

gtg act ctc att tct ttt ggt gcc ttt gtg gct aaa cac ttg aag acc     1048
Val Thr Leu Ile Ser Phe Gly Ala Phe Val Ala Lys His Leu Lys Thr
265                 270                 275                 280

ata aac caa gaa agc tgc atc gaa cca tta gca gaa agt atc aca gac     1096
Ile Asn Gln Glu Ser Cys Ile Glu Pro Leu Ala Glu Ser Ile Thr Asp
                285                 290                 295

gtt ctc gta agg aca aaa cgg gac tgg cta gtt aaa caa aga ggc tgg     1144
Val Leu Val Arg Thr Lys Arg Asp Trp Leu Val Lys Gln Arg Gly Trp
            300                 305                 310

gat ggg ttt gtg gag ttc ttc cat gta gag gac cta gaa ggt ggc atc     1192
Asp Gly Phe Val Glu Phe Phe His Val Glu Asp Leu Glu Gly Gly Ile
        315                 320                 325

agg aat gtg ctg ctg gct ttt gca ggt gtt gct gga gta gga gct ggt     1240
Arg Asn Val Leu Leu Ala Phe Ala Gly Val Ala Gly Val Gly Ala Gly
    330                 335                 340

ttg gca tat cta ata aga tag ccttactgta agtgcaatag ttgactttta        1291
Leu Ala Tyr Leu Ile Arg
345                 350

accaaccacc accaccacca aaaccagttt atgcagttgg actccaagct gtaacttcct   1351

agagttgcac cctagcaacc tagccagaaa agcaagtggc aagaggatta tggctaacaa   1411

gaataaatac atgggaagag tgctccccat tgattgaaga gtcactgtct gaaagaagca   1471

aagttcagtt tcagcaacaa acaaactttg tttgggaagc tatggaggag gacttttaga    1531

tttagtgaag atggtagggt ggaaagactt aatttccttg ttgagaacag gaaagtggcc   1591

agtagccagg caagtcatag aattgattac ccgccgaatt cattaattta ctgtagtgtt   1651

aagagaagca ctaagaatgc cagtgacctg tgtaaaagtt acaagtaata gaactatgac   1711

tgtaagcctc agtactgtac aagggaagct tttcctctct ctaattagct ttcccagtat   1771

acttcttaga aagtccaagt gttcaggact tttatacctg ttatactttg gcttggtttc   1831

catgattctt actttattag cctagtttat caccaataat acttgacgga aggctcagta 1891

attagttatg aatatggata tcctcaattc ttaagacagc ttgtaaatgt atttgtaaaa 1951

attgtatata tttttacaga aagtctattt ctttgaaacg aaggaagtat cgaatttaca 2011

ttagtttttt tcataccctt ttgaactttg caacttccgt aattaggaac ctgtttctta 2071

cagcttttct atgctaaact ttgttctgtt cagttctaga gtgtatacag aacgaattga 2131

tgtgtaactg tatgcagact ggttgtagtg gaacaaatct gataactatg caggtttaaa 2191

ttttcttatc tgattttggt aagtattcct tagataggtt tttctttgaa aacctgggat 2251

tgagaggttg atgaatggaa attctttcac ttcattatat gcaagttttc aataattagg 2311

tctaagtgga gttttaaggt tactgatgac ttacaaataa tgggctctga ttgggcaata 2371

ctcatttgag ttccttccat ttgacctaat ttaactggtg aaatttaaag tgaattcatg 2431

ggctcatctt taaagctttt actaaaagat tttcagctga atggaactca ttagctgtgt 2491

gcatataaaa agatcacatc aggtggatgg agagacattt gatcccttgt ttgcttaata 2551

aattataaaa tgatggcttg gaaaagcagg ctagtctaac catggtgcta ttattaggct 2611

tgcttgttac acacacaggt ctaagcctag tatgtcaata aagcaaatac ttactgtttt 2671

gtttctatta atgattccca aaccttgttg caagtttttg cattggcatc tttggatttc 2731

agtcttgatg tttgttctat cagacttaac cttttatttc ctgtccttcc ttgaaattgc 2791

tgattgttct gctccctcta cagatattta tatcaattcc tacagctttc ccctgccatc 2851

cctgaactct ttctagccct tttagatttt ggcactgtga aacccctgct ggaaacctga 2911

gtgaccctcc ctccccacca agagtccaca gacctttcat ctttcacgaa cttgatcctg 2971

ttagcaggtg gtaataccat gggtgctgtg acactaacag tcattgagag gtgggaggaa 3031

gtcccttttc cttggactgg tatcttttca actattgttt tatcctgtct ttgggggcaa 3091

tgtgtcaaaa gtcccctcag gaattttcag aggaaagaac attttatgag gctttctcta 3151

aagtttcctt tgtataggag tatgctcact taaatttaca gaaagaggtg agctgtgtta 3211

aacctcagag tttaaaagct actgataaac tgaagaaagt gtctatattg gaactagggt 3271

catttgaaag cttcagtctc ggaacatgac ctttagtctg tggactccat ttaaaaatag 3331

gtatgaataa gatgactaag aatgtaatgg ggaagaactg ccctgcctgc ccatctcaga 3391

gccataaggt catctttgct agagctattt ttacctatgt atttatcgtt cttgatcata 3451

agccgcttat ttatatcatg tatctctaag gacctaaaag cactttatgt agtttttaat 3511

taatcttaag atctggttac ggtaactaaa aaagcctgtc tgccaaatcc agtggaaaca 3571

agtgcataga tgtgaattgg tttttagggg ccccacttcc caattcatta ggtatgactg 3631

tggaaataca gacaaggatc ttagttgata ttttgggctt ggggcagtga gggcttagga 3691

caccccaagt ggtttgggaa aggaggaggg gagtggtggg tttatagggg gaggaggagg 3751

caggtggtct aagtgctgac tggctacgta gttcgggcaa atcctccaaa agggaaaggg 3811

aggatttgct tagaaggatg gcgctcccag tgactacttt ttgacttctg tttgtcttac 3871

gcttctctca gggaaaaaca tgcagtcctc tagtgtttca tgtacattct gtggggggtg 3931

aacaccttgg ttctggttaa acagctgtac ttttgatagc tgtgccagga agggttagga 3991

ccaactacaa attaatgttg gttgtcaaat gtagtgtgtt tccctaactt tctgtttttc 4051

ctgagaaaaa aaaataaatc ttttattcaa atacagggaa aaaaaaaaaa aaaaaa 4107

<210> 70
<211> 350
<212> PRT
<213> Homo sapiens

<400> 70

Met Phe Gly Leu Lys Arg Asn Ala Val Ile Gly Leu Asn Leu Tyr Cys
1               5                   10                  15

Gly Gly Ala Gly Leu Gly Ala Gly Ser Gly Gly Ala Thr Arg Pro Gly
            20                  25                  30

Gly Arg Leu Leu Ala Thr Glu Lys Glu Ala Ser Ala Arg Arg Glu Ile
        35                  40                  45

Gly Gly Gly Glu Ala Gly Ala Val Ile Gly Gly Ser Ala Gly Ala Ser
    50                  55                  60

Pro Pro Ser Thr Leu Thr Pro Asp Ser Arg Arg Val Ala Arg Pro Pro
65                  70                  75                  80

Pro Ile Gly Ala Glu Val Pro Asp Val Thr Ala Thr Pro Ala Arg Leu
                85                  90                  95

Leu Phe Phe Ala Pro Thr Arg Arg Ala Ala Pro Leu Glu Glu Met Glu
            100                 105                 110

Ala Pro Ala Ala Asp Ala Ile Met Ser Pro Glu Glu Glu Leu Asp Gly
        115                 120                 125

Tyr Glu Pro Glu Pro Leu Gly Lys Arg Pro Ala Val Leu Pro Leu Leu
    130                 135                 140

Glu Leu Val Gly Glu Ser Gly Asn Asn Thr Ser Thr Asp Gly Ser Leu
145                 150                 155                 160

Pro Ser Thr Pro Pro Pro Ala Glu Glu Glu Glu Asp Glu Leu Tyr Arg

165                 170                 175

Gln Ser Leu Glu Ile Ile Ser Arg Tyr Leu Arg Glu Gln Ala Thr Gly
            180                 185                 190

Ala Lys Asp Thr Lys Pro Met Gly Arg Ser Gly Ala Thr Ser Arg Lys
        195                 200                 205

Ala Leu Glu Thr Leu Arg Arg Val Gly Asp Gly Val Gln Arg Asn His
    210                 215                 220

Glu Thr Ala Phe Gln Gly Met Leu Arg Lys Leu Asp Ile Lys Asn Glu
225                 230                 235                 240

Asp Asp Val Lys Ser Leu Ser Arg Val Met Ile His Val Phe Ser Asp
                245                 250                 255

Gly Val Thr Asn Trp Gly Arg Ile Val Thr Leu Ile Ser Phe Gly Ala
            260                 265                 270

Phe Val Ala Lys His Leu Lys Thr Ile Asn Gln Glu Ser Cys Ile Glu
        275                 280                 285

Pro Leu Ala Glu Ser Ile Thr Asp Val Leu Val Arg Thr Lys Arg Asp
    290                 295                 300

Trp Leu Val Lys Gln Arg Gly Trp Asp Gly Phe Val Glu Phe Phe His
305                 310                 315                 320

Val Glu Asp Leu Glu Gly Gly Ile Arg Asn Val Leu Leu Ala Phe Ala
                325                 330                 335

Gly Val Ala Gly Val Gly Ala Gly Leu Ala Tyr Leu Ile Arg
            340                 345                 350

<210>  71
<211>  4560
<212>  DNA
<213>  Homo sapiens

<220>
<221>  CDS
<222>  (626)..(3022)

<400>  71
gggcccgcag cttccgtggc tgagaggagc cggcggcggc agtgtctgtg gtggccgcgg      60

cggcgagagc gacggtccca tctttcccac cctcttggtt gccgctggcc acacgccctc     120

cgctggcggc gacttctcag ctccgtgcgc ccgggctgga cagtgagcct cgagaggaga     180

cgcgggcggc tagagccgga gtggggcgag ccgcggaacc cggccgggag ccgcgcgagg 240

cgtgatcgga gggtatggtt ggcatggaat tgaatttcat ctgtctgtgg gaattgtaag 300

caagattgcc atcacgaaag ccaaagtgga tttctccagt gtggtgtgcc tgcccccttc 360

cgtcattgct gtgaatgggc tggacggagg aggggccggc gaaaatgatg atgaaccagt 420

gctcgtgtcc ttatctgcgg cacccagtcc ccagagtgaa gctgttgcca atgaactgca 480

ggagctctcc ttgcagcccg agctgaccct aggcctccac cctggcagga atcccaattt 540

gcctccactt agtgagcgga agaatgtgct acagttgaaa ctccagcagc gccggacccg 600

ggaagaactg gtgagccaag ggatc atg ccg cct ttg aaa agt cca gcc gca 652
Met Pro Pro Leu Lys Ser Pro Ala Ala
1               5

ttt cat gag cag aga agg agc ttg gag cgg gcc agg aca gag gac tat 700
Phe His Glu Gln Arg Arg Ser Leu Glu Arg Ala Arg Thr Glu Asp Tyr
10                  15                  20                  25

ctc aaa cgg aag att cgt tcc cgg ccg gag aga tcg gag ctg gtc agg 748
Leu Lys Arg Lys Ile Arg Ser Arg Pro Glu Arg Ser Glu Leu Val Arg
                30                  35                  40

atg cac att ttg gaa gag acc tcg gct gag cca tcc ctc cag gcc aag 796
Met His Ile Leu Glu Glu Thr Ser Ala Glu Pro Ser Leu Gln Ala Lys
            45                  50                  55

cag ctg aag ctg aag aga gcc aga cta gcc gat gac ctc aat gag aag 844
Gln Leu Lys Leu Lys Arg Ala Arg Leu Ala Asp Asp Leu Asn Glu Lys
        60                  65                  70

att gca cag agg cct ggc ccc atg gag ctg gtg gag aag aac atc ctt 892
Ile Ala Gln Arg Pro Gly Pro Met Glu Leu Val Glu Lys Asn Ile Leu
    75                  80                  85

cct gtt gag tcc agc ctg aag gaa gcc atc att gtg ggc cag gtg aac 940
Pro Val Glu Ser Ser Leu Lys Glu Ala Ile Ile Val Gly Gln Val Asn
90                  95                  100                 105

tat ccc aaa gta gca gac agc tct tcc ttc gat gag gac agc agc gat 988
Tyr Pro Lys Val Ala Asp Ser Ser Ser Phe Asp Glu Asp Ser Ser Asp
                110                 115                 120

gcc tta tcc ccc gag cag cct gcc agc cat gag tcc cag ggt tct gtg 1036
Ala Leu Ser Pro Glu Gln Pro Ala Ser His Glu Ser Gln Gly Ser Val
            125                 130                 135

ccg tca ccc ctg gag gcc cga gtc agc gaa cca ctg ctc agt gcc acc 1084
Pro Ser Pro Leu Glu Ala Arg Val Ser Glu Pro Leu Leu Ser Ala Thr
        140                 145                 150

tct gca tcc ccc acc cag gtt gtg tct caa ctt ccg atg ggc cgg gat 1132
Ser Ala Ser Pro Thr Gln Val Val Ser Gln Leu Pro Met Gly Arg Asp
    155                 160                 165

tcc aga gaa atg ctt ttc ctg gca gag cag cct cct ctg cct ccc cca 1180
Ser Arg Glu Met Leu Phe Leu Ala Glu Gln Pro Pro Leu Pro Pro Pro
170                 175                 180                 185

cct ctg ctg cct ccc agc ctc acc aat gga acc act atc ccc act gcc 1228
Pro Leu Leu Pro Pro Ser Leu Thr Asn Gly Thr Thr Ile Pro Thr Ala
                190                 195                 200

aag tcc acc ccc aca ctc att aag caa agc caa ccc aag tct gcc agt 1276
Lys Ser Thr Pro Thr Leu Ile Lys Gln Ser Gln Pro Lys Ser Ala Ser
            205                 210                 215

gag aag tca cag cgc agc aag aag gcc aag gag ctg aag cca aag gtg 1324
Glu Lys Ser Gln Arg Ser Lys Lys Ala Lys Glu Leu Lys Pro Lys Val
        220                 225                 230

aag aag ctc aag tac cac cag tac atc ccc ccg gac cag aag cag gac 1372
Lys Lys Leu Lys Tyr His Gln Tyr Ile Pro Pro Asp Gln Lys Gln Asp
    235                 240                 245

agg ggg gca ccc ccc atg gac tca tcc tac gcc aag atc ctg cag cag 1420
Arg Gly Ala Pro Pro Met Asp Ser Ser Tyr Ala Lys Ile Leu Gln Gln
250                 255                 260                 265

cag cag ctc ttc ctc cag ctg cag atc ctc aac cag cag cag cag cag 1468
Gln Gln Leu Phe Leu Gln Leu Gln Ile Leu Asn Gln Gln Gln Gln Gln
                270                 275                 280

cac cac aac tac cag gcc atc ctg cct gcc ccg cca aag tca gca ggc 1516
His His Asn Tyr Gln Ala Ile Leu Pro Ala Pro Pro Lys Ser Ala Gly
            285                 290                 295

gag gcc ctg gga agc agc ggg acc ccc cca gta cgc agc ctc tcc act 1564
Glu Ala Leu Gly Ser Ser Gly Thr Pro Pro Val Arg Ser Leu Ser Thr
        300                 305                 310

acc aat agc agc tcc agc tcg ggc gcc cct ggg ccc tgt ggg ctg gca 1612
Thr Asn Ser Ser Ser Ser Ser Gly Ala Pro Gly Pro Cys Gly Leu Ala
    315                 320                 325

cgt cag aac agc acc tca ctg act ggc aag ccg gga gcc ctg ccg gcc 1660
Arg Gln Asn Ser Thr Ser Leu Thr Gly Lys Pro Gly Ala Leu Pro Ala
330                 335                 340                 345

aac ctg gac gac atg aag gtg gca gag ctg aag cag gag ctg aag ttg 1708
Asn Leu Asp Asp Met Lys Val Ala Glu Leu Lys Gln Glu Leu Lys Leu
                350                 355                 360

cga tca ctg cct gtc tcg ggc acc aaa act gag ctg att gag cgc ctt 1756
Arg Ser Leu Pro Val Ser Gly Thr Lys Thr Glu Leu Ile Glu Arg Leu
            365                 370                 375

cga gcc tat caa gac caa atc agc cct gtg cca gga gcc ccc aag gcc 1804
Arg Ala Tyr Gln Asp Gln Ile Ser Pro Val Pro Gly Ala Pro Lys Ala
        380                 385                 390

cct gcc gcc acc tct atc ctg cac aag gct ggc gag gtg gtg gta gcc 1852
Pro Ala Ala Thr Ser Ile Leu His Lys Ala Gly Glu Val Val Val Ala
    395                 400                 405

ttc cca gcg gcc cgg ctg agc acg ggg cca gcc ctg gtg gca gca ggc 1900
Phe Pro Ala Ala Arg Leu Ser Thr Gly Pro Ala Leu Val Ala Ala Gly
410                 415                 420                 425

ctg gct cca gct gag gtg gtg gtg gcc acg gtg gcc agc agt ggg gtg 1948
Leu Ala Pro Ala Glu Val Val Val Ala Thr Val Ala Ser Ser Gly Val
                430                 435                 440

gtg aag ttt ggc agc acg ggc tcc acg ccc ccc gtg tct ccc acc ccc 1996
Val Lys Phe Gly Ser Thr Gly Ser Thr Pro Pro Val Ser Pro Thr Pro
            445                 450                 455

tcg gag cgc tca ctg ctc agc acg ggc gat gaa aac tcc acc ccc ggg 2044
Ser Glu Arg Ser Leu Leu Ser Thr Gly Asp Glu Asn Ser Thr Pro Gly
        460                 465                 470

gac acc ttt ggt gag atg gtg aca tca cct ctg acg cag ctg acc ctg 2092
Asp Thr Phe Gly Glu Met Val Thr Ser Pro Leu Thr Gln Leu Thr Leu
    475                 480                 485

cag gcc tcg cca ctg cag atc ctc gtg aag gag gag ggc ccc cgg gcc 2140
Gln Ala Ser Pro Leu Gln Ile Leu Val Lys Glu Glu Gly Pro Arg Ala
490                 495                 500                 505

ggg tcc tgt tgc ctg agc cct ggg ggg cgg gcg gag cta gag ggg cgc 2188
Gly Ser Cys Cys Leu Ser Pro Gly Gly Arg Ala Glu Leu Glu Gly Arg
                510                 515                 520

gac aag gac cag atg ctg cag gag aaa gac aag cag atc gag gcg ctg 2236
Asp Lys Asp Gln Met Leu Gln Glu Lys Asp Lys Gln Ile Glu Ala Leu
            525                 530                 535

acg cgc atg ctc cgg cag aag cag cag ctg gtg gag cgg ctc aag ctg 2284
Thr Arg Met Leu Arg Gln Lys Gln Gln Leu Val Glu Arg Leu Lys Leu
        540                 545                 550

cag ctg gag cag gag aag cga gcc cag cag ccc gcc ccc gcc ccc gcc 2332
Gln Leu Glu Gln Glu Lys Arg Ala Gln Gln Pro Ala Pro Ala Pro Ala
    555                 560                 565

ccc ctc ggc acc ccc gtg aag cag gag aac agc ttc tcc agc tgc cag 2380
Pro Leu Gly Thr Pro Val Lys Gln Glu Asn Ser Phe Ser Ser Cys Gln
570                 575                 580                 585

ctg agc cag cag ccc ctg ggc ccc gct cac cca ttc aac ccc agc ctg 2428
Leu Ser Gln Gln Pro Leu Gly Pro Ala His Pro Phe Asn Pro Ser Leu
                590                 595                 600

gcg gcc cca gcc acc aac cac ata gac cct tgt gct gtg gcc ccg ggg 2476
Ala Ala Pro Ala Thr Asn His Ile Asp Pro Cys Ala Val Ala Pro Gly
            605                 610                 615

ccc ccg tcc gtg gtg gtg aag cag gaa gcc ttg cag cct gag ccc gag 2524
Pro Pro Ser Val Val Val Lys Gln Glu Ala Leu Gln Pro Glu Pro Glu
        620                 625                 630

ccg gtc ccc gcc ccc cag ttg ctt ctg ggg cct cag ggc ccc agc ctc 2572
Pro Val Pro Ala Pro Gln Leu Leu Leu Gly Pro Gln Gly Pro Ser Leu
    635                 640                 645

atc aag ggg gtt gca cct ccc acc ctc atc acc gac tcc aca ggg acc 2620
Ile Lys Gly Val Ala Pro Pro Thr Leu Ile Thr Asp Ser Thr Gly Thr
650                 655                 660                 665

cac ctt gtc ctc acc gtg acc aat aag aat gca gac agc cct ggc ctg 2668
His Leu Val Leu Thr Val Thr Asn Lys Asn Ala Asp Ser Pro Gly Leu
                670                 675                 680

tcc agt ggg agc ccc cag cag ccc tcg tcc cag cct ggc tct cca gcg 2716
Ser Ser Gly Ser Pro Gln Gln Pro Ser Ser Gln Pro Gly Ser Pro Ala

685                 690                 695

cct gcc ccc tct gcc cag atg gac ctg gag cac cca ctg cag ccc ctc        2764
Pro Ala Pro Ser Ala Gln Met Asp Leu Glu His Pro Leu Gln Pro Leu
        700                 705                 710

ttt ggg acc ccc act tct ctg ctg aag aag gaa cca cct ggc tat gag        2812
Phe Gly Thr Pro Thr Ser Leu Leu Lys Lys Glu Pro Pro Gly Tyr Glu
    715                 720                 725

gaa gcc atg agc cag cag ccc aaa cag cag gaa aat ggt tcc tca agc        2860
Glu Ala Met Ser Gln Gln Pro Lys Gln Gln Glu Asn Gly Ser Ser Ser
730                 735                 740                 745

cag cag atg gac gac ctg ttt gac att ctc att cag agc gga ggt aac        2908
Gln Gln Met Asp Asp Leu Phe Asp Ile Leu Ile Gln Ser Gly Gly Asn
                750                 755                 760

ccc aga aat ttc agc aga ttt caa gga gcc gcc atc cct gcc agg gaa        2956
Pro Arg Asn Phe Ser Arg Phe Gln Gly Ala Ala Ile Pro Ala Arg Glu
            765                 770                 775

gga gaa gcc atc ccc gaa gac agt ctg tgg gtc ccc cct ggc agc aca        3004
Gly Glu Ala Ile Pro Glu Asp Ser Leu Trp Val Pro Pro Gly Ser Thr
        780                 785                 790

gcc atc acc ttc tgc tga gctcccccag gctgccccac ctcctccagg               3052
Ala Ile Thr Phe Cys
    795

ctcaccctcc ctccctggac gcctggagga cttcctggag agcagcacgg ggctgcccct      3112

gctgaccagt gggcatgacg ggccagagcc cctttccctc attgacgacc tccatagcca      3172

gatgctgagc agcactgcca tcctggacca ccccccgtca cccatggaca cctcggaatt      3232

gcactttgtt cctgagccca gcagcaccat gggcctggac ctggctgatg gccacctgga      3292

cagcatggac tggctggagc tgtcgtcagg tggtcccgtg ctgagcctag cccccctcag     3352

caccacagcc cccagcctct tctccacaga cttcctcgat ggccatgatt tgcagctgca      3412

ctgggattcc tgcttgtagc tctctggctc aagacggggt ggggaagggg ctgggagcca      3472

gggtactcca atgcgtggct ctcctgcgtg attcggcctc tccacatggt tgtgagtctt      3532

gacaatcaca gcccctgctt tttcccttcc ctgggaggct agaacagaga agcccttact      3592

cctggttcag tgccacgcag ggcagaggag agcagctgtc aagaagcagc cctggctctc      3652

acgctggggt tttggacaca cggtcagggt cagggccatt tcagcttgac ctcctttttt      3712

gaggtcaggg ggcactgtct gtctggctac aatttggcta aggtaggtga agcctggcca      3772

ggcgggaggc ttctcttctg acccagggct gagacaggtt aaggggtgaa tctccttcct      3832

ttctctccct gctttgctgt gaaggagaaa attagcctgg gcctctaccc cctattccct      3892

gtgtctgcca accccaggat cccagggctc cctgccattt tagtgtcttg gtgtagtgta      3952

accatttagt ggttggtggc aacaatttta tgtacaggtg tatatacctc tatattatat      4012

atcgacatac atatatattt ttgggggggg gcggacagga gatgggtgca actccctccc      4072

atcctactct cacagaaggg cctggatgca aggttaccct tgagctgtgt gccacagtct     4132

ggtgcccagt ctggcatgca gctacccagg cccacccatc acgtgtgatt gacatgtagg     4192

taccctgcca cggcctatgc cccacctgcc ctgcttcctg gctccttatc agtgccatga     4252

gggcagaggt gctacctggc cttcctgcca ggagctctcc acccactcac attccgtccc     4312

cgccgcctca ctgcagccag cgtggcccta ggacaggagg agcttcgggc ccagcttcac     4372

cctgcggtgg ggctgagggg tggccatctc ctgccctggg gccactggct tcacattctg     4432

ggctgactca taggggagta ggggtggagt caccaaaacc agtgctggga caaagatggg     4492

gaaggtgtgt gaacttttta aaataaacac aaaaacacag gcctgttaaa aaaaaaaaaa     4552

aaaaaaaa                                                              4560

<210>  72
<211>  798
<212>  PRT
<213>  Homo sapiens

<400>  72

Met Pro Pro Leu Lys Ser Pro Ala Ala Phe His Glu Gln Arg Arg Ser
1               5                   10                  15

Leu Glu Arg Ala Arg Thr Glu Asp Tyr Leu Lys Arg Lys Ile Arg Ser
            20                  25                  30

Arg Pro Glu Arg Ser Glu Leu Val Arg Met His Ile Leu Glu Glu Thr
        35                  40                  45

Ser Ala Glu Pro Ser Leu Gln Ala Lys Gln Leu Lys Leu Lys Arg Ala
    50                  55                  60

Arg Leu Ala Asp Asp Leu Asn Glu Lys Ile Ala Gln Arg Pro Gly Pro
65                  70                  75                  80

Met Glu Leu Val Glu Lys Asn Ile Leu Pro Val Glu Ser Ser Leu Lys
                85                  90                  95

Glu Ala Ile Ile Val Gly Gln Val Asn Tyr Pro Lys Val Ala Asp Ser
            100                 105                 110

Ser Ser Phe Asp Glu Asp Ser Ser Asp Ala Leu Ser Pro Glu Gln Pro
        115                 120                 125

Ala Ser His Glu Ser Gln Gly Ser Val Pro Ser Pro Leu Glu Ala Arg
    130                 135                 140

Val Ser Glu Pro Leu Leu Ser Ala Thr Ser Ala Ser Pro Thr Gln Val
145                 150                 155                 160

Val Ser Gln Leu Pro Met Gly Arg Asp Ser Arg Glu Met Leu Phe Leu
                165                 170                 175

Ala Glu Gln Pro Pro Leu Pro Pro Pro Pro Leu Leu Pro Pro Ser Leu
            180                 185                 190

Thr Asn Gly Thr Thr Ile Pro Thr Ala Lys Ser Thr Pro Thr Leu Ile
        195                 200                 205

Lys Gln Ser Gln Pro Lys Ser Ala Ser Glu Lys Ser Gln Arg Ser Lys
    210                 215                 220

Lys Ala Lys Glu Leu Lys Pro Lys Val Lys Lys Leu Lys Tyr His Gln
225                 230                 235                 240

Tyr Ile Pro Pro Asp Gln Lys Gln Asp Arg Gly Ala Pro Pro Met Asp
                245                 250                 255

Ser Ser Tyr Ala Lys Ile Leu Gln Gln Gln Gln Leu Phe Leu Gln Leu
            260                 265                 270

Gln Ile Leu Asn Gln Gln Gln Gln Gln His His Asn Tyr Gln Ala Ile
        275                 280                 285

Leu Pro Ala Pro Pro Lys Ser Ala Gly Glu Ala Leu Gly Ser Ser Gly
    290                 295                 300

Thr Pro Pro Val Arg Ser Leu Ser Thr Thr Asn Ser Ser Ser Ser Ser
305                 310                 315                 320

Gly Ala Pro Gly Pro Cys Gly Leu Ala Arg Gln Asn Ser Thr Ser Leu
                325                 330                 335

Thr Gly Lys Pro Gly Ala Leu Pro Ala Asn Leu Asp Asp Met Lys Val
            340                 345                 350

Ala Glu Leu Lys Gln Glu Leu Lys Leu Arg Ser Leu Pro Val Ser Gly
        355                 360                 365

Thr Lys Thr Glu Leu Ile Glu Arg Leu Arg Ala Tyr Gln Asp Gln Ile
    370                 375                 380

Ser Pro Val Pro Gly Ala Pro Lys Ala Pro Ala Ala Thr Ser Ile Leu
385                 390                 395                 400

His Lys Ala Gly Glu Val Val Val Ala Phe Pro Ala Ala Arg Leu Ser
                405                 410                 415

Thr Gly Pro Ala Leu Val Ala Ala Gly Leu Ala Pro Ala Glu Val Val
            420                 425                 430

Val Ala Thr Val Ala Ser Ser Gly Val Val Lys Phe Gly Ser Thr Gly
        435                 440                 445

Ser Thr Pro Pro Val Ser Pro Thr Pro Ser Glu Arg Ser Leu Leu Ser
    450                 455                 460

Thr Gly Asp Glu Asn Ser Thr Pro Gly Asp Thr Phe Gly Glu Met Val
465                 470                 475                 480

Thr Ser Pro Leu Thr Gln Leu Thr Leu Gln Ala Ser Pro Leu Gln Ile
                485                 490                 495

Leu Val Lys Glu Glu Gly Pro Arg Ala Gly Ser Cys Cys Leu Ser Pro
            500                 505                 510

Gly Gly Arg Ala Glu Leu Glu Gly Arg Asp Lys Asp Gln Met Leu Gln
        515                 520                 525

Glu Lys Asp Lys Gln Ile Glu Ala Leu Thr Arg Met Leu Arg Gln Lys
    530                 535                 540

Gln Gln Leu Val Glu Arg Leu Lys Leu Gln Leu Glu Gln Glu Lys Arg
545                 550                 555                 560

Ala Gln Gln Pro Ala Pro Ala Pro Ala Pro Leu Gly Thr Pro Val Lys
                565                 570                 575

Gln Glu Asn Ser Phe Ser Ser Cys Gln Leu Ser Gln Gln Pro Leu Gly
            580                 585                 590

Pro Ala His Pro Phe Asn Pro Ser Leu Ala Ala Pro Ala Thr Asn His
        595                 600                 605

Ile Asp Pro Cys Ala Val Ala Pro Gly Pro Pro Ser Val Val Val Lys
    610                 615                 620

Gln Glu Ala Leu Gln Pro Glu Pro Glu Pro Val Pro Ala Pro Gln Leu
625                 630                 635                 640

Leu Leu Gly Pro Gln Gly Pro Ser Leu Ile Lys Gly Val Ala Pro Pro
                645                 650                 655

Thr Leu Ile Thr Asp Ser Thr Gly Thr His Leu Val Leu Thr Val Thr
            660                 665                 670

Asn Lys Asn Ala Asp Ser Pro Gly Leu Ser Ser Gly Ser Pro Gln Gln
        675                 680                 685

Pro Ser Ser Gln Pro Gly Ser Pro Ala Pro Ala Pro Ser Ala Gln Met
    690                 695                 700

Asp Leu Glu His Pro Leu Gln Pro Leu Phe Gly Thr Pro Thr Ser Leu
705                 710                 715                 720

Leu Lys Lys Glu Pro Pro Gly Tyr Glu Glu Ala Met Ser Gln Gln Pro
                725                 730                 735

Lys Gln Gln Glu Asn Gly Ser Ser Ser Gln Gln Met Asp Asp Leu Phe
            740                 745                 750

Asp Ile Leu Ile Gln Ser Gly Gly Asn Pro Arg Asn Phe Ser Arg Phe
        755                 760                 765

Gln Gly Ala Ala Ile Pro Ala Arg Glu Gly Glu Ala Ile Pro Glu Asp
    770                 775                 780

Ser Leu Trp Val Pro Pro Gly Ser Thr Ala Ile Thr Phe Cys
785                 790                 795

<210>  73
<211>  3215
<212>  DNA
<213>  Homo sapiens

<220>
<221>  CDS
<222>  (178)..(2415)

<400>  73
gacaatatca ggtgagctgt ggaggtgggg tccttggaag ctggatgaca gcagctggca      60

aggggataag agagcagtga gcccctccct caaggaggtc tggctttatc catagacagg     120

gccctctgag gtggggctga ggtacaaagg gggattgagc agcccaggag aagagag        177

atg ggg gtt ccc ttc ttc tct tct ctc aga tgc atg gtg gac tta gga       225

1               5                   10                  15

cct tgc tgg gct ggg ggt ctc act gca gag atg aag ctg ctt ctg gcc       273

            20                  25                  30

cta gca ggg ctc ctg gcc att ctg gcc acg ccc cag ccc tct gaa ggt 321

        35                  40                  45

gct gct cca gct gtc ctg ggg gag gtg gac acc tcg ttg gtg ctg agc 369

    50                  55                  60

tcc atg gag gag gcc aag cag ctg gtg gac aag gcc tac aag gag cgg 417

65                  70                  75                  80

cgg gaa agc atc aag cag cgg ctt cgc agc ggc tca gcc agc ccc atg 465

                85                  90                  95

gaa ctc cta tcc tac ttc aag cag ccg gtg gca gcc acc agg acg gcg 513

            100                 105                 110

gtg agg gcc gct gac tac ctg cac gtg gct cta gac ctg ctg gag agg 561

        115                 120                 125

aag ctg cgg tcc ctg tgg cga agg cca ttc aat gtc act gat gtg ctg 609

    130                 135                 140

acg ccc gcc cag ctg aat gtg ttg tcc aag tca agc ggc tgc gcc tac 657

145                 150                 155                 160

cag gac gtg ggg gtg act tgc ccg gag cag gac aaa tac cgc acc atc 705

                165                 170                 175

acc ggg atg tgc aac aac aga cgc agc ccc acg ctg ggg gcc tcc aac 753

            180                 185                 190

cgt gcc ttt gtg cgc tgg ctg ccg gcg gag tat gag gac ggc ttc tct 801

        195                 200                 205

ctt ccc tac ggc tgg acg ccc ggg gtc aag cgc aac ggc ttc ccg gtg 849

    210                 215                 220

gct ctg gct cgc gcg gtc tcc aac gag atc gtg cgc ttc ccc act gat 897

225                 230                 235                 240

cag ctg act ccg gac cag gag cgc tca ctc atg ttc atg caa tgg ggc 945

                245                 250                 255

cag ctg ttg gac cac gac ctc gac ttc acc cct gag ccg gcc gcc cgg 993

            260                 265                 270

gcc tcc ttc gtc act ggc gtc aac tgc gag acc agc tgc gtt cag cag 1041

        275                 280                 285

ccg ccc tgc ttc ccg ctc aag atc ccg ccc aat gac ccc cgc atc aag 1089

    290                 295                 300

aac caa gcc gac tgc atc ccg ttc ttc cgc tcc tgc ccg gct tgc ccc 1137

305                 310                 315                 320

ggg agc aac atc acc atc cgc aac cag atc aac gcg ctc act tcc ttc 1185

                325                 330                 335

gtg gac gcc agc atg gtg tac ggc agc gag gag ccc ctg gcc agg aac 1233

            340                 345                 350

ctg cgc aac atg tcc aac cag ctg ggg ctg ctg gcc gtc aac cag cgc 1281

        355                 360                 365

ttc caa gac aac ggc cgg gcc ctg ctg ccc ttt gac aac ctg cac gat 1329

    370                 375                 380

gac ccc tgt ctc ctc acc aac cgc tca gcg cgc atc ccc tgc ttc ctg 1377

385                 390                 395                 400

gca ggg gac acc cgt tcc agt gag atg ccc gag ctc acc tcc atg cac 1425

                405                 410                 415

acc ctc tta ctt cgg gag cac aac cgg ctg gcc aca gag ctc aag agc 1473

            420                 425                 430

ctg aac cct agg tgg gat ggg gag agg ctc tac cag gaa gcc cgg aag 1521

        435                 440                 445

atc gtg ggg gcc atg gtc cag atc atc act tac cgg gac tac ctg ccc 1569

    450                 455                 460

ctg gtg ctg ggg cca acg gcc atg agg aag tac ctg ccc acg tac cgt 1617

465                 470                 475                 480

tcc tac aat gac tca gtg gac cca cgc atc gcc aac gtc ttc acc aat 1665

                485                 490                 495

gcc ttc cgc tac ggc cac acc ctc atc caa ccc ttc atg ttc cgc ctg 1713

            500                 505                 510

gac aat cgg tac cag ccc atg gaa ccc aac ccc cgt gtc ccc ctc agc 1761

        515                 520                 525

agg gtc ttt ttt gcc tcc tgg agg gtc gtg ctg gaa ggt ggc att gac 1809


530                 535                 540

ccc atc ctc cgg ggc ctc atg gcc acc cct gcc aag ctg aat cgt cag        1857

545                 550                 555                 560

aac caa att gca gtg gat gag atc cgg gag cga ttg ttt gag cag gtc        1905

                565                 570                 575

atg agg att ggg ctg gac ctg cct gct ctg aac atg cag cgc agc agg        1953

            580                 585                 590

gac cac ggc ctc cca gga tac aat gcc tgg agg cgc ttc tgt ggg ctc        2001

        595                 600                 605

ccg cag cct gaa act gtg ggc cag ctg ggc acg gtg ctg agg aac ctg        2049

    610                 615                 620

aaa ttg gcg agg aaa ctg atg gag cag tat ggc acg ccc aac aac atc        2097

625                 630                 635                 640

gac atc tgg atg ggc ggc gtg tcc gag cct ctg aag cgc aaa ggc cgc        2145

                645                 650                 655

gtg ggc cca ctc ctc gcc tgc atc atc ggt acc cag ttc agg aag ctc        2193

            660                 665                 670

cgg gat ggt gat cgg ttt tgg tgg gag aac gag ggt gtg ttc agc atg        2241

        675                 680                 685

cag cag cga cag gcc ctg gcc cag atc tca ttg ccc cgg atc atc tgc        2289

    690                 695                 700

gac aac aca ggc atc acc acc gtg tct aag aac aac atc ttc atg tcc        2337

705                 710                 715                 720

aac tca tat ccc cgg gac ttt gtc aac tgc agt aca ctt cct gca ttg        2385

                725                 730                 735

aac ctg gct tcc tgg agg gaa gcc tcc tag aggccaggta aggggggtgca        2435

            740                 745

gcagtgaggg gtatatctgg gctggccagt tggaaccacg gagatctcct tgccctagat        2495

gagcccagcc ctgttctggg tgcagctgag aaaatgagtg actagacgtt catttgtgtg        2555

ctcatgtatg tgcgaagtat ataaattggc ttttcatgcg tgtgtgttgt ctgaacatgg        2615

ggagtgtttc atgggttatg tgtatgtgcc atttatgtga gtgtgtgttt gtgctgatga        2675

gaatactgag tatgtggaag gcagcagagc ggactggtga ggagcacagc tcaggaacta        2735

gactgcctgg gttccaatcc tggctctgtg gcttgctagc tatgtgacct tgagcaaatt 2795

accctcctta aacaagagtt ttcttccttg taaattacat ctgtcatggt ttcttggagg 2855

gcccacttgt atcctctggt tcttcattta ttgagcacct actacatgca aggcactgta 2915

ctaggcgtga gaagcatata gaggcaagaa agagatacca agatgccatc tgtgtcctgg 2975

ttagcagagc tggaccagtg gtgccttgga gggataagcc agctgcagct gggctgtgtg 3035

gttgacttat gggcccagcc agccaggctc aggccatggc tccccttttt cttcctcacc 3095

ctgatttctt gcttattcac tgaagttctc ctgaagagga actgggcctg ttgccctttc 3155

tgtaccattt atttgctccc aatgtttatg ataataaagg caccgctgat ggggacctcc 3215

<210> 74
<211> 745
<212> PRT
<213> Homo sapiens

<400> 74

<210>  75
<211>  2535
<212>  DNA
<213>  Homo sapiens

<220>
<221>  CDS
<222>  (141)..(1667)

<400>  75
acccttcccc cttctccaac cgccaacggc cgcctgcgcc tgaggcccgg cccacgcccc      60

atcccgctcg ctccacgctg cggcagcccc ggcggcccca ggtgcgcggc ccccgccatc     120

ccgccccgcc gccctgcgcc atg gag gag ggc cct ctg ccg ggc ggg ctg ccc     173
                      Met Glu Glu Gly Pro Leu Pro Gly Gly Leu Pro
                      1               5                   10

agc ccc gag gat gcg atg gtg acg gag ctc tta agc ccc gag ggt ccg       221
Ser Pro Glu Asp Ala Met Val Thr Glu Leu Leu Ser Pro Glu Gly Pro
            15                  20                  25

ttc gct tcg gag aac atc ggc ctg aag gcc ccc gtg aag tac gag gag       269
Phe Ala Ser Glu Asn Ile Gly Leu Lys Ala Pro Val Lys Tyr Glu Glu
        30                  35                  40

gac gag ttc cac gtc ttc aaa gaa gcg tac ctg ggc ccg gcg gac ccc       317
Asp Glu Phe His Val Phe Lys Glu Ala Tyr Leu Gly Pro Ala Asp Pro
    45                  50                  55

aag gaa ccc gtc ctg cac gcg ttc aac ccc gcg ctg ggc gcc gac tgc       365
Lys Glu Pro Val Leu His Ala Phe Asn Pro Ala Leu Gly Ala Asp Cys
60                  65                  70                  75

aag ggc cag gtc aag gcg aag ctc gcg ggg ggc gac agc gac ggc ggg       413
Lys Gly Gln Val Lys Ala Lys Leu Ala Gly Gly Asp Ser Asp Gly Gly
                80                  85                  90

gag ctc ctc ggg gag tac ccc ggg atc cca gag ctc agc gcg ctg gag     461
Glu Leu Leu Gly Glu Tyr Pro Gly Ile Pro Glu Leu Ser Ala Leu Glu
            95                  100                 105

gac gtc gcg ctc ctg cag gcc ccg cag ccg ccc gcc tgc aac gtg cac     509
Asp Val Ala Leu Leu Gln Ala Pro Gln Pro Pro Ala Cys Asn Val His
        110                 115                 120

ttc ctg tcc tcg ctg cta ccc gcg cac cgc agc ccg gcg gtg ttg ccc     557
Phe Leu Ser Ser Leu Leu Pro Ala His Arg Ser Pro Ala Val Leu Pro
    125                 130                 135

ctg ggc gcc tgg gtc ctg gaa gga gcc tcc cac ccg ggc gtc cgc atg     605
Leu Gly Ala Trp Val Leu Glu Gly Ala Ser His Pro Gly Val Arg Met
140                 145                 150                 155

atc cca gtt gaa atc aag gaa gca ggt ggt act act aca agt aat aat     653
Ile Pro Val Glu Ile Lys Glu Ala Gly Gly Thr Thr Thr Ser Asn Asn
                160                 165                 170

ccg gaa gaa gca act ttg cag aat ctt ctt gct cag gaa tcc tgt tgc     701
Pro Glu Glu Ala Thr Leu Gln Asn Leu Leu Ala Gln Glu Ser Cys Cys
            175                 180                 185

aag ttc cca tcg tcc cag gaa cta gag gat gcc tcc tgc tgt tct ctt     749
Lys Phe Pro Ser Ser Gln Glu Leu Glu Asp Ala Ser Cys Cys Ser Leu
        190                 195                 200

aag aaa gat tcc aac cca atg gtg ata tgc caa ttg aaa ggg ggc aca     797
Lys Lys Asp Ser Asn Pro Met Val Ile Cys Gln Leu Lys Gly Gly Thr
    205                 210                 215

caa atg cta tgt ata gac aat tct aga aca aga gaa cta aaa gca ctc     845
Gln Met Leu Cys Ile Asp Asn Ser Arg Thr Arg Glu Leu Lys Ala Leu
220                 225                 230                 235

cat ttg gtt cct cag tat caa gat caa aat aat tat cta cag tca gat     893
His Leu Val Pro Gln Tyr Gln Asp Gln Asn Asn Tyr Leu Gln Ser Asp
                240                 245                 250

gtc cct aaa cca atg act gct tta gta ggg aga ttt ttg cca gca tca     941
Val Pro Lys Pro Met Thr Ala Leu Val Gly Arg Phe Leu Pro Ala Ser
            255                 260                 265

aca aaa tta aat ctc att aca caa caa ctt gag gga gcc tta cca tcg     989
Thr Lys Leu Asn Leu Ile Thr Gln Gln Leu Glu Gly Ala Leu Pro Ser
        270                 275                 280

gta gtc aac ggg tct gct ttc ccc tcg gga tca act ctt cca gga cca     1037
Val Val Asn Gly Ser Ala Phe Pro Ser Gly Ser Thr Leu Pro Gly Pro
    285                 290                 295

cca aaa ata act ttg gct ggg tac tgt gac tgc ttt gcc agt ggg gac     1085
Pro Lys Ile Thr Leu Ala Gly Tyr Cys Asp Cys Phe Ala Ser Gly Asp
300                 305                 310                 315

ttt tgc aac aac tgc aat tgt aat aat tgt tgc aac aac ttg cat cat     1133
Phe Cys Asn Asn Cys Asn Cys Asn Asn Cys Cys Asn Asn Leu His His
                320                 325                 330

gat att gaa cgg ttt aaa gcc att aag gca tgt ctt ggt aga aat cca     1181
Asp Ile Glu Arg Phe Lys Ala Ile Lys Ala Cys Leu Gly Arg Asn Pro
            335                 340                 345

gaa gct ttc cag cca aaa att ggg aag ggc caa ttg ggc aat gtc aag     1229
Glu Ala Phe Gln Pro Lys Ile Gly Lys Gly Gln Leu Gly Asn Val Lys
        350                 355                 360

ccc cag cac aac aaa ggg tgc aac tgc agg agg tca ggc tgc ctg aag     1277
Pro Gln His Asn Lys Gly Cys Asn Cys Arg Arg Ser Gly Cys Leu Lys
    365                 370                 375

aat tac tgc gag tgc tat gag gcc caa att atg tgt tct tct att tgc     1325
Asn Tyr Cys Glu Cys Tyr Glu Ala Gln Ile Met Cys Ser Ser Ile Cys
380                 385                 390                 395

aaa tgc att ggt tgc aaa aat tat gaa gaa agc cca gaa cga aag aca     1373
Lys Cys Ile Gly Cys Lys Asn Tyr Glu Glu Ser Pro Glu Arg Lys Thr
                400                 405                 410

cta atg agc atg cca aac tac atg cag act gga ggt ttg gaa ggc agc     1421
Leu Met Ser Met Pro Asn Tyr Met Gln Thr Gly Gly Leu Glu Gly Ser
            415                 420                 425

cat tac ctg cca cca acg aaa ttt tca gga ctt cca aga ttc agt cac     1469
His Tyr Leu Pro Pro Thr Lys Phe Ser Gly Leu Pro Arg Phe Ser His
        430                 435                 440

gat agg cgg cct tcc tca tgc atc tcc tgg gag gtg gtg gag gcc aca     1517
Asp Arg Arg Pro Ser Ser Cys Ile Ser Trp Glu Val Val Glu Ala Thr
    445                 450                 455

tgc gcc tgc ctg ctt gct cag gga gaa gag gcc gag aaa gaa cac tgc     1565
Cys Ala Cys Leu Leu Ala Gln Gly Glu Glu Ala Glu Lys Glu His Cys
460                 465                 470                 475

tcc aag tgc ctg gca gag cag atg atc ctg gag gaa ttt gga agg tgc     1613
Ser Lys Cys Leu Ala Glu Gln Met Ile Leu Glu Glu Phe Gly Arg Cys
                480                 485                 490

tta tca cag att ctc cac act gag ttt aaa tct aag gga ttg aaa atg     1661
Leu Ser Gln Ile Leu His Thr Glu Phe Lys Ser Lys Gly Leu Lys Met
            495                 500                 505

gag tag agtataaagt gtgaatgcat gttgattttg tcttagtcta gaaatctcta     1717
Glu

gtttagaaag gatgtttagg ggaacatgag gctggctctg cagcaacaac caggctcccc     1777

tgcatccctg ggcccaggga gtttactcag agctctctga agatgtggca acccatgccc     1837

ccttttctga ggaggtgcat ggcctgagca ttgtttgtct ggcccagagg agagagcttg     1897

ggttcccata gtcctgggag agtgtctgca gggcggcgga gggcagagca gggcagggga     1957

gggcacagca ggccctgcga agggcagagc ggggcagggg agggcagagc aggccctgcg     2017

gagagctcac tctggtcgac tcttcctctc agagaatgtt gctctggagg ctgctctgca     2077

tgaaaaccct aatggtttct tgtttgtttt tcaaattatt tagaaataag ttctccggat     2137

gggctgttgt gataccactt aaaatctcta gagaactact gaacacctaa agattttctg     2197

tagcgtagat atttccccag aggcacgcga actgtcagtc tttcctaagg cccccgggag     2257

acgcaggcaa tggggcctcg caggccaggc ttgcaccagc atgtcttgag ttagaggact 2317

taaaattatc cagtttcttc tgtgtttcta cttgaattgt ggaaaagctc tattatccaa 2377

ttaacttctc cataattatt gttgtaatat tattattgtt tgtaaaacat ggttcacata 2437

actagcttgt ggaaaccagc aggtaaaatg aattcttaag ttgacgcttt tggttctgtt 2497

gtaaagcaaa gatgaataaa aatttccaat gtcttcaa 2535

<210> 76
<211> 508
<212> PRT
<213> Homo sapiens

<400> 76

Met Glu Glu Gly Pro Leu Pro Gly Gly Leu Pro Ser Pro Glu Asp Ala
1               5                   10                  15

Met Val Thr Glu Leu Leu Ser Pro Glu Gly Pro Phe Ala Ser Glu Asn
            20                  25                  30

Ile Gly Leu Lys Ala Pro Val Lys Tyr Glu Glu Asp Glu Phe His Val
        35                  40                  45

Phe Lys Glu Ala Tyr Leu Gly Pro Ala Asp Pro Lys Glu Pro Val Leu
    50                  55                  60

His Ala Phe Asn Pro Ala Leu Gly Ala Asp Cys Lys Gly Gln Val Lys
65                  70                  75                  80

Ala Lys Leu Ala Gly Gly Asp Ser Asp Gly Gly Glu Leu Leu Gly Glu
                85                  90                  95

Tyr Pro Gly Ile Pro Glu Leu Ser Ala Leu Glu Asp Val Ala Leu Leu
            100                 105                 110

Gln Ala Pro Gln Pro Pro Ala Cys Asn Val His Phe Leu Ser Ser Leu
        115                 120                 125

Leu Pro Ala His Arg Ser Pro Ala Val Leu Pro Leu Gly Ala Trp Val
    130                 135                 140

Leu Glu Gly Ala Ser His Pro Gly Val Arg Met Ile Pro Val Glu Ile
145                 150                 155                 160

Lys Glu Ala Gly Gly Thr Thr Thr Ser Asn Asn Pro Glu Glu Ala Thr
                165                 170                 175

Leu Gln Asn Leu Leu Ala Gln Glu Ser Cys Cys Lys Phe Pro Ser Ser

            180                 185                 190

Gln Glu Leu Glu Asp Ala Ser Cys Cys Ser Leu Lys Lys Asp Ser Asn
        195                 200                 205

Pro Met Val Ile Cys Gln Leu Lys Gly Gly Thr Gln Met Leu Cys Ile
    210                 215                 220

Asp Asn Ser Arg Thr Arg Glu Leu Lys Ala Leu His Leu Val Pro Gln
225                 230                 235                 240

Tyr Gln Asp Gln Asn Asn Tyr Leu Gln Ser Asp Val Pro Lys Pro Met
                245                 250                 255

Thr Ala Leu Val Gly Arg Phe Leu Pro Ala Ser Thr Lys Leu Asn Leu
            260                 265                 270

Ile Thr Gln Gln Leu Glu Gly Ala Leu Pro Ser Val Val Asn Gly Ser
        275                 280                 285

Ala Phe Pro Ser Gly Ser Thr Leu Pro Gly Pro Pro Lys Ile Thr Leu
    290                 295                 300

Ala Gly Tyr Cys Asp Cys Phe Ala Ser Gly Asp Phe Cys Asn Asn Cys
305                 310                 315                 320

Asn Cys Asn Asn Cys Cys Asn Asn Leu His His Asp Ile Glu Arg Phe
                325                 330                 335

Lys Ala Ile Lys Ala Cys Leu Gly Arg Asn Pro Glu Ala Phe Gln Pro
            340                 345                 350

Lys Ile Gly Lys Gly Gln Leu Gly Asn Val Lys Pro Gln His Asn Lys
        355                 360                 365

Gly Cys Asn Cys Arg Arg Ser Gly Cys Leu Lys Asn Tyr Cys Glu Cys
    370                 375                 380

Tyr Glu Ala Gln Ile Met Cys Ser Ser Ile Cys Lys Cys Ile Gly Cys
385                 390                 395                 400

Lys Asn Tyr Glu Glu Ser Pro Glu Arg Lys Thr Leu Met Ser Met Pro
                405                 410                 415

Asn Tyr Met Gln Thr Gly Gly Leu Glu Gly Ser His Tyr Leu Pro Pro
            420                 425                 430

Thr Lys Phe Ser Gly Leu Pro Arg Phe Ser His Asp Arg Arg Pro Ser
        435                 440                 445

Ser Cys Ile Ser Trp Glu Val Val Glu Ala Thr Cys Ala Cys Leu Leu
    450                 455                 460

Ala Gln Gly Glu Glu Ala Glu Lys Glu His Cys Ser Lys Cys Leu Ala
465                 470                 475                 480

Glu Gln Met Ile Leu Glu Glu Phe Gly Arg Cys Leu Ser Gln Ile Leu
                485                 490                 495

His Thr Glu Phe Lys Ser Lys Gly Leu Lys Met Glu
            500                 505

<210>  77
<211>  2785
<212>  DNA
<213>  Homo sapiens

<220>
<221>  CDS
<222>  (266)..(2368)

<400>  77
actcaggccc ctcccccggc gcccgcgcgc caggactggg cggcgccgac gcgcttggcg      60

ggagatagaa aagtgcttca acccgcgccg gcggcgactg cagttcctgc gagcgaggag     120

cgcgggacct gctgacacgc tgacgccttc gagcgcggcc cggggcccgg agcggccgga     180

gcagcccggg tcctgacccc ggcccggctc ccgctccggg ctctgccggc gggcgggcga      240

gcgcggcgcg gtccgggccg ggggg atg tct cgg cgg acg cgc tgc gag gat      292
                            Met Ser Arg Arg Thr Arg Cys Glu Asp
                            1               5

ctg gat gag ctg cac tac cag gac aca gat tca gat gtg ccg gag cag      340
Leu Asp Glu Leu His Tyr Gln Asp Thr Asp Ser Asp Val Pro Glu Gln
10                  15                  20                  25

agg gat agc aag tgc aag gtc aaa tgg acc cat gag gag gac gag cag      388
Arg Asp Ser Lys Cys Lys Val Lys Trp Thr His Glu Glu Asp Glu Gln
                30                  35                  40

ctg agg gcc ctg gtg agg cag ttt gga cag cag gac tgg aag ttc ctg      436
Leu Arg Ala Leu Val Arg Gln Phe Gly Gln Gln Asp Trp Lys Phe Leu
            45                  50                  55

gcc agc cac ttc cct aac cgc act gac cag caa tgc cag tac agg tgg      484
Ala Ser His Phe Pro Asn Arg Thr Asp Gln Gln Cys Gln Tyr Arg Trp
        60                  65                  70

ctg aga gtt ttg aat cca gac ctt gtc aag ggg cca tgg acc aaa gag      532
Leu Arg Val Leu Asn Pro Asp Leu Val Lys Gly Pro Trp Thr Lys Glu
    75                  80                  85

gaa gac caa aaa gtc atc gag ctg gtt aag aag tat ggc aca aag cag 580
Glu Asp Gln Lys Val Ile Glu Leu Val Lys Lys Tyr Gly Thr Lys Gln
90                  95                  100                 105

tgg aca ctg att gcc aag cac ctg aag ggc cgg ctg ggg aag cag tgc 628
Trp Thr Leu Ile Ala Lys His Leu Lys Gly Arg Leu Gly Lys Gln Cys
                110                 115                 120

cgt gaa cgc tgg cac aac cac ctc aac cct gag gtg aag aag tct tgc 676
Arg Glu Arg Trp His Asn His Leu Asn Pro Glu Val Lys Lys Ser Cys
            125                 130                 135

tgg acc gag gag gag gac cgc atc atc tgc gag gcc cac aag gtg ctg 724
Trp Thr Glu Glu Glu Asp Arg Ile Ile Cys Glu Ala His Lys Val Leu
        140                 145                 150

ggc aac cgc tgg gcc gag atc gcc aag atg ttg cca ggg agg aca gac 772
Gly Asn Arg Trp Ala Glu Ile Ala Lys Met Leu Pro Gly Arg Thr Asp
    155                 160                 165

aat gct gtg aag aat cac tgg aac tct acc atc aaa agg aag gtg gac 820
Asn Ala Val Lys Asn His Trp Asn Ser Thr Ile Lys Arg Lys Val Asp
170                 175                 180                 185

aca gga ggc ttc ttg agc gag tcc aaa gac tgc aag ccc cca gtg tac 868
Thr Gly Gly Phe Leu Ser Glu Ser Lys Asp Cys Lys Pro Pro Val Tyr
                190                 195                 200

ttg ctg ctg gag ctc gag gac aag gac ggc ctc cag agt gcc cag ccc 916
Leu Leu Leu Glu Leu Glu Asp Lys Asp Gly Leu Gln Ser Ala Gln Pro
            205                 210                 215

acg gaa ggc cag gga agt ctt ctg acc aac tgg ccc tcc gtc cct cct 964
Thr Glu Gly Gln Gly Ser Leu Leu Thr Asn Trp Pro Ser Val Pro Pro
        220                 225                 230

acc ata aag gag gag gaa aac agt gag gag gaa ctt gca gca gcc acc 1012
Thr Ile Lys Glu Glu Glu Asn Ser Glu Glu Glu Leu Ala Ala Ala Thr
    235                 240                 245

aca tcg aag gaa cag gag ccc atc ggt aca gat ctg gac gca gtg cga 1060
Thr Ser Lys Glu Gln Glu Pro Ile Gly Thr Asp Leu Asp Ala Val Arg
250                 255                 260                 265

aca cca gag ccc ttg gag gaa ttc ccg aag cgt gag gac cag gaa ggc 1108
Thr Pro Glu Pro Leu Glu Glu Phe Pro Lys Arg Glu Asp Gln Glu Gly
                270                 275                 280

tcc cca cca gaa acg agc ctg cct tac aag tgg gtg gtg gag gca gct 1156
Ser Pro Pro Glu Thr Ser Leu Pro Tyr Lys Trp Val Val Glu Ala Ala
            285                 290                 295

aac ctc ctc atc cct gct gtg ggt tct agc ctc tct gaa gcc ctg gac 1204
Asn Leu Leu Ile Pro Ala Val Gly Ser Ser Leu Ser Glu Ala Leu Asp
        300                 305                 310

ttg atc gag tcg gac cct gat gct tgg tgt gac ctg agt aaa ttt gac 1252
Leu Ile Glu Ser Asp Pro Asp Ala Trp Cys Asp Leu Ser Lys Phe Asp
    315                 320                 325

ctc cct gag gaa cca tct gca gag gac agt atc aac aac agc cta gtg 1300
Leu Pro Glu Glu Pro Ser Ala Glu Asp Ser Ile Asn Asn Ser Leu Val
330                 335                 340                 345

cag ctg caa gcg tca cat cag cag caa gtc ctg cca ccc cgc cag cct 1348
Gln Leu Gln Ala Ser His Gln Gln Gln Val Leu Pro Pro Arg Gln Pro
                350                 355                 360

tcc gcc ctg gtg ccc agt gtg acc gag tac cgc ctg gat ggc cac acc 1396
Ser Ala Leu Val Pro Ser Val Thr Glu Tyr Arg Leu Asp Gly His Thr
            365                 370                 375

atc tca gac ctg agc cgg agc agc cgg ggc gag ctg atc ccc atc tcc 1444
Ile Ser Asp Leu Ser Arg Ser Ser Arg Gly Glu Leu Ile Pro Ile Ser
        380                 385                 390

ccc agc act gaa gtc ggg ggc tct ggc att ggc aca ccg ccc tct gtg 1492
Pro Ser Thr Glu Val Gly Gly Ser Gly Ile Gly Thr Pro Pro Ser Val
    395                 400                 405

ctc aag cgg cag agg aag agg cgt gtg gct ctg tcc cct gtc act gag 1540
Leu Lys Arg Gln Arg Lys Arg Arg Val Ala Leu Ser Pro Val Thr Glu
410                 415                 420                 425

aat agc acc agt ctg tcc ttc ctg gat tcc tgt aac agc ctc acg ccc 1588
Asn Ser Thr Ser Leu Ser Phe Leu Asp Ser Cys Asn Ser Leu Thr Pro
                430                 435                 440

aag agc aca cct gtt aag acc ctg ccc ttc tcg ccc tcc cag ttt ctg 1636
Lys Ser Thr Pro Val Lys Thr Leu Pro Phe Ser Pro Ser Gln Phe Leu
            445                 450                 455

aac ttc tgg aac aaa cag gac aca ttg gag ctg gag agc ccc tcg ctg 1684
Asn Phe Trp Asn Lys Gln Asp Thr Leu Glu Leu Glu Ser Pro Ser Leu
        460                 465                 470

aca tcc acc cca gtg tgc agc cag aag gtg gtg gtc acc aca cca ctg 1732
Thr Ser Thr Pro Val Cys Ser Gln Lys Val Val Val Thr Thr Pro Leu
    475                 480                 485

cac cgg gac aag aca ccc ctg cac cag aaa cat gct gcg ttt gta acc 1780
His Arg Asp Lys Thr Pro Leu His Gln Lys His Ala Ala Phe Val Thr
490                 495                 500                 505

cca gat cag aag tac tcc atg gac aac act ccc cac acg cca acc ccg 1828
Pro Asp Gln Lys Tyr Ser Met Asp Asn Thr Pro His Thr Pro Thr Pro
                510                 515                 520

ttc aag aac gcc ctg gag aag tac gga ccc ctg aag ccc ctg cca cag 1876
Phe Lys Asn Ala Leu Glu Lys Tyr Gly Pro Leu Lys Pro Leu Pro Gln
            525                 530                 535

acc ccg cac ctg gag gag gac ttg aag gag gtg ctg cgt tct gag gct 1924
Thr Pro His Leu Glu Glu Asp Leu Lys Glu Val Leu Arg Ser Glu Ala
        540                 545                 550

ggc atc gaa ctc atc atc gag gac gac atc agg ccc gag aag cag aag 1972
Gly Ile Glu Leu Ile Ile Glu Asp Asp Ile Arg Pro Glu Lys Gln Lys
    555                 560                 565

agg aag cct ggg ctg cgg cgg agc ccc atc aag aaa gtc cgg aag tct 2020
Arg Lys Pro Gly Leu Arg Arg Ser Pro Ile Lys Lys Val Arg Lys Ser
570                 575                 580                 585

ctg gct ctt gac att gtg gat gag gat gtg aag ctg atg atg tcc aca 2068
Leu Ala Leu Asp Ile Val Asp Glu Asp Val Lys Leu Met Met Ser Thr

590                 595                 600

ctg ccc aag tct cta tcc ttg ccg aca act gcc cct tca aac tct tcc        2116
Leu Pro Lys Ser Leu Ser Leu Pro Thr Thr Ala Pro Ser Asn Ser Ser
            605                 610                 615

agc ctc acc ctg tca ggt atc aaa gaa gac aac agc ttg ctc aac cag        2164
Ser Leu Thr Leu Ser Gly Ile Lys Glu Asp Asn Ser Leu Leu Asn Gln
        620                 625                 630

ggc ttc ttg cag gcc aag ccc gag aag gca gca gtg gcc cag aag ccc        2212
Gly Phe Leu Gln Ala Lys Pro Glu Lys Ala Ala Val Ala Gln Lys Pro
    635                 640                 645

cga agc cac ttc acg aca cct gcc cct atg tcc agt gcc tgg aag acg        2260
Arg Ser His Phe Thr Thr Pro Ala Pro Met Ser Ser Ala Trp Lys Thr
650                 655                 660                 665

gtg gcc tgc ggg ggg acc agg gac cag ctt ttc atg cag gag aaa gcc        2308
Val Ala Cys Gly Gly Thr Arg Asp Gln Leu Phe Met Gln Glu Lys Ala
                670                 675                 680

cgg cag ctc ctg ggc cgc ctg aag ccc agc cac aca tct cgg acc ctc        2356
Arg Gln Leu Leu Gly Arg Leu Lys Pro Ser His Thr Ser Arg Thr Leu
            685                 690                 695

atc ttg tcc tga ggtgttgagg gtgtcacgag cccattctca tgtttacagg        2408
Ile Leu Ser
        700

ggttgtgggg gcagaggggg tctgtgaatc tgagagtcat tcaggtgacc tcctgcaggg        2468

agccttctgc caccagcccc tccccagact ctcaggtgga ggcaacaggg ccatgtgctg        2528

ccctgttgcc gagcccagct gtgggcggct cctggtgcta acaacaaagt tccacttcca        2588

ggtctgcctg gttccctccc caaggccaca gggagctccg tcagcttctc ccaagcccac        2648

gtcaggcctg gcctcatctc agaccctgct taggatgggg gatgtggcca ggggtgctcc        2708

tgtgctcacc ctctcttggt gcattttttt ggaagaataa aattgcctct ctctttgtgc        2768

tggtctggaa aaaaaaa                                                     2785

<210>  78
<211>  700
<212>  PRT
<213>  Homo sapiens

<400>  78

Met Ser Arg Arg Thr Arg Cys Glu Asp Leu Asp Glu Leu His Tyr Gln
1               5                   10                  15

Asp Thr Asp Ser Asp Val Pro Glu Gln Arg Asp Ser Lys Cys Lys Val
            20                  25                  30

Lys Trp Thr His Glu Glu Asp Glu Gln Leu Arg Ala Leu Val Arg Gln
        35                  40                  45

Phe Gly Gln Gln Asp Trp Lys Phe Leu Ala Ser His Phe Pro Asn Arg
    50                  55                  60

Thr Asp Gln Gln Cys Gln Tyr Arg Trp Leu Arg Val Leu Asn Pro Asp
65                  70                  75                  80

Leu Val Lys Gly Pro Trp Thr Lys Glu Glu Asp Gln Lys Val Ile Glu
                85                  90                  95

Leu Val Lys Lys Tyr Gly Thr Lys Gln Trp Thr Leu Ile Ala Lys His
            100                 105                 110

Leu Lys Gly Arg Leu Gly Lys Gln Cys Arg Glu Arg Trp His Asn His
        115                 120                 125

Leu Asn Pro Glu Val Lys Lys Ser Cys Trp Thr Glu Glu Glu Asp Arg
    130                 135                 140

Ile Ile Cys Glu Ala His Lys Val Leu Gly Asn Arg Trp Ala Glu Ile
145                 150                 155                 160

Ala Lys Met Leu Pro Gly Arg Thr Asp Asn Ala Val Lys Asn His Trp
                165                 170                 175

Asn Ser Thr Ile Lys Arg Lys Val Asp Thr Gly Gly Phe Leu Ser Glu
            180                 185                 190

Ser Lys Asp Cys Lys Pro Pro Val Tyr Leu Leu Leu Glu Leu Glu Asp
        195                 200                 205

Lys Asp Gly Leu Gln Ser Ala Gln Pro Thr Glu Gly Gln Gly Ser Leu
    210                 215                 220

Leu Thr Asn Trp Pro Ser Val Pro Pro Thr Ile Lys Glu Glu Glu Asn
225                 230                 235                 240

Ser Glu Glu Glu Leu Ala Ala Ala Thr Thr Ser Lys Glu Gln Glu Pro
                245                 250                 255

Ile Gly Thr Asp Leu Asp Ala Val Arg Thr Pro Glu Pro Leu Glu Glu
            260                 265                 270

Phe Pro Lys Arg Glu Asp Gln Glu Gly Ser Pro Pro Glu Thr Ser Leu
        275                 280                 285

Pro Tyr Lys Trp Val Val Glu Ala Ala Asn Leu Leu Ile Pro Ala Val
    290                 295                 300

Gly Ser Ser Leu Ser Glu Ala Leu Asp Leu Ile Glu Ser Asp Pro Asp
305                 310                 315                 320

Ala Trp Cys Asp Leu Ser Lys Phe Asp Leu Pro Glu Glu Pro Ser Ala
                325                 330                 335

Glu Asp Ser Ile Asn Asn Ser Leu Val Gln Leu Gln Ala Ser His Gln
            340                 345                 350

Gln Gln Val Leu Pro Pro Arg Gln Pro Ser Ala Leu Val Pro Ser Val
        355                 360                 365

Thr Glu Tyr Arg Leu Asp Gly His Thr Ile Ser Asp Leu Ser Arg Ser
    370                 375                 380

Ser Arg Gly Glu Leu Ile Pro Ile Ser Pro Ser Thr Glu Val Gly Gly
385                 390                 395                 400

Ser Gly Ile Gly Thr Pro Pro Ser Val Leu Lys Arg Gln Arg Lys Arg
                405                 410                 415

Arg Val Ala Leu Ser Pro Val Thr Glu Asn Ser Thr Ser Leu Ser Phe
            420                 425                 430

Leu Asp Ser Cys Asn Ser Leu Thr Pro Lys Ser Thr Pro Val Lys Thr
        435                 440                 445

Leu Pro Phe Ser Pro Ser Gln Phe Leu Asn Phe Trp Asn Lys Gln Asp
    450                 455                 460

Thr Leu Glu Leu Glu Ser Pro Ser Leu Thr Ser Thr Pro Val Cys Ser
465                 470                 475                 480

Gln Lys Val Val Val Thr Thr Pro Leu His Arg Asp Lys Thr Pro Leu
                485                 490                 495

His Gln Lys His Ala Ala Phe Val Thr Pro Asp Gln Lys Tyr Ser Met
            500                 505                 510

Asp Asn Thr Pro His Thr Pro Thr Pro Phe Lys Asn Ala Leu Glu Lys
        515                 520                 525

Tyr Gly Pro Leu Lys Pro Leu Pro Gln Thr Pro His Leu Glu Glu Asp
    530                 535                 540

Leu Lys Glu Val Leu Arg Ser Glu Ala Gly Ile Glu Leu Ile Ile Glu

545                 550                 555                 560

Asp Asp Ile Arg Pro Glu Lys Gln Lys Arg Lys Pro Gly Leu Arg Arg
                565                 570                 575

Ser Pro Ile Lys Lys Val Arg Lys Ser Leu Ala Leu Asp Ile Val Asp
            580                 585                 590

Glu Asp Val Lys Leu Met Met Ser Thr Leu Pro Lys Ser Leu Ser Leu
        595                 600                 605

Pro Thr Thr Ala Pro Ser Asn Ser Ser Ser Leu Thr Leu Ser Gly Ile
    610                 615                 620

Lys Glu Asp Asn Ser Leu Leu Asn Gln Gly Phe Leu Gln Ala Lys Pro
625                 630                 635                 640

Glu Lys Ala Ala Val Ala Gln Lys Pro Arg Ser His Phe Thr Thr Pro
                645                 650                 655

Ala Pro Met Ser Ser Ala Trp Lys Thr Val Ala Cys Gly Gly Thr Arg
            660                 665                 670

Asp Gln Leu Phe Met Gln Glu Lys Ala Arg Gln Leu Leu Gly Arg Leu
        675                 680                 685

Lys Pro Ser His Thr Ser Arg Thr Leu Ile Leu Ser
    690                 695                 700

<210>  79
<211>  7591
<212>  DNA
<213>  Homo sapiens

<220>
<221>  CDS
<222>  (159)..(6323)

<400>  79
tccatccctg accgggccga ggctgctgga tgccgcgtct ccgcttctgc tgcctgccgg      60

gcgggctccg gtggccgcag caaagtgggg caccaaggcc ctgtgctaag cactcataat     120

cctctggggg tgctacccct acaaacagca cccccacc atg ttt aac cta atg aag     176
                                          Met Phe Asn Leu Met Lys
                                          1               5

aaa gac aag gac aaa gat ggc ggg cgg aag gag aag aag gag aaa aag       224
Lys Asp Lys Asp Lys Asp Gly Gly Arg Lys Glu Lys Lys Glu Lys Lys
            10                  15                  20

gag aaa aag gag cgg atg tca gcg gca gag ctt cgg agc ctg gag gag       272

Glu Lys Lys Glu Arg Met Ser Ala Ala Glu Leu Arg Ser Leu Glu Glu
        25                  30                  35

atg agc ctg cga cgt ggc ttc ttc aac ctg aac cgc tcc tcc aag cgt 320
Met Ser Leu Arg Arg Gly Phe Phe Asn Leu Asn Arg Ser Ser Lys Arg
    40                  45                  50

gaa tcc aag acg cgc ctg gaa atc tcc aac ccc atc ccc atc aag gtg 368
Glu Ser Lys Thr Arg Leu Glu Ile Ser Asn Pro Ile Pro Ile Lys Val
55                  60                  65                  70

gcc agc ggc tct gac ctg cac ctg act gac att gac tcc gat agt aac 416
Ala Ser Gly Ser Asp Leu His Leu Thr Asp Ile Asp Ser Asp Ser Asn
                75                  80                  85

cgg ggc agc gtc atc ctg gac tcg ggc cac cta agt aca gcc agc tcc 464
Arg Gly Ser Val Ile Leu Asp Ser Gly His Leu Ser Thr Ala Ser Ser
            90                  95                  100

agc gat gac ctc aag ggt gag gag ggt agc ttc cgt ggc tcg gtg ctg 512
Ser Asp Asp Leu Lys Gly Glu Glu Gly Ser Phe Arg Gly Ser Val Leu
        105                 110                 115

cag cgg gca gcc aag ttc ggc tca ctg gcc aag cag aac tca cag atg 560
Gln Arg Ala Ala Lys Phe Gly Ser Leu Ala Lys Gln Asn Ser Gln Met
    120                 125                 130

att gtc aag cgc ttt tcc ttc tcc cag cgt agc cgg gat gag agc gcc 608
Ile Val Lys Arg Phe Ser Phe Ser Gln Arg Ser Arg Asp Glu Ser Ala
135                 140                 145                 150

tca gaa acc tcg acg ccc tca gag cac tct gcc gcc ccc tcg cca cag 656
Ser Glu Thr Ser Thr Pro Ser Glu His Ser Ala Ala Pro Ser Pro Gln
                155                 160                 165

gtg gag gtg agg act cta gag gga cag ctg gtg cag cat cct ggc cca 704
Val Glu Val Arg Thr Leu Glu Gly Gln Leu Val Gln His Pro Gly Pro
            170                 175                 180

ggc atc cct cga cca ggg cac cga tcc cga gcc cct gag cta gtg act 752
Gly Ile Pro Arg Pro Gly His Arg Ser Arg Ala Pro Glu Leu Val Thr
        185                 190                 195

aaa aag ttc cca gtc gac ctg cgc ctg ccc ccc gtg gtg ccc ctg ccc 800
Lys Lys Phe Pro Val Asp Leu Arg Leu Pro Pro Val Val Pro Leu Pro
    200                 205                 210

cca cct acc ctc cgg gag ctg gag ctg caa cga cgg ccc act gga gac 848
Pro Pro Thr Leu Arg Glu Leu Glu Leu Gln Arg Arg Pro Thr Gly Asp
215                 220                 225                 230

ttt ggc ttc tcc ctg cgg cgc aca acc atg ctg gat cgg ggc ccc gag 896
Phe Gly Phe Ser Leu Arg Arg Thr Thr Met Leu Asp Arg Gly Pro Glu
                235                 240                 245

ggc cag gcc tgt cgg cgt gtg gtc cac ttt gct gag cct ggt gca ggc 944
Gly Gln Ala Cys Arg Arg Val Val His Phe Ala Glu Pro Gly Ala Gly
            250                 255                 260

acc aag gac ctg gcc ctg ggg ctg gtg cca gga gat cga ctg gtg gag 992
Thr Lys Asp Leu Ala Leu Gly Leu Val Pro Gly Asp Arg Leu Val Glu
        265                 270                 275

att aat ggg cac aat gtg gag agc aag tcc agg gat gag att gtg gag 1040
Ile Asn Gly His Asn Val Glu Ser Lys Ser Arg Asp Glu Ile Val Glu
    280                 285                 290

atg atc cgg cag tca ggg gac agc gtg cgg ctc aag gtg cag ccc att 1088
Met Ile Arg Gln Ser Gly Asp Ser Val Arg Leu Lys Val Gln Pro Ile
295                 300                 305                 310

cca gag ctc agc gag ctc agc agg agc tgg ctg cgg agc ggc gag gga 1136
Pro Glu Leu Ser Glu Leu Ser Arg Ser Trp Leu Arg Ser Gly Glu Gly
                315                 320                 325

cct cgc agg gag cca tcc gat gcg aaa aca gaa gaa cag att gca gca 1184
Pro Arg Arg Glu Pro Ser Asp Ala Lys Thr Glu Glu Gln Ile Ala Ala
            330                 335                 340

gaa gag gcc tgg aat gag acg gag aag gtg tgg ctg gtc cat agg gac 1232
Glu Glu Ala Trp Asn Glu Thr Glu Lys Val Trp Leu Val His Arg Asp
        345                 350                 355

ggc ttc tca ctg gcc agt caa ctc aaa tct gag gag ctc aac ttg cct 1280
Gly Phe Ser Leu Ala Ser Gln Leu Lys Ser Glu Glu Leu Asn Leu Pro
    360                 365                 370

gag ggg aag gtg cgt gtg aag ctg gac cac gat ggg gcc atc ctg gat 1328
Glu Gly Lys Val Arg Val Lys Leu Asp His Asp Gly Ala Ile Leu Asp
375                 380                 385                 390

gtg gat gag gat gac gtt gag aag gct aat gct ccc tcc tgc gac cgt 1376
Val Asp Glu Asp Asp Val Glu Lys Ala Asn Ala Pro Ser Cys Asp Arg
                395                 400                 405

ctg gag gat ctg gcc tca ctg gtg tac ctc aat gag tcc agc gtc ctg 1424
Leu Glu Asp Leu Ala Ser Leu Val Tyr Leu Asn Glu Ser Ser Val Leu
            410                 415                 420

cac acc ttg cgc cag cgc tat ggc gct agc ctg ctg cac acg tat gct 1472
His Thr Leu Arg Gln Arg Tyr Gly Ala Ser Leu Leu His Thr Tyr Ala
        425                 430                 435

ggc ccc agc ctg ctg gtt ctt ggc ccc cgt ggg gcc cct gct gtg tac 1520
Gly Pro Ser Leu Leu Val Leu Gly Pro Arg Gly Ala Pro Ala Val Tyr
    440                 445                 450

tct gag aag gtg atg cac atg ttc aag ggt tgt cgg cgg gag gac atg 1568
Ser Glu Lys Val Met His Met Phe Lys Gly Cys Arg Arg Glu Asp Met
455                 460                 465                 470

gca ccc cac atc tat gca gtg gcc cag acc gca tac agg gcg atg ctg 1616
Ala Pro His Ile Tyr Ala Val Ala Gln Thr Ala Tyr Arg Ala Met Leu
                475                 480                 485

atg agc cgt cag gat cag tca atc atc ctc ctg ggc agt agt ggc agt 1664
Met Ser Arg Gln Asp Gln Ser Ile Ile Leu Leu Gly Ser Ser Gly Ser
            490                 495                 500

ggc aag acc acc agc tgc cag cat ctg gtg cag tac ctg gcc acc atc 1712
Gly Lys Thr Thr Ser Cys Gln His Leu Val Gln Tyr Leu Ala Thr Ile
        505                 510                 515

gcg ggc atc agc ggg aac aag gtg ttt tct gtg gag aag tgg cag gct 1760
Ala Gly Ile Ser Gly Asn Lys Val Phe Ser Val Glu Lys Trp Gln Ala
    520                 525                 530

ctg tac acc ctc ctg gaa gcc ttt ggg aac agc ccc acc atc att aat 1808
Leu Tyr Thr Leu Leu Glu Ala Phe Gly Asn Ser Pro Thr Ile Ile Asn
535                 540                 545                 550

ggc aat gcc acc cgc ttc tcc cag atc ctc tcc ctg gac ttt gac caa 1856
Gly Asn Ala Thr Arg Phe Ser Gln Ile Leu Ser Leu Asp Phe Asp Gln
                555                 560                 565

gct ggc cag gtg gcc tca gcc tcc att cag aca atg ctt ctg gag aag 1904
Ala Gly Gln Val Ala Ser Ala Ser Ile Gln Thr Met Leu Leu Glu Lys
            570                 575                 580

ctg cgt gtg gct cgg cgc cca gcc agt gaa gcc aca ttc aac gtc ttc 1952
Leu Arg Val Ala Arg Arg Pro Ala Ser Glu Ala Thr Phe Asn Val Phe
        585                 590                 595

tac tac ctg ctg gcc tgt ggg gat ggc acc ctc agg aca gag ctc cac 2000
Tyr Tyr Leu Leu Ala Cys Gly Asp Gly Thr Leu Arg Thr Glu Leu His
    600                 605                 610

ctc aac cac ttg gca gag aac aat gtg ttt ggg att gtg cca ctg gcc 2048
Leu Asn His Leu Ala Glu Asn Asn Val Phe Gly Ile Val Pro Leu Ala
615                 620                 625                 630

aag cct gag gaa aag cag aag gca gct cag cag ttt agt aag ctg cag 2096
Lys Pro Glu Glu Lys Gln Lys Ala Ala Gln Gln Phe Ser Lys Leu Gln
                635                 640                 645

gcg gcc atg aag gtg ctg ggc atc tcc ccc gat gaa cag aag gcc tgc 2144
Ala Ala Met Lys Val Leu Gly Ile Ser Pro Asp Glu Gln Lys Ala Cys
            650                 655                 660

tgg ttc att ctg gct gcc atc tac cac ctg ggg gct gcg gga gcc acc 2192
Trp Phe Ile Leu Ala Ala Ile Tyr His Leu Gly Ala Ala Gly Ala Thr
        665                 670                 675

aaa gaa gct gct gaa gct ggg cgc aag cag ttt gcc cgc cat gag tgg 2240
Lys Glu Ala Ala Glu Ala Gly Arg Lys Gln Phe Ala Arg His Glu Trp
    680                 685                 690

gcc cag aag gct gcg tac cta ctg ggc tgc agc ctg gag gag ctg tcc 2288
Ala Gln Lys Ala Ala Tyr Leu Leu Gly Cys Ser Leu Glu Glu Leu Ser
695                 700                 705                 710

tca gcc atc ttc aag cac cag cac aag ggt ggc acc ctg cag cgc tcc 2336
Ser Ala Ile Phe Lys His Gln His Lys Gly Gly Thr Leu Gln Arg Ser
                715                 720                 725

acc tcc ttc cgc cag ggc ccc gag gag agt ggc ctg gga gat ggg aca 2384
Thr Ser Phe Arg Gln Gly Pro Glu Glu Ser Gly Leu Gly Asp Gly Thr
            730                 735                 740

ggc ccg aaa ctg agt gca ctg gag tgc ctt gag ggc atg gcg gcc ggc 2432
Gly Pro Lys Leu Ser Ala Leu Glu Cys Leu Glu Gly Met Ala Ala Gly
        745                 750                 755

ctc tac agc gag ctc ttc acc ctt ctc gtc tcc ctg gtg aat agg gct 2480
Leu Tyr Ser Glu Leu Phe Thr Leu Leu Val Ser Leu Val Asn Arg Ala
    760                 765                 770

ctc aag tcc agc cag cac tca ctc tgc tcc atg atg att gtc gac acc 2528
Leu Lys Ser Ser Gln His Ser Leu Cys Ser Met Met Ile Val Asp Thr

775                 780                 785                 790

ccg ggc ttc cag aac cct gag cag ggt ggg tca gcc cgc gga gcc tcc         2576
Pro Gly Phe Gln Asn Pro Glu Gln Gly Gly Ser Ala Arg Gly Ala Ser
                795                 800                 805

ttt gag gag ctg tgc cac aac tac acc caa gac cgg ctg cag agg ctc         2624
Phe Glu Glu Leu Cys His Asn Tyr Thr Gln Asp Arg Leu Gln Arg Leu
            810                 815                 820

ttc cac gag cgc acc ttc gtg cag gag ttg gaa aga tac aag gag gag         2672
Phe His Glu Arg Thr Phe Val Gln Glu Leu Glu Arg Tyr Lys Glu Glu
        825                 830                 835

aac atc gag ctg gcg ttt gac gac ttg gaa ccc ccg acg gat gac tct         2720
Asn Ile Glu Leu Ala Phe Asp Asp Leu Glu Pro Pro Thr Asp Asp Ser
    840                 845                 850

gtg gct gct gtg gac cag gcc tcc cat cag tcc ctg gtc cgc tcg ctg         2768
Val Ala Ala Val Asp Gln Ala Ser His Gln Ser Leu Val Arg Ser Leu
855                 860                 865                 870

gcc cgc aca gac gag gcg agg ggc ctg ctc tgg cta ttg gaa gag gag         2816
Ala Arg Thr Asp Glu Ala Arg Gly Leu Leu Trp Leu Leu Glu Glu Glu
                875                 880                 885

gct ctg gtg cca ggg gcc agt gag gac acc ctc ctg gag cgc ctt ttc         2864
Ala Leu Val Pro Gly Ala Ser Glu Asp Thr Leu Leu Glu Arg Leu Phe
            890                 895                 900

tcc tat tat ggc ccc cag gaa ggt gac aaa aaa ggc caa agc ccc ctt         2912
Ser Tyr Tyr Gly Pro Gln Glu Gly Asp Lys Lys Gly Gln Ser Pro Leu
        905                 910                 915

ctg cac agc agc aaa cca cac cac ttt ctc ctg ggc cac agc cat ggc         2960
Leu His Ser Ser Lys Pro His His Phe Leu Leu Gly His Ser His Gly
    920                 925                 930

acc aac tgg gta gag tac aat gtg act ggc tgg ctg aac tac acc aag         3008
Thr Asn Trp Val Glu Tyr Asn Val Thr Gly Trp Leu Asn Tyr Thr Lys
935                 940                 945                 950

cag aac cca gcc acc cag aat gcc ccc cgg ctc ctg cag gac tcc cag         3056
Gln Asn Pro Ala Thr Gln Asn Ala Pro Arg Leu Leu Gln Asp Ser Gln
                955                 960                 965

aaa aaa atc atc agc aac ctg ttt ctg ggc cgc gca ggc agt gcc acg         3104
Lys Lys Ile Ile Ser Asn Leu Phe Leu Gly Arg Ala Gly Ser Ala Thr
            970                 975                 980

gtg ctc tct ggc tcc atc gcg ggc ctg gag ggc ggc tcg cag ctg gca         3152
Val Leu Ser Gly Ser Ile Ala Gly Leu Glu Gly Gly Ser Gln Leu Ala
        985                 990                 995

ctg cgc  cgg gcc acc agc atg  cgg aaa acc ttt acc  aca ggc atg         3197
Leu Arg  Arg Ala Thr Ser Met  Arg Lys Thr Phe Thr  Thr Gly Met
    1000                 1005                 1010

gcg gct  gtc aaa aag aag tca  ctg tgc atc cag atg  aag cta cag         3242
Ala Ala  Val Lys Lys Lys Ser  Leu Cys Ile Gln Met  Lys Leu Gln
    1015                 1020                 1025

gtg gac  gcc ctc atc gac acc  atc aag aag tca aag  ctg cat ttt         3287

Val Asp Ala Leu Ile Asp Thr Ile Lys Lys Ser Lys Leu His Phe
    1030                1035                1040

gtg cac tgc ttc ctg cct gta gct gag ggc tgg gct ggg gag ccc 3332
Val His Cys Phe Leu Pro Val Ala Glu Gly Trp Ala Gly Glu Pro
    1045                1050                1055

cgt tcc gcc tcc tcc cgc cga gtc agc agc agc agt gag ctg gac 3377
Arg Ser Ala Ser Ser Arg Arg Val Ser Ser Ser Ser Glu Leu Asp
    1060                1065                1070

ctg ccc tcg gga gac cac tgc gag gct ggg ctc ctg cag ctc gac 3422
Leu Pro Ser Gly Asp His Cys Glu Ala Gly Leu Leu Gln Leu Asp
    1075                1080                1085

gtg ccc ctg ctc cgc acc cag ctc cgc ggc tcc cgc ctg ctc gat 3467
Val Pro Leu Leu Arg Thr Gln Leu Arg Gly Ser Arg Leu Leu Asp
    1090                1095                1100

gcc atg cgc atg tac cgc caa ggt tac cct gac cac atg gtg ttt 3512
Ala Met Arg Met Tyr Arg Gln Gly Tyr Pro Asp His Met Val Phe
    1105                1110                1115

tcc gag ttc cgc cgc cgc ttt gat gtc ctg gcc ccg cac ctg acc 3557
Ser Glu Phe Arg Arg Arg Phe Asp Val Leu Ala Pro His Leu Thr
    1120                1125                1130

aag aaa cac ggg cgt aac tac atc gtg gtg gat gaa agg cgg gca 3602
Lys Lys His Gly Arg Asn Tyr Ile Val Val Asp Glu Arg Arg Ala
    1135                1140                1145

gtg gag gag ctg ctg gag tgc ttg gat ctg gag aag agc agc tgc 3647
Val Glu Glu Leu Leu Glu Cys Leu Asp Leu Glu Lys Ser Ser Cys
    1150                1155                1160

tgc atg ggc ctg agc cgg gtg ttc ttc cgg gcg ggc acc ttg gca 3692
Cys Met Gly Leu Ser Arg Val Phe Phe Arg Ala Gly Thr Leu Ala
    1165                1170                1175

cgg ctg gag gag cag cgg gat gaa caa acc agc agg aac cta acc 3737
Arg Leu Glu Glu Gln Arg Asp Glu Gln Thr Ser Arg Asn Leu Thr
    1180                1185                1190

ctg ttc caa gca gcc tgc agg ggc tac ctg gcc cgc cag cac ttc 3782
Leu Phe Gln Ala Ala Cys Arg Gly Tyr Leu Ala Arg Gln His Phe
    1195                1200                1205

aag aag aga aag atc cag gac ctg gcc att cgc tgt gta cag aag 3827
Lys Lys Arg Lys Ile Gln Asp Leu Ala Ile Arg Cys Val Gln Lys
    1210                1215                1220

aac atc aag aag aac aaa ggg gtg aag gac tgg ccc tgg tgg aag 3872
Asn Ile Lys Lys Asn Lys Gly Val Lys Asp Trp Pro Trp Trp Lys
    1225                1230                1235

ctt ttt acc aca gtg agg ccc ctc atc gaa gta cag ctg tca gag 3917
Leu Phe Thr Thr Val Arg Pro Leu Ile Glu Val Gln Leu Ser Glu
    1240                1245                1250

gag cag atc cgg aac aaa gac gag gag atc cag cag ctg cgg agc 3962
Glu Gln Ile Arg Asn Lys Asp Glu Glu Ile Gln Gln Leu Arg Ser
    1255                1260                1265

aag ctc gag aag gcg gag aag gag agg aac gag ctg cgg ctc aac 4007
Lys Leu Glu Lys Ala Glu Lys Glu Arg Asn Glu Leu Arg Leu Asn
    1270                1275                1280

agt gac cgg ctg gag agc cgg atc tca gag ctg aca tcg gag ctg 4052
Ser Asp Arg Leu Glu Ser Arg Ile Ser Glu Leu Thr Ser Glu Leu
    1285                1290                1295

aca gat gag cgt aac aca gga gag tcc gcc tcc cag ctg ctg gac 4097
Thr Asp Glu Arg Asn Thr Gly Glu Ser Ala Ser Gln Leu Leu Asp
    1300                1305                1310

gcg gag aca gca gag agg ctc cgg gct gag aag gag atg aag gaa 4142
Ala Glu Thr Ala Glu Arg Leu Arg Ala Glu Lys Glu Met Lys Glu
    1315                1320                1325

ctg cag acc cag tac gat gca ctg aag aag cag atg gag gtt atg 4187
Leu Gln Thr Gln Tyr Asp Ala Leu Lys Lys Gln Met Glu Val Met
    1330                1335                1340

gaa atg gag gtg atg gag gcc cgt ctc atc cgg gca gcg gag atc 4232
Glu Met Glu Val Met Glu Ala Arg Leu Ile Arg Ala Ala Glu Ile
    1345                1350                1355

aac ggg gaa gtg gat gat gat gat gca ggt ggc gag tgg cgg ctg 4277
Asn Gly Glu Val Asp Asp Asp Asp Ala Gly Gly Glu Trp Arg Leu
    1360                1365                1370

aag tat gag cgg gct gtg cgg gag gtg gac ttc acc aag aaa cgg 4322
Lys Tyr Glu Arg Ala Val Arg Glu Val Asp Phe Thr Lys Lys Arg
    1375                1380                1385

ctc cag cag gag ttt gag gac aag ctg gag gtg gag cag cag aac 4367
Leu Gln Gln Glu Phe Glu Asp Lys Leu Glu Val Glu Gln Gln Asn
    1390                1395                1400

aag agg cag ctg gaa cgg cgg ctc ggg gac ctg cag gca gat agt 4412
Lys Arg Gln Leu Glu Arg Arg Leu Gly Asp Leu Gln Ala Asp Ser
    1405                1410                1415

gag gag agt cag cgg gct ctg cag cag ctc aag aag aag tgc cag 4457
Glu Glu Ser Gln Arg Ala Leu Gln Gln Leu Lys Lys Lys Cys Gln
    1420                1425                1430

cga ctg acg gct gag ctg caa gac acc aag ctg cac ctg gag ggc 4502
Arg Leu Thr Ala Glu Leu Gln Asp Thr Lys Leu His Leu Glu Gly
    1435                1440                1445

cag cag gtc cgc aac cac gaa ctg gag aag aag cag agg agg ttt 4547
Gln Gln Val Arg Asn His Glu Leu Glu Lys Lys Gln Arg Arg Phe
    1450                1455                1460

gac agt gag ctc tcg cag gcg cat gag gag gcc cag cgg gag aag 4592
Asp Ser Glu Leu Ser Gln Ala His Glu Glu Ala Gln Arg Glu Lys
    1465                1470                1475

ctg cag cgg gag aag ctg cag cgg gag aag gac atg ctc ctc gct 4637
Leu Gln Arg Glu Lys Leu Gln Arg Glu Lys Asp Met Leu Leu Ala
    1480                1485                1490

gag gct ttc agc ctg aag cag caa cta gag gaa aaa gac atg gac 4682
Glu Ala Phe Ser Leu Lys Gln Gln Leu Glu Glu Lys Asp Met Asp
    1495                1500                1505

att gca ggg ttc acc cag aag gtt gtg tct cta gag gca gag ctc 4727
Ile Ala Gly Phe Thr Gln Lys Val Val Ser Leu Glu Ala Glu Leu
    1510                1515                1520

cag gac att tct tcc caa gag tcc aag gat gag gct tct ctg gcc 4772
Gln Asp Ile Ser Ser Gln Glu Ser Lys Asp Glu Ala Ser Leu Ala
    1525                1530                1535

aag gtc aag aaa cag ctc cgg gac ctg gag gcc aaa gtc aag gat 4817
Lys Val Lys Lys Gln Leu Arg Asp Leu Glu Ala Lys Val Lys Asp
    1540                1545                1550

cag gaa gaa gag ctg gat gag cag gca ggg acc atc cag atg ctg 4862
Gln Glu Glu Glu Leu Asp Glu Gln Ala Gly Thr Ile Gln Met Leu
    1555                1560                1565

gaa cag gcc aag ctg cgt ctg gag atg gag atg gag cgg atg aga 4907
Glu Gln Ala Lys Leu Arg Leu Glu Met Glu Met Glu Arg Met Arg
    1570                1575                1580

cag acc cat tct aag gag atg gag agt cgg gat gag gag gtg gag 4952
Gln Thr His Ser Lys Glu Met Glu Ser Arg Asp Glu Glu Val Glu
    1585                1590                1595

gag gcc cgg cag tcg tgt cag aag aag tta aaa cag atg gag gtg 4997
Glu Ala Arg Gln Ser Cys Gln Lys Lys Leu Lys Gln Met Glu Val
    1600                1605                1610

cag cta gag gaa gag tat gag gac aag cag aag gtt ctg cga gag 5042
Gln Leu Glu Glu Glu Tyr Glu Asp Lys Gln Lys Val Leu Arg Glu
    1615                1620                1625

aag cgg gag ctg gag ggc aag ctc gcc acc ctc agc gac cag gtg 5087
Lys Arg Glu Leu Glu Gly Lys Leu Ala Thr Leu Ser Asp Gln Val
    1630                1635                1640

aac cgg cgg gac ttt gag tca gag aag cgg ctg cgg aag gac ctg 5132
Asn Arg Arg Asp Phe Glu Ser Glu Lys Arg Leu Arg Lys Asp Leu
    1645                1650                1655

aag cgc acc aag gcc ctg ctg gca gat gcc cag ctc atg ctg gac 5177
Lys Arg Thr Lys Ala Leu Leu Ala Asp Ala Gln Leu Met Leu Asp
    1660                1665                1670

cac ctg aag aac agt gct ccc agc aag cga gag att gcc cag ctc 5222
His Leu Lys Asn Ser Ala Pro Ser Lys Arg Glu Ile Ala Gln Leu
    1675                1680                1685

aag aac cag ctg gag gag tca gag ttc acc tgt gcg gca gcc gtg 5267
Lys Asn Gln Leu Glu Glu Ser Glu Phe Thr Cys Ala Ala Ala Val
    1690                1695                1700

aaa gca cgg aaa gca atg gag gtg gag atc gaa gac ctg cac ctg 5312
Lys Ala Arg Lys Ala Met Glu Val Glu Ile Glu Asp Leu His Leu
    1705                1710                1715

cag att gat gac atc gcc aaa gcc aag aca gcg ctg gag gag cag 5357
Gln Ile Asp Asp Ile Ala Lys Ala Lys Thr Ala Leu Glu Glu Gln
    1720                1725                1730

ctg agc cgc ctt cag cgt gag aag aat gag atc cag aac cgg ctg 5402
Leu Ser Arg Leu Gln Arg Glu Lys Asn Glu Ile Gln Asn Arg Leu

1735                    1740                    1745

gag gaa  gat cag gaa gac atg  aac gaa ttg atg aag  aag cac aag        5447
Glu Glu  Asp Gln Glu Asp Met  Asn Glu Leu Met Lys  Lys His Lys
    1750                    1755                    1760

gct gcc  gtg gct cag gct tcc  cgg gac ctg gct cag  ata aat gat        5492
Ala Ala  Val Ala Gln Ala Ser  Arg Asp Leu Ala Gln  Ile Asn Asp
    1765                    1770                    1775

ctc caa  gct cag cta gaa gaa  gcc aac aaa gag aag  cag gag ctg        5537
Leu Gln  Ala Gln Leu Glu Glu  Ala Asn Lys Glu Lys  Gln Glu Leu
    1780                    1785                    1790

cag gag  aag cta caa gcc ctc  cag agc cag gtg gag  ttc ctg gag        5582
Gln Glu  Lys Leu Gln Ala Leu  Gln Ser Gln Val Glu  Phe Leu Glu
    1795                    1800                    1805

cag tcc  atg gtg gac aag tcc  ctg gtg agc agg cag  gaa gct aag        5627
Gln Ser  Met Val Asp Lys Ser  Leu Val Ser Arg Gln  Glu Ala Lys
    1810                    1815                    1820

ata cgg  gag ctg gag aca cgc  ctg gag ttt gaa agg  acg caa gtg        5672
Ile Arg  Glu Leu Glu Thr Arg  Leu Glu Phe Glu Arg  Thr Gln Val
    1825                    1830                    1835

aaa cgg  ctg gag agc ctg gct  agc cgt ctc aag gaa  aac atg gag        5717
Lys Arg  Leu Glu Ser Leu Ala  Ser Arg Leu Lys Glu  Asn Met Glu
    1840                    1845                    1850

aag ctg  act gag gag cgg gat  cag cgc att gca gcc  gag aac cgg        5762
Lys Leu  Thr Glu Glu Arg Asp  Gln Arg Ile Ala Ala  Glu Asn Arg
    1855                    1860                    1865

gag aag  gaa cag aac aag cgg  cta cag agg cag ctc  cgg gac acc        5807
Glu Lys  Glu Gln Asn Lys Arg  Leu Gln Arg Gln Leu  Arg Asp Thr
    1870                    1875                    1880

aag gag  gag atg ggc gag ctt  gcc agg aag gag gcc  gag gcg agc        5852
Lys Glu  Glu Met Gly Glu Leu  Ala Arg Lys Glu Ala  Glu Ala Ser
    1885                    1890                    1895

cgc aag  aag cac gaa ctg gag  atg gat cta gaa agc  ctg gag gct        5897
Arg Lys  Lys His Glu Leu Glu  Met Asp Leu Glu Ser  Leu Glu Ala
    1900                    1905                    1910

gct aac  cag agc ctg cag gct  gac cta aag ttg gca  ttc aag cgc        5942
Ala Asn  Gln Ser Leu Gln Ala  Asp Leu Lys Leu Ala  Phe Lys Arg
    1915                    1920                    1925

atc ggg  gac ctg cag gct gcc  att gag gat gag atg  gag agt gat        5987
Ile Gly  Asp Leu Gln Ala Ala  Ile Glu Asp Glu Met  Glu Ser Asp
    1930                    1935                    1940

gag aat  gag gac ctc atc aac  agt ttg cag gac atg  gtg aca aag        6032
Glu Asn  Glu Asp Leu Ile Asn  Ser Leu Gln Asp Met  Val Thr Lys
    1945                    1950                    1955

tat cag  aaa aga aag aat aaa  ctt gag gga gac tct  gat gtg gac        6077
Tyr Gln  Lys Arg Lys Asn Lys  Leu Glu Gly Asp Ser  Asp Val Asp
    1960                    1965                    1970

tcg gag  ctg gag gac cgt gtt  gac ggg gtc aag tcc  tgg ttg tca        6122

Ser Glu Leu Glu Asp Arg Val Asp Gly Val Lys Ser Trp Leu Ser
    1975                1980                1985

aaa aac aag gga cct tcc aag gca gct tct gat gat ggc agc tta 6167
Lys Asn Lys Gly Pro Ser Lys Ala Ala Ser Asp Asp Gly Ser Leu
    1990                1995                2000

aag agt tcc agc ccc acc agc tac tgg aag tcc ctt gcc cct gat 6212
Lys Ser Ser Ser Pro Thr Ser Tyr Trp Lys Ser Leu Ala Pro Asp
    2005                2010                2015

cgg tca gat gat gag cac gac cct ctc gac aac acc tcc aga ccg 6257
Arg Ser Asp Asp Glu His Asp Pro Leu Asp Asn Thr Ser Arg Pro
    2020                2025                2030

cga tac tcc cac agt tat ctg agt gac agc gac aca gag gcc aag 6302
Arg Tyr Ser His Ser Tyr Leu Ser Asp Ser Asp Thr Glu Ala Lys
    2035                2040                2045

ctg acg gag act aac gca tag cccaggggag tggttggcag ccctctcacc 6353
Leu Thr Glu Thr Asn Ala
    2050

ccagggcctg tggctgcctg ggcacctctc ccaggaagtg gtggggcacc ggtctccccc 6413

acccgactgc tgatctgcat gggaaacacc ctgaccttct tctgtcaggg gcactttcca 6473

ggctatgggt gtctgatgtc tccacgtgga agaggtgggg gaaagaggag tttctgaaga 6533

gaactttttg ctcctctgtc tcaaaatgcc agactcttgg cttctaccct gtgtcaccgt 6593

gggcagtggc aggtggcctg gcactgcatg gagccagcac gttgacctcc ctctcagctc 6653

cctgctcagg gacggtggac aggttgccta ctgggacact ctaggttgct gggtccatgg 6713

ggaggattgg gggaggagaa gcagtgcctt ccctctcgtg tggggtgggg gctctctctt 6773

cttggtgcct gctgtctttc tactttttaa tttaaatacc caacctctcc atcacagctg 6833

catccctgag agtgggaggg ggctgtagtg gtagctgggg ctcccaagaa cgactcggga 6893

atgtcatctc catcttcacc cttcagagag cagtcctttc tctgtgcagc tggagacgct 6953

ggtgaggaga gccgggtcca ggttcttaag aatgaggtgc ggaggggctc tccggtgctg 7013

ctgggctggg ttgagcaagc ctacgcagac aagtgtgtgt gtggaccatc cgcacctcca 7073

gcccccaccc caccctcttt gtctcagcgt gttatgtgca atgacctatt taaggtaaac 7133

ccattccaac tacagcagtt cagggctgat ccaagcactg cctccctcct gctctgtcca 7193

ggtggtctgg accataaact caacttgaga gggaaggctt ggggttgagg acttgtgatc 7253

agaaaaactg aagatggaag ttttggccgg tgctcattag acatgagtcc tcactctgtg 7313

tcctgagccc gtgtcattct tccaacctcc ctgcccccac acacttatcc cagacacaac 7373

accatgtggt ctggaggtcc cagcccccac cctaaaaagg ttatccctga gaactccacc 7433

agacttggga gcccaagtgc agtgcctggt gctgctccca tctgccgccc cccttctctc 7493

ctgcaattgg tttgtactca ctgggctgtg ctctcccctg tttacccgat gtatggaaat 7553

aaaggccctt ttcctcctgg ctgcgccagt aaaaaaaa                                    7591

<210>  80
<211>  2054
<212>  PRT
<213>  Homo sapiens

<400>  80

Met Phe Asn Leu Met Lys Lys Asp Lys Asp Lys Asp Gly Gly Arg Lys
1               5                   10                  15

Glu Lys Lys Glu Lys Lys Glu Lys Lys Glu Arg Met Ser Ala Ala Glu
            20                  25                  30

Leu Arg Ser Leu Glu Glu Met Ser Leu Arg Arg Gly Phe Phe Asn Leu
        35                  40                  45

Asn Arg Ser Ser Lys Arg Glu Ser Lys Thr Arg Leu Glu Ile Ser Asn
    50                  55                  60

Pro Ile Pro Ile Lys Val Ala Ser Gly Ser Asp Leu His Leu Thr Asp
65                  70                  75                  80

Ile Asp Ser Asp Ser Asn Arg Gly Ser Val Ile Leu Asp Ser Gly His
                85                  90                  95

Leu Ser Thr Ala Ser Ser Ser Asp Asp Leu Lys Gly Glu Glu Gly Ser
            100                 105                 110

Phe Arg Gly Ser Val Leu Gln Arg Ala Ala Lys Phe Gly Ser Leu Ala
        115                 120                 125

Lys Gln Asn Ser Gln Met Ile Val Lys Arg Phe Ser Phe Ser Gln Arg
    130                 135                 140

Ser Arg Asp Glu Ser Ala Ser Glu Thr Ser Thr Pro Ser Glu His Ser
145                 150                 155                 160

Ala Ala Pro Ser Pro Gln Val Glu Val Arg Thr Leu Glu Gly Gln Leu
                165                 170                 175

Val Gln His Pro Gly Pro Gly Ile Pro Arg Pro Gly His Arg Ser Arg
            180                 185                 190

Ala Pro Glu Leu Val Thr Lys Lys Phe Pro Val Asp Leu Arg Leu Pro
        195                 200                 205

Pro Val Val Pro Leu Pro Pro Pro Thr Leu Arg Glu Leu Glu Leu Gln

210                 215                 220

Arg Arg Pro Thr Gly Asp Phe Gly Phe Ser Leu Arg Arg Thr Thr Met
225                 230                 235                 240

Leu Asp Arg Gly Pro Glu Gly Gln Ala Cys Arg Arg Val Val His Phe
                245                 250                 255

Ala Glu Pro Gly Ala Gly Thr Lys Asp Leu Ala Leu Gly Leu Val Pro
            260                 265                 270

Gly Asp Arg Leu Val Glu Ile Asn Gly His Asn Val Glu Ser Lys Ser
        275                 280                 285

Arg Asp Glu Ile Val Glu Met Ile Arg Gln Ser Gly Asp Ser Val Arg
    290                 295                 300

Leu Lys Val Gln Pro Ile Pro Glu Leu Ser Glu Leu Ser Arg Ser Trp
305                 310                 315                 320

Leu Arg Ser Gly Glu Gly Pro Arg Arg Glu Pro Ser Asp Ala Lys Thr
                325                 330                 335

Glu Glu Gln Ile Ala Ala Glu Glu Ala Trp Asn Glu Thr Glu Lys Val
            340                 345                 350

Trp Leu Val His Arg Asp Gly Phe Ser Leu Ala Ser Gln Leu Lys Ser
        355                 360                 365

Glu Glu Leu Asn Leu Pro Glu Gly Lys Val Arg Val Lys Leu Asp His
    370                 375                 380

Asp Gly Ala Ile Leu Asp Val Asp Glu Asp Asp Val Glu Lys Ala Asn
385                 390                 395                 400

Ala Pro Ser Cys Asp Arg Leu Glu Asp Leu Ala Ser Leu Val Tyr Leu
                405                 410                 415

Asn Glu Ser Ser Val Leu His Thr Leu Arg Gln Arg Tyr Gly Ala Ser
            420                 425                 430

Leu Leu His Thr Tyr Ala Gly Pro Ser Leu Leu Val Leu Gly Pro Arg
        435                 440                 445

Gly Ala Pro Ala Val Tyr Ser Glu Lys Val Met His Met Phe Lys Gly
    450                 455                 460

Cys Arg Arg Glu Asp Met Ala Pro His Ile Tyr Ala Val Ala Gln Thr
465                 470                 475                 480

Ala Tyr Arg Ala Met Leu Met Ser Arg Gln Asp Gln Ser Ile Ile Leu
                485                 490                 495

Leu Gly Ser Ser Gly Ser Gly Lys Thr Thr Ser Cys Gln His Leu Val
            500                 505                 510

Gln Tyr Leu Ala Thr Ile Ala Gly Ile Ser Gly Asn Lys Val Phe Ser
        515                 520                 525

Val Glu Lys Trp Gln Ala Leu Tyr Thr Leu Leu Glu Ala Phe Gly Asn
    530                 535                 540

Ser Pro Thr Ile Ile Asn Gly Asn Ala Thr Arg Phe Ser Gln Ile Leu
545                 550                 555                 560

Ser Leu Asp Phe Asp Gln Ala Gly Gln Val Ala Ser Ala Ser Ile Gln
                565                 570                 575

Thr Met Leu Leu Glu Lys Leu Arg Val Ala Arg Arg Pro Ala Ser Glu
            580                 585                 590

Ala Thr Phe Asn Val Phe Tyr Tyr Leu Leu Ala Cys Gly Asp Gly Thr
        595                 600                 605

Leu Arg Thr Glu Leu His Leu Asn His Leu Ala Glu Asn Asn Val Phe
    610                 615                 620

Gly Ile Val Pro Leu Ala Lys Pro Glu Glu Lys Gln Lys Ala Ala Gln
625                 630                 635                 640

Gln Phe Ser Lys Leu Gln Ala Ala Met Lys Val Leu Gly Ile Ser Pro
                645                 650                 655

Asp Glu Gln Lys Ala Cys Trp Phe Ile Leu Ala Ala Ile Tyr His Leu
            660                 665                 670

Gly Ala Ala Gly Ala Thr Lys Glu Ala Ala Glu Ala Gly Arg Lys Gln
        675                 680                 685

Phe Ala Arg His Glu Trp Ala Gln Lys Ala Ala Tyr Leu Leu Gly Cys
    690                 695                 700

Ser Leu Glu Glu Leu Ser Ser Ala Ile Phe Lys His Gln His Lys Gly
705                 710                 715                 720

Gly Thr Leu Gln Arg Ser Thr Ser Phe Arg Gln Gly Pro Glu Glu Ser
                725                 730                 735

Gly Leu Gly Asp Gly Thr Gly Pro Lys Leu Ser Ala Leu Glu Cys Leu
            740                 745                 750

Glu Gly Met Ala Ala Gly Leu Tyr Ser Glu Leu Phe Thr Leu Leu Val
        755                 760                 765

Ser Leu Val Asn Arg Ala Leu Lys Ser Ser Gln His Ser Leu Cys Ser
    770                 775                 780

Met Met Ile Val Asp Thr Pro Gly Phe Gln Asn Pro Glu Gln Gly Gly
785                 790                 795                 800

Ser Ala Arg Gly Ala Ser Phe Glu Glu Leu Cys His Asn Tyr Thr Gln
                805                 810                 815

Asp Arg Leu Gln Arg Leu Phe His Glu Arg Thr Phe Val Gln Glu Leu
            820                 825                 830

Glu Arg Tyr Lys Glu Glu Asn Ile Glu Leu Ala Phe Asp Asp Leu Glu
        835                 840                 845

Pro Pro Thr Asp Asp Ser Val Ala Ala Val Asp Gln Ala Ser His Gln
    850                 855                 860

Ser Leu Val Arg Ser Leu Ala Arg Thr Asp Glu Ala Arg Gly Leu Leu
865                 870                 875                 880

Trp Leu Leu Glu Glu Glu Ala Leu Val Pro Gly Ala Ser Glu Asp Thr
                885                 890                 895

Leu Leu Glu Arg Leu Phe Ser Tyr Tyr Gly Pro Gln Glu Gly Asp Lys
            900                 905                 910

Lys Gly Gln Ser Pro Leu Leu His Ser Ser Lys Pro His His Phe Leu
        915                 920                 925

Leu Gly His Ser His Gly Thr Asn Trp Val Glu Tyr Asn Val Thr Gly
    930                 935                 940

Trp Leu Asn Tyr Thr Lys Gln Asn Pro Ala Thr Gln Asn Ala Pro Arg
945                 950                 955                 960

Leu Leu Gln Asp Ser Gln Lys Lys Ile Ile Ser Asn Leu Phe Leu Gly
                965                 970                 975

Arg Ala Gly Ser Ala Thr Val Leu Ser Gly Ser Ile Ala Gly Leu Glu
            980                 985                 990

Gly Gly Ser Gln Leu Ala Leu Arg Arg Ala Thr Ser Met Arg Lys Thr
        995                 1000                1005

Phe Thr Thr Gly Met Ala Ala Val Lys Lys Lys Ser Leu Cys Ile
    1010                1015                1020

Gln Met Lys Leu Gln Val Asp Ala Leu Ile Asp Thr Ile Lys Lys
    1025                1030                1035

Ser Lys Leu His Phe Val His Cys Phe Leu Pro Val Ala Glu Gly
    1040                1045                1050

Trp Ala Gly Glu Pro Arg Ser Ala Ser Ser Arg Arg Val Ser Ser
    1055                1060                1065

Ser Ser Glu Leu Asp Leu Pro Ser Gly Asp His Cys Glu Ala Gly
    1070                1075                1080

Leu Leu Gln Leu Asp Val Pro Leu Leu Arg Thr Gln Leu Arg Gly
    1085                1090                1095

Ser Arg Leu Leu Asp Ala Met Arg Met Tyr Arg Gln Gly Tyr Pro
    1100                1105                1110

Asp His Met Val Phe Ser Glu Phe Arg Arg Arg Phe Asp Val Leu
    1115                1120                1125

Ala Pro His Leu Thr Lys Lys His Gly Arg Asn Tyr Ile Val Val
    1130                1135                1140

Asp Glu Arg Arg Ala Val Glu Glu Leu Leu Glu Cys Leu Asp Leu
    1145                1150                1155

Glu Lys Ser Ser Cys Cys Met Gly Leu Ser Arg Val Phe Phe Arg
    1160                1165                1170

Ala Gly Thr Leu Ala Arg Leu Glu Glu Gln Arg Asp Glu Gln Thr
    1175                1180                1185

Ser Arg Asn Leu Thr Leu Phe Gln Ala Ala Cys Arg Gly Tyr Leu
    1190                1195                1200

Ala Arg Gln His Phe Lys Lys Arg Lys Ile Gln Asp Leu Ala Ile

1205                1210                1215

Arg Cys Val Gln Lys Asn Ile Lys Lys Asn Lys Gly Val Lys Asp
    1220                1225                1230

Trp Pro Trp Trp Lys Leu Phe Thr Thr Val Arg Pro Leu Ile Glu
    1235                1240                1245

Val Gln Leu Ser Glu Glu Gln Ile Arg Asn Lys Asp Glu Glu Ile
    1250                1255                1260

Gln Gln Leu Arg Ser Lys Leu Glu Lys Ala Glu Lys Glu Arg Asn
    1265                1270                1275

Glu Leu Arg Leu Asn Ser Asp Arg Leu Glu Ser Arg Ile Ser Glu
    1280                1285                1290

Leu Thr Ser Glu Leu Thr Asp Glu Arg Asn Thr Gly Glu Ser Ala
    1295                1300                1305

Ser Gln Leu Leu Asp Ala Glu Thr Ala Glu Arg Leu Arg Ala Glu
    1310                1315                1320

Lys Glu Met Lys Glu Leu Gln Thr Gln Tyr Asp Ala Leu Lys Lys
    1325                1330                1335

Gln Met Glu Val Met Glu Met Glu Val Met Glu Ala Arg Leu Ile
    1340                1345                1350

Arg Ala Ala Glu Ile Asn Gly Glu Val Asp Asp Asp Asp Ala Gly
    1355                1360                1365

Gly Glu Trp Arg Leu Lys Tyr Glu Arg Ala Val Arg Glu Val Asp
    1370                1375                1380

Phe Thr Lys Lys Arg Leu Gln Gln Glu Phe Glu Asp Lys Leu Glu
    1385                1390                1395

Val Glu Gln Gln Asn Lys Arg Gln Leu Glu Arg Arg Leu Gly Asp
    1400                1405                1410

Leu Gln Ala Asp Ser Glu Glu Ser Gln Arg Ala Leu Gln Gln Leu
    1415                1420                1425

Lys Lys Lys Cys Gln Arg Leu Thr Ala Glu Leu Gln Asp Thr Lys
    1430                1435                1440

Leu His Leu Glu Gly Gln Gln Val Arg Asn His Glu Leu Glu Lys
    1445                1450                1455

Lys Gln Arg Arg Phe Asp Ser Glu Leu Ser Gln Ala His Glu Glu
    1460                1465                1470

Ala Gln Arg Glu Lys Leu Gln Arg Glu Lys Leu Gln Arg Glu Lys
    1475                1480                1485

Asp Met Leu Leu Ala Glu Ala Phe Ser Leu Lys Gln Gln Leu Glu
    1490                1495                1500

Glu Lys Asp Met Asp Ile Ala Gly Phe Thr Gln Lys Val Val Ser
    1505                1510                1515

Leu Glu Ala Glu Leu Gln Asp Ile Ser Ser Gln Glu Ser Lys Asp
    1520                1525                1530

Glu Ala Ser Leu Ala Lys Val Lys Lys Gln Leu Arg Asp Leu Glu
    1535                1540                1545

Ala Lys Val Lys Asp Gln Glu Glu Glu Leu Asp Glu Gln Ala Gly
    1550                1555                1560

Thr Ile Gln Met Leu Glu Gln Ala Lys Leu Arg Leu Glu Met Glu
    1565                1570                1575

Met Glu Arg Met Arg Gln Thr His Ser Lys Glu Met Glu Ser Arg
    1580                1585                1590

Asp Glu Glu Val Glu Glu Ala Arg Gln Ser Cys Gln Lys Lys Leu
    1595                1600                1605

Lys Gln Met Glu Val Gln Leu Glu Glu Glu Tyr Glu Asp Lys Gln
    1610                1615                1620

Lys Val Leu Arg Glu Lys Arg Glu Leu Glu Gly Lys Leu Ala Thr
    1625                1630                1635

Leu Ser Asp Gln Val Asn Arg Arg Asp Phe Glu Ser Glu Lys Arg
    1640                1645                1650

Leu Arg Lys Asp Leu Lys Arg Thr Lys Ala Leu Leu Ala Asp Ala
    1655                1660                1665

Gln Leu Met Leu Asp His Leu Lys Asn Ser Ala Pro Ser Lys Arg
    1670                1675                1680

Glu Ile Ala Gln Leu Lys Asn Gln Leu Glu Glu Ser Glu Phe Thr
    1685                1690                1695

Cys Ala Ala Ala Val Lys Ala Arg Lys Ala Met Glu Val Glu Ile
    1700                1705                1710

Glu Asp Leu His Leu Gln Ile Asp Asp Ile Ala Lys Ala Lys Thr
    1715                1720                1725

Ala Leu Glu Glu Gln Leu Ser Arg Leu Gln Arg Glu Lys Asn Glu
    1730                1735                1740

Ile Gln Asn Arg Leu Glu Glu Asp Gln Glu Asp Met Asn Glu Leu
    1745                1750                1755

Met Lys Lys His Lys Ala Ala Val Ala Gln Ala Ser Arg Asp Leu
    1760                1765                1770

Ala Gln Ile Asn Asp Leu Gln Ala Gln Leu Glu Glu Ala Asn Lys
    1775                1780                1785

Glu Lys Gln Glu Leu Gln Glu Lys Leu Gln Ala Leu Gln Ser Gln
    1790                1795                1800

Val Glu Phe Leu Glu Gln Ser Met Val Asp Lys Ser Leu Val Ser
    1805                1810                1815

Arg Gln Glu Ala Lys Ile Arg Glu Leu Glu Thr Arg Leu Glu Phe
    1820                1825                1830

Glu Arg Thr Gln Val Lys Arg Leu Glu Ser Leu Ala Ser Arg Leu
    1835                1840                1845

Lys Glu Asn Met Glu Lys Leu Thr Glu Glu Arg Asp Gln Arg Ile
    1850                1855                1860

Ala Ala Glu Asn Arg Glu Lys Glu Gln Asn Lys Arg Leu Gln Arg
    1865                1870                1875

Gln Leu Arg Asp Thr Lys Glu Glu Met Gly Glu Leu Ala Arg Lys
    1880                1885                1890

Glu Ala Glu Ala Ser Arg Lys Lys His Glu Leu Glu Met Asp Leu
    1895                1900                1905

Glu Ser Leu Glu Ala Ala Asn Gln Ser Leu Gln Ala Asp Leu Lys
    1910                1915                1920

Leu Ala Phe Lys Arg Ile Gly Asp Leu Gln Ala Ala Ile Glu Asp
    1925                1930                1935

Glu Met Glu Ser Asp Glu Asn Glu Asp Leu Ile Asn Ser Leu Gln
    1940                1945                1950

Asp Met Val Thr Lys Tyr Gln Lys Arg Lys Asn Lys Leu Glu Gly
    1955                1960                1965

Asp Ser Asp Val Asp Ser Glu Leu Glu Asp Arg Val Asp Gly Val
    1970                1975                1980

Lys Ser Trp Leu Ser Lys Asn Lys Gly Pro Ser Lys Ala Ala Ser
    1985                1990                1995

Asp Asp Gly Ser Leu Lys Ser Ser Ser Pro Thr Ser Tyr Trp Lys
    2000                2005                2010

Ser Leu Ala Pro Asp Arg Ser Asp Asp Glu His Asp Pro Leu Asp
    2015                2020                2025

Asn Thr Ser Arg Pro Arg Tyr Ser His Ser Tyr Leu Ser Asp Ser
    2030                2035                2040

Asp Thr Glu Ala Lys Leu Thr Glu Thr Asn Ala
    2045                2050

<210> 81
<211> 8733
<212> DNA
<213> Homo sapiens

<220>
<221> CDS
<222> (122)..(7771)

<400> 81
gctcccggct tagaggacag cggggaaggc gggcggtggg gcagggggcc tgaagcggcg 60

gtaccggtgc tggcggcggc agctgaggcc ttggccgaag ccgcgcgaac ctcagggcaa 120

g atg ctt gga acc gga cct gcc gcc gcc acc acc gct gcc acc aca tct 169
Met Leu Gly Thr Gly Pro Ala Ala Ala Thr Thr Ala Ala Thr Thr Ser
1               5                   10                  15

agc aat gtg agc gtc ctg cag cag ttt gcc agt ggc cta aag agc cgg 217
Ser Asn Val Ser Val Leu Gln Gln Phe Ala Ser Gly Leu Lys Ser Arg
            20                  25                  30

aat gag gaa acc agg gcc aaa gcc gcc aag gag ctc cag cac tat gtc 265
Asn Glu Glu Thr Arg Ala Lys Ala Ala Lys Glu Leu Gln His Tyr Val

35                  40                  45

acc atg gaa ctc cga gag atg agt caa gag gag tct act cgc ttc tat      313
Thr Met Glu Leu Arg Glu Met Ser Gln Glu Glu Ser Thr Arg Phe Tyr
    50                  55                  60

gac caa ctg aac cat cac att ttt gaa ttg gtt tcc agc tca gat gcc      361
Asp Gln Leu Asn His His Ile Phe Glu Leu Val Ser Ser Ser Asp Ala
65                  70                  75                  80

aat gag agg aaa ggt ggc atc ttg gcc ata gct agc ctc ata gga gtg      409
Asn Glu Arg Lys Gly Gly Ile Leu Ala Ile Ala Ser Leu Ile Gly Val
                85                  90                  95

gaa ggt ggg aat gcc acc cga att ggc aga ttt gcc aac tat ctt cgg      457
Glu Gly Gly Asn Ala Thr Arg Ile Gly Arg Phe Ala Asn Tyr Leu Arg
            100                 105                 110

aac ctc ctc ccc tcc aat gac cca gtt gtc atg gaa atg gca tcc aag      505
Asn Leu Leu Pro Ser Asn Asp Pro Val Val Met Glu Met Ala Ser Lys
        115                 120                 125

gcc att ggc cgt ctt gcc atg gca ggg gac act ttt acc gct gag tac      553
Ala Ile Gly Arg Leu Ala Met Ala Gly Asp Thr Phe Thr Ala Glu Tyr
    130                 135                 140

gtg gaa ttt gag gtg aag cga gcc ctg gaa tgg ctg ggt gct gac cgc      601
Val Glu Phe Glu Val Lys Arg Ala Leu Glu Trp Leu Gly Ala Asp Arg
145                 150                 155                 160

aat gag ggc cgg aga cat gca gct gtc ctg gtt ctc cgt gag ctg gcc      649
Asn Glu Gly Arg Arg His Ala Ala Val Leu Val Leu Arg Glu Leu Ala
                165                 170                 175

atc agc gtc cct acc ttc ttc ttc cag caa gtg caa ccc ttc ttt gac      697
Ile Ser Val Pro Thr Phe Phe Phe Gln Gln Val Gln Pro Phe Phe Asp
            180                 185                 190

aac att ttt gtg gcc gtg tgg gac ccc aaa cag gcc atc cgt gag gga      745
Asn Ile Phe Val Ala Val Trp Asp Pro Lys Gln Ala Ile Arg Glu Gly
        195                 200                 205

gct gta gcc gcc ctt cgt gcc tgt ctg att ctc aca acc cag cgt gag      793
Ala Val Ala Ala Leu Arg Ala Cys Leu Ile Leu Thr Thr Gln Arg Glu
    210                 215                 220

ccg aag gag atg cag aag cct cag tgg tac agg cac aca ttt gaa gaa      841
Pro Lys Glu Met Gln Lys Pro Gln Trp Tyr Arg His Thr Phe Glu Glu
225                 230                 235                 240

gca gag aag gga ttt gat gag acc ttg gcc aaa gag aag ggc atg aat      889
Ala Glu Lys Gly Phe Asp Glu Thr Leu Ala Lys Glu Lys Gly Met Asn
                245                 250                 255

cgg gat gat cgg atc cat gga gcc ttg ttg atc ctt aac gag ctg gtc      937
Arg Asp Asp Arg Ile His Gly Ala Leu Leu Ile Leu Asn Glu Leu Val
            260                 265                 270

cga atc agc agc atg gag gga gag cgt ctg aga gaa gaa atg gaa gaa      985
Arg Ile Ser Ser Met Glu Gly Glu Arg Leu Arg Glu Glu Met Glu Glu
        275                 280                 285

atc aca cag cag cag ctg gta cac gac aag tac tgc aaa gat ctc atg      1033

Ile Thr Gln Gln Gln Leu Val His Asp Lys Tyr Cys Lys Asp Leu Met
    290                 295                 300

ggc ttc gga aca aaa cct cgt cac att acc ccc ttc acc agt ttc cag      1081
Gly Phe Gly Thr Lys Pro Arg His Ile Thr Pro Phe Thr Ser Phe Gln
305                 310                 315                 320

gct gta cag ccc cag cag tca aat gcc ttg gtg ggg ctg ctg ggg tac      1129
Ala Val Gln Pro Gln Gln Ser Asn Ala Leu Val Gly Leu Leu Gly Tyr
                325                 330                 335

agc tct cac caa ggc ctc atg gga ttt ggg acc tcc ccc agt cca gct      1177
Ser Ser His Gln Gly Leu Met Gly Phe Gly Thr Ser Pro Ser Pro Ala
            340                 345                 350

aag tcc acc ctg gtg gag agc cgg tgt tgc aga gac ttg atg gag gag      1225
Lys Ser Thr Leu Val Glu Ser Arg Cys Cys Arg Asp Leu Met Glu Glu
        355                 360                 365

aaa ttt gat cag gtg tgc cag tgg gtg ctg aaa tgc agg aat agc aag      1273
Lys Phe Asp Gln Val Cys Gln Trp Val Leu Lys Cys Arg Asn Ser Lys
    370                 375                 380

aac tcg ctg atc caa atg aca atc ctt aat ttg ttg ccc cgc ttg gct      1321
Asn Ser Leu Ile Gln Met Thr Ile Leu Asn Leu Leu Pro Arg Leu Ala
385                 390                 395                 400

gca ttc cga cct tct gcc ttc aca gat acc cag tat ctc caa gat acc      1369
Ala Phe Arg Pro Ser Ala Phe Thr Asp Thr Gln Tyr Leu Gln Asp Thr
                405                 410                 415

atg aac cat gtc cta agc tgt gtc aag aag gag aag gaa cgt aca gcg      1417
Met Asn His Val Leu Ser Cys Val Lys Lys Glu Lys Glu Arg Thr Ala
            420                 425                 430

gcc ttc caa gcc ctg ggg cta ctt tct gtg gct gtg agg tct gag ttt      1465
Ala Phe Gln Ala Leu Gly Leu Leu Ser Val Ala Val Arg Ser Glu Phe
        435                 440                 445

aag gtc tat ttg cct cgc gtg ctg gac atc atc cga gcg gcc ctg ccc      1513
Lys Val Tyr Leu Pro Arg Val Leu Asp Ile Ile Arg Ala Ala Leu Pro
    450                 455                 460

cca aag gac ttc gcc cat aag agg cag aag gca atg cag gtg gat gcc      1561
Pro Lys Asp Phe Ala His Lys Arg Gln Lys Ala Met Gln Val Asp Ala
465                 470                 475                 480

aca gtc ttc act tgc atc agc atg ctg gct cga gca atg ggg cca ggc      1609
Thr Val Phe Thr Cys Ile Ser Met Leu Ala Arg Ala Met Gly Pro Gly
                485                 490                 495

atc cag cag gat atc aag gag ctg ctg gag ccc atg ctg gca gtg gga      1657
Ile Gln Gln Asp Ile Lys Glu Leu Leu Glu Pro Met Leu Ala Val Gly
            500                 505                 510

cta agc cct gcc ctc act gca gtg ctc tac gac ctg agc cgt cag att      1705
Leu Ser Pro Ala Leu Thr Ala Val Leu Tyr Asp Leu Ser Arg Gln Ile
        515                 520                 525

cca cag cta aag aag gac att caa gat ggg cta ctg aaa atg ctg tcc      1753
Pro Gln Leu Lys Lys Asp Ile Gln Asp Gly Leu Leu Lys Met Leu Ser
    530                 535                 540

ctg gtc ctt atg cac aaa ccc ctt cgc cac cca ggc atg ccc aag ggc 1801
Leu Val Leu Met His Lys Pro Leu Arg His Pro Gly Met Pro Lys Gly
545                 550                 555                 560

ctg gcc cat cag ctg gcc tct cct ggc ctc acg acc ctc cct gag gcc 1849
Leu Ala His Gln Leu Ala Ser Pro Gly Leu Thr Thr Leu Pro Glu Ala
                565                 570                 575

agc gat gtg ggc agc atc act ctt gcc ctc cga acg ctt ggc agc ttt 1897
Ser Asp Val Gly Ser Ile Thr Leu Ala Leu Arg Thr Leu Gly Ser Phe
            580                 585                 590

gaa ttt gaa ggc cac tct ctg acc caa ttt gtt cgc cac tgt gcg gat 1945
Glu Phe Glu Gly His Ser Leu Thr Gln Phe Val Arg His Cys Ala Asp
        595                 600                 605

cat ttc ctg aac agt gag cac aag gag atc cgc atg gag gct gcc cgc 1993
His Phe Leu Asn Ser Glu His Lys Glu Ile Arg Met Glu Ala Ala Arg
    610                 615                 620

acc tgc tcc cgc ctg ctc aca ccc tcc atc cac ctc atc agt ggc cat 2041
Thr Cys Ser Arg Leu Leu Thr Pro Ser Ile His Leu Ile Ser Gly His
625                 630                 635                 640

gct cat gtg gtt agc cag acc gca gtg caa gtg gtg gca gat gtg ctt 2089
Ala His Val Val Ser Gln Thr Ala Val Gln Val Val Ala Asp Val Leu
                645                 650                 655

agc aaa ctg ctc gta gtt ggg ata aca gat cct gac cct gac att cgc 2137
Ser Lys Leu Leu Val Val Gly Ile Thr Asp Pro Asp Pro Asp Ile Arg
            660                 665                 670

tac tgt gtc ttg gcg tcc ctg gac gag cgc ttt gat gca cac ctg gcc 2185
Tyr Cys Val Leu Ala Ser Leu Asp Glu Arg Phe Asp Ala His Leu Ala
        675                 680                 685

cag gcg gag aac ttg cag gcc ttg ttt gtg gct ctg aat gac cag gtg 2233
Gln Ala Glu Asn Leu Gln Ala Leu Phe Val Ala Leu Asn Asp Gln Val
    690                 695                 700

ttt gag atc cgg gag ctg gcc atc tgc act gtg ggc cga ctc agt agc 2281
Phe Glu Ile Arg Glu Leu Ala Ile Cys Thr Val Gly Arg Leu Ser Ser
705                 710                 715                 720

atg aac cct gcc ttt gtc atg cct ttc ctg cgc aag atg ctc atc cag 2329
Met Asn Pro Ala Phe Val Met Pro Phe Leu Arg Lys Met Leu Ile Gln
                725                 730                 735

att ttg aca gag ttg gag cac agt ggg att gga aga atc aaa gag cag 2377
Ile Leu Thr Glu Leu Glu His Ser Gly Ile Gly Arg Ile Lys Glu Gln
            740                 745                 750

agt gcc cgc atg ctg ggg cac ctg gtc tcc aat gcc ccc cga ctc atc 2425
Ser Ala Arg Met Leu Gly His Leu Val Ser Asn Ala Pro Arg Leu Ile
        755                 760                 765

cgc ccc tac atg gag cct att ctg aag gca tta att ttg aaa ctg aaa 2473
Arg Pro Tyr Met Glu Pro Ile Leu Lys Ala Leu Ile Leu Lys Leu Lys
    770                 775                 780

gat cca gac cct gat cca aac cca ggt gtg atc aat aat gtc ctg gca 2521
Asp Pro Asp Pro Asp Pro Asn Pro Gly Val Ile Asn Asn Val Leu Ala
785                 790                 795                 800

aca ata gga gaa ttg gca cag gtt agt ggc ctg gaa atg agg aaa tgg 2569
Thr Ile Gly Glu Leu Ala Gln Val Ser Gly Leu Glu Met Arg Lys Trp
                805                 810                 815

gtt gat gaa ctt ttt att atc atc atg gac atg ctc cag gat tcc tct 2617
Val Asp Glu Leu Phe Ile Ile Ile Met Asp Met Leu Gln Asp Ser Ser
            820                 825                 830

ttg ttg gcc aaa agg cag gtg gct ctg tgg acc ctg gga cag ttg gtg 2665
Leu Leu Ala Lys Arg Gln Val Ala Leu Trp Thr Leu Gly Gln Leu Val
        835                 840                 845

gcc agc act ggc tat gta gta gag ccc tac agg aag tac cct act ttg 2713
Ala Ser Thr Gly Tyr Val Val Glu Pro Tyr Arg Lys Tyr Pro Thr Leu
    850                 855                 860

ctt gag gtg cta ctg aat ttt ctg aag act gag cag aac cag ggt aca 2761
Leu Glu Val Leu Leu Asn Phe Leu Lys Thr Glu Gln Asn Gln Gly Thr
865                 870                 875                 880

cgc aga gag gcc atc cgt gtg tta ggg ctt tta ggg gct ttg gat cct 2809
Arg Arg Glu Ala Ile Arg Val Leu Gly Leu Leu Gly Ala Leu Asp Pro
                885                 890                 895

tac aag cac aaa gtg aac att ggc atg ata gac cag tcc cgg gat gcc 2857
Tyr Lys His Lys Val Asn Ile Gly Met Ile Asp Gln Ser Arg Asp Ala
            900                 905                 910

tct gct gtc agc ctg tca gaa tcc aag tca agt cag gat tcc tct gac 2905
Ser Ala Val Ser Leu Ser Glu Ser Lys Ser Ser Gln Asp Ser Ser Asp
        915                 920                 925

tat agc act agt gaa atg ctg gtc aac atg gga aac ttg cct ctg gat 2953
Tyr Ser Thr Ser Glu Met Leu Val Asn Met Gly Asn Leu Pro Leu Asp
    930                 935                 940

gag ttc tac cca gct gtg tcc atg gtg gcc ctg atg cgg atc ttc cga 3001
Glu Phe Tyr Pro Ala Val Ser Met Val Ala Leu Met Arg Ile Phe Arg
945                 950                 955                 960

gac cag tca ctc tct cat cat cac acc atg gtt gtc cag gcc atc acc 3049
Asp Gln Ser Leu Ser His His His Thr Met Val Val Gln Ala Ile Thr
                965                 970                 975

ttc atc ttc aag tcc ctg gga ctc aaa tgt gtg cag ttc ctg ccc cag 3097
Phe Ile Phe Lys Ser Leu Gly Leu Lys Cys Val Gln Phe Leu Pro Gln
            980                 985                 990

gtc atg ccc acg ttc ctt aac gtc att cga gtc tgt gat ggg gcc atc 3145
Val Met Pro Thr Phe Leu Asn Val Ile Arg Val Cys Asp Gly Ala Ile
        995                 1000                1005

cgg gaa ttt ttg ttc cag cag ctg gga atg ttg gtg tcc ttt gtg 3190
Arg Glu Phe Leu Phe Gln Gln Leu Gly Met Leu Val Ser Phe Val
    1010                1015                1020

aag agc cac atc aga cct tat atg gat gaa ata gtc acc ctc atg 3235
Lys Ser His Ile Arg Pro Tyr Met Asp Glu Ile Val Thr Leu Met
    1025                1030                1035

aga gaa ttc tgg gtc atg aac acc tca att cag agc acg atc att 3280
Arg Glu Phe Trp Val Met Asn Thr Ser Ile Gln Ser Thr Ile Ile

1040                    1045                    1050

ctt ctc att gag caa att gtg gta gct ctt ggg ggt gaa ttt aag          3325
Leu Leu Ile Glu Gln Ile Val Val Ala Leu Gly Gly Glu Phe Lys
    1055                    1060                    1065

ctc tac ctg ccc cag ctg atc cca cac atg ctg cgt gtc ttc atg          3370
Leu Tyr Leu Pro Gln Leu Ile Pro His Met Leu Arg Val Phe Met
    1070                    1075                    1080

cat gac aac agc cca ggc cgc att gtc tct atc aag tta ctg gct          3415
His Asp Asn Ser Pro Gly Arg Ile Val Ser Ile Lys Leu Leu Ala
    1085                    1090                    1095

gca atc cag ctg ttt ggc gcc aac ctg gat gac tac ctg cat tta          3460
Ala Ile Gln Leu Phe Gly Ala Asn Leu Asp Asp Tyr Leu His Leu
    1100                    1105                    1110

ctg ctg cct cct att gtt aag ttg ttt gat gcc cct gaa gct cca          3505
Leu Leu Pro Pro Ile Val Lys Leu Phe Asp Ala Pro Glu Ala Pro
    1115                    1120                    1125

ctg cca tct cga aag gca gcg cta gag act gtg gac cgc ctg acg          3550
Leu Pro Ser Arg Lys Ala Ala Leu Glu Thr Val Asp Arg Leu Thr
    1130                    1135                    1140

gag tcc ctg gat ttc act gac tat gcc tcc cgg atc att cac cct          3595
Glu Ser Leu Asp Phe Thr Asp Tyr Ala Ser Arg Ile Ile His Pro
    1145                    1150                    1155

att gtt cga aca ctg gac cag agc cca gaa ctg cgc tcc aca gcc          3640
Ile Val Arg Thr Leu Asp Gln Ser Pro Glu Leu Arg Ser Thr Ala
    1160                    1165                    1170

atg gac acg ctg tct tca ctt gtt ttt cag ctg ggg aag aag tac          3685
Met Asp Thr Leu Ser Ser Leu Val Phe Gln Leu Gly Lys Lys Tyr
    1175                    1180                    1185

caa att ttc att cca atg gtg aat aaa gtt ctg gtg cga cac cga          3730
Gln Ile Phe Ile Pro Met Val Asn Lys Val Leu Val Arg His Arg
    1190                    1195                    1200

atc aat cat cag cgc tat gat gtg ctc atc tgc aga att gtc aag          3775
Ile Asn His Gln Arg Tyr Asp Val Leu Ile Cys Arg Ile Val Lys
    1205                    1210                    1215

gga tac aca ctt gct gat gaa gag gag gat cct ttg att tac cag          3820
Gly Tyr Thr Leu Ala Asp Glu Glu Glu Asp Pro Leu Ile Tyr Gln
    1220                    1225                    1230

cat cgg atg ctt agg agt ggc caa ggg gat gca ttg gct agt gga          3865
His Arg Met Leu Arg Ser Gly Gln Gly Asp Ala Leu Ala Ser Gly
    1235                    1240                    1245

cca gtg gaa aca gga ccc atg aag aaa ctg cac gtc agc acc atc          3910
Pro Val Glu Thr Gly Pro Met Lys Lys Leu His Val Ser Thr Ile
    1250                    1255                    1260

aac ctc caa aag gcc tgg ggc gct gcc agg agg gtc tcc aaa gat          3955
Asn Leu Gln Lys Ala Trp Gly Ala Ala Arg Arg Val Ser Lys Asp
    1265                    1270                    1275

gac tgg ctg gaa tgg ctg aga cgg ctg agc ctg gag ctg ctg aag          4000

Asp Trp Leu Glu Trp Leu Arg Arg Leu Ser Leu Glu Leu Leu Lys
    1280                1285                1290

gac tca tca tcg ccc tcc ctg cgc tcc tgc tgg gcc ctg gca cag 4045
Asp Ser Ser Ser Pro Ser Leu Arg Ser Cys Trp Ala Leu Ala Gln
    1295                1300                1305

gcc tac aac ccg atg gcc agg gat ctc ttc aat gct gca ttt gtg 4090
Ala Tyr Asn Pro Met Ala Arg Asp Leu Phe Asn Ala Ala Phe Val
    1310                1315                1320

tcc tgc tgg tct gaa ctg aat gaa gat caa cag gat gag ctc atc 4135
Ser Cys Trp Ser Glu Leu Asn Glu Asp Gln Gln Asp Glu Leu Ile
    1325                1330                1335

aga agc atc gag ttg gcc ctc acc tca caa gac atc gct gaa gtc 4180
Arg Ser Ile Glu Leu Ala Leu Thr Ser Gln Asp Ile Ala Glu Val
    1340                1345                1350

aca cag acc ctc tta aac ttg gct gaa ttc atg gaa cac agt gac 4225
Thr Gln Thr Leu Leu Asn Leu Ala Glu Phe Met Glu His Ser Asp
    1355                1360                1365

aag ggc ccc ctg cca ctg aga gat gac aat ggc att gtt ctg ctg 4270
Lys Gly Pro Leu Pro Leu Arg Asp Asp Asn Gly Ile Val Leu Leu
    1370                1375                1380

ggt gag aga gct gcc aag tgc cga gca tat gcc aaa gca cta cac 4315
Gly Glu Arg Ala Ala Lys Cys Arg Ala Tyr Ala Lys Ala Leu His
    1385                1390                1395

tac aaa gaa ctg gag ttc cag aaa ggc ccc acc cct gcc att cta 4360
Tyr Lys Glu Leu Glu Phe Gln Lys Gly Pro Thr Pro Ala Ile Leu
    1400                1405                1410

gaa tct ctc atc agc att aat aat aag cta cag cag ccg gag gca 4405
Glu Ser Leu Ile Ser Ile Asn Asn Lys Leu Gln Gln Pro Glu Ala
    1415                1420                1425

gcg gcc gga gtg tta gaa tat gcc atg aaa cac ttt gga gag ctg 4450
Ala Ala Gly Val Leu Glu Tyr Ala Met Lys His Phe Gly Glu Leu
    1430                1435                1440

gag atc cag gct acc tgg tat gag aaa ctg cac gag tgg gag gat 4495
Glu Ile Gln Ala Thr Trp Tyr Glu Lys Leu His Glu Trp Glu Asp
    1445                1450                1455

gcc ctt gtg gcc tat gac aag aaa atg gac acc aac aag gac gac 4540
Ala Leu Val Ala Tyr Asp Lys Lys Met Asp Thr Asn Lys Asp Asp
    1460                1465                1470

cca gag ctg atg ctg ggc cgc atg cgc tgc ctc gag gcc ttg ggg 4585
Pro Glu Leu Met Leu Gly Arg Met Arg Cys Leu Glu Ala Leu Gly
    1475                1480                1485

gaa tgg ggt caa ctc cac cag cag tgc tgt gaa aag tgg acc ctg 4630
Glu Trp Gly Gln Leu His Gln Gln Cys Cys Glu Lys Trp Thr Leu
    1490                1495                1500

gtt aat gat gag acc caa gcc aag atg gcc cgg atg gct gct gca 4675
Val Asn Asp Glu Thr Gln Ala Lys Met Ala Arg Met Ala Ala Ala
    1505                1510                1515

gct gca tgg ggt tta ggt cag tgg gac agc atg gaa gaa tac acc 4720
Ala Ala Trp Gly Leu Gly Gln Trp Asp Ser Met Glu Glu Tyr Thr
    1520                1525                1530

tgt atg atc cct cgg gac acc cat gat ggg gca ttt tat aga gct 4765
Cys Met Ile Pro Arg Asp Thr His Asp Gly Ala Phe Tyr Arg Ala
    1535                1540                1545

gtg ctg gca ctg cat cag gac ctc ttc tcc ttg gca caa cag tgc 4810
Val Leu Ala Leu His Gln Asp Leu Phe Ser Leu Ala Gln Gln Cys
    1550                1555                1560

att gac aag gcc agg gac ctg ctg gat gct gaa tta act gcg atg 4855
Ile Asp Lys Ala Arg Asp Leu Leu Asp Ala Glu Leu Thr Ala Met
    1565                1570                1575

gca gga gag agt tac agt cgg gca tat ggg gcc atg gtt tct tgc 4900
Ala Gly Glu Ser Tyr Ser Arg Ala Tyr Gly Ala Met Val Ser Cys
    1580                1585                1590

cac atg ctg tcc gag ctg gag gag gtt atc cag tac aaa ctt gtc 4945
His Met Leu Ser Glu Leu Glu Glu Val Ile Gln Tyr Lys Leu Val
    1595                1600                1605

ccc gag cga cga gag atc atc cgc cag atc tgg tgg gag aga ctg 4990
Pro Glu Arg Arg Glu Ile Ile Arg Gln Ile Trp Trp Glu Arg Leu
    1610                1615                1620

cag ggc tgc cag cgt atc gta gag gac tgg cag aaa atc ctt atg 5035
Gln Gly Cys Gln Arg Ile Val Glu Asp Trp Gln Lys Ile Leu Met
    1625                1630                1635

gtg cgg tcc ctt gtg gtc agc cct cat gaa gac atg aga acc tgg 5080
Val Arg Ser Leu Val Val Ser Pro His Glu Asp Met Arg Thr Trp
    1640                1645                1650

ctc aag tat gca agc ctg tgc ggc aag agt ggc agg ctg gct ctt 5125
Leu Lys Tyr Ala Ser Leu Cys Gly Lys Ser Gly Arg Leu Ala Leu
    1655                1660                1665

gct cat aaa act tta gtg ttg ctc ctg gga gtt gat ccg tct cgg 5170
Ala His Lys Thr Leu Val Leu Leu Leu Gly Val Asp Pro Ser Arg
    1670                1675                1680

caa ctt gac cat cct ctg cca aca gtt cac cct cag gtg acc tat 5215
Gln Leu Asp His Pro Leu Pro Thr Val His Pro Gln Val Thr Tyr
    1685                1690                1695

gcc tac atg aaa aac atg tgg aag agt gcc cgc aag atc gat gcc 5260
Ala Tyr Met Lys Asn Met Trp Lys Ser Ala Arg Lys Ile Asp Ala
    1700                1705                1710

ttc cag cac atg cag cat ttt gtc cag acc atg cag caa cag gcc 5305
Phe Gln His Met Gln His Phe Val Gln Thr Met Gln Gln Gln Ala
    1715                1720                1725

cag cat gcc atc gct act gag gac cag cag cat aag cag gaa ctg 5350
Gln His Ala Ile Ala Thr Glu Asp Gln Gln His Lys Gln Glu Leu
    1730                1735                1740

cac aag ctc atg gcc cga tgc ttc ctg aaa ctt gga gag tgg cag 5395
His Lys Leu Met Ala Arg Cys Phe Leu Lys Leu Gly Glu Trp Gln
    1745                1750                1755

ctg aat cta cag ggc atc aat gag agc aca atc ccc aaa gtg ctg 5440
Leu Asn Leu Gln Gly Ile Asn Glu Ser Thr Ile Pro Lys Val Leu
    1760                1765                1770

cag tac tac agc gcc gcc aca gag cac gac cgc agc tgg tac aag 5485
Gln Tyr Tyr Ser Ala Ala Thr Glu His Asp Arg Ser Trp Tyr Lys
    1775                1780                1785

gcc tgg cat gcg tgg gca gtg atg aac ttc gaa gct gtg cta cac 5530
Ala Trp His Ala Trp Ala Val Met Asn Phe Glu Ala Val Leu His
    1790                1795                1800

tac aaa cat cag aac caa gcc cgc gat gag aag aag aaa ctg cgt 5575
Tyr Lys His Gln Asn Gln Ala Arg Asp Glu Lys Lys Lys Leu Arg
    1805                1810                1815

cat gcc agc ggg gcc aac atc acc aac gcc acc act gcc gcc acc 5620
His Ala Ser Gly Ala Asn Ile Thr Asn Ala Thr Thr Ala Ala Thr
    1820                1825                1830

acg gcc gcc act gcc acc acc act gcc agc acc gag ggc agc aac 5665
Thr Ala Ala Thr Ala Thr Thr Thr Ala Ser Thr Glu Gly Ser Asn
    1835                1840                1845

agt gag agc gag gcc gag agc acc gag aac agc ccc acc cca tcg 5710
Ser Glu Ser Glu Ala Glu Ser Thr Glu Asn Ser Pro Thr Pro Ser
    1850                1855                1860

ccg ctg cag aag aag gtc act gag gat ctg tcc aaa acc ctc ctg 5755
Pro Leu Gln Lys Lys Val Thr Glu Asp Leu Ser Lys Thr Leu Leu
    1865                1870                1875

atg tac acg gtg cct gcc gtc cag ggc ttc ttc cgt tcc atc tcc 5800
Met Tyr Thr Val Pro Ala Val Gln Gly Phe Phe Arg Ser Ile Ser
    1880                1885                1890

ttg tca cga ggc aac aac ctc cag gat aca ctc aga gtt ctc acc 5845
Leu Ser Arg Gly Asn Asn Leu Gln Asp Thr Leu Arg Val Leu Thr
    1895                1900                1905

tta tgg ttt gat tat ggt cac tgg cca gat gtc aat gag gcc tta 5890
Leu Trp Phe Asp Tyr Gly His Trp Pro Asp Val Asn Glu Ala Leu
    1910                1915                1920

gtg gag ggg gtg aaa gcc atc cag att gat acc tgg cta cag gtt 5935
Val Glu Gly Val Lys Ala Ile Gln Ile Asp Thr Trp Leu Gln Val
    1925                1930                1935

ata cct cag ctc att gca aga att gat acg ccc aga ccc ttg gtg 5980
Ile Pro Gln Leu Ile Ala Arg Ile Asp Thr Pro Arg Pro Leu Val
    1940                1945                1950

gga cgt ctc att cac cag ctt ctc aca gac att ggt cgg tac cac 6025
Gly Arg Leu Ile His Gln Leu Leu Thr Asp Ile Gly Arg Tyr His
    1955                1960                1965

ccc cag gcc ctc atc tac cca ctg aca gtg gct tct aag tct acc 6070
Pro Gln Ala Leu Ile Tyr Pro Leu Thr Val Ala Ser Lys Ser Thr
    1970                1975                1980

acg aca gcc cgg cac aat gca gcc aac aag att ctg aag aac atg 6115
Thr Thr Ala Arg His Asn Ala Ala Asn Lys Ile Leu Lys Asn Met

1985                1990                1995

tgt gag cac agc aac acc ctg gtc cag cag gcc atg atg gtg agc        6160
Cys Glu His Ser Asn Thr Leu Val Gln Gln Ala Met Met Val Ser
    2000                2005                2010

gag gag ctg atc cga gtg gcc atc ctc tgg cat gag atg tgg cat        6205
Glu Glu Leu Ile Arg Val Ala Ile Leu Trp His Glu Met Trp His
    2015                2020                2025

gaa ggc ctg gaa gag gca tct cgt ttg tac ttt ggg gaa agg aac        6250
Glu Gly Leu Glu Glu Ala Ser Arg Leu Tyr Phe Gly Glu Arg Asn
    2030                2035                2040

gtg aaa ggc atg ttt gag gtg ctg gag ccc ttg cat gct atg atg        6295
Val Lys Gly Met Phe Glu Val Leu Glu Pro Leu His Ala Met Met
    2045                2050                2055

gaa cgg ggc ccc cag act ctg aag gaa aca tcc ttt aat cag gcc        6340
Glu Arg Gly Pro Gln Thr Leu Lys Glu Thr Ser Phe Asn Gln Ala
    2060                2065                2070

tat ggt cga gat tta atg gag gcc caa gag tgg tgc agg aag tac        6385
Tyr Gly Arg Asp Leu Met Glu Ala Gln Glu Trp Cys Arg Lys Tyr
    2075                2080                2085

atg aaa tca ggg aat gtc aag gac ctc acc caa gcc tgg gac ctc        6430
Met Lys Ser Gly Asn Val Lys Asp Leu Thr Gln Ala Trp Asp Leu
    2090                2095                2100

tat tat cat gtg ttc cga cga atc tca aag cag ctg cct cag ctc        6475
Tyr Tyr His Val Phe Arg Arg Ile Ser Lys Gln Leu Pro Gln Leu
    2105                2110                2115

aca tcc tta gag ctg caa tat gtt tcc cca aaa ctt ctg atg tgc        6520
Thr Ser Leu Glu Leu Gln Tyr Val Ser Pro Lys Leu Leu Met Cys
    2120                2125                2130

cgg gac ctt gaa ttg gct gtg cca gga aca tat gac ccc aac cag        6565
Arg Asp Leu Glu Leu Ala Val Pro Gly Thr Tyr Asp Pro Asn Gln
    2135                2140                2145

cca atc att cgc att cag tcc ata gca ccg tct ttg caa gtc atc        6610
Pro Ile Ile Arg Ile Gln Ser Ile Ala Pro Ser Leu Gln Val Ile
    2150                2155                2160

aca tcc aag cag agg ccc cgg aaa ttg aca ctt atg ggc agc aac        6655
Thr Ser Lys Gln Arg Pro Arg Lys Leu Thr Leu Met Gly Ser Asn
    2165                2170                2175

gga cat gag ttt gtt ttc ctt cta aaa ggc cat gaa gat ctg cgc        6700
Gly His Glu Phe Val Phe Leu Leu Lys Gly His Glu Asp Leu Arg
    2180                2185                2190

cag gat gag cgt gtg atg cag ctc ttc ggc ctg gtt aac acc ctt        6745
Gln Asp Glu Arg Val Met Gln Leu Phe Gly Leu Val Asn Thr Leu
    2195                2200                2205

ctg gcc aat gac cca aca tct ctt cgg aaa aac ctc agc atc cag        6790
Leu Ala Asn Asp Pro Thr Ser Leu Arg Lys Asn Leu Ser Ile Gln
    2210                2215                2220

aga tac gct gtc atc cct tta tcg acc aac tcg ggc ctc att ggc        6835

Arg Tyr Ala Val Ile Pro Leu Ser Thr Asn Ser Gly Leu Ile Gly
    2225                2230                2235

tgg gtt ccc cac tgt gac aca ctg cac gcc ctc atc cgg gac tac 6880
Trp Val Pro His Cys Asp Thr Leu His Ala Leu Ile Arg Asp Tyr
    2240                2245                2250

agg gag aag aag aag atc ctt ctc aac atc gag cat cgc atc atg 6925
Arg Glu Lys Lys Lys Ile Leu Leu Asn Ile Glu His Arg Ile Met
    2255                2260                2265

ttg cgg atg gct ccg gac tat gac cac ttg act ctg atg cag aag 6970
Leu Arg Met Ala Pro Asp Tyr Asp His Leu Thr Leu Met Gln Lys
    2270                2275                2280

gtg gag gtg ttt gag cat gcc gtc aat aat aca gct ggg gac gac 7015
Val Glu Val Phe Glu His Ala Val Asn Asn Thr Ala Gly Asp Asp
    2285                2290                2295

ctg gcc aag ctg ctg tgg ctg aaa agc ccc agc tcc gag gtg tgg 7060
Leu Ala Lys Leu Leu Trp Leu Lys Ser Pro Ser Ser Glu Val Trp
    2300                2305                2310

ttt gac cga aga acc aat tat acc cgt tct tta gcg gtc atg tca 7105
Phe Asp Arg Arg Thr Asn Tyr Thr Arg Ser Leu Ala Val Met Ser
    2315                2320                2325

atg gtt ggg tat att tta ggc ctg gga gat aga cac cca tcc aac 7150
Met Val Gly Tyr Ile Leu Gly Leu Gly Asp Arg His Pro Ser Asn
    2330                2335                2340

ctg atg ctg gac cgt ctg agt ggg aag atc ctg cac att gac ttt 7195
Leu Met Leu Asp Arg Leu Ser Gly Lys Ile Leu His Ile Asp Phe
    2345                2350                2355

ggg gac tgc ttt gag gtt gct atg acc cga gag aag ttt cca gag 7240
Gly Asp Cys Phe Glu Val Ala Met Thr Arg Glu Lys Phe Pro Glu
    2360                2365                2370

aag att cca ttt aga cta aca aga atg ttg acc aat gct atg gag 7285
Lys Ile Pro Phe Arg Leu Thr Arg Met Leu Thr Asn Ala Met Glu
    2375                2380                2385

gtt aca ggc ctg gat ggc aac tac aga atc aca tgc cac aca gtg 7330
Val Thr Gly Leu Asp Gly Asn Tyr Arg Ile Thr Cys His Thr Val
    2390                2395                2400

atg gag gtg ctg cga gag cac aag gac agt gtc atg gcc gtg ctg 7375
Met Glu Val Leu Arg Glu His Lys Asp Ser Val Met Ala Val Leu
    2405                2410                2415

gaa gcc ttt gtc tat gac ccc ttg ctg aac tgg agg ctg atg gac 7420
Glu Ala Phe Val Tyr Asp Pro Leu Leu Asn Trp Arg Leu Met Asp
    2420                2425                2430

aca aat acc aaa ggc aac aag cga tcc cga acg agg acg gat tcc 7465
Thr Asn Thr Lys Gly Asn Lys Arg Ser Arg Thr Arg Thr Asp Ser
    2435                2440                2445

tac tct gct ggc cag tca gtc gaa att ttg gac ggt gtg gaa ctt 7510
Tyr Ser Ala Gly Gln Ser Val Glu Ile Leu Asp Gly Val Glu Leu
    2450                2455                2460

gga gag  cca gcc cat aag aaa  acg ggg acc aca gtg  cca gaa tct      7555
Gly Glu  Pro Ala His Lys Lys  Thr Gly Thr Thr Val  Pro Glu Ser
    2465                 2470                 2475

att cat  tct ttc att gga gac  ggt ttg gtg aaa cca  gag gcc cta      7600
Ile His  Ser Phe Ile Gly Asp  Gly Leu Val Lys Pro  Glu Ala Leu
    2480                 2485                 2490

aat aag  aaa gct atc cag att  att aac agg gtt cga  gat aag ctc      7645
Asn Lys  Lys Ala Ile Gln Ile  Ile Asn Arg Val Arg  Asp Lys Leu
    2495                 2500                 2505

act ggt  cgg gac ttc tct cat  gat gac act ttg gat  gtt cca acg      7690
Thr Gly  Arg Asp Phe Ser His  Asp Asp Thr Leu Asp  Val Pro Thr
    2510                 2515                 2520

caa gtt  gag ctg ctc atc aaa  caa gcg aca tcc cat  gaa aac ctc      7735
Gln Val  Glu Leu Leu Ile Lys  Gln Ala Thr Ser His  Glu Asn Leu
    2525                 2530                 2535

tgc cag  tgc tat att ggc tgg  tgc cct ttc tgg taa ctggaggccc        7781
Cys Gln  Cys Tyr Ile Gly Trp  Cys Pro Phe Trp
    2540                 2545

agatgtgccc atcacgtttt ttctgaggct tttgtacttt agtaaatgct tccactaaac      7841

tgaaaccatg gtgagaaagt ttgactttgt taaatatttt gaaatgtaaa tgaaaagaac      7901

tactgtatat taaaagttgg tttgaaccaa ctttctagct gctgttgaag aatatattgt      7961

cagaaacaca aggcttgatt tggttcccag gacagtgaaa catagtaata ccacgtaaat      8021

caagccattc attttgggga acagaagatc cataacttta gaaatacggg ttttgactta      8081

actcacaaga gaactcatca taagtacttg ctgatggaag aatgacctag ttgctcctct      8141

caacatgggt acagcaaact cagcacagcc aagaagcctc aggtcgtgga gaacatggat      8201

taggatccta gactgtaaag acacagaaga tgctgacctc acccctgcca cctatcccaa      8261

gacctcactg gtctgtggac agcagcagaa atgtttgcaa gataggccaa aatgagtaca      8321

aaaggtctgt cttccatcag acccagtgat gctgcgactc acacgcttca attcaagacc      8381

tgaccgctag tagggaggtt tattcagatc gctggcagcc tcggctgagc agatgcacag      8441

aggggatcac tgtgcagtgg gaccaccctc actggccttc tgcagcaggg ttctgggatg      8501

ttttcagtgg tcaaaatact ctgtttagag caagggctca gaaaacagaa atactgtcat      8561

ggaggtgctg aacacaggga aggtctggta catattggaa attatgagca gaacaaatac      8621

tcaactaaat gcacaaagta taaagtgtag ccatgtctag acaccatgtt gtatcagaat      8681

aatttttgtg ccaataaatg acatcagaat tttaaacata tgtaaaaaaa aa              8733

<210>  82
<211>  2549
<212>  PRT
<213>  Homo sapiens

<400>  82

Met Leu Gly Thr Gly Pro Ala Ala Ala Thr Thr Ala Ala Thr Thr Ser
1               5                   10                  15

Ser Asn Val Ser Val Leu Gln Gln Phe Ala Ser Gly Leu Lys Ser Arg
            20                  25                  30

Asn Glu Glu Thr Arg Ala Lys Ala Ala Lys Glu Leu Gln His Tyr Val
        35                  40                  45

Thr Met Glu Leu Arg Glu Met Ser Gln Glu Glu Ser Thr Arg Phe Tyr
    50                  55                  60

Asp Gln Leu Asn His His Ile Phe Glu Leu Val Ser Ser Ser Asp Ala
65                  70                  75                  80

Asn Glu Arg Lys Gly Gly Ile Leu Ala Ile Ala Ser Leu Ile Gly Val
                85                  90                  95

Glu Gly Gly Asn Ala Thr Arg Ile Gly Arg Phe Ala Asn Tyr Leu Arg
            100                 105                 110

Asn Leu Leu Pro Ser Asn Asp Pro Val Val Met Glu Met Ala Ser Lys
        115                 120                 125

Ala Ile Gly Arg Leu Ala Met Ala Gly Asp Thr Phe Thr Ala Glu Tyr
    130                 135                 140

Val Glu Phe Glu Val Lys Arg Ala Leu Glu Trp Leu Gly Ala Asp Arg
145                 150                 155                 160

Asn Glu Gly Arg Arg His Ala Ala Val Leu Val Leu Arg Glu Leu Ala
                165                 170                 175

Ile Ser Val Pro Thr Phe Phe Phe Gln Gln Val Gln Pro Phe Phe Asp
            180                 185                 190

Asn Ile Phe Val Ala Val Trp Asp Pro Lys Gln Ala Ile Arg Glu Gly
        195                 200                 205

Ala Val Ala Ala Leu Arg Ala Cys Leu Ile Leu Thr Thr Gln Arg Glu
    210                 215                 220

Pro Lys Glu Met Gln Lys Pro Gln Trp Tyr Arg His Thr Phe Glu Glu
225                 230                 235                 240

Ala Glu Lys Gly Phe Asp Glu Thr Leu Ala Lys Glu Lys Gly Met Asn
                245                 250                 255

Arg Asp Asp Arg Ile His Gly Ala Leu Leu Ile Leu Asn Glu Leu Val
            260                 265                 270

Arg Ile Ser Ser Met Glu Gly Glu Arg Leu Arg Glu Glu Met Glu Glu
        275                 280                 285

Ile Thr Gln Gln Gln Leu Val His Asp Lys Tyr Cys Lys Asp Leu Met
    290                 295                 300

Gly Phe Gly Thr Lys Pro Arg His Ile Thr Pro Phe Thr Ser Phe Gln
305                 310                 315                 320

Ala Val Gln Pro Gln Gln Ser Asn Ala Leu Val Gly Leu Leu Gly Tyr
                325                 330                 335

Ser Ser His Gln Gly Leu Met Gly Phe Gly Thr Ser Pro Ser Pro Ala
            340                 345                 350

Lys Ser Thr Leu Val Glu Ser Arg Cys Cys Arg Asp Leu Met Glu Glu
        355                 360                 365

Lys Phe Asp Gln Val Cys Gln Trp Val Leu Lys Cys Arg Asn Ser Lys
    370                 375                 380

Asn Ser Leu Ile Gln Met Thr Ile Leu Asn Leu Leu Pro Arg Leu Ala
385                 390                 395                 400

Ala Phe Arg Pro Ser Ala Phe Thr Asp Thr Gln Tyr Leu Gln Asp Thr
                405                 410                 415

Met Asn His Val Leu Ser Cys Val Lys Lys Glu Lys Glu Arg Thr Ala
            420                 425                 430

Ala Phe Gln Ala Leu Gly Leu Leu Ser Val Ala Val Arg Ser Glu Phe
        435                 440                 445

Lys Val Tyr Leu Pro Arg Val Leu Asp Ile Ile Arg Ala Ala Leu Pro
    450                 455                 460

Pro Lys Asp Phe Ala His Lys Arg Gln Lys Ala Met Gln Val Asp Ala
465                 470                 475                 480

Thr Val Phe Thr Cys Ile Ser Met Leu Ala Arg Ala Met Gly Pro Gly
                485                 490                 495

Ile Gln Gln Asp Ile Lys Glu Leu Leu Glu Pro Met Leu Ala Val Gly

500                 505                 510

Leu Ser Pro Ala Leu Thr Ala Val Leu Tyr Asp Leu Ser Arg Gln Ile
        515                 520                 525

Pro Gln Leu Lys Lys Asp Ile Gln Asp Gly Leu Leu Lys Met Leu Ser
    530                 535                 540

Leu Val Leu Met His Lys Pro Leu Arg His Pro Gly Met Pro Lys Gly
545                 550                 555                 560

Leu Ala His Gln Leu Ala Ser Pro Gly Leu Thr Thr Leu Pro Glu Ala
                565                 570                 575

Ser Asp Val Gly Ser Ile Thr Leu Ala Leu Arg Thr Leu Gly Ser Phe
            580                 585                 590

Glu Phe Glu Gly His Ser Leu Thr Gln Phe Val Arg His Cys Ala Asp
        595                 600                 605

His Phe Leu Asn Ser Glu His Lys Glu Ile Arg Met Glu Ala Ala Arg
    610                 615                 620

Thr Cys Ser Arg Leu Leu Thr Pro Ser Ile His Leu Ile Ser Gly His
625                 630                 635                 640

Ala His Val Val Ser Gln Thr Ala Val Gln Val Val Ala Asp Val Leu
                645                 650                 655

Ser Lys Leu Leu Val Val Gly Ile Thr Asp Pro Asp Pro Asp Ile Arg
            660                 665                 670

Tyr Cys Val Leu Ala Ser Leu Asp Glu Arg Phe Asp Ala His Leu Ala
        675                 680                 685

Gln Ala Glu Asn Leu Gln Ala Leu Phe Val Ala Leu Asn Asp Gln Val
    690                 695                 700

Phe Glu Ile Arg Glu Leu Ala Ile Cys Thr Val Gly Arg Leu Ser Ser
705                 710                 715                 720

Met Asn Pro Ala Phe Val Met Pro Phe Leu Arg Lys Met Leu Ile Gln
                725                 730                 735

Ile Leu Thr Glu Leu Glu His Ser Gly Ile Gly Arg Ile Lys Glu Gln
            740                 745                 750

Ser Ala Arg Met Leu Gly His Leu Val Ser Asn Ala Pro Arg Leu Ile
        755                 760                 765

Arg Pro Tyr Met Glu Pro Ile Leu Lys Ala Leu Ile Leu Lys Leu Lys
    770                 775                 780

Asp Pro Asp Pro Asp Pro Asn Pro Gly Val Ile Asn Asn Val Leu Ala
785                 790                 795                 800

Thr Ile Gly Glu Leu Ala Gln Val Ser Gly Leu Glu Met Arg Lys Trp
                805                 810                 815

Val Asp Glu Leu Phe Ile Ile Ile Met Asp Met Leu Gln Asp Ser Ser
            820                 825                 830

Leu Leu Ala Lys Arg Gln Val Ala Leu Trp Thr Leu Gly Gln Leu Val
        835                 840                 845

Ala Ser Thr Gly Tyr Val Val Glu Pro Tyr Arg Lys Tyr Pro Thr Leu
    850                 855                 860

Leu Glu Val Leu Leu Asn Phe Leu Lys Thr Glu Gln Asn Gln Gly Thr
865                 870                 875                 880

Arg Arg Glu Ala Ile Arg Val Leu Gly Leu Leu Gly Ala Leu Asp Pro
                885                 890                 895

Tyr Lys His Lys Val Asn Ile Gly Met Ile Asp Gln Ser Arg Asp Ala
            900                 905                 910

Ser Ala Val Ser Leu Ser Glu Ser Lys Ser Ser Gln Asp Ser Ser Asp
        915                 920                 925

Tyr Ser Thr Ser Glu Met Leu Val Asn Met Gly Asn Leu Pro Leu Asp
    930                 935                 940

Glu Phe Tyr Pro Ala Val Ser Met Val Ala Leu Met Arg Ile Phe Arg
945                 950                 955                 960

Asp Gln Ser Leu Ser His His His Thr Met Val Val Gln Ala Ile Thr
                965                 970                 975

Phe Ile Phe Lys Ser Leu Gly Leu Lys Cys Val Gln Phe Leu Pro Gln
            980                 985                 990

Val Met Pro Thr Phe Leu Asn Val  Ile Arg Val Cys Asp  Gly Ala Ile
        995                 1000                1005

Arg Glu Phe Leu Phe Gln Gln Leu Gly Met Leu Val Ser Phe Val
    1010                1015                1020

Lys Ser His Ile Arg Pro Tyr Met Asp Glu Ile Val Thr Leu Met
    1025                1030                1035

Arg Glu Phe Trp Val Met Asn Thr Ser Ile Gln Ser Thr Ile Ile
    1040                1045                1050

Leu Leu Ile Glu Gln Ile Val Val Ala Leu Gly Gly Glu Phe Lys
    1055                1060                1065

Leu Tyr Leu Pro Gln Leu Ile Pro His Met Leu Arg Val Phe Met
    1070                1075                1080

His Asp Asn Ser Pro Gly Arg Ile Val Ser Ile Lys Leu Leu Ala
    1085                1090                1095

Ala Ile Gln Leu Phe Gly Ala Asn Leu Asp Asp Tyr Leu His Leu
    1100                1105                1110

Leu Leu Pro Pro Ile Val Lys Leu Phe Asp Ala Pro Glu Ala Pro
    1115                1120                1125

Leu Pro Ser Arg Lys Ala Ala Leu Glu Thr Val Asp Arg Leu Thr
    1130                1135                1140

Glu Ser Leu Asp Phe Thr Asp Tyr Ala Ser Arg Ile Ile His Pro
    1145                1150                1155

Ile Val Arg Thr Leu Asp Gln Ser Pro Glu Leu Arg Ser Thr Ala
    1160                1165                1170

Met Asp Thr Leu Ser Ser Leu Val Phe Gln Leu Gly Lys Lys Tyr
    1175                1180                1185

Gln Ile Phe Ile Pro Met Val Asn Lys Val Leu Val Arg His Arg
    1190                1195                1200

Ile Asn His Gln Arg Tyr Asp Val Leu Ile Cys Arg Ile Val Lys
    1205                1210                1215

Gly Tyr Thr Leu Ala Asp Glu Glu Glu Asp Pro Leu Ile Tyr Gln
    1220                1225                1230

His Arg Met Leu Arg Ser Gly Gln Gly Asp Ala Leu Ala Ser Gly
    1235                1240                1245

Pro Val Glu Thr Gly Pro Met Lys Lys Leu His Val Ser Thr Ile
    1250                1255                1260

Asn Leu Gln Lys Ala Trp Gly Ala Ala Arg Arg Val Ser Lys Asp
    1265                1270                1275

Asp Trp Leu Glu Trp Leu Arg Arg Leu Ser Leu Glu Leu Leu Lys
    1280                1285                1290

Asp Ser Ser Ser Pro Ser Leu Arg Ser Cys Trp Ala Leu Ala Gln
    1295                1300                1305

Ala Tyr Asn Pro Met Ala Arg Asp Leu Phe Asn Ala Ala Phe Val
    1310                1315                1320

Ser Cys Trp Ser Glu Leu Asn Glu Asp Gln Gln Asp Glu Leu Ile
    1325                1330                1335

Arg Ser Ile Glu Leu Ala Leu Thr Ser Gln Asp Ile Ala Glu Val
    1340                1345                1350

Thr Gln Thr Leu Leu Asn Leu Ala Glu Phe Met Glu His Ser Asp
    1355                1360                1365

Lys Gly Pro Leu Pro Leu Arg Asp Asp Asn Gly Ile Val Leu Leu
    1370                1375                1380

Gly Glu Arg Ala Ala Lys Cys Arg Ala Tyr Ala Lys Ala Leu His
    1385                1390                1395

Tyr Lys Glu Leu Glu Phe Gln Lys Gly Pro Thr Pro Ala Ile Leu
    1400                1405                1410

Glu Ser Leu Ile Ser Ile Asn Asn Lys Leu Gln Gln Pro Glu Ala
    1415                1420                1425

Ala Ala Gly Val Leu Glu Tyr Ala Met Lys His Phe Gly Glu Leu
    1430                1435                1440

Glu Ile Gln Ala Thr Trp Tyr Glu Lys Leu His Glu Trp Glu Asp
    1445                1450                1455

Ala Leu Val Ala Tyr Asp Lys Lys Met Asp Thr Asn Lys Asp Asp
    1460                1465                1470

Pro Glu Leu Met Leu Gly Arg Met Arg Cys Leu Glu Ala Leu Gly

1475                1480                1485

Glu Trp Gly Gln Leu His Gln Gln Cys Cys Glu Lys Trp Thr Leu
    1490                1495                1500

Val Asn Asp Glu Thr Gln Ala Lys Met Ala Arg Met Ala Ala Ala
    1505                1510                1515

Ala Ala Trp Gly Leu Gly Gln Trp Asp Ser Met Glu Glu Tyr Thr
    1520                1525                1530

Cys Met Ile Pro Arg Asp Thr His Asp Gly Ala Phe Tyr Arg Ala
    1535                1540                1545

Val Leu Ala Leu His Gln Asp Leu Phe Ser Leu Ala Gln Gln Cys
    1550                1555                1560

Ile Asp Lys Ala Arg Asp Leu Leu Asp Ala Glu Leu Thr Ala Met
    1565                1570                1575

Ala Gly Glu Ser Tyr Ser Arg Ala Tyr Gly Ala Met Val Ser Cys
    1580                1585                1590

His Met Leu Ser Glu Leu Glu Glu Val Ile Gln Tyr Lys Leu Val
    1595                1600                1605

Pro Glu Arg Arg Glu Ile Ile Arg Gln Ile Trp Trp Glu Arg Leu
    1610                1615                1620

Gln Gly Cys Gln Arg Ile Val Glu Asp Trp Gln Lys Ile Leu Met
    1625                1630                1635

Val Arg Ser Leu Val Val Ser Pro His Glu Asp Met Arg Thr Trp
    1640                1645                1650

Leu Lys Tyr Ala Ser Leu Cys Gly Lys Ser Gly Arg Leu Ala Leu
    1655                1660                1665

Ala His Lys Thr Leu Val Leu Leu Leu Gly Val Asp Pro Ser Arg
    1670                1675                1680

Gln Leu Asp His Pro Leu Pro Thr Val His Pro Gln Val Thr Tyr
    1685                1690                1695

Ala Tyr Met Lys Asn Met Trp Lys Ser Ala Arg Lys Ile Asp Ala
    1700                1705                1710

Phe Gln His Met Gln His Phe Val Gln Thr Met Gln Gln Gln Ala
    1715                1720                1725

Gln His Ala Ile Ala Thr Glu Asp Gln Gln His Lys Gln Glu Leu
    1730                1735                1740

His Lys Leu Met Ala Arg Cys Phe Leu Lys Leu Gly Glu Trp Gln
    1745                1750                1755

Leu Asn Leu Gln Gly Ile Asn Glu Ser Thr Ile Pro Lys Val Leu
    1760                1765                1770

Gln Tyr Tyr Ser Ala Ala Thr Glu His Asp Arg Ser Trp Tyr Lys
    1775                1780                1785

Ala Trp His Ala Trp Ala Val Met Asn Phe Glu Ala Val Leu His
    1790                1795                1800

Tyr Lys His Gln Asn Gln Ala Arg Asp Glu Lys Lys Lys Leu Arg
    1805                1810                1815

His Ala Ser Gly Ala Asn Ile Thr Asn Ala Thr Thr Ala Ala Thr
    1820                1825                1830

Thr Ala Ala Thr Ala Thr Thr Thr Ala Ser Thr Glu Gly Ser Asn
    1835                1840                1845

Ser Glu Ser Glu Ala Glu Ser Thr Glu Asn Ser Pro Thr Pro Ser
    1850                1855                1860

Pro Leu Gln Lys Lys Val Thr Glu Asp Leu Ser Lys Thr Leu Leu
    1865                1870                1875

Met Tyr Thr Val Pro Ala Val Gln Gly Phe Phe Arg Ser Ile Ser
    1880                1885                1890

Leu Ser Arg Gly Asn Asn Leu Gln Asp Thr Leu Arg Val Leu Thr
    1895                1900                1905

Leu Trp Phe Asp Tyr Gly His Trp Pro Asp Val Asn Glu Ala Leu
    1910                1915                1920

Val Glu Gly Val Lys Ala Ile Gln Ile Asp Thr Trp Leu Gln Val
    1925                1930                1935

Ile Pro Gln Leu Ile Ala Arg Ile Asp Thr Pro Arg Pro Leu Val
    1940                1945                1950

Gly Arg Leu Ile His Gln Leu Leu Thr Asp Ile Gly Arg Tyr His
    1955                1960                1965

Pro Gln Ala Leu Ile Tyr Pro Leu Thr Val Ala Ser Lys Ser Thr
    1970                1975                1980

Thr Thr Ala Arg His Asn Ala Ala Asn Lys Ile Leu Lys Asn Met
    1985                1990                1995

Cys Glu His Ser Asn Thr Leu Val Gln Gln Ala Met Met Val Ser
    2000                2005                2010

Glu Glu Leu Ile Arg Val Ala Ile Leu Trp His Glu Met Trp His
    2015                2020                2025

Glu Gly Leu Glu Glu Ala Ser Arg Leu Tyr Phe Gly Glu Arg Asn
    2030                2035                2040

Val Lys Gly Met Phe Glu Val Leu Glu Pro Leu His Ala Met Met
    2045                2050                2055

Glu Arg Gly Pro Gln Thr Leu Lys Glu Thr Ser Phe Asn Gln Ala
    2060                2065                2070

Tyr Gly Arg Asp Leu Met Glu Ala Gln Glu Trp Cys Arg Lys Tyr
    2075                2080                2085

Met Lys Ser Gly Asn Val Lys Asp Leu Thr Gln Ala Trp Asp Leu
    2090                2095                2100

Tyr Tyr His Val Phe Arg Arg Ile Ser Lys Gln Leu Pro Gln Leu
    2105                2110                2115

Thr Ser Leu Glu Leu Gln Tyr Val Ser Pro Lys Leu Leu Met Cys
    2120                2125                2130

Arg Asp Leu Glu Leu Ala Val Pro Gly Thr Tyr Asp Pro Asn Gln
    2135                2140                2145

Pro Ile Ile Arg Ile Gln Ser Ile Ala Pro Ser Leu Gln Val Ile
    2150                2155                2160

Thr Ser Lys Gln Arg Pro Arg Lys Leu Thr Leu Met Gly Ser Asn
    2165                2170                2175

Gly His Glu Phe Val Phe Leu Leu Lys Gly His Glu Asp Leu Arg
    2180                2185                2190

Gln Asp Glu Arg Val Met Gln Leu Phe Gly Leu Val Asn Thr Leu
    2195                2200                2205

Leu Ala Asn Asp Pro Thr Ser Leu Arg Lys Asn Leu Ser Ile Gln
    2210                2215                2220

Arg Tyr Ala Val Ile Pro Leu Ser Thr Asn Ser Gly Leu Ile Gly
    2225                2230                2235

Trp Val Pro His Cys Asp Thr Leu His Ala Leu Ile Arg Asp Tyr
    2240                2245                2250

Arg Glu Lys Lys Lys Ile Leu Leu Asn Ile Glu His Arg Ile Met
    2255                2260                2265

Leu Arg Met Ala Pro Asp Tyr Asp His Leu Thr Leu Met Gln Lys
    2270                2275                2280

Val Glu Val Phe Glu His Ala Val Asn Asn Thr Ala Gly Asp Asp
    2285                2290                2295

Leu Ala Lys Leu Leu Trp Leu Lys Ser Pro Ser Ser Glu Val Trp
    2300                2305                2310

Phe Asp Arg Arg Thr Asn Tyr Thr Arg Ser Leu Ala Val Met Ser
    2315                2320                2325

Met Val Gly Tyr Ile Leu Gly Leu Gly Asp Arg His Pro Ser Asn
    2330                2335                2340

Leu Met Leu Asp Arg Leu Ser Gly Lys Ile Leu His Ile Asp Phe
    2345                2350                2355

Gly Asp Cys Phe Glu Val Ala Met Thr Arg Glu Lys Phe Pro Glu
    2360                2365                2370

Lys Ile Pro Phe Arg Leu Thr Arg Met Leu Thr Asn Ala Met Glu
    2375                2380                2385

Val Thr Gly Leu Asp Gly Asn Tyr Arg Ile Thr Cys His Thr Val
    2390                2395                2400

Met Glu Val Leu Arg Glu His Lys Asp Ser Val Met Ala Val Leu
    2405                2410                2415

Glu Ala Phe Val Tyr Asp Pro Leu Leu Asn Trp Arg Leu Met Asp

2420                    2425                    2430

Thr Asn Thr Lys Gly Asn Lys Arg Ser Arg Thr Arg Thr Asp Ser
    2435                    2440                    2445

Tyr Ser Ala Gly Gln Ser Val Glu Ile Leu Asp Gly Val Glu Leu
    2450                    2455                    2460

Gly Glu Pro Ala His Lys Lys Thr Gly Thr Thr Val Pro Glu Ser
    2465                    2470                    2475

Ile His Ser Phe Ile Gly Asp Gly Leu Val Lys Pro Glu Ala Leu
    2480                    2485                    2490

Asn Lys Lys Ala Ile Gln Ile Ile Asn Arg Val Arg Asp Lys Leu
    2495                    2500                    2505

Thr Gly Arg Asp Phe Ser His Asp Asp Thr Leu Asp Val Pro Thr
    2510                    2515                    2520

Gln Val Glu Leu Leu Ile Lys Gln Ala Thr Ser His Glu Asn Leu
    2525                    2530                    2535

Cys Gln Cys Tyr Ile Gly Trp Cys Pro Phe Trp
    2540                    2545

<210>  83
<211>  3352
<212>  DNA
<213>  Homo sapiens

<220>
<221>  CDS
<222>  (80)..(1981)

<400>  83
cgcggacccg gccggcccag gcccgcgccc gccgcggccc tgagaggccc cggcaggtcc      60

cggcccggcg gcggcagcc atg gcc ggg ggg ccg ggc ccg ggg gag ccc gca      112
                     Met Ala Gly Gly Pro Gly Pro Gly Glu Pro Ala
                     1               5                   10

gcc ccc ggc gcc cag cac ttc ttg tac gag gtg ccg ccc tgg gtc atg      160
Ala Pro Gly Ala Gln His Phe Leu Tyr Glu Val Pro Pro Trp Val Met
            15                  20                  25

tgc cgc ttc tac aaa gtg atg gac gcc ctg gag ccc gcc gac tgg tgc      208
Cys Arg Phe Tyr Lys Val Met Asp Ala Leu Glu Pro Ala Asp Trp Cys
        30                  35                  40

cag ttc gcc gcc ctg atc gtg cgc gac cag acc gag ctg cgg ctg tgc      256
Gln Phe Ala Ala Leu Ile Val Arg Asp Gln Thr Glu Leu Arg Leu Cys
    45                  50                  55

gag cgc tcc ggg cag cgc acg gcc agc gtc ctg tgg ccc tgg atc aac 304
Glu Arg Ser Gly Gln Arg Thr Ala Ser Val Leu Trp Pro Trp Ile Asn
60                  65                  70                  75

cgc aac gcc cgt gtg gcc gac ctc gtg cac atc ctc acg cac ctg cag 352
Arg Asn Ala Arg Val Ala Asp Leu Val His Ile Leu Thr His Leu Gln
                80                  85                  90

ctg ctc cgt gcg cgg gac atc atc aca gcc tgg cac cct ccc gcc ccg 400
Leu Leu Arg Ala Arg Asp Ile Ile Thr Ala Trp His Pro Pro Ala Pro
            95                  100                 105

ctt ccg tcc cca ggc acc act gcc ccg agg ccc agc agc atc cct gca 448
Leu Pro Ser Pro Gly Thr Thr Ala Pro Arg Pro Ser Ser Ile Pro Ala
        110                 115                 120

ccc gcc gag gcc gag gcc tgg agc ccc cgg aag ttg cca tcc tca gcc 496
Pro Ala Glu Ala Glu Ala Trp Ser Pro Arg Lys Leu Pro Ser Ser Ala
    125                 130                 135

tcc acc ttc ctc tcc cca gct ttt cca ggc tcc cag acc cat tca ggg 544
Ser Thr Phe Leu Ser Pro Ala Phe Pro Gly Ser Gln Thr His Ser Gly
140                 145                 150                 155

cct gag ctc ggc ctg gtc cca agc cct gct tcc ctg tgg cct cca ccg 592
Pro Glu Leu Gly Leu Val Pro Ser Pro Ala Ser Leu Trp Pro Pro Pro
                160                 165                 170

cca tct cca gcc cct tct tct acc aag cca ggc cca gag agc tca gtg 640
Pro Ser Pro Ala Pro Ser Ser Thr Lys Pro Gly Pro Glu Ser Ser Val
            175                 180                 185

tcc ctc ctg cag gga gcc cgc ccc ttt ccg ttt tgc tgg ccc ctc tgt 688
Ser Leu Leu Gln Gly Ala Arg Pro Phe Pro Phe Cys Trp Pro Leu Cys
        190                 195                 200

gag att tcc cgg ggc acc cac aac ttc tcg gag gag ctc aag atc ggg 736
Glu Ile Ser Arg Gly Thr His Asn Phe Ser Glu Glu Leu Lys Ile Gly
    205                 210                 215

gag ggt ggc ttt ggg tgc gtg tac cgg gcg gtg atg agg aac acg gtg 784
Glu Gly Gly Phe Gly Cys Val Tyr Arg Ala Val Met Arg Asn Thr Val
220                 225                 230                 235

tat gct gtg aag agg ctg aag gag aac gct gac ctg gag tgg act gca 832
Tyr Ala Val Lys Arg Leu Lys Glu Asn Ala Asp Leu Glu Trp Thr Ala
                240                 245                 250

gtg aag cag agc ttc ctg acc gag gtg gag cag ctg tcc agg ttt cgt 880
Val Lys Gln Ser Phe Leu Thr Glu Val Glu Gln Leu Ser Arg Phe Arg
            255                 260                 265

cac cca aac att gtg gac ttt gct ggc tac tgt gct cag aac ggc ttc 928
His Pro Asn Ile Val Asp Phe Ala Gly Tyr Cys Ala Gln Asn Gly Phe
        270                 275                 280

tac tgc ctg gtg tac ggc ttc ctg ccc aac ggc tcc ctg gag gac cgt 976
Tyr Cys Leu Val Tyr Gly Phe Leu Pro Asn Gly Ser Leu Glu Asp Arg
    285                 290                 295

ctc cac tgc cag acc cag gcc tgc cca cct ctc tcc tgg cct cag cga 1024
Leu His Cys Gln Thr Gln Ala Cys Pro Pro Leu Ser Trp Pro Gln Arg

300                 305                 310                 315

ctg gac atc ctt ctg ggt aca gcc cgg gca att cag ttt cta cat cag        1072
Leu Asp Ile Leu Leu Gly Thr Ala Arg Ala Ile Gln Phe Leu His Gln
                320                 325                 330

gac agc ccc agc ctc atc cat gga gac atc aag agt tcc aac gtc ctt        1120
Asp Ser Pro Ser Leu Ile His Gly Asp Ile Lys Ser Ser Asn Val Leu
            335                 340                 345

ctg gat gag agg ctg aca ccc aag ctg gga gac ttt ggc ctg gcc cgg        1168
Leu Asp Glu Arg Leu Thr Pro Lys Leu Gly Asp Phe Gly Leu Ala Arg
        350                 355                 360

ttc agc cgc ttt gcc ggg tcc agc ccc agc cag agc agc atg gtg gcc        1216
Phe Ser Arg Phe Ala Gly Ser Ser Pro Ser Gln Ser Ser Met Val Ala
    365                 370                 375

cgg aca cag aca gtg cgg ggc acc ctg gcc tac ctg ccc gag gag tac        1264
Arg Thr Gln Thr Val Arg Gly Thr Leu Ala Tyr Leu Pro Glu Glu Tyr
380                 385                 390                 395

atc aag acg gga agg ctg gct gtg gac acg gac acc ttc agc ttt ggg        1312
Ile Lys Thr Gly Arg Leu Ala Val Asp Thr Asp Thr Phe Ser Phe Gly
                400                 405                 410

gtg gta gtg cta gag acc ttg gct ggt cag agg gct gtg aag acg cac        1360
Val Val Val Leu Glu Thr Leu Ala Gly Gln Arg Ala Val Lys Thr His
            415                 420                 425

ggt gcc agg acc aag tat ctg gtg tac gag agg cta gag aag ctg cag        1408
Gly Ala Arg Thr Lys Tyr Leu Val Tyr Glu Arg Leu Glu Lys Leu Gln
        430                 435                 440

gca gtg gtg gcg ggg gtg ccc ggg cat tcg gag gcc gcc agc tgc atc        1456
Ala Val Val Ala Gly Val Pro Gly His Ser Glu Ala Ala Ser Cys Ile
    445                 450                 455

ccc cct tcc ccg cag gag aac tcc tac gtg tcc agc act ggc aga gcc        1504
Pro Pro Ser Pro Gln Glu Asn Ser Tyr Val Ser Ser Thr Gly Arg Ala
460                 465                 470                 475

cac agt ggg gct gct cca tgg cag ccc ctg gca gcg cca tca gga gcc        1552
His Ser Gly Ala Ala Pro Trp Gln Pro Leu Ala Ala Pro Ser Gly Ala
                480                 485                 490

agt gcc cag gca gca gag cag ctg cag aga ggc ccc aac cag ccc gtg        1600
Ser Ala Gln Ala Ala Glu Gln Leu Gln Arg Gly Pro Asn Gln Pro Val
            495                 500                 505

gag agt gac gag agc cta ggc ggc ctc tct gct gcc ctg cgc tcc tgg        1648
Glu Ser Asp Glu Ser Leu Gly Gly Leu Ser Ala Ala Leu Arg Ser Trp
        510                 515                 520

cac ttg act cca agc tgc cct ctg gac cca gca ccc ctc agg gag gcc        1696
His Leu Thr Pro Ser Cys Pro Leu Asp Pro Ala Pro Leu Arg Glu Ala
    525                 530                 535

ggc tgt cct cag ggg gac acg gca gga gaa tcg agc tgg ggg agt ggc        1744
Gly Cys Pro Gln Gly Asp Thr Ala Gly Glu Ser Ser Trp Gly Ser Gly
540                 545                 550                 555

cca gga tcc cgg ccc aca gcc gtg gaa gga ctg gcc ctt ggc agc tct        1792

Pro Gly Ser Arg Pro Thr Ala Val Glu Gly Leu Ala Leu Gly Ser Ser
                560                 565                 570

gca tca tcg tcg tca gag cca ccg cag att atc atc aac cct gcc cga      1840
Ala Ser Ser Ser Ser Glu Pro Pro Gln Ile Ile Ile Asn Pro Ala Arg
            575                 580                 585

cag aag atg gtc cag aag ctg gcc ctg tac gag gat ggg gcc ctg gac      1888
Gln Lys Met Val Gln Lys Leu Ala Leu Tyr Glu Asp Gly Ala Leu Asp
        590                 595                 600

agc ctg cag ctg ctg tcg tcc agc tcc ctc cca ggc ttg ggc ctg gaa      1936
Ser Leu Gln Leu Leu Ser Ser Ser Ser Leu Pro Gly Leu Gly Leu Glu
    605                 610                 615

cag gac agg cag ggg ccc gaa gaa agt gat gaa ttt cag agc tga          1981
Gln Asp Arg Gln Gly Pro Glu Glu Ser Asp Glu Phe Gln Ser
620                 625                 630

tgtgttcacc tgggcagatc ccccaaatcc ggaagtcaaa gttctcatgg tcagaagttc      2041

tcatggtgca cgagtcctca gcactctgcc ggcagtgggg gtgggggccc atgcccgcgg      2101

gggagagaag gaggtggccc tgctgttcta ggctctgtgg gcataggcag gcagagtgga      2161

accctgcctc catgccagca tctgggggca aggaaggctg gcatcatcca gtgaggaggc      2221

tggcgcatgt tgggaggctg ctggctgcac agacccgtga ggggaggaga ggggctgctg      2281

tgcaggggtg tggagtaggg agctggctcc cctgagagcc atgcagggcg tctgcagccc      2341

aggcctctgg cagcagctct ttgcccatct ctttggacag tggccaccct gcacaatggg      2401

gccgacgagg cctagggccc tcctacctgc ttacaatttg gaaaagtgtg gccgggtgcg      2461

gtggctcacg cctgtaatcc cagcactttg ggaggccaag gcaggaggat cgctggagcc      2521

cagtaggtca agaccagcca gggcaacatg atgagaccct gtctctgcca aaaaattttt      2581

taaactatta gcctggcgtg gtagcgcacg cctgtggtcc cagctgctgg ggaggctgaa      2641

gtaggaggat catttatgct tgggaggtcg aggctgcagt gagtcatgat tgtatgactg      2701

cactccagcc tgggtgacag agcaagaccc tgtttcaaaa agaaaaaccc tgggaaaagt      2761

gaagtatggc tgtaagtctc atggttcagt cctagcaaga agcgagaatt ctgagatcct      2821

ccagaaagtc gagcagcacc cacctccaac ctcgggccag tgtcttcagg ctttactggg      2881

gacctgcgag ctggcctaat gtggtggcct gcaagccagg ccatccctgg gcgccacaga      2941

cgagctccga gccaggtcag gcttcggagg ccacaagctc agcctcaggc ccaggcactg      3001

attgtggcag aggggccact acccaaggtc tagctaggcc caagacctag ttacccagac      3061

agtgagaagc ccctggaagg cagaaaagtt gggagcatgg cagacaggga agggaaacat      3121

tttcagggaa aagacatgta tcacatgtct tcagaagcaa gtcaggtttc atgtaaccga      3181

gtgtcctctt gcgtgtccaa aagtagccca gggctgtagc acaggcttca cagtgatttt      3241

gtgttcagcc gtgagtcaca ctacatgccc ccgtgaagct gggcattggt gacgtccagg      3301

ttgtccttga gtaataaaaa cgtatgttgc aataaaaaaa aaaaaaaaaa a 3352

<210> 84
<211> 633
<212> PRT
<213> Homo sapiens

<400> 84

Met Ala Gly Gly Pro Gly Pro Gly Glu Pro Ala Ala Pro Gly Ala Gln
1               5                   10                  15

His Phe Leu Tyr Glu Val Pro Pro Trp Val Met Cys Arg Phe Tyr Lys
            20                  25                  30

Val Met Asp Ala Leu Glu Pro Ala Asp Trp Cys Gln Phe Ala Ala Leu
        35                  40                  45

Ile Val Arg Asp Gln Thr Glu Leu Arg Leu Cys Glu Arg Ser Gly Gln
    50                  55                  60

Arg Thr Ala Ser Val Leu Trp Pro Trp Ile Asn Arg Asn Ala Arg Val
65                  70                  75                  80

Ala Asp Leu Val His Ile Leu Thr His Leu Gln Leu Leu Arg Ala Arg
                85                  90                  95

Asp Ile Ile Thr Ala Trp His Pro Pro Ala Pro Leu Pro Ser Pro Gly
            100                 105                 110

Thr Thr Ala Pro Arg Pro Ser Ser Ile Pro Ala Pro Ala Glu Ala Glu
        115                 120                 125

Ala Trp Ser Pro Arg Lys Leu Pro Ser Ser Ala Ser Thr Phe Leu Ser
    130                 135                 140

Pro Ala Phe Pro Gly Ser Gln Thr His Ser Gly Pro Glu Leu Gly Leu
145                 150                 155                 160

Val Pro Ser Pro Ala Ser Leu Trp Pro Pro Pro Pro Ser Pro Ala Pro
                165                 170                 175

Ser Ser Thr Lys Pro Gly Pro Glu Ser Ser Val Ser Leu Leu Gln Gly
            180                 185                 190

Ala Arg Pro Phe Pro Phe Cys Trp Pro Leu Cys Glu Ile Ser Arg Gly
        195                 200                 205

Thr His Asn Phe Ser Glu Glu Leu Lys Ile Gly Glu Gly Gly Phe Gly

210                 215                 220

Cys Val Tyr Arg Ala Val Met Arg Asn Thr Val Tyr Ala Val Lys Arg
225                 230                 235                 240

Leu Lys Glu Asn Ala Asp Leu Glu Trp Thr Ala Val Lys Gln Ser Phe
                245                 250                 255

Leu Thr Glu Val Glu Gln Leu Ser Arg Phe Arg His Pro Asn Ile Val
            260                 265                 270

Asp Phe Ala Gly Tyr Cys Ala Gln Asn Gly Phe Tyr Cys Leu Val Tyr
        275                 280                 285

Gly Phe Leu Pro Asn Gly Ser Leu Glu Asp Arg Leu His Cys Gln Thr
    290                 295                 300

Gln Ala Cys Pro Pro Leu Ser Trp Pro Gln Arg Leu Asp Ile Leu Leu
305                 310                 315                 320

Gly Thr Ala Arg Ala Ile Gln Phe Leu His Gln Asp Ser Pro Ser Leu
                325                 330                 335

Ile His Gly Asp Ile Lys Ser Ser Asn Val Leu Leu Asp Glu Arg Leu
            340                 345                 350

Thr Pro Lys Leu Gly Asp Phe Gly Leu Ala Arg Phe Ser Arg Phe Ala
        355                 360                 365

Gly Ser Ser Pro Ser Gln Ser Ser Met Val Ala Arg Thr Gln Thr Val
    370                 375                 380

Arg Gly Thr Leu Ala Tyr Leu Pro Glu Glu Tyr Ile Lys Thr Gly Arg
385                 390                 395                 400

Leu Ala Val Asp Thr Asp Thr Phe Ser Phe Gly Val Val Val Leu Glu
                405                 410                 415

Thr Leu Ala Gly Gln Arg Ala Val Lys Thr His Gly Ala Arg Thr Lys
            420                 425                 430

Tyr Leu Val Tyr Glu Arg Leu Glu Lys Leu Gln Ala Val Val Ala Gly
        435                 440                 445

Val Pro Gly His Ser Glu Ala Ala Ser Cys Ile Pro Pro Ser Pro Gln
    450                 455                 460

Glu Asn Ser Tyr Val Ser Ser Thr Gly Arg Ala His Ser Gly Ala Ala
465                 470                 475                 480

Pro Trp Gln Pro Leu Ala Ala Pro Ser Gly Ala Ser Ala Gln Ala Ala
                485                 490                 495

Glu Gln Leu Gln Arg Gly Pro Asn Gln Pro Val Glu Ser Asp Glu Ser
            500                 505                 510

Leu Gly Gly Leu Ser Ala Ala Leu Arg Ser Trp His Leu Thr Pro Ser
        515                 520                 525

Cys Pro Leu Asp Pro Ala Pro Leu Arg Glu Ala Gly Cys Pro Gln Gly
    530                 535                 540

Asp Thr Ala Gly Glu Ser Ser Trp Gly Ser Gly Pro Gly Ser Arg Pro
545                 550                 555                 560

Thr Ala Val Glu Gly Leu Ala Leu Gly Ser Ser Ala Ser Ser Ser Ser
                565                 570                 575

Glu Pro Pro Gln Ile Ile Ile Asn Pro Ala Arg Gln Lys Met Val Gln
            580                 585                 590

Lys Leu Ala Leu Tyr Glu Asp Gly Ala Leu Asp Ser Leu Gln Leu Leu
        595                 600                 605

Ser Ser Ser Ser Leu Pro Gly Leu Gly Leu Glu Gln Asp Arg Gln Gly
    610                 615                 620

Pro Glu Glu Ser Asp Glu Phe Gln Ser
625                 630

<210>  85
<211>  8743
<212>  DNA
<213>  Homo sapiens

<220>
<221>  CDS
<222>  (53)..(3781)

<400>  85
   ggttgttttt cggagcctcc ctctgctcag cgttggtggt ggcggtggca gc atg gcg      58
                                                             Met Ala
                                                             1
   agc cct ccg gag agc gat ggc ttc tcg gac gtg cgc aag gtg ggc tac      106
   Ser Pro Pro Glu Ser Asp Gly Phe Ser Asp Val Arg Lys Val Gly Tyr
           5                   10                  15
   ctg cgc aaa ccc aag agc atg cac aaa cgc ttc ttc gta ctg cgc gcg 154
   Leu Arg Lys Pro Lys Ser Met His Lys Arg Phe Phe Val Leu Arg Ala
   20 25 30
   gcc agc gag gct ggg ggc ccg gcg cgc ctc gag tac tac gag aac gag 202
   Ala Ser Glu Ala Gly Gly Pro Ala Arg Leu Glu Tyr Tyr Glu Asn Glu
   35 40 45 50
   aag aag tgg cgg cac aag tcg agc gcc ccc aaa cgc tcg atc ccc ctt 250
   Lys Lys Trp Arg His Lys Ser Ser Ala Pro Lys Arg Ser Ile Pro Leu
   55 60 65
   gag agc tgc ttc aac atc aac aag cgg gct gac tcc aag aac aag cac 298
   Glu Ser Cys Phe Asn Ile Asn Lys Arg Ala Asp Ser Lys Asn Lys His
   70 75 80
   ctg gtg gct ctc tac acc cgg gac gag cac ttt gcc atc gcg gcg gac 346
   Leu Val Ala Leu Tyr Thr Arg Asp Glu His Phe Ala Ile Ala Ala Asp
   85 90 95
   agc gag gcc gag caa gac agc tgg tac cag gct ctc cta cag ctg cac 394
   Ser Glu Ala Glu Gln Asp Ser Trp Tyr Gln Ala Leu Leu Gln Leu His
   100 105 110
   aac cgt gct aag ggc cac cac gac gga gct gcg gcc ctc ggg gcg gga 442
   Asn Arg Ala Lys Gly His His Asp Gly Ala Ala Ala Leu Gly Ala Gly
   115 120 125 130
   ggt ggt ggg ggc agc tgc agc ggc agc tcc ggc ctt ggt gag gct ggg 490
   Gly Gly Gly Gly Ser Cys Ser Gly Ser Ser Gly Leu Gly Glu Ala Gly
   135 140 145
   gag gac ttg agc tac ggt gac gtg ccc cca gga ccc gca ttc aaa gag 538
   Glu Asp Leu Ser Tyr Gly Asp Val Pro Pro Gly Pro Ala Phe Lys Glu
   150 155 160
   gtc tgg caa gtg atc ctg aag ccc aag ggc ctg ggt cag aca aag aac 586
   Val Trp Gln Val Ile Leu Lys Pro Lys Gly Leu Gly Gln Thr Lys Asn
   165 170 175
   ctg att ggt atc tac cgc ctt tgc ctg acc agc aag acc atc agc ttc 634
   Leu Ile Gly Ile Tyr Arg Leu Cys Leu Thr Ser Lys Thr Ile Ser Phe
   180 185 190
   gtg aag ctg aac tcg gag gca gcg gcc gtg gtg ctg cag ctg atg aac 682
   Val Lys Leu Asn Ser Glu Ala Ala Ala Val Val Leu Gln Leu Met Asn
   195 200 205 210
   atc agg cgc tgt ggc cac tcg gaa aac ttc ttc ttc atc gag gtg ggc 730
   Ile Arg Arg Cys Gly His Ser Glu Asn Phe Phe Phe Ile Glu Val Gly
   215 220 225
   cgt tct gcc gtg acg ggg ccc ggg gag ttc tgg atg cag gtg gat gac 778
   Arg Ser Ala Val Thr Gly Pro Gly Glu Phe Trp Met Gln Val Asp Asp
   230 235 240
   tct gtg gtg gcc cag aac atg cac gag acc atc ctg gag gcc atg cgg 826
   Ser Val Val Ala Gln Asn Met His Glu Thr Ile Leu Glu Ala Met Arg
   245 250 255
   gcc atg agt gat gag ttc cgc cct cgc agc aag agc cag tcc tcg tcc 874
   Ala Met Ser Asp Glu Phe Arg Pro Arg Ser Lys Ser Gln Ser Ser Ser
   260 265 270
   aac tgc tct aac ccc atc agc gtc ccc ctg cgc cgg cac cat ctc aac 922
   Asn Cys Ser Asn Pro Ile Ser Val Pro Leu Arg Arg His His Leu Asn
   275 280 285 290
   aat ccc ccg ccc agc cag gtg ggg ctg acc cgc cga tca cgc act gag 970
   Asn Pro Pro Pro Ser Gln Val Gly Leu Thr Arg Arg Ser Arg Thr Glu
   295 300 305
   agc atc acc gcc acc tcc ccg gcc agc atg gtg ggc ggg aag cca ggc 1018
   Ser Ile Thr Ala Thr Ser Pro Ala Ser Met Val Gly Gly Lys Pro Gly
   310 315 320
   tcc ttc cgt gtc cgc gcc tcc agt gac ggc gaa ggc acc atg tcc cgc 1066
   Ser Phe Arg Val Arg Ala Ser Ser Asp Gly Glu Gly Thr Met Ser Arg
   325 330 335
   cca gcc tcg gtg gac ggc agc cct gtg agt ccc agc acc aac aga acc 1114
   Pro Ala Ser Val Asp Gly Ser Pro Val Ser Pro Ser Thr Asn Arg Thr
   340 345 350
   cac gcc cac cgg cat cgg ggc agc gcc cgg ctg cac ccc ccg ctc aac 1162
   His Ala His Arg His Arg Gly Ser Ala Arg Leu His Pro Pro Leu Asn
   355 360 365 370
   cac agc cgc tcc atc ccc atg ccg gct tcc cgc tgc tcg cct tcg gcc 1210
   His Ser Arg Ser Ile Pro Met Pro Ala Ser Arg Cys Ser Pro Ser Ala
   375 380 385
   acc agc ccg gtc agt ctg tcg tcc agt agc acc agt ggc cat ggc tcc 1258
   Thr Ser Pro Val Ser Leu Ser Ser Ser Ser Thr Ser Gly His Gly Ser
   390 395 400
   acc tcg gat tgt ctc ttc cca cgg cga tct agt gct tcg gtg tct ggt 1306
   Thr Ser Asp Cys Leu Phe Pro Arg Arg Ser Ser Ala Ser Val Ser Gly
   405 410 415
   tcc ccc agc gat ggc ggt ttc atc tcc tcg gat gag tat ggc tcc agt 1354
   Ser Pro Ser Asp Gly Gly Phe Ile Ser Ser Asp Glu Tyr Gly Ser Ser
   420 425 430
   ccc tgc gat ttc cgg agt tcc ttc cgc agt gtc act ccg gat tcc ctg 1402
   Pro Cys Asp Phe Arg Ser Ser Phe Arg Ser Val Thr Pro Asp Ser Leu
   435 440 445 450
   ggc cac acc cca cca gcc cgc ggt gag gag gag cta agc aac tat atc 1450
   Gly His Thr Pro Pro Ala Arg Gly Glu Glu Glu Leu Ser Asn Tyr Ile
   455 460 465
   tgc atg ggt ggc aag ggg ccc tcc acc ctg acc gcc ccc aac ggt cac 1498
   Cys Met Gly Gly Lys Gly Pro Ser Thr Leu Thr Ala Pro Asn Gly His
   470 475 480
   tac att ttg tct cgg ggt ggc aat ggc cac cgc tgc acc cca gga aca 1546
   Tyr Ile Leu Ser Arg Gly Gly Asn Gly His Arg Cys Thr Pro Gly Thr
   485 490 495
   ggc ttg ggc acg agt cca gcc ttg gct ggg gat gaa gca gcc agt gct 1594
   Gly Leu Gly Thr Ser Pro Ala Leu Ala Gly Asp Glu Ala Ala Ser Ala
   500 505 510
   gca gat ctg gat aat cgg ttc cga aag aga act cac tcg gca ggc aca 1642
   Ala Asp Leu Asp Asn Arg Phe Arg Lys Arg Thr His Ser Ala Gly Thr
   515                 520                 525                 530
   tcc cct acc att acc cac cag aag acc ccg tcc cag tcc tca gtg gct      1690
   Ser Pro Thr Ile Thr His Gln Lys Thr Pro Ser Gln Ser Ser Val Ala
                   535                 540                 545
   tcc att gag gag tac aca gag atg atg cct gcc tac cca cca gga ggt      1738
   Ser Ile Glu Glu Tyr Thr Glu Met Met Pro Ala Tyr Pro Pro Gly Gly
               550                 555                 560
   ggc agt gga ggc cga ctg ccg gga cac agg cac tcc gcc ttc gtg ccc      1786
   Gly Ser Gly Gly Arg Leu Pro Gly His Arg His Ser Ala Phe Val Pro
           565                 570                 575
   acc cgc tcc tac cca gag gag ggt ctg gaa atg cac ccc ttg gag cgt      1834
   Thr Arg Ser Tyr Pro Glu Glu Gly Leu Glu Met His Pro Leu Glu Arg
       580                 585                 590
   cgg ggg ggg cac cac cgc cca gac agc tcc acc ctc cac acg gat gat      1882
   Arg Gly Gly His His Arg Pro Asp Ser Ser Thr Leu His Thr Asp Asp
   595                 600                 605                 610
   ggc tac atg ccc atg tcc cca ggg gtg gcc cca gtg ccc agt ggc cga      1930
   Gly Tyr Met Pro Met Ser Pro Gly Val Ala Pro Val Pro Ser Gly Arg
                   615                 620                 625
   aag ggc agt gga gac tat atg ccc atg agc ccc aag agc gta tct gcc      1978
   Lys Gly Ser Gly Asp Tyr Met Pro Met Ser Pro Lys Ser Val Ser Ala
               630                 635                 640
   cca cag cag atc atc aat ccc atc aga cgc cat ccc cag aga gtg gac      2026
   Pro Gln Gln Ile Ile Asn Pro Ile Arg Arg His Pro Gln Arg Val Asp
           645                 650                 655
   ccc aat ggc tac atg atg atg tcc ccc agc ggt ggc tgc tct cct gac      2074
   Pro Asn Gly Tyr Met Met Met Ser Pro Ser Gly Gly Cys Ser Pro Asp
       660                 665                 670
   att gga ggt ggc ccc agc agc agc agc agc agc agc aac gcc gtc cct      2122
   Ile Gly Gly Gly Pro Ser Ser Ser Ser Ser Ser Ser Asn Ala Val Pro
   675                 680                 685                 690
   tcc ggg acc agc tat gga aag ctg tgg aca aac ggg gta ggg ggc cac      2170
   Ser Gly Thr Ser Tyr Gly Lys Leu Trp Thr Asn Gly Val Gly Gly His
                   695                 700                 705
   cac tct cat gtc ttg cct cac ccc aaa ccc cca gtg gag agc agc ggt      2218
   His Ser His Val Leu Pro His Pro Lys Pro Pro Val Glu Ser Ser Gly
               710                 715                 720
   ggt aag ctc tta cct tgc aca ggt gac tac atg aac atg tca cca gtg      2266
   Gly Lys Leu Leu Pro Cys Thr Gly Asp Tyr Met Asn Met Ser Pro Val
           725                 730                 735
   ggg gac tcc aac acc agc agc ccc tcc gac tgc tac tac ggc cct gag      2314
   Gly Asp Ser Asn Thr Ser Ser Pro Ser Asp Cys Tyr Tyr Gly Pro Glu
       740                 745                 750
   gac ccc cag cac aag cca gtc ctc tcc tac tac tca ttg cca aga tcc      2362
   Asp Pro Gln His Lys Pro Val Leu Ser Tyr Tyr Ser Leu Pro Arg Ser
   755                 760                 765                 770
   ttt aag cac acc cag cgc ccc ggg gag ccg gag gag ggt gcc cgg cat      2410
   Phe Lys His Thr Gln Arg Pro Gly Glu Pro Glu Glu Gly Ala Arg His
                   775                 780                 785
   cag cac ctc cgc ctt tcc act agc tct ggt cgc ctt ctc tat gct gca      2458
   Gln His Leu Arg Leu Ser Thr Ser Ser Gly Arg Leu Leu Tyr Ala Ala
               790                 795                 800
   aca gca gat gat tct tcc tct tcc acc agc agc gac agc ctg ggt ggg      2506
   Thr Ala Asp Asp Ser Ser Ser Ser Thr Ser Ser Asp Ser Leu Gly Gly
           805                 810                 815
   gga tac tgc ggg gct agg ctg gag ccc agc ctt cca cat ccc cac cat      2554
   Gly Tyr Cys Gly Ala Arg Leu Glu Pro Ser Leu Pro His Pro His His
       820                 825                 830
   cag gtt ctg cag ccc cat ctg cct cga aag gtg gac aca gct gct cag      2602
   Gln Val Leu Gln Pro His Leu Pro Arg Lys Val Asp Thr Ala Ala Gln
   835                 840                 845                 850
   acc aat agc cgc ctg gcc cgg ccc acg agg ctg tcc ctg ggg gat ccc      2650
   Thr Asn Ser Arg Leu Ala Arg Pro Thr Arg Leu Ser Leu Gly Asp Pro
                   855                 860                 865
   aag gcc agc acc tta cct cgg gcc cga gag cag cag cag cag cag cag      2698
   Lys Ala Ser Thr Leu Pro Arg Ala Arg Glu Gln Gln Gln Gln Gln Gln
               870                 875                 880
   ccc ttg ctg cac cct cca gag ccc aag agc ccg ggg gaa tat gtc aat      2746
   Pro Leu Leu His Pro Pro Glu Pro Lys Ser Pro Gly Glu Tyr Val Asn
           885                 890                 895
   att gaa ttt ggg agt gat cag tct ggc tac ttg tct ggc ccg gtg gct      2794
   Ile Glu Phe Gly Ser Asp Gln Ser Gly Tyr Leu Ser Gly Pro Val Ala
       900                 905                 910
   ttc cac agc tca cct tct gtc agg tgt cca tcc cag ctc cag cca gct      2842
   Phe His Ser Ser Pro Ser Val Arg Cys Pro Ser Gln Leu Gln Pro Ala
   915                 920                 925                 930
   ccc aga gag gaa gag act ggc act gag gag tac atg aag atg gac ctg      2890
   Pro Arg Glu Glu Glu Thr Gly Thr Glu Glu Tyr Met Lys Met Asp Leu
                   935                 940                 945
   ggg ccg ggc cgg agg gca gcc tgg cag gag agc act ggg gtc gag atg      2938
   Gly Pro Gly Arg Arg Ala Ala Trp Gln Glu Ser Thr Gly Val Glu Met
               950                 955                 960
   ggc aga ctg ggc cct gca cct ccc ggg gct gct agc att tgc agg cct      2986
   Gly Arg Leu Gly Pro Ala Pro Pro Gly Ala Ala Ser Ile Cys Arg Pro
           965                 970                 975
   acc cgg gca gtg ccc agc agc cgg ggt gac tac atg acc atg cag atg      3034
   Thr Arg Ala Val Pro Ser Ser Arg Gly Asp Tyr Met Thr Met Gln Met
       980                 985                 990
   agt tgt ccc cgt cag agc  tac gtg gac acc tcg  cca gct gcc cct      3079
   Ser Cys Pro Arg Gln Ser  Tyr Val Asp Thr Ser  Pro Ala Ala Pro
   995                 1000                1005
   gta  agc tat gct gac atg  cga aca ggc att gct  gca gag gag gtg      3124
   Val  Ser Tyr Ala Asp Met  Arg Thr Gly Ile Ala  Ala Glu Glu Val
   1010                1015                1020
   agc ctg ccc agg gcc acc atg gct gct gcc tcc tca tcc tca gca 3169
   Ser Leu Pro Arg Ala Thr Met Ala Ala Ala Ser Ser Ser Ser Ala
   1025 1030 1035
   gcc tct gct tcc ccg act ggg cct caa ggg gca gca gag ctg gct 3214
   Ala Ser Ala Ser Pro Thr Gly Pro Gln Gly Ala Ala Glu Leu Ala
   1040 1045 1050
   gcc cac tcg tcc ctg ctg ggg ggc cca caa gga cct ggg ggc atg 3259
   Ala His Ser Ser Leu Leu Gly Gly Pro Gln Gly Pro Gly Gly Met
   1055 1060 1065
   agc gcc ttc acc cgg gtg aac ctc agt cct aac cgc aac cag agt 3304
   Ser Ala Phe Thr Arg Val Asn Leu Ser Pro Asn Arg Asn Gln Ser
   1070 1075 1080
   gcc aaa gtg atc cgt gca gac cca caa ggg tgc cgg cgg agg cat 3349
   Ala Lys Val Ile Arg Ala Asp Pro Gln Gly Cys Arg Arg Arg His
   1085 1090 1095
   agc tcc gag act ttc tcc tca aca ccc agt gcc acc cgg gtg ggc 3394
   Ser Ser Glu Thr Phe Ser Ser Thr Pro Ser Ala Thr Arg Val Gly
   1100 1105 1110
   aac aca gtg ccc ttt gga gcg ggg gca gca gta ggg ggc ggt ggc 3439
   Asn Thr Val Pro Phe Gly Ala Gly Ala Ala Val Gly Gly Gly Gly
   1115 1120 1125
   ggt agc agc agc agc agc gag gat gtg aaa cgc cac agc tct gct 3484
   Gly Ser Ser Ser Ser Ser Glu Asp Val Lys Arg His Ser Ser Ala
   1130 1135 1140
   tcc ttt gag aat gtg tgg ctg agg cct ggg gag ctt ggg gga gcc 3529
   Ser Phe Glu Asn Val Trp Leu Arg Pro Gly Glu Leu Gly Gly Ala
   1145 1150 1155
   ccc aag gag cca gcc aaa ctg tgt ggg gct gct ggg ggt ttg gag 3574
   Pro Lys Glu Pro Ala Lys Leu Cys Gly Ala Ala Gly Gly Leu Glu
   1160 1165 1170
   aat ggt ctt aac tac ata gac ctg gat ttg gtc aag gac ttc aaa 3619
   Asn Gly Leu Asn Tyr Ile Asp Leu Asp Leu Val Lys Asp Phe Lys
   1175 1180 1185
   cag tgc cct cag gag tgc acc cct gaa ccg cag cct ccc cca ccc 3664
   Gln Cys Pro Gln Glu Cys Thr Pro Glu Pro Gln Pro Pro Pro Pro
   1190 1195 1200
   cca ccc cct cat caa ccc ctg ggc agc ggt gag agc agc tcc acc 3709
   Pro Pro Pro His Gln Pro Leu Gly Ser Gly Glu Ser Ser Ser Thr
   1205 1210 1215
   cgc cgc tca agt gag gat tta agc gcc tat gcc agc atc agt ttc 3754
   Arg Arg Ser Ser Glu Asp Leu Ser Ala Tyr Ala Ser Ile Ser Phe
   1220 1225 1230
   cag aag cag cca gag gac cgt cag tag ctcaactgga catcacagca 3801
   Gln Lys Gln Pro Glu Asp Arg Gln
   1235 1240
   gaatgaagac ctaaatgacc tcagcaaatc ctcttctaac tcatgggtac ccagactcta 3861
   aatatttcat gattcacaac taggacctca tatcttcctc atcagtagat ggtacgatgc 3921
   atccatttca gtttgtttac tttatccaat cctcaggatt tcattgactg aactgcacgt 3981
   tctatattgt gccaagcgaa aaaaaaaaat gcactgtgac accagaataa tgagtctgca 4041
   taaacttcat cttcaacctt aaggacttag ctggccacag tgagctgatg tgcccaccac 4101
   cgtgtcatga gagaatgggt ttactctcaa tgcattttca agatacattt catctgctgc 4161
   tgaaactgtg tacgacaaag catcattgta aattatttca tacaaaactg ttcacgttgg 4221
   gtggagagag tattaaatat ttaacatagg ttttgattta tatgtgtaat tttttaaatg 4281
   aaaatgtaac ttttcttaca gcacatcttt tttttggatg tgggatggag gtatacaatg 4341
   ttctgttgta aagagtggag caaatgctta aaacaaggct taaaagagta gaatagggta 4401
   tgatccttgt tttaagattg taattcagaa aacataatat aagaatcata gtgccataga 4461
   tggttctcaa ttgtatagtt atatttgctg atactatctc ttgtcatata aacctgatgt 4521
   tgagctgagt tccttataag aattaatctt aattttgtat tttttcctgt aagacaatag 4581
   gccatgttaa ttaaactgaa gaaggatata tttggctggg tgttttcaaa tgtcagctta 4641
   aaattggtaa ttgaatggaa gcaaaattat aagaagagga aattaaagtc ttccattgca 4701
   tgtattgtaa acagaaggag atgggtgatt ccttcaattc aaaagctctc tttggaatga 4761
   acaatgtggg cgtttgtaaa ttctggaaat gtctttctat tcataataaa ctagatactg 4821
   ttgatctttt cttctgtccc ctccccccac cacttctgta agtttcctgc tctattccca 4881
   ccattttttt ctgtgcacac attatgatat atttcatttc ctgcattgtc ttgagaaaga 4941
   tggtaaggca agtgagctgt tgctaaccag aaattaaaat tccagtaagt gtttttcatt 5001
   atgaccaggg ctatgtgtca ccttccctaa gactcttacc ttatctcata ttttttgaga 5061
   acttccagtg ttacattatt taactgaatg taattggccc atttgccttg gtgggtgctg 5121
   gcctattagt gattagttaa caaaacacag cgtacagaga gcacagaaaa gcttaatgac 5181
   ctgctactga aacacctagc cagcagtgaa aatgttaatt cttttcttgt ttggaaagta 5241
   tacacgtctt ggaatttttt ccacgtgaaa aacaaatggc aatgaatgca tttaaagata 5301
   ttgccgacag atttttaaat ctttttacca ggaaacttcc taaaggttaa atgaattaat 5361
   gcaaatatca ggctccctct gagtctgtgg gagcctctat ctctctatca ggaattcgca 5421
   tccctactat tgggaggagc aacattttat ttctctgaac gcctaagctc cctgggtggg 5481
   agtggggact acaaggtagg ggccagggtt ggagggcatt gtagtggctg ctgcctcctg 5541
   atgaactgtt tggggacccc agctctactc aaaagggagc ggagataaat ggaacccctc 5601
   acactgctga ggcccgtgtt actgttcatt cagccaggtg gcatgtacct cacagactgt 5661
   tgtgcagtgt ccgttattgc agattttaat catttgcatg ctcatcaatt tctaagataa 5721
   atagggtcta gagtcataag aatccattgt tttcaaggaa cttgcagaat tacatcattt 5781
   cctattagta gagagcacta cccattttga aaatctgata tgaaagttgt tttttactct 5841
   tgtaaaaaaa gactttctta gtcaaactaa cttttcatat tttcaagcat tctgattcat 5901
   actcttgcta gtggaagaag agagcaagct gccctgctct tttcctttga ggactgaaat 5961
   agttaaagag aaatcaatga acaaagtcac tcccaaccat tttcctgtaa agctggttat 6021
   tatttctcaa ggaacctaca ctttgaatat gtgttaccga gatacctcta catgtggaat 6081
   tatcaacatg ttttgaatga gagcagaaat gaacagactg gaaaaatcta tctcttggtt 6141
   tctatttctc tgactttttg agtcgaaaag cataaaggta gaaattctta tttaagctgc 6201
   ttctagtggt gcctgagctg gttttgatgg tggcatcaaa ctaccgattt aaaactggaa 6261
   gttgctggta ctcaaaccaa aagttcatac tctggcgaca cgaagggttt cctttgagca 6321
   acgtcagctg ctgagtcctt gtgttcagtt cccattgagg agagttggct tttatccatt 6381
   tcaaagcatt tgtaggccag ccaagggctt tcattattga ggcttctagt ggcctctggt 6441
   taacctagaa gttagtgggt ttttcttgat gacaccaacc tctcacagcg tttttcctta 6501
   gagacttaag cagagtttta aaatcctctt ttgcgaaaag aacaaatatg ttttatgact 6561
   ttgatgatat cttcattctg ggcaaaagaa tggccctaga accagctaga agtgaagaga 6621
   atccattaat gatcaaccac ctgagtcaat aatgagaaat cagtactgat gttacatttg 6681
   tggctatttc ttgctgactt tcaaaggtca aggactcttg actaatccag tgactgcaaa 6741
   aatggatcta ctaaaagtca tctagccaga agtagagatt tttaaccttt ctttccctgg 6801
   cttttgtctt ctagctatca tttaaatttg agacatttga agtattaaga aacaattttt 6861
   ctgtatggta agaaacagta ttttacaata ctgaagccct gttttattca atcttgcatc 6921
   ttgaatacaa tataccacaa agtcggaaac tttatattta tttactgcag gtggttaaaa 6981
   aaagggggga aagggtttca ccatccactg acaacgagag ccatgacaaa tagtatccat 7041
   gtgcagtctt ccaactgctg gtgacaatga ccccatattt ggtctcatgc tgcttttgca 7101
   gagcactctg taggttagtc catcacacaa ggaggccctg aatccagaca ctgtgaatta 7161
   agaccttggc ggggagagat gtgacccttt tggtaggaat gggaagaaga atgggtggaa 7221
   gccaatatga aatttcttct ttgcaatgac ttgacagggg agttaatgtt cctaggatgc 7281
   catgaatgat gaatgttagt tggaggtaat gctgtatatg tgtgtgtgtg tgtgtgtgtg 7341
   tgtttgtgtg tgtgtatata tatgtgtgtg tgtatatata tgtgacatgt gtgtgtcttt 7401
   gtgtgtgtgt atatatatgt gacgtgtgtg tgggatgtgt gtgtgtatat atagatgaat 7461
   atatacaaat atatagatat atacacacat atatagatat atatacacat atatagatat 7521
   atatacacac atatatagat acacatatgt gtgtgtgtgt atatatatat atatatatat 7581
   atagatgtat aggcttgtga gaaacttgag aggaagaagc atgctcttct aggaatgtga 7641
   ggaaatatga ccttgccaag actaaaagac ctctagactg tgagctcagt tatggagaac 7701
   aaaaacagct tcatagtgag tagaacaccg aggataaaca ctggggccat gggtcctttc 7761
   tgaggcagcg ccacagaaga tctttgtggt ccttccgtag ttctgtaagt ctgtctccta 7821
   agtatgggta gagaatatgt agcctgttgt gtgtctccca ctacttgtaa acagagcatc 7881
   acattagggg cagggaggag gtggaatgat attggaggtg cttaacccta ctcgaggaat 7941
   taattatgaa taaagagctt ataattagct aacatgacta gaaaacacat gacttaggtg 8001
   gagagttagc tttcttttct agtttgtgta tgacttgcca tttgtgacgt atacaccaaa 8061
   agatctggtg ttttagactt ctgccattca cttggcattt aaatctctct ttgcttatgc 8121
   tgttaacgag tatgccatag gataggacaa attcagtaaa caggaaaact tgtccatatt 8181
   tgcatagaca tttgtagggt tttttttttc tttttctttt tagaacttca ccattggctt 8241
   aagaatgtag ttcccaaaac aattttttct tgcaaagtac tttccttaca cctcttggct 8301
   acagggtggg ccaaattaaa catatatgta ttttcattta atgtatgtgc agtttggttt 8361
   atcatcttaa gatggtggtg ctgccccggt gctacttcat ctgtgtacac aaagaccaat 8421
   gcatggtctg tattgctacc aaaacattta ctgtatatat gtttataaca tgtattatgt 8481
   atatatgtaa tgggtgccag gccaggtata tattttttat ttagaagtgt ttcacttttc 8541
   caagttttct ttatagtgtt atgcttattt tcaatttttt ttttcctgat tctgtctggt 8601
   acttagaatt gtagtgtctt catcatcaat taaagaaaac tgtctaaatg aattcatgga 8661
   tgtaaatatt agtggtcctt aatgtctttg attgctggac atgaaacaaa ctgccaatta 8721
   aattttgcgg agacaaaaaa aa 8743

<210> 86
<211> 1242
<212> PRT
<213> Homo sapiens

<400> 86

Met Ala Ser Pro Pro Glu Ser Asp Gly Phe Ser Asp Val Arg Lys Val
1               5                   10                  15

Gly Tyr Leu Arg Lys Pro Lys Ser Met His Lys Arg Phe Phe Val Leu
            20                  25                  30

Arg Ala Ala Ser Glu Ala Gly Gly Pro Ala Arg Leu Glu Tyr Tyr Glu
        35                  40                  45

Asn Glu Lys Lys Trp Arg His Lys Ser Ser Ala Pro Lys Arg Ser Ile
    50                  55                  60

Pro Leu Glu Ser Cys Phe Asn Ile Asn Lys Arg Ala Asp Ser Lys Asn
65                  70                  75                  80

Lys His Leu Val Ala Leu Tyr Thr Arg Asp Glu His Phe Ala Ile Ala
                85                  90                  95

Ala Asp Ser Glu Ala Glu Gln Asp Ser Trp Tyr Gln Ala Leu Leu Gln
            100                 105                 110

Leu His Asn Arg Ala Lys Gly His His Asp Gly Ala Ala Ala Leu Gly
        115                 120                 125

Ala Gly Gly Gly Gly Gly Ser Cys Ser Gly Ser Ser Gly Leu Gly Glu
    130                 135                 140

Ala Gly Glu Asp Leu Ser Tyr Gly Asp Val Pro Pro Gly Pro Ala Phe
145                 150                 155                 160

Lys Glu Val Trp Gln Val Ile Leu Lys Pro Lys Gly Leu Gly Gln Thr
                165                 170                 175

Lys Asn Leu Ile Gly Ile Tyr Arg Leu Cys Leu Thr Ser Lys Thr Ile
            180                 185                 190

Ser Phe Val Lys Leu Asn Ser Glu Ala Ala Ala Val Val Leu Gln Leu
        195                 200                 205

Met Asn Ile Arg Arg Cys Gly His Ser Glu Asn Phe Phe Phe Ile Glu
    210                 215                 220

Val Gly Arg Ser Ala Val Thr Gly Pro Gly Glu Phe Trp Met Gln Val
225                 230                 235                 240

Asp Asp Ser Val Val Ala Gln Asn Met His Glu Thr Ile Leu Glu Ala
                245                 250                 255

Met Arg Ala Met Ser Asp Glu Phe Arg Pro Arg Ser Lys Ser Gln Ser
            260                 265                 270

Ser Ser Asn Cys Ser Asn Pro Ile Ser Val Pro Leu Arg Arg His His
        275                 280                 285

Leu Asn Asn Pro Pro Pro Ser Gln Val Gly Leu Thr Arg Arg Ser Arg
    290                 295                 300

Thr Glu Ser Ile Thr Ala Thr Ser Pro Ala Ser Met Val Gly Gly Lys
305                 310                 315                 320

Pro Gly Ser Phe Arg Val Arg Ala Ser Ser Asp Gly Glu Gly Thr Met

                325                 330                 335

Ser Arg Pro Ala Ser Val Asp Gly Ser Pro Val Ser Pro Ser Thr Asn
            340                 345                 350

Arg Thr His Ala His Arg His Arg Gly Ser Ala Arg Leu His Pro Pro
        355                 360                 365

Leu Asn His Ser Arg Ser Ile Pro Met Pro Ala Ser Arg Cys Ser Pro
    370                 375                 380

Ser Ala Thr Ser Pro Val Ser Leu Ser Ser Ser Ser Thr Ser Gly His
385                 390                 395                 400

Gly Ser Thr Ser Asp Cys Leu Phe Pro Arg Arg Ser Ser Ala Ser Val
                405                 410                 415

Ser Gly Ser Pro Ser Asp Gly Gly Phe Ile Ser Ser Asp Glu Tyr Gly
            420                 425                 430

Ser Ser Pro Cys Asp Phe Arg Ser Ser Phe Arg Ser Val Thr Pro Asp
        435                 440                 445

Ser Leu Gly His Thr Pro Pro Ala Arg Gly Glu Glu Glu Leu Ser Asn
    450                 455                 460

Tyr Ile Cys Met Gly Gly Lys Gly Pro Ser Thr Leu Thr Ala Pro Asn
465                 470                 475                 480

Gly His Tyr Ile Leu Ser Arg Gly Gly Asn Gly His Arg Cys Thr Pro
                485                 490                 495

Gly Thr Gly Leu Gly Thr Ser Pro Ala Leu Ala Gly Asp Glu Ala Ala
            500                 505                 510

Ser Ala Ala Asp Leu Asp Asn Arg Phe Arg Lys Arg Thr His Ser Ala
        515                 520                 525

Gly Thr Ser Pro Thr Ile Thr His Gln Lys Thr Pro Ser Gln Ser Ser
    530                 535                 540

Val Ala Ser Ile Glu Glu Tyr Thr Glu Met Met Pro Ala Tyr Pro Pro
545                 550                 555                 560

Gly Gly Gly Ser Gly Gly Arg Leu Pro Gly His Arg His Ser Ala Phe
                565                 570                 575

Val Pro Thr Arg Ser Tyr Pro Glu Glu Gly Leu Glu Met His Pro Leu
            580                 585                 590

Glu Arg Arg Gly Gly His His Arg Pro Asp Ser Ser Thr Leu His Thr
        595                 600                 605

Asp Asp Gly Tyr Met Pro Met Ser Pro Gly Val Ala Pro Val Pro Ser
    610                 615                 620

Gly Arg Lys Gly Ser Gly Asp Tyr Met Pro Met Ser Pro Lys Ser Val
625                 630                 635                 640

Ser Ala Pro Gln Gln Ile Ile Asn Pro Ile Arg Arg His Pro Gln Arg
                645                 650                 655

Val Asp Pro Asn Gly Tyr Met Met Met Ser Pro Ser Gly Gly Cys Ser
            660                 665                 670

Pro Asp Ile Gly Gly Gly Pro Ser Ser Ser Ser Ser Ser Ser Asn Ala
        675                 680                 685

Val Pro Ser Gly Thr Ser Tyr Gly Lys Leu Trp Thr Asn Gly Val Gly
    690                 695                 700

Gly His His Ser His Val Leu Pro His Pro Lys Pro Pro Val Glu Ser
705                 710                 715                 720

Ser Gly Gly Lys Leu Leu Pro Cys Thr Gly Asp Tyr Met Asn Met Ser
                725                 730                 735

Pro Val Gly Asp Ser Asn Thr Ser Ser Pro Ser Asp Cys Tyr Tyr Gly
            740                 745                 750

Pro Glu Asp Pro Gln His Lys Pro Val Leu Ser Tyr Tyr Ser Leu Pro
        755                 760                 765

Arg Ser Phe Lys His Thr Gln Arg Pro Gly Glu Pro Glu Glu Gly Ala
    770                 775                 780

Arg His Gln His Leu Arg Leu Ser Thr Ser Ser Gly Arg Leu Leu Tyr
785                 790                 795                 800

Ala Ala Thr Ala Asp Asp Ser Ser Ser Ser Thr Ser Ser Asp Ser Leu
                805                 810                 815

Gly Gly Gly Tyr Cys Gly Ala Arg Leu Glu Pro Ser Leu Pro His Pro
            820                 825                 830

His His Gln Val Leu Gln Pro His Leu Pro Arg Lys Val Asp Thr Ala
        835                 840                 845

Ala Gln Thr Asn Ser Arg Leu Ala Arg Pro Thr Arg Leu Ser Leu Gly
    850                 855                 860

Asp Pro Lys Ala Ser Thr Leu Pro Arg Ala Arg Glu Gln Gln Gln Gln
865                 870                 875                 880

Gln Gln Pro Leu Leu His Pro Pro Glu Pro Lys Ser Pro Gly Glu Tyr
                885                 890                 895

Val Asn Ile Glu Phe Gly Ser Asp Gln Ser Gly Tyr Leu Ser Gly Pro
            900                 905                 910

Val Ala Phe His Ser Ser Pro Ser Val Arg Cys Pro Ser Gln Leu Gln
        915                 920                 925

Pro Ala Pro Arg Glu Glu Glu Thr Gly Thr Glu Glu Tyr Met Lys Met
    930                 935                 940

Asp Leu Gly Pro Gly Arg Arg Ala Ala Trp Gln Glu Ser Thr Gly Val
945                 950                 955                 960

Glu Met Gly Arg Leu Gly Pro Ala Pro Pro Gly Ala Ala Ser Ile Cys
                965                 970                 975

Arg Pro Thr Arg Ala Val Pro Ser Ser Arg Gly Asp Tyr Met Thr Met
            980                 985                 990

Gln Met Ser Cys Pro Arg Gln Ser Tyr Val Asp Thr Ser Pro Ala Ala
        995                 1000                1005

Pro Val Ser Tyr Ala Asp Met Arg Thr Gly Ile Ala Ala Glu Glu
    1010                1015                1020

Val Ser Leu Pro Arg Ala Thr Met Ala Ala Ala Ser Ser Ser Ser
    1025                1030                1035

Ala Ala Ser Ala Ser Pro Thr Gly Pro Gln Gly Ala Ala Glu Leu
    1040                1045                1050

Ala Ala His Ser Ser Leu Leu Gly Gly Pro Gln Gly Pro Gly Gly
    1055                1060                1065

Met Ser Ala Phe Thr Arg Val Asn Leu Ser Pro Asn Arg Asn Gln
    1070                1075                1080

Ser Ala Lys Val Ile Arg Ala Asp Pro Gln Gly Cys Arg Arg Arg
    1085                1090                1095

His Ser Ser Glu Thr Phe Ser Ser Thr Pro Ser Ala Thr Arg Val
    1100                1105                1110

Gly Asn Thr Val Pro Phe Gly Ala Gly Ala Ala Val Gly Gly Gly
    1115                1120                1125

Gly Gly Ser Ser Ser Ser Ser Glu Asp Val Lys Arg His Ser Ser
    1130                1135                1140

Ala Ser Phe Glu Asn Val Trp Leu Arg Pro Gly Glu Leu Gly Gly
    1145                1150                1155

Ala Pro Lys Glu Pro Ala Lys Leu Cys Gly Ala Ala Gly Gly Leu
    1160                1165                1170

Glu Asn Gly Leu Asn Tyr Ile Asp Leu Asp Leu Val Lys Asp Phe
    1175                1180                1185

Lys Gln Cys Pro Gln Glu Cys Thr Pro Glu Pro Gln Pro Pro Pro
    1190                1195                1200

Pro Pro Pro Pro His Gln Pro Leu Gly Ser Gly Glu Ser Ser Ser
    1205                1210                1215

Thr Arg Arg Ser Ser Glu Asp Leu Ser Ala Tyr Ala Ser Ile Ser
    1220                1225                1230

Phe Gln Lys Gln Pro Glu Asp Arg Gln
    1235                1240

<210> 87
<211> 2886
<212> DNA
<213> Homo sapiens

<220>
<221> CDS
<222> (185)..(1138)

<400> 87
agattcctac ttcttacgcc ccccacatca cccgcctcga gacctcaagg gtagaggtgg 60

gcacccccgc ctccgcactt ttgctcgggg ctccagattg tagggcaggg cggcgcttct 120

cggaaagcga aagccggcgg ggcggggcgg gtgccgcagg agaaagagga agcgctggca 180

gaca atg cga ccc gac cgc gct gag gct cca gga ccg ccc gcc atg gct 229
Met Arg Pro Asp Arg Ala Glu Ala Pro Gly Pro Pro Ala Met Ala
1               5                   10                  15

gca gga ggt ccc ggc gcg ggg tct gcg gcc ccg gtc tcc tcc aca tcc 277
Ala Gly Gly Pro Gly Ala Gly Ser Ala Ala Pro Val Ser Ser Thr Ser
                20                  25                  30

tcc ctt ccc ctg gct gct ctc aac atg cga gtg cgg cgc cgc ctg tct 325
Ser Leu Pro Leu Ala Ala Leu Asn Met Arg Val Arg Arg Arg Leu Ser
            35                  40                  45

ctg ttc ttg aac gtg cgg aca cag gtg gcg gcc gac tgg acc gcg ctg 373
Leu Phe Leu Asn Val Arg Thr Gln Val Ala Ala Asp Trp Thr Ala Leu
        50                  55                  60

gcg gag gag atg gac ttt gag tac ttg gag atc cgg caa ctg gag aca 421
Ala Glu Glu Met Asp Phe Glu Tyr Leu Glu Ile Arg Gln Leu Glu Thr
    65                  70                  75

caa gcg gac ccc act ggc agg ctg ctg gac gcc tgg cag gga cgc cct 469
Gln Ala Asp Pro Thr Gly Arg Leu Leu Asp Ala Trp Gln Gly Arg Pro
80                  85                  90                  95

ggc gcc tct gta ggc cga ctg ctc gag ctg ctt acc aag ctg ggc cgc 517
Gly Ala Ser Val Gly Arg Leu Leu Glu Leu Leu Thr Lys Leu Gly Arg
                100                 105                 110

gac gac gtg ctg ctg gag ctg gga ccc agc att gag gag gat tgc caa 565
Asp Asp Val Leu Leu Glu Leu Gly Pro Ser Ile Glu Glu Asp Cys Gln
            115                 120                 125

aag tat atc ttg aag cag cag cag gag gag gct gag aag cct tta cag 613
Lys Tyr Ile Leu Lys Gln Gln Gln Glu Glu Ala Glu Lys Pro Leu Gln
        130                 135                 140

gtg gcc gct gta gac agc agt gtc cca cgg aca gca gag ctg gcg ggc 661
Val Ala Ala Val Asp Ser Ser Val Pro Arg Thr Ala Glu Leu Ala Gly
    145                 150                 155

atc acc aca ctt gat gac ccc ctg ggg cat atg cct gag cgt ttc gat 709
Ile Thr Thr Leu Asp Asp Pro Leu Gly His Met Pro Glu Arg Phe Asp
160                 165                 170                 175

gcc ttc atc tgc tat tgc ccc agc gac atc cag ttt gtg cag gag atg 757
Ala Phe Ile Cys Tyr Cys Pro Ser Asp Ile Gln Phe Val Gln Glu Met
                180                 185                 190

atc cgg caa ctg gaa cag aca aac tat cga ctg aag ttg tgt gtg tct 805
Ile Arg Gln Leu Glu Gln Thr Asn Tyr Arg Leu Lys Leu Cys Val Ser
            195                 200                 205

gac cgc gat gtc ctg cct ggc acc tgt gtc tgg tct att gct agt gag 853
Asp Arg Asp Val Leu Pro Gly Thr Cys Val Trp Ser Ile Ala Ser Glu
        210                 215                 220

ctc atc gaa aag agg ttg gct aga agg cca cgg ggt ggg tgc cgc cgg 901
Leu Ile Glu Lys Arg Leu Ala Arg Arg Pro Arg Gly Gly Cys Arg Arg
    225                 230                 235

atg gtg gtg gtt gtc tct gat gat tac ctg cag agc aag gaa tgt gac 949
Met Val Val Val Val Ser Asp Asp Tyr Leu Gln Ser Lys Glu Cys Asp
240                 245                 250                 255

ttc cag acc aaa ttt gca ctc agc ctc tct cca ggt gcc cat cag aag 997
Phe Gln Thr Lys Phe Ala Leu Ser Leu Ser Pro Gly Ala His Gln Lys
                260                 265                 270

cga ctg atc ccc atc aag tac aag gca atg aag aaa gag ttc ccc agc 1045
Arg Leu Ile Pro Ile Lys Tyr Lys Ala Met Lys Lys Glu Phe Pro Ser
            275                 280                 285

atc ctg agg ttc atc act gtc tgc gac tac acc aac ccc tgc acc aaa 1093
Ile Leu Arg Phe Ile Thr Val Cys Asp Tyr Thr Asn Pro Cys Thr Lys
        290                 295                 300

tct tgg ttc tgg act cgc ctt gcc aag gcc ttg tcc ctg ccc tga 1138
Ser Trp Phe Trp Thr Arg Leu Ala Lys Ala Leu Ser Leu Pro
    305                 310                 315

agactgttct gaggccctgg gtgtgtgtgt atctgtctgc ctgtccatgt acttctgccc 1198

tgcctcctcc tttcgttgta ggaggaatct gtgctctact tacctctcaa ttcctggaga 1258

tgccaacttc acagacacgt ctgcagcagc tggacatcac atttcatgtc ctgcatggaa 1318

ccagtggctg tgagtggcat gtccacttgc tggattatca gccaggacac tatagaacag 1378

gaccagctga gactaagaag gaccagcaga gccagctcag ctctgagcca ttcacacatc 1438

ttcaccctca gtttcctcac ttgaggagtg ggatggggag aacagagagt agctgtgttt 1498

gaatccctgt aggaaatggt gaagcatagc tctgggtctc ctgggggaga ccaggcttgg 1558

ctgcgggaga gctggctgtt gctggactac atgctggcca ctgctgtgac cacgacactg 1618

ctggggcagc ttcttccaca gtgatgccta ctgatgcttc agtgcctctg cacaccgccc 1678

attccacttc ctccttcccc acagggcagg tggggaagca gtttggccca gcccaaggag 1738

accccacctt gagccttatt tcctaatggg tccacctctc atctgcatct ttcacacctc 1798

ccagcttctg cccaaccttc agcagtgaca agtccccaag agactcgcct gagcagcttg 1858

ggctgctttt catttccacc tgtcaggatg cctgtggtca tgctctcagc tccacctggc 1918

atgagaaggg atcctggcct ctggcatatt catcaagtat gagttctggg gatgagtcac 1978

tgtaatgatg tgagcaggga gccttcctcc ctgggccacc tgcagagagc tttcccacca 2038

actttgtacc ttgattgcct tacaaagtta tttgtttaca aacagcgacc atataaaagc 2098

ctcctgcccc aaagcttgtg ggcacatggg cacatacaga ctcacataca gacacacaca 2158

tatatgtaca gacatgtact ctcacacaca caggcaccag catacacacg tttttctagg 2218

tacagctccc aggaacagct aggtgggaaa gtcccatcac tgagggagcc taaccatgtc 2278

cctgaacaaa aattgggcac tcatctattc cttttctctt gtgtccctac tcattgaaac 2338

caaactctgg aaaggaccca atgtaccagt atttatacct ctaatgaagc acagagagag 2398

gaagagagct gcttaaactc acacaacaat gaactgcaga cacagctgtt ctctccctct 2458

ctccttccca gagcaattta tactttaccc tcaggctgtc ctctggggag aaggtgccat 2518

ggtcttaggt gtctgtgccc caggacagac cctaggaccc taaatccaat agaaaatgca 2578

tatctttgct ccactttcag ccaggctgga gcaaggtacc ttttcttagg atcttgggag 2638

ggaatggatg cccctctctg catgatcttg ttgaggcatt tagctgccat gcacctgtcc 2698

ccctttaata ctgggcattt taaagccatc tcaagaggca tcttctacat gttttgtacg 2758

cattaaaata atttcaaaga tatctgagaa aagccgatat ttgccattct tcctatatcc 2818

tggaatatat cttgcatcct gagtttataa taataaataa tattctacct tggaaaaaaa 2878

aaaaaaaa 2886

<210> 88
<211> 317
<212> PRT
<213> Homo sapiens

<400> 88

Met Arg Pro Asp Arg Ala Glu Ala Pro Gly Pro Pro Ala Met Ala Ala
1               5                   10                  15

Gly Gly Pro Gly Ala Gly Ser Ala Ala Pro Val Ser Ser Thr Ser Ser
            20                  25                  30

Leu Pro Leu Ala Ala Leu Asn Met Arg Val Arg Arg Arg Leu Ser Leu
        35                  40                  45

Phe Leu Asn Val Arg Thr Gln Val Ala Ala Asp Trp Thr Ala Leu Ala
    50                  55                  60

Glu Glu Met Asp Phe Glu Tyr Leu Glu Ile Arg Gln Leu Glu Thr Gln
65                  70                  75                  80

Ala Asp Pro Thr Gly Arg Leu Leu Asp Ala Trp Gln Gly Arg Pro Gly
                85                  90                  95

Ala Ser Val Gly Arg Leu Leu Glu Leu Leu Thr Lys Leu Gly Arg Asp
            100                 105                 110

Asp Val Leu Leu Glu Leu Gly Pro Ser Ile Glu Glu Asp Cys Gln Lys
        115                 120                 125

Tyr Ile Leu Lys Gln Gln Gln Glu Glu Ala Glu Lys Pro Leu Gln Val
    130                 135                 140

Ala Ala Val Asp Ser Ser Val Pro Arg Thr Ala Glu Leu Ala Gly Ile
145                 150                 155                 160

Thr Thr Leu Asp Asp Pro Leu Gly His Met Pro Glu Arg Phe Asp Ala

165                 170                 175

Phe Ile Cys Tyr Cys Pro Ser Asp Ile Gln Phe Val Gln Glu Met Ile
        180                 185                 190

Arg Gln Leu Glu Gln Thr Asn Tyr Arg Leu Lys Leu Cys Val Ser Asp
    195                 200                 205

Arg Asp Val Leu Pro Gly Thr Cys Val Trp Ser Ile Ala Ser Glu Leu
    210                 215                 220

Ile Glu Lys Arg Leu Ala Arg Arg Pro Arg Gly Gly Cys Arg Arg Met
225                 230                 235                 240

Val Val Val Val Ser Asp Asp Tyr Leu Gln Ser Lys Glu Cys Asp Phe
                245                 250                 255

Gln Thr Lys Phe Ala Leu Ser Leu Ser Pro Gly Ala His Gln Lys Arg
            260                 265                 270

Leu Ile Pro Ile Lys Tyr Lys Ala Met Lys Lys Glu Phe Pro Ser Ile
        275                 280                 285

Leu Arg Phe Ile Thr Val Cys Asp Tyr Thr Asn Pro Cys Thr Lys Ser
    290                 295                 300

Trp Phe Trp Thr Arg Leu Ala Lys Ala Leu Ser Leu Pro
305                 310                 315

<210>  89
<211>  4093
<212>  DNA
<213>  Homo sapiens

<220>
<221>  CDS
<222>  (468)..(3377)

<400>  89
gtgagagagt gagcgagaca gaaagagaga gaagtgcacc agcgagccgg ggcaggaaga      60
ggaggtttcg ccaccggagc ggcccggcga cgcgctgaca gcttcccctg cccttcccgt     120
cggtcgggcc gccagccgcc gcagccctcg gcctgcacgc agccaccggc cccgctcccg     180
gagcccagcg ccgccgaggc cgcagccgcc cggccagtaa ggcggcgccg ccgcccggcc     240
accgcgcgcc ctgcgcttcc ctccgcccgc gctgcggcca tggcgcggcg ctgactggcc     300
tggcccggcc ccgccgcgct cccgctcgcc ccgacccgca ctcgggcccg cccgggctcc     360
ggcctgccgc cgcctcttcc ttctccagcc ggcaggcccg cgccgcttag gagggagagc     420
ccacccgcgc caggaggccg aacgcggact cgccacccgg cttcaga atg gca gaa 476
gat gat cca tat ttg gga agg cct gaa caa atg ttt cat ttg gat cct 524
tct ttg act cat aca ata ttt aat cca gaa gta ttt caa cca cag atg 572
gca ctg cca aca gca gat ggc cca tac ctt caa ata tta gag caa cct 620
aaa cag aga gga ttt cgt ttc cgt tat gta tgt gaa ggc cca tcc cat 668
ggt gga cta cct ggt gcc tct agt gaa aag aac aag aag tct tac cct 716
cag gtc aaa atc tgc aac tat gtg gga cca gca aag gtt att gtt cag 764
ttg gtc aca aat gga aaa aat atc cac ctg cat gcc cac agc ctg gtg 812
gga aaa cac tgt gag gat ggg atc tgc act gta act gct gga ccc aag 860
gac atg gtg gtc ggc ttc gca aac ctg ggt ata ctt cat gtg aca aag 908
aaa aaa gta ttt gaa aca ctg gaa gca cga atg aca gag gcg tgt ata 956
agg ggc tat aat cct gga ctc ttg gtg cac cct gac ctt gcc tat ttg 1004
caa gca gaa ggt gga ggg gac cgg cag ctg gga gat cgg gaa aaa gag 1052
cta atc cgc caa gca gct ctg cag cag acc aag gag atg gac ctc agc 1100
gtg gtg cgg ctc atg ttt aca gct ttt ctt ccg gat agc act ggc agc 1148
ttc aca agg cgc ctg gaa ccc gtg gta tca gac gcc atc tat gac agt 1196
aaa gcc ccc aat gca tcc aac ttg aaa att gta aga atg gac agg aca          1244
gct gga tgt gtg act gga ggg gag gaa att tat ctt ctt tgt gac aaa          1292
gtt cag aaa gat gac atc cag att cga ttt tat gaa gag gaa gaa aat          1340
ggt gga gtc tgg gaa gga ttt gga gat ttt tcc ccc aca gat gtt cat          1388
aga caa ttt gcc att gtc ttc aaa act cca aag tat aaa gat att aat          1436
att aca aaa cca gcc tct gtg ttt gtc cag ctt cgg agg aaa tct gac          1484
ttg gaa act agt gaa cca aaa cct ttc ctc tac tat cct gaa atc aaa          1532
gat aaa gaa gaa gtg cag agg aaa cgt cag aag ctc atg ccc aat ttt          1580
tcg gat agt ttc ggc ggt ggt agt ggt gct gga gct gga ggc gga ggc          1628
atg ttt ggt agt ggc ggt gga gga ggg ggc act gga agt aca ggt cca          1676
ggg tat agc ttc cca cac tat gga ttt cct act tat ggt ggg att act          1724
ttc cat cct gga act act aaa tct aat gct ggg atg aag cat gga acc          1772
atg gac act gaa tct aaa aag gac cct gaa ggt tgt gac aaa agt gat          1820
gac aaa aac act gta aac ctc ttt ggg aaa gtt att gaa acc aca gag          1868
caa gat cag gag ccc agc gag gcc acc gtt ggg aat ggt gag gtc act          1916
cta acg tat gca aca gga aca aaa gaa gag agt gct gga gtt cag gat          1964
aac ctc ttt cta gag aag gct atg cag ctt gca aag agg cat gcc aat       2012
gcc ctt ttc gac tac gcg gtg aca gga gac gtg aag atg ctg ctg gcc       2060
gtc cag cgc cat ctc act gct gtg cag gat gag aat ggg gac agt gtc       2108
tta cac tta gca atc atc cac ctt cat tct caa ctt gtg agg gat cta       2156
cta gaa gtc aca tct ggt ttg att tct gat gac att atc aac atg aga       2204
aat gat ctg tac cag acg ccc ttg cac ttg gca gtg atc act aag cag       2252
gaa gat gtg gtg gag gat ttg ctg agg gct ggg gcc gac ctg agc ctt       2300
ctg gac cgc ttg ggt aac tct gtt ttg cac cta gct gcc aaa gaa gga       2348
cat gat aaa gtt ctc agt atc tta ctc aag cac aaa aag gca gca cta       2396
ctt ctt gac cac ccc aac ggg gac ggt ctg aat gcc att cat cta gcc       2444
atg atg agc aat agc ctg cca tgt ttg ctg ctg ctg gtg gcc gct ggg       2492
gct gac gtc aat gct cag gag cag aag tcc ggg cgc aca gca ctg cac       2540
ctg gct gtg gag cac gac aac atc tca ttg gca ggc tgc ctg ctc ctg       2588
gag ggt gat gcc cat gtg gac agt act acc tac gat gga acc aca ccc       2636
ctg cat ata gca gct ggg aga ggg tcc acc agg ctg gca gct ctt ctc       2684
aaa gca gca gga gca gat ccc ctg gtg gag aac ttt gag cct ctc tat     2732
gac ctg gat gac tct tgg gaa aat gca gga gag gat gaa gga gtt gtg     2780
cct gga acc acg cct cta gat atg gcc acc agc tgg cag gta ttt gac     2828
ata tta aat ggg aaa cca tat gag cca gag ttt aca tct gat gat tta     2876
cta gca caa gga gac atg aaa cag ctg gct gaa gat gtg aag ctg cag     2924
ctg tat aag tta cta gaa att cct gat cca gac aaa aac tgg gct act     2972
ctg gcg cag aaa tta ggt ctg ggg ata ctt aat aat gcc ttc cgg ctg     3020
agt cct gct cct tcc aaa aca ctt atg gac aac tat gag gtc tct ggg     3068
ggt aca gtc aga gag ctg gtg gag gcc ctg aga caa atg ggc tac acc     3116
gaa gca att gaa gtg atc cag gca gcc tcc agc cca gtg aag acc acc     3164
tct cag gcc cac tcg ctg cct ctc tcg cct gcc tcc aca agg cag caa     3212
ata gac gag ctc cga gac agt gac agt gtc tgc gac agc ggc gtg gag     3260
aca tcc ttc cgc aaa ctc agc ttt acc gag tct ctg acc agt ggt gcc     3308
tca ctg cta act ctc aac aaa atg ccc cat gat tat ggg cag gaa gga     3356
cct cta gaa ggc aaa att tag cctgctgaca atttcccaca ccgtgtaaac     3407
caaagcccta aaattccact gcgttgtcca caagacagaa gctgaagtgc atccaaaggt     3467
gctcagagag ccggcccgcc tgaatcattc tcgatttaac tcgagacctt ttcaacttgg     3527
cttcctttct tggttcataa atgaatttta gtttggttca cttacagata gtatctagca 3587
atcacaacac tggctgagcg gatgcatctg gggatgaggt tgcttactaa gctttgccag 3647
ctgctgctgg atcacagctg ctttctgttg tcattgctgt tgtccctctg ctacgttcct 3707
attgtcatta aaggtatcac ggtcgccacc tggcattcct tctgaccaca gcatcatttt 3767
gcattcaaat taagggttaa gaaaagagat attttaaaat gagagtcact tgatgtgcca 3827
ttttaaaaaa aaaggcatat tgctttttct aatgtggtta tttctctgat ttgcaaaaaa 3887
aaaaaaaaaa aaaatacttg tcaatattta aacatggtta caatcattgc tgaaaatggt 3947
attttccccc ttttctgcat tttgctattg taaatatgtt ttttagatca aatactttaa 4007
aggaaaaaat gttggattta taaatgctat tttttatttt acttttataa taaaaggaaa 4067
agcaaattga tgacctcaaa aaaaaa 4093

<210> 90
<211> 969
<212> PRT
<213> Homo sapiens

<400> 90

<210>  91
<211>  7069
<212>  DNA
<213>  Homo sapiens

<220>
<221>  CDS
<222>  (162)..(3470)

<400>  91
aactgcaaca cttcctctgc atctggatat gaagggagcc ccagaaaagc ggaagaattt      60

agacgcacac tgggtaggtt tgaatttgtt ttgttttcaa aaattaaaca aatgatcctt     120

cagcatcatc gcctccgctg ctttatcagg tcgcataggg c atg gag ctg gag aac     176
                                              Met Glu Leu Glu Asn
                                              1               5

tat aaa cag ccc gtg gtg ctg aga gag gac aac tgc cga agg cgc cgg       224
Tyr Lys Gln Pro Val Val Leu Arg Glu Asp Asn Cys Arg Arg Arg Arg
                10                  15                  20

agg atg aag ccg cgc agt gct gcg gcc agc ctg tcc tcc atg gag ctc       272
Arg Met Lys Pro Arg Ser Ala Ala Ala Ser Leu Ser Ser Met Glu Leu
            25                  30                  35

atc ccc atc gag ttc gtg ctg ccc acc agc cag cgc aaa tgc aag agc       320
Ile Pro Ile Glu Phe Val Leu Pro Thr Ser Gln Arg Lys Cys Lys Ser
        40                  45                  50

ccc gaa acg gcg ctg ctg cac gtg gcc ggc cac ggc aac gtg gag cag       368
Pro Glu Thr Ala Leu Leu His Val Ala Gly His Gly Asn Val Glu Gln
    55                  60                  65

atg aag gcc cag gtg tgg ctg cga gcg ctg gag acc agc gtg gcg gcg       416
Met Lys Ala Gln Val Trp Leu Arg Ala Leu Glu Thr Ser Val Ala Ala
70                  75                  80                  85

gac ttc tac cac cgg ctg gga ccg cat cac ttc ctc ctg ctc tat cag       464
Asp Phe Tyr His Arg Leu Gly Pro His His Phe Leu Leu Leu Tyr Gln
                90                  95                  100

aag aag ggg cag tgg tac gag atc tac gac aag tac cag gtg gtg cag 512
Lys Lys Gly Gln Trp Tyr Glu Ile Tyr Asp Lys Tyr Gln Val Val Gln
            105                 110                 115

act ctg gac tgc ctg cgc tac tgg aag gcc acg cac cgg agc ccg ggc 560
Thr Leu Asp Cys Leu Arg Tyr Trp Lys Ala Thr His Arg Ser Pro Gly
        120                 125                 130

cag atc cac ctg gtg cag cgg cac ccg ccc tcc gag gag tcc caa gcc 608
Gln Ile His Leu Val Gln Arg His Pro Pro Ser Glu Glu Ser Gln Ala
    135                 140                 145

ttc cag cgg cag ctc acg gcg ctg att ggc tat gac gtc act gac gtc 656
Phe Gln Arg Gln Leu Thr Ala Leu Ile Gly Tyr Asp Val Thr Asp Val
150                 155                 160                 165

agc aac gtg cac gac gat gag ctg gag ttc acg cgc cgt ggc ttg gtg 704
Ser Asn Val His Asp Asp Glu Leu Glu Phe Thr Arg Arg Gly Leu Val
                170                 175                 180

acc ccg cgc atg gcg gag gtg gcc agc cgc gac ccc aag ctc tac gcc 752
Thr Pro Arg Met Ala Glu Val Ala Ser Arg Asp Pro Lys Leu Tyr Ala
            185                 190                 195

atg cac ccg tgg gtg acg tcc aag ccc ctc ccg gag tac ctg tgg aag 800
Met His Pro Trp Val Thr Ser Lys Pro Leu Pro Glu Tyr Leu Trp Lys
        200                 205                 210

aag att gcc aac aac tgc atc ttc atc gtc att cac cgc agc acc acc 848
Lys Ile Ala Asn Asn Cys Ile Phe Ile Val Ile His Arg Ser Thr Thr
    215                 220                 225

agc cag acc att aag gtc tca ccc gac gac acc ccc ggc gcc atc ctg 896
Ser Gln Thr Ile Lys Val Ser Pro Asp Asp Thr Pro Gly Ala Ile Leu
230                 235                 240                 245

cag agc ttc ttc acc aag atg gcc aag aag aaa tct ctg atg gat att 944
Gln Ser Phe Phe Thr Lys Met Ala Lys Lys Lys Ser Leu Met Asp Ile
                250                 255                 260

ccc gaa agc caa agc gaa cag gat ttt gtg ctg cgc gtc tgt ggc cgg 992
Pro Glu Ser Gln Ser Glu Gln Asp Phe Val Leu Arg Val Cys Gly Arg
            265                 270                 275

gat gag tac ctg gtg ggc gaa acg ccc atc aaa aac ttc cag tgg gtg 1040
Asp Glu Tyr Leu Val Gly Glu Thr Pro Ile Lys Asn Phe Gln Trp Val
        280                 285                 290

agg cac tgc ctc aag aac gga gaa gag att cac gtg gta ctg gac acg 1088
Arg His Cys Leu Lys Asn Gly Glu Glu Ile His Val Val Leu Asp Thr
    295                 300                 305

cct cca gac ccg gcc cta gac gag gtg agg aag gaa gag tgg cca ctg 1136
Pro Pro Asp Pro Ala Leu Asp Glu Val Arg Lys Glu Glu Trp Pro Leu
310                 315                 320                 325

gtg gat gac tgc acg gga gtc acc ggc tac cat gag cag ctt acc atc 1184
Val Asp Asp Cys Thr Gly Val Thr Gly Tyr His Glu Gln Leu Thr Ile
                330                 335                 340

cac ggc aag gac cac gag agt gtg ttc acc gtg tcc ctg tgg gac tgc 1232
His Gly Lys Asp His Glu Ser Val Phe Thr Val Ser Leu Trp Asp Cys

345                 350                 355

gac cgc aag ttc agg gtc aag atc aga ggc att gat atc ccc gtc ctg        1280
Asp Arg Lys Phe Arg Val Lys Ile Arg Gly Ile Asp Ile Pro Val Leu
        360                 365                 370

cct cgg aac acc gac ctc aca gtt ttt gta gag gca aac atc cag cat        1328
Pro Arg Asn Thr Asp Leu Thr Val Phe Val Glu Ala Asn Ile Gln His
    375                 380                 385

ggg caa caa gtc ctt tgc caa agg aga acc agc ccc aaa ccc ttc aca        1376
Gly Gln Gln Val Leu Cys Gln Arg Arg Thr Ser Pro Lys Pro Phe Thr
390                 395                 400                 405

gag gag gtg ctg tgg aat gtg tgg ctt gag ttc agt atc aaa atc aaa        1424
Glu Glu Val Leu Trp Asn Val Trp Leu Glu Phe Ser Ile Lys Ile Lys
                410                 415                 420

gac ttg ccc aaa ggg gct cta ctg aac ctc cag atc tac tgc ggt aaa        1472
Asp Leu Pro Lys Gly Ala Leu Leu Asn Leu Gln Ile Tyr Cys Gly Lys
            425                 430                 435

gct cca gca ctg tcc agc aag gcc tct gca gag tcc ccc agt tct gag        1520
Ala Pro Ala Leu Ser Ser Lys Ala Ser Ala Glu Ser Pro Ser Ser Glu
        440                 445                 450

tcc aag ggc aaa gtt cag ctt ctc tat tat gtg aac ctg ctg ctg ata        1568
Ser Lys Gly Lys Val Gln Leu Leu Tyr Tyr Val Asn Leu Leu Leu Ile
    455                 460                 465

gac cac cgt ttc ctc ctg cgc cgt gga gaa tac gtc ctc cac atg tgg        1616
Asp His Arg Phe Leu Leu Arg Arg Gly Glu Tyr Val Leu His Met Trp
470                 475                 480                 485

cag ata tct ggg aag gga gaa gac caa gga agc ttc aat gct gac aaa        1664
Gln Ile Ser Gly Lys Gly Glu Asp Gln Gly Ser Phe Asn Ala Asp Lys
                490                 495                 500

ctc acg tct gca act aac cca gac aag gag aac tca atg tcc atc tcc        1712
Leu Thr Ser Ala Thr Asn Pro Asp Lys Glu Asn Ser Met Ser Ile Ser
            505                 510                 515

att ctt ctg gac aat tac tgc cac ccg ata gcc ctg cct aag cat cag        1760
Ile Leu Leu Asp Asn Tyr Cys His Pro Ile Ala Leu Pro Lys His Gln
        520                 525                 530

ccc acc cct gac ccg gaa ggg gac cgg gtt cga gca gaa atg ccc aac        1808
Pro Thr Pro Asp Pro Glu Gly Asp Arg Val Arg Ala Glu Met Pro Asn
    535                 540                 545

cag ctt cgc aag caa ttg gag gcg atc ata gcc act gat cca ctt aac        1856
Gln Leu Arg Lys Gln Leu Glu Ala Ile Ile Ala Thr Asp Pro Leu Asn
550                 555                 560                 565

cct ctc aca gca gag gac aaa gaa ttg ctc tgg cat ttt aga tac gaa        1904
Pro Leu Thr Ala Glu Asp Lys Glu Leu Leu Trp His Phe Arg Tyr Glu
                570                 575                 580

agc ctt aag cac cca aaa gca tat cct aag cta ttt agt tca gtg aaa        1952
Ser Leu Lys His Pro Lys Ala Tyr Pro Lys Leu Phe Ser Ser Val Lys
            585                 590                 595

tgg gga cag caa gaa att gtg gcc aaa aca tac caa ttg ttg gcc aga        2000

Trp Gly Gln Gln Glu Ile Val Ala Lys Thr Tyr Gln Leu Leu Ala Arg
        600                 605                 610

agg gaa gtc tgg gat caa agt gct ttg gat gtt ggg tta aca atg cag      2048
Arg Glu Val Trp Asp Gln Ser Ala Leu Asp Val Gly Leu Thr Met Gln
    615                 620                 625

ctc ctg gac tgc aac ttc tca gat gaa aat gta aga gcc att gca gtt      2096
Leu Leu Asp Cys Asn Phe Ser Asp Glu Asn Val Arg Ala Ile Ala Val
630                 635                 640                 645

cag aaa ctg gag agc ttg gag gac gat gat gtt ctg cat tac ctt cta      2144
Gln Lys Leu Glu Ser Leu Glu Asp Asp Asp Val Leu His Tyr Leu Leu
                650                 655                 660

caa ttg gtc cag gct gtg aaa ttt gaa cca tac cat gat agc gcc ctt      2192
Gln Leu Val Gln Ala Val Lys Phe Glu Pro Tyr His Asp Ser Ala Leu
            665                 670                 675

gcc aga ttt ctg ctg aag cgt ggt tta aga aac aaa aga att ggt cac      2240
Ala Arg Phe Leu Leu Lys Arg Gly Leu Arg Asn Lys Arg Ile Gly His
        680                 685                 690

ttt ttg ttt tgg ttc ttg aga agt gag ata gcc cag tcc aga cac tat      2288
Phe Leu Phe Trp Phe Leu Arg Ser Glu Ile Ala Gln Ser Arg His Tyr
    695                 700                 705

cag cag agg ttc gct gtg att ctg gaa gcc tat ctg agg ggc tgt ggc      2336
Gln Gln Arg Phe Ala Val Ile Leu Glu Ala Tyr Leu Arg Gly Cys Gly
710                 715                 720                 725

aca gcc atg ctg cac gac ttt acc caa caa gtc caa gta atc gag atg      2384
Thr Ala Met Leu His Asp Phe Thr Gln Gln Val Gln Val Ile Glu Met
                730                 735                 740

tta caa aaa gtc acc ctt gat att aaa tcg ctc tct gct gaa aag tat      2432
Leu Gln Lys Val Thr Leu Asp Ile Lys Ser Leu Ser Ala Glu Lys Tyr
            745                 750                 755

gac gtc agt tcc caa gtt att tca caa ctt aaa caa aag ctt gaa aac      2480
Asp Val Ser Ser Gln Val Ile Ser Gln Leu Lys Gln Lys Leu Glu Asn
        760                 765                 770

ctg cag aat tct caa ctc ccc gaa agc ttt aga gtt cca tat gat cct      2528
Leu Gln Asn Ser Gln Leu Pro Glu Ser Phe Arg Val Pro Tyr Asp Pro
    775                 780                 785

gga ctg aaa gca gga gcg ctg gca att gaa aaa tgt aaa gta atg gcc      2576
Gly Leu Lys Ala Gly Ala Leu Ala Ile Glu Lys Cys Lys Val Met Ala
790                 795                 800                 805

tcc aag aaa aaa cca cta tgg ctt gag ttt aaa tgt gcc gat cct aca      2624
Ser Lys Lys Lys Pro Leu Trp Leu Glu Phe Lys Cys Ala Asp Pro Thr
                810                 815                 820

gcc cta tca aat gaa aca att gga att atc ttt aaa cat ggt gat gat      2672
Ala Leu Ser Asn Glu Thr Ile Gly Ile Ile Phe Lys His Gly Asp Asp
            825                 830                 835

ctg cgc caa gac atg ctt att tta cag att cta cga atc atg gag tct      2720
Leu Arg Gln Asp Met Leu Ile Leu Gln Ile Leu Arg Ile Met Glu Ser
        840                 845                 850

att tgg gag act gaa tct ttg gat cta tgc ctc ctg cca tat ggt tgc     2768
Ile Trp Glu Thr Glu Ser Leu Asp Leu Cys Leu Leu Pro Tyr Gly Cys
    855                 860                 865

att tca act ggt gac aaa ata gga atg atc gag att gtg aaa gac gcc     2816
Ile Ser Thr Gly Asp Lys Ile Gly Met Ile Glu Ile Val Lys Asp Ala
870                 875                 880                 885

acg aca att gcc aaa att cag caa agc aca gtg ggc aac acg gga gca     2864
Thr Thr Ile Ala Lys Ile Gln Gln Ser Thr Val Gly Asn Thr Gly Ala
                890                 895                 900

ttt aaa gat gaa gtc ctg aat cac tgg ctc aaa gaa aaa tcc cct act     2912
Phe Lys Asp Glu Val Leu Asn His Trp Leu Lys Glu Lys Ser Pro Thr
            905                 910                 915

gaa gaa aag ttt cag gca gca gtg gag aga ttt gtt tat tcc tgt gca     2960
Glu Glu Lys Phe Gln Ala Ala Val Glu Arg Phe Val Tyr Ser Cys Ala
        920                 925                 930

ggc tac tgt gtg gca acc ttt gtt ctt gga ata ggc gac aga cac aat     3008
Gly Tyr Cys Val Ala Thr Phe Val Leu Gly Ile Gly Asp Arg His Asn
    935                 940                 945

gac aat att atg atc acc gag aca gga aac cta ttt cat att gac ttc     3056
Asp Asn Ile Met Ile Thr Glu Thr Gly Asn Leu Phe His Ile Asp Phe
950                 955                 960                 965

ggg cac att ctt ggg aat tac aaa agt ttc ctg ggc att aat aaa gag     3104
Gly His Ile Leu Gly Asn Tyr Lys Ser Phe Leu Gly Ile Asn Lys Glu
                970                 975                 980

aga gtg cca ttt gtg cta acc cct gac ttc ctc ttt gtg atg gga act     3152
Arg Val Pro Phe Val Leu Thr Pro Asp Phe Leu Phe Val Met Gly Thr
            985                 990                 995

tct gga aag  aag aca agc cca cac  ttc cag aaa ttt cag  gac atc     3197
Ser Gly Lys  Lys Thr Ser Pro His  Phe Gln Lys Phe Gln  Asp Ile
        1000                 1005                 1010

tgt gtt aag  gct tat cta gcc ctt  cgt cat cac aca aac  cta ctg     3242
Cys Val Lys  Ala Tyr Leu Ala Leu  Arg His His Thr Asn  Leu Leu
        1015                 1020                 1025

atc atc ctg  ttc tcc atg atg ctg  atg aca gga atg ccc  cag tta     3287
Ile Ile Leu  Phe Ser Met Met Leu  Met Thr Gly Met Pro  Gln Leu
        1030                 1035                 1040

aca agc aaa  gaa gac att gaa tat  atc cgg gat gcc ctc  aca gtg     3332
Thr Ser Lys  Glu Asp Ile Glu Tyr  Ile Arg Asp Ala Leu  Thr Val
        1045                 1050                 1055

ggg aaa aat  gag gag gat gct aaa  aag tat ttt ctt gat  cag atc     3377
Gly Lys Asn  Glu Glu Asp Ala Lys  Lys Tyr Phe Leu Asp  Gln Ile
        1060                 1065                 1070

gaa gtt tgc  aga gac aaa gga tgg  act gtg cag ttt aat  tgg ttt     3422
Glu Val Cys  Arg Asp Lys Gly Trp  Thr Val Gln Phe Asn  Trp Phe
        1075                 1080                 1085

cta cat ctt  gtt ctt ggc atc aaa  caa gga gag aaa cat  tca gcc     3467
Leu His Leu  Val Leu Gly Ile Lys  Gln Gly Glu Lys His  Ser Ala
        1090                 1095                 1100

taa tactttaggc tagaatcaaa aacaagttag tgttctatgg tttaaattag 3520

catagcaatc atcgaacttg gatttcaaat gcaatagaca ttgtgaaagc tggcatttca 3580

gaagtatagc tcttttccta cctgaactct tccctggaga aaagatgttg gcattgctga 3640

ttgtttggtt aagcaatgtc cagtgctagg attatttgca ggtttggttt tttctcattt 3700

gtctgtggca ttggagaata ttctcggttt aaacagacta atgacttcct tattgtccct 3760

gatattttga ctatcttact attgagtgct tctggaaatt ctttggaata attgatgaca 3820

tctattttca tctgggttta gtctcaattt tggttatctt tgtgttcctc aagctcttta 3880

aagaaaaaga tgtaatcgtt gtaacctttg tctcattcct taaatgatgc ttccaaacat 3940

ctccttagtg tctgcaggtg ttagtggtgt gctaaaagca aggaaagcga gttagtcttt 4000

tcagtgtctt ttgcaattca attcttttgt catgtataac tgagacacac aaacacagca 4060

ggagaaatct aaaccgttgt gccttgacct tcctctgctg gtcttgttcc agggttatga 4120

atatgaaaaa atagagatga gactttttgt gtcaactctg tccacaagag tgagttatct 4180

agtatgatta gtatagcttt ctccagcatg gcagcaggaa gtaactacag ggcctctttt 4240

atgcctgaca tttcttccct tcctttttcc ctgcctccct ttttcatcaa ttgcgatgct 4300

cccacaactc tttacagact tgtgaaatct tcaagaacac ctttactcta taactcaaaa 4360

attagttgaa aaataattac ttctcaagga ttattagaat cttaggtact tatttgtaaa 4420

gatgtttagt gacttttttt tcaagtatct tattaaagga ggcattctag aaaatatgaa 4480

ttagtttcca aatgccttaa ttttaaactt tggcctgaac agttttttct ttttcttaat 4540

ggaagaagat atttaatatc ttaaaaatat tccaagttag gaagaacact acttgcctta 4600

tccatttccc atttaaagga cttttaaact ttgacacatc cttcagattt cctgaaaata 4660

attgaaatat cttactttaa aaatattttc atctctgaaa tatctcgtta tttattggag 4720

gtattgttta accttagaga gaccattaaa ttatttataa aatattttgt aattacctgt 4780

agctaataca ttacatagaa aaaaactatg ttaacagtgt ctctgtttaa gtataatcag 4840

atataaatat atacttaatt ttttaatttt aaaaatagat acctgtttga ctttgaggta 4900

gtccagacct tttctttttt tttttttttt taatgtgtgc aaaagcccaa aggttcctaa 4960

gcctggctgc aaagaagaat caacagggac actttttaaa aacactctta tcagcctggg 5020

caacacagtg agactccatc tcttaaaaaa aaaattagct gggtatagtg gtatgtgcct 5080

gtagtcccag gtactcagga ggctgaggca ggaggattgc ctgagcccag gaggtggaaa 5140

ctgcagagag tcatgatcat gtccttacac tccagcctgg ataacagagc gagaccctgt 5200

ctcaaaaaaa taaaataaaa aataaaaaca cccttgcctg cgctccattc ccaggttata 5260

atttaattac tgtgggatga gacccagaca tcaatatttt tctagattct tcaggtgatt 5320

ctaattcaca gccagagttg gaagccctgc gtggcctttg aaggtctaga tgattcttct 5380

tccttgccct ttgagctttt ccccatctca caggtatcta gaaaaaaact cctcttcttt 5440

ggcaacctgt ccttttaaaa tcacactcta cccacctgta cagaagacca tgccctataa 5500

tgaaatgttt attcctatct ataaatggag gataaacatt ttgtggcact tctggaccaa 5560

ctattcccta ctattctttt gaagaaaggc aggaagagta ctttctaatt cagaagagga 5620

tgttttcact attctgataa acaatagcca agttcagacc ttgtacagat tctttttatt 5680

tgaattgctg aaataattta ttgatgatga aaaaaagatt agagaggaat acatttatat 5740

ttagcttatt ggcacatgtg catacatatt tcctcttcaa atgaaccagt tctttcattt 5800

cattatgcta atatatatat ataacatata tatgctaata tatatataac atatatatgc 5860

taatatatat atatcacaca tatatatcac agttttatat atatatatgt atgtgtgtgt 5920

gtatatatat atatatatat cacagttggg cttgattctt ccgtattcca aagagcataa 5980

tttcagttct atagacttat agataaataa aaatcatctt tgtgggcttc cttcctttac 6040

tgttcgcagt gaattacatg acgaacaact tctatacctt tgaaaatgtt ctagaactag 6100

aatcatcctg ctactgggaa ctacccacag ctctatcttc aatgccaggt gaaaacacag 6160

atcacaagtc agatgaatca ggccaaagca acttttatgt atatctagga ctggcgacat 6220

aatttgcaga gcccagtgaa cagtgaaaat gcagtcccat cattcaaaaa ttattaagaa 6280

attcgagatg gcaacagcag ttgtcacacc aagcacagtg cccttctgag catgaggctc 6340

tgtgtgactg catgggtcca tgagaccagc actgtgtggg tctgaatgat ggctttggtg 6400

ttcagtttag cacacgcggt ctaccacgtc tgcatgagtg gtaaatgtag tgcctgggtc 6460

aaaatgttcc ttctgttcaa atcgaaatac ctcatctcta tggctctatg gctgtacatt 6520

aggacctaga acagtggccc attgctctta gactggaacc atgtccacta aaataaacct 6580

aagcagatgt tgtagaccta gccccacagg actgcattta gctgcttcag tgacactttg 6640

atgaaagtat ggagaagtgg agacattata gataaaatat atcaattccc agagaaaact 6700

cttgacttaa aaacttaact gtagtaaata tatctttttc aggtgatgaa ttattttttt 6760

aaaaaaggtt acatatagga attctgcagt ataatttgga ggctattagt gctatattaa 6820

tggaaattaa ttatttttta agtaagtcca aaaaataatc tagaaagtaa gtttccagag 6880

caaatctgac ctagcatttg gtatgctagg ctctgctttt catgattttg aaataaatca 6940

taattagact taacaatatg gagaaaataa acttgtattt ttaagtgttc tgttggctta 7000

ttttctgttt catccaactc aataattctg ataaataaat ttggttctag tttggtgctt 7060

gaaaaaaaa 7069

<210> 92
<211> 1102

<212>  PRT
<213>  Homo sapiens

<400>  92

Met Glu Leu Glu Asn Tyr Lys Gln Pro Val Val Leu Arg Glu Asp Asn
1               5                   10                  15

Cys Arg Arg Arg Arg Arg Met Lys Pro Arg Ser Ala Ala Ala Ser Leu
            20                  25                  30

Ser Ser Met Glu Leu Ile Pro Ile Glu Phe Val Leu Pro Thr Ser Gln
        35                  40                  45

Arg Lys Cys Lys Ser Pro Glu Thr Ala Leu Leu His Val Ala Gly His
    50                  55                  60

Gly Asn Val Glu Gln Met Lys Ala Gln Val Trp Leu Arg Ala Leu Glu
65                  70                  75                  80

Thr Ser Val Ala Ala Asp Phe Tyr His Arg Leu Gly Pro His His Phe
                85                  90                  95

Leu Leu Leu Tyr Gln Lys Lys Gly Gln Trp Tyr Glu Ile Tyr Asp Lys
            100                 105                 110

Tyr Gln Val Val Gln Thr Leu Asp Cys Leu Arg Tyr Trp Lys Ala Thr
        115                 120                 125

His Arg Ser Pro Gly Gln Ile His Leu Val Gln Arg His Pro Pro Ser
    130                 135                 140

Glu Glu Ser Gln Ala Phe Gln Arg Gln Leu Thr Ala Leu Ile Gly Tyr
145                 150                 155                 160

Asp Val Thr Asp Val Ser Asn Val His Asp Asp Glu Leu Glu Phe Thr
                165                 170                 175

Arg Arg Gly Leu Val Thr Pro Arg Met Ala Glu Val Ala Ser Arg Asp
            180                 185                 190

Pro Lys Leu Tyr Ala Met His Pro Trp Val Thr Ser Lys Pro Leu Pro
        195                 200                 205

Glu Tyr Leu Trp Lys Lys Ile Ala Asn Asn Cys Ile Phe Ile Val Ile
    210                 215                 220

His Arg Ser Thr Thr Ser Gln Thr Ile Lys Val Ser Pro Asp Asp Thr
225                 230                 235                 240

Pro Gly Ala Ile Leu Gln Ser Phe Phe Thr Lys Met Ala Lys Lys Lys
                245                 250                 255

Ser Leu Met Asp Ile Pro Glu Ser Gln Ser Glu Gln Asp Phe Val Leu
            260                 265                 270

Arg Val Cys Gly Arg Asp Glu Tyr Leu Val Gly Glu Thr Pro Ile Lys
        275                 280                 285

Asn Phe Gln Trp Val Arg His Cys Leu Lys Asn Gly Glu Glu Ile His
    290                 295                 300

Val Val Leu Asp Thr Pro Pro Asp Pro Ala Leu Asp Glu Val Arg Lys
305                 310                 315                 320

Glu Glu Trp Pro Leu Val Asp Asp Cys Thr Gly Val Thr Gly Tyr His
                325                 330                 335

Glu Gln Leu Thr Ile His Gly Lys Asp His Glu Ser Val Phe Thr Val
            340                 345                 350

Ser Leu Trp Asp Cys Asp Arg Lys Phe Arg Val Lys Ile Arg Gly Ile
        355                 360                 365

Asp Ile Pro Val Leu Pro Arg Asn Thr Asp Leu Thr Val Phe Val Glu
    370                 375                 380

Ala Asn Ile Gln His Gly Gln Gln Val Leu Cys Gln Arg Arg Thr Ser
385                 390                 395                 400

Pro Lys Pro Phe Thr Glu Glu Val Leu Trp Asn Val Trp Leu Glu Phe
                405                 410                 415

Ser Ile Lys Ile Lys Asp Leu Pro Lys Gly Ala Leu Leu Asn Leu Gln
            420                 425                 430

Ile Tyr Cys Gly Lys Ala Pro Ala Leu Ser Ser Lys Ala Ser Ala Glu
        435                 440                 445

Ser Pro Ser Ser Glu Ser Lys Gly Lys Val Gln Leu Leu Tyr Tyr Val
    450                 455                 460

Asn Leu Leu Leu Ile Asp His Arg Phe Leu Leu Arg Arg Gly Glu Tyr
465                 470                 475                 480

Val Leu His Met Trp Gln Ile Ser Gly Lys Gly Glu Asp Gln Gly Ser

                485                 490                 495

Phe Asn Ala Asp Lys Leu Thr Ser Ala Thr Asn Pro Asp Lys Glu Asn
            500                 505                 510

Ser Met Ser Ile Ser Ile Leu Leu Asp Asn Tyr Cys His Pro Ile Ala
        515                 520                 525

Leu Pro Lys His Gln Pro Thr Pro Asp Pro Glu Gly Asp Arg Val Arg
    530                 535                 540

Ala Glu Met Pro Asn Gln Leu Arg Lys Gln Leu Glu Ala Ile Ile Ala
545                 550                 555                 560

Thr Asp Pro Leu Asn Pro Leu Thr Ala Glu Asp Lys Glu Leu Leu Trp
                565                 570                 575

His Phe Arg Tyr Glu Ser Leu Lys His Pro Lys Ala Tyr Pro Lys Leu
            580                 585                 590

Phe Ser Ser Val Lys Trp Gly Gln Gln Glu Ile Val Ala Lys Thr Tyr
        595                 600                 605

Gln Leu Leu Ala Arg Arg Glu Val Trp Asp Gln Ser Ala Leu Asp Val
    610                 615                 620

Gly Leu Thr Met Gln Leu Leu Asp Cys Asn Phe Ser Asp Glu Asn Val
625                 630                 635                 640

Arg Ala Ile Ala Val Gln Lys Leu Glu Ser Leu Glu Asp Asp Asp Val
                645                 650                 655

Leu His Tyr Leu Leu Gln Leu Val Gln Ala Val Lys Phe Glu Pro Tyr
            660                 665                 670

His Asp Ser Ala Leu Ala Arg Phe Leu Leu Lys Arg Gly Leu Arg Asn
        675                 680                 685

Lys Arg Ile Gly His Phe Leu Phe Trp Phe Leu Arg Ser Glu Ile Ala
    690                 695                 700

Gln Ser Arg His Tyr Gln Gln Arg Phe Ala Val Ile Leu Glu Ala Tyr
705                 710                 715                 720

Leu Arg Gly Cys Gly Thr Ala Met Leu His Asp Phe Thr Gln Gln Val
                725                 730                 735

Gln Val Ile Glu Met Leu Gln Lys Val Thr Leu Asp Ile Lys Ser Leu
            740                 745                 750

Ser Ala Glu Lys Tyr Asp Val Ser Ser Gln Val Ile Ser Gln Leu Lys
        755                 760                 765

Gln Lys Leu Glu Asn Leu Gln Asn Ser Gln Leu Pro Glu Ser Phe Arg
    770                 775                 780

Val Pro Tyr Asp Pro Gly Leu Lys Ala Gly Ala Leu Ala Ile Glu Lys
785                 790                 795                 800

Cys Lys Val Met Ala Ser Lys Lys Lys Pro Leu Trp Leu Glu Phe Lys
                805                 810                 815

Cys Ala Asp Pro Thr Ala Leu Ser Asn Glu Thr Ile Gly Ile Ile Phe
            820                 825                 830

Lys His Gly Asp Asp Leu Arg Gln Asp Met Leu Ile Leu Gln Ile Leu
        835                 840                 845

Arg Ile Met Glu Ser Ile Trp Glu Thr Glu Ser Leu Asp Leu Cys Leu
    850                 855                 860

Leu Pro Tyr Gly Cys Ile Ser Thr Gly Asp Lys Ile Gly Met Ile Glu
865                 870                 875                 880

Ile Val Lys Asp Ala Thr Thr Ile Ala Lys Ile Gln Gln Ser Thr Val
                885                 890                 895

Gly Asn Thr Gly Ala Phe Lys Asp Glu Val Leu Asn His Trp Leu Lys
            900                 905                 910

Glu Lys Ser Pro Thr Glu Glu Lys Phe Gln Ala Ala Val Glu Arg Phe
        915                 920                 925

Val Tyr Ser Cys Ala Gly Tyr Cys Val Ala Thr Phe Val Leu Gly Ile
    930                 935                 940

Gly Asp Arg His Asn Asp Asn Ile Met Ile Thr Glu Thr Gly Asn Leu
945                 950                 955                 960

Phe His Ile Asp Phe Gly His Ile Leu Gly Asn Tyr Lys Ser Phe Leu
                965                 970                 975

Gly Ile Asn Lys Glu Arg Val Pro Phe Val Leu Thr Pro Asp Phe Leu
            980                 985                 990

Phe Val Met Gly Thr Ser Gly Lys  Lys Thr Ser Pro His  Phe Gln Lys
      995                  1000                 1005

Phe Gln  Asp Ile Cys Val Lys  Ala Tyr Leu Ala Leu  Arg His His
    1010                 1015                 1020

Thr Asn  Leu Leu Ile Ile Leu  Phe Ser Met Met Leu  Met Thr Gly
    1025                 1030                 1035

Met Pro  Gln Leu Thr Ser Lys  Glu Asp Ile Glu Tyr  Ile Arg Asp
    1040                 1045                 1050

Ala Leu  Thr Val Gly Lys Asn  Glu Glu Asp Ala Lys  Lys Tyr Phe
    1055                 1060                 1065

Leu Asp  Gln Ile Glu Val Cys  Arg Asp Lys Gly Trp  Thr Val Gln
    1070                 1075                 1080

Phe Asn  Trp Phe Leu His Leu  Val Leu Gly Ile Lys  Gln Gly Glu
    1085                 1090                 1095

Lys His  Ser Ala
    1100

<210>  93
<211>  2398
<212>  DNA
<213>  Homo sapiens

<220>
<221>  CDS
<222>  (242)..(877)

<400>  93
   gggaggccgg atgtgagtgg agcggccatt tcctgtttct ctgcagtttt cctcagcttt      60
   gggtggtggc cgctgccggg catcggcttc cagtccgcgg agggcgaggc ggcgtggaca     120
   gcggccccgg cacccagcgc cccgccgccc gcaagccgcg cgcccgtccg ccgcgccccg     180
   agcccgccgc ttcctatctc agcgccctgc cgccgccgcc gcggcccagc gagcggccct     240
   g atg cag gcc atc aag tgt gtg gtg gtg gga gac gga gct gta ggt aaa     289
   act tgc cta ctg atc agt tac aca acc aat gca ttt cct gga gaa tat     337
   atc cct act gtc ttt gac aat tat tct gcc aat gtt atg gta gat gga     385
   aaa ccg gtg aat ctg ggc tta tgg gat aca gct gga caa gaa gat tat     433
   gac aga tta cgc ccc cta tcc tat ccg caa aca gtt gga gaa acg tac     481
   ggt aag gat ata acc tcc cgg ggc aaa gac aag ccg att gcc gat gtg     529
   ttc tta att tgc ttt tcc ctt gtg agt cct gca tca ttt gaa aat gtc     577
   cgt gca aag tgg tat cct gag gtg cgg cac cac tgt ccc aac act ccc     625
   atc atc cta gtg gga act aaa ctt gat ctt agg gat gat aaa gac acg     673
   atc gag aaa ctg aag gag aag aag ctg act ccc atc acc tat ccg cag     721
   ggt cta gcc atg gct aag gag att ggt gct gta aaa tac ctg gag tgc     769
   tcg gcg ctc aca cag cga ggc ctc aag aca gtg ttt gac gaa gcg atc     817
   cga gca gtc ctc tgc ccg cct ccc gtg aag aag agg aag aga aaa tgc     865
   ctg ctg ttg taa atgtctcagc ccctcgttct tggtcctgtc ccttggaacc 917
   tttgtacgct ttgctcaaaa aaaaacaaaa aaaaaaaaca aaaaaaaaaa acaacggtgg     977
   agccttcgca ctcaatgcca actttttgtt acagattaat ttttccataa aaccattttt     1037
   tgaaccaatc agtaatttta aggttttgtt tgttctaaat gtaagagttc agactcacat     1097
   tctattaaaa tttagcccta aaatgacaag ccttcttaaa gccttatttt tcaaaagcgc     1157
   cccccccatt cttgttcaga ttaagagttg ccaaaatacc ttctgaacta cactgcattg     1217
   ttgtgccgag aacaccgagc actgaacttt gcaaagacct tcgtctttga gaagacggta     1277
   gcttctgcag ttaggaggtg cagacacttg ctctcctatg tagttctcag atgcgtaaag     1337
   cagaacagcc tcccgaatga agcgttgcca ttgaactcac cagtgagtta gcagcacgtg     1397
   ttcccgacat aacattgtac tgtaatggag tgagcgtagc agctcagctc tttggatcag     1457
   tctttgtgat ttcatagcga gttttctgac cagcttttgc ggagattttg aacagaactg 1517
   ctatttcctc taatgaagaa ttctgtttag ctgtgggtgt gccgggtggg gtgtgtgtga 1577
   tcaaaggaca aagacagtat tttgacaaaa tacgaagtgg agatttacac tacattgtac 1637
   aaggaatgaa agtgtcacgg gtaaaaactc taaaaggtta atttctgtca aatgcagtag 1697
   atgatgaaag aaaggttggt attatcagga aatgttttct taagcttttc ctttctctta 1757
   cacctgccat gcctccccaa attgggcatt taattcatct ttaaactggt tgttctgtta 1817
   gtcgctaact tagtaagtgc ttttcttata gaaccccttc tgactgagca atatgcctcc 1877
   ttgtattata aaatctttct gataatgcat tagaaggttt ttttgtcgat tagtaaaagt 1937
   gctttccatg ttactttatt cagagctaat aagtgctttc cttagttttc tagtaactag 1997
   gtgtaaaaat catgtgttgc agctttatag tttttaaaat attttagata attcttaaac 2057
   tatgaacctt cttaacatca ctgtcttgcc agattaccga cactgtcact tgaccaatac 2117
   tgaccctctt tacctcgccc acgcggacac acgcctcctg tagtcgcttt gcctattgat 2177
   gttcctttgg gtctgtgagg ttctgtaaac tgtgctagtg ctgacgatgt tctgtacaac 2237
   ttaactcact ggcgagaata cagcgtggga cccttcagcc actacaacag aattttttaa 2297
   attgacagtt gcagaattgt ggagtgtttt tacattgatc ttttgctaat gcaattagca 2357
   ttatgttttg catgtatgac ttaataaatc cttgaatcat a 2398

<210> 94
<211> 211
<212> PRT
<213> Homo sapiens

<400> 94

<210>  95
<211>  1734
<212>  DNA
<213>  Homo sapiens

<220>
<221>  CDS
<222>  (113)..(1510)

<400>  95
gctgagtcat cactagagag tgggaagggc agcagcagca gagaatccaa accctaaagc      60

tgatatcaca aagtaccatt tctccaagtt gggggctcag aggggagtca tc atg agc     118
                                                          Met Ser
                                                          1

gat gtt acc att gtg aaa gaa ggt tgg gtt cag aag agg gga gaa tat       166
Asp Val Thr Ile Val Lys Glu Gly Trp Val Gln Lys Arg Gly Glu Tyr
        5                   10                  15

ata aaa aac tgg agg cca aga tac ttc ctt ttg aag aca gat ggc tca       214
Ile Lys Asn Trp Arg Pro Arg Tyr Phe Leu Leu Lys Thr Asp Gly Ser
    20                  25                  30

ttc ata gga tat aaa gag aaa cct caa gat gtg gat tta cct tat ccc       262
Phe Ile Gly Tyr Lys Glu Lys Pro Gln Asp Val Asp Leu Pro Tyr Pro
35                  40                  45                  50

ctc aac aac ttt tca gtg gca aaa tgc cag tta atg aaa aca gaa cga       310

Leu Asn Asn Phe Ser Val Ala Lys Cys Gln Leu Met Lys Thr Glu Arg
                55                  60                  65

cca aag cca aac aca ttt ata atc aga tgt ctc cag tgg act act gtt      358
Pro Lys Pro Asn Thr Phe Ile Ile Arg Cys Leu Gln Trp Thr Thr Val
            70                  75                  80

ata gag aga aca ttt cat gta gat act cca gag gaa agg gaa gaa tgg      406
Ile Glu Arg Thr Phe His Val Asp Thr Pro Glu Glu Arg Glu Glu Trp
        85                  90                  95

aca gaa gct atc cag gct gta gca gac aga ctg cag agg caa gaa gag      454
Thr Glu Ala Ile Gln Ala Val Ala Asp Arg Leu Gln Arg Gln Glu Glu
    100                 105                 110

gag aga atg aat tgt agt cca act tca caa att gat aat ata gga gag      502
Glu Arg Met Asn Cys Ser Pro Thr Ser Gln Ile Asp Asn Ile Gly Glu
115                 120                 125                 130

gaa gag atg gat gcc tct aca acc cat cat aaa aga aag aca atg aat      550
Glu Glu Met Asp Ala Ser Thr Thr His His Lys Arg Lys Thr Met Asn
                135                 140                 145

gat ttt gac tat ttg aaa cta cta ggt aaa ggc act ttt ggg aaa gtt      598
Asp Phe Asp Tyr Leu Lys Leu Leu Gly Lys Gly Thr Phe Gly Lys Val
            150                 155                 160

att ttg gtt cga gag aag gca agt gga aaa tac tat gct atg aag att      646
Ile Leu Val Arg Glu Lys Ala Ser Gly Lys Tyr Tyr Ala Met Lys Ile
        165                 170                 175

ctg aag aaa gaa gtc att att gca aag gat gaa gtg gca cac act cta      694
Leu Lys Lys Glu Val Ile Ile Ala Lys Asp Glu Val Ala His Thr Leu
    180                 185                 190

act gaa agc aga gta tta aag aac act aga cat ccc ttt tta aca tcc      742
Thr Glu Ser Arg Val Leu Lys Asn Thr Arg His Pro Phe Leu Thr Ser
195                 200                 205                 210

ttg aaa tat tcc ttc cag aca aaa gac cgt ttg tgt ttt gtg atg gaa      790
Leu Lys Tyr Ser Phe Gln Thr Lys Asp Arg Leu Cys Phe Val Met Glu
                215                 220                 225

tat gtt aat ggg ggc gag ctg ttt ttc cat ttg tcg aga gag cgg gtg      838
Tyr Val Asn Gly Gly Glu Leu Phe Phe His Leu Ser Arg Glu Arg Val
            230                 235                 240

ttc tct gag gac cgc aca cgt ttc tat ggt gca gaa att gtc tct gcc      886
Phe Ser Glu Asp Arg Thr Arg Phe Tyr Gly Ala Glu Ile Val Ser Ala
        245                 250                 255

ttg gac tat cta cat tcc gga aag att gtg tac cgt gat ctc aag ttg      934
Leu Asp Tyr Leu His Ser Gly Lys Ile Val Tyr Arg Asp Leu Lys Leu
    260                 265                 270

gag aat cta atg ctg gac aaa gat ggc cac ata aaa att aca gat ttt      982
Glu Asn Leu Met Leu Asp Lys Asp Gly His Ile Lys Ile Thr Asp Phe
275                 280                 285                 290

gga ctt tgc aaa gaa ggg atc aca gat gca gcc acc atg aag aca ttc     1030
Gly Leu Cys Lys Glu Gly Ile Thr Asp Ala Ala Thr Met Lys Thr Phe
                295                 300                 305

tgt ggc act cca gaa tat ctg gca cca gag gtg tta gaa gat aat gac     1078
Cys Gly Thr Pro Glu Tyr Leu Ala Pro Glu Val Leu Glu Asp Asn Asp
            310                 315                 320

tat ggc cga gca gta gac tgg tgg ggc cta ggg gtt gtc atg tat gaa     1126
Tyr Gly Arg Ala Val Asp Trp Trp Gly Leu Gly Val Val Met Tyr Glu
        325                 330                 335

atg atg tgt ggg agg tta cct ttc tac aac cag gac cat gag aaa ctt     1174
Met Met Cys Gly Arg Leu Pro Phe Tyr Asn Gln Asp His Glu Lys Leu
    340                 345                 350

ttt gaa tta ata tta atg gaa gac att aaa ttt cct cga aca ctc tct     1222
Phe Glu Leu Ile Leu Met Glu Asp Ile Lys Phe Pro Arg Thr Leu Ser
355                 360                 365                 370

tca gat gca aaa tca ttg ctt tca ggg ctc ttg ata aag gat cca aat     1270
Ser Asp Ala Lys Ser Leu Leu Ser Gly Leu Leu Ile Lys Asp Pro Asn
                375                 380                 385

aaa cgc ctt ggt gga gga cca gat gat gca aaa gaa att atg aga cac     1318
Lys Arg Leu Gly Gly Gly Pro Asp Asp Ala Lys Glu Ile Met Arg His
            390                 395                 400

agt ttc ttc tct gga gta aac tgg caa gat gta tat gat aaa aag ctt     1366
Ser Phe Phe Ser Gly Val Asn Trp Gln Asp Val Tyr Asp Lys Lys Leu
        405                 410                 415

gta cct cct ttt aaa cct caa gta aca tct gag aca gat act aga tat     1414
Val Pro Pro Phe Lys Pro Gln Val Thr Ser Glu Thr Asp Thr Arg Tyr
    420                 425                 430

ttt gat gaa gaa ttt aca gct cag act att aca ata aca cca cct gaa     1462
Phe Asp Glu Glu Phe Thr Ala Gln Thr Ile Thr Ile Thr Pro Pro Glu
435                 440                 445                 450

aaa tgt cag caa tca gat tgt ggc atg ctg ggt aac tgg aaa aaa taa     1510
Lys Cys Gln Gln Ser Asp Cys Gly Met Leu Gly Asn Trp Lys Lys
                455                 460                 465

taaaaatcgg cttcctacag ccagcagcac agtcacccat ggaactgttg gctttggatt     1570

aaatgtggaa ttgaacgact acccagaagt gttctggaaa gaagcgagat gtgtggcctg     1630

cctcaccgtc ctcacccatc aaaagcacca gcaggcacgt taactcgaat tctcacaagg     1690

aaaaggccat taaagctcaa ggtgcatttc aaactccagg ctac                      1734

<210>  96
<211>  465
<212>  PRT
<213>  Homo sapiens

<400>  96

Met Ser Asp Val Thr Ile Val Lys Glu Gly Trp Val Gln Lys Arg Gly
1               5                   10                  15

Glu Tyr Ile Lys Asn Trp Arg Pro Arg Tyr Phe Leu Leu Lys Thr Asp
            20                  25                  30

Gly Ser Phe Ile Gly Tyr Lys Glu Lys Pro Gln Asp Val Asp Leu Pro
        35                  40                  45

Tyr Pro Leu Asn Asn Phe Ser Val Ala Lys Cys Gln Leu Met Lys Thr
    50                  55                  60

Glu Arg Pro Lys Pro Asn Thr Phe Ile Ile Arg Cys Leu Gln Trp Thr
65                  70                  75                  80

Thr Val Ile Glu Arg Thr Phe His Val Asp Thr Pro Glu Glu Arg Glu
                85                  90                  95

Glu Trp Thr Glu Ala Ile Gln Ala Val Ala Asp Arg Leu Gln Arg Gln
            100                 105                 110

Glu Glu Glu Arg Met Asn Cys Ser Pro Thr Ser Gln Ile Asp Asn Ile
        115                 120                 125

Gly Glu Glu Glu Met Asp Ala Ser Thr Thr His His Lys Arg Lys Thr
    130                 135                 140

Met Asn Asp Phe Asp Tyr Leu Lys Leu Leu Gly Lys Gly Thr Phe Gly
145                 150                 155                 160

Lys Val Ile Leu Val Arg Glu Lys Ala Ser Gly Lys Tyr Tyr Ala Met
                165                 170                 175

Lys Ile Leu Lys Lys Glu Val Ile Ile Ala Lys Asp Glu Val Ala His
            180                 185                 190

Thr Leu Thr Glu Ser Arg Val Leu Lys Asn Thr Arg His Pro Phe Leu
        195                 200                 205

Thr Ser Leu Lys Tyr Ser Phe Gln Thr Lys Asp Arg Leu Cys Phe Val
    210                 215                 220

Met Glu Tyr Val Asn Gly Gly Glu Leu Phe Phe His Leu Ser Arg Glu
225                 230                 235                 240

Arg Val Phe Ser Glu Asp Arg Thr Arg Phe Tyr Gly Ala Glu Ile Val
                245                 250                 255

Ser Ala Leu Asp Tyr Leu His Ser Gly Lys Ile Val Tyr Arg Asp Leu
            260                 265                 270

Lys Leu Glu Asn Leu Met Leu Asp Lys Asp Gly His Ile Lys Ile Thr
        275                 280                 285

Asp Phe Gly Leu Cys Lys Glu Gly Ile Thr Asp Ala Ala Thr Met Lys
    290                 295                 300

Thr Phe Cys Gly Thr Pro Glu Tyr Leu Ala Pro Glu Val Leu Glu Asp
305                 310                 315                 320

Asn Asp Tyr Gly Arg Ala Val Asp Trp Trp Gly Leu Gly Val Val Met
                325                 330                 335

Tyr Glu Met Met Cys Gly Arg Leu Pro Phe Tyr Asn Gln Asp His Glu
            340                 345                 350

Lys Leu Phe Glu Leu Ile Leu Met Glu Asp Ile Lys Phe Pro Arg Thr
        355                 360                 365

Leu Ser Ser Asp Ala Lys Ser Leu Leu Ser Gly Leu Leu Ile Lys Asp
    370                 375                 380

Pro Asn Lys Arg Leu Gly Gly Gly Pro Asp Asp Ala Lys Glu Ile Met
385                 390                 395                 400

Arg His Ser Phe Phe Ser Gly Val Asn Trp Gln Asp Val Tyr Asp Lys
                405                 410                 415

Lys Leu Val Pro Pro Phe Lys Pro Gln Val Thr Ser Glu Thr Asp Thr
            420                 425                 430

Arg Tyr Phe Asp Glu Glu Phe Thr Ala Gln Thr Ile Thr Ile Thr Pro
        435                 440                 445

Pro Glu Lys Cys Gln Gln Ser Asp Cys Gly Met Leu Gly Asn Trp Lys
    450                 455                 460

Lys
465

<210>  97
<211>  4076
<212>  DNA
<213>  Homo sapiens

<220>
<221>  CDS
<222>  (227)..(2077)

<400>  97
aacccaggcc ccgcccettt ggccacatgt cgcgcatgtc ttcccgtcgg acggcgtgcc        60

acctcgccgc gcagctttac gaacctagag cagcgccgcc ccgcctcctg tctccgtcct 120

cacctccccg ccccctccca gcttcgcgtc tcctagctcg acgcgcccgc tataatcacg 180

tgattgcctc atccgggtct tttgcgttct ctttccctct cccaac atg gcg gcc 235
Met Ala Ala
1

tca gca aaa aag aag aat aag aag ggg aag act atc tcc cta aca gac 283
Ser Ala Lys Lys Lys Asn Lys Lys Gly Lys Thr Ile Ser Leu Thr Asp
    5                   10                  15

ttt ctg gct gag gat ggg ggt act ggt gga gga agc acc tat gtt tcc 331
Phe Leu Ala Glu Asp Gly Gly Thr Gly Gly Gly Ser Thr Tyr Val Ser
20                  25                  30                  35

aaa cca gtc agc tgg gct gat gaa acg gat gac ctg gaa gga gat gtt 379
Lys Pro Val Ser Trp Ala Asp Glu Thr Asp Asp Leu Glu Gly Asp Val
                40                  45                  50

tcg acc act tgg cac agt aac gat gac gat gtg tat agg gcg cct cca 427
Ser Thr Thr Trp His Ser Asn Asp Asp Asp Val Tyr Arg Ala Pro Pro
            55                  60                  65

att gac cgt tcc atc ctt ccc act gct cca cgg gct gct cgg gaa ccc 475
Ile Asp Arg Ser Ile Leu Pro Thr Ala Pro Arg Ala Ala Arg Glu Pro
        70                  75                  80

aat atc gac cgg agc cgt ctt ccc aaa tcg cca ccc tac act gct ttt 523
Asn Ile Asp Arg Ser Arg Leu Pro Lys Ser Pro Pro Tyr Thr Ala Phe
    85                  90                  95

cta gga aac cta ccc tat gat gtt aca gaa gag tca att aag gaa ttc 571
Leu Gly Asn Leu Pro Tyr Asp Val Thr Glu Glu Ser Ile Lys Glu Phe
100                 105                 110                 115

ttt cga gga tta aat atc agt gca gtg cgt tta cca cgt gaa ccc agc 619
Phe Arg Gly Leu Asn Ile Ser Ala Val Arg Leu Pro Arg Glu Pro Ser
                120                 125                 130

aat cca gag agg ttg aaa ggt ttt ggt tat gct gaa ttt gag gac ctg 667
Asn Pro Glu Arg Leu Lys Gly Phe Gly Tyr Ala Glu Phe Glu Asp Leu
            135                 140                 145

gat tcc ctg ctc agt gcc ctg agt ctc aat gaa gag tct cta ggt aac 715
Asp Ser Leu Leu Ser Ala Leu Ser Leu Asn Glu Glu Ser Leu Gly Asn
        150                 155                 160

agg aga att cga gtg gac gtt gct gat caa gca cag gat aaa gac agg 763
Arg Arg Ile Arg Val Asp Val Ala Asp Gln Ala Gln Asp Lys Asp Arg
    165                 170                 175

gat gat cgt tct ttt ggc cgt gat aga aat cgg gat tct gac aaa aca 811
Asp Asp Arg Ser Phe Gly Arg Asp Arg Asn Arg Asp Ser Asp Lys Thr
180                 185                 190                 195

gat aca gac tgg agg gct cgt cct gct aca gac agc ttt gat gac tac 859
Asp Thr Asp Trp Arg Ala Arg Pro Ala Thr Asp Ser Phe Asp Asp Tyr
                200                 205                 210

cca cct aga aga ggt gat gat agc ttt gga gac aag tat cga gat cgt 907
Pro Pro Arg Arg Gly Asp Asp Ser Phe Gly Asp Lys Tyr Arg Asp Arg
            215                 220                 225

tat gat tca gac cgg tat cgg gat ggg tat cgg gat ggg tat cgg gat 955
Tyr Asp Ser Asp Arg Tyr Arg Asp Gly Tyr Arg Asp Gly Tyr Arg Asp
        230                 235                 240

ggc cca cgc cgg gat atg gat cga tat ggt ggc cgg gat cgc tat gat 1003
Gly Pro Arg Arg Asp Met Asp Arg Tyr Gly Gly Arg Asp Arg Tyr Asp
    245                 250                 255

gac cga ggc agc aga gac tat gat aga ggc tat gat tcc cgg ata ggc 1051
Asp Arg Gly Ser Arg Asp Tyr Asp Arg Gly Tyr Asp Ser Arg Ile Gly
260                 265                 270                 275

agt ggc aga aga gca ttt ggc agt ggg tat cgc agg gat gat gac tac 1099
Ser Gly Arg Arg Ala Phe Gly Ser Gly Tyr Arg Arg Asp Asp Asp Tyr
                280                 285                 290

aga gga ggc ggg gac cgc tat gaa gac cga tat gac aga cgg gat gat 1147
Arg Gly Gly Gly Asp Arg Tyr Glu Asp Arg Tyr Asp Arg Arg Asp Asp
            295                 300                 305

cgg tcg tgg agc tcc aga gat gat tac tct cgg gat gat tat agg cgt 1195
Arg Ser Trp Ser Ser Arg Asp Asp Tyr Ser Arg Asp Asp Tyr Arg Arg
        310                 315                 320

gat gat aga ggt ccc ccc caa aga ccc aaa ctg aat cta aag cct cgg 1243
Asp Asp Arg Gly Pro Pro Gln Arg Pro Lys Leu Asn Leu Lys Pro Arg
    325                 330                 335

agt act cct aag gaa gat gat tcc tct gct agt acc tcc cag tcc act 1291
Ser Thr Pro Lys Glu Asp Asp Ser Ser Ala Ser Thr Ser Gln Ser Thr
340                 345                 350                 355

cga gct gct tct atc ttt gga ggg gca aag cct gtt gac aca gct gct 1339
Arg Ala Ala Ser Ile Phe Gly Gly Ala Lys Pro Val Asp Thr Ala Ala
                360                 365                 370

aga gaa aga gaa gta gaa gaa cgg cta cag aag gaa caa gag aag ttg 1387
Arg Glu Arg Glu Val Glu Glu Arg Leu Gln Lys Glu Gln Glu Lys Leu
            375                 380                 385

cag cgt cag ctg gat gag cca aaa cta gaa cga cgg cct cgg gag aga 1435
Gln Arg Gln Leu Asp Glu Pro Lys Leu Glu Arg Arg Pro Arg Glu Arg
        390                 395                 400

cac cca agc tgg cga agt gaa gaa act cag gaa cgg gaa cgg tcg agg 1483
His Pro Ser Trp Arg Ser Glu Glu Thr Gln Glu Arg Glu Arg Ser Arg
    405                 410                 415

aca gga agt gag tca tca caa act ggg acc tcc acc aca tct agc aga 1531
Thr Gly Ser Glu Ser Ser Gln Thr Gly Thr Ser Thr Thr Ser Ser Arg
420                 425                 430                 435

agt aag tca gac cag gat gca cga agg aga gag agt gag aag tct cta 1579
Ser Lys Ser Asp Gln Asp Ala Arg Arg Arg Glu Ser Glu Lys Ser Leu
                440                 445                 450

gaa aat gaa aca ctc aat aag gag gaa gat tgc cac tct cca act tct 1627
Glu Asn Glu Thr Leu Asn Lys Glu Glu Asp Cys His Ser Pro Thr Ser
            455                 460                 465

aaa cct ccc aaa cct gat cag ccc cta aag gta atg cca gcc cct cca 1675
Lys Pro Pro Lys Pro Asp Gln Pro Leu Lys Val Met Pro Ala Pro Pro

470                 475                 480

cca aag gag aat gct tgg gtg aag cga agt tct aac cct cct gct cga     1723
Pro Lys Glu Asn Ala Trp Val Lys Arg Ser Ser Asn Pro Pro Ala Arg
    485                 490                 495

tct cag agc tca gac aca gag cag cag tcc cct aca agt ggt ggg gga     1771
Ser Gln Ser Ser Asp Thr Glu Gln Gln Ser Pro Thr Ser Gly Gly Gly
500                 505                 510                 515

aaa gta gct cca gct caa cca tct gag gaa gga cca gga agg aaa gat     1819
Lys Val Ala Pro Ala Gln Pro Ser Glu Glu Gly Pro Gly Arg Lys Asp
                520                 525                 530

gaa aat aaa gta gat ggg atg aat gcc cca aaa ggc caa act ggg aac     1867
Glu Asn Lys Val Asp Gly Met Asn Ala Pro Lys Gly Gln Thr Gly Asn
            535                 540                 545

tct agc cgt ggt cca gga gac gga ggg aac aga gac cac tgg aag gag     1915
Ser Ser Arg Gly Pro Gly Asp Gly Gly Asn Arg Asp His Trp Lys Glu
        550                 555                 560

tca gat agg aaa gat ggc aaa aag gat caa gac tcc aga tct gca cct     1963
Ser Asp Arg Lys Asp Gly Lys Lys Asp Gln Asp Ser Arg Ser Ala Pro
    565                 570                 575

gag cca aag aaa cct gag gaa aat cca gct tcc aag ttc agt tct gca     2011
Glu Pro Lys Lys Pro Glu Glu Asn Pro Ala Ser Lys Phe Ser Ser Ala
580                 585                 590                 595

agc aag tat gct gct ctc tct gtt gat ggt gaa gat gaa aat gag gga     2059
Ser Lys Tyr Ala Ala Leu Ser Val Asp Gly Glu Asp Glu Asn Glu Gly
                600                 605                 610

gaa gat tat gcc gaa tag acctctacat cctgtgcttt tctcctagtt            2107
Glu Asp Tyr Ala Glu
            615

tctctccacc ctggaacatt cgagagcaaa tcaaaacctc tatccagaca agacaaaata   2167

aaactcacca tctcctgaag acctttctta ccttttttta aaaacaaaaa atgaaattat   2227

tttgcatgct gctgcagcct ttaaagtatt gaagtaactg gagaattgcc aatacagcca   2287

gagagaaagg gactacagct ttttagagga aaagttgtgg tgcgttatgt caccatgcag   2347

ttgccagtgt gattagtgcc taggggtctc catttagcag aaatggtaat gacagtgata   2407

taatgcctgg aacctggttg ggcagtaggg gagggaggta gaaggaaaag tgtgagattt   2467

ctacctttta gtttttatcc tattgtggca tatatgaatt ctcaaacatt atctgaataa   2527

attttccact cttggaaagg tagatttagc ctcaagttgt tctagtctcc aggaggctgc   2587

cagcccctcc tcttatttaa ttctgagttt tgggggccag cctagaggga attccttttt   2647

tttttttaac cccccagggg ggtagttggg agtgagacta taggccataa agaatgggac   2707

tgcattggac caaaataaat gggaaaatcg tggtttgaaa agaagctttt gggaagtgat   2767

gagtcatttt gcaccaggta ataggggaaa attgtgtgac ctccagcaaa cacatgaatg   2827

gttatttcct ggagccggaa gcacttgggg gtcgtggtaa ttcccagtgt tttctgtgtc   2887

ctagttttac cctttctaaa cactgtcctt tttgaaagtt ttgaatatat ccacattcta 2947

ttgaaacctt gaaactaaaa atttagactc ttatcatcat cttaagttct tcatgctact 3007

cttaacctcc caaaaagcag tatctaagtc acatacatga tgtcttgggc attttctcag 3067

ccatggagaa ctctgaaagg aagaatcgct gcttttctca agcaaatcgg tttcttgatg 3127

tcttttggtt ctccttgcct gctcctgatg cttggacccc ttttattgat cagagtgctc 3187

tagaataatg gatggtcttg gatgatggat aaatagggac agggacagtt aaattgggag 3247

cctttcttac aaccttgatg ggatttttcc ccccaagttt ccttctccac tgaaatgcca 3307

cactaatgct tgttggattc atgaggtggc cagaccaatg tgttgttttg ttgttgtttt 3367

tttaagcttc ccttgagaga ataaatggta atggagagaa ctatttaaca aggtcctggt 3427

ttctcttgca acacagtagc taaacttgcc tgcttttata tgcatttttg tagggatcag 3487

cttggtagac agtattagcg gagaaacacc ttgatcttgg tttgcaagcc cttctcccat 3547

cagtcctaga ttaggccctg ttcagccatg caggggtgtt ggtttatgcg tgctgcagca 3607

gtgggcataa tgaatataat ttacccagtg gacaaaggtg tgtaccaagt gaatttaaat 3667

aattggtgtg gattggccag tagctaagaa gtgggctttt aaagagtatt gaagattgaa 3727

agggtttttc tttctttttt aaaaaagaaa aacaaactat tgattgtaga taatgaaaag 3787

ctagggtttg ccctcttcat gtctactctc cttccaaata gttatatcca aaactgtttt 3847

tccctctccc ctaccttgtc ccccctatta aaatagaaac agggattgat taatgtcccg 3907

ctcctgaata catgtaaaat ttgtacaaaa atatcttcta tgaaaatgat ttgtaatctg 3967

tagacttatt acctgggaga tgtcttgatg taaaatccca tcctttgggt tgtgggtttt 4027

ttgttttctc caaataaatc tgatctttaa agttcaaaaa aaaaaaaaa 4076

<210> 98
<211> 616
<212> PRT
<213> Homo sapiens

<400> 98

Met Ala Ala Ser Ala Lys Lys Lys Asn Lys Lys Gly Lys Thr Ile Ser
1               5                   10                  15

Leu Thr Asp Phe Leu Ala Glu Asp Gly Gly Thr Gly Gly Gly Ser Thr
            20                  25                  30

Tyr Val Ser Lys Pro Val Ser Trp Ala Asp Glu Thr Asp Asp Leu Glu
        35                  40                  45

Gly Asp Val Ser Thr Thr Trp His Ser Asn Asp Asp Asp Val Tyr Arg
    50                  55                  60

Ala Pro Pro Ile Asp Arg Ser Ile Leu Pro Thr Ala Pro Arg Ala Ala
65                  70                  75                  80

Arg Glu Pro Asn Ile Asp Arg Ser Arg Leu Pro Lys Ser Pro Pro Tyr
                85                  90                  95

Thr Ala Phe Leu Gly Asn Leu Pro Tyr Asp Val Thr Glu Glu Ser Ile
            100                 105                 110

Lys Glu Phe Phe Arg Gly Leu Asn Ile Ser Ala Val Arg Leu Pro Arg
        115                 120                 125

Glu Pro Ser Asn Pro Glu Arg Leu Lys Gly Phe Gly Tyr Ala Glu Phe
    130                 135                 140

Glu Asp Leu Asp Ser Leu Leu Ser Ala Leu Ser Leu Asn Glu Glu Ser
145                 150                 155                 160

Leu Gly Asn Arg Arg Ile Arg Val Asp Val Ala Asp Gln Ala Gln Asp
                165                 170                 175

Lys Asp Arg Asp Asp Arg Ser Phe Gly Arg Asp Arg Asn Arg Asp Ser
            180                 185                 190

Asp Lys Thr Asp Thr Asp Trp Arg Ala Arg Pro Ala Thr Asp Ser Phe
        195                 200                 205

Asp Asp Tyr Pro Pro Arg Arg Gly Asp Asp Ser Phe Gly Asp Lys Tyr
    210                 215                 220

Arg Asp Arg Tyr Asp Ser Asp Arg Tyr Arg Asp Gly Tyr Arg Asp Gly
225                 230                 235                 240

Tyr Arg Asp Gly Pro Arg Arg Asp Met Asp Arg Tyr Gly Gly Arg Asp
                245                 250                 255

Arg Tyr Asp Asp Arg Gly Ser Arg Asp Tyr Asp Arg Gly Tyr Asp Ser
            260                 265                 270

Arg Ile Gly Ser Gly Arg Arg Ala Phe Gly Ser Gly Tyr Arg Arg Asp
        275                 280                 285

Asp Asp Tyr Arg Gly Gly Gly Asp Arg Tyr Glu Asp Arg Tyr Asp Arg
    290                 295                 300

Arg Asp Asp Arg Ser Trp Ser Ser Arg Asp Asp Tyr Ser Arg Asp Asp

305                 310                 315                 320

Tyr Arg Arg Asp Asp Arg Gly Pro Pro Gln Arg Pro Lys Leu Asn Leu
                325                 330                 335

Lys Pro Arg Ser Thr Pro Lys Glu Asp Asp Ser Ser Ala Ser Thr Ser
            340                 345                 350

Gln Ser Thr Arg Ala Ala Ser Ile Phe Gly Gly Ala Lys Pro Val Asp
        355                 360                 365

Thr Ala Ala Arg Glu Arg Glu Val Glu Glu Arg Leu Gln Lys Glu Gln
    370                 375                 380

Glu Lys Leu Gln Arg Gln Leu Asp Glu Pro Lys Leu Glu Arg Arg Pro
385                 390                 395                 400

Arg Glu Arg His Pro Ser Trp Arg Ser Glu Glu Thr Gln Glu Arg Glu
                405                 410                 415

Arg Ser Arg Thr Gly Ser Glu Ser Ser Gln Thr Gly Thr Ser Thr Thr
            420                 425                 430

Ser Ser Arg Ser Lys Ser Asp Gln Asp Ala Arg Arg Arg Glu Ser Glu
        435                 440                 445

Lys Ser Leu Glu Asn Glu Thr Leu Asn Lys Glu Glu Asp Cys His Ser
    450                 455                 460

Pro Thr Ser Lys Pro Pro Lys Pro Asp Gln Pro Leu Lys Val Met Pro
465                 470                 475                 480

Ala Pro Pro Pro Lys Glu Asn Ala Trp Val Lys Arg Ser Ser Asn Pro
                485                 490                 495

Pro Ala Arg Ser Gln Ser Ser Asp Thr Glu Gln Gln Ser Pro Thr Ser
            500                 505                 510

Gly Gly Gly Lys Val Ala Pro Ala Gln Pro Ser Glu Glu Gly Pro Gly
        515                 520                 525

Arg Lys Asp Glu Asn Lys Val Asp Gly Met Asn Ala Pro Lys Gly Gln
    530                 535                 540

Thr Gly Asn Ser Ser Arg Gly Pro Gly Asp Gly Gly Asn Arg Asp His
545                 550                 555                 560

Trp Lys Glu Ser Asp Arg Lys Asp Gly Lys Lys Asp Gln Asp Ser Arg
                565                 570                 575

Ser Ala Pro Glu Pro Lys Lys Pro Glu Glu Asn Pro Ala Ser Lys Phe
            580                 585                 590

Ser Ser Ala Ser Lys Tyr Ala Ala Leu Ser Val Asp Gly Glu Asp Glu
        595                 600                 605

Asn Glu Gly Glu Asp Tyr Ala Glu
    610                 615

<210>  99
<211>  4749
<212>  DNA
<213>  Homo sapiens

<220>
<221>  CDS
<222>  (1524)..(2177)

<400>  99
gcacaggcag cctgcataca ctccttttcc tggtgtcaac attatttaaa agcatgggaa      60
atagtaatga gacagtgtct tcttcattag aaccttagga gtctactaga tttcttcatc     120
tctatttgtt gttattagta gccaaactgt gcaaaaaaca cggtcttgag aaatgacagc     180
acagtatctt agagggaaag gaaatgtagg atgccagtgt ggggacaaat ttctgattgc     240
cagtgattgt tgtgagcata acaataattt catgaacatt aaagcctcta ttgagggcag     300
ctgcagttgt aaaggaaaaa aaatggtcct gaacatttaa aactacactg gtgtacatca     360
taatcaaaca aagtaaacag aaaaaaattt aaactttgct aaaaaaaaaa agcagaagca     420
cttgatcttt aggaaggcac gcagttgctt attatgaatc atttctagag tccgatgcat     480
tttcaaagcc ggttacagtc attacgaagc acacccttgt gaggtaagtg tatcatcacc     540
tttggttcat aaataaaaaa gctgagacgc cgagcgatta agtcactcgc ctaaggagaa     600
tgagtcaacg tcaagagtca tagttgaccc ggcctaaaga ctccagacca tcagtccagg     660
gcttagtcag cggggcccgg agtggcttcc ctggctggca tctggactta ggctatttcc     720
gtgcacgtaa aagcggaata ttggaacggt tgcacagaac ttccaaataa tttttaccgc     780
cacgcaagat ttagccctga ggtcttaatc tcaggatttg ggacagtaaa agctgtcgtc     840
cctccccctc gtccagccgg tggcaagcgg gtactgcggg cggttccgtc cgtccccttt     900
cgcagaaatg gcaacgaatg accaccagca ttagctgagc caggggacgt gggagggttg     960
attgcctaaa cgactctgca tcgccgcctc tttttgaaac taagagaaaa tggtgggaga    1020
tcaaaagaaa actaaataaa cacacaggca acttgtcctg ggacctcaac taagcaaatg    1080
aagccttatt gtgtgtgctg agcctgcagt tcccaacctt ccggggaaga tgggaggaca 1140
gggcgacaaa gggcacagta ggcttgcctg gcagtaagtg tgaccgcagc tatccaggcg 1200
gaagagcaga ggactgaaac caccctccag caagcgagtg tccgccgcgt tgagaaccgc 1260
gcaccctacc catcggccac gtgaccagtc ctttttaaaa aaaatttctt taccttaaaa 1320
aaaaaaaaaa aaaaaaggtg ggggagagac tccacttccc agaagcctct cgttactcac 1380
gcagccgcag tcttgcgcag gtgccgccag ggccaaacgg acatatccgt cacgtggcca 1440
gaagctggcc aatccggttt gaatctcatt tttttcctct taccccccct tctggagcgg 1500
ttgtgcgatc agatcgatct aag atg gcg act gtc gaa ccg gaa acc acc cct 1553
act cct aat ccc ccg act aca gaa gag gag aaa acg gaa tct aat cag 1601
gag gtt gct aac cca gaa cac tat att aaa cat ccc cta cag aac aga 1649
tgg gca ctc tgg ttt ttt aaa aat gat aaa agc aaa act tgg caa gca 1697
aac ctg cgg ctg atc tcc aag ttt gat act gtt gaa gac ttt tgg gct 1745
ctg tac aac cat atc cag ttg tct agt aat tta atg cct ggc tgt gac 1793
tac tca ctt ttt aag gat ggt att gag cct atg tgg gaa gat gag aaa 1841
aac aaa cgg gga gga cga tgg cta att aca ttg aac aaa cag cag aga 1889
cga agt gac ctc gat cgc ttt tgg cta gag aca ctt ctg tgc ctt att 1937
gga gaa tct ttt gat gac tac agt gat gat gta tgt ggc gct gtt gtt 1985
aat gtt aga gct aaa ggt gat aag ata gca ata tgg act act gaa tgt 2033
gaa aac aga gaa gct gtt aca cat ata ggg agg gta tac aag gaa agg 2081
tta gga ctt cct cca aag ata gtg att ggt tat cag tcc cac gca gac 2129
aca gct act aag agc ggc tcc acc act aaa aat agg ttt gtt gtt taa      2177
gaagacacct tctgagtatt ctcataggag actgcgtcaa gcaatcgaga tttgggagct      2237
gaaccaaagc ctcttcaaaa agcagagtgg actgcattta aatttgattt ccatcttaat      2297
gttactcaga tataagagaa gtctcattcg cctttgtctt gtacttctgt gttcattttt      2357
tttttttttt ttggctagag tttccactat cccaatcaaa gaattacagt acacatcccc      2417
agaatccata aatgtgttcc tggcccactc tgtaatagtt cagtagaatt accattaatt      2477
acatacagat tttacctatc cacaatagtc agaaaacaac ttggcatttc tatactttac      2537
aggaaaaaaa attctgttgt tccattttat gcagaagcat attttgctgg tttgaaagat      2597
tatgatgcat acagttttct agcaattttc tttgtttctt tttacagcat tgtctttgct      2657
gtactcttgc tgatggctgc tagattttaa tttatttgtt tccctacttg ataatattag      2717
tgattctgat ttcagttttt catttgtttt gcttttgttt ttttcctcat gtaacattgg      2777
tgaaggatcc aggaatatga cacaaaggtg gaataaacat taattttgtg cattctttgg      2837
taattttttt tgttttttgt aactacaaag ctttgctaca aatttatgca tttcattcaa      2897
atcagtgatc tatgtttgtg tgatttccta aacataattg tggattataa aaaatgtaac      2957
atcataatta cattcctaac tagaattagt atgtctgttt ttgtatcttt atgctgtatt      3017
ttaacacttt gtattactta ggttattttg ctttggttaa aaatggctca agtagaaaag      3077
cagtcccatt catattaaga cagtgtacaa aactgtaaat aaaatgtgta cagtgaattg      3137
tcttttagac aactagattt gtccttttat ttctccatct ttatagaagg aatttgtact      3197
tcttattgca aggcagtctc tatattatgt cttcttttgt ggtgtcttcc atgtgaacag      3257
cataagtttg gagcactagt ttgattatta tgtttattac aatttttaat aaattgaata      3317
ggtagtatca tatatatgga attaaattga tgtggctatc tttgtttttt tataaagtaa      3377
ggcacagtca ttcagtctta ggtaaataat gtactctctt aatatgttaa tactcatgag      3437
aattgggatc tgatgcatca ccatttgatt ggtagcaaca gtggttgtaa aacttggttg      3497
ctgaattgag ttgtttctat gttaagtgtc aaaatgatag tgtagggaaa gtacaggtgg      3557
tggggacata tgcattaaga atcttgttag tgttgcaatc taaatagaat ggaataaaca      3617
ggtgttaaga catatttata gtggtaaatt gttgtagtat ggtattctgt aaacttgaaa      3677
acttgatcta ctctttgtag gtatcatttg aaagcaaact tgaaaatgtt ttgtacatag      3737
tacatacttg tatagtcctg tgagatgaag tatggctatc agaccaaagg ataagccaaa      3797
ctgtaggtag cagaatggaa attattattt tgagaggaaa atttgtcttt gaatggtgat      3857
tatgacttaa tcattttaaa actgataaac ttgacaaaaa ccctgtatga aataaacatg 3917
aaattaatag cactgatttc attgtaaaat tttaaagcag tttaaagggt accacaggtt 3977
atcacagtac tctcaatgcc acaaacacct cttgttcagt attctagaaa tactgaatca 4037
gaattctgtg tttattataa tctcagcata ctgtacataa tatctgctag ttaaacttgg 4097
gtaattggtt aaggtgactt actgtctatg tcaatatgta tagttttgag tacttcaaga 4157
gtttacttaa aagtgatgat gttactggta tgttggcagt gggtgggact gaagtagtgt 4217
atctattata aattgatcta ttttcttaat tctaagatga agtccaattt taagcatcag 4277
cttttaggtg caaaggagga attaacacat taaatgtata cagttctaaa tttttgaaat 4337
aactgatgtg tagcatttga ttattggtat taccatttta gaatcatgat gttattttaa 4397
acctttttcc tggggacaag aaaggataat aaattacgct gaatcacttt tggcagttgc 4457
cacttaaata gtacagtgac ttgcaacttt tataacttta tcagcatctt ctctaaatac 4517
aaaattaggc tatatgttat tttccaactt actgttttct ctctgtttag caggatatta 4577
taaatagatt aaatagatat attttctttt tttttttttt tttttgagac ggagtctcgc 4637
tttgtctccc aggctggagt gcagtggcgt gatctcccag tagctgggac tacaagcacc 4697
tgccaccatg cccggctaat tttttttgta tttttagtag agacggggtt tc 4749

<210> 100
<211> 217
<212> PRT
<213> Homo sapiens

<400> 100

<210> 101
<211> 1843
<212> DNA
<213> Homo sapiens

<220>
<221> CDS
<222> (208)..(1566)

<400> 101
agggacggag gcccgcgctg cccaagagcg ccacgggcgg ggcggggccg gcggcgggct 60

gcgggcgcgg ccggacggga gttccccgga gaaggatcct gcagcccgag tcccgaggat 120

aaagcttggg gttcatcctc cttccctgga tcactccaca gtcctcaggc ttccccaatc 180

caggggactc ggcgccggga cgctgct atg gac gac att ttc act cag tgc cgg 234
Met Asp Asp Ile Phe Thr Gln Cys Arg
1               5

gag ggc aac gca gtc gcc gtt cgc ctg tgg ctg gac aac acg gag aac 282
Glu Gly Asn Ala Val Ala Val Arg Leu Trp Leu Asp Asn Thr Glu Asn
10                  15                  20                  25

gac ctc aac cag ggg gac gat cat ggc ttc tcc ccc ttg cac tgg gcc 330
Asp Leu Asn Gln Gly Asp Asp His Gly Phe Ser Pro Leu His Trp Ala
                30                  35                  40

tgc cga gag ggc cgc tct gct gtg gtt gag atg ttg atc atg cgg ggg 378
Cys Arg Glu Gly Arg Ser Ala Val Val Glu Met Leu Ile Met Arg Gly
            45                  50                  55

gca cgg atc aat gta atg aac cgt ggg gat gac acc ccc ctg cat ctg 426
Ala Arg Ile Asn Val Met Asn Arg Gly Asp Asp Thr Pro Leu His Leu
        60                  65                  70

gca gcc agt cat gga cac cgt gat att gta cag aag cta ttg cag tac 474
Ala Ala Ser His Gly His Arg Asp Ile Val Gln Lys Leu Leu Gln Tyr
    75                  80                  85

aag gca gac atc aat gca gtg aat gaa cac ggg aat gtg ccc ctg cac 522
Lys Ala Asp Ile Asn Ala Val Asn Glu His Gly Asn Val Pro Leu His
90                  95                  100                 105

tat gcc tgt ttt tgg ggc caa gat caa gtg gca gag gac ctg gtg gca 570
Tyr Ala Cys Phe Trp Gly Gln Asp Gln Val Ala Glu Asp Leu Val Ala
                110                 115                 120

aat ggg gcc ctt gtc agc atc tgt aac aag tat gga gag atg cct gtg 618
Asn Gly Ala Leu Val Ser Ile Cys Asn Lys Tyr Gly Glu Met Pro Val
            125                 130                 135

gac aaa gcc aag gca ccc ctg aga gag ctt ctc cga gag cgg gca gag 666
Asp Lys Ala Lys Ala Pro Leu Arg Glu Leu Leu Arg Glu Arg Ala Glu
        140                 145                 150

aag atg ggc cag aat ctc aac cgt att cca tac aag gac aca ttc tgg 714
Lys Met Gly Gln Asn Leu Asn Arg Ile Pro Tyr Lys Asp Thr Phe Trp
    155                 160                 165

aag ggg acc acc cgc act cgg ccc cga aat gga acc ctg aac aaa cac 762
Lys Gly Thr Thr Arg Thr Arg Pro Arg Asn Gly Thr Leu Asn Lys His
170                 175                 180                 185

tct ggc att gac ttc aaa cag ctt aac ttc ctg acg aag ctc aac gag 810
Ser Gly Ile Asp Phe Lys Gln Leu Asn Phe Leu Thr Lys Leu Asn Glu
                190                 195                 200

aat cac tct gga gag cta tgg aag ggc cgc tgg cag ggc aat gac att 858
Asn His Ser Gly Glu Leu Trp Lys Gly Arg Trp Gln Gly Asn Asp Ile
            205                 210                 215

gtc gtg aag gtg ctg aag gtt cga gac tgg agt aca agg aag agc agg 906
Val Val Lys Val Leu Lys Val Arg Asp Trp Ser Thr Arg Lys Ser Arg
        220                 225                 230

gac ttc aat gaa gag tgt ccc cgg ctc agg att ttc tcg cat cca aat 954
Asp Phe Asn Glu Glu Cys Pro Arg Leu Arg Ile Phe Ser His Pro Asn
    235                 240                 245

gtg ctc cca gtg cta ggt gcc tgc cag tct cca cct gct cct cat cct 1002
Val Leu Pro Val Leu Gly Ala Cys Gln Ser Pro Pro Ala Pro His Pro
250                 255                 260                 265

act ctc atc aca cac tgg atg ccg tat gga tcc ctc tac aat gta cta 1050
Thr Leu Ile Thr His Trp Met Pro Tyr Gly Ser Leu Tyr Asn Val Leu
                270                 275                 280

cat gaa ggc acc aat ttc gtc gtg gac cag agc cag gct gtg aag ttt 1098
His Glu Gly Thr Asn Phe Val Val Asp Gln Ser Gln Ala Val Lys Phe
            285                 290                 295

gct ttg gac atg gca agg ggc atg gcc ttc cta cac aca cta gag ccc 1146
Ala Leu Asp Met Ala Arg Gly Met Ala Phe Leu His Thr Leu Glu Pro

300                 305                 310

ctc atc cca cga cat gca ctc aat agc cgt agt gta atg att gat gag       1194
Leu Ile Pro Arg His Ala Leu Asn Ser Arg Ser Val Met Ile Asp Glu
    315                 320                 325

gac atg act gcc cga att agc atg gct gat gtc aag ttc tct ttc caa       1242
Asp Met Thr Ala Arg Ile Ser Met Ala Asp Val Lys Phe Ser Phe Gln
330                 335                 340                 345

tgt cct ggt cgc atg tat gca cct gcc tgg gta gcc ccc gaa gct ctg       1290
Cys Pro Gly Arg Met Tyr Ala Pro Ala Trp Val Ala Pro Glu Ala Leu
                350                 355                 360

cag aag aag cct gaa gac aca aac aga cgc tca gca gac atg tgg agt       1338
Gln Lys Lys Pro Glu Asp Thr Asn Arg Arg Ser Ala Asp Met Trp Ser
            365                 370                 375

ttt gca gtg ctt ctg tgg gaa ctg gtg aca cgg gag gta ccc ttt gct       1386
Phe Ala Val Leu Leu Trp Glu Leu Val Thr Arg Glu Val Pro Phe Ala
        380                 385                 390

gac ctc tcc aat atg gag att gga atg aag gtg gca ttg gaa ggc ctt       1434
Asp Leu Ser Asn Met Glu Ile Gly Met Lys Val Ala Leu Glu Gly Leu
    395                 400                 405

cgg cct acc atc cca cca ggt att tcc cct cat gtg tgt aag ctc atg       1482
Arg Pro Thr Ile Pro Pro Gly Ile Ser Pro His Val Cys Lys Leu Met
410                 415                 420                 425

aag atc tgc atg aat gaa gac cct gca aag cga ccc aaa ttt gac atg       1530
Lys Ile Cys Met Asn Glu Asp Pro Ala Lys Arg Pro Lys Phe Asp Met
                430                 435                 440

att gtg cct atc ctt gag aag atg cag gac aag tag gactggaagg            1576
Ile Val Pro Ile Leu Glu Lys Met Gln Asp Lys
            445                 450

tccttgcctg aactccagag gtgtcgggac atggttgggg gaatgcacct ccccaaagca     1636

gcaggcctct ggttgcctcc cccgcctcca gtcatggtac taccccagcc atggggtcca     1696

tccccttccc ccatccctac cactgtggcc ccaagagggg cgggctcaga gctttgtcac     1756

ttgccacatg gtgtctccca acatgggagg gatcagcccc gcctgtcaca ataaagttta     1816

ttatgaaaac aggaaaaaaa aaaaaaa                                         1843

<210>  102
<211>  452
<212>  PRT
<213>  Homo sapiens

<400>  102

Met Asp Asp Ile Phe Thr Gln Cys Arg Glu Gly Asn Ala Val Ala Val
1               5                   10                  15

Arg Leu Trp Leu Asp Asn Thr Glu Asn Asp Leu Asn Gln Gly Asp Asp
            20                  25                  30

His Gly Phe Ser Pro Leu His Trp Ala Cys Arg Glu Gly Arg Ser Ala
        35                  40                  45

Val Val Glu Met Leu Ile Met Arg Gly Ala Arg Ile Asn Val Met Asn
    50                  55                  60

Arg Gly Asp Asp Thr Pro Leu His Leu Ala Ala Ser His Gly His Arg
65                  70                  75                  80

Asp Ile Val Gln Lys Leu Leu Gln Tyr Lys Ala Asp Ile Asn Ala Val
                85                  90                  95

Asn Glu His Gly Asn Val Pro Leu His Tyr Ala Cys Phe Trp Gly Gln
            100                 105                 110

Asp Gln Val Ala Glu Asp Leu Val Ala Asn Gly Ala Leu Val Ser Ile
        115                 120                 125

Cys Asn Lys Tyr Gly Glu Met Pro Val Asp Lys Ala Lys Ala Pro Leu
    130                 135                 140

Arg Glu Leu Leu Arg Glu Arg Ala Glu Lys Met Gly Gln Asn Leu Asn
145                 150                 155                 160

Arg Ile Pro Tyr Lys Asp Thr Phe Trp Lys Gly Thr Thr Arg Thr Arg
                165                 170                 175

Pro Arg Asn Gly Thr Leu Asn Lys His Ser Gly Ile Asp Phe Lys Gln
            180                 185                 190

Leu Asn Phe Leu Thr Lys Leu Asn Glu Asn His Ser Gly Glu Leu Trp
        195                 200                 205

Lys Gly Arg Trp Gln Gly Asn Asp Ile Val Val Lys Val Leu Lys Val
    210                 215                 220

Arg Asp Trp Ser Thr Arg Lys Ser Arg Asp Phe Asn Glu Glu Cys Pro
225                 230                 235                 240

Arg Leu Arg Ile Phe Ser His Pro Asn Val Leu Pro Val Leu Gly Ala
                245                 250                 255

Cys Gln Ser Pro Pro Ala Pro His Pro Thr Leu Ile Thr His Trp Met
            260                 265                 270

Pro Tyr Gly Ser Leu Tyr Asn Val Leu His Glu Gly Thr Asn Phe Val
        275                 280                 285

Val Asp Gln Ser Gln Ala Val Lys Phe Ala Leu Asp Met Ala Arg Gly
    290                 295                 300

Met Ala Phe Leu His Thr Leu Glu Pro Leu Ile Pro Arg His Ala Leu
305                 310                 315                 320

Asn Ser Arg Ser Val Met Ile Asp Glu Asp Met Thr Ala Arg Ile Ser
                325                 330                 335

Met Ala Asp Val Lys Phe Ser Phe Gln Cys Pro Gly Arg Met Tyr Ala
            340                 345                 350

Pro Ala Trp Val Ala Pro Glu Ala Leu Gln Lys Lys Pro Glu Asp Thr
        355                 360                 365

Asn Arg Arg Ser Ala Asp Met Trp Ser Phe Ala Val Leu Leu Trp Glu
    370                 375                 380

Leu Val Thr Arg Glu Val Pro Phe Ala Asp Leu Ser Asn Met Glu Ile
385                 390                 395                 400

Gly Met Lys Val Ala Leu Glu Gly Leu Arg Pro Thr Ile Pro Pro Gly
                405                 410                 415

Ile Ser Pro His Val Cys Lys Leu Met Lys Ile Cys Met Asn Glu Asp
            420                 425                 430

Pro Ala Lys Arg Pro Lys Phe Asp Met Ile Val Pro Ile Leu Glu Lys
        435                 440                 445

Met Gln Asp Lys
    450

<210>  103
<211>  2329
<212>  DNA
<213>  Homo sapiens

<220>
<221>  CDS
<222>  (627)..(950)

<400>  103
cagcacctgg gaaacgcccg cgcggtttgg ggcaggcgtg gccgtcgggg cggagggcgg      60

tggccggccg cggtccgggc gtcaggccgg gcggcggggg cggcagggag gcggcgctct     120

ggggccgcag gcggacgtac tctgtgagcg gcgcccgggc gcctgggcag gcgcctcccg     180

agtaccggcg cccattttgc ccgcccgcta aggagacccg tgcgcggcct acacgcttgg 240

gggacagccc tttaagacga catctccgcg aacgccggcc tcttgagcca gggggacgct 300

cttttgaaag tcagtcccaa aacagttaag gttttatttt attttgtttt atttccaagt 360

cacagccccc gaatctcgta caactctccc tggaccactt ccggttgccg gcccgcgcct 420

cataacttcg gttcgagtga cctcggcccg ggatgcccct cggctcaccc cgggctcacg 480

tcgcgatcct ggcttgagtg gcccagggtg ggagacctgg aagttttagc ctagctgttc 540

tcaaggcgga ttaattagcc caagaaacat tatattcatg cttaggtgga gaaaagcagg 600

gctagaattg gaacccaaag cccaga atg gca gga gag gat gtg ggg gct cca 653
Met Ala Gly Glu Asp Val Gly Ala Pro
1               5

ccc gat cac ctc tgg gtt cac caa gag ggt atc tac cgc gac gaa tac 701
Pro Asp His Leu Trp Val His Gln Glu Gly Ile Tyr Arg Asp Glu Tyr
10                  15                  20                  25

cag cgc acg tgg gtg gcc gtc gtg gaa gag gag acg agt ttc cta agg 749
Gln Arg Thr Trp Val Ala Val Val Glu Glu Glu Thr Ser Phe Leu Arg
                30                  35                  40

gca cga gtc cag caa att cag gtt ccc tta ggt gac gca gct agg cca 797
Ala Arg Val Gln Gln Ile Gln Val Pro Leu Gly Asp Ala Ala Arg Pro
            45                  50                  55

agt cac ctt ctt acc tcc cag cta cct ctc atg tgg caa ctc tac ccg 845
Ser His Leu Leu Thr Ser Gln Leu Pro Leu Met Trp Gln Leu Tyr Pro
        60                  65                  70

gag gag cgc tac atg gat aac aac tct cgc ttg tgg cag ata cag cat 893
Glu Glu Arg Tyr Met Asp Asn Asn Ser Arg Leu Trp Gln Ile Gln His
    75                  80                  85

cat tta atg gtc agg gga gta cag gag ctg ttg ctt aag ctt ttg cct 941
His Leu Met Val Arg Gly Val Gln Glu Leu Leu Leu Lys Leu Leu Pro
90                  95                  100                 105

gat gac taa cctggtgctg ggattctacg aagatatgct cctttgttct 990
Asp Asp

gttcagtatt tggcaatcac ttcatccact actgcagtgc acccaccctt gggcctgggg 1050

ggagggagtg ggttgaaaaa ctgctcaaga aacagaagtt tagcaagggt catgaagaat 1110

actgcaagtg aaactgcaga gagaggttac gtaggcagaa agcaagtcaa caaaagcact 1170

tagtcaggat ccgtaacttg aaattgactc ctttggaaat tgccatagaa cccttaatgg 1230

acatcatcgg ctggacctgg gatctgatga atcccacaaa agtcagcacc ttctacagaa 1290

cagatgccct gatcaccaag gacttggtac tgatttagag agaagagagc agctcctagc 1350

agcatcaaca tctatttgtc gcttatttgc cctgcagcaa ttcacctgcc tttccttctc 1410

ccatcctgta aatatcctag gttttgctcc agtgtttccc atcccagtac tgacctaatt 1470

tggatctgct gagtatttag agggctctga aaagagatat gcattgatga aattagctac 1530

aaaggcttgt gggccttttt ttctatctga aatcgtgtct tcaaattact ggaagttgct 1590

gatcaaaatt gtgggctctt gaattcattc tctgattaca gatattttaa ataagctggt 1650

ttctataaga acttcaagaa aaaccatttt agctgcttaa ctggcacctt tctcaggaag 1710

taataacacc aaatattgct gattcctagg aaaacattta ctacttgtaa agaatttcct 1770

gagttaaagt agttataaca gtttatatac attaaaagta aggttttact ttaaaaaaaa 1830

aagtcttggg aataatagct accatgtatt ggagtccttt ccaggcacta ttgtatgtac 1890

tttatatact ttatcacatt taatcctcct gtcaatgctt tgaggtaggt atgattccca 1950

ttttatatat gagtaataaa aaaagctcaa ggagtttaat catttatcaa gggtttcttc 2010

tgcgaatgat agagccaggc tgcgccgtcg gctcctctag cctaacgttg gtttacagta 2070

ttcaaaaaga gttgattgaa ttcaaggttt tttttccatg taaagctatt tatatcttcc 2130

aagcctaatg aaatggatat tataaaaata cagttgattc atcctatgtt tgggaagagt 2190

atttttagtg taattatttt ccagcaattg taagggcata tactgcttgt ctcagaagaa 2250

aattccacgt agaggggctt taaagtcttt atggaaaaaa gaaaaataaa aatcattttt 2310

cttgttttag aaaaaaaaa 2329

<210> 104
<211> 107
<212> PRT
<213> Homo sapiens

<400> 104

Met Ala Gly Glu Asp Val Gly Ala Pro Pro Asp His Leu Trp Val His
1               5                   10                  15

Gln Glu Gly Ile Tyr Arg Asp Glu Tyr Gln Arg Thr Trp Val Ala Val
            20                  25                  30

Val Glu Glu Glu Thr Ser Phe Leu Arg Ala Arg Val Gln Gln Ile Gln
        35                  40                  45

Val Pro Leu Gly Asp Ala Ala Arg Pro Ser His Leu Leu Thr Ser Gln
    50                  55                  60

Leu Pro Leu Met Trp Gln Leu Tyr Pro Glu Glu Arg Tyr Met Asp Asn
65                  70                  75                  80

Asn Ser Arg Leu Trp Gln Ile Gln His His Leu Met Val Arg Gly Val
                85                  90                  95

Gln Glu Leu Leu Leu Lys Leu Leu Pro Asp Asp
            100                 105

<210>  105
<211>  3724
<212>  DNA
<213>  Homo sapiens


<220>
<221>  CDS
<222>  (158)..(3364)

<400>  105
tctccctcgg cgccgccgcc gccgcccgcg gggctgggac ccgatgcggt tagagccgcg      60
gagcctggaa gagccccgag cgtttctgct ttgggacaac catacatcta attccttaaa     120
gtagttttat atgtaaaact tgcaaagaat cagaaca atg cct cca cga cca tca      175
tca ggt gaa ctg tgg ggc atc cac ttg atg ccc cca aga atc cta gta      223
gaa tgt tta cta cca aat gga atg ata gtg act tta gaa tgc ctc cgt      271
gag gct aca tta ata acc ata aag cat gaa cta ttt aaa gaa gca aga      319
aaa tac ccc ctc cat caa ctt ctt caa gat gaa tct tct tac att ttc      367
gta agt gtt act caa gaa gca gaa agg gaa gaa ttt ttt gat gaa aca      415
aga cga ctt tgt gac ctt cgg ctt ttt caa ccc ttt tta aaa gta att      463
gaa cca gta ggc aac cgt gaa gaa aag atc ctc aat cga gaa att ggt      511
ttt gct atc ggc atg cca gtg tgt gaa ttt gat atg gtt aaa gat cca      559
gaa gta cag gac ttc cga aga aat att ctg aac gtt tgt aaa gaa gct      607
gtg gat ctt agg gac ctc aat tca cct cat agt aga gca atg tat gtc      655
tat cct cca aat gta gaa tct tca cca gaa ttg cca aag cac ata tat      703
aat aaa tta gat aaa ggg caa ata ata gtg gtg atc tgg gta ata gtt        751
tct cca aat aat gac aag cag aag tat act ctg aaa atc aac cat gac        799
tgt gta cca gaa caa gta att gct gaa gca atc agg aaa aaa act cga        847
agt atg ttg cta tcc tct gaa caa cta aaa ctc tgt gtt tta gaa tat        895
cag ggc aag tat att tta aaa gtg tgt gga tgt gat gaa tac ttc cta        943
gaa aaa tat cct ctg agt cag tat aag tat ata aga agc tgt ata atg        991
ctt ggg agg atg ccc aat ttg atg ttg atg gct aaa gaa agc ctt tat       1039
tct caa ctg cca atg gac tgt ttt aca atg cca tct tat tcc aga cgc       1087
att tcc aca gct aca cca tat atg aat gga gaa aca tct aca aaa tcc       1135
ctt tgg gtt ata aat agt gca ctc aga ata aaa att ctt tgt gca acc       1183
tac gtg aat gta aat att cga gac att gat aag atc tat gtt cga aca       1231
ggt atc tac cat gga gga gaa ccc tta tgt gac aat gtg aac act caa       1279
aga gta cct tgt tcc aat ccc agg tgg aat gaa tgg ctg aat tat gat       1327
ata tac att cct gat ctt cct cgt gct gct cga ctt tgc ctt tcc att       1375
tgc tct gtt aaa ggc cga aag ggt gct aaa gag gaa cac tgt cca ttg       1423
gca tgg gga aat ata aac ttg ttt gat tac aca gac act cta gta tct       1471
gga aaa atg gct ttg aat ctt tgg cca gta cct cat gga tta gaa gat        1519
ttg ctg aac cct att ggt gtt act gga tca aat cca aat aaa gaa act        1567
cca tgc tta gag ttg gag ttt gac tgg ttc agc agt gtg gta aag ttc        1615
cca gat atg tca gtg att gaa gag cat gcc aat tgg tct gta tcc cga        1663
gaa gca gga ttt agc tat tcc cac gca gga ctg agt aac aga cta gct        1711
aga gac aat gaa tta agg gaa aat gac aaa gaa cag ctc aaa gca att        1759
tct aca cga gat cct ctc tct gaa atc act gag cag gag aaa gat ttt        1807
cta tgg agt cac aga cac tat tgt gta act atc ccc gaa att cta ccc        1855
aaa ttg ctt ctg tct gtt aaa tgg aat tct aga gat gaa gta gcc cag        1903
atg tat tgc ttg gta aaa gat tgg cct cca atc aaa cct gaa cag gct        1951
atg gaa ctt ctg gac tgt aat tac cca gat cct atg gtt cga ggt ttt        1999
gct gtt cgg tgc ttg gaa aaa tat tta aca gat gac aaa ctt tct cag        2047
tat tta att cag cta gta cag gtc cta aaa tat gaa caa tat ttg gat        2095
aac ttg ctt gtg aga ttt tta ctg aag aaa gca ttg act aat caa agg        2143
att ggg cac ttt ttc ttt tgg cat tta aaa tct gag atg cac aat aaa        2191
aca gtt agc cag agg ttt ggc ctg ctt ttg gag tcc tat tgt cgt gca 2239
tgt ggg atg tat ttg aag cac ctg aat agg caa gtc gag gca atg gaa 2287
aag ctc att aac tta act gac att ctc aaa cag gag aag aag gat gaa 2335
aca caa aag gta cag atg aag ttt tta gtt gag caa atg agg cga cca 2383
gat ttc atg gat gct cta cag ggc ttt ctg tct cct cta aac cct gct 2431
cat caa cta gga aac ctc agg ctt gaa gag tgt cga att atg tcc tct 2479
gca aaa agg cca ctg tgg ttg aat tgg gag aac cca gac atc atg tca 2527
gag tta ctg ttt cag aac aat gag atc atc ttt aaa aat ggg gat gat 2575
tta cgg caa gat atg cta aca ctt caa att att cgt att atg gaa aat 2623
atc tgg caa aat caa ggt ctt gat ctt cga atg tta cct tat ggt tgt 2671
ctg tca atc ggt gac tgt gtg gga ctt att gag gtg gtg cga aat tct 2719
cac act att atg caa att cag tgc aaa ggc ggc ttg aaa ggt gca ctg 2767
cag ttc aac agc cac aca cta cat cag tgg ctc aaa gac aag aac aaa 2815
gga gaa ata tat gat gca gcc att gac ctg ttt aca cgt tca tgt gct 2863
gga tac tgt gta gct acc ttc att ttg gga att gga gat cgt cac aat 2911
agt aac atc atg gtg aaa gac gat gga caa ctg ttt cat ata gat ttt 2959
gga cac ttt ttg gat cac aag aag aaa aaa ttt ggt tat aaa cga gaa 3007
cgt gtg cca ttt gtt ttg aca cag gat ttc tta ata gtg att agt aaa 3055
gga gcc caa gaa tgc aca aag aca aga gaa ttt gag agg ttt cag gag 3103
atg tgt tac aag gct tat cta gct att cga cag cat gcc aat ctc ttc 3151
ata aat ctt ttc tca atg atg ctt ggc tct gga atg cca gaa cta 3196
caa tct ttt gat gac att gca tac att cga aag acc cta gcc tta 3241
gat aaa act gag caa gag gct ttg gag tat ttc atg aaa caa atg 3286
aat gat gca cat cat ggt ggc tgg aca aca aaa atg gat tgg atc 3331
ttc cac aca att aaa cag cat gca ttg aac tga aaagataact 3374
gagaaaatga aagctcactc tggattccac actgcactgt taataactct cagcaggcaa 3434
agaccgattg cataggaatt gcacaatcca tgaacagcat tagaatttac agcaagaaca 3494
gaaataaaat actatataat ttaaataatg taaacgcaaa cagggtttga tagcacttaa 3554
actagttcat ttcaaaatta agctttagaa taatgcgcaa tttcatgtta tgccttaagt 3614
ccaaaaaggt aaactttgaa gattgtttgt atcttttttt aaaaaacaaa acaaaacaaa 3674
aatccccaaa atatatagaa atgatggaga aggaaaaaaa aaaaaaaaaa 3724

<210> 106
<211> 1068
<212> PRT
<213> Homo sapiens

<400> 106

<210> 107
<211> 4230
<212> DNA
<213> Homo sapiens

<220>
<221> CDS
<222> (363)..(1889)

<400> 107
gaagggtcgg gggatccctc cgccgccagc gcgtggtccc ggccccctcc acccgccgtc 60

tcggccgcgg ccagcagccc ctgccccccg ggggacgctg acggccgccc ggcgcgccgc 120

cctagcagac ggacaggggg cgctgcgcgc ggcctggggc aacccgggcc acaggggcag 180

gaaagtgagg gcccaggtcg gcccgggcgt gcaggggccc cgggttcgca gcggcggccg 240

cggcagcgat agcggcacta gcagcagcgg gagtgccggg ttgagccggg aagccgatgg 300

cggcggctgc ggcggctccg attcctcgct gactgcccgt ccgccctcct gcatcgagcg 360

cc atg tta ccg acc caa gct ggg gcc gcg gcg gct ctg ggc cgg ggc 407
Met Leu Pro Thr Gln Ala Gly Ala Ala Ala Ala Leu Gly Arg Gly
1               5                   10                  15

tcg gcc ctg ggg ggc agc ctg aac cgg acc ccg acg ggg cgg ccg ggc 455
Ser Ala Leu Gly Gly Ser Leu Asn Arg Thr Pro Thr Gly Arg Pro Gly
                20                  25                  30

ggc ggc ggc ggg aca cgc ggg gct aac ggg ggc cgg gtc ccc ggg aat 503
Gly Gly Gly Gly Thr Arg Gly Ala Asn Gly Gly Arg Val Pro Gly Asn
            35                  40                  45

ggc gcg ggg ctc ggg ccc ggc cgc ctg gag cgg gag gct gcg gca gcg 551
Gly Ala Gly Leu Gly Pro Gly Arg Leu Glu Arg Glu Ala Ala Ala Ala
        50                  55                  60

gcg gca acc acc ccg gcg ccc acc gcg ggg gcc ctc tac agc ggc agc 599
Ala Ala Thr Thr Pro Ala Pro Thr Ala Gly Ala Leu Tyr Ser Gly Ser
    65                  70                  75

gag ggc gac tcg gag tcg ggc gag gag gag gag ctg ggc gcc gag cgg 647
Glu Gly Asp Ser Glu Ser Gly Glu Glu Glu Glu Leu Gly Ala Glu Arg
80                  85                  90                  95

cgc ggc ctg aag cgg agc ctg agc gag atg gag atc ggt atg gtg gtc 695
Arg Gly Leu Lys Arg Ser Leu Ser Glu Met Glu Ile Gly Met Val Val
                100                 105                 110

ggt ggg ccc gag gcg tcg gca gcg gcc acc ggg ggc tac ggg ccg gtg 743
Gly Gly Pro Glu Ala Ser Ala Ala Ala Thr Gly Gly Tyr Gly Pro Val

115                 120                 125

agc ggc gcg gtg agc ggg gcc aag ccg ggt aag aag acc cgg ggc cgc      791
Ser Gly Ala Val Ser Gly Ala Lys Pro Gly Lys Lys Thr Arg Gly Arg
        130                 135                 140

gtg aag atc aag atg gag ttc atc gac aac aag ctg cgg cgc tac acg      839
Val Lys Ile Lys Met Glu Phe Ile Asp Asn Lys Leu Arg Arg Tyr Thr
    145                 150                 155

acc ttc agc aag agg aag acg ggc atc atg aag aag gcc tat gag ctg      887
Thr Phe Ser Lys Arg Lys Thr Gly Ile Met Lys Lys Ala Tyr Glu Leu
160                 165                 170                 175

tcc acg ctg aca ggg aca cag gtg ctg ttg ctg gtg gcc agt gag aca      935
Ser Thr Leu Thr Gly Thr Gln Val Leu Leu Leu Val Ala Ser Glu Thr
                180                 185                 190

ggc cat gtg tat acc ttt gcc acc cga aaa ctg cag ccc atg atc acc      983
Gly His Val Tyr Thr Phe Ala Thr Arg Lys Leu Gln Pro Met Ile Thr
            195                 200                 205

agt gag acc ggc aag gca ctg att cag acc tgc ctc aac tcg cca gac      1031
Ser Glu Thr Gly Lys Ala Leu Ile Gln Thr Cys Leu Asn Ser Pro Asp
        210                 215                 220

tct cca ccc cgt tca gac ccc aca aca gac cag aga atg agt gcc act      1079
Ser Pro Pro Arg Ser Asp Pro Thr Thr Asp Gln Arg Met Ser Ala Thr
    225                 230                 235

ggc ttt gaa gag aca gat ctc acc tac cag gtg tcg gag tct gac agc      1127
Gly Phe Glu Glu Thr Asp Leu Thr Tyr Gln Val Ser Glu Ser Asp Ser
240                 245                 250                 255

agt ggg gag acc aag gac aca ctg aag ccg gcg ttc aca gtc acc aac      1175
Ser Gly Glu Thr Lys Asp Thr Leu Lys Pro Ala Phe Thr Val Thr Asn
                260                 265                 270

ctg ccg ggt aca acc tcc acc atc caa aca gca cct agc acc tct acc      1223
Leu Pro Gly Thr Thr Ser Thr Ile Gln Thr Ala Pro Ser Thr Ser Thr
            275                 280                 285

acc atg caa gtc agc agc ggc ccc tcc ttt ccc atc acc aac tac ctg      1271
Thr Met Gln Val Ser Ser Gly Pro Ser Phe Pro Ile Thr Asn Tyr Leu
        290                 295                 300

gca cca gtg tct gct agt gtc agc ccc agt gct gtc agc agt gcc aat      1319
Ala Pro Val Ser Ala Ser Val Ser Pro Ser Ala Val Ser Ser Ala Asn
    305                 310                 315

ggg act gtg ctg aag agt aca ggc agc ggc cct gtc tcc tct ggg ggc      1367
Gly Thr Val Leu Lys Ser Thr Gly Ser Gly Pro Val Ser Ser Gly Gly
320                 325                 330                 335

ctt atg cag ctg cct acc agc ttc acc ctc atg cct ggt ggg gca gtg      1415
Leu Met Gln Leu Pro Thr Ser Phe Thr Leu Met Pro Gly Gly Ala Val
                340                 345                 350

gcc cag cag gtc cca gtg cag gcc att caa gtg cac cag gcc cca cag      1463
Ala Gln Gln Val Pro Val Gln Ala Ile Gln Val His Gln Ala Pro Gln
            355                 360                 365

caa gcg tct ccc tcc cgt gac agc agc aca gac ctc acg cag acc tcc      1511

Gln Ala Ser Pro Ser Arg Asp Ser Ser Thr Asp Leu Thr Gln Thr Ser
        370                 375                 380

tcc agc ggg aca gtg acg ctg ccc gcc acc atc atg acg tca tcc gtg      1559
Ser Ser Gly Thr Val Thr Leu Pro Ala Thr Ile Met Thr Ser Ser Val
    385                 390                 395

ccc aca act gtg ggt ggc cac atg atg tac cct agc ccg cat gcg gtg      1607
Pro Thr Thr Val Gly Gly His Met Met Tyr Pro Ser Pro His Ala Val
400                 405                 410                 415

atg tat gcc ccc acc tcg ggc ctg ggt gat ggc agc ctc acc gtg ctg      1655
Met Tyr Ala Pro Thr Ser Gly Leu Gly Asp Gly Ser Leu Thr Val Leu
                420                 425                 430

aat gcc ttc tcc cag gca cca tcc acc atg cag gtg tca cac agc cag      1703
Asn Ala Phe Ser Gln Ala Pro Ser Thr Met Gln Val Ser His Ser Gln
            435                 440                 445

gtc cag gag cca ggt ggc gtc ccc cag gtg ttc ctg aca gca tca tct      1751
Val Gln Glu Pro Gly Gly Val Pro Gln Val Phe Leu Thr Ala Ser Ser
        450                 455                 460

ggg aca gtg cag atc cct gtt tca gca gtt cag ctc cac cag atg gct      1799
Gly Thr Val Gln Ile Pro Val Ser Ala Val Gln Leu His Gln Met Ala
    465                 470                 475

gtg ata ggg cag cag gcc ggg agc agc agc aac ctc acc gag cta cag      1847
Val Ile Gly Gln Gln Ala Gly Ser Ser Ser Asn Leu Thr Glu Leu Gln
480                 485                 490                 495

gtg gtg aac ctg gac acc gcc cac agc acc aag agt gaa tga              1889
Val Val Asn Leu Asp Thr Ala His Ser Thr Lys Ser Glu
                500                 505

tccgcccgcc gccctggaca gatggcccaa gggatggcac cacttattta ttgttgcctt      1949

ttcacgtttt ctttacacac acgttgacgg gccgcaggag ggaggcgggg aggaggaacg      2009

ggcagccaca ggactgagcc ctctcactcc agccaaagaa atgggcctgc ctgcctccac      2069

ccgtcctccc tcagcctccc cttcttcccg ccccacctcc catttctgtt gctggagggg      2129

ctgtcctcct tcctgggacc ccctcgccag cttggctcga tgtttgccat gagtattagc      2189

ttacccaatg ggaccgtgcc ccacctcccc acacacaggc cttctgtggg gctgggcacc      2249

gtgtcctcct ctgaggaagc agttggggcc ctcttgccag cctccttgct gaccccaggt      2309

cagccctgtg tctgtcacag gctgggtcaa aagagccctg gctctgcccc tcagggggcc      2369

agctggggag atgggggctt cttcctcaca ctgctgtcct ctcccccttc agctcctgag      2429

tagctgggcc tgtgcactgg gcaggttcct ggggccgcct gccctgcctt gccgctcccc      2489

ttggacctcc aggggctcct gggttggagg gaaccaccag cgttcccttc tcccccttgt      2549

cttcccccct ctcctcccag ctgctttact taaagttgat tttgaacttt ttatttgagg      2609

agacgaagtg aaaacaaatc tataaatata tatttttaaa atatttaact tttttttatg      2669

gcgtttttct cgtccccctc cctgcccaaa ctccccttcc ctggggagcc ctcaggctcc      2729

ccagaactgg ctgggcccct ggggacagag ccaccccatg agctcggggt ccaccagtgt 2789

gtgggggaga ttctgggttt gcccagtcct gggttgtttc caggagaaag ccgggggagg 2849

ggccctcagg ccattcccca acggggtggg gagggtgacc cacagctctg ggcctctttt 2909

tgccctttag ggctgttgct agggagaggg aagagggaga ccaaatgtcg gggttggggt 2969

gggagggcgt caggcagagg caactgactt catttgtgcc acacgcatgg gcattgcagc 3029

cttgcgctgt cccaggcatg cagctgcctg gggcccaagt tgcagtgagc agggtggggt 3089

ctgggagggg gtgagaggca ggaatggggg tcagaagaag tgggagcagc ttcttgggct 3149

gagtgcagcc aaaggggagc cagaaatggg cagttctccc agggagtgag cagctactgt 3209

aactttttta aattaagaca aaaagccttg aagaaaatga ctttattttt ctaagtgtaa 3269

cctcagtatt tatgtaattt gtacaggggc catgccccac ccccctcctc cccctttggg 3329

gtagaccttg agggtgggcc agcatagggg ggagggtctt ttaccctgtg tcagagccta 3389

ccttcaccac ctatatccag aaggggagct ttttcagaaa cagggcagca gtggggtgaa 3449

attttcttaa cccctaagac tgccttcagt aggaacaagc tggcttctgt gattaggtga 3509

agggatgggg gaagatttta tgcacagcct agttatcaag gggatgattt gccgacatgt 3569

ttgagaaccc cctaacctct aaccctcatt gctgtcttgc cccagtttgg ggtgccaaga 3629

tggaagtcac ctttctgggc tttctcctgg agatagctgg ggcttatggg tggctttcaa 3689

ggctggggca tggcaaatca ggggccagag agcaggggag cttgggactc aggtctgtaa 3749

ctgcccagcc ccttttctct gctcttgttt cactccacca tcactcactc actccccact 3809

cccccaccca tggggaggag acctttgatg aattcttcct ctccttccca caaaagacag 3869

acccagtgag tgaatcaggc aaagtgctta taatgtgtgt tgtgtgagcg tggccttggg 3929

aggacatgcg tgtgtcaggg atgagttgag gtgatatttt tatgtgcagc gacccttggt 3989

gtttcccttc ctcggtggct ctggggtatg tgtgtgtggg tgtgtgcgcc tgagtgagtg 4049

tgtgtgcttg aatgtgagtg tgtatgtcag tggtttctac ttcccctggg atgctgaccc 4109

aggaatagtg gacatggtca cagtcctatg tacagagctt tcttttgtat taaaaaaaaa 4169

tactctttca ataaatgtat catttttgtg cacagaaaaa aaaaaaaaaa aaaaaaaaaa 4229

a 4230

<210> 108
<211> 508
<212> PRT
<213> Homo sapiens

<400> 108

Met Leu Pro Thr Gln Ala Gly Ala Ala Ala Leu Gly Arg Gly Ser
1               5                   10                  15

Ala Leu Gly Gly Ser Leu Asn Arg Thr Pro Thr Gly Arg Pro Gly Gly
            20                  25                  30

Gly Gly Gly Thr Arg Gly Ala Asn Gly Gly Arg Val Pro Gly Asn Gly
        35                  40                  45

Ala Gly Leu Gly Pro Gly Arg Leu Glu Arg Glu Ala Ala Ala Ala Ala
    50                  55                  60

Ala Thr Thr Pro Ala Pro Thr Ala Gly Ala Leu Tyr Ser Gly Ser Glu
65                  70                  75                  80

Gly Asp Ser Glu Ser Gly Glu Glu Glu Glu Leu Gly Ala Glu Arg Arg
                85                  90                  95

Gly Leu Lys Arg Ser Leu Ser Glu Met Glu Ile Gly Met Val Val Gly
            100                 105                 110

Gly Pro Glu Ala Ser Ala Ala Ala Thr Gly Gly Tyr Gly Pro Val Ser
        115                 120                 125

Gly Ala Val Ser Gly Ala Lys Pro Gly Lys Lys Thr Arg Gly Arg Val
    130                 135                 140

Lys Ile Lys Met Glu Phe Ile Asp Asn Lys Leu Arg Arg Tyr Thr Thr
145                 150                 155                 160

Phe Ser Lys Arg Lys Thr Gly Ile Met Lys Lys Ala Tyr Glu Leu Ser
                165                 170                 175

Thr Leu Thr Gly Thr Gln Val Leu Leu Leu Val Ala Ser Glu Thr Gly
            180                 185                 190

His Val Tyr Thr Phe Ala Thr Arg Lys Leu Gln Pro Met Ile Thr Ser
        195                 200                 205

Glu Thr Gly Lys Ala Leu Ile Gln Thr Cys Leu Asn Ser Pro Asp Ser
    210                 215                 220

Pro Pro Arg Ser Asp Pro Thr Thr Asp Gln Arg Met Ser Ala Thr Gly
225                 230                 235                 240

Phe Glu Glu Thr Asp Leu Thr Tyr Gln Val Ser Glu Ser Asp Ser Ser
                245                 250                 255

Gly Glu Thr Lys Asp Thr Leu Lys Pro Ala Phe Thr Val Thr Asn Leu
            260                 265                 270

Pro Gly Thr Thr Ser Thr Ile Gln Thr Ala Pro Ser Thr Ser Thr Thr
        275                 280                 285


    290                 295                 300


305                 310                 315                 320


                325                 330                 335


            340                 345                 350


        355                 360                 365


    370                 375                 380


385                 390                 395                 400


                405                 410                 415


            420                 425                 430


        435                 440                 445


    450                 455                 460


465                 470                 475                 480


                485                 490                 495


            500                 505

<210>  109

<211>  21
<212>  DNA
<213>  Artificial

<220>
<223>  Combined DNA/RNA molecule: Synthetic oligonucleotide


<220>
<221>  misc_feature
<222>  (1)..(19)
<223>  RNA

<400>  109
ccuuuugaga cccugcugut t                                                21


<210>  110
<211>  21
<212>  DNA
<213>  Artificial

<220>
<223>  Combined DNA/RNA molecule: Synthetic oligonucleotide


<220>
<221>  misc_feature
<222>  (1)..(19)
<223>  RNA

<400>  110
acagcagggu cucaaaaggt t                                                21


<210>  111
<211>  21
<212>  DNA
<213>  Artificial

<220>
<223>  Combined DNA/RNA molecule: Synthetic oligonucleotide


<220>
<221>  misc_feature
<222>  (1)..(19)
<223>  RNA

<400>  111
cuggggaaga aggugaguat t                                                21


<210>  112
<211>  21
<212>  DNA
<213>  Artificial

<220>
<223>  Combined DNA/RNA molecule: Synthetic oligonucleotide


<220>

<221>  misc_feature
<222>  (1)..(19)
<223>  RNA

<400>  112
uacucaccuu cuuccccagt t                                                21


<210>  113
<211>  21
<212>  DNA
<213>  Artificial

<220>
<223>  Combined DNA/RNA molecule: Synthetic oligonucleotide


<220>
<221>  misc_feature
<222>  (1)..(19)
<223>  RNA

<400>  113
caagauagcc agaguuuaat t                                                21


<210>  114
<211>  21
<212>  DNA
<213>  Artificial

<220>
<223>  Combined DNA/RNA molecule: Synthetic oligonucleotide


<220>
<221>  misc_feature
<222>  (1)..(19)
<223>  RNA

<400>  114
uuaaacucug gcuaucuugt t                                                21